(19) Europäisches Patentamt / European Patent Office / Office européen des brevets

(11) **EP 2 749 642 A1**

(12) **EUROPEAN PATENT APPLICATION**
published in accordance with Art. 153(4) EPC

(43) Date of publication:
**02.07.2014 Bulletin 2014/27**

(51) Int Cl.:
*C12N 5/095* (2010.01)     *C12N 5/09* (2010.01)
*C12N 5/10* (2006.01)

(21) Application number: **12853085.4**

(22) Date of filing: **30.11.2012**

(86) International application number:
**PCT/JP2012/081723**

(87) International publication number:
**WO 2013/081188 (06.06.2013 Gazette 2013/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2011   US 201161565064 P**

(71) Applicants:
• **National Cancer Center**
**Tokyo 104-0045 (JP)**

• **LSIP, LLC**
**Chiyoda-ku**
**Tokyo 100-0005 (JP)**

(72) Inventor: **ISHIKAWA, Tetsuya**
**Tokyo 104-0045 (JP)**

(74) Representative: **Cabinet Plasseraud**
**52, rue de la Victoire**
**75440 Paris Cedex 09 (FR)**

(54) **INDUCED MALIGNANT STEM CELLS**

(57)    There are provided induced malignant stem cells capable *of in vitro* proliferation that are useful in cancer research and drug discovery for cancer therapy, as well as processes for production thereof, cancer cells derived from these cells, and applications of these cells.

An induced malignant stem cell capable *of in vitro* proliferation are characterized by satisfying the following two requirements:
(1) having at least one aberration selected from among (a) an aberration of methylation (high or low degree of methylation) in a tumor suppressor gene or a cancer-related genetic region in endogenous genomic DNA, (b) a somatic mutation of a tumor suppressor gene or a somatic mutation of an endogenous cancer-related gene in endogenous genomic DNA, (c) abnormal expression (increased or reduced/lost expression) of an endogenous oncogene or an endogenous tumor suppressor gene, (d) abnormal expression (increased or reduced/lost expression) of a noncoding RNA such as an endogenous cancer-related microRNA, (e) abnormal expression of an endogenous cancer-related protein, (f) an aberration of endogenous cancer-related metabolism (hypermetabolism or hypometabolism), (g) an aberration of endogenous cancer-related sugar chain, (h) an aberration of copy number variations in endogenous genomic DNA , and (i) instability of microsatellites in endogenous genomic DNA in an induced malignant stem cell; and
(2) expressing genes including POU5F1 gene, NANOG gene, SOX2 gene, and ZFP42 gene.

**Description**

TECHNICAL FIELD

The present invention relates to induced malignant stem cells. More particularly, the present invention relates to induced malignant stem cells capable of in vitro proliferation that have genomic or epigenetic aberrations involved in cancer and which express four genes, POU5F1 gene (also referred to as OCT3/4 gene), NANOG gene, SOX2 gene, and ZFP42 gene, as well as processes for production thereof, cancer cells derived from these malignant stem cells, and applications of these cells.

BACKGROUND ART

In recent years, research on creation of clone animals as well as on stem cells including embryonic stem cells (also called "ES cells" but hereinafter referred to as "embryonic stem cells") has led to the postulation that epigenetics (DNA methylation and histone modification) is capable of reprogramming (also called "initializing" but hereinafter referred to as "reprogramming"). As a matter of fact, there is a report of experimental results showing that when the nucleus of a mouse melanoma cell which is a cancer cell was transplanted into an enucleated oocyte, the nucleus transplanted oocyte initiated embryogenesis, and the embryonic stem cell (also called "ES cell") obtained from the embryo differentiating into such cells as melanocytes, lymphocytes, and fibroblasts (Non-Patent Document 1).

It has recently been reported that, by transduction of OCT3/4 gene (sometimes designated as "OCT3" gene, "OCT4" gene or "POU5F1" gene), SOX2 gene, KLF4 gene, and c-MYC gene (Patent Document 1) or by transduction of OCT3/4 gene, SOX2 gene, and KLF4 gene in the presence of a basic fibroblast growth factor (bFGF) (Non-Patent Document 2), induced pluripotent stem cells which are as undifferentiated as embryonic stem cells can be prepared from human somatic cells as the result of reprogramming (Patent Document 2). Human induced pluripotent stem cells (hereinafter also called "iPS cells") are known to have two features, (1) pluripotency for differentiation into three germ layers which are capable of differentiating into all cells that form a body and (2) proliferating ability (self-renewal ability) by which the cells can be subjected to passage culture without limit in a culture dish under conditions for expansion culture of human embryonic stem cells while remaining undifferentiated state. It also has been reported that such human induced pluripotent stem cells are very similar to human embryonic stem cells in terms of morphology, gene expression, cell surface antigen, long-term proliferating ability (self-renewal ability), and teratoma (differentiation into three germ layers *in vivo)* forming ability (Non-Patent Documents 3 and 4), and that the genotypes of HLA are completely identical to those of somatic cells which are derived cells (Non-Patent Document 4). In connection with the method of preparing these cells, it is held that a differentiated somatic cell can be "reprogrammed" to an induced pluripotent stem cell (iPS cell) by simply transducing the aforementioned genes, (i.e., OCT3/4 gene, SOX2 gene, KLF4 gene, and c-MYC gene, or OCT3/4 gene, SOX2 gene, and KLF4 gene in the presence ofbFGF).

It is generally understood that on account of a genomic and/or an epigenetic aberration that is related to cancer, gene expression abnormally increases or decreases or even disappears, thus generating the carcinogenesis of cells. It is therefore postulated that by using the above-described reprogramming technology, the cancer cell having various aberrations will be reprogrammed and returned to the normal cells, having lost its cancerous properties.

As a matter of fact, a report recently made at a meeting of the International Society for Stem Cell Research (ISSCR) states as follows: "When two kinds of chemical substance including a cancer-control agent (noncyclic retinoid and tolrestat) were added to cancer stem cells derived from a human hepatocyte line (HuH7-derived CD133 positive cells) on a culture dish, 85-90% of the cancer cells were returned to normal hepatocytes in 2 days. Upon further addition of two genes (SOX2 gene and KLF4 gene) and two chemical substances (5-AZAC and TSA), the hepatocytes became iPS cells which, by means of a protocol for differentiation into hepatocytes, could successfully be differentiated into hepatocytes (AFP or ALB positive cells.)" (Non-Patent Document 5). There are also a paper describing a successful reprogramming of mouse melanoma cells as cancer cells to induced pluripotent stem cells (Non-Patent Document 6), as well as a report disclosing that, as the result of reprogramming by transduction with OCT3/4 gene, SOX2 gene, KLF4 gene, and c-MYC gene, iPS cells having lost BCR-ABL tyrosine kinase dependency were prepared from a chronic bone marrow leukemia (CML) cell line having BCR-ABL tyrosine kinase activity as an etiology of carcinogenesis (Non-Patent Document 7). According to yet another report, when OCT3/4 gene, SOX2 gene, KLF4 gene, and c-MYC gene were transduced into a cancer cell line, it was reprogrammed to lose drug resistance and tumorigenicity but an extended culture caused canceration involving the activation of the exogenous c-MYC gene transduced into the cellular genome (Non-Patent Document 8).

However, the expression of self-renewal related genes (e.g. OCT3/4 gene, SOX2 gene, NANOG gene, and ZFP42 gene) was not fully induced and, instead, c-MYC gene, an etiology of carcinogenesis, was transduced into the cellular genome (Non-Patent Document 8). Thus, reprogramming therapy which involves application of genes or chemical substances to revert the cancer cell to the normal cell holds promise as a potential cancer treatment and is being studied by many researchers (Non-Patent Document 9).

The fact, however, is that even if the cancer cell can be reprogrammed to the normal cell, a clinically successful cancer treatment requires that cancer cells in the living body rather than on a culture dish be reprogrammed to the normal cell in a 100% efficiency. What is more, even an early-stage cancer which is generally detected by imaging test is considered to consist of as many as a hundred million cancer cells, which means that a hundred thousand cancer cells will survive even if the efficiency of reprogramming from cancer cells to the normal cell is 99.9%; it is therefore concluded that no method of cancer treatment can be described as being effective unless the efficiency of the above-described reprogramming is 100%.

The cancer cell lines used in conventional cancer research are those which are first established by culture for cell immortalization through forced expression of the E6, E7 and TERT genes of exogenous SV40 and HPV or by immortalization or canceration through transduction of oncogenes such as c-MYC gene and RAS gene into the cellular genome and are further cultured in common conventional media.

However, even in the absence of such gene transduction, the cancer cell lines established in common conventional media significantly generate in vitro artifact aberrations during extended culture, including chromosomal aberrations (e.g. dislocation and deletion), genomic aberrations (genetic mutations), and epigenetic aberrations which might lead to abnormal gene expression (Non-Patent Document 10). This gives rise to a problem that it is difficult to retain the aberrations such as mutations that occurred in cancer cells which were inherent causes of carcinogenesis or malignant transformation in vivo as such within the cells while minimizing the in vitro artifact aberrations. Strictly, these cell lines are not the cells themselves established and maintained by culture that permits self-renewal in vitro.

In cancer therapy research and the research for cancer-related drug discovery, even if the genomic or epigenetic aberrations in the cancer cell lines established by extended culture in such conventional media are analyzed, it is extremely difficult or even impossible to determine whether those aberrations were inherent in mammalian cancer cells as an etiology of carcinogenesis or malignant transformation, or in vitro artifact aberrations that occurred during culture and, hence, it is difficult to unravel an appropriate etiology of carcinogenesis or malignant transformation on the basis of the results of those analyses. It has been inappropriate to use such cells to search for a target in the discovery of a cancer therapeutic drug, screen for a candidate for cancer therapeutic drug, and the like.

A further problem is that despite the fact that cancer stem cells are highlighted as an important target in drug discovery, the cancer cells that are contained in a fresh cancer tissue make up a hierarchical and heterogeneous cell population and it is not easy to identify which cancer cells are cancer stem cells. Recently, there was reported a study for identifying cancer stem cells from a cancer cell line or primary cultured cancer cells (Non-Patent Document 11) but there is no report of successful in vitro proliferation and extended culture of monoclonal cancer cells, nor has been reported any technology by which they can be proliferated and subjected to in vitro expansion culture until their number reaches the necessary level for application in drug discovery and for use in cancer research.

As noted hereinbefore, the cells contained in a cancer tissue to be examined as a clinical specimen form a heterogeneous cell population which is a mixture of a variety of normal cells, non-cancer cells, and cancer cells. Similarly, the cancer cells contained in a cancer tissue are hierarchical and do not form a clonal cell population (Non-Patent Document 12). Multi-level omics analysis that can provide a huge volume of analytical data, as typified by a next-generation sequencer, is one of the techniques that are recently considered to be most attractive in the art. However, when a heterogeneous cancer tissue which is hierarchical and is not clonal is analyzed, data for the average genome of the cancer cell populations involved or the genome of the most abundant cancer cell population will be presented as a result but the problem is that it cannot be positively determined whether the result originates from the cancer cells in the cancer tissue that are an etiology of malignant transformations (development and metastasis).

In recent years, it has become possible to perform genomic analysis on a single cell and even cancer cells that are found in only small numbers can now be profiled (Non-Patent Document 13). If a plurality of single cells can be analyzed from the same cancer tissue, even a subpopulation comprising minor proportions of clones that indicate the development, metastasis and drug resistance of cancer can be monitored and detected (Non-Patent Document 14). In other words, among the somatic mutations accumulated by cancer cells, driver mutations (somatic mutations that are critical to carcinogenesis and malignant transformation) which are not passenger mutations (secondary mutations) can be explored effectively.

However, as of today, no cells have been established that correspond to the results of analyses and which are amenable to expansion culture and it has been impossible to perform functional analysis, XENOGRAFT modeling, and target/compound screening in drug discovery using the available cell lines.

CITATION LIST

PATENT LITERATURES

Patent Document 1: Japanese Patent Public Disclosure No. 2008-283972 A (JP2008283972A)
Patent Document 2: Japanese Patent Public Disclosure No. 2008-307007 A (JP2008307007A)

NON-PATENT LITERATURES

Non-Patent Document 1: Hochedlinger K, Jaenisch R et al., Genes Dev., 2004, 18:1875-1885
Non-Patent Document 2: Nakagawa M, Yamanaka S et al." Nat Biotechnol., 2008:26,101-106
Non-Patent Document 3: Takahashi K, Yamanaka S et al., Cell, 2007, 131:861-872
Non-Patent Document 4: Masaki H, Ishikawa T et al., Stem Cell Res., 2008, 1:105-115
Non-Patent Document 5: International Society for Stem Cell Research, 2009, Abstract Number 1739 (page 285)
Non-Patent Document 6: Utikal J et al., J Cell Sci., 2009, 122(Pt 19):3502-3510
Non-Patent Document 7: Carette JE et al., Blood, 2010, 115:4039-4042
Non-Patent Document 8: Nagai K et al., Biochem Biophys Res Commun., 2010, 395:258-263
Non-Patent Document 9: Miyoshi et al., Proc Natl Acad Sci USA. 2010, 107:40-5
Non-Patent Document 10: Gisselsson D., et al., Exp Cell Res 2010, 316: 3379-3386
Non-Patent Document 11: Visvader JE, Lindeman GJ, Nat Rev Cancer., 2008, 8:755-768
Non-Patent Document 12: Stephens P.J., et al., Cell 2011, 144: 27-40
Non-Patent Document 13: Navin N. and Hicks J., Genome Med 2011, 3:31
Non-Patent Document 14: Navin N., Nature 2011, 472:90-94

SUMMARY OF INVENTION

TECHNICAL PROBLEM

An object of the present invention is to develop a technique by which cells can be subjected to expansion culture irrespective of which marker is used and without involving transplanting into an immunodeficient mouse. Another object of the present invention is to establish clonal induced malignant stem cells as derived from a human cancer tissue or non-cancer tissue (clinical specimen) that have medical information.

Once a plurality of clonal cancer cell populations are obtained from the same cancer tissue, they can be subjected to a great variety of analyses by a next-generation sequencer that have been impossible to perform on single cells on account of their quantitative limits, such as multi-level omics analyses including genomic analysis (for target sequences such as a whole genome, exosome, and quinome), genome-wide DNA methylation analysis, comprehensive expression analysis (mRNA and miRNA), comprehensive protein expression analysis, comprehensive sugar-chain analysis, and metabolome analysis, as well as copy number variation (CNV) by array-based comparative genomic hybridization (array CGH), and microsatellite instability test.

It is also expected that, analysis of a plurality of clonal cancer cell populations will be clue to elucidate etiology, as well as the mechanism of development and the driver mutations of tumors composed of the originating hierarchical and multi-clonal heterogeneous cells. Such a plurality of single-cell derived, monoclonal cancer cell populations obtained from the same cancer tissue can be subjected to further analyses of cell functions, xenograft modeling, and target/compound screening in drug discovery. If, in the future, such clonal induced malignant stem cells are established from donor tissues of different races, sexes, ages and cancer species are collected as database to make a bank, they may be integrated with the clinical records, pathological information, and epidemiological data about the donors to enable the construction of a biobank of induced malignant stem cells that has medical information as well as information on multi-level omics analyses, and the bank is expected to be used in the development of innovative cancer therapeutic drugs.

A first object, therefore, of the present invention is to provide an induced malignant stem cell capable of in vitro proliferation that has a genomic or epigenetic aberration related to cancer and which can be used in various applications including screening for a target in the discovery of a cancer therapeutic drug, a candidate for cancer therapeutic drug, a cancer diagnostic drug, etc. as well as preparing cancer vaccines, and cancer model animals.

A second object of the present invention is to provide a process for producing an induced malignant stem cell capable of in vitro proliferation that has a genomic or epigenetic aberration related to cancer.

A third object of the present invention is to provide a method of screening, such as screening for a target in the discovery of a cancer therapeutic drug, screening for a candidate for cancer therapeutic drug, screening for a cancer diagnostic drug, etc., which uses the above-mentioned induced malignant stem cell capable of in vitro proliferation.

A fourth object of the present invention is to provide a process for producing a cancer vaccine, which uses the above-mentioned induced malignant stem cell capable of in vitro proliferation.

A fifth object of the present invention is to provide a process for producing a cancer model animal, which comprises transplanting the above-mentioned induced malignant stem cell capable of in vitro proliferation into a laboratory animal.

SOLUTION TO PROBLEM

To be more specific, the present invention provides in its first aspect an induced malignant stem cell capable of in vitro proliferation that is characterized by satisfying the following two requirements:

(1) having at least one aberration selected from among (a) an aberration of methylation (high or low degree of methylation) in a tumor suppressor gene or a cancer-related genetic region in endogenous genomic DNA, (b) a somatic mutation of a tumor suppressor gene or a somatic mutation of an endogenous cancer-related gene in endogenous genomic DNA, (c) abnormal expression (increased or reduced/lost expression) of an endogenous oncogene or an endogenous tumor suppressor gene, (d) abnormal expression (increased or reduced/lost expression) of a noncoding RNA such as an endogenous cancer-related microRNA, (e) abnormal expression of an endogenous cancer-related protein, (f) an aberration of endogenous cancer-related metabolism (hypermetabolism or hypometabolism), (g) an aberration of endogenous cancer-related sugar chain, (h) an aberration of copy number variations in endogenous genomic DNA , and (i) instability of microsatellites in endogenous genomic DNA in an induced malignant stem cell; and
(2) expressing genes including POU5F1 gene, NANOG gene, SOX2 gene, and ZFP42 gene.

In its second aspect, the present invention provides a process for producing an induced malignant stem cell capable of in vitro proliferation from a non-embryonic starter somatic cell taken from a cancer tissue in a mammal having a genomic or epigenetic aberration related to cancer. This process is characterized in that either one to six genes selected from among POU5F1 gene, SOX2 gene, c-Myc gene, KLF4 gene, LIN28 gene, and NANOG gene, or one to six RNAs selected from among POU5F1 RNA, SOX2 RNA, c-Myc RNA, KLF4 RNA, LIN28 RNA, and NANOG RNA, or one to six proteins selected from among POU5F1 protein, SOX2 protein, c-Myc protein, KLF4 protein, LIN28 protein, and NANOG protein are transferred into a starter somatic cell prepared from a fresh cancer tissue or a non-cancer tissue taken from a carcinogenic mammal. When the cell is described as being "non-embryonic", it shall be construed as being neither an embryonic stem cell nor an embryo nor a germ cell nor a primordial germ cell.

In the method described above, the fresh cancer tissue is one of a solid cancer or one of a carcinoma, and the starter somatic cell is characterized by being prepared from a fresh cancer tissue selected from stomach cancer, colon cancer, breast cancer, kidney cancer, lung cancer, and liver cancer.

In its third aspect, the present invention provides a screening method selected from a method of screening for a target in the discovery of a cancer therapeutic drug, a method of screening for a cancer therapeutic drug (candidate), and a method of screening for a cancer diagnostic drug (candidate), which is characterized by using the induced malignant stem cell of the present invention.

In its fourth aspect, the present invention provides a process for preparing a cancer vaccine, which is characterized by using the induced malignant stem cell of the invention. In its fifth aspect, the present invention provides a process for preparing a cancer model animal, which is characterized by transplanting the induced malignant stem cell of the invention into a laboratory animal.

ADVANTAGEOUS EFFECTS OF INVENTION

According to the present invention, there is provided an induced malignant stem cell capable of in vitro proliferation that is characterized by (1) having a genomic or epigenetic aberration related to cancer such as (a) an aberration of methylation (high or low degree of methylation) in a tumor suppressor gene or a cancer-related genetic region in endogenous genomic DNA, (b) a somatic mutation of a tumor suppressor gene or a somatic mutation of an endogenous cancer-related gene in endogenous genomic DNA, (c) abnormal expression (increased or reduced/lost expression) of an endogenous oncogene or an endogenous tumor suppressor gene, (d) abnormal expression (increased or reduced/lost expression) of a noncoding RNA such as an endogenous cancer-related microRNA, (e) abnormal expression of an endogenous cancer-related protein, (f) an aberration of endogenous cancer-related metabolism (hypermetabolism or hypometabolism), (g) an aberration of endogenous cancer-related sugar chain, (h) an aberration of copy number variations in endogenous genomic DNA , or (i) instability of microsatellites in endogenous genomic DNA in an induced malignant stem cell, and (2) expressing a self-renewal related gene such as POU5F1 gene (also referred to as OCT3/4 gene), NANOG gene, SOX2 gene, or ZFP42 gene, as well as processes for production thereof, and applications of these cells.

BRIEF DESCRIPTION OF DRAWINGS

Fig. 1 is a set of diagrams showing the occurrence of instability of endogenous genomic DNA microsatellites in induced malignant stem cells.

Fig. 2 is a graph showing the results of primary components analysis of metabolites from induced malignant stem cells.

Fig. 3 is a set of charts showing the results of analyses of cancer-related sugar chains in induced malignant stem cells.

Fig. 4 is a set of diagrams showing that induced malignant stem cells express ES cell specific genes at comparable levels to induced pluripotent stem cells.

The induced malignant stem cells of the present invention not only maintain the aberrations inherent in the starter somatic cell such as (1)(a) to (1)(i) but they also have a distinct feature of stem cells, i.e., being capable of proliferation. Hence, the induced malignant stem cells of the present invention can be subjected to passage culture for an extended period so that they are easily induced to cancer cells having the nature of differentiated cells; thus, they are extremely useful in medical research, such as integrative omics analyses (such as analyses of epigenome, genome, transcriptome, proteome, glycome, and metabolome), analyses in molecular cell biology, in screening method, such as screenings in drug discovery (such as compound screening, target screening (siRNA, antisense DNA/RNA, or cDNA screening)), in methods of screening such as a method of screening for cancer diagnostic drugs, in methods of preparing cancer vaccines and cancer model animals as well as cancer therapy research and the research for cancer-related drug discovery.

DESCRIPTION OF EMBODIMENTS

A currently established concept in the art is that just like somatic cells which are reprogrammed to induced pluripotent stem cells, cancer cells can be reverted to normal cells through reprogramming

The present inventor challenged this concept, considering as follows: since a fresh cancer tissue and a primary cultured cancer cell population are generally both heterogeneous, and so cells obtained from the cancer tissue or primary cultured cancer cell population are likely to include normal cells or non-cancer cells having genomes or epigenetics either identical or approximate to the normal cells; based on this observation, the present inventor provided a hypothesis that cancer cells would not be reprogrammed to normal cells but that the normal cells contained in the fresh cancer tissue and the primary cultured cancer cell population would be induced to normal induced pluripotent stem cells whereas from the cancer cells that are present in the fresh cancer tissue and the primary cultured cancer cell population and which have genomic or epigenetic aberrations related to cancer and other aberrations, there would be induced malignant stem cells having the genomic or epigenetic aberrations related to cancer and other aberrations.

If this hypothesis is correct, it is expected that induced malignant stem cells capable of in vitro proliferation can be prepared by making use of techniques for making induced pluripotent stem cells where POUF5F1 gene, SOX2 gene, KLF4 gene, and c-MYC gene are transduced or POU5F1 gene, SOX2 gene, and KLF4 gene are transduced, and furthermore, by proliferating the resulting induced malignant stem cells in vitro, the induced malignant stem cells capable of in vitro prolieration that maintain the genomic or epigenetic characteristics of malignancy of cancer used as the starter somatic cell can be caused to proliferate without limit under culture conditions.

On the basis of this hypothesis, the present inventor made an intensive study and found that by using a starter somatic cell having a genomic or epigenetic aberration related to cancer and then by causing at least one self-renewal related gene selected from among POU5F1 gene, KLF4 gene, SOX2 gene, c-MYC gene, LIN28 gene, NANOG gene, etc. or a protein as the translation product of any of such genes to be present in said starter somatic cell, there could be obtained an induced malignant stem cell capable of in vitro proliferation.

Thus, the present inventor discovered the induced malignant stem cells of the present invention which are characterized in that the malignancy of cancer as the starter somatic cell, namely, the genomic or epigenetic aberration related to cancer that is inherent in the starter somatic cell is maintained in vivo and that they are also capable of proliferation and amenable to extended passage culture; the present inventors also found that these cells could be applied to drug discovery in vitro or used in cancer research. The present invention has been accomplished on the basis of these findings.

On the pages that follow, the induced malignant stem cells of the present invention, the process for producing them, the cancer cells derived from these cells, and the applications of these cells are described in detail.

Induced malignant stem cells

The induced malignant stem cell that is provided in the first aspect of the present invention is an induced malignant stem cell capable of in vitro proliferation which is characterized by satisfying the following two requirements:

(1) having at least one aberration selected from among (a) an aberration of methylation (high or low degree of methylation) in a tumor suppressor gene or a cancer-related genetic region in endogenous genomic DNA, (b) a somatic mutation of a tumor suppressor gene or a somatic mutation of an endogenous cancer-related gene in endogenous genomic DNA, (c) abnormal expression (increased or reduced/lost expression) of an endogenous oncogene or an endogenous tumor suppressor gene, (d) abnormal expression (increased or reduced/lost expression) of a noncoding RNA such as an endogenous cancer-related microRNA, (e) abnormal expression of an endogenous cancer-related protein, (f) an aberration of endogenous cancer-related metabolism (hypermetabolism or hypometabolism), (g) an aberration of endogenous cancer-related sugar chain, (h) an aberration of copy number variations in endogenous genomic DNA , and (i) instability of microsatellites in endogenous genomic DNA in an induced malignant stem cell; and
(2) expressing genes including POU5F1 gene, NANOG gene, SOX2 gene, and ZFP42 gene.

The "induced malignant stem cells" as referred to in the present invention means cancer cells that have a function as stem cells (which is substantially at least proliferating ability or self-renewal ability). The term "stem cells" as generally used in the technical field contemplated by the present invention refers to cells having both the ability to differentiate into a specific cell (i.e., differentiating ability) and the ability to maintain the same property as the original cell (differentiating ability) even after cell divisions (i.e., self-renewal ability). The term "self-renewal ability" specifically refers to the ability to create the same cell after division, and in the case of the induced malignant stem cell of the present invention, it means that the cell can be subjected to expansion culture or passage culture for at least 3 days.

Genomic or epigenetic aberration related to cancer in the induced malignant stem cell

The induced malignant stem cell of the present invention is characterized by having a genomic or epigenetic aberration related to cancer. Specifically, the induced malignant stem cell of the present invention is characterized by having at least one aberration selected from among (a) an aberration of methylation (high or low degree of methylation) in a tumor suppressor gene or a cancer-related genetic region in endogenous genomic DNA, (b) a somatic mutation of a tumor suppressor gene or a somatic mutation of an endogenous cancer-related gene in endogenous genomic DNA, (c) abnormal expression (increased or reduced/lost expression) of an endogenous oncogene or an endogenous tumor suppressor gene, (d) abnormal expression (increased or reduced/lost expression) of a noncoding RNA such as an endogenous cancer-related microRNA, (e) abnormal expression of an endogenous cancer-related protein, (f) an aberration of endogenous cancer-related metabolism (hypermetabolism or hypometabolism), (g) an aberration of endogenous cancer-related sugar chain, (h) an aberration of copy number variations in endogenous genomic DNA , and (i) instability of microsatellites in endogenous genomic DNA in an induced malignant stem cell. In addition, the induced malignant stem cell of the present invention which is capable of in vitro proliferation may have a metabolomic aberration compared to induced pluripotent stem cells (such as showing an enhancement in the glycolysis system as compared with induced pluripotent stem cells) or a karyotypic or chromosomal aberration compared to induced pluripotent stem cells. These aberrations are identical to the aberrations inherent in the starter somatic cell from which the induced malignant stem cell of the present invention originate; in other words, the aberrations inherent in the starter somatic cell have been passed on to the induced malignant stem cell of the present invention.

In the present invention, the induced malignant stem cell capable of in vitro proliferation may have (1) (a) an aberration of methylation in a tumor suppressor gene or a cancer-related genetic region in endogenous genomic DNA. Examples of the tumor suppressor gene or cancer-related genetic region in endogenous genomic DNA that might cause such an aberration of methylation and exemplary sites where such methylation is likely to occur include an aberration of methylation at the 5 position of cytosine base (C) in CpGs located between the genome start point and the genome terminal point of the genomic DNAs (GeneSymbol_NO.) listed in the following table:

[Table 1]

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 1 | ABL2 | 177465262 | 177465849 | 587 | 68 |
| 1 | AF1Q | 149298304 | 149298628 | 324 | 19 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 1 | ALU_cons | 159390719 | 159391402 | 683 | 26 |
| 1 | ALU_M1 | 151803096 | 151804508 | 1412 | 23 |
| 1 | ARNT | 149115560 | 149115763 | 203 | 15 |
| 1 | BCL9 | 145181137 | 145181448 | 311 | 31 |
| 1 | CD34_01 | 206150852 | 206151248 | 396 | 33 |
| 1 | CR2_01 | 205694281 | 205694660 | 379 | 34 |
| 1 | EPS15_01 | 51757105 | 51757518 | 413 | 47 |
| 1 | FH | 239748987 | 239749478 | 491 | 47 |
| 1 | HRPT2_001 | 191357448 | 191357799 | 351 | 45 |
| 1 | MUC1_001 | 153429956 | 153430351 | 395 | 23 |
| 1 | MUTYH | 45578118 | 45578622 | 504 | 57 |
| 1 | MYCL1 | 40140552 | 40140860 | 308 | 25 |
| 1 | NTRK_02 | 155095323 | 155095679 | 356 | 24 |
| 1 | NTRK1_001 | 155097132 | 155097659 | 527 | 46 |
| 1 | PAX7_001 | 18829422 | 18829659 | 237 | 15 |
| 1 | PAX7_002 | 18830507 | 18830936 | 429 | 38 |
| 1 | PBX1_001 | 162812066 | 162812615 | 549 | 51 |
| 1 | PDE4DIP | 143751199 | 143751586 | 387 | 32 |
| 1 | PLOD_01 | 11917237 | 11917716 | 479 | 40 |
| 1 | PMX1 | 168900324 | 168900826 | 502 | 36 |
| 1 | PRCC_01 | 155004541 | 155004781 | 240 | 13 |
| 1 | PRDM16_001 | 2975298 | 2975662 | 364 | 29 |
| 1 | RBM15_001 | 110682735 | 110683276 | 541 | 35 |
| 1 | rhoC_01 | 113051149 | 113051563 | 414 | 41 |
| 1 | RUNX3_01_01 | 25130851 | 25131313 | 462 | 36 |
| 1 | RUNX3_02_01 | 25129323 | 25129742 | 419 | 37 |
| 1 | SATalpha | 121151051 | 121151958 | 907 | 22 |
| 1 | SDHB | 17252913 | 17253355 | 442 | 33 |
| 1 | SDHC_01 | 159550943 | 159551171 | 228 | 15 |
| 1 | SDHC_02 | 159550688 | 159550946 | 258 | 16 |
| 1 | SFPQ_001 | 35430695 | 35431047 | 352 | 35 |
| 1 | SIL_001 | 47552254 | 47552599 | 345 | 33 |
| 1 | STL_01 | 112963027 | 112963505 | 478 | 30 |
| 1 | STL_02 | 112963759 | 112964145 | 386 | 33 |
| 1 | TAF15 | 28842061 | 28842518 | 457 | 39 |

Table 1: Tumor suppressor genes or cancer-related genetic regions that might cause an aberration of methylation (condition (1) (a))

| Chromosome | GeneSymbol No. | genome start point | genome terminal point | length of Genome | No. of CpG |
|---|---|---|---|---|---|
| 1 | TAL1 | 47463678 | 47464167 | 489 | 66 |
| 1 | THRAP3_001 | 36462320 | 36462698 | 378 | 39 |
| 1 | TPM3_001 | 152422158 | 152422410 | 252 | 19 |
| 1 | TPR_001 | 184610787 | 184611141 | 354 | 35 |
| 1 | TRIM33_001 | 114855575 | 114855910 | 335 | 24 |
| 2 | ALK | 29997217 | 29997654 | 437 | 29 |
| 2 | ALU_M5 | 201833637 | 201834637 | 1000 | 24 |
| 2 | ATIC | 215884932 | 215885516 | 584 | 51 |
| 2 | BCL11A_001 | 60634369 | 60634750 | 381 | 32 |
| 2 | CCT4_01 | 61968924 | 61969309 | 385 | 38 |
| 2 | CMKOR1_001 | 237141716 | 237142026 | 310 | 21 |
| 2 | COX7A2L_01 | 42441636 | 42441989 | 353 | 27 |
| 2 | DBI_1_01 | 119840575 | 119840964 | 389 | 31 |
| 2 | ERCC3 | 127767790 | 127768382 | 592 | 51 |
| 2 | FEV | 219557998 | 219558487 | 489 | 55 |
| 2 | HOXD11_001 | 176679790 | 176680265 | 475 | 47 |
| 2 | HOXD13_001 | 176665504 | 176665745 | 241 | 22 |
| 2 | MSH2 | 47483700 | 47484020 | 320 | 34 |
| 2 | MSH6_01 | 47863130 | 47863577 | 447 | 37 |
| 2 | MYCN_01 | 15999936 | 16000536 | 600 | 74 |
| 2 | MYCN_02 | 15998311 | 15998682 | 371 | 23 |
| 2 | NEDD5_1_01 | 241903199 | 241903499 | 300 | 38 |
| 2 | NEDD5_2 01 | 241903907 | 241904446 | 539 | 65 |
| 2 | PAX3_001 | 222871383 | 222871886 | 503 | 38 |
| 2 | PAX8_01 | 113751340 | 113751782 | 442 | 39 |
| 2 | PAX8_02 | 113751758 | 113752141 | 383 | 31 |
| 2 | PAX8_03 | 113751013 | 113751358 | 345 | 21 |
| 2 | PMS1_01 | 190356952 | 190357548 | 596 | 66 |
| 2 | PMS1_02 | 190357523 | 190357814 | 291 | 25 |
| 2 | REL_001 | 60961862 | 60962359 | 497 | 56 |
| 3 | AF3p21 | 48697853 | 48698277 | 424 | 41 |
| 3 | APOD_01 | 196827214 | 196827678 | 464 | 36 |
| 3 | BCL5_001 | 188937964 | 188938293 | 329 | 19 |
| 3 | BCL6 | 188944627 | 188944968 | 341 | 18 |
| 3 | CTNNB1 | 41215651 | 41216173 | 522 | 58 |

(continued)

| | Table 1: Tumor suppressor genes or cancer-related genetic regions that might cause an aberration of methylation (condition (1) (a)) | | | | |
|---|---|---|---|---|---|
| Chromosome | GeneSymbol No. | genome start point | genome terminal point | length of Genome | No. of CpG |
| 3 | ECT2_2_01 | 173951176 | 173951692 | 516 | 33 |
| 3 | EIF4A2 | 187984488 | 187984899 | 411 | 37 |
| 3 | EVI1 | 170346825 | 170347202 | 377 | 31 |
| 3 | FANCD2 | 10042822 | 10043297 | 475 | 41 |
| 3 | GMPS | 157071533 | 157072104 | 571 | 60 |
| 3 | MDS1_001 | 170862884 | 170863415 | 531 | 54 |
| 3 | MLF1_001 | 159771428 | 159771821 | 393 | 32 |
| 3 | MLH1_001 | 37009285 | 37009728 | 443 | 39 |
| 3 | MRPL3_01 | 132704180 | 132704683 | 503 | 39 |
| 3 | PIK3CA_001 | 180349337 | 180349623 | 286 | 30 |
| 3 | PPARG | 12304698 | 12305108 | 410 | 53 |
| 3 | RAR_beta_01 | 25444793 | 25445114 | 321 | 15 |
| 3 | RASSF1 | 50352936 | 50353401 | 465 | 52 |
| 3 | RPN1_001 | 129851885 | 129852431 | 546 | 52 |
| 3 | TFG_001 | 101910877 | 101911384 | 507 | 56 |
| 3 | TFRC_001 | 197293205 | 197293682 | 477 | 47 |
| 3 | VHL_001 | 10158220 | 10158764 | 544 | 69 |
| 3 | ZNF9_001 | 130385378 | 130385695 | 317 | 41 |
| 4 | ARHH_01 | 39734501 | 39735101 | 600 | 81 |
| 4 | ARHH_02 | 39734502 | 39735102 | 600 | 81 |
| 4 | CCNA2_01 | 122964129 | 122964654 | 525 | 50 |
| 4 | CD38_01 | 15389339 | 15389561 | 222 | 22 |
| 4 | CHIC2_001 | 54625371 | 54625921 | 550 | 65 |
| 4 | FBXW7_001 | 153675457 | 153675857 | 400 | 27 |
| 4 | FGFR3 001 | 1765563 | 1766100 | 537 | 52 |
| 4 | FIP1L1 | 53938235 | 53938640 | 405 | 30 |
| 4 | KIT_001 | 55218601 | 55219070 | 469 | 54 |
| 4 | MLLT2 | 88147105 | 88147579 | 474 | 54 |
| 4 | NMU_01 | 56196612 | 56197197 | 585 | 49 |
| 4 | PDGFRA | 54789115 | 54789455 | 340 | 23 |
| 4 | PHOX2B_001 | 41444001 | 41444391 | 390 | 30 |
| 4 | RAP1GDS1_001 | 99401375 | 99401820 | 445 | 56 |
| 4 | TEC | 47966387 | 47966780 | 393 | 42 |
| 5 | AF5q31 | 132327102 | 132327594 | 492 | 49 |
| 5 | APC | 112224722 | 112225026 | 304 | 26 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 5 | ATP6V0E_01 | 172343262 | 172343800 | 538 | 45 |
| 5 | CCNB1_01 | 68498405 | 68499005 | 600 | 44 |
| 5 | CCNH_1_01 | 86743924 | 86744401 | 477 | 28 |
| 5 | CCNH_2_01 | 86744377 | 86744807 | 430 | 35 |
| 5 | F2R_001 | 76047137 | 76047535 | 398 | 28 |
| 5 | FACL6 | 131374976 | 131375381 | 405 | 46 |
| 5 | FLT4 | 180009095 | 180009474 | 379 | 53 |
| 5 | GNB2L1_01 | 180602827 | 180603383 | 556 | 37 |
| 5 | GRAF | 142130593 | 142130977 | 384 | 37 |
| 5 | hB23_1_01 | 170747765 | 170748284 | 519 | 43 |
| 5 | hB23_2_01 | 170747765 | 170748284 | 519 | 43 |
| 5 | HDAC3_01 | 140996361 | 140996781 | 420 | 36 |
| 5 | KCNMB1_01 | 169748654 | 169748935 | 281 | 8 |
| 5 | NPM1 | 170747765 | 170748284 | 519 | 43 |
| 5 | NSD1 | 176492070 | 176492590 | 520 | 51 |
| 5 | NSD1 | 176492070 | 176492590 | 520 | 51 |
| 5 | OXCT_01 | 41906021 | 41906603 | 582 | 50 |
| 5 | RANBP17_001 | 170221621 | 170222169 | 548 | 61 |
| 5 | TLX3_001 | 170669340 | 170669753 | 413 | 38 |
| 5 | U2AF1RS1_001 | 112255229 | 112255506 | 277 | 6 |
| 6 | C2_1_01 | 31977367 | 31977872 | 505 | 57 |
| 6 | CCNC_01 | 100122997 | 100123403 | 406 | 34 |
| 6 | CCND3_001 | 42016614 | 42017089 | 475 | 34 |
| 6 | DEK_001 | 18372723 | 18373251 | 528 | 65 |
| 6 | ERalpha_02 | 152170751 | 152171138 | 387 | 34 |
| 6 | ESR1_01_01 | 152170469 | 152170794 | 325 | 27 |
| 6 | FANCE | 35527814 | 35528258 | 444 | 42 |
| 6 | FGFR1OP_01 | 167331449 | 167331820 | 371 | 39 |
| 6 | FGFR1OP_02 | 167331449 | 167331821 | 372 | 39 |
| 6 | FOXO3A_01 | 108988490 | 108988869 | 379 | 48 |
| 6 | FOXO3A_02 | 108988061 | 108988515 | 454 | 38 |
| 6 | GOPC_001 | 118030207 | 118030715 | 508 | 40 |
| 6 | HIST1H4I | 27215048 | 27215399 | 351 | 32 |
| 6 | HMGA1_001 | 34312298 | 34312861 | 563 | 51 |
| 6 | HSPCB_001 | 44322980 | 44323329 | 349 | 25 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 6 | IGF2R_001 | 160310331 | 160310780 | 449 | 59 |
| 6 | IGF2R_002 | 160346693 | 160347065 | 372 | 29 |
| 6 | IGF2R_003 | 160431853 | 160432481 | 628 | 45 |
| 6 | IRF4_001 | 336391 | 336863 | 472 | 48 |
| 6 | MLLT4 | 167971238 | 167971475 | 237 | 18 |
| 6 | Notch4_01 | 32271333 | 32271746 | 413 | 41 |
| 6 | PIM1_01 | 37246325 | 37246801 | 476 | 47 |
| 6 | PIM1_02 | 37246775 | 37247064 | 289 | 18 |
| 6 | PLAGL1_001 | 144371140 | 144371644 | 504 | 47 |
| 6 | PRDM1_01 | 106640781 | 106641136 | 355 | 32 |
| 6 | SFRS3_001 | 36669855 | 36670055 | 200 | 22 |
| 6 | SLC22A1_001 | 160474825 | 160475241 | 416 | 23 |
| 6 | SLC22A2_001 | 160599289 | 160599657 | 368 | 22 |
| 6 | SLC22A3_001 | 160688805 | 160689077 | 272 | 20 |
| 6 | SLC22A3_002 | 160703745 | 160704226 | 481 | 33 |
| 6 | TFEB_001 | 41810490 | 41811016 | 526 | 54 |
| 7 | ASB4_001 | 94995075 | 94995493 | 418 | 22 |
| 7 | BRAF | 140270275 | 140270618 | 343 | 16 |
| 7 | CAS1_001 | 93977337 | 93977656 | 319 | 34 |
| 7 | CBL | 107171268 | 107171726 | 458 | 34 |
| 7 | CDK6_001 | 92300956 | 92301485 | 529 | 42 |
| 7 | COPG2_001 | 130004373 | 130004597 | 224 | 14 |
| 7 | DNCI1_001 | 95239821 | 95240171 | 350 | 40 |
| 7 | EGFR | 55053588 | 55053949 | 361 | 20 |
| 7 | ELN_01 | 73080258 | 73080525 | 267 | 20 |
| 7 | ETV1_001 | 13995856 | 13996164 | 308 | 19 |
| 7 | GRB10_001 | 50817597 | 50818104 | 507 | 64 |
| 7 | HIP1 | 75205858 | 75206444 | 586 | 55 |
| 7 | HLXB9_001 | 156496339 | 156496819 | 480 | 41 |
| 7 | HOXA1_AB01 | 27101762 | 27102043 | 281 | 18 |
| 7 | HOXA1_SQ05 | 27109677 | 27110061 | 384 | 24 |
| 7 | HOXA10_AB01 | 27180431 | 27180694 | 263 | 23 |
| 7 | HOXA10_SQ02 | 27180440 | 27180963 | 523 | 40 |
| 7 | HOXA11_AB01 | 27191976 | 27192283 | 307 | 17 |
| 7 | HOXA11_SQ01 | 27191540 | 27192000 | 460 | 28 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 7 | HOXA13_SQ01 | 27205189 | 27205499 | 310 | 20 |
| 7 | HOXA13_SQ03 | 27205751 | 27206281 | 530 | 72 |
| 7 | HOXA3_AB01 | 27116719 | 27117005 | 286 | 26 |
| 7 | HOXA3_SQ01 | 27116526 | 27117002 | 476 | 45 |
| 7 | HOXA4_AB01 | 27136693 | 27136896 | 203 | 17 |
| 7 | HOXA4_SQ02 | 27136272 | 27136715 | 443 | 55 |
| 7 | HOXA5_AB01 | 27149932 | 27150276 | 344 | 30 |
| 7 | HOXA5_SQ03 | 27149843 | 27150375 | 532 | 39 |
| 7 | HOXA6_AB01 | 27153596 | 27153836 | 240 | 18 |
| 7 | HOXA7_AB01 | 27162508 | 27162921 | 413 | 31 |
| 7 | HOXA7_SQ03 | 27162898 | 27163116 | 218 | 23 |
| 7 | HOXA9_AB01 | 27171578 | 27171938 | 360 | 26 |
| 7 | HOXA9_SQ03 | 27171098 | 27171594 | 496 | 48 |
| 7 | JAZF1_001 | 28186641 | 28187157 | 516 | 47 |
| 7 | MEST_001 | 129913454 | 129913912 | 458 | 35 |
| 7 | MESTIT1_001 | 129918328 | 129918858 | 530 | 34 |
| 7 | MET_001 | 116099294 | 116099611 | 317 | 40 |
| 7 | PDK4_001 | 95063383 | 95063843 | 460 | 39 |
| 7 | PEG10_001 | 94131513 | 94131935 | 422 | 23 |
| 7 | PIK3CG_01 | 106295442 | 106295890 | 448 | 36 |
| 7 | PMS2 | 6014874 | 6015442 | 568 | 43 |
| 7 | PON1_001 | 94791654 | 94792056 | 402 | 20 |
| 7 | PON2_001 | 94901962 | 94902368 | 406 | 41 |
| 7 | PON3_001 | 94863460 | 94863887 | 427 | 43 |
| 7 | PTPRN2_2_01 | 158073675 | 158074048 | 373 | 34 |
| 7 | SBDS_001 | 66097520 | 66098025 | 505 | 46 |
| 7 | SGCE_001 | 94123033 | 94123358 | 325 | 22 |
| 7 | SMO | 128616273 | 128616798 | 525 | 47 |
| 7 | TIF1_001 | 137795321 | 137795843 | 522 | 59 |
| 8 | AL080059_1_01 | 98359116 | 98359534 | 418 | 57 |
| 8 | AL080059_2_01 | 98358787 | 98359140 | 353 | 35 |
| 8 | CA3_01 | 86537987 | 86538472 | 485 | 33 |
| 8 | CBFA2T1_01 | 93184596 | 93185070 | 474 | 54 |
| 8 | CBFA2T1_02 | 93184184 | 93184618 | 434 | 55 |
| 8 | COX6C_001 | 100974721 | 100974933 | 212 | 19 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 8 | MYC | 128819501 | 128820026 | 525 | 40 |
| 8 | NBS1_001 | 91065688 | 91066174 | 486 | 54 |
| 8 | NCOA2_01 | 71478600 | 71479056 | 456 | 28 |
| 8 | NCOA2_02 | 71479039 | 71479412 | 373 | 26 |
| 8 | PCM1 | 17824948 | 17825351 | 403 | 33 |
| 8 | PLAG1_001 | 57286077 | 57286414 | 337 | 26 |
| 8 | RECQL4 | 145713246 | 145713583 | 337 | 23 |
| 8 | TCEA1_001 | 55097189 | 55097737 | 548 | 59 |
| 8 | WHSC1L1_001 | 38359472 | 38360010 | 538 | 54 |
| 9 | ABL1 | 132577525 | 132577958 | 433 | 34 |
| 9 | CDKN2A_01_02 | 21964963 | 21965374 | 411 | 26 |
| 9 | CDKN2A_02_01 | 21984999 | 21985288 | 289 | 27 |
| 9 | CDKN2A_p14ARF | 21985592 | 21986033 | 441 | 39 |
| 9 | CKS2_2_01 | 91115463 | 91115793 | 330 | 28 |
| 9 | CKS2_3_01 | 91115773 | 91116340 | 567 | 62 |
| 9 | COL5A1_01 | 136673725 | 136674245 | 520 | 68 |
| 9 | FANCC_01 | 97119241 | 97119819 | 578 | 61 |
| 9 | FANCC_02 | 97119240 | 97119819 | 579 | 61 |
| 9 | FANCG_001 | 35069478 | 35070016 | 538 | 41 |
| 9 | FNBP1_001 | 131845061 | 131845514 | 453 | 61 |
| 9 | JAK2_001 | 4974748 | 4975284 | 536 | 56 |
| 9 | MLLT3 | 20610652 | 20611134 | 482 | 37 |
| 9 | NOTCH1_001 | 138560542 | 138560790 | 248 | 23 |
| 9 | NR4A3 | 101624591 | 101625034 | 443 | 26 |
| 9 | NUP214 | 132990566 | 132991025 | 459 | 42 |
| 9 | p16_01 | 21964963 | 21965171 | 208 | 13 |
| 9 | PAX5_001 | 37024038 | 37024514 | 476 | 36 |
| 9 | PAX6_01 | 37027794 | 37028366 | 572 | 45 |
| 9 | PAX6_02 | 37026880 | 37027346 | 466 | 27 |
| 9 | PAX6_03 | 37024512 | 37024773 | 261 | 21 |
| 9 | PSIP2 | 15500124 | 15500613 | 489 | 52 |
| 9 | PSIP2_001 | 15500616 | 15501143 | 527 | 64 |
| 9 | PTCH_01 | 97308581 | 97308982 | 401 | 39 |
| 9 | PTCH_02 | 97308959 | 97309439 | 480 | 24 |
| 9 | PTCH_03 | 97309851 | 97310140 | 289 | 12 |

(continued)

Table 1: Tumor suppressor genes or cancer-related genetic regions that might cause an aberration of methylation (condition (1) (a))

| Chromosome | GeneSymbol No. | genome start point | genome terminal point | length of Genome | No. of CpG |
|---|---|---|---|---|---|
| 9 | SET_001 | 130490719 | 130490890 | 171 | 8 |
| 9 | SYK_001 | 92603461 | 92603893 | 432 | 37 |
| 9 | TAL2 | 107458199 | 107458779 | 580 | 67 |
| 9 | TSC1_001 | 134809948 | 134810385 | 437 | 39 |
| 10 | BMPR1A_02 | 88506944 | 88507236 | 292 | 22 |
| 10 | COPEB_001 | 3816825 | 3817186 | 361 | 39 |
| 10 | D10S170_01 | 61335497 | 61335783 | 286 | 14 |
| 10 | D10S171_02 | 61336400 | 61336698 | 298 | 27 |
| 10 | FGFR2 | 123347301 | 123347592 | 291 | 33 |
| 10 | FRAT1_001 | 99070069 | 99070493 | 424 | 23 |
| 10 | GDI2_01 | 5895432 | 5896023 | 591 | 66 |
| 10 | MGMT_01_03 | 131155099 | 131155394 | 295 | 38 |
| 10 | MKI67_01 | 129813761 | 129814000 | 239 | 14 |
| 10 | MLLT_10 | 21862747 | 21863293 | 546 | 60 |
| 10 | mpp5_01 | 57790897 | 57791267 | 370 | 30 |
| 10 | MYST4_01 | 76256270 | 76256743 | 473 | 43 |
| 10 | MYST4_02 | 76255917 | 76256358 | 441 | 23 |
| 10 | NCOA4_001 | 51242282 | 51242680 | 398 | 35 |
| 10 | NFKB2_001 | 104143617 | 104144117 | 500 | 40 |
| 10 | NFKB2_002 | 104144801 | 104145274 | 473 | 23 |
| 10 | NFKB2_003 | 104145185 | 104145668 | 483 | 30 |
| 10 | NFKB2_004 | 104144093 | 104144445 | 352 | 29 |
| 10 | NFKB2_005 | 104144383 | 104144828 | 445 | 33 |
| 10 | PTEN_02 | 89613072 | 89613626 | 554 | 65 |
| 10 | RAI17_001 | 80591728 | 80592105 | 377 | 12 |
| 10 | RET_001 | 42891820 | 42892158 | 338 | 20 |
| 10 | SSH3BP1 | 27189110 | 27189610 | 500 | 40 |
| 10 | SUFU | 104253634 | 104254215 | 581 | 58 |
| 10 | TLX1_001 | 102881084 | 102881395 | 311 | 22 |
| 11 | ARHGEF12 | 119712481 | 119712891 | 410 | 49 |
| 11 | ASCL2_001 | 2247867 | 2248329 | 462 | 59 |
| 11 | ATM_001 | 107598808 | 107599243 | 435 | 38 |
| 11 | BC050616_001 | 2377913 | 2378292 | 379 | 33 |
| 11 | CARS_001 | 3035200 | 3035521 | 321 | 31 |
| 11 | CARS_001 | 3034784 | 3035181 | 397 | 31 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 11 | CCND1_01 | 69160261 | 69160818 | 557 | 54 |
| 11 | CCND1_02 | 69160263 | 69160817 | 554 | 54 |
| 11 | CCND1_1_01 | 69160261 | 69160817 | 556 | 54 |
| 11 | CCND1_2_01 | 69162041 | 69162617 | 576 | 56 |
| 11 | CCND1_3_01 | 69164429 | 69164933 | 504 | 34 |
| 11 | CD44_01 | 35117193 | 35117609 | 416 | 34 |
| 11 | CD59_01 | 33713926 | 33714365 | 439 | 36 |
| 11 | CD81_001 | 2354853 | 2355382 | 529 | 76 |
| 11 | CD81_002 | 2363131 | 2363578 | 447 | 34 |
| 11 | CD81_003 | 2374118 | 2374563 | 445 | 27 |
| 11 | CDKN1C_001 | 2861490 | 2861724 | 234 | 15 |
| 11 | CDKN1C_002 | 2863931 | 2864321 | 390 | 39 |
| 11 | CRY2_01 | 45825594 | 45826171 | 577 | 52 |
| 11 | DDB2 | 47193104 | 47193534 | 430 | 24 |
| 11 | DDX10_001 | 108040712 | 108041221 | 509 | 38 |
| 11 | DDX6_001 | 118166720 | 118167251 | 531 | 60 |
| 11 | EXT2 | 44073738 | 44074158 | 420 | 54 |
| 11 | FANCF_01 | 22603534 | 22603929 | 395 | 34 |
| 11 | FANCF_02 | 22603322 | 22603606 | 284 | 19 |
| 11 | FLI1_01 | 128067717 | 128068237 | 520 | 31 |
| 11 | FLI1_02 | 128070143 | 128070375 | 232 | 19 |
| 11 | H19_001 | 1969797 | 1970340 | 543 | 29 |
| 11 | H19_002 | 1974299 | 1974540 | 241 | 22 |
| 11 | H19_003 | 1975988 | 1976465 | 477 | 32 |
| 11 | H19_004 | 1983261 | 1983752 | 491 | 38 |
| 11 | H19_005 | 1990257 | 1990744 | 487 | 28 |
| 11 | HCCA2_001 | 1726222 | 1726591 | 369 | 36 |
| 11 | HCCA2_002 | 1731116 | 1731640 | 524 | 35 |
| 11 | HCCA2_003 | 1741642 | 1741958 | 316 | 46 |
| 11 | HEAB_001 | 57181484 | 57181963 | 479 | 38 |
| 11 | HRAS_001 | 526559 | 527157 | 598 | 61 |
| 11 | HRAS_002 | 524576 | 524948 | 372 | 28 |
| 11 | HSPA8_1_01 | 122438457 | 122438798 | 341 | 25 |
| 11 | HSPA8_2_01 | 122438090 | 122438482 | 392 | 36 |
| 11 | IFITM1_01 | 300575 | 300909 | 334 | 19 |

(continued)

| Chromosome | GeneSymbol No. | genome start point | genome terminal point | length of Genome | No. of CpG |
|:---:|:---:|:---:|:---:|:---:|:---:|
| | | | Table 1: Tumor suppressor genes or cancer-related genetic regions that might cause an aberration of methylation (condition (1) (a)) | | |
| 11 | IGF2_001 | 2110661 | 2111061 | 400 | 24 |
| 11 | IGF2_002 | 2118423 | 2118844 | 421 | 49 |
| 11 | IGF2_003 | 2121965 | 2122388 | 423 | 38 |
| 11 | IGF2_004 | 2133388 | 2133777 | 389 | 29 |
| 11 | IL10RA_01 | 117361721 | 117362144 | 423 | 29 |
| 11 | KCNQ1_001 | 2421953 | 2422332 | 379 | 25 |
| 11 | KCNQ1_002 | 2423321 | 2423593 | 272 | 13 |
| 11 | KCNQ1_003 | 2510596 | 2510967 | 371 | 19 |
| 11 | KCNQ1_004 | 2511955 | 2512234 | 279 | 16 |
| 11 | KCNQ1_005 | 2550439 | 2550859 | 420 | 24 |
| 11 | KCNQ1_006 | 2552907 | 2553207 | 300 | 22 |
| 11 | KCNQ1_007 | 2559808 | 2560120 | 312 | 18 |
| 11 | KCNQ1_008 | 2677736 | 2678041 | 305 | 27 |
| 11 | KCNQ1_009 | 2769537 | 2769998 | 461 | 45 |
| 11 | KCNQ1_010 | 2774363 | 2774757 | 394 | 22 |
| 11 | KCNQ1_011 | 2785075 | 2785484 | 409 | 27 |
| 11 | KCNQ1_012 | 2828008 | 2828543 | 535 | 40 |
| 11 | KCNQ1_013 | 2840667 | 2841147 | 480 | 40 |
| 11 | KCNQ1ON_001 | 2846868 | 2847276 | 408 | 26 |
| 11 | MEN1_01 | 64334283 | 64334680 | 397 | 27 |
| 11 | MEN1_02 | 64333711 | 64334310 | 599 | 48 |
| 11 | MLL_02 | 117811321 | 117811673 | 352 | 22 |
| 11 | MRPL23_001 | 1925380 | 1925658 | 278 | 25 |
| 11 | MRPL23_002 | 1930709 | 1931081 | 372 | 17 |
| 11 | MRPL23_003 | 1934031 | 1934272 | 241 | 17 |
| 11 | MRPL23_004 | 1934636 | 1935034 | 398 | 23 |
| 11 | MRPL23_005 | 1939642 | 1939882 | 240 | 12 |
| 11 | MRPL23_006 | 1942563 | 1942961 | 398 | 27 |
| 11 | MRPL23_007 | 1947611 | 1947817 | 206 | 13 |
| 11 | MYOD_01_02 | 17697769 | 17698203 | 434 | 47 |
| 11 | NAP1L4_001 | 2922455 | 2922829 | 374 | 20 |
| 11 | NAP1L4_002 | 2969310 | 2969834 | 524 | 46 |
| 11 | NUMA1 | 71469069 | 71469354 | 285 | 27 |
| 11 | NUP98_01 | 3774899 | 3775243 | 344 | 28 |
| 11 | NUP98_02 | 3775642 | 3775908 | 266 | 20 |

(continued)

Table 1: Tumor suppressor genes or cancer-related genetic regions that might cause an aberration of methylation (condition (1) (a))

| Chromosome | GeneSymbol No. | genome start point | genome terminal point | length of Genome | No. of CpG |
|---|---|---|---|---|---|
| 11 | OSBPL5_001 | 3071292 | 3071628 | 336 | 21 |
| 11 | OSBPL5_002 | 3098082 | 3098500 | 418 | 18 |
| 11 | OSBPL5_003 | 3138080 | 3138622 | 542 | 38 |
| 11 | OSBPL5_004 | 3142680 | 3143112 | 432 | 59 |
| 11 | OSBPL5_005 | 3180978 | 3181397 | 419 | 24 |
| 11 | OSBPL5_006 | 3195952 | 3196500 | 548 | 46 |
| 11 | OSBPL5_007 | 3210207 | 3210674 | 467 | 26 |
| 11 | OSBPL5_008 | 3210650 | 3211020 | 370 | 17 |
| 11 | PAFAH1B2_001 | 116519863 | 116520360 | 497 | 43 |
| 11 | PCSK7_001 | 116607790 | 116608224 | 434 | 41 |
| 11 | PHLDA2_001 | 2906487 | 2907015 | 528 | 63 |
| 11 | PICALM_001 | 85457748 | 85458234 | 486 | 43 |
| 11 | PICALM_002 | 85457381 | 85457760 | 379 | 29 |
| 11 | PICALM_01 | 85457748 | 85458189 | 441 | 43 |
| 11 | PRO1073 | 65021396 | 65021824 | 428 | 42 |
| 11 | SDHD | 111462512 | 111462918 | 406 | 30 |
| 11 | SDHD_001 | 111462512 | 111462918 | 406 | 30 |
| 11 | SLC22A18_001 | 2880014 | 2880448 | 434 | 28 |
| 11 | SLC22A18_002 | 2886839 | 2887277 | 438 | 39 |
| 11 | SLC22A18_003 | 2899297 | 2899799 | 502 | 32 |
| 11 | SYTB_001 | 1803689 | 1803982 | 293 | 18 |
| 11 | TH_001 | 2144026 | 2144524 | 498 | 49 |
| 11 | TNNT3_001 | 1904057 | 1904357 | 300 | 20 |
| 11 | TNNT3_002 | 1905454 | 1905833 | 379 | 22 |
| 11 | TNNT3_003 | 1906788 | 1906996 | 208 | 10 |
| 11 | TNNT3_004 | 1915394 | 1915868 | 474 | 26 |
| 11 | TRPM5_001 | 2391916 | 2392414 | 498 | 32 |
| 11 | TRPM5_002 | 2398577 | 2399002 | 425 | 30 |
| 11 | WT1_001 | 32413036 | 32413392 | 356 | 47 |
| 11 | WT1_001 | 32411966 | 32412340 | 374 | 19 |
| 11 | WT1_002 | 32412703 | 32413062 | 359 | 28 |
| 11 | ZNF145_001 | 113435107 | 113435523 | 416 | 31 |
| 11 | ZNF195_001 | 3391545 | 3391986 | 441 | 21 |
| 11 | ZNF195_002 | 3401174 | 3401406 | 232 | 20 |
| 11 | ZNF215_001 | 6904285 | 6904809 | 524 | 43 |

(continued)

| | Table 1: Tumor suppressor genes or cancer-related genetic regions that might cause an aberration of methylation (condition (1) (a)) | | | | |
|---|---|---|---|---|---|
| Chromosome | GeneSymbol No. | genome start point | genome terminal point | length of Genome | No. of CpG |
| 12 | ATF1 | 49443878 | 49444208 | 330 | 30 |
| 12 | BCL7A | 120840600 | 120841134 | 534 | 40 |
| 12 | BTG1 | 91062777 | 91063262 | 485 | 48 |
| 12 | CCND2_001 | 4253140 | 4253668 | 528 | 30 |
| 12 | CCND2_002 | 4253833 | 4254352 | 519 | 35 |
| 12 | CDK4 | 56435611 | 56436146 | 535 | 59 |
| 12 | ELKS | 970533 | 971067 | 534 | 66 |
| 12 | G3PD_01 | 6513830 | 6514401 | 571 | 57 |
| 12 | GLI_01 | 56139875 | 56140298 | 423 | 29 |
| 12 | HAL_01 | 94913485 | 94913865 | 380 | 24 |
| 12 | HMGA_01 | 64505847 | 64506048 | 201 | 17 |
| 12 | HMGA2_001 | 64504118 | 64504535 | 417 | 32 |
| 12 | HOXC11_001 | 52652861 | 52653329 | 468 | 23 |
| 12 | HOXC13_001 | 52619125 | 52619630 | 505 | 52 |
| 12 | NACA | 55405193 | 55405733 | 540 | 37 |
| 12 | PTPN_11 | 111341140 | 111341705 | 565 | 60 |
| 12 | SLC38A4_001 | 45511305 | 45511701 | 396 | 29 |
| 12 | TCF1 | 119900742 | 119901144 | 402 | 21 |
| 12 | ZNF384_001 | 6668855 | 6669283 | 428 | 27 |
| 13 | AL137718_01 | 59635924 | 59636310 | 386 | 29 |
| 13 | BRCA2 | 31787393 | 31787925 | 532 | 48 |
| 13 | ERCC5 | 102296508 | 102296807 | 299 | 19 |
| 13 | FLT1_3_01 | 27966522 | 27966938 | 416 | 35 |
| 13 | FLT3 | 27572720 | 27573293 | 573 | 45 |
| 13 | FOXO1A_01 | 40139038 | 40139631 | 593 | 56 |
| 13 | FOXO1A_02 | 40139039 | 40139631 | 592 | 56 |
| 13 | FOXO1A_03 | 40136475 | 40136743 | 268 | 20 |
| 13 | HTR2A_001 | 46367732 | 46368191 | 459 | 9 |
| 13 | RB1_001 | 47775605 | 47776155 | 550 | 70 |
| 13 | ZNF198_001 | 19429932 | 19430275 | 343 | 24 |
| 14 | BCL11B_001 | 98808281 | 98808691 | 410 | 25 |
| 14 | CHGA_01 | 92458933 | 92459492 | 559 | 53 |
| 14 | CR601144_001 | 20528074 | 20528492 | 418 | 23 |
| 14 | DAD1_01 | 22127736 | 22128244 | 508 | 39 |
| 14 | DI03_001 | 101095604 | 101096110 | 506 | 49 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 14 | DLK1_001 | 100190642 | 100191182 | 540 | 34 |
| 14 | DLK1_002 | 100245239 | 100245459 | 220 | 12 |
| 14 | DLK1_003 | 100262866 | 100263271 | 405 | 59 |
| 14 | DLK1_004 | 100270299 | 100270717 | 418 | 28 |
| 14 | DLK1_005 | 100271281 | 100271557 | 276 | 15 |
| 14 | GOLGA5 | 92330376 | 92330722 | 346 | 28 |
| 14 | GPHN_01 | 66045075 | 66045469 | 394 | 32 |
| 14 | GPHN_02 | 66044621 | 66045096 | 475 | 52 |
| 14 | GPHN_03 | 66044061 | 66044371 | 310 | 23 |
| 14 | HSPCA_001 | 101675929 | 101676415 | 486 | 36 |
| 14 | KTN1_001 | 55116307 | 55116841 | 534 | 71 |
| 14 | MEG3_001 | 100360100 | 100360493 | 393 | 25 |
| 14 | MEG3_001 | 100419237 | 100419637 | 400 | 28 |
| 14 | MEG3_002 | 100362061 | 100362394 | 333 | 19 |
| 14 | MEG3_003 | 100362585 | 100362810 | 225 | 13 |
| 14 | MEG3_004 | 100363911 | 100364143 | 232 | 16 |
| 14 | MEG3_005 | 100418029 | 100418475 | 446 | 27 |
| 14 | N_MYC_1_01 | 20563331 | 20563858 | 527 | 38 |
| 14 | N MYC_2_01 | 20562467 | 20562884 | 417 | 29 |
| 14 | NIN_001 | 50368041 | 50368421 | 380 | 15 |
| 14 | PSME2_01 | 23686078 | 23686449 | 371 | 19 |
| 14 | RAD51L1 | 67211132 | 67211662 | 530 | 64 |
| 14 | TCL1A_01 | 95249899 | 95250387 | 488 | 33 |
| 14 | TCL1A_02 | 95250513 | 95250722 | 209 | 9 |
| 14 | TRIP11_001 | 91576058 | 91576347 | 289 | 29 |
| 14 | TSHR_001 | 80490972 | 80491378 | 406 | 27 |
| 15 | AF15Q14 | 38673556 | 38673925 | 369 | 24 |
| 15 | ANXA2_01 | 58477484 | 58477917 | 433 | 32 |
| 15 | ATP10A_001 | 23509898 | 23510365 | 467 | 29 |
| 15 | ATP10A_002 | 23532141 | 23532509 | 368 | 20 |
| 15 | ATP10A_003 | 23658607 | 23659121 | 514 | 43 |
| 15 | ATP10A_004 | 23785703 | 23786045 | 342 | 23 |
| 15 | ATP10A_005 | 23878503 | 23878788 | 285 | 16 |
| 15 | Beta_NAP_01 | 81175787 | 81176040 | 253 | 19 |
| 15 | BLM_001 | 89061315 | 89061847 | 532 | 47 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 15 | BUB1B_001 | 38240272 | 38240679 | 407 | 31 |
| 15 | GABRB3_001 | 24425349 | 24425703 | 354 | 26 |
| 15 | GABRB3_002 | 24466474 | 24466763 | 289 | 18 |
| 15 | GABRB3_003 | 24568107 | 24568366 | 259 | 13 |
| 15 | NDN_001 | 21482868 | 21483396 | 528 | 42 |
| 15 | NDN_002 | 21674174 | 21674644 | 470 | 29 |
| 15 | NDN_003 | 21897782 | 21898201 | 419 | 32 |
| 15 | NDN_004 | 22057019 | 22057428 | 409 | 23 |
| 15 | NDN_005 | 22223252 | 22223670 | 418 | 29 |
| 15 | NTRK3 | 86600898 | 86601498 | 600 | 59 |
| 15 | PML_001 | 72077492 | 72077906 | 414 | 27 |
| 15 | RAD51_1_01 | 38774114 | 38774530 | 416 | 33 |
| 15 | RAD51_2_01 | 38774749 | 38775137 | 388 | 37 |
| 15 | RASGRF1_001 | 77169886 | 77170323 | 437 | 46 |
| 15 | SNRPN_001 | 22273701 | 22274205 | 504 | 35 |
| 15 | SNRPN_002 | 22471921 | 22472397 | 476 | 41 |
| 15 | SNRPN_003 | 22569356 | 22569698 | 342 | 28 |
| 15 | SNRPN_004 | 22644252 | 22644787 | 535 | 41 |
| 15 | SNRPN_005 | 22674608 | 22674881 | 273 | 23 |
| 15 | SNRPN_006 | 22751410 | 22751904 | 494 | 31 |
| 15 | TCF12_01 | 54998178 | 54998751 | 573 | 69 |
| 15 | TCF12_02 | 54997492 | 54997723 | 231 | 20 |
| 15 | UBE3A_001 | 23234955 | 23235465 | 510 | 73 |
| 15 | UBE3A_002 | 23392822 | 23393324 | 502 | 29 |
| 16 | CBFA2T3_001 | 87534056 | 87534546 | 490 | 36 |
| 16 | CBFB | 65619930 | 65620344 | 414 | 22 |
| 16 | CDH1_001 | 67328704 | 67329209 | 505 | 50 |
| 16 | CDH11_001 | 63713205 | 63713703 | 498 | 47 |
| 16 | CREBBP_001 | 3870965 | 3871413 | 448 | 46 |
| 16 | CYLD | 49333974 | 49334203 | 229 | 19 |
| 16 | DC13_1_01 | 79597712 | 79598092 | 380 | 41 |
| 16 | DC13_2_01 | 79598348 | 79598723 | 375 | 32 |
| 16 | DDIT3 | 31098230 | 31098474 | 244 | 13 |
| 16 | E_cad_02 | 67329401 | 67329750 | 349 | 24 |
| 16 | ERCC4 | 13921687 | 13921995 | 308 | 20 |

(continued)

Table 1: Tumor suppressor genes or cancer-related genetic regions that might cause an aberration of methylation (condition (1) (a))

| Chromosome | GeneSymbol No. | genome start point | genome terminal point | length of Genome | No. of CpG |
|---|---|---|---|---|---|
| 16 | FANCA | 88410663 | 88411053 | 390 | 46 |
| 16 | FUS | 31098697 | 31099112 | 415 | 39 |
| 16 | KIAA0683_01 | 1483654 | 1483960 | 306 | 30 |
| 16 | MAF_001 | 78191338 | 78191880 | 542 | 69 |
| 16 | MHC2TA_01 | 10880484 | 10880911 | 427 | 28 |
| 16 | MYH11_001 | 15858290 | 15858793 | 503 | 44 |
| 16 | TSC2_001 | 2037916 | 2038277 | 361 | 43 |
| 17 | ALO17 | 75849710 | 75850074 | 364 | 19 |
| 17 | ASPSCR1 | 77529129 | 77529451 | 322 | 32 |
| 17 | BHD | 17080723 | 17081162 | 439 | 27 |
| 17 | BIRC5_01 | 73721633 | 73722084 | 451 | 42 |
| 17 | BRCA1 | 38531626 | 38532076 | 450 | 25 |
| 17 | CA4_01 | 55582147 | 55582640 | 493 | 50 |
| 17 | CLTC_001 | 55051668 | 55052177 | 509 | 45 |
| 17 | COL1A1_001 | 45633408 | 45633912 | 504 | 36 |
| 17 | ERBB2_01 | 35110079 | 35110362 | 283 | 23 |
| 17 | ERBB2_02 | 35110081 | 35110361 | 280 | 23 |
| 17 | ETV4_01 | 38978023 | 38978479 | 456 | 36 |
| 17 | ETV4_02 | 38978021 | 38978479 | 458 | 36 |
| 17 | EXOC7_01 | 71611344 | 71611677 | 333 | 29 |
| 17 | FOXK2_01 | 78070361 | 78070585 | 224 | 29 |
| 17 | GAS7_001 | 10042696 | 10043211 | 515 | 61 |
| 17 | HCMOGT_1_001 | 19999746 | 20000273 | 527 | 56 |
| 17 | HLF | 50697142 | 50697471 | 329 | 45 |
| 17 | MAP2K4_001 | 11864591 | 11865051 | 460 | 49 |
| 17 | MAP2K4_002 | 11865434 | 11865718 | 284 | 22 |
| 17 | MLLT6_01 | 34113070 | 34113580 | 510 | 32 |
| 17 | MLLT6_03 | 34114090 | 34114402 | 312 | 15 |
| 17 | MSF | 72789206 | 72789610 | 404 | 33 |
| 17 | MSI2_001 | 52688381 | 52688824 | 443 | 48 |
| 17 | NF1 | 26445739 | 26446339 | 600 | 45 |
| 17 | Nm23_01 | 46585758 | 46586275 | 517 | 40 |
| 17 | p53_03 | 7532346 | 7532539 | 193 | 20 |
| 17 | PECAM1_01 | 59817588 | 59817941 | 353 | 14 |
| 17 | PER1_001 | 7996232 | 7996656 | 424 | 31 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 17 | PRKAR1A | 64019428 | 64019890 | 462 | 41 |
| 17 | PSMB6_01 | 4646233 | 4646687 | 454 | 23 |
| 17 | RARA | 35751090 | 35751589 | 499 | 47 |
| 17 | SUZ12_001 | 27287847 | 27288203 | 356 | 43 |
| 17 | TNFRSF6_001 | 71448337 | 71448803 | 466 | 42 |
| 17 | TP53_001 | 7532164 | 7532609 | 445 | 36 |
| 18 | ATP5A1_1_01 | 41938228 | 41938674 | 446 | 33 |
| 18 | BCL2_001 | 59138023 | 59138387 | 364 | 50 |
| 18 | FVT1_001 | 59184906 | 59185126 | 220 | 21 |
| 18 | IMPACT_001 | 20260282 | 20260730 | 448 | 41 |
| 18 | MADH4 | 46810401 | 46810721 | 320 | 33 |
| 18 | SS18 | 21924328 | 21924906 | 578 | 51 |
| 18 | TCEB3C_001 | 42809465 | 42809852 | 387 | 28 |
| 19 | AKT2_01 | 45482785 | 45483311 | 526 | 57 |
| 19 | AKT2_02 | 45482787 | 45483311 | 524 | 57 |
| 19 | AURKC_001 | 62433770 | 62434304 | 534 | 38 |
| 19 | AURKC_002 | 62443713 | 62443972 | 259 | 24 |
| 19 | BCL3 | 49943692 | 49944195 | 503 | 67 |
| 19 | BCL3_001 | 49943692 | 49944195 | 503 | 67 |
| 19 | CDC34_01 | 482976 | 483323 | 347 | 31 |
| 19 | CEBPA_01 | 38485154 | 38486420 | 1266 | 156 |
| 19 | COL5A3_01 | 9981936 | 9982276 | 340 | 36 |
| 19 | COX6B1_1_01 | 40825966 | 40826291 | 325 | 31 |
| 19 | COX6B1_2_01 | 40825956 | 40826257 | 301 | 28 |
| 19 | ELL_001 | 18494063 | 18494512 | 449 | 32 |
| 19 | ERCC2 | 50565436 | 50565898 | 462 | 41 |
| 19 | FSTL3_001 | 626625 | 626920 | 295 | 18 |
| 19 | ICAM1_01 | 10241875 | 10242277 | 402 | 35 |
| 19 | KSRP_1_01 | 6376068 | 6376343 | 275 | 13 |
| 19 | KSRP_2_01 | 6376069 | 6376343 | 274 | 13 |
| 19 | MECT1 | 18655112 | 18655621 | 509 | 63 |
| 19 | MLLT1_001 | 6230380 | 6230801 | 421 | 45 |
| 19 | STK11_01 | 1157536 | 1157912 | 376 | 27 |
| 19 | STK11_02 | 1157893 | 1158270 | 377 | 24 |
| 19 | TCF3_01 | 1597499 | 1597737 | 238 | 14 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 19 | TFPT_001 | 59310656 | 59311052 | 396 | 31 |
| 19 | TPM4_001 | 16048692 | 16049124 | 432 | 42 |
| 19 | USP29_001 | 62302435 | 62302863 | 428 | 31 |
| 19 | USP29_002 | 62309367 | 62309891 | 524 | 46 |
| 19 | USP29_003 | 62322196 | 62322469 | 273 | 27 |
| 19 | ZIM2_001 | 61968659 | 61968953 | 294 | 13 |
| 19 | ZIM2_002 | 61998579 | 61998953 | 374 | 30 |
| 19 | ZIM2_003 | 62041908 | 62042346 | 438 | 27 |
| 19 | ZIM2_004 | 62043142 | 62043554 | 412 | 29 |
| 19 | ZIM2_005 | 62043954 | 62044200 | 246 | 9 |
| 19 | ZIM2_006 | 62067585 | 62067965 | 380 | 30 |
| 19 | ZIM3_001 | 62375472 | 62375840 | 368 | 24 |
| 19 | ZNF264_001 | 62394699 | 62395208 | 509 | 49 |
| 19 | ZNF272_001 | 62483493 | 62483962 | 469 | 44 |
| 19 | ZNF331_001 | 58715785 | 58716233 | 448 | 24 |
| 20 | DSTN_01 | 17498585 | 17499165 | 580 | 68 |
| 20 | GNAS_001 | 56848822 | 56849135 | 313 | 30 |
| 20 | GNAS_01 | 56897562 | 56898110 | 548 | 56 |
| 20 | GNAS_02 | 56898967 | 56899284 | 317 | 33 |
| 20 | MAFB_001 | 38750860 | 38751343 | 483 | 59 |
| 20 | MYBL2_1_01 | 41729003 | 41729471 | 468 | 57 |
| 20 | MYBL2_2_01 | 41729004 | 41729471 | 467 | 57 |
| 20 | NNAT_001 | 35581984 | 35582269 | 285 | 24 |
| 20 | SS18L1_001 | 60151349 | 60151613 | 264 | 37 |
| 20 | SS18L1_002 | 60152674 | 60153181 | 507 | 55 |
| 20 | TOP1_001 | 39090892 | 39091362 | 470 | 57 |
| 20 | TPD52L2_001 | 61966654 | 61966989 | 335 | 22 |
| 21 | COL6A2_01 | 46356772 | 46357061 | 289 | 24 |
| 21 | ERG_001 | 38955346 | 38955681 | 335 | 20 |
| 21 | OLIG2 | 33317392 | 33317712 | 320 | 22 |
| 21 | RUNX1_001 | 35184917 | 35185243 | 326 | 24 |
| 21 | TMPRSS2_001 | 41802132 | 41802569 | 437 | 30 |
| 22 | BCR_01 | 21853331 | 21853838 | 507 | 69 |
| 22 | BCR_02 | 21853333 | 21853838 | 505 | 69 |
| 22 | CHEK2_001 | 27467870 | 27468262 | 392 | 27 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol No. | genome start point | genome terminal point | length of Genome | No. of CpG |
| 22 | CLTCL1_001 | 17659116 | 17659652 | 536 | 56 |
| 22 | EP300 | 39817467 | 39817757 | 290 | 22 |
| 22 | EWSR1 | 27994181 | 27994700 | 519 | 58 |
| 22 | GNAZ_01 | 21742354 | 21742845 | 491 | 86 |
| 22 | MKL1_001 | 39362391 | 39363197 | 806 | 76 |
| 22 | MN1 | 26526421 | 26527018 | 597 | 45 |
| 22 | MYH9_001 | 35113893 | 35114426 | 533 | 43 |
| 22 | NDUFA6_01 | 40816187 | 40816786 | 599 | 49 |
| 22 | NF2_001 | 28329371 | 28329908 | 537 | 63 |
| 22 | PDGFB | 37970352 | 37970936 | 584 | 63 |
| 22 | ZNF278_001 | 30072715 | 30073093 | 378 | 30 |
| x | GPC3 | 132947001 | 132947234 | 233 | 21 |
| x | MLLT7 | 70232993 | 70233375 | 382 | 25 |
| x | MSN | 64804313 | 64804586 | 273 | 18 |
| x | MTCP1_001 | 153952418 | 153952966 | 548 | 70 |
| x | NONO_001 | 70420123 | 70420434 | 311 | 24 |
| x | NPD017_01 | 102727169 | 102727608 | 439 | 24 |
| x | PAK_3_01 | 110225987 | 110226378 | 391 | 30 |
| x | SEPT6_001 | 118710422 | 118710923 | 501 | 51 |
| x | TFE3 | 48787429 | 48787872 | 443 | 40 |

Such aberration of methylation in tumor suppressor genes or cancer-related genetic regions on the genomic DNA can be identified by a method comprising the steps of preparing a genomic DNA from cells, performing a comprehensive analysis of the methylated genome using a suitable genome analyzer such as Infinium HumanMethylation450 BeadChip or Infinium HumanMethylation27 BeadChip of Illumina, Inc., Cancer EpiPanel of Sequenom, Inc., or EpiTect Methyl qPCR Array system of SABiosciences, and comparing the detected genomic methylation with that of a standard cell. Among these genome analyzers, Cancer EpiPanel is known to contain 400 genes and over 12,000 CpG sites in promoter regions of genes known to be involved in neoplastic transformation and imprinting.

In the present invention, the induced malignant stem cells capable of *in vitro* proliferation may also have (1) (b) a somatic mutation of a tumor suppressor gene or a somatic mutation of an endogenous cancer-related gene in endogenous genomic DNA. The term "somatic mutation" as used herein covers mutations in tumor suppressor genes or those genes which are recognized as oncogenes in endogenous genomic DNA, as well as driver mutations which are carcinogenic genetic mutations other than the mutations in tumor suppressor genes or those genes which are recognized as oncogenes in endogenous genomic DNA. Examples of such somatic mutation of a tumor suppressor gene or somatic mutation of an endogenous cancer-related gene in endogenous genomic DNA preferably occur in at least one of the genes listed in the following table:

[Table 2]

| Table 2: Tumor suppressor genes or endogenous cancer-related genes that might cause somatic mutation (condition (1) (b)) | | |
|---|---|---|
| Chromosome | GeneSymbol | refseq id |
| 1 | CDK11B | NM_033486, NM_033487, NM_033488, NM_033489, NM_033492, NM_033493, NM_024011, NM_033529 |
| 1 | CDK11A | NM_024011, NM_033486, NM_033487, NM_033488, NM_033489, NM_033492, NM_033493, NM_033529 |
| 1 | PRKCZ | NM_002744, NM_001033581, NM_001033582, NM_001146310 |
| 1 | C1orf86 | NM_001033581, NM_001033582, NM_001146310, NM_002744 |
| 1 | PIK3CD | NM_005026, NM_001009566, NM_014944 |
| 1 | CLSTN1 | NM_005026, NM_001009566, NM_014944 |
| 1 | SRM | NM_003132 |
| 1 | MTOR | NM_004958 |
| 1 | EPHA2 | NM 004431 |
| 1 | PINK1 | NM_032409 |
| 1 | EPHA8 | NM_001006943, NM_020526 |
| 1 | EPHB2 | NM_004442, NM_017449 |
| 1 | PDIK1L | NR_026685, NM_152835, NR_026686 |
| 1 | RPS6KA1 | NM_002953, NM_001006665, NR_031740 |
| 1 | MIR1976 | NM_001006665, NM_002953, NR_031740 |
| 1 | MAP3K6 | NM_004672 |
| 1 | FGR | NM_001042729, NM_001042747, NM_005248 |
| 1 | LCK | NM_005356, NM_001042771 |
| 1 | TSSK3 | NM_052841 |
| 1 | STK40 | NM_032017 |
| 1 | EPHA10 | NM_001099439, NM_173641 |
| 1 | TIE1 | NM_005424 |
| 1 | RNF220 | NM_018150 |
| 1 | PLK3 | NM_004073, NM_001013632 |
| 1 | TCTEX1D4 | NM_004073, NM_001013632 |
| 1 | TOE1 | NM_007170, NM_025077 |
| 1 | TESK2 | NM_007170, NM_025077 |
| 1 | MAST2 | NM_015112 |
| 1 | PIK3R3 | NM_001114172, NM_003629 |
| 1 | MKNK1 | NM_001135553, NM_003684, NM_198973, NR_024174, NR_024176 |
| 1 | SPATA6 | NM_019073 |
| 1 | PRKAA2 | NM_006252 |
| 1 | ROR1 | NM_001083592, NM_005012 |
| 1 | RAVER2 | NM_002227, NM_018211 |
| 1 | JAK1 | NM_002227, NM_018211 |

(continued)

| Chromosome | GeneSymbol | refseq id |
|---|---|---|
| | | Table 2: Tumor suppressor genes or endogenous cancer-related genes that might cause somatic mutation (condition (1) (b)) |
| 1 | FPGT | NM_001112808, NM_003838 |
| 1 | TNNI3K | NM_001112808, NM_003838, NM_015978 |
| 1 | PRKACB | NM_002731, NM_207578, NM_182948 |
| 1 | PKN2 | NM_006256 |
| 1 | CDC7 | NM_001134419, NM_003503, NM_001134420 |
| 1 | BRDT | NM_207189, NM_001726 |
| 1 | HIPK1 | NM_152696, NM_198268, NM_198269, NM_181358 |
| 1 | TRIM33 | NM_015906, NM_033020 |
| 1 | NRAS | NM_002524 |
| 1 | PIP5K1A | NM_001135636, NM_001135637, NM_001135638, NM_003557 |
| 1 | PSMD4 | NM_002810 |
| 1 | PI4KB | NM_002651 |
| 1 | NPR1 | NM_000906 |
| 1 | CLK2 | NM_003993 |
| 1 | HCN3 | NM_000298, NM_020897, NM_181871 |
| 1 | PKLR | NM_000298, NM_020897, NM_181871 |
| 1 | SH2D2A | NM_001007792, NM_001161441, NM_001161442, NM_001161443, NM_001161444, NM_003975 |
| 1 | NTRK1 | NM_001007792, NM_001161441, NM_001161442, NM_001161443, NM_001161444, NM_003975, NM_014215, NM_001012331, NM_002529 |
| 1 | INSRR | NM_001007792, NM_014215 |
| 1 | UHMK1 | NM_001184763, NM_144624, NM_175866 |
| 1 | DDR2 | NM_001014796, NM_006182 |
| 1 | C1orf112 | NM_018186, NM_020423, NM_181093 |
| 1 | SCYL3 | NM_018186, NM_020423, NM_181093 |
| 1 | ABL2 | NM_001136000, NM_001168236, NM_001168237, NM_001168238, NM_001168239, NM_005158, NM_007314, NM_001136001 |
| 1 | RNASEL | NM_021133 |
| 1 | NEK7 | NM_133494 |
| 1 | PIK3C2B | NM_002646 |
| 1 | DSTYK | NM_015375, NM_199462 |
| 1 | NUAK2 | NM_030952 |
| 1 | CDK18 | NM_002596, NM_212502, NM_212503 |
| 1 | IKBKE | NM_014002 |
| 1 | DYRK3 | NM_001004023, NM_003582 |
| 1 | MAPKAPK2 | NM_004759, NM_032960 |

(continued)

| Chromosome | GeneSymbol | refseq id |
|---|---|---|
| | | Table 2: Tumor suppressor genes or endogenous cancer-related genes that might cause somatic mutation (condition (1) (b)) |
| 1 | CAMK1G | NM_020439 |
| 1 | NEK2 | NM_002497 |
| 1 | RPS6KC1 | NM_001136138, NM_012424 |
| 1 | MARK1 | NM_018650 |
| 1 | ITPKB | NM_002221 |
| 1 | CABC1 | NM_020247 |
| 1 | CDC42BPA | NM_003607, NM_014826 |
| 1 | OBSCN | NM_001098623, NM_052843 |
| 1 | KIAA1804 | NM_032435 |
| 1 | SDCCAG8 | NM_006642, NM_181690 |
| 1 | AKT3 | NM_006642, NM_181690, NM_005465 |
| 2 | ROCK2 | NM_004850 |
| 2 | TRIB2 | NM_021643, NR_027303 |
| 2 | NRBP1 | NM_013392 |
| 2 | ALK | NM_004304 |
| 2 | EIF2AK2 | NM_001135651, NM_001135652, NM_002759 |
| 2 | PRKD3 | NM_005813 |
| 2 | CDKL4 | NM_001009565 |
| 2 | MAP4K3 | NM_003618 |
| 2 | PKDCC | NM_138370 |
| 2 | PRKCE | NM_005400 |
| 2 | VRK2 | NM_001130480, NM_001130481, NM_001130482, NM_001130483, NM_001136027, NM_006296, NM_001114636, NM_018062 |
| 2 | FANCL | NM_001114636, NM_001130480, NM_001130481, NM_001130482, NM_001130483, NM_001136027, NM_006296, NM_018062 |
| 2 | ACTR2 | NM_001005386, NM_005722 |
| 2 | AAK1 | NM_014911 |
| 2 | EIF2AK3 | NM_004836 |
| 2 | ZAP70 | NM_001079, NM_207519 |
| 2 | INPP4A | NM_001134224, NM_001134225, NM_001566, NM_004027 |
| 2 | MAP4K4 | NM_004834, NM_145686, NM_145687 |
| 2 | BUB1 | NM_004336 |
| 2 | MERTK | NM_006343 |
| 2 | MAP3K2 | NM_006609 |
| 2 | YSK4 | NM_001018046, NM_025052 |
| 2 | ACVR2A | NM_001616 |
| 2 | ACVR1C | NM_001111031, NM_001111032, NM_001111033, NM_145259 |

(continued)

| Chromosome | GeneSymbol | refseq id |
|---|---|---|
| | | Table 2: Tumor suppressor genes or endogenous cancer-related genes that might cause somatic mutation (condition (1) (b)) |
| 2 | ACVR1 | NM_001105, NM_001111067 |
| 2 | STK39 | NM_013233 |
| 2 | MYO3B | NM_001083615, NM_001171642, NM_138995 |
| 2 | TLK1 | NM_001136554, NM_001136555, NM_012290 |
| 2 | PDK1 | NM_002610 |
| 2 | ZAK | NM_016653, NM_133646 |
| 2 | MIR548N | NM_003319, NM_133378, NM_133432, NM_133437, NR_031666 |
| 2 | TTN | NM_003319, NM_133378, NM_133432, NM_133437, NR_031666, NM_133379 |
| 2 | STK17B | NM_004226 |
| 2 | CLK1 | NM_001162407, NM_004071, NR_027855, NR_027856 |
| 2 | STRADB | NM_018571 |
| 2 | CDK15 | NM_139158 |
| 2 | BMPR2 | NM_001204 |
| 2 | IDH1 | NM_005896 |
| 2 | PIKFYVE | NM_001178000, NM_015040, NM_152671 |
| 2 | ERBB4 | NM_001042599, NM_005235 |
| 2 | RNF25 | NM_015690, NM_022453 |
| 2 | STK36 | NM_015690, NM_022453 |
| 2 | STK16 | NM_001008910, NR_026909, NM_006000 |
| 2 | TUBA4A | NM_001008910, NR_026909, NM_006000 |
| 2 | SPEG | NM_005876, NM_001173476 |
| 2 | EPHA4 | NM_004438 |
| 2 | DGKD | NM_003648, NM_152879 |
| 2 | PASK | NM_015148 |
| 2 | STK25 | NM_006374 |
| 3 | OGG1 | NM_002542, NM_003656, NM_016819, NM_016820, NM_016821, NM_016826, NM_016827, NM_016828, NM_016829 |
| 3 | CAMK1 | NM_002542, NM_003656, NM_016819, NM_016820, NM_016821, NM_016826, NM_016827, NM_016828, NM_016829 |
| 3 | IRAK2 | NM_001570 |
| 3 | ATG7 | NM_001136031, NM_001144912, NM_006395 |
| 3 | RAF1 | NM_002880 |
| 3 | KCNH8 | NM_144633 |
| 3 | NEK10 | NM_199347 |
| 3 | TGFBR2 | NM_001024847, NM_003242 |
| 3 | DCLK3 | NM_033403 |

(continued)

| Chromosome | GeneSymbol | refseq id |
|---|---|---|
| | Table 2: Tumor suppressor genes or endogenous cancer-related genes that might cause somatic mutation (condition (1) (b)) |||
| 3 | MLH1 | NM_000249, NM_001167617, NM_001167618, NM_001167619 |
| 3 | OXSR1 | NM_005109 |
| 3 | ACVR2B | NM_001106 |
| 3 | CTNNB1 | NM_001098209, NM_001098210, NM_001904 |
| 3 | ULK4 | NM_017886 |
| 3 | SNRK | NM_001100594, NM_017719 |
| 3 | IP6K2 | NM_001005909, NM_016291, NM_001005910, NM_001005911, NM_001146178, NM_001146179, NR_027437, NR_027438 |
| 3 | IP6K1 | NM_001006115, NM_153273 |
| 3 | CAMKV | NM_024046 |
| 3 | MST1R | NM_002447 |
| 3 | MAPKAPK3 | NM_004635 |
| 3 | NEK4 | NM_003157 |
| 3 | PRKCD | NM_006254, NM_212539 |
| 3 | PXK | NM_017771 |
| 3 | EPHA3 | NM_005233, NM_182644 |
| 3 | EPHA6 | NM_001080448, NM_173655 |
| 3 | GSK3B | NM_001146156, NM_002093 |
| 3 | MYLK | NM_053025, NM_053026, NM_053027, NM_053028, NM_053031, NM_053032 |
| 3 | KALRN | NM_001024660, NM_003947, NR_028136, NM_007064 |
| 3 | SNX4 | NM_003794 |
| 3 | PIK3R4 | NM_014602 |
| 3 | NEK11 | NM_001146003, NM_024800, NM_145910 |
| 3 | RYK | NM_001005861, NM_002958 |
| 3 | EPHB1 | NM_004441 |
| 3 | PIK3CB | NM_006219 |
| 3 | GRK7 | NM_139209 |
| 3 | ATR | NM_001184 |
| 3 | PRKCI | NM_002740 |
| 3 | TNIK | NM_001161560, NM_001161561, NM_001161562, NM_001161563, NM_001161564, NM_001161565, NM_001161566, NM_015028, NR_027767 |
| 3 | PIK3CA | NM_006218 |
| 3 | EPHB3 | NM_004443 |
| 3 | MAP3K13 | NM_004721 |
| 3 | DGKG | NM_001080744, NM_001080745, NM_001346 |

(continued)

| Chromosome | GeneSymbol | refseq id |
|---|---|---|
| | | Table 2: Tumor suppressor genes or endogenous cancer-related genes that might cause somatic mutation (condition (1) (b)) |
| 3 | TNK2 | NM_001010938, NM_005781 |
| 3 | PAK2 | NM_002577 |
| 4 | GAK | NM_005255 |
| 4 | DGKQ | NM_001347 |
| 4 | FGFR3 | NM_000142, NM_001163213, NM_022965 |
| 4 | POLN | NM_024511, NM_181808 |
| 4 | HAUS3 | NM_024511, NM_181808 |
| 4 | GRK4 | NM_001004056, NM_001004057, NM_182982 |
| 4 | STK32B | NM_018401 |
| 4 | KCNIP4 | NM_001035003, NM_001035004, NM_147182, NM_147183 |
| 4 | PI4K2B | NM_018323 |
| 4 | TXK | NM_003328 |
| 4 | TEC | NM_003215 |
| 4 | PDGFRA | NM_006206 |
| 4 | KIT | NM_000222, NM_001093772 |
| 4 | KDR | NM_002253 |
| 4 | EPHA5 | NM_004439, NM_182472 |
| 4 | CDKL2 | NM_003948 |
| 4 | BMP2K | NM_017593, NM_198892 |
| 4 | PRKG2 | NM_006259 |
| 4 | MAPK10 | NM_002753, NM_138980, NM_138981, NM_138982 |
| 4 | BMPR1B | NM_001203 |
| 4 | NFKB1 | NM_001165412, NM_003998 |
| 4 | TBCK | NM_001163435, NM_001163436, NM_001163437, NM_033115, NM_001142415, NM_004757, NM_001142416 |
| 4 | AIMP1 | NM_001142415, NM_001163435, NM_001163436, NM_001163437, NM_004757, NM_033115, NM_001142416 |
| 4 | ALPK1 | NM_001102406, NM_025144 |
| 4 | CAMK2D | NM_001221, NM_172127, NM_172128, NM_172114, NM_172115, NM_172129 |
| 4 | PLK4 | NM_014264 |
| 4 | ELF2 | NM_201999 |
| 4 | GAB1 | NM_002039, NM_207123 |
| 4 | DCLK2 | NM_001040260, NM_001040261 |
| 4 | FBXW7 | NM_001013415, NM_018315, NM_033632 |
| 4 | NEK1 | NM_012224 |
| 5 | TERT | NM_198253, NM_198255 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 5 | TRIO | NM_007118 |
| 5 | PRKAA1 | NM_006251, NM_206907 |
| 5 | MGC42105 | NM_153361 |
| 5 | MAP3K1 | NM_005921 |
| 5 | PLK2 | NM_006622 |
| 5 | MAST4 | NM_001164664, NM_198828, NM_015183 |
| 5 | PIK3R1 | NM_181523, NM_181524, NM_181504 |
| 5 | CDK7 | NM_001799 |
| 5 | SV2C | NM_014979 |
| 5 | RIOK2 | NM_018343, NM_001159749 |
| 5 | FER | NM_005246 |
| 5 | CAMK4 | NM_001744 |
| 5 | APC | NM_001127511, NM_000038, NM_001127510 |
| 5 | MCC | NM_001085377, NM_032028 |
| 5 | TSSK1B | NM_001085377, NM_032028 |
| 5 | CSNK1G3 | NM_001031812, NM_001044722, NM_001044723, NM_004384 |
| 5 | CDKL3 | NM_001113575, NM_016508 |
| 5 | STK32A | NM_001112724, NM_145001 |
| 5 | CSNK1A1 | NM_001025105, NM_001892 |
| 5 | CSF1R | NM_005211 |
| 5 | PDGFRB | NM_002609 |
| 5 | CAMK2A | NM_015981, NM_171825 |
| 5 | ITK | NM_005546 |
| 5 | ODZ2 | NM_001122679 |
| 5 | STK10 | NM_005990 |
| 5 | FGFR4 | NM_002011, NM_213647, NM_022963 |
| 5 | GRK6 | NM_001004105, NM_001004106, NM_002082 |
| 5 | COL23A1 | NM_173465 |
| 5 | CLK4 | NM_020666 |
| 5 | MAPK9 | NM_002752, NM_139068, NM_139069, NM_139070, NM_001135044 |
| 5 | FLT4 | NM_182925, NM_002020 |
| 6 | MYLK4 | NM_001012418 |
| 6 | RIPK1 | NM_003804 |
| 6 | PRPF4B | NM_003913 |
| 6 | RIOK1 | NM_031480, NM_153005 |
| 6 | PIP5K1P1 | NR_027712 |

(continued)

| Chromosome | GeneSymbol | refseq id |
|---|---|---|
| | Table 2: Tumor suppressor genes or endogenous cancer-related genes that might cause somatic mutation (condition (1) (b)) | |
| 6 | MAK | NM_005906 |
| 6 | DDR1 | NM_001954, NM_013993, NM_013994 |
| 6 | DOM3Z | NM_005510, NR_026717, NM_004197, NM_032454 |
| 6 | STK19 | NM_005510, NR_026717, NM_004197, NM_032454 |
| 6 | BRD2 | NM_005104, NM_001113182 |
| 6 | IP6K3 | NM_001142883, NM_054111 |
| 6 | SRPK1 | NM_003137 |
| 6 | MAPK14 | NM_001315, NM_139012, NM_139013, NM_139014 |
| 6 | MAPK13 | NM_002754 |
| 6 | STK38 | NM_007271 |
| 6 | PIM1 | NM_002648 |
| 6 | CCND3 | NM_001136017, NM_001136125, NM_001136126, NM_001760 |
| 6 | PTK7 | NM_002821, NM_152880, NM_152881, NM_152882 |
| 6 | TTBK1 | NM_032538 |
| 6 | POLH | NM_006502 |
| 6 | NFKBIE | NM_004556 |
| 6 | ICK | NM_014920, NM_016513 |
| 6 | TTK | NM_001166691, NM_003318 |
| 6 | MAP3K7 | NM_003188, NM_145331, NM_145332, NM_145333 |
| 6 | EPHA7 | NM_004440 |
| 6 | CDK19 | NM_015076 |
| 6 | FYN | NM_002037, NM_153047, NM_153048 |
| 6 | FRK | NM_002031 |
| 6 | ROS1 | NM_002944 |
| 6 | LAMA2 | NM_000426, NM_001079823 |
| 6 | SGK1 | NM_001143676, NM_001143677, NM_001143678, NM_005627 |
| 6 | MAP3K5 | NM_005923 |
| 6 | LATS1 | NM_004690 |
| 6 | ESR1 | NM_000125, NM_001122740, NM_001122741, NM_001122742 |
| 6 | MAP3K4 | NM_005922, NM_006724 |
| 6 | RPS6KA2 | NM_001006932, NM_021135 |
| 7 | AIMP2 | NM_001134335, NM_006303, NM_014413 |
| 7 | EIF2AK1 | NM_001134335, NM_006303, NM_014413 |
| 7 | RAC1 | NM_006908, NM_018890 |
| 7 | DGKB | NM_004080, NM_145695 |
| 7 | STK31 | NM_001122833, NM_031414, NM_032944 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 7 | CDK13 | NM_003718, NM_031267 |
| 7 | STK17A | NM_004760 |
| 7 | GCK | NM_000162, NM_033507, NM_033508 |
| 7 | CAMK2B | NM_001220, NM_172078, NM_172079, NM_172080, NM_172081, NM_172082, NM_172083, NM_172084 |
| 7 | EGFR | NM_005228, NM_201282, NM_201283, NM_201284 |
| 7 | PHKG1 | NM_006213 |
| 7 | LIMK1 | NM_002314 |
| 7 | CDK14 | NM_012395 |
| 7 | CDK6 | NM_001145306, NM_001259 |
| 7 | PDK4 | NM_002612 |
| 7 | LMTK2 | NM_014916 |
| 7 | TRRAP | NM_003496 |
| 7 | EPHB4 | NM_004444 |
| 7 | SRPK2 | NM_182691, NM_182692 |
| 7 | PIK3CG | NM_002649 |
| 7 | MET | NM_000245, NM_001127500 |
| 7 | DGKI | NM_004717 |
| 7 | TRIM24 | NM_003852, NM_015905 |
| 7 | HIPK2 | NM_001113239, NM_022740 |
| 7 | BRAF | NM_004333 |
| 7 | AGK | NM_018238 |
| 7 | FLJ40852 | NM_001105558, NR_015392 |
| 7 | WEE2 | NM_001105558, NR_015392 |
| 7 | EPHB6 | NM_004445 |
| 7 | EPHA1 | NM_005232 |
| 7 | CDK5 | NM_001164410, NM_004935 |
| 7 | FASTK | NM_006712, NM_033015 |
| 7 | RHEB | NM_005614 |
| 8 | SGK223 | NM_001080826 |
| 8 | BLK | NM_001715 |
| 8 | PTK2B | NM_004103, NM_173174, NM_173175, NM_173176 |
| 8 | PBK | NM_018492 |
| 8 | FGFR1 | NM_001174063, NM_001174064, NM_001174065, NM_001174066, NM_001174067, NM_015850, NM_023105, NM_023106, NM_023110, NM_023107, NM_023108 |
| 8 | IKBKB | NM_001556 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 8 | SGK196 | NM_032237 |
| 8 | PRKDC | NM_001081640, NM_006904 |
| 8 | LYN | NM_001111097, NM_002350 |
| 8 | MOS | NM_005372 |
| 8 | SGK3 | NM_001033578, NM_013257, NM_170709 |
| 8 | PSKH2 | NM_033126 |
| 8 | RIPK2 | NM_003821 |
| 8 | STK3 | NM_006281 |
| 8 | PKHD1L1 | NM_177531 |
| 8 | TRIB1 | NM_025195 |
| 8 | MYC | NM_002467 |
| 8 | PTK2 | NM_005607, NM_153831 |
| 8 | MAPK15 | NM_139021 |
| 8 | NRBP2 | NM_178564 |
| 8 | ADCK5 | NM_174922, NM_013291 |
| 8 | CPSF1 | NM_174922, NM_013291 |
| 9 | JAK2 | NM_004972 |
| 9 | CDKN2A | NM_000077, NM_058195, NM_058197 |
| 9 | CDKN2BAS | NM_004936, NM_078487, NR_003529 |
| 9 | CDKN2B | NM_004936, NM_078487, NR_003529 |
| 9 | TEK | NM_000459 |
| 9 | TAF1L | NM_153809 |
| 9 | PTENP1 | NR_023917 |
| 9 | TESK1 | NM_006285, NM_001782 |
| 9 | CD72 | NM_006285, NM_001782 |
| 9 | NPR2 | NM_003995, NM_172312 |
| 9 | SPAG8 | NM_003995, NM_172312 |
| 9 | MELK | NM_014791 |
| 9 | PIP5K1B | NM_003558 |
| 9 | PRKACG | NM_002732 |
| 9 | TRPM6 | NM_001177310, NM_001177311, NM_017662 |
| 9 | NTRK2 | NM_001018064, NM_006180, NM_001007097, NM_001018065, NM_001018066 |
| 9 | DAPK1 | NM_004938 |
| 9 | CDK20 | NM_001039803, NM_001170639, NM_001170640, NM_012119, NM_178432 |
| 9 | SYK | NM_001135052, NM_003177, NM_001174168, NM_001174167 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 9 | ROR2 | NM_004560 |
| 9 | CENPP | NM_001012267, NM_022755 |
| 9 | IPPK | NM_001012267, NM_022755 |
| 9 | WNK2 | NM_006648, NM_001098808 |
| 9 | C9orf129 | NM_001098808, NM_006648 |
| 9 | TGFBR1 | NM_001130916, NM_004612 |
| 9 | MUSK | NM_001166280, NM_001166281, NM_005592 |
| 9 | NEK6 | NM_001166167, NM_001145001, NM_001166168, NM_001166170, NM_001166171, NM_014397, NM_001166169 |
| 9 | CDK9 | NM_001261 |
| 9 | PIP5KL1 | NM_001135219, NM_173492 |
| 9 | PKN3 | NM_013355, NM_032799 |
| 9 | ZDHHC12 | NM_013355, NM_032799 |
| 9 | ABL1 | NM_007313, NM_005157 |
| 9 | C9orf96 | NM_153710, NM_020385 |
| 9 | REXO4 | NM_153710, NM_020385 |
| 9 | NCRNA00094 | NM_007371, NR_015427 |
| 9 | BRD3 | NM_007371, NR_015427 |
| 10 | PRKCQ | NM_006257 |
| 10 | GATA3 | NM_001002295, NM_002051 |
| 10 | CAMK1D | NM_020397, NM_153498 |
| 10 | PIP4K2A | NM_005028 |
| 10 | MYO3A | NM_017433 |
| 10 | MASTL | NM_001172303, NM_001172304, NM_032844 |
| 10 | MAP3K8 | NM_005204 |
| 10 | RET | NM_020630, NM_020975 |
| 10 | FAM35B | NR_027632 |
| 10 | FAM35B2 | NR_027634 |
| 10 | MAPK8 | NM_002750, NM_139046, NM_139047, NM_139049 |
| 10 | PRKG1 | NM_001098512, NM_006258 |
| 10 | IPMK | NM_152230 |
| 10 | CDK1 | NM_001170406, NM_001170407, NM_001786, NM_033379 |
| 10 | CAMK2G | NM_001222, NM_172169, NM_172170, NM_172171, NM_172173 |
| 10 | BMPR1A | NM_004329 |
| 10 | PTEN | NM_000314 |
| 10 | PIPSL | NR_002319 |

(continued)

| Chromosome | GeneSymbol | refseq id |
|---|---|---|
| | | Table 2: Tumor suppressor genes or endogenous cancer-related genes that might cause somatic mutation (condition (1) (b)) |
| 10 | PI4K2A | NM_018425 |
| 10 | CHUK | NM_001278 |
| 10 | SLK | NM_014720 |
| 10 | GRK5 | NM_005308 |
| 10 | FGFR2 | NM_000141, NM_001144914, NM_001144915, NM_001144916, NM_001144917, NM_001144918, NM_022970, NM_001144913, NM_001144919 |
| 10 | STK32C | NM_173575 |
| 11 | HRAS | NM_001130442, NM_005343, NM_176795 |
| 11 | BRSK2 | NM_003957 |
| 11 | ILK | NM_001014794, NM_001014795, NM_004517, NM_006284 |
| 11 | TAF10 | NM_001014794, NM_001014795, NM_004517, NM_006284 |
| 11 | STK33 | NM_030906 |
| 11 | WEE1 | NM_003390, NM_001143976 |
| 11 | CSNK2A1P | NM_198516, NR_002207 |
| 11 | GALNTL4 | NM_198516, NR_002207 |
| 11 | PIK3C2A | NM_002645 |
| 11 | HIPK3 | NM_001048200, NM_005734 |
| 11 | DGKZ | NM_201532, NM_201533, NM_003646, NM_001105540 |
| 11 | MARK2 | NM_001039469, NM_001163296, NM_001163297, NM_004954, NM_017490 |
| 11 | RPS6KA4 | NM_001006944, NM_003942, NR_031602 |
| 11 | MIR1237 | NM_001006944, NM_003942, NR_031602 |
| 11 | MAP4K2 | NM_004579 |
| 11 | CDC42BPG | NM_017525 |
| 11 | SCYL1 | NM_001048218, NM_020680, NM_001130144, NM_001164266, NM_021070 |
| 11 | LTBP3 | NM_001048218, NM_020680, NM_001130144, NM_001164266, NM_021070 |
| 11 | MAP3K11 | NM_002419 |
| 11 | ADRBK1 | NM_001619 |
| 11 | RPS6KB2 | NM_003952 |
| 11 | CCND1 | NM_053056 |
| 11 | PAK1 | NM_001128620, NM_002576 |
| 11 | ATM | NM_000051, NM_138292 |
| 11 | SIK2 | NM_015191, NM_181699, NM_181700 |
| 11 | PPP2R1B | NM_015191, NM_181699, NM_181700 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 11 | ANKK1 | NM_178510 |
| 11 | USP28 | NM_020886 |
| 11 | SIK3 | NM_025164 |
| 11 | CHEK1 | NM_001114121, NM_001114122, NM_001274 |
| 12 | WNK1 | NM_001184985, NM_014823, NM_018979, NM_213655 |
| 12 | CCND2 | NM_001759 |
| 12 | DYRK4 | NM_003845 |
| 12 | STYK1 | NM_018423 |
| 12 | GUCY2C | NM_004963 |
| 12 | PIK3C2G | NM_004570 |
| 12 | KRAS | NM_004985, NM_033360 |
| 12 | STK38L | NM_015000 |
| 12 | LRRK2 | NM_198578 |
| 12 | YAF2 | NM_005748 |
| 12 | IRAK4 | NM_001114182, NM_001145256, NM_001145257, NM_001145258, NM_016123 |
| 12 | ACVRL1 | NM_000020, NM_001077401 |
| 12 | ACVR1B | NM_004302, NM_020327, NM_020328 |
| 12 | SP1 | NM_138473, NM_003109 |
| 12 | AMHR2 | NM_001164690, NM_001164691, NM_020547 |
| 12 | PCBP2 | NM_001098620, NM_001128911, NM_001128912, NM_001128913, NM_001128914, NM_005016, NM_006301, NM_031989 |
| 12 | MAP3K12 | NM_001098620, NM_001128911, NM_001128912, NM_001128913, NM_001128914, NM_005016, NM_006301, NM_031989 |
| 12 | DGKA | NM_001345, NM_201444, NM_201445, NM_201554 |
| 12 | CDK2 | NM_001798, NM_052827 |
| 12 | ERBB3 | NM_001005915, NM_001982 |
| 12 | PIP4K2C | NM_001146258, NM_001146259, NM_001146260, NM_024779 |
| 12 | TSPAN31 | NM_000075, NM_005981 |
| 12 | CDK4 | NM_000075, NM_005981 |
| 12 | TBK1 | NM_013254 |
| 12 | IRAK3 | NM_001142523, NM_007199 |
| 12 | DYRK2 | NM_003583, NM_006482 |
| 12 | CDK17 | NM_002595, NM_001170464 |
| 12 | SCYL2 | NM_017988 |
| 12 | NUAK1 | NM_014840 |
| 12 | C12orf47 | NM_003668, NM_139078, NR_015404 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 12 | MAPKAPK5 | NM_003668, NM_139078, NR_015404 |
| 12 | KSR2 | NM_173598 |
| 12 | TAOK3 | NM_016281 |
| 12 | HSPB8 | NM_014365 |
| 12 | CIT | NM_007174, NR_031589 |
| 12 | MIR1178 | NM_007174, NR_031589 |
| 12 | CAMKK2 | NM_006549, NM_153499, NM_153500, NM_172216, NM_172226, NM_172214, NM_172215 |
| 12 | ULK1 | NM_003565 |
| 13 | LATS2 | NM_014572 |
| 13 | CDK8 | NM_001260 |
| 13 | FLT3 | NM_004119 |
| 13 | FLT1 | NM_002019, NM_001160030, NM_001159920, NM_001160031 |
| 13 | BRCA2 | NM_000059 |
| 13 | MIR548F5 | NM_004734, NR_031646 |
| 13 | DCLK1 | NM_004734, NR_031646 |
| 13 | CSNK1A1L | NM_145203 |
| 13 | DGKH | NM_152910, NM_178009 |
| 13 | RB1 | NM_000321 |
| 13 | NEK5 | NM_199289 |
| 13 | NEK3 | NM_001146099, NM_002498, NM_152720, NR_027415 |
| 13 | STK24 | NM_001032296, NM_003576 |
| 13 | IRS2 | NM_003749 |
| 13 | GRK1 | NM_002929 |
| 14 | TSSK4 | NM_001184739, NM_174944 |
| 14 | RIPK3 | NM_006871 |
| 14 | PRKD1 | NM_002742 |
| 14 | NFKBIA | NM_020529 |
| 14 | CDKL1 | NM_004196 |
| 14 | MAP4K5 | NM_006575, NM_198794 |
| 14 | PRKCH | NM_006255 |
| 14 | ESR2 | NM_001040275, NM_001040276, NM_001437 |
| 14 | MAP3K9 | NM_033141 |
| 14 | RPS6KL1 | NM_031464 |
| 14 | NEK9 | NM_033116 |
| 14 | ADCK1 | NM_001142545, NM_020421 |

(continued)

| Chromosome | GeneSymbol | refseq id |
|---|---|---|
| | Table 2: Tumor suppressor genes or endogenous cancer-related genes that might cause somatic mutation (condition (1) (b)) | | |
| 14 | RPS6KA5 | NM_004755, NM_182398 |
| 14 | ITPK1 | NM_001142594, NM_001142593, NM_014216 |
| 14 | VRK1 | NM_003384 |
| 14 | RAGE | NM_014226 |
| 14 | CDC42BPB | NM_006035 |
| 14 | MARK3 | NM_001128918, NM_001128919, NM_001128920, NM_001128921, NM_002376 |
| 14 | AKT1 | NM_001014431, NM_001014432, NM_005163 |
| 15 | NF1P1 | NR_028506 |
| 15 | LOC646214 | NR_027053 |
| 15 | FAM7A3 | NR_026859, NR_026858, NR_027470 |
| 15 | FAM7A | NM_139320, NM_148911 |
| 15 | FAM7A2 | NR_026858, NR_027470, NR_026859 |
| 15 | FAM7A1 | NR_026858, NR_027470, NR_026859 |
| 15 | EIF2AK4 | NM_001013703 |
| 15 | BUB1B | NM_001211, NM_001128628, NM_001128629 |
| 15 | PAK6 | NM_001128628, NM_001128629, NM_001211, NM_020168 |
| 15 | ITPKA | NM_002220 |
| 15 | LTK | NM_001135685, NM_002344, NM_206961 |
| 15 | TYRO3 | NM_006293 |
| 15 | TTBK2 | NM_173500 |
| 15 | TRPM7 | NM_017672 |
| 15 | MAPK6 | NM_002748 |
| 15 | DAPK2 | NM_014326 |
| 15 | CSNK1G1 | NM_022048 |
| 15 | MAP2K1 | NM_002755, NM_006049 |
| 15 | SNAPC5 | NM_002755, NM_006049 |
| 15 | MAP2K5 | NM_002757, NM_145160 |
| 15 | CLK3 | NM_003992, NM_001130028 |
| 15 | CSK | NM_001127190, NM_004383 |
| 15 | ULK3 | NM_001099436 |
| 15 | PTPN9 | NM_002833 |
| 15 | ETFA | NM_000126, NM_001127716 |
| 15 | SGK269 | NM_024776 |
| 15 | ALPK3 | NM_020778 |
| 15 | NTRK3 | NM_001012338, NM_002530, NM_001007156 |

(continued)

| Chromosome | GeneSymbol | refseq id |
|---|---|---|
| | Table 2: Tumor suppressor genes or endogenous cancer-related genes that might cause somatic mutation (condition (1) (b)) | |
| 15 | IDH2 | NM_002168 |
| 15 | FES | NM_001143785, NM_002005, NM_001143783, NM_001143784 |
| 15 | IGF1R | NM_000875 |
| 15 | LRRK1 | NM_024652 |
| 16 | PDPK1 | NM_002613, NM_031268 |
| 16 | LOC652276 | NR_015441 |
| 16 | FLJ42627 | NR_024492 |
| 16 | PAQR4 | NM_004203, NM_152341, NM_182687 |
| 16 | PKMYT1 | NM_004203, NM_152341, NM_182687 |
| 16 | SMG1 | NM_015092 |
| 16 | LOC100271836 | NR_027155 |
| 16 | EEF2K | NM_013302 |
| 16 | LOC641298 | NR_027154 |
| 16 | PALB2 | NM_024675 |
| 16 | PLK1 | NM_005030, NM_033266 |
| 16 | ERN2 | NM_005030, NM_033266 |
| 16 | PRKCB | NM_002738, NM_212535 |
| 16 | SBK1 | NM_001024401 |
| 16 | LOC440354 | NR_002473, NR_002453 |
| 16 | TAOK2 | NM_004783, NM_016151 |
| 16 | LOC100271831 | NM_001040056, NM_001109891, NM_002746, NR_027081 |
| 16 | MAPK3 | NM_001040056, NM_001109891, NM_002746, NR_027081 |
| 16 | LOC595101 | NR_002453, NR_002473 |
| 16 | PHKG2 | NM_000294, NM_001172432 |
| 16 | BCKDK | NM_001122957, NM_005881 |
| 16 | MYLK3 | NM_182493 |
| 16 | CSNK2A2 | NM_001896 |
| 16 | PSKH1 | NM_006742, NM_001907 |
| 16 | CTRL | NM_006742, NM_001907 |
| 16 | CDH1 | NM_004360 |
| 16 | MLKL | NM_001142497, NM_152649 |
| 16 | CDK10 | NM_001098533, NM_001160367, NM_052987, NM_052988, NR_027702, NR_027703, NM_152339 |
| 16 | SPATA2L | NM_001098533, NM_001160367, NM_052987, NM_052988, NR_027702, NR_027703, NM_152339 |
| 17 | ITGAE | NM_002208, NM_031965 |
| 17 | GSG2 | NM_002208, NM_031965 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 17 | CAMKK1 | NM_032294, NM_172206, NM_172207 |
| 17 | ANKFY1 | NM_016376, NM_020740 |
| 17 | MINK1 | NM_001024937, NM_015716, NM_153827, NM_170663, NM_000080 |
| 17 | NE | NM_001024937, NM_015716, NM_153827, NM_170663, NM_000080 |
| 17 | TNK1 | NM_003985, NM_020360 |
| 17 | PLSCR3 | NM_003985, NM_020360 |
| 17 | TP53 | NM_000546, NM_001126112, NM_001126113, NM_001126114, NM_001126115, NM_001126116, NM_001126117, NM_001143990, NM_001143991 |
| 17 | WRAP53 | NM_000546, NM_001126112, NM_001126113, NM_001126114, NM_001143990, NM_001143991 |
| 17 | CHD3 | NM_001005271, NM_001005273, NM_005852 |
| 17 | GUCY2D | NM_000180 |
| 17 | AURKB | NM_004217 |
| 17 | PIK3R6 | NM_001010855 |
| 17 | PIK3R5 | NM_001142633, NM_014308 |
| 17 | MAP2K4 | NM_003010 |
| 17 | MAPK7 | NM_139032, NM_139033, NM_002749, NM_139034 |
| 17 | ULK2 | NM_001142610, NM_014683 |
| 17 | MAP2K3 | NM_145109, NM_002756 |
| 17 | KSR1 | NM_014238 |
| 17 | NLK | NM_016231 |
| 17 | SGK494 | NM_001174103 |
| 17 | NEK8 | NM_178170 |
| 17 | TAOK1 | NM_020791 |
| 17 | NF1 | NM_000267, NM_001042492, NM_001128147 |
| 17 | MYO1D | NM_015194 |
| 17 | ACCN1 | NM_001094 |
| 17 | PIP4K2B | NM_003559 |
| 17 | CDK12 | NM_015083, NM_016507 |
| 17 | ERBB2 | NM_001005862, NM_004448 |
| 17 | CDC6 | NM_001254 |
| 17 | WNK4 | NM_032387 |
| 17 | BRCA1 | NM_007294, NM_007297, NM_007298, NM_007299, NM_007300, NR_027676 |
| 17 | C17orf65 | NM_178542 |
| 17 | LOC100133991 | NM_003954, NR_024434, NR_024435 |

(continued)

| Chromosome | GeneSymbol | refseq id |
|:---:|:---:|:---|
| Table 2: Tumor suppressor genes or endogenous cancer-related genes that might cause somatic mutation (condition (1) (b)) | | |
| 17 | MAP3K14 | NM_003954, NR_024434, NR_024435 |
| 17 | PDK2 | NM_002611 |
| 17 | COL1A1 | NM_000088 |
| 17 | ANKFN1 | NM_153228 |
| 17 | DGKE | NM_003647 |
| 17 | TEX14 | NM_031272, NM_198393 |
| 17 | RPS6KB1 | NM_003161 |
| 17 | TLK2 | NM_001112707, NM_006852 |
| 17 | MAP3K3 | NM_002401, NM_203351, NM_030576 |
| 17 | LIMD2 | NM_002401, NM_203351, NM_030576 |
| 17 | STRADA | NM_001003786, NM_001003787, NM_001003788, NM_001165969, NM_001165970, NM_153335 |
| 17 | ERN1 | NM_001433 |
| 17 | PRKCA | NM_002737 |
| 17 | MAP2K6 | NM_002758 |
| 17 | CDK3 | NM_001258 |
| 17 | SPHK1 | NM_001142601, NM_021972, NM_182965, NM_001142602 |
| 17 | BAIAP2 | NM_001080395, NM_001144888, NM_006340, NM_017451 |
| 17 | AATK | NM_001080395, NM_001144888, NM_006340, NM_017451 |
| 17 | CSNK1D | NM_001893, NM_139062 |
| 18 | YES1 | NM_005433 |
| 18 | ROCK1 | NM_005406 |
| 18 | RIOK3 | NM_003831 |
| 18 | PIK3C3 | NM_002647 |
| 18 | MAPK4 | NM_002747 |
| 18 | ALPK2 | NM_052947 |
| 18 | KIAA1468 | NM_020854 |
| 19 | STK11 | NM_000455 |
| 19 | CSNK1G2 | NM_001319 |
| 19 | MKNK2 | NM_017572, NM_199054 |
| 19 | PIP5K1C | NM_012398 |
| 19 | MATK | NM_002378, NM_139354, NM_139355 |
| 19 | DAPK3 | NM_001348 |
| 19 | MAP2K2 | NM_030662 |
| 19 | INSR | NM_000208, NM_001079817 |
| 19 | MAP2K7 | NM_145185 |

(continued)

| Table 2: Tumor suppressor genes or endogenous cancer-related genes that might cause somatic mutation (condition (1) (b)) | | |
|---|---|---|
| Chromosome | GeneSymbol | refseq id |
| 19 | TYK2 | NM_003331 |
| 19 | MAST1 | NM_014975 |
| 19 | PRKACA | NM_002730, NM_207518 |
| 19 | PKN1 | NM_002741, NM_213560 |
| 19 | BRD4 | NM_058243, NM_014299 |
| 19 | JAK3 | NM_000215 |
| 19 | MAST3 | NM_015016 |
| 19 | PIK3R2 | NM_005027 |
| 19 | TSSK6 | NM_032037 |
| 19 | LOC284441 | NR_003128 |
| 19 | CCNE1 | NM_001238, NM_057182 |
| 19 | MAP4K1 | NM_001042600, NM_007181 |
| 19 | PAK4 | NM_001014831, NM_001014832, NM_001014834, NM_001014835, NM_005884 |
| 19 | DYRK1B | NM_004714, NM_006483, NM_006484 |
| 19 | MAP3K10 | NM_002446 |
| 19 | AKT2 | NM_001626 |
| 19 | HIPK4 | NM_144685 |
| 19 | ADCK4 | NM_001142555, NM_024876 |
| 19 | ITPKC | NM_025194, NM_198476 |
| 19 | C19orf54 | NM_025194, NM_198476 |
| 19 | AXL | NM_001699, NM_021913 |
| 19 | GSK3A | NM_019884 |
| 19 | MARK4 | NM_031417 |
| 19 | DMPK | NM_001081560, NM_001081562, NM_001081563, NM_004409 |
| 19 | PRKD2 | NM_001079880, NM_001079881, NM_001079882, NM_016457 |
| 19 | LMTK3 | NM_001080434 |
| 19 | SPHK2 | NM_020126 |
| 19 | VRK3 | NM_001025778, NM_016440 |
| 19 | PRKCG | NM_002739 |
| 19 | BRSK1 | NM_032430 |
| 19 | SBK2 | NM_001101401 |
| 19 | AURKC | NM_001015878, NM_001015879, NM_003160 |
| 19 | TRIM28 | NM_005762 |
| 20 | TRIB3 | NM_021158 |
| 20 | CSNK2A1 | NM_001895, NM_177559, NM_177560 |

(continued)

| Chromosome | GeneSymbol | refseq id |
|---|---|---|
| | Table 2: Tumor suppressor genes or endogenous cancer-related genes that might cause somatic mutation (condition (1) (b)) |||
| 20 | STK35 | NM_080836 |
| 20 | PAK7 | NM_020341, NM_177990 |
| 20 | MYLK2 | NM_033118 |
| 20 | HCK | NM_001172129, NM_001172130, NM_001172131, NM_001172132, NM_001172133, NM_002110 |
| 20 | RALY | NM_007367, NM_016732 |
| 20 | SRC | NM_005417, NM_198291 |
| 20 | SGK2 | NM_170693, NM_016276 |
| 20 | STK4 | NM_006282 |
| 20 | TP53RK | NM_033550 |
| 20 | AURKA | NM_003600, NM_198433, NM_198434, NM_198435, NM_198436, NM_198437 |
| 20 | PTK6 | NM_005975 |
| 20 | SRMS | NM_080823 |
| 21 | HUNK | NM_014586 |
| 21 | DYRK1A | NM_101395, NM_130436, NM_001396, NM_130438 |
| 21 | RIPK4 | NM_020639 |
| 21 | SIK1 | NM_173354 |
| 22 | TSSK2 | NM_022719, NM_053006 |
| 22 | DGCR14 | NM_022719, NM_053006 |
| 22 | PI4KAP1 | NR_003563 |
| 22 | PI4KA | NM_002650, NM_058004 |
| 22 | PI4KAP2 | NR_003700 |
| 22 | MAPK1 | NM_002745, NM_138957 |
| 22 | ADRBK2 | NM_005160 |
| 22 | CHEK2 | NM_001005735, NM_007194, NM_145862 |
| 22 | NF2 | NM_000268, NM_016418, NM_181825, NM_181828, NM_181829, NM_181830, NM_181831, NM_181832, NM_181833 |
| 22 | LIMK2 | NM_005569, NM_001031801, NM_016733 |
| 22 | CSNK1E | NM_001894, NM_152221 |
| 22 | CERK | NM_022766 |
| 22 | PIM3 | NM_001001852 |
| 22 | MAPK12 | NM_002969 |
| 22 | MAPK11 | NM_002751 |
| x | PRKX | NM_005044 |
| x | BMX | NM_203281, NM_001721 |
| x | CDKL5 | NM_003159, NM_001037343, NM_000330 |

(continued)

| | Table 2: Tumor suppressor genes or endogenous cancer-related genes that might cause somatic mutation (condition (1) (b)) | |
|---|---|---|
| Chromosome | GeneSymbol | refseq id |
| x | RS1 | NM_000330, NM_001037343, NM_003159 |
| x | PDHA1 | NM_000284, NM_001001671, NM_001173454, NM_001173455, NM_001173456 |
| x | MAP3K15 | NM_000284, NM_001001671, NM_001173454, NM_001173455, NM_001173456 |
| x | RPS6KA3 | NM_004586 |
| x | CNKSR2 | NM_001168647, NM_001168648, NM_001168649, NM_014927 |
| x | PDK3 | NM_001142386, NM_005391 |
| x | CASK | NM_001126054, NM_001126055, NM_003688 |
| x | CDK16 | NM_033018, NM_006201, NM_001170460 |
| x | ARAF | NM_001654, NM_006950, NM_133499 |
| x | SYN1 | NM_001654, NM_006950, NM_133499 |
| x | PIM2 | NM_006875 |
| x | WNK3 | NM_001002838, NM_020922 |
| x | TAF1 | NM_004606, NM_138923, NR_001568 |
| x | BCYRN1 | NM_004606, NM_138923, NR_001568 |
| x | PHKA1 | NM_001122670, NM_001172436, NM_002637 |
| x | LOC139201 | NR_029423 |
| x | NCRNA00182 | NR_028379 |
| x | RPS6KA6 | NM_014496 |
| x | KLHL4 | NM_019117, NM_057162 |
| x | BTK | NM_000061 |
| x | NRK | NM_198465 |
| x | IRS4 | NM_003604 |
| x | GUCY2F | NM_001522 |
| x | PAK3 | NM_001128166, NM_001128167, NM_002578, NM_001128168, NM_001128172, NM_001128173 |
| x | MST4 | NM_001042453, NM_016542, NM_001042452 |
| x | PNCK | NM_001039582, NM_001135740 |
| x | SRPK3 | NM_001170760, NM_001170761, NM_014370, NM_004135, NM_174869 |
| x | IDH3G | NM_001170760, NM_001170761, NM_014370, NM_004135, NM_174869 |
| x | IRAK1 | NM_001025242, NM_001025243, NM_001569 |
| Y | PRKY | NR_028062 |

or they may preferably be depicted in at least one amino acid mutation (mutation ID) in the proteins listed in the following table:

[Table 3]

Table 3: Tumor suppressor genes or endogenous cancer-related genes that might cause somatic mutation (condition (1) (b))

| Chromosome | Gene Symbol | Mut_ID | | | | |
|---|---|---|---|---|---|---|
| 1 | AKT3 | E17K | 3 | CTNNB1 | G34V | |
| 1 | EPHA10 | E124K | 3 | CTNNB1 | S33/F/Y/C | |
| 1 | KRAS | Q61L/Q61R/Q61P | 3 | CTNNB1 | S33C | |
| 1 | NRAS | A18T | 3 | CTNNB1 | S33F | |
| 1 | NRAS | A59T | 3 | CTNNB1 | S33P | |
| 1 | NRAS | G12 | 3 | CTNNB1 | S33Y | |
| 1 | NRAS | G12C/G12R/G12S | 3 | CTNNB1 | S37A | |
| 1 | NRAS | G12V/G12A/G12D | 3 | CTNNB1 | S37A | |
| 1 | NRAS | G13 | 3 | CTNNB1 | S37C | |
| 1 | NRAS | G13C/G13R/G13S | 3 | CTNNB1 | S37C/F/Y | |
| 1 | NRAS | G13V/G13A/G13D | 3 | CTNNB1 | S37F | |
| 1 | NRAS | G48S | 3 | CTNNB1 | S37P | |
| 1 | NRAS | Q61 | 3 | CTNNB1 | S37Y | |
| 1 | NRAS | Q61 | 3 | CTNNB1 | S45 | |
| 1 | NRAS | Q61 | 3 | CTNNB1 | S45A | |
| 1 | NRAS | Q61E/Q61K | 3 | CTNNB1 | S45C | |
| 1 | NRAS | Q61H | 3 | CTNNB1 | S45C/F/Y | |
| 1 | NRAS | Q61L/Q61R/Q61P | 3 | CTNNB1 | S45F | |
| 2 | CXCR4 | V160I | 3 | CTNNB1 | S45P | |
| 2 | ERBB4 | E452K | 3 | CTNNB1 | S45P | |
| 2 | ERBB4 | R393W | 3 | CTNNB1 | S45Y | |
| 2 | SOS1 | H888Q | 3 | CTNNB1 | T41A | |
| 2 | SOS1 | R248H | 3 | CTNNB1 | T41A/S | |
| 2 | SOS1 | R688Q | 3 | CTNNB1 | T41I | |
| 3 | CTNNB1 | A13T | 3 | CTNNB1 | T41I | |
| 3 | CTNNB1 | A21T | 3 | CTNNB1 | T41I | |
| 3 | CTNNB1 | D32A | 3 | CTNNB1 | T41P | |
| 3 | CTNNB1 | D32G | 3 | CTNNB1 | T41S | |
| 3 | CTNNB1 | D32H/N/Y | 3 | CTNNB1 | V22_G38del | |
| 3 | CTNNB1 | D32V | 3 | CTNNB1 | V22A | |
| 3 | CTNNB1 | G34E | 3 | CTNNB1 | W25_D32del | |
| 3 | CTNNB1 | G34E/V | 3 | MLH1 | V384D | |
| 3 | CTNNB1 | G34R | 3 | NEK10 | E379K | |
| 3 | CTNNB1 | G34R | 3 | PIK3CA | A1035T | |
| | | | 3 | PIK3CA | A1035V | |
| | | | 3 | PIK3CA | C420R | |

| 3 | PIK3CA | C901F | 4 | KIT | D52N |
|---|--------|-------|---|-----|------|
| 3 | PIK3CA | E418K | 4 | KIT | D579del |
| 3 | PIK3CA | E542K | 4 | KIT | D716N |
| 3 | PIK3CA | E542Q/K | 4 | KIT | D816E |
| 3 | PIK3CA | E542V | 4 | KIT | D816F |
| 3 | PIK3CA | E545A | 4 | KIT | D816H/D816Y |
| 3 | PIK3CA | E545G | 4 | KIT | D816V |
| 3 | PIK3CA | E545G/A | 4 | KIT | D816V/G/A |
| 3 | PIK3CA | E545K | 4 | KIT | D820E |
| 3 | PIK3CA | E545Q/K | 4 | KIT | D820G/A |
| 3 | PIK3CA | G1007R | 4 | KIT | D820H/Y |
| 3 | PIK3CA | H1047R/H1047L | 4 | KIT | D820Y |
| 3 | PIK3CA | H1047Y | 4 | KIT | E561K |
| 3 | PIK3CA | H1065L | 4 | KIT | E839K |
| 3 | PIK3CA | H701P | 4 | KIT | F584S |
| 3 | PIK3CA | I1058F | 4 | KIT | G565R |
| 3 | PIK3CA | M1004I | 4 | KIT | K492R |
| 3 | PIK3CA | M1043I/M1043I | 4 | KIT | K550_K558del |
| 3 | PIK3CA | M1043V | 4 | KIT | K558_E562del |
| 3 | PIK3CA | N1044K | 4 | KIT | K558_V560del |
| 3 | PIK3CA | N1068fs*4 | 4 | KIT | K558N |
| 3 | PIK3CA | N345K | 4 | KIT | K558R |
| 3 | PIK3CA | P539R | 4 | KIT | K642E |
| 3 | PIK3CA | Q546E/K | 4 | KIT | K642E |
| 3 | PIK3CA | Q546H | 4 | KIT | K685E |
| 3 | PIK3CA | Q546K | 4 | KIT | L576P |
| 3 | PIK3CA | Q546R/P | 4 | KIT | L576P |
| 3 | PIK3CA | R1023Q | 4 | KIT | M535I |
| 3 | PIK3CA | R38H | 4 | KIT | M535T |
| 3 | PIK3CA | R88Q | 4 | KIT | M535V |
| 3 | PIK3CA | R88Q | 4 | KIT | M552L |
| 3 | PIK3CA | S326F | 4 | KIT | N566D |
| 3 | PIK3CA | T1025A | 4 | KIT | N655K |
| 3 | PIK3CA | T1025S/I | 4 | KIT | N822H/Y |
| 3 | PIK3CA | Y1021C | 4 | KIT | N822K |
| 3 | PIK3CA | Y1021C | 4 | KIT | N822K |
| 3 | PIK3CA | Y1021H | 4 | KIT | P551_V555del |
| 3 | VHl | F148fs*11 | 4 | KIT | P551_V555del |
| 3 | VHl | L158Q | 4 | KIT | P573A |
| 3 | VHl | L85P | 4 | KIT | P573L |
| 3 | VHl | L89H | 4 | KIT | P585P |
| 3 | VHl | P81S | 4 | KIT | R634W |
| 3 | VHl | R161* | 4 | KIT | R739G |
| 3 | VHl | R167W | 4 | KIT | S709F |
| 4 | FBXW7 | R465C | 4 | KIT | T574A |
| 4 | FBXW7 | R465H | 4 | KIT | T670E |
| 4 | FBXW7 | R479G | 4 | KIT | T670I |
| 4 | FBXW7 | R479Q/L | 4 | KIT | T670I |
| 4 | FGFR3 | A281V | 4 | KIT | T753A |
| 4 | FGFR3 | A391E | 4 | KIT | V559_V560del |
| 4 | FGFR3 | G370C | 4 | KIT | V559A |
| 4 | FGFR3 | K650Q/K650E | 4 | KIT | V559D/V559A/V559G |
| 4 | FGFR3 | K650T/K650M | 4 | KIT | V559del |
| 4 | FGFR3 | Y373C | 4 | KIT | V559I |
| 4 | KIT | A829P | 4 | KIT | V560D/V560G |
| 4 | KIT | C809G | 4 | KIT | V560del |

| 4 | KIT | V560E | 5 | CSF1R | L301S |
|---|---|---|---|---|---|
| 4 | KIT | V569G | 5 | CSF1R | Y969* |
| 4 | KIT | V654A | 5 | CSF1R | Y969C |
| 4 | KIT | V654A | 5 | CSF1R | Y969F |
| 4 | KIT | V825A | 5 | CSF1R | Y969H |
| 4 | KIT | W557R | 5 | FBX4 | G30N |
| 4 | KIT | W557R/W557R/W557G | 5 | FBX4 | L23Q |
| 4 | KIT | Y503_F504insAY | 5 | FBX4 | P76T |
| 4 | KIT | Y553_Q556del | 5 | FBX4 | S12L |
| 4 | KIT | Y553K | 5 | FBX4 | S8R |
| 4 | KIT | Y553N | 5 | FBX4 | S8R |
| 4 | KIT | Y568D | 5 | MEK | P124L |
| 4 | KIT | Y570_L576del | 5 | MEK | Q56P |
| 4 | KIT | Y675C | 5 | MET | R1170Q |
| 4 | KIT | Y823D | 5 | MET | T992I |
| 4 | PDGFRA | D1071N | 7 | BRAF | D587A |
| 4 | PDGFRA | D842_D846>E | 7 | BRAF | D587E |
| 4 | PDGFRA | D842_D846>G | 7 | BRAF | D594E |
| 4 | PDGFRA | D842_D846>N | 7 | BRAF | D594V/D594G |
| 4 | PDGFRA | D842_H845del | 7 | BRAF | E586K |
| 4 | PDGFRA | D842_M844del | 7 | BRAF | E586K |
| 4 | PDGFRA | D842_S847>EA | 7 | BRAF | F468C |
| 4 | PDGFRA | D842F | 7 | BRAF | F595L |
| 4 | PDGFRA | D842I | 7 | BRAF | F595S |
| 4 | PDGFRA | D842V | 7 | BRAF | G464R |
| 4 | PDGFRA | D842V | 7 | BRAF | G464V/G464E |
| 4 | PDGFRA | D842Y | 7 | BRAF | G466 |
| 4 | PDGFRA | D842Y | 7 | BRAF | G466R |
| 4 | PDGFRA | D846Y | 7 | BRAF | G466V |
| 4 | PDGFRA | E996K | 7 | BRAF | G469 |
| 4 | PDGFRA | F808L | 7 | BRAF | G469 |
| 4 | PDGFRA | H845_N848>P | 7 | BRAF | G469 |
| 4 | PDGFRA | I843_D846del | 7 | BRAF | G469A |
| 4 | PDGFRA | I843_S847>T | 7 | BRAF | G469S/G469E/G469A |
| 4 | PDGFRA | N659K | 7 | BRAF | G469S/G469E/G469A |
| 4 | PDGFRA | N870S | 7 | BRAF | G469S/G469E/G469A |
| 4 | PDGFRA | R841_D842del | 7 | BRAF | G469V/G469R |
| 4 | PDGFRA | S566_E571>K | 7 | BRAF | G469V/G469R |
| 4 | PDGFRA | S566_E571>R | 7 | BRAF | G469V/G469R |
| 4 | PDGFRA | S566_E571>R | 7 | BRAF | G596R |
| 4 | PDGFRA | T674I | 7 | BRAF | G615E |
| 4 | PDGFRA | V561D | 7 | BRAF | I463S |
| 4 | PDGFRA | Y849C | 7 | BRAF | I592M |
| 5 | APC | APC_E1379* | 7 | BRAF | I592V |
| 5 | APC | APC_Q1338* | 7 | BRAF | K601del |
| 5 | APC | E1306* | 7 | BRAF | K601E |
| 5 | APC | E1309fs*4 | 7 | BRAF | K601E |
| 5 | APC | Q1367* | 7 | BRAF | K601N |
| 5 | APC | Q1378* | 7 | BRAF | L597 |
| 5 | APC | Q1429* | 7 | BRAF | L597 |
| 5 | APC | R1114* | 7 | BRAF | L597Q/L597V |
| 5 | APC | R1450* | 7 | BRAF | L597Q/L597V |
| 5 | APC | R876* | 7 | BRAF | L597S/L597R |
| 5 | APC | S1465fs*3 | 7 | BRAF | L597S/L597R |
| 5 | APC | T1661fs*9 | 7 | BRAF | N581S |
| 5 | CSF1R | L301* | 7 | BRAF | R443T |

| 7 | BRAF | R444Q | | 7 | EGFR | K745R |
|---|------|-------|---|---|------|-------|
| 7 | BRAF | R444W | | 7 | EGFR | L730F |
| 7 | BRAF | R444W | | 7 | EGFR | L747_E749del,A750P |
| 7 | BRAF | R462I | | 7 | EGFR | L747_E749del,A750P |
| 7 | BRAF | S605F | | 7 | EGFR | L747_P753>Q |
| 7 | BRAF | S605N | | 7 | EGFR | L747_P753>S |
| 7 | BRAF | T599_V600insTT | | 7 | EGFR | L747_R748>FP |
| 7 | BRAF | T599I | | 7 | EGFR | L747_S752del, P753S |
| 7 | BRAF | V471F | | 7 | EGFR | L747_S752del,Q ins |
| 7 | BRAF | V600 | | 7 | EGFR | L747_S752del,Q ins |
| 7 | BRAF | V600 | | 7 | EGFR | L747_T750del, P ins |
| 7 | BRAF | V600A | | 7 | EGFR | L747_T750del,P ins |
| 7 | BRAF | V600D | | 7 | EGFR | L747_T751>P |
| 7 | BRAF | V600D | | 7 | EGFR | L747_T751>P |
| 7 | BRAF | V600E/V600K | | 7 | EGFR | L747_T751>S |
| 7 | BRAF | V600E/V600K | | 7 | EGFR | L747_T751del |
| 7 | BRAF | V600M | | 7 | EGFR | L747_T751del |
| 7 | BRAF | V600R/V600L | | 7 | EGFR | L858M |
| 7 | BRAF | V600R/V600L | | 7 | EGFR | L858R |
| 7 | EGFR | A289V | | 7 | EGFR | L858R |
| 7 | EGFR | A750P | | 7 | EGFR | L861Q |
| 7 | EGFR | D761N | | 7 | EGFR | M766_A767insAI |
| 7 | EGFR | D761Y | | 7 | EGFR | N771_P772>SVDNR |
| 7 | EGFR | D770_N771>AGG | | 7 | EGFR | N771_P772>SVDNR |
| 7 | EGFR | D770_N771>AGG | | 7 | EGFR | P733L |
| 7 | EGFR | D770_N771insG | | 7 | EGFR | P753S |
| 7 | EGFR | D770_N771insG | | 7 | EGFR | P772_H773insV |
| 7 | EGFR | E709A/E709G/E709V | | 7 | EGFR | R108K |
| 7 | EGFR | E709K/E709H | | 7 | EGFR | S752_I759del |
| 7 | EGFR | E734K | | 7 | EGFR | S752_I759del |
| 7 | EGFR | E746_A750del | | 7 | EGFR | S752_I759del |
| 7 | EGFR | E746_A750del | | 7 | EGFR | S752Y |
| 7 | EGFR | E746_A750del, V ins | | 7 | EGFR | S768I |
| 7 | EGFR | E746_A750del, V ins | | 7 | EGFR | SNP C2255T |
| 7 | EGFR | E746_A750del,T751A | | 7 | EGFR | T263P |
| 7 | EGFR | E746_S752>A | | 7 | EGFR | T751A |
| 7 | EGFR | E746_S752>D | | 7 | EGFR | T790M |
| 7 | EGFR | E746_T751>A | | 7 | EGFR | T790M |
| 7 | EGFR | E746_T751del | | 7 | EGFR | V742A |
| 7 | EGFR | E746_T751del, I ins | | 7 | EGFR | V769_D770insASV |
| 7 | EGFR | E746_T751del,I ins | | 7 | EGFR | V769_D770insASV |
| 7 | EGFR | E746_T751del,S752D | | 7 | EGFR | V769_D770insASV |
| 7 | EGFR | E746_T751del,V ins | | 7 | EGFR | V769_D770insASV |
| 7 | EGFR | E746K | | 7 | EGFR | V769_D770insCV |
| 7 | EGFR | G598V | | 7 | EGFR | V774_C775insHV |
| 7 | EGFR | G719A | | 7 | EGFR | W731* |
| 7 | EGFR | G719D | | 7 | EPHB6 | G404S |
| 7 | EGFR | G719S/G719C | | 7 | EPHB6 | R679Q |
| 7 | EGFR | G735S | | 7 | MAP2K2 | F57C |
| 7 | EGFR | G810D | | 7 | MAP2K2 | F57I |
| 7 | EGFR | G810S | | 7 | MAP2K2 | F57L |
| 7 | EGFR | H773_V774insH | | 7 | MAP2K2 | K61E |
| 7 | EGFR | H773_V774insNPH | | 7 | MAP2K2 | R388Q |
| 7 | EGFR | H773_V774insPH | | 7 | MET | H1112R |
| 7 | EGFR | H773>NPY | | 7 | MET | H1112Y |
| 7 | EGFR | H773R | | 7 | MET | M1250T |

| | | | | | | |
|---|---|---|---|---|---|---|
| 7 | MET | M1268T | | 10 | PTEN | R130Q |
| 7 | MET | R970C | | 10 | PTEN | R173C |
| 7 | MET | T1010I | | 10 | PTEN | R173H |
| 7 | MET | T992I | | 10 | PTEN | R233* |
| 7 | MET | Y1230C | | 10 | PTEN | R335* |
| 7 | MET | Y1235D | | 10 | PTEN | V317fs*3 |
| 7 | MET | Y1248C | | 10 | RET | A664D |
| 7 | MET | Y1248H | | 10 | RET | A883F |
| 8 | FGFR1 | P252T | | 10 | RET | C634R |
| 8 | FGFR1 | S125L | | 10 | RET | C634R |
| 8 | MYC | A59V | | 10 | RET | C634W |
| 8 | MYC | N101T | | 10 | RET | C634W |
| 8 | MYC | P260A | | 10 | RET | C634Y |
| 8 | MYC | P57S | | 10 | RET | C634Y |
| 8 | MYC | S77F | | 10 | RET | D631_L633>E |
| 8 | MYC | T73I | | 10 | RET | D631G |
| 8 | PTK2B | G414V | | 10 | RET | D898_E901del |
| 8 | PTK2B | R429C | | 10 | RET | E632_A640>VRP |
| 9 | ABL1 | D276G | | 10 | RET | E632_L633>V |
| 9 | ABL1 | E255K | | 10 | RET | E632_L633del |
| 9 | ABL1 | E255V | | 10 | RET | E632_L633del |
| 9 | ABL1 | E355G | | 10 | RET | E768D |
| 9 | ABL1 | F311L | | 10 | RET | F612_C620del |
| 9 | ABL1 | F317L | | 10 | RET | F612_C620del |
| 9 | ABL1 | F359V | | 10 | RET | M918T |
| 9 | ABL1 | G250E | | 10 | RET | M918T |
| 9 | ABL1 | H396R | | 11 | HRAS | G12C |
| 9 | ABL1 | L248V | | 11 | HRAS | G12R |
| 9 | ABL1 | M244V | | 11 | HRAS | G12V/G12D |
| 9 | ABL1 | M351T | | 11 | HRAS | G13C/G13R/G13S |
| 9 | ABL1 | Q252H | | 11 | HRAS | Q61H/Q61H |
| 9 | ABL1 | T315I | | 11 | HRAS | Q61K |
| 9 | ABL1 | Y253F | | 11 | HRAS | Q61L/Q61R/Q61P |
| 9 | ABL1 | Y253H | | 12 | CDK | R24C |
| 9 | CDKN2A | D84Y | | 12 | CDK | R24H |
| 9 | CDKN2A | E61* | | 12 | CDK4 | R24C |
| 9 | CDKN2A | E69* | | 12 | CDK4 | R24H |
| 9 | CDKN2A | E88* | | 12 | KRAS | A146T |
| 9 | CDKN2A | H83Y | | 12 | KRAS | A59T |
| 9 | CDKN2A | R58* | | 12 | KRAS | A59V |
| 9 | CDKN2A | R80* | | 12 | KRAS | G12 |
| 9 | GNAQ | Q209L | | 12 | KRAS | G12 |
| 9 | GNAQ | Q209L/P | | 12 | KRAS | G12A/G12C/G12D |
| 9 | GNAQ | R183Q | | 12 | KRAS | G12A/G12C/G12D |
| 9 | JAK2 | V617F | | 12 | KRAS | G12F/G12R |
| 9 | ROR2 | A793S | | 12 | KRAS | G12F/G12R |
| 10 | FGFR2 | S252W | | 12 | KRAS | G12S/G12V |
| 10 | FGFR2 | Y376C | | 12 | KRAS | G12S/G12V |
| 10 | PTEN | K267fs*9 | | 12 | KRAS | G13A |
| 10 | PTEN | K6fs*4 | | 12 | KRAS | G13A/D/V |
| 10 | PTEN | N323fs*2 | | 12 | KRAS | G13R |
| 10 | PTEN | N323fs*21 | | 12 | KRAS | G13V/G13D |
| 10 | PTEN | P248fs*5 | | 12 | KRAS | G60D |
| 10 | PTEN | R130* | | 12 | KRAS | L19F |
| 10 | PTEN | R130fs*4 | | 12 | KRAS | Q22K |
| 10 | PTEN | R130G | | 12 | KRAS | Q61 |

| | | | | | | |
|---|---|---|---|---|---|---|
| 12 | KRAS | Q61E/Q61K | | 17 | ERBB2 | P780_Y781 insGSP |
| 12 | KRAS | Q61H/Q61H | | 17 | ERBB2 | S779_P780 insVGS |
| 12 | KRAS | T58I | | 17 | ERBB3 | V777L |
| 12 | PTPN11 | T507K | | 17 | TP53 | D281G |
| 13 | FLT3 | D835del | | 17 | TP53 | D281H/Y |
| 13 | FLT3 | D835H/D835Y | | 17 | TP53 | G245R/S/C |
| 13 | FLT3 | I836del | | 17 | TP53 | G245S |
| 13 | FLT3 | | | 17 | TP53 | R175H |
| 13 | FLT3 | | | 17 | TP53 | R175H/L |
| 13 | FLT4 | D835E | | 17 | TP53 | R248G/W |
| 13 | FLT4 | D835E | | 17 | TP53 | R248Q |
| 13 | FLT5 | I836M | | 17 | TP53 | R248W |
| 13 | RB1 | C706F | | 17 | TP53 | R273C |
| 13 | RB1 | E137* | | 17 | TP53 | R273C |
| 13 | RB1 | E748* | | 17 | TP53 | R273H |
| 13 | RB1 | L199* | | 17 | TP53 | R273H/L |
| 13 | RB1 | L660fs*2 | | 17 | TP53 | R306* |
| 13 | RB1 | R320* | | 17 | TP53 | V143A |
| 13 | RB1 | R358* | | 19 | AKT2 | R371H |
| 13 | RB1 | R455* | | 19 | AKT2 | S302G |
| 13 | RB1 | R552* | | 19 | GNA11 | Q209 |
| 13 | RB1 | R556* | | 19 | GNA11 | R183C |
| 13 | RB1 | R579* | | 19 | JAK3 | A572V |
| 14 | AKT1 | E17del | | 19 | JAK3 | P132T |
| 14 | AKT1 | E319G | | 19 | JAK3 | V722I |
| 14 | AKT1 | E17K | | 19 | STK11 | D194N |
| 14 | AKT1 | E17K | | 19 | STK11 | D194V |
| 14 | AKT1 | L357P | | 19 | STK11 | E199* |
| 14 | AKT1 | P388T | | 19 | STK11 | E199K |
| 14 | AKT1 | Q43X | | 19 | STK11 | E57fs*7 |
| 14 | AKT1 | V167A | | 19 | STK11 | F264fs*22 |
| 14 | AKT1 | V461L | | 19 | STK11 | G196V |
| 15 | MAP2K1 | D67N | | 19 | STK11 | P281fs*6 |
| 15 | MAP2K1 | E203Q/K | | 19 | STK11 | P281L |
| 15 | MAP2K1 | F53S | | 19 | STK11 | Q170* |
| 15 | MAP2K1 | K57N | | 19 | STK11 | Q37* |
| 15 | MAP2K1 | Y134C | | 19 | STK11 | W332* |
| 17 | ERBB2 | A775_G776 insYVMA | | 20 | SRC | Q531* |
| 17 | ERBB2 | D769H | | | | |
| 17 | ERBB2 | G776S/G776LC | | | | |
| 17 | ERBB2 | G776VC | | | | |
| 17 | ERBB2 | L755P | | | | |
| 17 | ERBB2 | P780_Y781 insGSP | | | | |

Such somatic mutations in tumor suppressor genes or somatic mutations in cancer-related genes can be detected by the following procedure: a genomic DNA is prepared from cells and subjected to a whole genome sequencing; in addition, a library for next-generation sequencer is prepared from the genomic DNA and subjected to exon enrichment by an enrichment kit such as TruSeq exome enrichment system (illumina, Inc.), SeqCap EZ (NimbleGen), Agilent Sure Select (Agilent), or Agilent Sure Select Human Kinome Kit (Agilent); followed by, a comprehensive analysis of genetic mutations is performed using a sequence on HiSeq2000 (e.g. 100 bp, paired-end) to detect sequence alterations, which are analytically compared with normal reference sequences to thereby identify somatic sequence alterations in genes. Another procedure that can be used is analysis of somatic mutations by Oncocarta Ver1, 2, and 3 of Sequenome, Inc.

In the present invention, the induced malignant stem cell capable of *in vitro* proliferation may also have (1) (c) abnormal expression (increased or reduced/lost expression) of an endogenous oncogene or an endogenous tumor suppressor gene. Examples of (1) (c) abnormal expression (increased or reduced/lost expression) of an endogenous oncogene or an endogenous tumor suppressor gene include increased expressions of endogenous oncogenes or reduced/lost expressions of endogenous tumor suppressor genes. Such abnormal expressions (increased or reduced/lost expressions) of endogenous oncogenes or endogenous tumor suppressor genes preferably occur in at least one of the genes listed in connection with (1) (b).

Such increased expressions of endogenous oncogenes or reduced/lost expressions of endogenous tumor suppressor genes can be detected by the following procedure: mRNA is prepared from cells and gene expression is comprehensively analyzed using a mRNA microarray (Agilent SurePrint G3 Human GE Microarray Kit 8x60K, Whole Human Genome oligo-DNA Microarray Kit Ver2.0 (4x44K), Whole Human Genome Oligo-DNA Microarray Kit (4x44K)) and compared with the gene expression in standard cells, whereby the abnormal expression of mRNA is comprehensively identified.

In the present invention, the induced malignant stem cell capable of proliferation *in vitro* may also have (1) (d) abnormal expression (increased expression or reduced/lost expression) of a non-coding RNA such as an endogenous cancer-related microRNA. Such abnormal expression (increased expression or reduced/lost expression) of a non-coding RNA such as an endogenous cancer-related microRNA preferably occurs in at least one of the microRNAs listed in the following table:

[Table 4]

| Table 4: Cancer-related microRNAs that might cause abnormal expression (condition (1) (d)) | |
|---|---|
| Cancer | miRNA |
| Brain Cancer | let-7g ; mir-10a ; mir-124-2 ; mir-126 ; mir-149 ; mir-155 ; mir-15b Cluster (mir-15b, mir-16-2) ; mir-17 cluster (mir-17, mir-18a, mir-19a, mir-19b-1, mir-20a, mir-92a-1) ; miR-191 Cluster (miR-191, miR-425) ; mir-210 ; mir-218-1 ; mir-218-2 ; mir-23b Cluster (mir-23b, mir-24-1, mir-27b) ; mir-301a ; mir-30c-1 Cluster (mir-30c-1, mir-30e) ; mir-32 ; mir-34a ; mir-378 ; mir-7-1 |
| Breast Cancer | mir-155 ; mir-17 cluster (mir-17, mir-18a, mir-19a, mir-19b-1, mir-20a, mir-92a-1) |
| Colon Cancer | mir-17 cluster (mir-17, mir-18a, mir-19a, mir-19b-1, mir-20a, mir-92a-1) ; mir-378 |
| Head & Neck Cancer | let-7i ; mir-10a ; mir-155 ; mir-15b Cluster (mir-15b, mir-16-2) ; mir-17 cluster (mir-17, mir-18a, mir-19a, mir-19b-1, mir-20a, mir-92a-1) mir-210 ; mir-218-1 ; mir-218-2 ; mir-23b Cluster (mir-23b, mir-24-1, mir-27b) ; mir-30c-1 Cluster (mir-30c-1, mir-30e) ; mir-34a ; mir-378 |
| Kidney Cancer | mir-210 |
| Liver Cancer | mir-126 ; mir-17 cluster (mir-17, mir-18a, mir-19a, mir-19b-1, mir-20a, mir-92a-1) ; miR-191 Cluster (miR-191, miR-425) ; mir-193b ; mir-23b Cluster (mir-23b, mir-24-1, mir-27b) ; mir-30c-1 Cluster (mir-30c-1, mir-30e) |
| Lung Cancer | let-7i ; mir-1-1 ; mir-126 |
| Lymphoma | mir-155 ; mir-23b Cluster (mir-23b, mir-24-1, mir-27b) ; mir-378 |
| Ovarian Cancer | let-7i ; mir-126 ; mir-155 ; mir-196a-1 ; mir-34a ; mir-34c Cluster (mir-34c, mir-34b) |
| Pancreatic Cancer | mir-10a ; mir-155 ; mir-210 ; mir-23b Cluster (mir-23b, mir-24-1, mir-27b) |
| Prostate Cancer | mir-149 ; mir-15b Cluster (mir-15b, mir-16-2) |
| Skin Cancer | mir-149 ; mir-15b Cluster (mir-15b, mir-16-2) ; mir-17 cluster (mir-17 ; mir-18a ; mir-19a ; mir-19b-1 ; mir-20a ; mir-92a-1) ; mir-193b ; mir-23b Cluster (mir-23b, mir-24-1, mir-27b) |

Specific sequences of these microRNAs are each known in the technical field of interest art, as shown in the following table.

[Table 5]

| Table 5: Cancer-related microRNAs that might cause abnormal expression (condition (1) (d)) | | | |
|---|---|---|---|
| Precursor miRNA ID | Precursor miRNA Accession No. | Matured miRNA ID | matured miRNA Accession No. |
| let-7g | MI0000433 | let-7g | MIMAT0000414 |
| let-7i | MI0000434 | let-7i | MIMAT0000415 |
| mir-10a | MI0000266 | miR-10a | MIMAT0000253 |
| mir-1-1 | MI0000651 | miR-1 | MIMAT0000416 |
| mir-124-2 | MI0000444 | miR-124 | MIMAT0000422 |
| mir-126 | MI0000471 | miR-126 | MIMAT0000445 |
| mir-149 | MI0000478 | miR-149 | MIMAT0000450 |
| mir-155 | MI0000681 | miR-155 | MIMAT0000646 |
| mir-15b | MI0000438 | miR-15b | MIMAT0000417 |
| mir-16-2 | MI0000115 | miR-16 | MIMAT0000069 |
| mir-17 | MI0000071 | miR-17 | MIMAT0000070 |
| mir-18a | MI0000072 | miR-18a | MIMAT0000072 |
| mir-19a | MI0000073 | miR-19a | MIMAT0000073 |
| mir-19b-1 | MI0000074 | miR-19b | MIMAT0000074 |
| mir-20a | MI0000076 | miR-20a | MIMAT0000075 |
| mir-92a-1 | MI0000093 | miR-92a | MIMAT0000092 |
| mir-191 | MI0000465 | miR-191 | MIMAT0000440 |
| mir-425 | MI0001448 | miR-425 | MIMAT0003393 |
| mir-193b | MI0003137 | miR-193b | MIMAT0002819 |
| mir-196a-1 | MI0000238 | miR-196a | MIMAT0000226 |
| mir-210 | MI0000286 | miR-210 | MIMAT0000267 |
| mir-218-1 | MI0000294 | miR-218 | MIMAT0000275 |
| mir-218-2 | MI0000295 | miR-218 | MIMAT0000275 |
| mir-23b | MI0000439 | miR-23b | MIMAT0000418 |
| mir-24-1 | MI0000080 | miR-24 | MIMAT0000080 |
| mir-27b | MI0000440 | miR-27b | MIMAT0000419 |
| mir-301a | MI0000745 | miR-301a | MIMAT0000688 |
| mir-30c-1 | MI0000736 | miR-30c | MIMAT0000244 |
| mir-30e | MI0000749 | miR-30e | MIMAT0000692 |
| mir-32 | MI0000090 | miR-32 | MIMAT0000090 |
| mir-34a | MI0000268 | miR-34a | MIMAT0000255 |
| mir-34b | MI0000742 | miR-34b | MIMAT0004676 |
| mir-34c | MI0000743 | miR-34c-3p | MIMAT0004677 |
| | | miR-34c-5p | MIMAT0000686 |
| mir-378 | MI0000786 | miR-378 | MIMAT0000732 |
| mir-7-1 | MI0000263 | miR-7 | MIMAT0000252 |

Such abnormal expressions (increased expression or reduced/lost expression) of a non-coding RNA such as a cancer-related microRNA can be detected by the following procedure: mRNA is prepared from cells and gene expression is comprehensively analyzed using a miRNA microarray (Agilent Human miRNA Microarray Rel.12.) and compared with the gene expression in standard cells, whereby the abnormal expression of microRNA is comprehensively identified.

In the present invention, the induced malignant stem cell capable *of in vitro* proliferation may also have (1) (e) abnormal expression of an endogenous cancer-related protein. An increased expression or reduced/lost expression of such an endogenous cancer-related protein means that the expression of the same protein is high or low or entirely absent from the induced malignant stem cell as compared with the expression in induced pluripotent stem cells (iPS cells) or that the induced malignant stem cell expresses a cancer-specific antigen.

In the present invention, the endogenous cancer-related protein that might show abnormal expression (increased expression or reduced/lost expression) or the cancer-specific antigen may be exemplified by any one of Muc-1,VEGF-C, HnRNP A2/B1, E2F3, MAGE A4, MMP-9, Cytokeratin-19, E2F1, c-kit, Muc-4, Cytokeratin-20, c-met, L-myc, MDR1, hCGβ, COX-2, CA125, MAGE A12, NSE, c-myc, CD44, Her2/Neu, RCAS1, bcl-2, FGFR2, HIF-1α, GPC3, Cyclin D1, mdm2, Cytokeratin-7, MMP-2, Survivin, hTERT, Gli1, Thyroglobulin, VEGF-A, AFP, CEA, CGA, EGFR, MAGE A1, MAGE A3/A6, Muc-7, ProGRP, PSA, SCC, IGF2, DLK-1, and WT-1.

Such abnormal expression (increased expression or reduced/lost expression) of cancer-related proteins can be detected by the following procedure: a protein is prepared from cells and using iTRAQ (registered trademark) (AB SCIEX), protein expression, relative quantitative analysis and mass spectrometry are performed comprehensively and comparison is made with the protein expressed in standard cells, whereby the abnormally expressed protein is identified. The reagent iTRAQ of AB SCIEX is an amine-specific reagent set for stable isotopes that simultaneously labels all peptides in up to four or eight different biosamples and which enables both relative and absolute quantification from MS/MS spectra.

In the present invention, the induced malignant stem cell capable of *in vitro* proliferation may also have (1) (f) an aberration of endogenous cancer-related metabolism (hypermetabolism or hypometabolism). Such aberration of endogenous cancer-related metabolism may involve an aberration of metabolome as compared with induced pluripotent stem cells or enhancement in the glycolysis system as compared with induced pluripotent stem cells.

To analyze an aberration of metabolism, such as enhancement in the glycolysis system, on the basis of metabolome, an analytical technique that allows the metabolome to be measured in a high throughput manner within a short period, such as capillary electrophoresis-mass spectrometry (CE-MS), may be employed.

In CE-MS, upon voltage application to the capillary, all of the cationic metabolites are moved toward the cathode. Within the capillary, analytes are separated by differences in the charge on the analytes and their hydrated ionic radius and introduced into the mass spectrometer connected to the cathode. In the mass spectrometer, each analyte is detected selectively and in high sensitivity based on its mass number. What is best about this method is that, on account of the hollow capillary that is employed, all of the cationic metabolites can be introduced into the mass spectrometer under a single analytical condition. If, on the other hand, anionic metabolites are to be measured, the mass spectrometer suffices to be connected to the anode. Thus, intracellular metabolites can be analyzed simultaneously under only two conditions of measurement.

In the present invention, the induced malignant stem cell capable *of in vitro* proliferation may also have (1) (g) an aberration of endogenous cancer-related sugar chain. Such aberrations of cancer-related sugar chain may involve the expression of cancer-specific sugar chains.

Such aberrations of endogenous cancer-related sugar chains can be identified by the following procedure: a solution of cell lysate is prepared from cells; asparagine-linked (N-linked) sugar chains that are conjugated to proteins are subjected to a comprehensive structural analysis; the result obtained is analyzed by comparison with the result of sugar chain analysis in standard cells. For example, N-linked sugar chains conjugated to a protein are cleaved with an enzyme and, thereafter, information from sequential analysis by three different HPLC columns is searched with database software GALAXY loaded with information on about 600 sugar chains (GALAXY: Glycoanalysis by the three axes of MS and chromatography; sugar-chain map for structural determination and prediction of sugar chains) to narrow down candidate sugar chains and their structures can be identified by coloading of authentic candidate sugar chains and the sample sugar chains.

In the present invention, the induced malignant stem cell capable of *in vitro* proliferation may also have (1) (h) an aberration of copy number variations in endogenous genomic DNA of genetic copy number. The term ** as used herein may cover the case where the genetic copy number of the endogenous genomic DNA is **. Examples of the endogenous genomic DNA that might cause such variations in copy gene number include the genes listed in the following tables.

[Table 6]

Table 6: Genomic DNA genetic regions having an aberration of variations in copy number (condition (1) (h))

AD0040_Set01

| No. | Chr | Cytoband | Position | Amp/Del | P-value | Gene Names, Annotations |
|---|---|---|---|---|---|---|
| 1 | 17 | p13.3 | 1959569-1959686 | 1.958922 | 4.24E-24 | SMG6, CNV_72769 |

AD0040_Set02

| No. | Chr | Cytoband | Position | Amp/Del | P-value | Gene Names, Annotations |
|---|---|---|---|---|---|---|
| 1 | 1 | q32.1 | 203188201-203193723 | -1.154289 | 7.26E-11 | NFASC |
| 2 | 2 | p21 | 44083711-44096180 | -1.466304 | 1.64E-14 | CNV_78526, CNV_89620, CNV_73443... |
| 3 | 4 | p16.1 | 8575302-8575359 | -1.004219 | 2.68E-12 | CNV_3479 |
| 4 | 4 | p16.1 | 8881212-8882547 | -1.338401 | 2.49E-12 | CNV_3479, CNV_2497, CNV_0347... |
| 5 | 4 | q22.1 | 93434342-93961504 | -0.977201 | 9.66E-277 | GRID2, CNV_10054, CNV_4406... |
| 6 | 5 | p13.3 | 34268357-34369165 | -1.613071 | 5.91E-13 | CNV_3553, CNV_4438, CNV_2087... |
| 7 | 11 | p12 | 41852545-42432402 | -1.001829 | 1.55E-69 | CNV_65929, CNV_61127 |

AD0040_Set03

| No. | Chr | Cytoband | Position | Amp/Del | P-value | Gene Names, Annotations |
|---|---|---|---|---|---|---|
| 1 | 1 | p36.22 | 11506047-11510410 | -0.91124 | 1.45E-10 | PTCHD2 |

| 2 | 1 | p36.13 | 19384235-19393273 | -1.077065 | 8.52E-16 | UBR4 |
|---|---|---|---|---|---|---|
| 3 | 2 | p21 | 44083711-44100016 | -0.835072 | 4.02E-13 | CNV_78526, CNV_89620, CxV_73443… |
| 4 | 2 | q37.3 | 237339565-237344964 | -1.262658 | 5.45E-13 | |
| 5 | 3 | p21.31 | 44941989-44944920 | -0.96966 | 1.85E-10 | ZDHHC3 |
| 6 | 3 | p21.1 | 51941606-51945028 | -1.390224 | 6.96E-37 | RRP9 |
| 7 | 3 | p21.1 | 52157853-52166715 | -0.741786 | 2.84E-11 | WDR51A, CNV_51113 |
| 8 | 3 | q26.31 | 172536286-172538403 | 0.77431 | 4.00E-11 | TNIK |
| 9 | 4 | p16.1 | 8575302-8575359 | -1.394291 | 2.38E-22 | CNV_3479 |
| 10 | 4 | p16.1 | 8882456-8882653 | -1.522356 | 1.87E-22 | CNV_3479, CNV_2497, CNV_0347… |
| 11 | 4 | p16.1 | 8884585-8885187 | -1.388218 | 1.04E-14 | CNV_3479, CNV_2497, CNV_0347… |
| 12 | 5 | p15.33 | 39807-103486 | -0.566404 | 4.31E-17 | CNV_3536, CNV_8470, CNV_37739… |
| 13 | 5 | q35.3 | 178915974-178920549 | -0.867024 | 7.47E-11 | RUFY1, CNV_3590, CNV_2611… |
| 14 | 6 | p21.32 | 31913390-31914895 | -0.936089 | 5.63E-11 | C6orf48, CNV_3602, CNV_4492 |
| 15 | 6 | q24.2 | 144681079-145176611 | -0.803721 | 0 | UTRN, CNV_5395, CNV_51815… |
| 16 | 7 | p22.3 | 1976966-1985089 | -1.174472 | 1.33E-14 | MAD1L1, CNV_4523, CNV_30253… |
| 17 | 7 | p13 | 45115983-45117728 | -0.997113 | 1.17E-10 | TBRG4 |
| 18 | 7 | q36.3 | 158315132-158317964 | -1.435031 | 1.34E-11 | CNV_70131, CNV_65009 |
| 19 | 8 | p23.3 | 1322720-1340312 | -1.256454 | 4.83E-22 | CNV_100233, CNV_70182, CNV_36754… |
| 20 | 8 | p21.3 | 20951820-20964831 | -1.229753 | 1.85E-15 | CNV_3726, CNV_82520, CNV_9531… |
| 21 | 8 | p21.3 | 22263358-22269438 | -1.053818 | 1.67E-12 | PIWIL2, CNV_3726, CNV_2746 |
| 22 | 8 | p12 | 37827143-37827202 | -1.073419 | 3.14E-18 | |
| 23 | 8 | q24.21 | 129012024-129012764 | -1.283134 | 3.75E-15 | PVT1, CNV_37296 |
| 24 | 8 | q24.3 | 145783328-145788273 | -1.211438 | 2.21E-14 | KIAA1688, CNV_4614, CNV_70495 |
| 25 | 9 | p22.1 | 19760010-19770175 | -1.098789 | 8.48E-12 | SLC24A2, CNV_52762 |
| 26 | 9 | q34.3 | 137332375-137332434 | -1.133146 | 1.90E-16 | CNV_30337, CNV_4660 |
| 27 | 10 | q24.33 | 105011210-105018167 | -0.890401 | 3.98E-12 | |
| 28 | 10 | q26.3 | 134889416-134893492 | -1.403986 | 1.89E-11 | KNDC1, CNV_3829, CNV_29875… |
| 29 | 10 | q26.3 | 134978689-134996216 | -0.759088 | 2.38E-12 | CALY, CNV_3829, CNV_4721… |
| 30 | 11 | p15.5 | 1962010-1967283 | -1.272235 | 9.22E-16 | LOC100133545, CNV_29893, CNV_37117… |

| 31 | 11 | p13 | 35269915-35269974 | -1.58566 | 4.59E-21 | SLC1A2 |
|----|----|-----|-------------------|----------|----------|--------|
| 32 | 11 | p11.2 | 45446292-45455071 | -0.999829 | 1.27E-10 | |
| 33 | 11 | p11.2 | 45536937-45547269 | -1.0113 | 3.33E-13 | |
| 34 | 11 | q13.2 | 68845113-68855981 | -0.849465 | 1.22E-14 | CNV_29915 |
| 35 | 11 | q13.5 | 75055163-75056846 | -1.095161 | 4.03E-13 | MAP6 |
| 36 | 11 | q23.2 | 114474724-114494671 | -1.04596 | 2.07E-18 | CNV_3867, CNV_4763, CNV_30567... |
| 37 | 12 | p11.1 | 34417392-34756209 | -1.388555 | 8.75E-18 | CNV_3885, CNV_8723, CNV_9691... |
| 38 | 12 | q13.2 | 54376360-54377782 | -1.314247 | 1.93E-15 | ITGA7,CNV_3890 |
| 39 | 12 | q24.11 | 107744503-107749896 | -1.312198 | 5.02E-15 | SSH1 |
| 40 | 12 | q24.11 | 110069463-110074263 | -1.00593 | 6.16E-11 | CUX2 |
| 41 | 12 | q24.32 | 124804453-124812355 | -1.02832 | 1.86E-13 | CNV_9699, CNV_29926 |
| 42 | 12 | q24.33 | 133173882-133177340 | -1.318578 | 9.51E-14 | CNV_4404 |
| 43 | 13 | q12.11 | 19566409-19568792 | -1.815439 | 3.23E-30 | CNV_71680, CNV_71679 |
| 44 | 13 | q34 | 112553940-112565338 | -1.045415 | 5.56E-12 | ATP11A |
| 45 | 13 | q34 | 114770686-114776626 | -1.49958 | 1.55E-14 | CNV_29947, CNV_71818, CNV_101882... |
| 46 | 14 | q32.31 | 101314727-101318356 | -0.864536 | 6.27E-15 | CNV_8776 |
| 47 | 15 | q26.3 | 101555153-101558598 | -1.35669 | 5.78E-14 | CNV_3982, CNV_8807, CNV_7087 |
| 48 | 16 | p13.13 | 11173868-11178626 | -1.419855 | 4.38E-16 | CLEC16A |
| 49 | 16 | q24.1 | 85302753-85306926 | -0.995341 | 1.46E-12 | CNV_49791, CNV_58781, CNV_67070... |
| 50 | 16 | q24.2 | 86529114-86536801 | -1.053071 | 4.11E-11 | CNV_3134, CNV_30795 |
| 51 | 17 | q21.31 | 37885447-37885501 | -0.74336 | 2.82E-12 | ATP6V0A1 |
| 52 | 17 | q23.2 | 56404749-56407334 | -1.054468 | 7.56E-13 | BCAS3, CNV_4410, CNV_49891... |
| 53 | 17 | q25.2 | 72541570-72547858 | -1.107359 | 5.34E-17 | CNV_5336, CNV_53066, CNV_34522... |
| 54 | 17 | q25.3 | 75476251-75483572 | -0.90787 | 3.74E-13 | |
| 55 | 19 | p12 | 21094293-21098244 | -2.544831 | 6.72E-25 | ZNF714, CNV_78137, CNV_50112... |
| 56 | 19 | q13.11 | 39810209-39814923 | -1.255844 | 1.46E-16 | CNV_73367 |
| 57 | 19 | q13.31 | 48895798-48900793 | -0.799759 | 2.22E-11 | CNV_32261, CNV_47965, CNV_5106... |
| 58 | 19 | q13.32 | 52729604-52729663 | -1.310343 | 1.75E-24 | ZNF541 |
| 59 | 20 | q13.33 | 61437907-61448929 | 0.973892 | 2.88E-15 | CHRNA4, CNV_31044 |
| 60 | 22 | q11.21 | 19712255-19715734 | -1.075087 | 7.15E-12 | P2RX6, SLC7A4, CNV_31071... |
| 61 | 22 | q13.32 | 47558995-47566106 | -0.956944 | 6.21E-12 | CNV_4134, CNV_50883 |
| 62 | 22 | q13.33 | 50695995-50697227 | -1.147529 | 2.70E-13 | CNV_30166 |
| 63 | X | p22.33 | 155819-169113 | -1.217427 | 1.36E-46 | PLCXD1, GTPBP6, CNV_83235... |

| 64 | X | p22.33 | 189104-190572 | -0.996498 | 7.72E-11 | CNV_67918 |
|---|---|---|---|---|---|---|
| 65 | X | p22.33 | 699908-706191 | -0.791549 | 1.88E-13 | CNV_34411 |
| 66 | X | p22.33 | 1562369-1566850 | -1.112982 | 2.22E-22 | P2RY8 |
| 67 | X | p22.33 | 1637614-1639274 | -0.69266 | 3.10E-11 | |
| 68 | X | p22.33 | 2646756-2647777 | -1.242813 | 1.07E-17 | CD99, CNV_4142, CNV_8292... |
| 69 | Y | p11.32 | 105819-119113 | -1.217427 | 1.36E-46 | CNV_83894, CNV_97143 |
| 70 | Y | p11.32 | 139104-140572 | -0.996498 | 7.72E-11 | PLCXD1 |
| 71 | Y | p11.32 | 649908-656191 | -0.791549 | 1.93E-13 | |
| 72 | Y | p11.32 | 1512369-1516850 | -1.112982 | 2.30E-22 | ASMTL |
| 73 | Y | p11.32 | 1587614-1589274 | -0.69266 | 3.18E-11 | P2RY8 |
| 74 | Y | p11.31 | 2596756-2597777 | -1.242813 | 1.10E-17 | |

AD0040_Set04

| No. | Chr | Cytoband | Position | Amp/Del | P-value | Gene Names, Annotations |
|---|---|---|---|---|---|---|
| 1 | 2 | p25.2 | 6148711-6875000 | -0.564855 | 5.96E-120 | CNV_4274, CNV_35845, CNV_9920... |
| 2 | 3 | p21.31 | 50358198-50366080 | -0.859205 | 9.66E-11 | TUSC4, CYB561D2, CNV_3429... |
| 3 | 3 | p21.1 | 51937265-51945028 | -0.591105 | 1.32E-10 | RRP9 |
| 4 | 4 | p16.1 | 8575302-8575359 | -1.15809 | 1.02E-18 | CNV_3479 |
| 5 | 4 | p16.1 | 8882456-8882653 | -1.362018 | 6.33E-21 | CNV_3479, CNV_2497, CNV_0347... |
| 6 | 6 | p25.3 - p11.2 | 167917-58197184 | 0.17055 | 0 | DUSP22, IRF4, EXOC2... |
| 7 | 6 | p22.1 - p21.33 | 29854870-29902314 | -0.571876 | 8.20E-20 | HCG4, CNV_64460, CNV_64462... |
| 8 | 6 | q11.1 - q27 | 62023384-170890108 | 0.151534 | 0 | KHDRBS2, LGSN, PTP4A1... |
| 9 | 7 | p22.3 | 1976966-1981109 | -1.268264 | 5.89E-12 | MAD1L1, CNV_4523, CNV_30253... |
| 10 | 7 | p21.3 - p21.2 | 13055490-13506713 | -0.56786 | 2.42E-78 | CNV_52086, CNV_1723, CNV_94383... |
| 11 | 7 | q11.23 | 72831668-72832641 | -1.0686 | 3.47E-10 | CNV_3685 |
| 12 | 8 | p12 | 37827143-37827202 | -0.769092 | 3.36E-12 | |
| 13 | 8 | q24.21 | 129012024-129012764 | -1.791079 | 2.12E-24 | PVT1, CNV_37296 |
| 14 | 8 | q24.3 | 142383673-142390195 | -1.229718 | 2.62E-12 | CNV_30288 |
| 15 | 10 | q26.3 | 134978689-134993118 | -0.606783 | 1.34E-10 | CALY, CNV_3829, CNV_4721... |
| 16 | 11 | p15.5 | 1114014-1115396 | -1.077322 | 7.79E-12 | CNV_3831, CNV_29887 |
| 17 | 11 | p13 | 35269915-35269974 | -1.947078 | 2.67E-30 | SLC1A2 |
| 18 | 11 | q13.2 | 68845113-68849973 | -1.00041 | 5.40E-10 | CNV_29915 |
| 19 | 11 | q13.3 | 69357011-69478523 | -0.261822 | 1.53E-11 | CNV_5631, CNV_4755, CNV_85835 |
| 20 | 11 | q13.5 | 75055163-75056846 | -1.196246 | 3.98E-15 | MAP6 |
| 21 | 11 | q23.2 | 114474724-114494671 | -1.007421 | 1.10E-17 | CNV_3867, CNV_4763, CNV_30567... |
| 22 | 13 | q12.11 | 19566409-19568792 | -1.034896 | 2.93E-13 | CNV_71680, CNV_71679 |

| 23 | 13 | q14.2 - q34 | 48225461-115105297 | 0.370811 | 0 | FNDC3A, MLNR, CDADC1... |
| 24 | 13 | q34 | 114743988-114747979 | -0.528156 | 1.97E-10 | CNV_29947 |
| 25 | 13 | q34 | 114769518-114788319 | -0.408921 | 2.71E-17 | CNV_29947, CNV_71818, CNV_101882... |
| 26 | 16 | q24.2 | 86530833-86536801 | -1.078159 | 1.87E-11 | CNV_3134, CNV_30795 |
| 27 | 17 | q25.3 | 75476251-75483572 | -0.9129 | 2.28E-12 | |
| 28 | 19 | p12 | 21094293-21098244 | -1.806828 | 8.86E-16 | ZNF714, CNV_78137, CNV_50112... |
| 29 | 20 | p12.3 - p11.1 | 8891768-26075841 | 0.388611 | 0 | PLCB4, C20orf103, PAK7... |
| 30 | 20 | q11.21 - q13.33 | 29844444-62949149 | 0.408412 | 0 | TPX2, MYLK2, FOXS1... |
| 31 | 22 | q11.21 | 19712255-19715734 | -1.186638 | 5.68E-14 | P2RX6, SLC7A4, CNV_31071... |
| 32 | 22 | q11.21 | 20125513-20144135 | -0.844823 | 3.83E-16 | HIC2, CNV_31071, CNV_4117... |
| 33 | 22 | q13.32 | 47558995-47566106 | -0.695915 | 2.91E-10 | CNV_4134, CNV_50883 |
| 34 | X | p22.33 | 155819-164781 | -1.341537 | 2.66E-46 | PLCXD1, GTPBP6, CNV_83235... |
| 35 | X | p22.33 | 187113-190572 | -1.174457 | 2.36E-26 | CNV_67918 |
| 36 | X | p22.33 | 303009-314555 | -0.483352 | 2.29E-11 | CNV_73888 |
| 37 | X | p22.33 | 1471240-1472998 | -1.153505 | 1.48E-15 | CNV_73906 |
| 38 | X | p22.33 | 1562369-1566850 | -1.120176 | 1.44E-19 | P2RY8 |
| 39 | Y | p11.32 | 105819-114781 | -1.341537 | 2.66E-46 | CNV_83894, CNV_97143 |
| 40 | Y | p11.32 | 137113-140572 | -1.174457 | 2.36E-26 | PLCXD1 |
| 41 | Y | p11.32 | 253009-264555 | -0.483352 | 2.29E-11 | PPP2R3B |
| 42 | Y | p11.32 | 1421240-1422998 | -1.153505 | 1.48E-15 | IL3RA |
| 43 | Y | p11.32 | 1512369-1516850 | -1.120176 | 1.44E-19 | ASMTL |

AD0040_Set05

| No. | Chr | Cytoband | Position | Amp/Del | P-value | Gene Names, Annotations |
|---|---|---|---|---|---|---|
| 1 | 1 | p36.33 - p11.1 | 759762-121329506 | -0.311631 | 0 | LOC643837, FAM41C, FLJ39609... |
| 2 | 1 | p36.22 | 11506047-11510410 | -1.623647 | 1.52E-15 | PTCHD2 |
| 3 | 1 | p34.3 | 34590539-34590598 | -1.185761 | 1.16E-12 | CNV_29576, CNV_29577 |
| 4 | 2 | p25.2 | 6148711-6875000 | -1.046482 | 0 | CNV_4274, CNV_35845, CNV_9920... |
| 5 | 2 | p21 | 44083711-44100016 | -0.962256 | 9.63E-17 | CNV_78526, CNV_89620, CNV_73443... |
| 6 | 2 | q37.3 | 237339565-237344964 | -1.12891 | 5.17E-11 | |
| 7 | 3 | p21.31 | 44941989-44944920 | -1.10293 | 2.01E-12 | ZDHHC3 |
| 8 | 3 | p21.1 | 51941606-51941665 | -1.570327 | 5.43E-42 | |
| 9 | 3 | p21.1 | 52157853-52166715 | -0.764702 | 1.40E-11 | WDR51A, CNV_51113 |
| 10 | 3 | p14.3 | 55514963-55520108 | -0.807534 | 3.32E-11 | ERC2, CNV_3430 |
| 11 | 4 | p16.1 | 8575302-8575359 | -1.596547 | 6.08E-27 | CNV_3479 |

| 12 | 4 | p16.1 | 8881212-8885187 | -0.836567 | 6.94E-15 | CNV_3479, CNV_2497, CNV_0347 |
|---|---|---|---|---|---|---|
| 13 | 4 | q13.1 | 64932715-64958903 | -1.212414 | 2.11E-10 | SRD5A2L2 |
| 14 | 4 | q25 | 108607270-108770678 | 0.347478 | 7.39E-35 | PAPSS1 |
| 15 | 4 | q31.21 | 143422425-143437437 | -0.798176 | 1.20E-12 | INPP4B |
| 16 | 4 | q35.1 | 186948059-186972601 | -0.780162 | 7.04E-11 | SORBS2, CNV_53588, CNV_68870 |
| 17 | 6 | p25.3 | 1603954-1615979 | -0.826792 | 2.96E-11 | GMDS |
| 18 | 6 | p22.1 - p21.33 | 29854870-29917547 | -1.780237 | 1.89E-72 | HCG4, HLA-G, CNV_64460… |
| 19 | 6 | p21.32 | 32605385-32631881 | -0.849512 | 1.39E-20 | HLA-DRB5, HLA-DRB6, CNV_3603… |
| 20 | 6 | p21.2 | 37661196-37665381 | -1.211464 | 4.15E-14 | CNV_8512 |
| 21 | 6 | p12.3 | 45968671-45975445 | -0.688034 | 2.69E-12 | CLIC5, CNV_0078 |
| 22 | 6 | p12.1 | 53929240-53934834 | -3.201252 | 1.28E-18 | CNV_3614, CNV_31288, CNV_8516… |
| 23 | 6 | q16.1 | 95408458-95417756 | -0.921046 | 1.17E-12 | CNV_52028, CNV_34592, CNV_52029 |
| 24 | 6 | q16.3 | 103910750-103946150 | -2.99956 | 1.93E-25 | CNV_53366, CNV_99645, CNV_99646 |
| 25 | 6 | q25.3 | 159115154-159119516 | -1.197527 | 1.06E-12 | EZR |
| 26 | 6 | q27 | 166262779-166267277 | -1.136934 | 1.12E-12 | C6orf176, CNV_3652 |
| 27 | 7 | p22.1 | 5770846-5779002 | -0.826805 | 2.09E-10 | RNF216, CNV_53516 |
| 28 | 7 | p21.3 - p21.2 | 13055490-13506713 | -0.968181 | 1.09E-192 | CNV_52086, CNV_1723, CNV_94383… |
| 29 | 7 | p11.2 | 55538137-55543418 | -1.237556 | 4.78E-11 | ECOP |
| 30 | 7 | q22.1 | 100239082-100247277 | -0.804057 | 3.13E-11 | EPHB4, CNV_4550 |
| 31 | 7 | q36.1 | 151531289-151531319 | -1.121547 | 7.31E-12 | MLL3 |
| 32 | 8 | p21.3 | 20951820-20964831 | -1.007687 | 3.40E-11 | CNV_3726, CNV_82520, CNV_95311… |
| 33 | 8 | p21.3 | 22263358-22269438 | -0.89917 | 3.41E-10 | PIWIL2, CNV_3726, CNV_2746 |
| 34 | 8 | p12 | 37827143-37827202 | -1.36743 | 2.58E-26 | |
| 35 | 8 | q24.21 | 129012024-129012764 | -1.861445 | 2.64E-25 | PVT1, CNV_37296 |
| 36 | 9 | q34.12 | 132642863-132652875 | -0.67207 | 2.11E-11 | ABL1 |
| 37 | 9 | q34.13 | 134879638-134884316 | -1.056401 | 2.75E-10 | |
| 38 | 9 | q34.3 | 137332375-137332434 | -0.900988 | 2.47E-14 | CNV_30337, CNV_4660 |
| 39 | 10 | p12.31 | 21459641-21463968 | -1.099716 | 5.33E-13 | NEBL, C10orf113 |
| 40 | 10 | q24.33 | 105011210-105018167 | -0.808046 | 1.54E-10 | |
| 41 | 10 | q26.3 | 132819421-132829669 | -0.947973 | 6.81E-11 | TCERG1L |
| 42 | 10 | q26.3 | 134987375-134991871 | -1.053537 | 6.23E-12 | CALY, CNV_3829, CNV_4721… |
| 43 | 10 | q26.3 | 135281682-135287473 | -1.2869 | 1.47E-12 | CNV_2896, CNV_8673, CNV_8671… |
| 44 | 11 | p15.5 | 417922-438827 | -0.538444 | 3.01E-11 | ANO9, CNV_29880, CNV_29882… |

| 45 | 11 | p15.5 | 1962010-1967283 | -1.137617 | 8.69E-14 | LOC100133545, CNV_29893, CNV_37117… |
|----|----|-------|-----------------|-----------|----------|------|
| 46 | 11 | p13 | 35269915-35269974 | -2.362248 | 1.00E-32 | SLC1A2 |
| 47 | 11 | p11.2 | 45446292-45455071 | -1.35674 | 1.76E-15 | |
| 48 | 11 | p11.2 | 45536937-45547269 | -1.261453 | 1.54E-16 | |
| 49 | 11 | q13.1 | 64373699-64389963 | -0.539648 | 4.61E-11 | EHD1, CNV_5422, CNV_4752… |
| 50 | 11 | q13.2 | 68845113-68855981 | -0.686771 | 1.60E-11 | CNV_29915 |
| 51 | 11 | q13.5 | 75055163-75056846 | -1.181678 | 8.74E-15 | MAP6 |
| 52 | 11 | q14.1 | 79146889-79150365 | -0.988344 | 6.22E-11 | |
| 53 | 11 | q23.2 | 114474724-114494671 | -1.222935 | 1.46E-22 | CNV_3867, CNV_4763, CNV_30567… |
| 54 | 12 | p13.33 | 1603701-1609148 | -1.002602 | 4.34E-11 | WNT5B |
| 55 | 12 | p13.33 | 2459007-2462164 | -1.00103 | 1.59E-11 | CACNA1C |
| 56 | 12 | q13.13 | 50170516-50187346 | -0.683817 | 1.06E-10 | SLC4A8, CNV_86368 |
| 57 | 12 | q13.2 | 54376360-54377782 | -2.255378 | 1.44E-30 | ITGA7, CNV_3890 |
| 58 | 13 | q12.11 | 19566409-19568792 | -1.412693 | 2.83E-20 | CNV_71680, CNV_71679 |
| 59 | 13 | q12.3 | 30605647-30656414 | -0.695439 | 8.71E-16 | HSPH1 |
| 60 | 13 | q14.2 - q34 | 48225461-115105297 | 0.525393 | 0 | FNDC3A, MLNR, CDADC1… |
| 61 | 13 | q32.3 | 100080292-100084653 | -0.515989 | 5.93E-11 | TMTC4 |
| 62 | 13 | q33.1 | 100621234-100625172 | -0.546582 | 6.26E-12 | NALCN |
| 63 | 13 | q34 | 112346947-112529339 | -0.110391 | 8.73E-16 | C13orf35, ATP11A, CNV_3926 |
| 64 | 13 | q34 | 112553940-112565338 | -0.317672 | 1.46E-10 | ATP11A |
| 65 | 13 | q34 | 114769518-114788319 | -0.256096 | 7.01E-17 | CNV_29947, CNV_71818, CNV_101882… |
| 66 | 13 | q34 | 114912404-114924113 | -0.175258 | 7.39E-11 | CNV_29948, CNV_71824, CNV_71823 |
| 67 | 14 | q32.31 | 100635039-100643492 | -0.954125 | 3.26E-10 | CNV_76722, CNV_87348 |
| 68 | 14 | q32.31 | 101132700-101136328 | -1.233816 | 8.99E-13 | CNV_47864, CNV_8776 |
| 69 | 14 | q32.31 | 101314727-101318356 | -0.770087 | 8.77E-13 | CNV_8776 |
| 70 | 15 | q26.3 | 100833003-100835108 | -1.990568 | 6.52E-26 | |
| 71 | 15 | q26.3 | 101555153-101558598 | -1.202154 | 1.99E-13 | CNV_3982, CNV_8807, CNV_7087 |
| 72 | 16 | q24.2 | 86529114-86536801 | -1.129622 | 2.27E-16 | CNV_3134, CNV_30795 |
| 73 | 17 | p13.3 | 2246758-2258130 | -0.70325 | 4.76E-12 | MNT, LOC284009, CNV_67107 |
| 74 | 17 | q25.2 | 72510034-72513509 | -1.558487 | 2.02E-19 | CNV_5336, CNV_53066, CNV_34522… |
| 75 | 17 | q25.2 | 72541570-72547858 | -1.030258 | 1.02E-15 | CNV_5336, CNV_53066, CNV_34522… |
| 76 | 17 | q25.3 | 74127687-74135747 | -0.797471 | 3.04E-11 | |
| 77 | 19 | p13.3 | 5652790-5656012 | -1.400095 | 3.53E-18 | LONP1 |
| 78 | 19 | p13.2 | 11589908-11592624 | -0.988635 | 1.65E-10 | ZNF627 |

| 79 | 19 | p12 | 21094293-21098244 | -2.160212 | 6.95E-20 | ZNF714, CNV_78137, CNV_50112... |
| 80 | 19 | q13.11 | 37739553-37743272 | -1.188304 | 2.87E-14 | CNV_78177, CNV_89217 |
| 81 | 19 | q13.11 | 39810209-39814923 | -1.252695 | 8.59E-15 | CNV_73367 |
| 82 | 19 | q13.32 | 52729604-52729663 | -1.123367 | 6.31E-20 | ZNF541 |
| 83 | 19 | q13.33 | 56185087-56190375 | -1.032553 | 3.03E-12 | |
| 84 | 20 | p12.3 - p11.1 | 8900134-26075841 | 0.575452 | 0 | PLCB4, C20orf103, PAK7... |
| 85 | 20 | p11.21 | 23912869-23925414 | -0.344259 | 2.48E-13 | GGTLC1, CNV_5129 |
| 86 | 20 | q11.21 - q13.33 | 29652452-62911874 | 0.592922 | 0 | ID1, COX4I2, BCL2L1... |
| 87 | 20 | q11.23 | 34796540-34803426 | -0.243389 | 3.26E-11 | NDRG3 |
| 88 | 20 | q13.32 | 57462934-57470482 | -0.379787 | 4.92E-14 | CNV_67720 |
| 89 | 20 | q13.33 | 61290383-61294386 | -0.665497 | 9.88E-15 | CNV_5347, CNV_4106, CNV_5144 |
| 90 | 21 | q22.3 | 41510016-41514904 | -1.296857 | 8.53E-13 | BACE2 |
| 91 | 22 | q11.21 | 20125513-20147529 | -0.707834 | 1.98E-14 | HIC2, CNV_31071, CNV_4117... |
| 92 | 22 | q13.32 | 47558995-47566106 | -0.853665 | 1.76E-11 | CNV_4134, CNV_50883 |
| 93 | X | p22.33 | 155819-169113 | -1.326927 | 4.52E-52 | PLCXD1, GTPBP6, CNV_83235... |
| 94 | X | p22.33 | 187113-190572 | -1.064823 | 8.62E-18 | CNV_67918 |
| 95 | X | p22.33 | 699908-706191 | -1.17171 | 2.02E-17 | CNV_34411 |
| 96 | X | p22.33 | 1562369-1566850 | -1.091515 | 2.82E-15 | P2RY8 |
| 97 | X | p22.33 | 1820491-1831380 | -1.061682 | 1.00E-11 | CNV_67930, CNV_33161, CNV_4142 |
| 98 | X | p22.33 | 2194563-2201252 | -1.147887 | 5.04E-13 | DHRSX, CNV_4142 |
| 99 | X | p22.33 | 2309297-2310369 | -1.404613 | 2.43E-12 | DHRSX, CNV_4142 |
| 100 | X | p22.33 | 2646756-2647777 | -1.782833 | 8.15E-23 | CD99, CNV_4142, CNV_8292... |
| 101 | X | p22.13 | 17789072-17792098 | -1.216957 | 1.03E-14 | RAI2, CNV_67948 |
| 102 | X | q26.2 | 130912192-130913849 | -1.27548 | 7.72E-16 | |
| 103 | Y | p11.32 | 105819-119113 | -1.326927 | 8.24E-56 | CNV_83894, CNV_97143 |
| 104 | Y | p11.32 | 137113-140572 | -1.064823 | 1.51E-16 | PLCXD1 |
| 105 | Y | p11.32 | 649908-656191 | -1.17171 | 2.56E-16 | |
| 106 | Y | p11.32 | 1512369-1516850 | -1.091515 | 2.99E-14 | ASMTL |
| 107 | Y | p11.31 | 1770491-1781380 | -1.061682 | 6.06E-11 | CNV_33187 |
| 108 | Y | p11.31 | 2144563-2151252 | -1.147887 | 3.28E-12 | DHRSX, CNV_83906, CNV_83907... |
| 109 | Y | p11.31 | 2259297-2260369 | -1.404613 | 1.02E-11 | DHRSX |
| 110 | Y | p11.31 | 2596756-2597777 | -1.782833 | 7.51E-22 | |

Amp = Amplification
Del = Deletion

In the present invention, the induced malignant stem cell capable of *in vitro* proliferation may also have (1) (i) instability of microsatellites in endogenous genomic DNA in an induced malignant stem cell. Microsatellites which are repeating sequences of one to several base pairs of DNA are regions that are prone to errors in the number of repetitions (repeats) during DNA replication. A dysfunction of the mismatch repair mechanism causes differences (variations) in the number of repeats in microsatellites between a tumor tissue and the normal tissue. This is called microsatellite instability (MSI). MSI is found in about 90% of the tissues of colon cancer diagnosed as Lynch syndrome (hereditary nonpolyposis colorectal cancer.) An instability of microsatellites is known to be caused by mutations in the germline of mismatch repair genes *MLH1 gene, MSH2 gene, MSH6 gene,* and *PMS2 gene.*

In the present invention, the induced malignant stem cell capable of proliferation *in vitro* may also have a karyotypic aberration or a chromosomal aberration. Such karyotypic or chromosomal aberrations are preferably ones that are related to carcinogenesis and may include chromosomal dislocations and deletions.

These karyotypic or chromosomal aberrations can be identified by a differential staining (G band) technique and multi-color FISH.

The starter somatic cell that may be used to prepare the induced malignant stem cell of the present invention which is capable of *in vitro* proliferation is characterized by being primary cultured cells or cells of fewer passages as prepared from a fresh cancer tissue or a non-cancer tissue that have been taken from a carcinogenic mammal. Examples of the fresh cancer tissue include those of solid cancers or carcinomas, as selected from among stomach cancer, colon cancer, breast cancer, kidney cancer, lung cancer, and liver cancer.

Gene expression in induced malignant stem cells

In the present invention, the induced malignant stem cell capable of *in vitro* proliferation is characterized in that in addition to the specific genomic or epigenetic aberrations related to cancer that are mentioned in (1), it expresses one or more self-renewal related genes, as noted in (2). Hence, these genes (2) in the present invention shall be further explained below.

The genes referred to in (2) are marker genes for undifferentiated embryonic stem cells and they are genes (self-renewal related genes) by which the induced malignant stem cell of the present invention is specified to be a cell that has such a property that it can be subjected to extended passage culture as it remains an induced malignant stem cell that theoretically proliferates without limit and is practically capable of *in vitro* proliferation. These self-renewal related genes are known as genes that are characteristically expressed in pluripotent stem cells. Specifically, these self-renewal related genes include the ones listed in the following table:

[Table 7]

| Table 7: Marker genes for undifferentiated embryonic stem cells (condition (2)) | |
|---|---|
| GeneSymbol | GenbankAccession |
| ACVR2B | MM_001106 |
| CD24 | L33930 |
| CDH1 | NM_004360 |
| CYP26A1 | NM_057157 |
| DNMT3B | NM_175850 |
| DPPA4 | NM_018189 |
| EDNRB | NM_003991 |
| FLT1 | NM_002019 |
| GABRB3 | NM_000814 |
| GATA6 | NM_005257 |
| GDF3 | NM_020634 |
| GRB7 | NM_005310 |
| LIN28 | NM_024674 |
| NANOG | NM_024865 |
| NODAL | NM_018055 |
| PODXL | NM_005397 |
| POU5F1 | NM_002701 |
| SALL4 | NM_020436 |
| SOX2 | NM_003106 |

(continued)

| Table 7: Marker genes for undifferentiated embryonic stem cells (condition (2)) | |
|---|---|
| GeneSymbol | GenbankAccession |
| TDGF1 | NM_003212 |
| TERT | NM_198253 |
| ZFP42 | NM_174900 |
| ZIC3 | NM_003413 |

Among these genes, at least four genes, *POU5F1* gene, *NANOG* gene, *SOX2* gene, and *ZFP42* gene, are preferably expressed in the induced malignant stem cell of the present invention which is capable *of in vitro* proliferation.

In the present invention, it is also preferred that, in addition to *POU5F1* gene, *NANOG* gene, *SOX2* gene, and *ZFP42* gene, seven other genes, *TDGF1* gene, *DNMT3B* gene, *TERT* gene, *GDF3* gene, *SALL4* gene, *GABRB3* gene, and *LIN28* gene are expressed, and in yet another preferred embodiment, all genes listed in Table 7 may be expressed, i.e., *POU5F1* gene, *NANOG* gene, *SOX2* gene, *ZFP42* gene, *ACVR2B* gene, *CD24* gene, *CDH1* gene, *CYP26A1* gene, *DNMT3B* gene, *DPPA4* gene, *EDNRB* gene, *FLT1* gene, *GABRB3* gene, *GATA6* gene, *GDF3* gene, *GRB7* gene, *LIN28* gene, *NODAL* gene, *PODXL* gene, *SALL4* gene, *TDGF1* gene, *TERT gene,* and *ZIC3* gene.

To ensure that the induced malignant stem cell of the present invention remains undifferentiated, it is essential that four genes, *POU5F1* gene, *NANOG* gene, *SOX2* gene, and *ZFP42* gene, as selected from the group of genes listed in Table 7 should be expressed, and the more genes that are expressed, the more preferred.

In the present invention, the endogenous self-renewal related genes which are referred to in (2) above are preferably expressed in the induced cancer stem cells of the present invention in amounts ranging from one eighth to eight times, more preferably from one fourth to four times, most preferably from one half to twice, the amounts of the genes expressed in undifferentiated embryonic stem cells or induced pluripotent stem cells that serve as a control.

The above-mentioned undifferentiated embryonic stem cells that can be used as a control may be either one of hES_H9 (GSM194390), hES_BG03 (GSM194391), and hES_ES01 (GSM194392). Data for the expression of these genes can be downloaded from the database Gene Expression Omnibus [GEO] (Gene Expression Omnibus [GEO], [online], [accessed on January 28, 20100], Internet <http://www.ncbi.nlm.nih.gov/geo/>).

The induced malignant stem cells of the present invention can be subjected to expansion culture or passage culture for at least 3 days but they are induced malignant stem cells capable *of in vitro* proliferation that can effectively be proliferated for at least a month, half a year or even one year and longer; this means that they are theoretically capable of proliferation without limit.

Media to be used and culture methods

Media for expansion culture or passage culture of the induced malignant stem cells of the present invention are not particularly limited as long as they permit the expansion culture or passage culture of embryonic stem cells, pluripotent stem cells, and the like; media suitable for the culture of embryonic stem cells, pluripotent stem cells, and the like are preferably used. Examples of such media include, but are not limited to, an ES medium [40% Dulbecco's modified Eagle medium (DMEM), 40% F12 medium (Sigma), 2 mM L-glutamine or GlutaMAX (Sigma), 1% non-essential amino acid (Sigma), 0.1 mM β-mercaptoethanol (Sigma), 15-20% Knockout Serum Replacement (Invitrogen), 10 μg/ml of gentamicin (Invitrogen), and 4-10 ng/ml of FGF2 factor]; a medium which is prepared by supplementing 0.1 mM β-mercaptoethanol and 10 ng/ml of FGF2 to a conditioned medium that is the supernatant of a 24-hr culture of mouse embryonic fibroblasts (hereinafter referred to as MEF) on an ES medium lacking 0.1 mM β-mercaptoethanol (this medium is hereinafter referred to as MEF conditioned ES medium), an optimum medium for iPS cells (iPSellon), an optimum medium for feeder cells (iPSellon), StemPro (registered trademark) hESC SFM (Invitrogen), mTeSR1 (STEMCELL Technologies/VERITAS), an animal protein free, serum-free medium for the maintenance of human ES/iPS cells, named TeSR2 [ST-05860] (STEMCELL Technologies/VERITAS), a medium for primate ES/iPS cells (ReproCELL), ReproStem (ReproCELL), and ReproFF (ReproCELL). For human cells, media suitable for culturing human embryonic stem cells may be used.

The techniques for effecting expansion culture or passage culture of the induced malignant stem cells of the present invention are not particularly limited if they are methods commonly used by the skilled artisan to culture embryonic stem cells, pluripotent stem cells, and the like. A specific example that may be given is the following: the medium is eliminated from the cells, which are washed with PBS(-); a dissociation solution is added and after standing for a given period, the dissociation solution is removed; after adding a D-MEM (high glucose) medium supplemented with 1X antibiotic-antimycotic and 10% FBS, the cells are subjected to centrifugation and the supernatant is removed; thereafter, 1X antibiotic-antimycotic, mTeSR1 and Y-27632 are added and the cell suspension is seeded on an MEF-seeded gelatin or collagen coat for effecting passage culture.

Preferably, FGF2 (bFGF) is further added to the above-mentioned media, and the preferred amount of addition ranges from 1 to 100 ng/mL. FGF2 (bFGF) is selected depending on the type of the somatic cell to be induced and there can be used FGF2 (bFGF) derived from human, mouse, bovine, equine, porcine, zebrafish, etc. What is more, the aforementioned pituitary gland extract, serum, LIF, Z-VAD-FMK, ALK5 inhibitor, PD032591, CHIR00921, etc. can be added.

Furthermore, inhibitors of Rho associated kinase (Rho-associated coiled coil containing protein kinase), such as Y-27632 (Calbiochem; water soluble) and Fasudil (HA1077: Calbiochem) can also be added to the medium during passage.

Other inhibitors that can be added include: three low-molecular weight inhibitors of FGF receptor tyrosine kinase, MEK (mitogen activated protein kinase)/ERK (extracellular signal regulated kinases 1 and 2) pathway, and GSK (Glycogen Synthase Kinase) 3 [SU5402, PD184352, and CHIR99021], two low-molecular weight inhibitors of MEK/ERK pathway and GSK3 [PD0325901 and CHIR99021], a low-molecular weight compound as an inhibitor of the histone methylating enzyme G9a [BIX-01294 (BIX)], azacitidine, trichostatin A (TSA), 7-hydroxyflavone, lysergic acid ethylamide, kenpaullone, an inhibitor of TGF-β receptor I kinase/activin-like kinase 5 (ALK5) [EMD 616452], inhibitors of TGF-β receptor 1 (TGFBR1) kinase [E-616452 and E-616451], an inhibitor of Src-family kinase [EI-275], thiazovivin, PD0325901, CHIR99021, SU5402, PD184352, SB431542, anti-TGF-β neutralizing antibody, A-83-01, Nr5a2, a p53 inhibiting compound, siRNA against p53, an inhibitor of p53 pathway, etc.

Further, the induced malignant stem cells of the present invention can be frozen or thawed according to known methods. An exemplary method of freezing that may be used is the following: the medium is eliminated from the cells, which are washed with PBS(-); a dissociation solution is added and after standing for a given period, the dissociation solution is removed; after adding a D-MEM (high glucose) medium supplemented with 1X antibiotic-antimycotic and 10% FBS, the cells are subjected to centrifugation and the supernatant is removed; thereafter, a stock solution for freezing is added and the mixture is distributed into cryogenic vials, frozen overnight at -80°C and thereafter stored in liquid nitrogen. An exemplary method of thawing is the following: the frozen sample is thawed in a thermostated bath of 37°C and then suspended in a D-MEM (high glucose) medium supplemented with 1X antibiotic-antimycotic and 10% FBS before use.

To perform its expansion culture, the induced malignant stem cell of the present invention is preferably subjected to co-culture with feeder cells, where it is cultured on feeder cells using an embryonic stem medium that does not require feeder cells. A preferably used embryonic stem medium that does not require feeder cells is mTeSR1 (STEMCELL Technologies), a medium for human embryonic stem cells/human induced pluripotent stem cells, or ReproStem (ReproCELL) supplemented with 5-10 ng/mL of bFGF; both are serum-free media that permit culture under a condition that is free of feeder cells (MEF: mouse embryonic fibroblasts).

Medium is most preferably changed every day. In this case, passage culture is preferably performed once or twice a week using trypsin or collagenase or a mixture thereof.

To determine whether normal human iPS cells could be cultured by the above-described method of expansion culture or passage culture without causing *in vitro* artifact chromosomal aberrations, the present inventor performed karyotypic analyses using such a method as multi-color FISH or differential staining (G band). As a result, all of the normal human iPS cells were confirmed to have normal karyotypes, thus verifying that the culture methods described above are advantageous methods that enable an extended culture without causing any chromosomal aberration during culuture. Therefore, if the malignant stem cells induced by the present invention are found to be of a normal karyotype, the starter somatic cell is also found to be of a normal karyotype. If the malignant stem cells induced by the present invention have a chromosomal aberration related to cancer, the chromosomal aberration related to cancer is found to originate from the starter somatic cell. Similarly, if the malignant stem cells induced by the present invention have an aberration related to cancer, the starter somatic cell may also be considered to have an aberration related to cancer.

Other media that are preferably used for expansion culture or passage culture of the induced malignant stem cells of the present invention include those which are suitable for the culture of embryonic stem cells or induced pluripotent stem cells. Examples of such media include an ES medium [40% Dulbecco's modified Eagle medium (DMEM), 40% F12 medium (Sigma), 2 mM L-glutamine or GlutaMAX (Sigma), 1% non-essential amino acid (Sigma), 0.1 mM β-mercaptoethanol (Sigma), 15-20% Knockout Serum Replacement (Invitrogen), and 10 μg/ml of gentamicin (Invitrogen)]; an MEF conditioned ES medium which is the supernatant of a 24-hr culture of mouse embryonic fibroblasts (hereinafter referred to as MEF) on an ES medium supplemented with 5-10 ng/ml of FGF-2; an optimum medium for induced pluripotent stem cells (iPSellon); an optimum medium for feeder cells (iPSellon); StemPro (Invitrogen); an animal protein free, serum-free medium for the maintenance of human embryonic stem cells/induced pluripotent stem cells, named TeSR2 [ST-05860] (STEMCELL Technologies/VERITAS). In particular, if somatic cells taken from a human are to be used, media suitable for culturing human embryonic stem cells may be mentioned as preferred examples.

It should be noted that if the above-described ES medium or any other medium that is not feeder-free is used, co-culture with feeder cells must be performed.

Furthermore, Y-27632 (Calbiochem; water soluble) or Fasudil (HA1077: Calbiochem), both being inhibitors of Rho associated kinase (Rho-associated coiled coil containing protein kinase), can also be added to the medium during passage.

Preferably, a fibroblast growth factor FGF2 (bFGF) is further added to the above-described media, and the preferred amount of addition ranges from 1 to 100 ng/mL. The fibroblast growth factor is selected depending on the type of the somatic cell to be induced and there can be used a fibroblast growth factor derived from human, mouse, bovine, equine, porcine, zebrafish, etc. What is more, fibroblast growth factors other than the aforementioned FGF2, a pituitary gland extract, serum, LIF, Z-VAD-FMK, ALK5 inhibitor, PD032591, CHIR00921, etc. can be added.

Furthermore, it is rpeferred to supplement the media with neutralizing antibodies such as IGF-II inhibitors, anti-IGF-II antibodies, anti-IGF-R1 antibodies, anti-TGF-$\beta$1 antibodies, and anti-activin A antibodies, and in particular, if the induced malignant stem cell of the present invention expresses *IGF-II gene, IGF-R1 gene, TGF-$\beta$1 gene,* or *activin A gene* in high yield, addition of these components is preferred for the purpose of maintaining the proliferation of the induced malignant stem cell of the present invention.

Other inhibitors that can be added include: three low-molecular weight inhibitors of FGF tyrosine kinase receptor, Mek (mitogen activated protein kinase)/Erk (extracellular signal regulated kinases 1 and 2) pathway, and GSK3 [SU5402, PD184352, and CHIR99021 (products of Axon Medchem: Cat no. 1386)]; a low-molecular weight inhibitor of FGF receptor [PD173074]; a low-molecular weight inhibitor of Mek pathway [PD0325901]; a low-molecular weight inhibitor of GSK3 [BIO]; 7-hydroxyflavone; lysergic acid ethylamide; kenpaullone; an inhibitor of TGF-$\beta$ receptor I kinase/activin-like kinase 5 (Alk5 inhibitor) [EMD 616452, A-83-01 (products of Sigma Aldrich: Cat no. A5480)]; an inhibitor of TGF-$\beta$ receptor 1 (TGFBR1) kinase [E-616451]; an inhibitor of Src-family kinase [EI-275]; thiazovivin; SB431542; Nr5a2, etc.

The techniques for effecting expansion culture or passage culture of the induced malignant stem cells of the present invention are not particularly limited if they are methods commonly used by the skilled artisan to culture embryonic stem cells or induced pluripotent stem cells. A specific preferred example that may be given is the following: the medium is eliminated from the cells, which are washed with PBS(-); a dissociation solution is added and after standing for a given period, the dissociation solution is removed; after adding a D-MEM (high glucose) medium supplemented with 1 x antibiotic-antimycotic and 10% FBS, the cells are subjected to centrifugation and the supernatant is removed; thereafter, 1 x antibiotic-antimycotic, mTeSR1 and Y-27632 are added and the cell suspension is seeded on an MEF-seeded gelatin coat for effecting passage culture.

Further, the induced malignant stem cells of the present invention can be frozen or thawed according to known methods. An exemplary preferred method of freezing that may be used is the following: the medium is eliminated from the cells, which are washed with PBS(-); a dissociation solution is added and after standing for a given period, the dissociation solution is removed; after adding a D-MEM (high glucose) medium supplemented with 1 x antibiotic-antimycotic and 10% FBS, the cells are subjected to centrifugation and the supernatant is removed; thereafter, a stock solution for freezing is added and the mixture is distributed into cryogenic vials, frozen overnight at -80°C and thereafter stored in liquid nitrogen. An exemplary preferred method of thawing is the following: the frozen sample is thawed in a thermostated bath of 37°C and then suspended in a D-MEM (high glucose) medium supplemented with 1 x antibiotic-antimycotic and 10% FBS before use.

Method of producing induced malignant stem cells

In its second aspect, the present invention provides a process for producing the above-described induced malignant stem cell capable of *in vitro* proliferation, which is characterized by performing an induction step in which a starter somatic cell prepared from a fresh cancer tissue or a non-cancer tissue taken from a carcinogenic mammal is brought to such a state that the genetic product or products of one to six genes selected from among *POU5F1* gene, *SOX2* gene, *c-Myc* gene, *KLF4* gene, *LIN28* gene, and *NANOG* gene are present within said starter somatic cell.

To state in detail, the starter somatic cell is prepared by shredding the fresh cancer tissue with scissors and treating the same with collagenase and seeded on a culture dish coated with Matrigel and, one day later, exogenous human genes, *OCT3/4* gene, *SOX2* gene, *KLF4* gene, *c-Myc* gene, (*LIN28* gene and *NANOG* gene) are transduced using a Sendai viral vector (preferably by a method that realizes long-term expression without changing the genomic sequence of the starter cell). One day after the gene introduction, a co-culture with mouse embryonic fibroblasts (MEF) as feeder cells is performed using ReproStem plus bFGF (5-10 ng/mL) which is a medium for human embryonic stem cells/induced pluripotent stem cells for 1-2 months (medium changed every 1-3 days, and MEF seeded every 7-10 days), whereupon colonies of induced malignant stem cells appear. Each colonies are transferred to one well, for example, in a 24-well plate and, 7-14 days later, transferred to a 6-well plate. An additional 5-10 days later, the cells were passaged to a culture dish having a diameter of 10 cm for further culture. After reaching sub-confluency, an additional 1-3 passaging procedure is performed. Thereafter, a culture is performed under a condition free of feeder cells on a culture dish coated with Matrigel or the like to thereby prepare induced malignant stem cells.

This process is characterized in that the starter somatic cell is brought to such a state that the genetic product or products of one to six genes selected from among *POU5F1* gene, *SOX2* gene, *c-Myc* gene, *KLF4* gene, *LIN28* gene, and *NANOG* gene are present within said starter somatic cell. As a result, the genes under (2) above (self-renewal related genes) which are inherent in said starter somatic cell are expressed, whereupon the induced malignant stem of the present invention is eventually induced. The term "bringing the starter somatic cell to such a state" should be understood as a comprehensive concept that covers not only the case of modifying the cell to have such a state but also the case of selecting a cell that has been brought to such a state and conditioning the same.

The phrase as used herein which reads "the genetic product or products of one to six genes selected from among *POU5F1* gene, *SOX2* gene, *c-Myc* gene, *KLF4* gene, *LIN28* gene, and *NANOG* gene" refers to either the respective genes, their RNAs, or the proteins therefrom.

The induced malignant stem cell of the present invention is characterized in that the genomic or epigenetic aberration related to cancer that was inherent in the starter somatic cell from which it originates, such as (a) an aberration of methylation (high or low degree of methylation) of a tumor suppressor gene or a cancer-related genetic region in endogenous genomic DNA, (b) a somatic mutation of a tumor suppressor gene or a somatic mutation of an endogenous cancer-related gene in endogenous genomic DNA, (c) abnormal expression (increased or reduced/lost expression) of an endogenous oncogene or an endogenous tumor suppressor gene, (d) abnormal expression (increased or reduced/lost expression) of a noncoding RNA such as an endogenous cancer-related microRNA, (e) abnormal expression of an endogenous cancer-related protein, (f) an aberration of endogenous cancer-related metabolism (hypermetabolism or hypometabolism), or (g) an aberration of endogenous cancer-related sugar chain, is inherited intact by said induced malignant stem cell. Hence, the somatic cell that serves as the starter must be a starter somatic cell prepared from a fresh cancer tissue or a non-cancer tissue taken from a carcinogenic mammal having these genomic or epigenetic aberrations related to cancer.

The mammal from which said starter somatic cell is to be taken is not particularly limited as long as it is a mammal and may be exemplified by rat, mouse, guinea pig, dog, cat, porcine such as minipig, bovine, equine, primates such as monkeys including a cynomolgus monkey, and human, with rat, mouse, guinea pig, dog, cat, minipig, equine, cynomolgus monkey, and human being preferred, and human is used with particular preference.

The nonembryonic starter somatic cell to be used in the present invention may be somatic cells taken from a fresh cancer tissue of a solid cancer or a fresh cancer tissue of a carcinoma. Specific examples include, but are not limited to, a fresh cancer tissue of the brain, a fresh cancer tissue of the liver, a fresh cancer tissue of the esophagus, a fresh cancer tissue of the stomach, a fresh cancer tissue of the duodenum, a fresh cancer tissue of the small intestine, a fresh cancer tissud of the large intestine, a fresh cancer tissue of the colon, a fresh cancer tissue of the pancreas, a fresh cancer tissue of the kidney, a fresh cancer tissue of the lung, a fresh cancer tissue of the mammary gland, a fresh cancer tissue of the skin, and a fresh cancer tissue of the skeletal muscle. It is particularly preferred to use a fresh cancer tissue selected from among stomach cancer, large intestine cancer, breast cancer, kidney cancer, lung cancer, and liver cancer. Most of these fresh cancer tissues or non-cancer tissues are readily available as medical waste, typically during operation in cancer therapy.

Since it is difficult to isolate only cancer cells from a tissue, cells in a cancer tissue which is substantially made up of cancer cells are preferably used in practice. Another option is to use cells in a non-cancer tissue that might contain cancer cells, though in very small amounts.

In the present invention, the tissue taken from a mammal is most preferably used as soon as possible, but if necessary, for the purpose of, such as transportation, it may be chilled in a stock solution such as Hank's balanced salt solution supplemented with an antibiotic and an antimycotic and stored for up to about 24 hours before use. If the tissue is not to be used immediately after being taken, the cells may be frozen until they are thawed just before use.

Alternatively, the starter somatic cell may be used after culture for a short period. The fewer the days of culture of the starter somatic cell to be used, the more preferred. The medium to be used should be one that is suitable for the specific type of cells to be cultured. For culturing endodermal cells, media for endodermal cells, epithelial cells and the like, or the above-mentioned media for embryonic stem cells or induced pluripotent stem cells may be used. In the case of human cells, media for humans are preferably used. Examples that may be used are commercial media for primary culturing of human cells. However, since the starter somatic cell is cultured for relatively a short period, it is also possible to perform culture in a conventional serum-containing medium, such as a 10% fetal bovine serum containing Dulbecco's modified Eagle medium.

Since the nature of a cell usually changes as the number of passages increases, it is preferred in the present invention to use primary cultured cells or cells that have been subjected to culture between one to four passages, and it is more preferred to use primary cultured cells or cells that have been subjected to culture through one to two passages. It is most preferred to use primary cultured cells.

The term "primary culture" as used herein means culturing immediately after somatic cells are taken from a mammal; primary cultured cells (P0), when subjected to one passage culture, give rise to cells of a first passage culture (P1) which in turn may be subjected to one more passage culture, giving rise to cells of a second passage culture (P2).

In the above-described induction step of the process for producing the induced malignant stem cell of the present invention, it suffices that the starter somatic cell is brought to such a state that the genetic product or products of one to six genes as selected from among *POU5F1* gene, *SOX2* gene, *c-Myc* gene, *KLF4* gene, *LIN28* gene, and *NANOG* gene are present within said starter somatic cell; methods of achieving this state include, but are not limited to, ones that are known as techniques for generating induced pluripotent stem cells.

In the above-described induction step of the process for producing the induced malignant stem cell of the present invention, genes that may be used to elevate the intensity of expression of one to six genes as selected from among *POU5F1* gene, *SOX2* gene, *c-Myc* gene, *KLF4* gene, *LIN28* gene, and *NANOG* gene are the one to six genes per se that are selected from among *POU5F1* gene, *SOX2* gene, *c-Myc* gene, *KLF4* gene, *LIN28* gene, and *NANOG* gene. If one to six genes selected from among *POU5F1* gene, *SOX2* gene, *c-Myc* gene, *KLF4* gene, *LIN28* gene, and *NANOG* gene have not been sufficiently expressed in the starter somatic cell, the insufficient genes or genetic products thereof are transduced into the same cell; alternatively, if one to six genes selected from among *POU5F1* gene, *SOX2* gene, *c-Myc* gene, *KLF4* gene, *LIN28* gene, and *NANOG* gene have been expressed in the starter somatic cell, other genes or genetic products thereof may be transduced in place of said one to six genes selected from among *POU5F1* gene, *SOX2* gene, *c-Myc* gene, *KLF4* gene, *LIN28* gene, and *NANOG* gene.

The gene symbols for *POU5F1* gene, *SOX2* gene, *c-Myc* gene, *KLF4* gene, *LIN28* gene, and *NANOG* gene, as well as the corresponding Genbank accession numbers are given in the following table.

[Table 8]

| Table 8: Genes characterizing induced pluripotent stem cells (condition (2)) | |
|---|---|
| GeneSymbol | GenBank Accession No. |
| *POU5F1* | NM_002701 |
| *SOX2* | NM_003106 |
| *c-Myc* | NM_002467 |
| *KLF4* | NM_004235 |
| *LIN28* | NM_024674 |
| *NANOG* | NM_024865 |

The induced malignant stem cell of the present invention can also be induced by transducing genes that are capable of establishing induced pluripotent stem cells, as exemplfieid by *POU5F1* gene, *SOX2* gene, *c-MYC* gene, *KLF4* gene, *LIN28* gene, *NANOG* gene, *OCT* gene family, *SOX* gene family, *Myc* gene family, *KLF* gene family, *TBX3* gene, *PRDM14* gene, *L-MYC* gene, *N-MYC* gene, *SALL1* gene, *SALL4* gene, *UTF1* gene, *ESRRB* gene, *NRSA2* gene, *REM2 GTPase* gene, *TCL-1A* gene, the Yes-associated protein (YAP) gene, the E-cadherin gene, the p53 dominant negative mutant gene, *p53shRNA* gene, *Glis1* gene, *Rarg* gene, etc.

By transducing the above-mentioned genes (e.g. *POU5F1* gene, *SOX2* gene, *c-MYC* gene, *KLF4* gene, *LIN28* gene, and *NANOG* gene) so that the starter somatic cell is brought to such a state that their genetic products are present therein, the genetic products of such genes as *POU5F1* gene, *SOX2* gene, *c-MYC* gene, *KLF4* gene, *LIN28* gene, and *NANOG* gene which are present in the cell will induce the expression of the group of endogenous self-renewal related genes, such as *POU5F1* gene *(OCT3/4* gene), *SOX2* gene, *LIN28* gene, *KLF4* gene, and *NANOG* gene, whereupon the cell starts self-renewal.

A plausible mechanism for this event is that when DNA binding transcription activating factors such as *POU5F1* gene, *SOX2* gene, *c-MYC* gene, *KLF4* gene, *LIN28* gene, and *NANOG* gene bind to a target gene, transcription activators such as PCAF and CBP/p300 are recruited. These transcription activators have histone acetylating enzyme (HAT) activity and acetylate histones in the neighborhood. It is speculated that when the amino group of the lysine residue in a histone is acetylated, the positive charge on the amino group is neutralized, weakening the interaction between nucleosomes. Being triggered by this acetylation, chromatin remodeling factors would be recruited to induce chromatin remodeling, whereupon transcription is started by a basic transcription factor and RNA polymerase.

Transduction of genetic products into the induced malignant stem cells

Methods by which one to six genes selected from among *POU5F1* gene, *SOX2* gene, *c-Myc* gene, *KLF4* gene, *LIN28* gene, and *NANOG* gene, as well as proteins, mRNAs or the like that are genetic products of these genes and which are substitutes for these genes can be transduced into the aforementioned starter somatic cell include, but are not limited to, those which are known as induction techniques for giving rise to induced pluripotent stem cells.

The methods that can be used to transduce the starter somatic cell with one to six genes selected from among *POU5F1* gene, *SOX2* gene, *c-Myc* gene, *KLF4* gene, *LIN28* gene, and *NANOG* gene are not particularly limited if they are known methods, and it is possible to use various vectors including viral vectors, plasmids, human artificial chromosomes (HAC), episomal vectors (EBV), mini-circle vectors, polycistronic expression vectors, vectors as an application of the Cre/loxP system, vectors making use of a phage integrase, and a transposon such as a piggyback.

Viral vectors that can be used to transduce genes into the somatic cell include lentiviral vectors, retroviral vectors, adenoviral vectors, Sendai virus vectors, etc. The most preferred viral vector is Sendai virus vectors. Sendai virus vectors, which are capable of prolonged expression of self-replication genes without changing the genomic sequence of the starter cell (with no RNA gene in the virus being inserted into the cellular genome), are advantageous for the purpose of identifying any somatic mutation in the induced malignant stem cell as prepared.

Viral vector plasmids that can be used may be of any known types of viral vector plasmids. Examples of preferred retroviral vector plasmids are pMXs, pMXs-IB, pMXs-puro, and pMXs-neo (pMXs-IB being prepared by replacing the puromycin registance gene in pMXs-puro with a blasticidin resistance gene) [Toshio Kitamura et. al., "Retrovirus-mediated gene transfer and expression cloning: Powerful tools in functional genomics", Experimental Hematology, 2003, 31(11):1007-14], and other examples include MFG [Proc. Natl. Acad. Sci. USA, 92, 6733-6737 (1995)], pBabePuro [Nucleic Acids Research, 18, 3587-3596 (1990)], LL-CG, CL-CG, CS-CG, CLG [Journal of Virology, 72, 8150-8157 (1998)], etc. Adenoviral vector plasmids that can be used include pAdex1 [Nucleic Acids Res., 23, 3816-3821 (1995)], etc. Sendai virus vectors that are preferably used are vectors of DNAVEC Corporation that harbor *POU5F1* gene, *SOX2* gene, *c-Myc* gene, *KLF4* gene, *LIN28* gene, or *NANOG* gene.

If a recombinant viral vector plasmid is deficient of at least one of the genes encoding the proteins necessary for virus packaging, a packaging cell may be used that is capable of compensating for that lacking protein, and examples are packaging cells based on human kidney derived HEK293 cells or mouse fibroblast cells HIH3T3. PLAT-A cells and PLAT-GP cells are preferably used as the packaging cells based on HEK293 cells.

The proteins to be compensated by packaging cells depend on the type of the viral vector to be used; in the case of a retrovial rector, retrovirus-derived proteins such as gag, pol, and env may be mentioned as examples; in the case of a lentiviral vector, HIV virus-derived proteins such as gag, pol, env, vpr, vpu, vif, tat, rev, and nef may be mentioned; and in the case of an adenoviral vector, adenovirus-derived proteins such as E1A and E1B may be mentioned.

Recombinant viral vectors can be produced by introducing the above-mentioned recombinant viral vector plasmids into the above-described packaging cells. The methods for introducing the viral vector plasmids into the packaging cells are not particularly limited if they are of known types and examples are gene transfer methods such as the calcium phosphate method (JP Hei 2-227075 A), lipofection [Proc. Natl. Acad. Sci., USA, 84, 7413 (1987)], and electroporation. In the present invention, it is particularly preferred to use transfection agents such as FuGENE HD (Roche) and FuGENE6 (Roche).

If desired, the genes of interest may be transduced using plasmids, transposon vectors, episomal vectors, etc. in place of the above-mentioned viral vectors.

In the aforementioned induction step, in order to increase the efficiency of induction to the induced malignant stem cell, compounds that are known to give rise to induced pluripotent stem cells may further be added to the culture media used to give rise to the induced malignant stem cell of the present invention, and these compounds are exemplified by inhibitors including: three low-molecular weight inhibitors of FGF receptor tyrosine kinase, MEK (mitogen activated protein kinase)/ERK (extracellular signal regulated kinases 1 and 2) pathway, and GSK (Glycogen Synthase Kinase) 3 [SU5402, PD184352, and CHIR99021]; two low-molecular weight inhibitors of MEK/ERK pathway and GSK3 [PD0325901 and CHIR99021]; a low-molecular weight compound as an inhibitor of the histone methylating enzyme G9a [BIX-01294 (BIX)], azacitidine, trichostatin A (TSA), 7-hydroxyflavone, lysergic acid ethylamide, kenpaullone, an inhibitor of TGF-β receptor I kinase/activin-like kinase 5 (ALK5) [EMD 616452], inhibitors of TGF-β receptor 1 (TGFBR1) kinase [E-616452 and E-616451], an inhibitor of Src-family kinase [EI-275], thiazovivin, PD0325901, CHIR99021, SU5402, PD184352, SB431542, anti-TGF-β neutralizing antibody, A-83-01, Nr5a2, a p53 inhibiting compound, siRNA against p53, an inhibitor of p53 pathway, ctc. If necessary, hypoxic culture may be performed to achieve efficient induction of the induced malignant stem cell of the present invention.

It is also possible to use microRNAs for the purpose of increasing the efficiency of induction to the induced malignant stem cell. Any methods commonly applied by the skilled artisan may be employed and a specific example is the introduction of microRNAs into the cells of the aforementioned mammals using expression vectors or the addition of microRNAs to media.

The method of using microRNAs for the purpose of increasing the efficiency of induction to the induced malignant stem cell may be exemplified by: the use of a miR-130/301/721 cluster; the use of a miR-302/367 cluster; the removal of miR-21 and miR-29a; the use of miRNA in mir-200c, mir-302 s and mir-369 s families; the use of miR-302b and miR-372; the use of two miRNA clusters, mir-106a-363 cluster and mir-302-367 cluster, especially the use of mir-302-367 cluster; and the use of miR-17-92 cluster, miR-106b-25 cluster, and miR-106a-363 cluster. The methods of using these microRNAs may be used either singly or in combination of two or more kinds.

Information about these microRNAs is accessible from the website of miRBase (http://www.mirbase.org/). Relating to the information on this website, accession numbers of miRBase are parenthesized.

In the step of induction to the induced malignant stem cell, the addition of the aforementioned genes may be combined with the use of the following fibroblast growth factors: FGF1 (aFGF), FGF2 (bFGF), FGF3, FGF4, FGF5, FGF6, FGF7 (KGF), FGF8, FGF9, FGF10, FGF11, FGF12, FGF 13, FGF14, FGF15, FGF16, FGF17, FGF18, FGF19, FGF20, FGF21, FGF22, FGF23, and FGF24. The fibroblast growth factors that may be used with particular preference are FGF1 (aFGF), FGF2 (bFGF), FGF4, and FGF7 (KGF). These fibroblast growth factors are selected in accordance with the species of the somatic cell to be induced and examples that can be used are the fibroblast growth factors derived from human, mouse, bovine, equine, porcine, zebrafish, etc.

In the present invention, it is preferred that the medium used in the step where the somatic cell isolated from the aforementioned mammal is induced to the induced malignant stem cell capable of *in vitro* proliferation contains at least one of the aforementioned fibroblast growth factors added thereto, and this component is preferably added to the medium in amounts of about 1-100 ng/mL.

Aside from the fibroblast growth factors, pituitary extract, serum, LIF, Z-VAD-FMK, etc. can also be used. LIF is preferably added to the medium in about 10-10,000 units and serum is preferably added to make 2-20% of the medium.

In particular, Z-VAD-FMK which is a cell-permeable, general caspase inhibitor irreversibly binds to the catalytic site of each caspase so as to inhibit the induction of apoptosis; hence, it is preferably added to the medium to give a final concentration of 0.1-100 $\mu$M.

It is also preferred to add a neutralizing antibody to the medium, as exemplified by IGF-II inhibitor, anti-IGF-II antibody, anti-IGF-R1 antibody, anti-TGF-$\beta$1 antibody, or anti-activin A antibody; particularly in the case where such a gene as *IGF-II* gene, *IGF-R1* gene, *TGF-$\beta$1* gene, or *activin A* gene is highly expressed in the induced malignant stem cell of the present invention, addition of the above-mentioned component is preferred for the purpose of maintaining the proliferation of the induced malignant stem cell.

The agent that may also be added to the medium is exemplified by the following: low-molecular weight inhibitors of each of FGF tyrosine kinase receptor, Mek/Erk pathway, and GSK, respectively [SU5402, PD184352, and CHIR99021]; a low-molecular weight inhibitor of FGF receptor [PD 173074]; a low-molecular weight inhibitor of Mek pathway [PD0325901]; a low-molecular weight inhibitor of GSK3 [BIO]; 7-hydroxyflavone; lysergic acid ethylamide; kenpaullone; inhibitors of TGF-$\beta$ receptor I kinase/activin-like kinase 5 (ALK5 inhibitor) [EMD 616452, A-83-01]; an inhibitor of Tgf-$\beta$ receptor 1 (Tgfbr1) kinase [E-616451]; an inhibitor of Src-family kinase [EI-275]; thiazovivin; SB431542; Nr5a2; Y-27632; and fasudil.

If necessary, hypoxic culture may be performed to achieve efficient induction for giving rise to the induced malignant stem cell of the present invention.

It should, however, be noted that low-molecular weight compounds that directly act upon epigenetic modification (DNA methylation and histone modification), as exemplified by lysine specific demethylating enzyme 1 inhibitor, methyltransferase [G9a] inhibitor, DNA methylating enzyme (Dnmt) inhibitor, and histone deacetylating enzyme (HDAC) inhibitor, are not preferably added to the medium because they change the epigenetic modification of the starter cell. The lysine specific demethylating enzyme 1 inhibitor may be exemplified by Parnate (also called tranylcypromin); the methyltransferase [G9a] inhibitor may be exemplified by BIX-01294; the DNA methylating enzyme (Dnmt) inhibitor may be exemplified by 5-azacitidine, RG108, and 5-aza-deoxycitidine (5-AZA); the histone deacetylating enzyme (HDAC) inhibitor may be exemplified by suberoylanilide hydroxamic acid (SAHA), trichostatin A, valproic acid (VPA), and sodium butyrate (NaB).

Specifically, butyric acid, as well as the five low-molecular weight chromatin modifying substances (i.e., 5-aza-deoxycitidine (5-AZA), RG108, BIX-01294, valproic acid (VPA), and sodium butyrate (NaB)) should preferably not be added since they change the epigenetic modification of the starter cell.

In the step of induction for giving rise to the induced malignant stem cell, culture is performed using media suitable for the culture of embryonic stem cells or induced pluripotent stem cells. Such media include the ES medium, MEF-conditioned ES medium, optimum medium for induced pluripotent stem cells, optimum medium for feeder cells, StemPro, animal protein free, serum-free medium for the maintenance of human embryonic stem cells/induced pluripotent stem cells, named TeSR2 [ST-05860], etc. that have been enumerated as typical examples of the medium for culturing the induced malignant stem cell of the present invention; it is particularly preferred to use the MEF-conditioned ES medium. If somatic cells isolated from humans are used, media suitable for the culture of human embryonic stem cells are preferably used.

If the derived cell is not a fibroblast, for example, in the case of using epithelial cells such as somatic cells derived from patients with stomach or colon cancer, co-culture is preferably performed using feeder cells seeded after gene transduction.

In addition to the above-described induction step, the process for producing the induced malignant stem cell of the present invention may further include the step of sorting a single cell in one well and proliferating the same. In this step, cells, either stained or not stained with any one specific antibody selected from the group consisting of an anti-ALB antibody, an anti-FABP1 antibody, an anti-IGF-II antibody, an anti-DLK1 antibody, an anti-PDGFR α antibody, an anti-VEGFR2 antibody, an anti-E-cadherin antibody, an anti-CXCR4 antibody, an anti-PDGFR β antibody, an anti-cadherin 11 antibody, an anti-CD34 antibody, and an anti-IGF-R1, are proliferated with a single cell being sorted in one well.

In an exemplary method, the induced malignant stem cells of the present invention are stained with one of specific antibodies against the E-cadherin and so on and, then, using PERFLOW™ Sort (Furukawa Electric Co., Ltd.), the specific antibody stained cells are single cell-sorted on a 96-well plate or the like such that one cell is contained in one well. It is also possible to use unstained cells instead of the cells stained with the specific antibody.

The process for producing the induced malignant stem cell of the present invention may further include a selection step in which the malignancy or a specific marker of the induced malignant stem cell capable *of in vitro* proliferation is identified to select the cell of interest.

The term "malignancy" as used herein refers to various properties of cancer cells such as those which are associated with their ability to proliferate without limit, infiltration, metastasis, resistance, and recurrence. The term "specific marker" refers to any one of the genomic or epigenetic aberrations (1) (a) to (1) (g) that are related to cancer. These genomic or epigenetic aberrations (1) (a) to (1) (g) that are related to cancer are detected and identified by the methods already described above.

The aforementioned step of identifying and selecting the malignancy or specific marker is performed in such a way that the induced malignant stem cell of the present invention obtained by induction treatment of a non-embryonic starter somatic cell isolated from a carcinogenic mammal that has any one of the genomic or epigenetic aberrations (1) (a) to (1) (g) which are related to cancer is compared with an induced pluripotent stem cell as induced from a reference somatic cell isolated from a mammal, or an undifferentiated embryonic stem cell. As regards the aberration in genome, it should be noted that a genomic aberration (e.g. somatic mutation) in the induced malignant cell of the present invention which has been obtained by induction treatment may be compared with the genome of a cell group (such as a group of corpuscular cells) before induction treatment which are mostly made of normal cells or one of the reference sequences registered in a public database (e.g. NCBI GeneBank). The induced malignant stem cell is theoretically a clonal cell and has a somatic mutation of a tumor suppressor gene in an endogenous genomic DNA derived from a single cancer cell or a somatic mutation in an endogenous cancer-related gene.

The above-mentioned reference somatic cell isolated from a mammal is not particularly limited if it is a somatic cell isolated from various tissues of the mammal at various stages. Such various mammalian tissues may be exemplified by the various tissues listed earlier as examples of the tissues from which the starter somatic cell is obtained and used to prepare the aforementioned induced malignant stem cell of the present invention.

The above-mentioned reference somatic cell isolated from a mammal may be a normal cell in a healthy individual, a normal cell derived from a healthy neonate (either animal or human), or a normal cell derived from a healthy neonatal (either animal or human) skin; moreover, even somatic cells in a carcinogenic mammal can be used if they are non-cancer cells that are substantially free of aberrations or normal cells in the carcinogenic individual. It is especially preferred to use those somatic cells which are derived from healthy individuals, neonates (either animal or human), or neonatal (either animal or human) skins since these are considered to be substantially free of the various aberrations that are found in the starter somatic cell to be used in the present invention.

It should be noted here that since it is difficult to select only a single normal cell or non-cancer cell from a tissue and isolate the same to prepare an iPS cell, a cell group that is recognized to be a normal tissue is used in practice.

If the starter somatic cell is a cancer cell in a carcinogenic mammal, a normal or a non-cancer cell in the same individual as the carcinogenic mammal is preferably used as the aforementioned reference somatic cell isolated from a mammal. In particular, if a normal cell and a non-cancer cell isolated from the same organ in the same individual are used, the difference in malignancy between these two cells (i.e., the starter somatic cell and the reference somatic cell) is distinct because of the commonality of the features that are unique to the individual or organ. Hence, the above-described step of making comparison with the tissue of the same individual as the one from which the starter somatic cell has been isolated does more than identifying the malignancy or specific marker of the induced malignant stem cell; it also serves as a useful analysis tool that may be applied to identify carcinogenic mechanisms and its utility even covers use as a method of screening for a target in the discovery of cancer therapeutic drugs (for details, see below.)

As already noted, it is difficult to isolate only a single cell from a tissue, so a cell group in a normal tissue or a non-cancer tissue in a carcinogenic mammal is used in practice.

As in the case of the starter somatic cell, the reference somatic cell isolated from a mammal is preferably a somatic cell in a fresh tissue or a frozen tissue.

The mammal from which the reference somatic cell is to be isolated is preferably a human and, in a particularly preferred case, it is the same as the individual from which the starter somatic cell has been isolated.

In addition, the induced pluripotent stem cell as induced from the reference somatic cell isolated from a mammal is not particularly limited if it been induced from the above-described reference somatic cell isolated from a mammal, but those which are obtained by the same method of induction (in such terms as the genetic set and culture medium) as employed to give rise to the induced malignant stem cell of the present invention are preferably used.

In addition, the induced pluripotent stem cell as induced from the reference somatic cell isolated from a mammal is not particularly limited if it has been prepared by known methods of giving rise to induced pluripotent stem cells, but those which are obtained by the same method of induction as employed to give rise to the induced malignant stem cell of the present invention are preferably used. Other examples that can be used include: the induced pluripotent stem cells that are described in Patent Documents 1 and 2, as well as in "Methods of establishing human iPS cells", Center for iPS Cell Research and Application, Institute for Integrated Cell-Material Sciences, Kyoto University, CiRA/M&M, p. 1-14, 2008, 7.4; induced pluripotent stem cells that are available from known supply sources such as RIKEN BioResource Center and Kyoto University; and known gene expression data for induced pluripotent stem cells that are available from the aforementioned Gene Expression Omnibus [GEO].

Further in addition, undifferentiated embryonic stem cells can also be used as the reference for comparison and any such cells that have been prepared by known methods can be used. It is also possible to use undifferentiated embryonic stem cells as obtained by the methods descried in Thomson JA et al., "Embryonic stem cell lines derived from human blastocysts", Science, 1998 Nov 6, 282 (5391): 1145-7, Erratum in Science, 1998 Dec 4, 282 (5395): 1827 and Hirofumi Suemori et al., "Efficient establishment of human embryonic stem cell lines and long term maintenance with stable karyotype by enzymatic bulk passage", Biochemical and Biophysical Research Communications, 345, 926-32 (2006)); undifferentiated embryonic stem cells as available from known supply sources such as RIKEN BioResource Center and Institute for Frontier Medical Sciences, Kyoto University; and known gene expression data such as hES_H9 (GSM194390), hES_BG03 (GSM194391), and hES_ES01 (GSM194392). These gene expression data are available from the aforementioned Gene Expression Omnibus [GEO].

The aforementioned step of identifying and selecting the malignancy or specific marker is such that both the cell obtained by subjecting the starter somatic cell to induction treatment and the induced pluripotent stem cell as induced from the reference somatic cell isolated from a mammal or an undifferentiated embryonic stem cell are subjected to genomic analysis, epigenome analysis, transcriptome analysis, proteome analysis, cell surface antigen analysis, sugar chain analysis (glycome analysis), metabolic analysis (metabolome analysis), post-analysis following transplanting into laboratory animal, and the like, and the malignancy or specific marker of the induced malignant stem cell is identified on the basis of the results of these analyses and if identified as "malignant", it is selected as the induced malignant stem cell of the present invention.

Therefore, if the induced malignant stem cell capable of *in vitro* proliferation that has been prepared by the method of the present invention is subjected to omics analyses (genomic analysis, epigenome analysis, transcriptome analysis, proteome analysis, glycome analysis, and metabolome analysis), there can be identified a methylator phenotype, a mutator phenotype, a driver mutation, or a target in the discovery of cancer therapeutic drugs, all being characteristic of cancer. Such cancer-characteristic methylator phenotype, mutator phenotype, driver mutation, or target in the discovery of cancer therapeutic drugs can be used to screen for pharmaceutical candidates such as low-molecular weight compounds, antibodies or siRNAs and, consequently, pharmaceutical candidates can be provided.

The term "genomic analysis" as used hereinabove means an analysis that determines all genomic nucleotide sequences in a particular species of organism. Specifically, the entire nucleotide sequences in the genome are determined and all genes described in the genome are identified to eventually determine the amino acid sequences. To determine the entire nucleotide sequences in the genome, analysis is performed by genome sequencing and other techniques.

If mutations are noted and identified in genes that have such properties as are associated with the ability of cancer cells to proliferate without limit, infiltration, metastasis, resistance, and recurrence, the induced malignant stem cell of the present invention is selected as such. It suffices for the purposes of the present invention that mutations are noted in oncogenes, tumor suppressor genes, or genes having such properties as are associated with the ability of cancer cells to proliferate without limit, infiltration, metastasis, resistance, and recurrence, and there is no need to perform analysis for the entire genome.

Epigenome analysis refers to analyses for DNA methylation and histone modification, which are chemical modifications that do not directly affect the DNA of genes but alter the expression of genes.

The induced malignant stem cell of the present invention can also be selected as such if, in comparison with the reference cell, abnormal expression is noted and identified in genes having such properties as are associated with the ability of cancer cells to proliferate without limit, infiltration, metastasis, resistance, and recurrence, or in cancer-related genes or tumor suppression-related genes.

Transcriptome analysis refers to the analysis of all mRNAs (or the primary transcripts) that are found in a single organism cell or proliferated, similarly differentiated cells of organism under given biological conditions of cell. Since mRNA generates various abberations on account of accumulating extracellular effects that occur in the process of development, transcriptome analysis makes it possible to determine the properties of the current cell in detail. Specifically, transcriptome analysis is performed using microarrays and the like.

For example, the induced malignant stem cell of the present invention can be selected as such if mRNAs involved in the ability of cancer cells to proliferate without limit, infiltration, metastasis, resistance, and recurrence, or mRNAs corresponding to mutated oncogenes, mRNAs corresponding to mutated tumor suppressor genes, or mRNAs corresponding to cancer-related genes are found in said cell in greater amounts than in the reference cell.

Proteome analysis refers to a large-scale analysis of proteins that specifically relates to their structures and functions and it analyzes the set of all proteins that a certain organism has or the set of all proteins that a certain cell expresses at a certain moment.

For example, the induced malignant stem cell of the present invention can be selected as such if, after separately culturing the induced malignant stem cell and the reference cell, an analysis of the proteins extracted after secretion from the respective cells shows that proteins involved in the ability of cancer cells to proliferate without limit, infiltration, metastasis, resistance, and recurrence are found in the induced malignant stem cell in greater amounts than in the reference cell.

Cell surface antigen analysis involves analyzing various molecules, commonly called surface antigens or surface markers, which are made of proteins or glycoproteins that are expressed on the cell surface.

For example, the induced malignant stem cell of the present invention can be selected as such if a cell surface antigen analysis shows that surface antigens specific to cancer cells are expressed in it.

Sugar chain analysis (glycome analysis) involves analyzing sugar chains that cover like fuzzy hairs the entire surface of proteins or lipids that are found on the cell membrane at the cell surface. Unlike ordinary saccharides, sugar chains make up the sugar moiety of a glycoconjugate (composed of glycoproteins, glycolipids, and proteoglycans.) These sugar chains are composed of sialic acid, glucose, galactose, mannose, fucose, N-acetylgalactosamine, N-acetylglucosamine, etc.

For example, the induced malignant stem cell of the present invention can be selected as such if sugar chains specific to cancer cells are found in it as the result of a sugar chain analysis (glycome analysis).

Metabolome analysis means comprehensive analysis of metabolites and generally involves the separation and identification of organic compounds (metabolites) by chromatography, spectrometer, or other measurement instruments. The induced malignant stem cell of the present invention can be selected as such if, after separately culturing the induced malignant stem cell and a reference cell, analysis of the organic compounds (metabolites) isolated after secretion from the respective cells shows that organic compounds (metabolites) that are involved as in the ability of cancer cells to proliferate without limit, infiltration, metastasis, resistance, and recurrence are found in the induced malignant stem cell in greater amounts than in the reference cell.

Cancer cells as induced from the induced malignant stem cell

In its second aspect, the present invention provides a cancer cell as induced from the induced malignant stem cell according to the first aspect of the present invention. The cancer cell according to the second aspect of the present invention is not particularly limited if it is a cancer cell obtained by induction from the induced malignant stem cell according to the first aspect of the present invention.

Specifically, if the above-described media to be used in expansion culture or passage culture or the media used in the step of induction for giving rise to the induced malignant stem cell have added thereto a matrix (e.g. collagen, gelatin, or matrigel), a neutralizing antibody such as anti-TGF-β1 antibody, anti-activin A antibody, anti-IGF-II antibody, or anti-IGF-R1 antibody, an IGF inhibitor, or a fibroblast growth factor such as bFGF, induction to cancer cells can be accomplished by performing culture in media from which those components have been removed. Cancer cells can also be induced by culturing in a non-ES medium, such as Dulbecco's modified medium supplemented with 10% serum, for about one week or longer. Induction for differentiation into cancer cells can also be realized by removing feeder cells or through suspension culture. In the case of preparing cancer model animals as will be described later, the induced malignant stem cell of the present invention may be directly transplanted to a laboratory animal, which is induced to cancer cells.

Methods of screening using the induced malignant stem cell

In its third aspect, the present invention provides a method of screening characterized by using the induced malignant stem cell according to its first aspect or the cancer cell as induced therefrom, and it is advantageously used as a method of screening for a target in the discovery of a cancer therapeutic drug, a method of screening for a candidate for a cancer therapeutic drug, or as a method of screening for a cancer diagnostic drug.

The screening method of the present invention preferably involves a step of contacting the test substance with both the induced malignant stem cell of the present invention and an induced pluripotent stem cell as induced from the reference somatic cell isolated from a mammal, or an undifferentiated embryonic stem cell.

In the case where this method is used to screen for a target in the discovery of a cancer therapeutic drug, it may be the same as the step in the production process of the present invention where the malignancy or specific marker of the induced malignant stem cell is identified and selected. To be more specific, a gene or protein that is a potential target in the discovery of a cancer therapeutic drug can be searched for by comparing the induced malignant stem cell of the present invention or the cancer cell as induced therefrom with an induced pluripotent stem cell as induced from the reference somatic cell isolated from a mammal, or an undifferentiated embryonic stem cell.

Following the search, antisense RNA, siRNA, low-molecular weight compounds, peptides or antibodies that suppress the expression of a gene as a putative target in the discovery of a cancer therapeutic drug are added to a culture dish on which the induced malignant stem cell of the present invention or the cancer cell induced therefrom has been cultured and thereafter the properties and the like of the cell are examined to determine if the gene can be used as a target in the discovery of a cancer therapeutic drug.

In the case where the method of interest is used to screen for a candidate for a cancer therapeutic drug, a medicine that is a candidate for an anti-cancer agent or vaccine (e.g. anti-cancer vaccine) is added to a culture dish on which the induced malignant stem cell of the present invention or the cancer cell induced therefrom has been cultured and thereafter the properties and the like of the cell are evaluated to determine the pharmaceutical efficacy of the medicine.

More specifically, it is possible to verify usefulness as anti-cancer agents by performing an anti-tumor test, a cancer metastasis test, a drug resistance test, a drug metabolism test, as well as metabolizing enzyme induction/inhibition tests using the induced malignant stem cell of the present invention or the cancer cell induced therefrom.

In the case where the method of interest is used to screen for a cancer diagnostic drug, the question of whether a certain cancer diagnostic drug is duly effective can be evaluated by adding to it various types of the induced malignant stem cell of the present invention or the cancer cell induced therefrom and checking to see if they are accurately diagnosed as cancerous.

Method of preparing an anti-cancer vaccine using the induced malignant stem cell

In its fourth aspect, the present invention provides a method of preparing an anti-cancer vaccine using the induced malignant stem cell according to its first aspect or the cancer cell as induced therefrom.

More specifically, anti-cancer vaccines useful in CTL therapy, dendritic cell therapy, cancer peptide vaccine therapy, and other therapies can be prepared by using the induced malignant stem cell of the present invention or the cancer cell as induced therefrom.

CTL (cytotoxic T-lymphocyte) therapy is a therapeutic method in which lymphocytes isolated from a patient are activated through their learning of the features of the cancer to be attacked and then a large amount of the cytotoxic T lymphocytes (CTL cells) are returned to the body of the patient.

In CTL therapy, learning of lymphocytes is generally achieved by using the antigen of cancer cells present in the patient or by using an artificial antigen. Using the antigen of cancer cells present in the patient is considered to have the greater efficacy. However, the need for isolating cancer cells exerts a great physical burden on the patient and, what is more, the isolated cancer cells need to be preliminarily proliferated to an adequate number *ex vivo,* but then they are difficult to culture; hence, this method is only applicable in the case where a relatively large tumor mass has been excised by surgery and the antigen isolated successfully.

The induced malignant stem cell of the present invention is capable *of in vitro* proliferation, so induced malignant stem cells or cancer cells as induced therefrom can be made available in the required amount and, in addition, the physical burden to be exerted on the cancer patient by the process of isolating cancer cells can be sufficiently reduced to provide significant utility.

In a more specific production process, T cells capable of attacking cancer cells are extracted from a patient's blood as by component blood sampling, to which the induced malignant stem cells of the present invention or cancer cells as induced therefrom, a lysate of these cells, as well as a cancer antigen protein or peptide obtained on the basis of these cells are added, so that the T cells will learn the cancer antigen. Subsequently, the T cells are activated by an anti-CD3 antibody or the like and then cultured in the presence of interleukin 2 or the like to prepare a large amount of cytotoxic T lymphocytes which can serve as an anti-cancer vaccine. In the case where induced malignant stem cells or cancer cells as induced therefrom or a lysate of these cells is used as a cancer antigen, a preferred source of supply for the induced malignant stem cells is a cancer tissue excised by surgery from the patient to be treated or cancer cells isolated from the ascites or the like of the patient.

Dendritic cell therapy is a therapeutic method in which dendritic cells isolated from the patient are caused to learn the features of the cancer to be attacked and are then returned to the body of the patient; the dendritic cells returned to the patient's body stimulate the T lymphocytes so that they become killer T cells which in turn attack the cancer cells for cancer treatment.

This therapeutic method has the same problem as the aforementioned CTL therapy in that it is only applicable in the case where a relatively large tumor mass has been excised by surgery and the antigen isolated successfully. In contrast, the induced malignant stem cell of the present invention is capable *of in vitro* proliferation, so the induced malignant stem cells or cancer cells induced therefrom can be made available in the required amount and, in addition, the physical burden to be exerted on the cancer patient by the process of isolating cancer cells can be sufficiently reduced to provide significant utility.

In a more specific production process, dendritic cells are extracted from the samples obtained by component blood sampling, to which induced malignant stem cells or cancer cells as induced therefrom, a lysate of these cells, as well as a cancer antigen protein or peptide obtained on the basis of these cells are added, so that they will learn the cancer antigen to become an anti-cancer vaccine. In the case where induced malignant stem cells or cancer cells as induced therefrom or a lysate of these cells is used as a cancer antigen, a preferred source of supply for induced malignant stem cells is a cancer tissue excised by surgery from the patient to be treated or cancer cells isolated from the ascites or the like of the patient.

The aforementioned dendritic cells are such that even a single dendritic cell is capable of stimulating from several hundred to several thousand lymphocytes, so the therapeutic method in which the dendritic cells are caused to learn the features of the target cancer and then returned to the body of the patient is believed to be extremely efficient. However, dendritic cells account for only about 0.1 to 0.5% of leucocytes in number, so instead of using them directly, monocytes that are abundant in the blood and which can change to dendritic cells are acquired in large quantities by a separated component blood sampling method and cultured in the presence of a cell stimulating substance such as cytokine to grow into dendritic cells for use in therapy.

Cancer peptide vaccine therapy is a therapeutic method in which a peptide (peptide vaccine) as a specific antigen possessed by cancer cells is injected into the patient so that the immunity of the patient is sufficiently enhanced to suppress tumor growth. Specifically, when the peptide (a small one with a sequence of 9 or 10 amino acids) is administered into the body of the patient, killer T cells stimulated by the peptide are activated and further proliferated to become capable of attacking the cancer cells; cancer peptide vaccine therapy uses this nature of the peptide to eliminate (regress) the cancer.

Since the induced malignant stem cell of the present invention is capable *of in vitro* proliferation and enables various types of induced malignant stem cells to be amplified in large quantities, the induced malignant stem cell of the present invention prepared from cancer cells or cancer tissues derived from various cancer patients can be cultured in large quantities to prepare the desired anti-cancer vaccines. The thus obtained anti-cancer vaccines can also be used in CTL therapy or dendritic cell therapy.

The anti-cancer vaccines described above are extremely useful in preventive cancer therapy or for preventing possible recurrence after the application of standard therapies including chemotherapy, radiation therapy, and surgical therapy.

Method of preparing a cancer model animal using the induced malignant stem cell

In its fifth aspect, the present invention provides a method of preparing a cancer model animal using the induced malignant stem cell according to its first aspect or cancer cells as induced therefrom.

According to the method of preparing a cancer model animal of the present invention, the induced malignant stem cell of the present invention or cancer cells as induced therefrom may be transplanted to laboratory animals such as mouse to thereby prepare tumor bearing mice, which are then administered with an anti-cancer agent, an antibody, a vaccine and the like; their pharmacological efficacy can be verified by subjecting the tumor bearing mice to a blood test, a urine test, autopsy, and the like.

The induced malignant stem cell of the present invention or cancer cells as induced therefrom can be used for various other applications than in the aforementioned methods of screening, methods of preparing anti-cancer vaccines, and methods of preparing cancer model animals.

For example, secretory proteins and membrane proteins are screened genome-widely from the genetic information about induced malignant stem cells or cancer cells as induced therefrom and those secretory proteins and membrane proteins that are specific for the induced malignant stem cell of the present invention or cancer cells as induced therefrom and which hence are useful as cancer diagnostic markers are identified to prepare therapeutic or diagnostic antibodies. An exemplary method for exhaustive screening of secretory proteins and membrane proteins is the "signal sequence trapping method" (Japanese Patent Nos. 3229590 and 3499528) which is characterized by gene identification targeted to a signal sequence that is common to the secretory proteins and membrane proteins.

In addition, by performing sugar-chain structural analysis on the induced malignant stem cell of the present invention or cancer cells as induced therefrom, sugar chains that are specific for the induced malignant stem cell of the present invention or cancer cells as induced therefrom and which hence are useful as cancer diagnostic markers are identified to prepare therapeutic or diagnostic antibodies, as well as natural or artificial lectins.

An exemplary process of sugar-chain structural analysis is described below. First, from an expression profile of sugar-chain genes produced by the induced malignant stem cell of the present invention or cancer cells as induced therefrom, sugar-chain structures characteristically produced by cancer cells are estimated and, at the same time, the sugar chains which are actually produced and secreted as glycoproteins are subjected to lectin microarray analysis and the lectins or anti-sugar chain antibodies that react with the sugar chains characteristically produced by cancer cells are selected as probes. Subsequently, with using the selected probes, a group of glycoproteins (or their fragmentary glycopeptides) that have cancerous sugar chains are captured from among the glycoproteins secreted by the cancer cells and structures of their core glycoproteins are identified by a MS-based method such as IGOT. From a culture medium of the induced malignant stem cell of the present invention or cancer cells as induced therefrom, the identified glycoproteins are purified and checked again for any changes in the sugar-chain structure by a lectin-array based method, whereby a candidate for a sugar-chain disease marker can be located.

Mannan-binding proteins (MBP) which are calcium-dependent lectins that are found in various mammals are known to selectively bind to certain types of cancer cells and exhibit a cytotoxic action; ligand sugar chains that specifically bind to MBP have been isolated from the human colon cancer cell strain SW1116. Thus, ligand sugar chains or the like which bind to serum lectins that are specifically expressed in the induced malignant stem cell of the present invention or cancer cells as induced therefrom are identified and clonal antibodies against the identified ligand sugar chains can be prepared. The thus obtained antibodies are also useful as therapeutic or diagnostic antibodies.

As further applications of the induced malignant stem cells of the present invention, there are provided a genome, epigenome (DNA methylome), transcriptome, proteome, total sugar chains (glycome), and metabolome that can be used in various analyses of these cells, including genomic analysis, epigenome analysis, transcriptome analysis, proteome analysis, cell surface antigen analysis, sugar-chain analysis (glycome analysis), and metabolome analysis. Also provided is the information acquired by these analyses which comprises DNA methylation information, profiling of histone modification, genome-wide RNA expression information (transcriptome information), protein expression information (proteome information), lectin-binding profiling information, and metabolome information; these kinds of information are applicable in drug discovery.

For example, it is also possible to search for and identify targets in the discovery of cancer therapeutic drugs on the basis of mRNAs, microRNAs or total RNAs containing non-coding RNAs that are expressed in the induced malignant stem cell of the present invention.

On the pages that follow, the present invention is illustrated more specifically by means of Examples but it should be understood that the scope of the present invention is by no means limited by those Examples.

EXAMPLES

Example 1: Preparation of induced malignant stem cells from cells (GC2) derived from cancer tissues of a gastric cancer patient

The fresh cancer tissues of a gastric cancer patient of donor No. 1 (medical information: a 67-year-old Japanese woman with a gastric caner, blood type O, no chemotherapy, no radiotherapy, no immunosuppressive therapy, no smoking history, no drinking history, no drug addiction, no drug therapy, HIV-negative, HCV-negative, HBV-negative, syphilis-negative) which had been refrigerated for several hours and transported in a preservation solution (Hanks' solution supplemented with kanamycin and Fungizone) were used to isolate cells (GC2). The fresh non-cancer tissues of the patient were also used to isolate cells (NGC2). To the resultant cells derived from the gastric (solid) cancer tissues, the solution of the four Sendai viral vectors containing any of four genes (POU5F1, KLF4, SOX2, c-Myc) (DNAVEC CytoTune iPS (DV-0301-1)) was added for genetic transduction, whereby human induced malignant stem cells were prepared from the gastric (solid) cancer tissues. The details of the procedure are as described below. The Sendai viral vector is an RNA viral vector that does not insert an exogenous DNA into the genomic DNAs of cells.

Part (0.5-1 g) of the gastric (solid) cancer tissues obtained during operation was washed with Hank's balanced salt solution (Phenol Red-free) (Invitrogen; Cat No. 14175-095) and minced with scissors into pieces of about 0.1-1 mm$^2$. The pieces were further washed with Hank's balanced salt solution (Phenol Red-free) until a supernatant became clear. Then, after removal of the supernatant, 3 mL of the DMEM medium (Invitrogen) supplemented with 0.1% collagenase (Wako Pure Chemical; Cat No. 034-10533) and 1X antibiotic/antimycotic (Invitrogen; anti-anti) was added to the tissue precipitate, and stirring was performed at 37°C for 90 minutes with a shaker.

After confirming that the precipitated tissue has been fully digested, 35 mL of a D-MEM (high glucose) medium supplemented with 1X antibiotic/antimycotic and 10% FBS was added, and the suspension was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, and the suspension was centrifuged again at 1000 rpm at 4°C for 5 minutes. Then, after removal of the supernatant, 10 mL of a D-MEM (high glucose) medium supplemented with 1X antibiotic/antimycotic and 10% FBS was added to part of the cells, and the cell suspension was seeded on a matrigel (BD; Cat No. 356234)-coated culture dish (100 mm) (coated for an hour with 60 $\mu$L matrigel/6 mL/60 cm$^2$ PBS) to conduct primary culture. The remaining cells were stored in liquid nitrogen while being suspended in a preservation solution. At a later date, part of the cells was thawed and subjected to primary culture.

After one day culture, the solution of the four Sendai viral vectors containing any of four genes (POU5F1, KLF4, SOX2, c-Myc) was added, and the suspension was infected at 37°C for one day. The viral supernatant was removed, and mitomycin-treated mouse embryonic fibroblasts (MEFs) as feeder cells were suspended in 10 mL of a D-MEM (high glucose) medium supplemented with 1X antibiotic/antimycotic and 10% FBS, and the cell suspension was then seeded at a density of $1.5 \times 10^6$ cells/60 cm$^2$ on the matrigel-coated culture dish (100 mm) in which the transduced cells derived from the cancer tissues of the gastric cancer patient had been cultured, whereby co-culture was performed.

Thereafter, the medium was replaced every one to three days with the ReproCell ReproStem medium (supplemented with 10 ng/mL bFGF, 1X antibiotic/antimycotic, and 10 μg/mL gentamicin) or the STEMCELL Technologies medium for a feeder cell-free culture of human ES/iPS cells, mTeSR1 (supplemented with 1X antibiotic/antimycotic and 10 μg/mL gentamicin). The MEFs were seeded at a density of $1.5 \times 10^6$ cell/60 cm$^2$ about once a week.

Gentamicin (Invitrogen; Cat No. 15750-060)

bFGF (PeproTech; Cat No. 100-18B)

anti-anti (antibiotic/antimycotic) (Invitrogen)

At least one month after the genetic transduction, colonies of eight clones (GCK2_1, GC2_2, GC2_4, GC2_5, GC2_7, GC2_10, GC2_13, GC2_16) were picked up and subjected to passage culture onto a gelatin- or matrigel-coated 24-well plate on which MEFs had been seeded. The MEFs as feeder cells, which are mitomycin-treated mouse embryonic fibroblasts, had been seeded in a gelatin- or matrigel-coated 24-well plate at a density of $1.5 \times 10^6$ cell/24-well plate the day before the pickup of the induced malignant stem cells.

After 7 to 10 days culture, the human induced malignant stem cells proliferated in the 24-well plate (passage 1) were subjected to passage culture onto 6-well plates (passage 2). Seven to ten days after the second passage, the human induced malignant stem cells proliferated in the 6-well plates (passage 2) were subjected to passage culture onto 10 cm culture dishes (passage 3). Seven to ten days after the third passage, part of the human induced malignant stem cells proliferated in the 10 cm culture dishes (passage 3) was subjected to passage culture onto 10 cm culture dishes (passage 4) and the remainder was cryopreserved. Four to ten days after the fourth passage, part of the human induced malignant stem cells proliferated in the 10 cm culture dishes (passage 4) was subjected to passage culture onto 10 cm culture dishes (passage 5) and the remainder was cryopreserved. The culture dishes had been coated with gelatin or matrigel before use.

The genomic DNAs of the cells derived from the cancer tissues of the gastric cancer patient, the cells derived from the non-cancer tissues of the gastric cancer patient, and the human induced malignant stem cells derived from the gastric cancer cancer tissues of the gastric cancer patient were purified using Qiagen DNeasy Blood & Tissue Kit (Cat. No. 69504), and the total RNAs of these cells were purified using Qiagen miRNeasy Mini Kit (Cat. No. 217004). Cryopreservation of the cell was performed by the following procedure.

The medium was removed from the cells, which were then washed with 10 mL of PBS (-) in a 100 mm-diameter (about 60 cm$^2$) culture dish, and thereafter 3 mL of a dissociation solution was added to the 10 cm (about 60 cm$^2$) culture dish. The dissociation solution used for passage culture was a 0.25% trypsin/1 mM EDTA solution (Invitrogen; Cat No. 25200-056).

After placing at 37°C for 3-5 minutes, the dissociation solution was removed, 17 mL of the ReproStem medium (10 ng/mL bFGF-free) was added, and the suspension was then centrifuged at 1000 rpm at 4°C for 5 minutes. Next, after removing the supernatant, 2 mL of a cryopreservation solution was added, and the suspension was dispensed into four serum tubes. Thereafter, the serum tubes were placed into an animal cell freezing container (BICELL), freezed at -80°C overnight, and then stored in liquid nitrogen. The cryopreservation solution used was TC-Protector (DS Pharma Biomedical Co.Ltd.).

As described above, the induced malignant stem cells (having no exogenous DNA inserted into their genomic DNAs) derived from the cancer tissues of the gastric cancer patient could be prepared with MEFs in a gelatin- or matrigel-coated culture dish using mTeSR1 or ReproStem (supplemented with 10 ng/mL bFGF) and proliferated in vitro. The culture just before the collection of genomic DNAs or total RNAs was conducted for the induced malignant stem cells in a feeder cell-free, matrigel (BD; Cat No. 356234)-coated (60 μL/60 cm$^2$) culture dish using mTeSR1.

Example 2: Preparation of human induced malignant stem cells from cells (CC3) derived from cancer tissues of a colon cancer patient

The fresh cancer tissues of a colon cancer patient of donor No. 2 (medical information: a 77-year-old Japanese man with a sigmoidal colon caner, blood type A, no chemotherapy, no radiotherapy, no immunosuppressive therapy, no smoking history, drinking history: 1 bottle of beer/day, no drug addiction, no drug therapy, HIV-negative, HCV-negative, HBV-negative, syphilis-negative) which had been refrigerated for several hours and transported in a preservation solution (Hanks' solution supplemented with kanamycin and Fungizone) were used to isolate cells (CC3). The fresh non-cancer tissues of the same donor were also used to isolate cells (NCC3). To the resultant cells derived from the cancer tissues of the colon cancer patient, the solution of the four Sendai viral vectors containing any of four genes (POU5F1, KLF4, SOX2, c-Myc) (DNAVEC CytoTune iPS (DV-0301-1)) was added for genetic transduction, whereby human induced malignant stem cells were prepared from the colon (solid) cancer tissues. The details of the procedure are as described below. The Sendai viral vector is an RNA viral vector that does not insert an exogenous DNA into the genomic DNAs of cells.

Part (0.5-1 g) of the colon (solid) cancer tissues obtained during operation was washed with Hank's balanced salt solution (Phenol Red-free) (Invitrogen; Cat No. 14175-095) and minced with scissors into pieces of about 0.1-1 $mm^2$. The pieces were further washed with Hank's balanced salt solution (Phenol Red-free) until a supernatant became clear. Then, after removal of the supernatant, 3 mL of the DMEM medium (Invitrogen) supplemented with 0.1% collagenase (Wako Pure Chemical; Cat No. 034-10533) and 1X antibiotic/antimycotic (Invitrogen; anti-anti) was added to the tissue precipitate, and stirring was performed at 37°C for 90 minutes with a shaker.

After confirming that the precipitated tissue has been fully digested, 35 mL of a D-MEM (high glucose) medium supplemented with 1X antibiotic/antimycotic and 10% FBS was added, and the suspension was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, and the suspension was centrifuged again at 1000 rpm at 4°C for 5 minutes. Then, after removal of the supernatant, 10 mL of a D-MEM (high glucose) medium supplemented with 1X antibiotic/antimycotic and 10% FBS was added to part of the cells, and the cell suspension was seeded on a matrigel (BD; Cat No. 356234)-coated culture dish (100 mm) (coated for an hour with 60 $\mu$L matrigel/6 mL/60 $cm^2$ PBS) to conduct primary culture. The remaining cells were stored in liquid nitrogen while being suspended in a preservation solution. At a later date, part of the cells was thawed and subjected to primary culture.

After one day culture, the solution of the four Sendai viral vectors containing four genes (POU5F1, KLF4, SOX2, c-Myc) was added, and the suspension was infected at 37°C for one day. The viral supernatant was removed, and mitomycin-treated mouse embryonic fibroblasts (MEFs) as feeder cells were suspended in 10 mL of a D-MEM (high glucose) medium supplemented with 1X antibiotic/antimycotic and 10% FBS, and the cell suspension was then seeded at a density of $5.0 \times 10^6$ cells/60 $cm^2$ on the matrigel-coated culture dish (100 mm) in which the transduced cells derived from the cancer tissues of the colon cancer patient had been cultured, whereby co-culture was performed.

Thereafter, the medium was replaced every one to three days with the ReproStem medium (supplemented with 10 ng/mL bFGF, 1X antibiotic/antimycotic, and 10 $\mu$g/mL gentamicin) or mTeSR1 (supplemented with 1X antibiotic/antimycotic and 10 $\mu$g/mL gentamicin). The MEFs were seeded at a density of $1.5 \times 10^6$ cell/60 $cm^2$ about once a week.

At least one month after the genetic transduction, colonies of two clones (CC3_5, CC3_6) were picked up and subjected to passage culture onto a gelatin- or matrigel-coated 24-well plate on which MEFs had been seeded. The MEFs as feeder cells, which are mitomycin-treated mouse embryonic fibroblasts, had been seeded in a gelatin- or matrigel-coated 24-well plate at a density of $1.5 \times 10^6$ cell/24-well plate the day before the pickup of the induced malignant stem cells.

After 7 to 10 days culture, the human induced malignant stem cells proliferated in the 24-well plate (passage 1) were subjected to passage culture onto 6-well plates (passage 2). Seven to ten days after the second passage, the human induced malignant stem cells proliferated in the 6-well plates (passage 2) were subjected to passage culture onto 10 cm culture dishes (passage 3). Seven to ten days after the third passage, part of the human induced malignant stem cells proliferated in the 10 cm culture dishes (passage 3) was subjected to passage culture onto 10 cm culture dishes (passage 4) and the remainder was cryopreserved. Four to ten days after the fourth passage, part of the human induced malignant stem cells proliferated in the 10 cm culture dishes (passage 4) was subjected to passage culture onto 10 cm culture dishes (passage 5) and the remainder was cryopreserved. The culture dishes had been coated with gelatin or matrigel before use.

The genomic DNAs of the cells derived from the cancer tissues of the colon cancer patient, the cells derived from the non-cancer tissues of the colon cancer patient, and the human induced malignant stem cells derived from the cancer tissues of the colon cancer patient were purified using Qiagen DNeasy Blood & Tissue Kit (Cat. No. 69504), and the total RNAs of these cells were purified using Qiagen miRNeasy Mini Kit (Cat. No. 217004). Cryopreservation of the cell performed in Examples of the present invention is as described above.

The induced malignant stem cells (having no exogenous DNA inserted into their genomic DNAs) derived from the cancer tissues of the colon cancer patient could be prepared with MEFs in a gelatin- or matrigel-coated culture dish using mTeSR1 or ReproStem (supplemented with 10 ng/mL bFGF) and *proliferated in vitro*. The culture just before the collection of genomic DNAs or total RNAs was conducted for the induced malignant stem cells in a feeder cell-free, matrigel (BD; Cat No. 356234)-coated (60 $\mu$L/60 $cm^2$) culture dish using mTeSR1.

Cryopreservation of the cell is as described above.

Example 3: Preparation of retroviral vectors

The plasmids of the three retroviral vectors containing any of three genes, POU5F1-pMXs, KLF4-pMXs, and SOX2-pMXs, were transduced into Plat-GP cells (packaging cells for preparing a pantropic retroviral vectors) using Fugene HD (Roche; Cat No. 4709691) to thereby prepare solutions of the retroviral vectors. The details of the procedure are as described below.

<Preparation of a retroviral vector solution for transducing the genes into cells (GC1) derived from gastric cancer tissues>

POU5F1-pMXs, KLF4-pMXs, and SOX2-pMXs were the constructed vectors (Table 9).

The amounts of the respective vectors were as follows: 5 $\mu$g of POU5F1-pMXs, 2.5 $\mu$g of KLF4-pMXs, 1.25 $\mu$g of SOX2-pMXs, 1.25 $\mu$g of Venus-pCS2, 5 $\mu$g of VSV-G-pCMV, 1.25 $\mu$g of GFP-pMXs (Cell Biolab), and 45 $\mu$L of FuGENE HD.

<Preparation of a retroviral vector solution for transducing the genes into cells (NGC1) derived from non-gastric cancer tissues>

POU5F1-pMXs, KLF4-pMXs, and SOX2-pMXs were the constructed vectors (Table 9).

The amounts of the respective vectors were as follows: 5 $\mu$g of POU5F1-pMXs, 2.5 $\mu$g of KLF4-pMXs, 1.25 $\mu$g of SOX2-pMXs, 1.25 $\mu$g of Venus-pCS2, 5 $\mu$g of VSV-G-pCMV, 1.25 $\mu$g of GFP-pMXs, and 45 $\mu$L of FuGENE HD.

<Preparation of a retroviral vectors solution for transducing the genes into cells (CC1) derived from colon cancer tissues>

POU5F1-pMXs, KLF4-pMXs, and SOX2-pMXs were the constructed vectors (Table 9).

The amounts of the respective vectors were as follows: 5 $\mu$g of POU5F1-pMXs, 2.5 $\mu$g of KLF4-pMXs, 1.25 $\mu$g of SOX2-pMXs, 1.25 $\mu$g of Venus-pCS2, 5 $\mu$g of VSV-G-pCMV, 1.25 $\mu$g of GFP-pMXs, and 45 $\mu$L of FuGENE HD.

The Plat-GP cells into which the retroviral vector plasmids had been transduced were cultured for at least 48 hours; thereafter, the supernatant was collected three times every 24 hours and stored at 4°C, and filtration was performed using the Steriflip-HV Filter unit (pore size 0.45 $\mu$m filter; Millipore; Cat No. SE1M003M00). The above-noted procedure was used to prepare pantropic retroviral vector solutions containing the three genes. The pantropic retroviral vectors, which enable genetic transduction into various cells, can efficiently transduce the genes into human cells as well.

[Table 9]

| Table 9: Details of constructed retroviral vector plasmids | | | | | | |
| --- | --- | --- | --- | --- | --- | --- |
| Gene | NCBI No. | Vector | 5' restriction enzyme | 3' restriction enzyme | Clone ID | Supplier |
| Human OCT3/4 | BC117435 | pMXs | EcoRI | EcoRI | 40125986 | Open Biosystems |
| Human KLF4 | BC029923 | pMXs | EcoRI | EcoRI | 5111134 | Open Biosystems |
| Human SOX2 | BC013923 | pMXs | EcoRI | XhoI | 2823424 | Open Biosystems |

Example 4: Preparation of induced malignant stem cells from cells (GC1) derived from cancer tissues of a gastric cancer patient

The fresh cancer tissues of a gastric cancer patient of donor No. 3 (medical information: a 67-year-old Japanese man with a progressive gastric caner, blood type AB, no chemotherapy, no radiotherapy, no immunosuppressive therapy, no smoking history, no drinking history, no drug addiction, no drug therapy, HIV-negative, HCV-negative, HBV-negative, syphilis-negative) which had been refrigerated for several hours and transported in a preservation solution (Hanks' solution supplemented with kanamycin and Fungizone) were used to isolate cells (GC1). The fresh non-cancer tissues of the same donor were also used to isolate cells (NGC1). To the resultant cells derived from the cancer tissues of the gastric cancer patient, the solution of the three retroviral vector containing any of three genes (POU5F1, KLF4, SOX2) prepared in Example 3 was added for genetic transduction, whereby human induced malignant stem cells were prepared from the gastric (solid) cancer tissues. The details of the procedure are as described below.

Part (0.5-1 g) of the gastric (solid) cancer tissues obtained during operation was washed with Hank's balanced salt solution (Phenol Red-free) (Invitrogen; Cat No. 14175-095) and minced with scissors into pieces of about 0.1-1 mm$^2$. The pieces were further washed with Hank's balanced salt solution (Phenol Red-free) until a supernatant became clear. Then, after removal of the supernatant, 5 mL of the DMEM medium (Invitrogen) supplemented with 0.1% collagenase (Wako Pure Chemical; Cat No. 034-10533) and 1X antibiotic/antimycotic was added to the tissue precipitate, and stirring was performed at 37°C for 60 minutes with a shaker.

After confirming that the precipitated tissue has been fully digested, 35 mL of a D-MEM (high glucose) medium supplemented with 1X antibiotic/antimycotic and 10% FBS was added, and the suspension was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, and the suspension was centrifuged again at 1000 rpm at 4°C for 5 minutes. Then, after removal of the supernatant, 5 mL of a D-MEM (high glucose) medium supplemented with 1X antibiotic/antimycotic and 10% FBS was added to part of the cells, and the cell suspension was seeded on a collagen-coated dish (60 mm) (Iwaki; Cat No. 11-018-004) to conduct primary culture. The remaining cells were stored in liquid nitrogen while being suspended in a preservation solution. At a later date, part of the cells was thawed and subjected to primary culture.

After 24 hours culture, the medium was removed, 5 mL of the solution of the three retroviral vectors containing any of three genes was added, and the suspension was infected at 37°C for 24 hours. The viral supernatant was removed, and mitomycin-treated mouse embryonic fibroblasts as feeder cells were suspended in 5 mL of a D-MEM (high glucose) medium supplemented with 1X antibiotic/antimycotic and 10% FBS, and the cell suspension was then seeded at a density of $5.0 \times 10^4$ cells/cm$^2$ on a collagen-coated dish (60 mm) (Iwaki; Cat No. 11-018-004) in which the transduced cells derived from the cancer tissues of the gastric cancer patient had been cultured, whereby co-culture was performed.

Thereafter, the medium was repeatedly replaced with a MEF conditioned ES medium every three days, and from 15 days after the genetic transduction, the medium was replaced everyday with mTeSR1. The MEFs were seeded at a density of $1.5 \times 10^6$ cell/60 cm$^2$ about once a week.

The MEF conditioned ES medium and its preparation procedure which were used in Examples are described below.

<MEF conditioned ES medium>

MEF

Mitomycin C-treated primary mouse embryonic fibroblasts (DS Pharma Biomedical; Cat No. R-PMEF-CF)

ES medium for MEF conditioning

Knockout D-MEM (Invitrogen; Cat No. 10829-018), 500 mL
2 mM GlutaMAX (Invitrogen)
10% knockout serum replacement (Invitrogen; Cat No. 10828-028)
50 $\mu$g/mL gentamicin (Invitrogen; Cat No. 15750-060)
MEM non-essential amino acid solution (Invitrogen; Cat No. 11140-050)
10 ng/mL bFGF (PeproTech; Cat No. 100-18B)

<Preparation of a MEF conditioned ES medium>

First, $5 \times 10^6$ cells of mitomycin-treated mouse embryonic fibroblasts (DS Pharma Biomedical; Cat No. R-PMEF-CF) were suspended in 40 mL of a D-MEM (high glucose) medium supplemented with 1X antibiotic/antimycotic and 10% FBS, and the cell suspension was then seeded on four gelatin-coated dishes (100 mm) (Iwaki; Cat No. 11-020-006). After 24 hours culture, the medium was removed and 10 mL of an ES medium for MEF conditioning was added.

To the supernatant collected every 24 hours, 10% knockout serum replacement, 10 ng/mL bFGF, and 0.1 mM 2-mercaptoethanol were newly added, so that the resultant suspension was used as a MEF conditioned ES medium.

[Establishment of human induced malignant stem cells derived from the cancer tissues of the gastric cancer patient]

At least 25 days after the three-gene transduction, colonies of six clones (GC1_4, GC1_6, GC1_7, GC1_8, GC1_9, GC1_10) of the induced malignant stem cell were picked up and transferred onto feeder cells in a gelatin-coated 24-well plate. The feeder cells, which are mitomycin-treated mouse embryonic fibroblasts, had been seeded in a gelatin-coated 24-well plate at a density of $5.0 \times 10^4$ cell/cm$^2$ the day before the pickup of the induced malignant stem cells.

After 7 to 10 days culture, the human induced malignant stem cells proliferated in the 24-well plate (passage 1) were subjected to passage culture onto 6-well plates (passage 2). Seven to ten days after the second passage, the human induced malignant stem cells proliferated in the 6-well plates (passage 2) were subjected to passage culture onto 10 cm culture dishes (passage 3). Seven to ten days after the third passage, part of the human induced malignant stem cells proliferated in the 10 cm culture dishes (passage 3) was subjected to passage culture onto 10 cm culture dishes (passage 4) and the remainder was cryopreserved. Four to ten days after the fourth passage, part of the human induced malignant stem cells proliferated in the 10 cm culture dishes (passage 4) was subjected to passage culture onto 10 cm culture dishes (passage 5) and the remainder was cryopreserved. The culture dishes had been coated with gelatin or matrigel before use. The genomic DNAs of the human induced malignant stem cells were purified using Qiagen DNeasy Blood & Tissue Kit (Cat. No. 69504), and the total RNAs of the cells were purified using Qiagen miRNeasy Mini Kit (Cat. No. 217004). Cryopreservation of the cell is as described above.

The following two dissociation solutions were used for passage culture:

(i) 0.25% trypsin/1 mM EDTA solution (Invitrogen; Cat No. 25200-056), and

(ii) Prepared dissociation solution [solution prepared by blending 10 mL of 10 mg/mL type IV collagenase (Invitrogen; Cat No. 17104-019), 1 mL of a 100 mM calcium chloride solution (Sigma), 59 mL of PBS, 10 mL of a 2.5% trypsin solution (Invitrogen; Cat No. 15090-046), and 20 mL of knockout serum replacemen (KSR) (Invitrogen; Cat No. 10828-028) and then sterilizing the blend through a 0.22 $\mu$m filter].

After placing at 37°C for 5 minutes, the dissociation solution was removed, 20 mL of a D-MEM (high glucose) medium supplemented with 1X antibiotic/antimycotic and 10% FBS was added, and the suspension was then centrifuged at 1000 rpm at 4°C for 5 minutes. Next, after removal of the supernatant, 1 mL of a cryopreservation solution was added, and the suspension was dispensed into two serum tubes. Thereafter, the serum tubes were placed into an animal cell freezing container (BICELL), freezed at -80°C overnight, and then stored in liquid nitrogen.

The following three cryopreservation solutions were used:

(i) CELLBANKER 3 (Nippon Zenyaku Kogyo; Cat No. BLC-3S),
(ii) Mixed solution of 50% mTeSR1, 40% KSR, and 10% DMSO, and
(iii) TC-Protector (DS Pharma Biomedical).

The induced malignant stem cells derived from the gastric cancer tissues of the gastric cancer patient could be prepared with MEFs in a gelatin- or matrigel-coated culture dish using mTeSR1 or ReproStem (supplemented with 10 ng/mL bFGF) and proliferated in *vitro.* The culture just before the collection of genomic DNAs or total RNAs was conducted for the induced malignant stem cells in a feeder cell-free, matrigel (BD; Cat No. 356234)-coated (60 $\mu$L/60 cm$^2$) culture dish using mTeSR1.

Example 5: Preparation of human induced malignant stem cells from cells (NGC1) derived from non-cancer tissues of a gastric cancer patient

The fresh non-cancer tissues of a gastric cancer patient of donor No. 3 (medical information: a 67-year-old Japanese man with a progressive gastric caner, blood type AB, no chemotherapy, no radiotherapy, no immunosuppressive therapy, no smoking history, no drinking history, no drug addiction, no drug therapy, HIV-negative, HCV-negative, HBV-negative, syphilis-negative) which had been refrigerated for several hours and transported in a preservation solution (Hanks' solution supplemented with kanamycin and Fungizone) were used to isolate cells, which were then subjected to primary culture. To the resultant cells derived from the non-cancer tissues of the gastric cancer patient, the solution of the three retroviral vectors containing any of three genes prepared in Example 3 was added for genetic transduction, whereby human induced malignant stem cells were prepared. The details of the procedure are as described below.

Part of the fresh non-cancer tissues of the gastric cancer patient (a 67-year-old Japanese man with a progressive gastric cancer) which had been obtained during operation was washed with Hank's balanced salt solution (Phenol Red-free) and minced with scissors into pieces of about 0.1-1 mm$^2$. The pieces were further washed with Hank's balanced salt solution (Phenol Red-free) until a supernatant became clear. Then, after removal of the supernatant, 5 mL of the DMEM medium (Invitrogen) supplemented with 0.1 % collagenase and 1X antibiotic/antimycotic was added to the tissue precipitate, and stirring was performed at 37°C for 60 minutes with a shaker.

After confirming that the precipitated tissue has been fully digested, 35 mL of a D-MEM (high glucose) medium supplemented with 1X antibiotic/antimycotic and 10% FBS was added, and the suspension was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, and the suspension was centrifuged again at 1000 rpm at 4°C for 5 minutes. Then, after removal of the supernatant, 10 mL of a D-MEM (high glucose) medium supplemented with 1X antibiotic/antimycotic and 10% FBS was added to part of the cells, and the cell suspension was seeded on a collagen-coated dish (100 mm) (Iwaki; Cat No. 11-018-006). The remaining cells were stored in liquid nitrogen while being suspended in a preservation solution. At a later date, part of the cells was thawed and subjected to primary culture.

After about 24 hours culture, the medium was removed, 10 mL of the solution of the three retroviral vectors containing any of three genes was added, and the suspension was infected at 37°C for about 24 hours. The viral supernatant was removed, and mitomycin-treated mouse embryonic fibroblasts were suspended in 10 mL of a D-MEM (high glucose) medium supplemented with 1X antibiotic/antimycotic and 10% FBS, and the cell suspension was then seeded at a density of $5.0 \times 10^4$ cells/cm$^2$ on a collagen-coated dish (100 mm) (Iwaki; Cat No. 11-018-006) in which the transduced cells derived from the non-cancer tissues of the gastric cancer patient had been cultured, whereby co-culture was performed.

[Establishment of human induced malignant stem cells derived from the non-cancer tissues of the gastric cancer patient]

Thereafter, the medium was repeatedly replaced with a MEF conditioned ES medium every three days, and from 31 days after the three-gene transduction, the medium was replaced everyday with mTeSR1. The MEFs were seeded at a density of $1.5 \times 10^6$ cell/60 cm$^2$ about once a week. At least 41 days after the three-gene transduction, colonies of two clones (NGC1_6, NGC1_7) of the induced malignant stem cell were picked up and transferred onto feeder cells in a gelatin-coated 24-well plate. The feeder cells, which are mitomycin-treated mouse embryonic fibroblasts, had been seeded in a gelatin-coated 24-well plate at a density of $5.0 \times 10^4$ cell/cm$^2$ the day before the pickup of the induced malignant stem cells.

After 7 to 10 days culture, the human induced malignant stem cells proliferated in the 24-well plate (passage 1) were subjected to passage culture onto 6-well plates (passage 2). Seven to ten days after the second passage, the human induced malignant stem cells proliferated in the 6-well plates (passage 2) were subjected to passage culture onto 10 cm culture dishes (passage 3). Seven to ten days after the third passage, part of the human induced malignant stem cells proliferated in the 10 cm culture dishes (passage 3) was subjected to passage culture onto 10 cm culture dishes (passage 4) and the remainder was cryopreserved. Four to ten days after the fourth passage, part of the human induced malignant stem cells proliferated in the 10 cm culture dishes (passage 4) was subjected to passage culture onto 10 cm culture dishes (passage 5) and the remainder was cryopreserved. The culture dishes had been coated with gelatin or matrigel before use. The genomic DNAs of the human induced malignant stem cells derived from the non-cancer tissues of the gastric cancer patient were purified using Qiagen DNeasy Blood & Tissue Kit (Cat. No. 69504), and the total RNAs of the cells were purified using Qiagen miRNeasy Mini Kit (Cat. No. 217004). Cryopreservation of the cell performed in Examples of the present invention is as described above.

The induced malignant stem cells derived from the non-cancer tissues of the gastric cancer patient could be prepared with MEFs in a matrigel- or gelatin-coated culture dish using mTeSR1 or ReproStem (supplemented with 10 ng/mL bFGF) and proliferated in *vitro.* The culture just before the collection of genomic DNAs or total RNAs was conducted for the induced malignant stem cells in a feeder cell-free, matrigel (BD; Cat No. 356234)-coated (60 μL/60 cm$^2$) culture dish using mTeSR1.

Example 6: Preparation of human induced malignant stem cells from cells (CC1) derived from cancer tissues of a colon cancer patient

The fresh cancer tissues of a colon cancer patient of donor No. 4 (medical information: a 55-year-old Japanese man with a sigmoidal colon caner, blood type B, no chemotherapy, no radiotherapy, no immunosuppressive therapy, no smoking history, no drinking history, no drug addiction, no drug therapy, no diabetes, fasting blood glucose level: 94, HbA1c level: 4.8, blood triglyceride level: 56, LDL-cholesterol level: 122, height: 172 cm, weight: 68.6 kg, HIV-negative, HCV-negative, HBV-negative, syphilis-negative) which had been refrigerated for several hours and transported in a preservation solution (Hanks' solution supplemented with kanamycin and Fungizone) were used to isolate cells (CC1). The fresh non-cancer tissues of the same donor were also used to isolate cells (NCC1). To the resultant cells derived from the cancer tissues of the colon cancer patient, the solution of the three retroviral vectors containing any of three genes (POU5F1, KLF4, SOX2) prepared in Example 3 was added for genetic transduction, whereby human induced malignant stem cells were prepared. The details of the procedure are as described below.

Part of the colon (solid) cancer tissues obtained during operation was washed with Hank's balanced salt solution (Phenol Red-free) and minced with scissors into pieces of about 0.1-1 mm$^2$. The pieces were further washed with Hank's balanced salt solution (Phenol Red-free) until a supernatant became clear. Then, after removal of the supernatant, 5 mL of the DMEM medium (Invitrogen) supplemented with 0.1% collagenase and 1X antibiotic/antimycotic was added to the tissue precipitate, and stirring was performed at 37°C for 60 minutes with a shaker.

After confirming that the precipitated tissue has been fully digested, 35 mL of a D-MEM (high glucose) medium supplemented with 1X antibiotic/antimycotic and 10% FBS was added, and the suspension was then centrifuged at 1000 rpm at 4°C for 5 minutes. After removal of the supernatant, 40 mL of a D-MEM (high glucose) medium supplemented with 1X antibiotic/antimycotic and 10% FBS was added, and the suspension was centrifuged again at 1000 rpm at 4°C for 5 minutes. After removal of the supernatant, 10 mL of a D-MEM (high glucose) medium supplemented with 1X antibiotic/antimycotic and 10% FBS was added to part of the cells, and the cell suspension was seeded on a collagen-coated dish (100 mm) (Iwaki; Cat No. 11-018-006). The remaining cells were stored in liquid nitrogen while being suspended in a preservation solution. At a later date, part of the cells was thawed and subjected to primary culture.

After about 24 hours culture, the medium was removed, 10 mL of the solution of the three retroviral vectors containing any of three genes was added, and after 5 hours incubation, 5 mL of the Luc-IRES-GFP retroviral vector was infected at 37°C for about 24 hours. The viral supernatant was removed, and mitomycin-treated MEFs were suspended in 10 mL of a D-MEM (high glucose) medium supplemented with 1X antibiotic/antimycotic and 10% FBS, and the cell suspension was then seeded at a density of $5.0 \times 10^4$ cells/cm$^2$ on a collagen-coated dish (100 mm) (Iwaki; Cat No. 11-018-006) in which the transduced cells derived from the cancer tissues of the colon cancer patient had been cultured, whereby co-culture was performed.

[*In vitro* culture of human induced malignant stem cells derived from the cancer tissues of the colon cancer patient]

Thereafter, the medium was repeatedly replaced with a MEF conditioned ES medium every three days, and from 22 days after the genetic transduction, the medium was replaced everyday with mTeSR1. The MEFs were seeded at a density of $1.5 \times 10^6$ cell/60 cm$^2$ about once a week. At least 31 days after the three-gene transduction, colonies often clones (CC1_1, CC1_2, CC1_7, CC1_8, CC1_9, CC1_11, CC1_12, CC1_17, CC1_18, CC1_25) were picked up and subjected to passage culture onto feeder cells in a gelatin-coated 24-well plate. The feeder cells, which are mitomycin-treated mouse embryonic fibroblasts, had been seeded in a gelatin-coated 24-well plate at a density of $5.0 \times 10^4$ cell/cm$^2$ the day before the pickup of the induced malignant stem cells.

After 7 to 10 days culture, the human induced malignant stem cells proliferated in the 24-well plate (passage 1) were subjected to passage culture onto 6-well plates (passage 2). Seven to ten days after the second passage, the human induced malignant stem cells proliferated in the 6-well plates (passage 2) were subjected to passage culture onto 10 cm culture dishes (passage 3). Seven to ten days after the third passage, part of the human induced malignant stem cells proliferated in the 10 cm culture dishes (passage 3) was subjected to passage culture onto 10 cm culture dishes (passage 4) and the remainder was cryopreserved. Four to ten days after the fourth passage, part of the human induced malignant stem cells proliferated in the 10 cm culture dishes (passage 4) was subjected to passage culture onto 10 cm culture dishes (passage 5) and the remainder was cryopreserved. The culture dishes had been coated with gelatin or matrigel before use. The genomic DNAs of the human induced malignant stem cells derived from the non-cancer tissues of the colon cancer patient were purified using Qiagen DNeasy Blood & Tissue Kit (Cat. No. 69504), and the total RNAs of the cells were purified using Qiagen miRNeasy Mini Kit (Cat. No. 217004). Cryopreservation of the cell is as described above.

The induced malignant stem cells derived from the cancer tissues of the colon cancer patient could be prepared with MEFs in a matrigel- or gelatin-coated culture dish using mTeSR1 or ReproStem (supplemented with 10 ng/mL bFGF) and proliferated *in vitro.* The culture just before the collection of genomic DNAs or total RNAs was conducted for the induced malignant stem cells in a feeder cell-free, matrigel (BD; Cat No. 356234)-coated (60 $\mu$L/60 cm$^2$) culture dish using mTeSR1.

Example 7: Preparation of induced malignant stem cells from cells (CC4) derived from cancer tissues of a colon cancer patient

The fresh cancer tissues of a colon cancer patient of donor No. 5 (medical information: a 77-year-old Japanese woman with a colon caner, blood type AB, no chemotherapy, no radiotherapy, no immunosuppressive therapy, no smoking history, no drinking history, no drug addiction, drug therapy (Amaryl: 1.5 mg, Melbin: 25 mg$\times$3 pcs, Micardis: 20 mg, Crestor: 2.5 mg), HIV-negative, HCV-negative, HBV-negative, syphilis-negative) which had been refrigerated for several hours and transported in a preservation solution (Hanks' solution supplemented with kanamycin and Fungizone) were used to isolate cells (CC4). The fresh colon non-cancer tissues were also used to isolate cells (NCC4). To the resultant cells (CC4) derived from the colon (solid) cancer tissues, the solution of the four Sendai viral vectors containing any of four genes (POU5F1, KLF4, SOX2, c-Myc) (DNAVEC CytoTune iPS (DV-0301-1)) was added for genetic transduction, whereby human induced malignant stem cells were prepared from the colon (solid) cancer tissues. The Sendai viral vector is an RNA viral vector that does not insert an exogenous DNA into the genomic DNAs of cells. The details of the procedure are as described below.

Part (0.5-1 g) of the colon (solid) cancer tissues obtained during operation was washed with Hank's balanced salt solution (Phenol Red-free) (Invitrogen; Cat No. 14175-095) and minced with scissors into pieces of about 0.1-1 mm$^2$. The pieces were further washed with Hank's balanced salt solution (Phenol Red-free) until a supernatant became clear. After removal of the supernatant, 3 mL of the DMEM medium (Invitrogen) supplemented with 1% collagenase (Wako Pure Chemical; Cat No. 034-10533) and 1X antibiotic/antimycotic was added to the tissue precipitate, and stirring was performed at 37°C for 90 minutes with a shaker.

After confirming that the precipitated tissue has been fully digested, 35 mL of a D-MEM (high glucose) medium supplemented with 1X antibiotic/antimycotic was added, and the suspension was then centrifuged at 1000 rpm at 4°C for 5 minutes. Next, after removal of the supernatant, 40 mL of a D-MEM (high glucose) medium supplemented with 1X antibiotic/antimycotic was added, and the suspension was centrifuged again at 1000 rpm at 4°C for 5 minutes. Then, after removal of the supernatant, 10 mL of StemPro (Invitrogen), mTeSR1, or ReproStem (supplemented with 10 ng/mL bFGF) which have been supplemented with 1X antibiotic/antimycotic and 10 µg/mL-gentamicin was added to part of the cells, and the cell suspension was seeded on a matrigel-coated culture dish (100 mm) (coated for an hour with 60 µL matrigel/6 mL PBS) or a 6-well plate (coated for an hour with 60 µL matrigel/6 mL PBS/6-well) to conduct primary culture. The remaining cells were stored in liquid nitrogen while being suspended in a preservation solution. At a later date, part of the cells was thawed and subjected to primary culture.

To the cells (CC4) derived from the colon (solid) cancer tissues, the solution of the four Sendai viral vectors containing any of four genes was added, and the suspension was infected at 37°C for one day. Mitomycin-treated mouse embryonic fibroblasts (MEFs) as feeder cells were suspended in 10 mL of ReproStem (supplemented with 10 ng/mL bFGF), and the cell suspension was then seeded at a density of $1.5 \times 10^6$ cells on the matrigel-coated culture dish (100 mm) or 6-well plate in which the transduced cells derived from the cancer tissues of the colon cancer patient had been cultured, whereby co-culture was performed.

Thereafter, the medium was repeatedly replaced with ReproStem (supplemented with 10 ng/mL bFGF) every three days.

At least 2 weeks after the genetic transduction, colonies of twelve clones (CC4_(9)_5, CC4_(9)_7, CC4_(9)_11, CC4_(9)_13, CC4_(3)_10, CC4_4, CC4_6, CC4_30, CC4-10, CC4-31, CC4_1, CC4_2) were picked up and subjected to passage culture onto mouse embryonic fibroblasts in a gelatin- or matrigel-coated 24-well plate. The feeder cells, which are mitomycin-treated mouse embryonic fibroblasts, had been seeded in a gelatin- or matrigel-coated 24-well plate at a density of $1.5 \times 10^6$ cell/6-well plate the day before the pickup of the induced malignant stem cells.

After 7 to 10 days culture, the human induced malignant stem cells proliferated in the 24-well plate (passage 1) were subjected to passage culture onto 6-well plates (passage 2). Seven to ten days after the second passage, the human induced malignant stem cells proliferated in the 6-well plates (passage 2) were subjected to passage culture onto 10 cm culture dishes (passage 3).

All of the human induced malignant stem cells in each well of 6-well plates were laveled as CC4_(3), CC4_(4), and CC4_(6) and subjected to passage culture onto 10 cm culture dishes (passage 1).

Seven to ten days after the passage, part of the human induced malignant stem cells proliferated in the 10 cm culture dishes was subjected to passage culture onto 10 cm culture dishes and the remainder was cryopreserved. Four to ten days after the preceding passage, part of the human induced malignant stem cells proliferated in the 10 cm culture dishes was subjected to passage culture onto 10 cm culture dishes and the remainder was cryopreserved. The culture dishes had been coated with gelatin or matrigel before use.

The genomic DNAs of the cells derived from the cancer tissues of the colon cancer patient, the cells derived from the non-cancer tissues of the colon cancer patient, and the human induced malignant stem cells derived from the cancer tissues of the colon cancer patient were purified using Qiagen DNeasy Blood & Tissue Kit (Cat. No. 69504), and the total RNAs of these cells were purified using Qiagen miRNeasy Mini Kit (Cat. No. 217004). Cryopreservation of the cell is as described above.

The induced malignant stem cells derived from the cancer tissues of the colon cancer patient could be proliferated *in vitro* with feeder cells (MEFs) in a matrigel- or gelatin-coated culture dish using mTeSR1 or ReproStem (supplemented with 10 ng/mL bFGF) and *proliferated in vitro.* The culture just before the collection of genomic DNAs or total RNAs was conducted for the induced malignant stem cells in a feeder cell-free, matrigel (BD; Cat No. 356234)-coated (1 µL/cm$^2$) culture dish using ReproStem (supplemented with 10 ng/mL bFGF).

Example 8: Preparation of induced malignant stem cells from cells (CC4) derived from cancer tissues of a colon cancer patient

The fresh cancer tissues of a colon cancer patient of donor No.5 (medical information: a 77-year-old Japanese woman with a colon caner, blood type AB, no chemotherapy, no radiotherapy, no immunosuppressive therapy, no smoking history, no drinking history, no drug addiction, drug therapy (Amaryl: 1.5 mg, Melbin: 25 mg×3 pcs, Micardis: 20 mg, Crestor: 2.5 mg), HIV-negative, HCV-negative, HBV-negative, syphilis-negative) which had been refrigerated for several hours and transported in a preservation solution (Hanks' solution supplemented with kanamycin and Fungizone) were used to isolate cells (CC4). The fresh colon non-cancer tissues were also used to isolate cells (NCC4).

Human induced malignant stem cells were prepared from the colon (solid) tissues without genetic transduction. The details of the procedure are as described below.

Part (0.5-1 g) of the colon (solid) cancer tissues obtained during operation was washed with Hank's balanced salt solution (Phenol Red-free) (Invitrogen; Cat No. 14175-095) and minced with scissors into pieces of about 0.1-1 mm$^2$. The pieces were further washed with Hank's balanced salt solution (Phenol Red-free) until a supernatant became clear. After removal of the supernatant, 3 mL of the DMEM medium (Invitrogen) supplemented with 1% collagenase (Wako Pure Chemical; Cat No. 034-10533) and 1X antibiotic/antimycotic was added to the tissue precipitate, and stirring was performed at 37°C for 90 minutes with a shaker.

After confirming that the precipitated tissue has been fully digested, 35 mL of a D-MEM (high glucose) medium supplemented with 1X antibiotic/antimycotic was added, and the suspension was then centrifuged at 1000 rpm at 4°C for 5 minutes. Next, after removal of the supernatant, 40 mL of a D-MEM (high glucose) medium supplemented with 1X antibiotic/antimycotic was added, and the suspension was centrifuged again at 1000 rpm at 4°C for 5 minutes. Then, after removal of the supernatant, 10 mL of mTeSR1 or ReproStem (supplemented with 10 ng/mL bFGF) supplemented with 1X antibiotic/antimycotic was added to part of the cells, and the cell suspension was seeded on a matrigel-coated culture dish (100 mm) (coated for an hour with 60 $\mu$L matrigel/6 mL PBS) or a 6-well plate (coated for an hour with 60 $\mu$L matrigel/6 mL PBS/6-well) to conduct primary culture. The remaining cells were stored in liquid nitrogen while being suspended in a preservation solution. At a later date, part of the cells was thawed and subjected to primary culture.

The suspension was cultured at 37°C for one day; thereafter, $1.5 \times 10^6$ of mitomycin-treated mouse embryonic fibroblasts (MEFs) as feeder cells were suspended in 10 mL of ReproStem (supplemented with 10 ng/mL bFGF), and the cell suspension was then seeded on the matrigel-coated culture dish (100 mm) on which the cells derived from the cancer tissues of the colon cancer patient had been cultured, whereby co-culture was performed. Thereafter, the medium was repeatedly replaced with ReproStem (supplemented with 10 ng/mL bFGF) every three days.

At least 2 weeks after the coculture, colonies of two clones (CC4-c, CC4-D) were picked up and subjected to passage culture onto mouse embryonic fibroblasts in a gelatin- or matrigel-coated 24-well plate. The feeder cells, which are mitomycin-treated mouse embryonic fibroblasts, had been seeded in a gelatin-coated 24-well plate at a density of $5.0 \times 10^4$ cell/cm$^2$ the day before the pickup of the induced malignant stem cells.

After 7 to 10 days culture, the human induced malignant stem cells proliferated in the 24-well plate (passage 1) were subjected to passage culture onto 6-well plates (passage 2). Seven to ten days after the second passage, the human induced malignant stem cells proliferated in the 6-well plates (passage 2) were subjected to passage culture onto 10 cm culture dishes (passage 3). Seven to ten days after the third passage, part of the human induced malignant stem cells proliferated in the 10 cm culture dishes (passage 3) was subjected to passage culture onto 10 cm culture dishes (passage 4) and the remainder was cryopreserved. Four to ten days after the fourth passage, part of the human induced malignant stem cells proliferated in the 10 cm culture dishes (passage 4) was subjected to passage culture onto 10 cm culture dishes (passage 5) and the remainder was cryopreserved. The culture dishes had been coated with gelatin or matrigel before use. The genomic DNAs of the cells derived from the cancer tissues of the colon cancer patient, the cells derived from the non-cancer tissues of the colon cancer patient, and the human induced malignant stem cells derived from the cancer tissues of the colon cancer patient were purified using Qiagen DNeasy Blood & Tissue Kit (Cat. No. 69504), and the total RNAs of these cells were purified using Qiagen miRNeasy Mini Kit (Cat. No. 217004). Cryopreservation of the cell is as described above.

As described above, the induced malignant stem cells (not transduced) derived from the cancer tissues of the colon cancer patient could be prepared with feeder cells (MEFs) using mTeSR1 or ReproStem (supplemented with 10 ng/mL bFGF) and proliferated *in vitro*. The culture just before the collection of genomic DNAs or total RNAs was conducted for the induced malignant stem cells in a feeder cell-free, matrigel (BD; Cat No. 356234)-coated (1 $\mu$L/cm$^2$) culture dish using ReproStem (supplemented with 10 ng/mL bFGF).

Reference Example: Fibroblast-derived induced pulriponent stem cells

The solution of the four Sendai viral vectors containing any of four genes (POU5F1, KLF4, SOX2, c-Myc) (DNAVEC CytoTune iPS (DV-0301-1)) or the solution of the three retroviral vectors containing any of three genes was added to commercially available fibroblasts (derived from normal tissues) for genetic transduction, whereby human induced stem cells such as human induced pulriponent stem cells were prepared. The details of the procedure are as described below. The Sendai viral vector is an RNA viral vector that does not insert an exogenous DNA into the genomic DNAs of cells.

Neonatal fibroblasts (Lonza; Donor No. 7f3956 (donor No. 6) or 7f3949 (donor No. 7)) were cultured for one day; thereafter, the solution of the four Sendai viral vectors containing any of four genes (POU5F1, KLF4, SOX2, c-Myc) or the solution of the three retroviral vectors containing any of three genes was added, and the suspension was infected at 37°C for one day. The viral supernatant was removed, and mitomycin-treated mouse embryonic fibroblasts (MEFs) as feeder cells were suspended in 10 mL of a D-MEM (high glucose) medium supplemented with 1X antibiotic/antimycotic and 10% FBS, and the cell suspension was then seeded at a density of $1.5 \times 10^6$ cell/60 cm$^2$ on a matrigel-coated culture dish (100 mm) in which the transduced fibroblasts had been cultured, whereby co-culture was performed.

Thereafter, the medium was replaced every one to three days with the ReproStem medium (supplemented with 10 ng/mL bFGF, 1X antibiotic/antimycotic, and 10 $\mu$g/mL gentamicin) or mTeSR1 (supplemented with 1X antibiotic/antimycotic and 10 $\mu$g/mL gentamicin). The MEFs were seeded at a density of $1.5 \times 10^6$ cell/60 cm$^2$ about once a week.

At least one month after the genetic transduction, colonies of three clones (nfb1_2, nfb1_4, nfb2-17) were picked up and subjected to passage culture onto a gelatin- or matrigel-coated 24-well plate on which MEFs had been seeded. The MEFs as feeder cells, which are mitomycin-treated mouse embryonic fibroblasts, had been seeded in a gelatin- or matrigel-coated 24-well plate at a density of $1.5 \times 10^6$ cell/24-well plate the day before the pickup of the induced stem cells.

After 7 to 10 days culture, the human induced stem cells proliferated in the 24-well plate (passage 1) were subjected to passage culture onto 6-well plates (passage 2). Seven to ten days after the second passage, the human induced stem cells proliferated in the 6-well plates (passage 2) were subjected to passage culture onto 10 cm culture dishes (passage 3). Seven to ten days after the third passage, part of the human induced stem cells proliferated in the 10 cm culture dishes (passage 3) was subjected to passage culture onto 10 cm culture dishes (passage 4) and the remainder was cryopreserved. Four to ten days after the fourth passage, part of the human induced stem cells proliferated in the 10 cm culture dishes (passage 4) was subjected to passage culture onto 10 cm culture dishes (passage 5) and the remainder was cryopreserved. The culture dishes had been coated with gelatin or matrigel before use.

The genomic DNAs of the human induced stem cells were purified using Qiagen DNeasy Blood & Tissue Kit (Cat. No. 69504), and the total RNAs of the cells were purified using Qiagen miRNeasy Mini Kit (Cat. No. 217004). Cryopreservation of the cell is as described above.

The following induced stem cells: nfb1_2 (derived from Donor No. 7f3956; prepared using the retroviral vectors), nfb1_4 (derived from Donor No. 7f3956; prepared using the retroviral vectors), and nfb2-17 (derived from Donor No. 7f3949; prepared using the Sendai viral vectors), were derived from cells of neonatal skin (normal tissues). Like the induced pulriponent stem cells 201B7 prepared from adult-skin-derived fibroblasts, those induced stem cells had normal genome, epigenome, gene expressions (mRNA and miRNA), protein expression, sugar chain, and metabolome, and expressed the embryonic stem (ES) cell-specific genes (OCT3/4, SOX2, NANOG, ZFP42). Thus, those cells were used as standard cells for various analyses. The cells nfb1_2 nfb1_4, nfb2-17, and 201B7 also expressed the embryonic stem (ES) cell-specific genes listed in the following table.

[Table 10]

| Table 10: ES cell-specific genes | |
|---|---|
| GeneSymbol | GenbankAccession |
| ACVR2B | M_001106 |
| CD24 | L33930 |
| CDH1 | M_004360 |
| CYP26A1 | NM_057157 |
| DNMT3B | NM_175850 |
| DPPA4 | NM_018189 |
| EDNRB | NM_003991 |
| FLT1 | NM_002019 |
| GABRB3 | NM_000814 |
| GATA6 | NM_005257 |
| GDF3 | NM_020634 |
| GRB7 | NM_005310 |
| LIN28 | NM_024674 |
| NANOG | NM_024865 |
| NODAL | M_018055 |
| PODXL | NM_005397 |
| POU5F1 | NM_002701 |
| SALL4 | NM_020436 |
| SOX2 | NM_003106 |
| TDGF1 | NM_003212 |

(continued)

| Table 10: ES cell-specific genes | |
|---|---|
| GeneSymbol | GenbankAccession |
| TERT | NM_198253 |
| ZFP42 | NM_174900 |
| ZIC3 | NM_003413 |

Example 9: Aberration of methylations of endogenous genomic DNAs of induced malignant stem cells

In this Example, (1)(a) aberration of methylations (hypermethylations or hypomethylations) of tumor suppressor gene or cancer-related gene regions in endogenous genomic DNAs of induced malignant stem cells were detected, in comparison with those of induced pluripotent stem cells, induced non-malignant stem cells, or non-cancer site tissues.

(9-1) Materials

The aberration of methylations (hypermethylations or hypomethylations) of tumor suppressor gene or cancer-related gene regions in endogenous genomic DNAs were detected using a commercially available methylation analysis tool such as Infinium ® HumanMethylation450 BeadChip (illumina) following the instructions of the manufacturer.
The following samples were used in the analysis for aberration of methylations (hypermethylations or hypomethylations) of tumor suppressor gene or cancer-related gene regions in endogenous genomic DNAs:

- induced malignant stem cells (GC2_1) prepared from fresh gastric cancer tissues collected from the individual of donor No. 1;
- induced malignant stem cells (CC3_5) prepared from fresh colon cancer tissues collected from the individual of donor No. 2;
- induced malignant stem cells (GC1_4, NGC1_6) prepared from fresh gastric cancer tissues collected from the individual of donor No. 3;
- induced malignant stem cells (CC1_1) prepared from fresh colon cancer tissues collected from the individual of donor No. 4;
- cell population (ncc4) derived from colon non-cancer site tissues, cell population (cc4) derived from fresh colon cancer site tissues, and induced malignant stem cells (CC4_c) prepared from fresh colon cancer tissues, which were collected from the individual of donor No. 5;
- induced pluripotent stem cells (NFB1_4) prepared from fibroblasts (7F3956) collected from the individual of donor No. 6; and
- induced pluripotent stem cells (NFB2_17) prepared from fibroblasts (7F3949) collected from the individual of donor No. 7.

(9-2) Whole-genome DNA methylations

In this Example, intracellular methylations were genome-widey compared between the induced malignant stem cells and the induced pluripotent stem cells, the induced non-malignant stem cells or the non-cancer site tissues. Such comparisons can be made using a commercially available methylation analysis tool such as Infinium ® HumanMethylation450 BeadChip (illumina) following the instructions of the manufacturer.
Infinium ® HumanMethylation450K BeadChip (illumina), which is intended for identifying methylation states genome-widely, targets multiple sites in the promotor region, 5' untranslated region, first exon, gene, and 3' untranslated region, and is capable of covering the whole genetic regions. The information on the genetic regions covered by Infinium ® HumanMethylation450K BeadChip (illumina) is publicly available by illumina. And differential analyses in this Example were made using probes (a total of 6659 probes; the detailed information is publicly available by illumina) capable of detecting regions that show different methylations between normal and cancer cells as observed in various tissues and multiple carcinomas. BeadChip enables exhaustive analyses of 99% reference sequence genes including genes in the regions whose methylation had not been detectable with conventional methods.
In this Example, testing was made on the following points:

- comparison of methylations between the induced malignant stem cells (CC4_c) and the normal cells (ncc4) (Table 11),

- comparison of methylations between the induced malignant stem cells (CC1_1) and the induced pluripotent stem cells (nfb1-4) (Table 12),
- comparison of methylations between the induced malignant stem cells (GC1_4) and the induced pluripotent stem cells (nfb1-4) (Table 13),
- comparison of methylations between the induced non-malignant stem cells (NGC1_6) and the induced pluripotent stem cells (nfb1-4) (Table 14),
- comparison of methylations between the induced malignant stem cells (CC3_5) and the induced pluripotent stem cells (nfb2-17) (Table 15),
- comparison of methylations between the induced malignant stem cells (GCK2-1) and the induced pluripotent stem cells (nfb2-17) (Table 16), and
- comparison of methylations between the induced malignant stem cells (GC1_4) and the induced non-malignant stem cells (NGC1_6) (Table 17).

(9-3) Results of whole-genome DNA methylations and comparative analyses

The results of the respective comparisons are listed in the following tables.

In these tables, "TargetID" represents the IDs of the probes used in Infinium ® HumanMethylation450 BeadChip (illumina), and "UCSC_RefGene_Name" represents the notations of the genes present in a methylation site. "Absolute differential value" means an absolute value of the difference between the methylation levels in each TargetID of two kinds of cells; the absolute differential value is taken as zero when the methylation level in cancer cells or induced malignant stem cells is identical to the level in normal cells or induced pluripotent stem cells, and when the former level is higher or lower than the latter level, the difference is indicated in absolute values. "CHR" represents a chromosome number on which a methylation site was located.

Table 11 below lists the top 20 of the highest absolute differential values among the results of the comparison of methylations between the induced malignant stem cells (CC4_c) and the normal cells (ncc4).

[Table 11]

| Table 11: Comparison of methylations between the induced malignant stem cells (CC4_c) and the normal cells (ncc4) | | | |
|---|---|---|---|
| TargetID | UCSC_RefGene_Name | Absolute differential value | CHR |
| cg24446548 | TWIST1;TWIST1 | 0.9738708 | 7 |
| cg02012576 | | 0.96991509 | 12 |
| cg20893717 | EPO | 0.96268476 | 7 |
| cg17839237 | TWIST1;TWIST1 | 0.96265799 | 7 |
| cg02723533 | CCND1 | 0.96218878 | 11 |
| cg08684639 | WDR17;WDR17 | 0.9510549 | 4 |
| cg26770917 | OLIG1;OLIG1 | 0.9450131 | 21 |
| cg14646111 | SEC23B;SEC23B;SEC23B | 0.94440604 | 20 |
| cg27542341 | RPP25;RPP25 | 0.943493746 | 15 |
| cg26365854 | ALX4 | 0.94348861 | 11 |
| cg15245095 | SYT1;SYT1 | 0.94039545 | 12 |
| cg22260952 | CHST11 | 0.94032282 | 12 |
| cg16532755 | JAM2 | 0.93869771 | 21 |
| cg11409659 | SLC6A15;SLC6A15;SLC6A15 | 0.93766733 | 12 |
| cg21433912 | | 0.93732436 | 7 |
| cg25115993 | ULBP1 | 0.93283064 | 6 |
| cg22834653 | FGF12 | 0.93260221 | 3 |
| cg08347500 | | 0.93162251 | 16 |

(continued)

| Table 11: Comparison of methylations between the induced malignant stem cells (CC4_c) and the normal cells (ncc4) | | | |
|---|---|---|---|
| TargetID | UCSC_RefGene_Name | Absolute differential value | CHR |
| cg04123776 | | 0.93023146 | 1 |
| cg09822538 | NTNG1;NTNG1;NTNG1;NTNG1;NTNG1;NTNG1 | 0.9243868 | 1 |

There were 4,546 probes, in addition to the above-listed 20 probes, that showed differential values (absolute values) of 0.15 or higher between the CC4_c and ncc4 samples (no detailed data shown).

Table 12 below lists the top 20 of the highest absolute differential values among the results of the comparison of methylations between the induced malignant stem cells (CC1_1) and the induced pluripotent stem cells (nfb1-4).

[Table 12]

| Table 12: Comparison of methylations between the induced malignant stem cells (CC1_1) and the induced pluripotent stem cells (nfb1-4) | | | |
|---|---|---|---|
| TargetID | UCSC_RefGene_Name | Absolute differential value | CHR |
| cg23690264 | SLITRK4 | 0.91062368 | X |
| cg05135828 | SLITRK4 | 0.90882514 | X |
| cg04642759 | SLITRK2;SLITRK2;SLITRK2;SLITRK2 | 0.86572393 | X |
| eg25237542 | SLITRK4 | 0.82931549 | X |
| cg23784675 | BRUNOL4;BRUNOL4;BRUNOL4;BRUNOL4 | 0.8210511 | 18 |
| cg20976286 | OCA2 | 0.81596715 | 15 |
| cg10016783 | SLITRK2;SLITRK2;SLITRK2;SLITRK2 | 0.81370707 | X |
| cg03020597 | SLITRK2;SLITRK2;SLITRK2;SLITRK2 | 0.78593668 | X |
| cg09720420 | SLITRK4 | 0.78450744 | X |
| cg19932577 | | 0.77379555 | 8 |
| cg18701656 | SLITRK2;SLITRK2;SLITRK2;SLITRK2 | 0.76418439 | X |
| cg00546757 | | 0.72277891 | 5 |
| cg01560464 | SLITRK4 | 0.7187806 | X |
| cg12087615 | KRT1 | 0.68419805 | 12 |
| cg03398919 | | 0.6419687 | 2 |
| cg14752426 | SLITRK4 | 0.64067302 | X |
| cg13868165 | FAM19A5 | 0.6277231 | 22 |
| cg22623223 | PTPRN2;PTPRN2;PTPRN2 | 0.61213679 | 7 |
| cg17838026 | KCNC3 | 0.61075544 | 19 |
| cg13670833 | KCNC3 | 0.54178615 | 19 |

There were 228 probes, in addition to the above-listed 20 probes, that showed differential values (absolute values) of 0.15 or higher between the CC1_1 and nfb1-4 samples (no detailed data shown).

Table 13 below lists the top 20 of the highest absolute differential values among the results of the comparison of methylations between the induced malignant stem cells (GC1_4) and the induced pluripotent stem cells (nfb1-4).

[Table 13]

| Table 13: Comparison of methylations between the induced malignant stem cells (GC1_4) and the induced pluripotent stem cells (nfb1-4) | | | |
|---|---|---|---|
| TargetID | UCSC_RefGene_Name | Absolute differential value | CHR |
| cg05135828 | SLITRK4 | 0.91624837 | X |
| cg23690264 | SLITRK4 | 0.89657667 | X |
| cg04642759 | SLITRK2;SLITRK2;SLITRK2;SLITRK2 | 0.83346912 | X |
| cg100016783 | SLITRK2;SLITRK2;SLITRK2;SLITRK2 | 0.82566516 | X |
| cg23784675 | BRUNOL4;BRUNOL4;BRUNOL4;BRUNOL4 | 0.8188375 | 18 |
| cg20976286 | OCA2 | 0.81232925 | 15 |
| cg25237542 | SLITRK4 | 0.80931326 | X |
| cg19932577 | | 0.80485815 | 8 |
| cg09720420 | SLITRK4 | 0.7675395 | X |
| cg03020597 | SLITRK2;SLITRK2;SLITRK2;SLITRK2 | 0.76094945 | X |
| cg1870656 | SLITRK2;SLITRK2;SLITRK2;SLITRK2 | 0.75933589 | X |
| cg10662395 | HCN2 | 0.7575475 | 19 |
| cg03398919 | | 0.7195255 | 2 |
| cg01560464 | SLITRK4 | 0.7117922 | X |
| cg21035907 | | 0.7065175 | 8 |
| cg13868165 | FAM19A5 | 0.65275568 | 22 |
| cg12087615 | KRT1 | 0.64903985 | 12 |
| cg14752426 | SLITRK4 | 0.63076927 | X |
| cg22623223 | PTPRN2;PTPRN2;PTPRN2 | 0.60374616 | 7 |
| cg17838026 | KCNC3 | 0.57998745 | 19 |

There were 175 probes, in addition to the above-listed 20 probes, that showed differential values (absolute values) of 0.15 or higher between the GC1_4 and nfb1-4 samples (no detailed data shown).

Table 14 below lists the top 20 of the highest absolute differential values among the results of the comparison of methylations between the induced malignant stem cells (NGC1_6) and the induced pluripotent stem cells (nfb1-4).

[Table 14]

| Table 14: Comparison of methylations between the induced malignant stem cells (NGC1_6) and the induced pluripotent stem cells (nfb1-4) | | | |
|---|---|---|---|
| TargetID | UCSC_RefGene_Name | Absolute differential value | CHR |
| cg05135828 | SLITRK4 | 0.961054888 | X |
| cg23690264 | SLITRK4 | 0.91160146 | X |
| cg04642759 | SLITRK2;SLITRK2;SLITRK2;SLITRK2 | 0.86422331 | X |
| cg10016783 | SLITRK2;SLITRK2;SLITRK2;SLITRK2 | 0.085374327 | X |
| cg23784675 | BRUNOL4;BRUNOL4;BRUNOL4;BRUNOL4 | 0.8461818 | 18 |
| cg20976286 | OCA2 | 0.84257465 | 15 |
| cg25237542 | SLITRK4 | 0.8275699 | X |

(continued)

| Table 14: Comparison of methylations between the induced malignant stem cells (NGC1_6) and the induced pluripotent stem cells (nfb1-4) | | | |
|---|---|---|---|
| TargetID | UCSC_RefGene_Name | Absolute differential value | CHR |
| cg09720420 | SLITRK4 | 0.80247599 | X |
| cg19932577 | | 0.79393255 | 8 |
| cg01560464 | SLITRK4 | 0.784122 | X |
| cg03020597 | SLITRK2;SLITRK2;SLITRK2;SLITRK2 | 0.7792446 | X |
| cg18701656 | SLITRK2;SLITRK2;SLITRK2;SLITRK2 | 0.743398 | X |
| cg03398919 | | 0.6984975 | 2 |
| cg21035907 | | 0.6888733 | 8 |
| cg10662395 | HCN2 | 0.6845959 | 19 |
| cg12087615 | KRT1 | 0.68453145 | 17 |
| cg13868165 | FAM19A5 | 0.65106594 | 22 |
| cg14752426 | SLITRK4 | 0.63967026 | X |
| cg22623223 | PTPRN2;PTPRN2;PTPRN2 | 0.61275744 | 7 |
| cg17838026 | KCNC3 | 0.57469071 | 19 |

There were 328 probes, in addition to the above-listed 20 probes, that showed differential values (absolute values) of 0.15 or higher between the NGC1_6 and nfb1-4 samples (no detailed data shown).

Table 15 below lists the top 20 of the highest absolute differential values among the results of the comparison of methylations between the induced malignant stem cells (CC3_5) and the induced pluripotent stem cells (nfb2-17).

[Table 15]

| Table 15: Comparison of methylations between the induced malignant stem cells (CC3_5) and the induced pluripotent stem cells (nfb2-17) | | | |
|---|---|---|---|
| TargetID | UCSC_RefGene_Name | Absolute differential value | CHR |
| cg14175690 | TBX15 | 0.87136775 | 1 |
| cg12848223 | NRK | 0.81811498 | X |
| cg22508145 | CPAMD8 | 0.7880505 | 19 |
| cg04707332 | TBX15 | 0.776628474 | 1 |
| cg0778529 | IL1RAPL2 | 0.7421806 | X |
| cg09628195 | | 0.73383247 | 1 |
| cg19791271 | | 0.7238544 | 17 |
| cg24434800 | | 0.72053399 | 1 |
| cg23949973 | NRK | 0.69776378 | X |
| cg17436134 | | 0.6946001 | 1 |
| cg26104752 | TBX15 | 0.67629192 | 1 |
| cg14825735 | | 0.6676352 | 6 |
| cg22198853 | | 0.6293045 | 6 |
| cg05135828 | SLITRK4 | 0.61202168 | X |
| cg13107768 | | 0.6070508 | 1 |

(continued)

| Table 15: Comparison of methylations between the induced malignant stem cells (CC3_5) and the induced pluripotent stem cells (nfb2-17) | | | |
|---|---|---|---|
| TargetID | UCSC_RefGene_Name | Absolute differential value | CHR |
| cg74189340 | OPCML | 0.60688017 | 11 |
| cg10145246 | TBX15 | 0.60502888 | 1 |
| cg08848171 | IL1RAPL2 | 0.5737146 | X |
| cg00597445 | CRMP1;CRMP1 | 0.573366 | 4 |
| cg08380440 | | 0.5724823 | 11 |

There were 253 probes, in addition to the above-listed 20 probes, that showed differential values (absolute values) of 0.15 or higher between the CC3_5 and nfb2-17 samples (no detailed data shown).

Table 16 below lists the top 20 of the highest absolute differential values among the results of the comparison of methylations between the induced malignant stem cells (GC2_1) and the induced pluripotent stem cells (nfb2-17).

[Table 16]

| Table 16: Comparison of methylations between the induced malignant stem cells (GC2_1) and the induced pluripotent stem cells (nfb2-17) | | | |
|---|---|---|---|
| TargetID | UCSC_RefGene_Name | Absolute differential value | CHR |
| cg14175690 | TBX15 | 0.85855773 | 1 |
| cg17436134 | | 0.81054302 | 1 |
| cg22508145 | CPAMD8 | 0.7978189 | 19 |
| cg04707332 | TBX15 | 0.75819962 | 1 |
| cg24434800 | | 0.75558122 | 1 |
| cg09628195 | | 0.74526895 | 1 |
| cg26104752 | TBX15 | 0.68761959 | 1 |
| cg24189340 | OPCML | 0.68303887 | 11 |
| cg26411441 | HSPA12B | 0.6738652 | 20 |
| cg13107768 | | 0.64345786 | 1 |
| cg14010405 | GTF2B | 0.6364644 | 1 |
| cg10145246 | TBX15 | 0.61224518 | 1 |
| cg17811845 | GTF2B | 0.6093257 | 1 |
| cg16415058 | SORCS1;SORCS1 | 0.60199244 | 10 |
| cg14825735 | | 0.5866293 | 6 |
| cg03398919 | | 0.5851406 | 2 |
| cg20405017 | CA10;CA10;CA10;CA10 | 0.5805757 | 17 |
| cg16692538 | | 0.56036213 | 5 |
| cg22623223 | PTPRN2;PTPRN2;PTPRN2 | 0.54931728 | 7 |
| cg21966410 | AR | 0.53896224 | X |

There were 366 probes, in addition to the above-listed 20 probes, that showed differential values (absolute values) of 0.15 or higher between the GC2-1 and nfb2-17 samples (no detailed data shown).

Table 17 below lists the top 20 of the highest absolute differential values among the results of the comparison of methylations between the induced malignant stem cells (GC1_4) and the induced malignant stem cells (NGC1_6).

[Table 17]

Table 17: Comparison of methylations between the induced malignant stem cells (GC1_4) and the induced malignant stem cells (NGC1_6)

| TargetID | UCSC_RefGene_Name | Absolute differential value | CHR |
|---|---|---|---|
| cg07194250 | MGC16121;MIR503 | 0.2867206 | X |
| cg11285003 | HCN1 | 0.2442868 | 5 |
| cg22955387 | MGC16121;MIR503 | 0.2352464 | X |
| cg01972979 | MGC16121;MIR503 | 0.2352133 | X |
| cg10764762 | EDNRB;EDNRB;EDNRB | 0.2170448 | 13 |
| cg17541715 | | 0.2004952 | 7 |
| cg13294849 | SOX2OT | 0.1984132 | 3 |
| cg01817364 | | 0.1940695 | 5 |
| cg16499677 | C14orf37 | 0.189448 | 14 |
| cg04109661 | MGC16121 | 0.1864054 | X |
| cg08380440 | | 0.1861819 | 11 |
| cg21858113 | SCN4B;SCN4B;SCN4B;SCN4B | 0.1789004 | 11 |
| cg21117734 | | 0.17773098 | 20 |
| cg05449100 | | 0.1746908 | 11 |
| cg20978230 | MIR503;MGC 16121 | 0.1746845 | X |
| cg11931762 | | 0.169656 | 20 |
| cg26444951 | | 0.1690091 | 4 |
| cg02650401 | SOX2OT | 0.1644524 | 3 |
| cg11750736 | TMEM220 | 0.1642664 | 17 |
| cg19449948 | | 0.1604955 | 15 |

There were 26 probes, in addition to the above-listed 20 probes, that showed differential values (absolute values) of 0.15 or higher between the GC1_4 and NGC1_6 samples (no detailed data shown).

In these analyses, which used probes (a total of 6659 probes) capable of detecting regions that show different methylations between normal and cancer cells as observed in various tissues and multiple carcinomas, the methylation levels were deemed different between the two samples when the difference represented by a differential value (absolute value) of 0.15 or higher was observed, and therefore the probes that exhibited a differential methylation value (absolute value) of 0.15 were selected.

The induced malignant stem cells analyzed in this Example can be considered as cells characterized both by aberration of methylations in endogenous genomic DNAs in the regions that show different methylations between normal and cancer cells as observed in multiple carcinomas, and by expression of the ES cell-specific genes (OCT3/4, NANOG, SOX2, ZFP42).

Preferably, the induced malignant stem cells analyzed in this Example can be considered as cells characterized both by aberration of methylations in endogenous genomic DNAs as represented by a differential value (absolute value) of 0.15 or higher in at least 20 sites of the regions that show different methylations between normal and cancer cells as observed in multiple carcinomas, and by expression of the ES cell-specific genes (OCT3/4, NANOG, SOX2, ZFP42).

More preferably, the induced malignant stem cells analyzed in this Example can be described as cells characterized both by aberration of methylations of endogenous genomic DNAs as represented by a differential value (absolute value) of 0.30 or higher in at least 20 sites of the regions that show different methylations between normal and cancer cells as observed in multiple carcinomas, and by expression of the ES cell-specific genes (OCT3/4, NANOG, SOX2, ZFP42).

Example 10: Detection for somatic mutations of endogenous genomic DNAs of induced malignant stem cells

In this Example, (1)(b) somatic mutations of cancer-related gene regions (oncogenes, tumor suppressor genes, kinase genes) in endogenous genomic DNAs of induced malignant stem cells were detected, in comparison with those in genomic DNAs of cells derived from non-cancer site tissues.

(10-1) Materials

The (1)(b) somatic mutations of cancer-related gene regions (oncogenes, tumor suppressor genes, kinase genes) in endogenous genomic DNAs of induced malignant stem cells were detected by SNV (Single Nucleotide Variants) analysis.

The following samples were used in the detection for (1)(b) somatic mutations of cancer-related gene regions (oncogenes, tumor suppressor genes, kinase genes) in endogenous genomic DNAs of induced malignant stem cells:

- cell population (ngc2) derived from fresh gastric non-cancer site tissues, cell population (gc2) derived from fresh gastric-cancer site tissues, and induced malignant stem cells (GC2_1, GC2_2, GC2_4, GC2_5, GC2_7, GC2_10, GC2_13, GC2_16) prepared from fresh gastric cancer tissues (gc2), which were collected from the individual of donor No. 1;
- cell population (ngc3) derived from fresh colon non-cancer site tissues, and induced malignant stem cells (CC3_5, CC3_6) prepared from fresh colon cancer tissues (cc3), which were collected from the individual of donor No. 2;
- cell population (ngc1) derived from fresh gastric non-cancer site tissues, induced malignant stem cells (GC1_4, GC1_6, GC1_7, GC1_8, GC1_9) prepared from fresh gastric cancer tissues (gc1), and induced non-malignant stem cells (NGC1_6, NGC1_7) prepared from fresh gastric non-cancer site tissues (ngc1), which were collected from the individual of donor No. 3;
- cell population (ncc1) derived from colon non-cancer site tissues, and induced malignant stem cells (CC1_1, CC1_2, CC1_7, CC1_8, CC1_9, CC1_11, CC1_12, CC1_17, CC1_18, CC1_25) prepared from fresh colon cancer tissues (cc1), which were collected from the individual of donor No. 4; and
- cell population (ncc4) derived from fresh colon non-cancer site tissues, and induced malignant stem cells (CC4_c, CC4_(3), CC4_(6), CC4_(9)_5, CC4_(9)_7, CC4_(9)_11, CC4_(9)_13, CC4_(3)_10, CC4_(4), CC4_6, CC4_30) prepared from fresh colon cancer tissues (cc4), which were collected from the individual of donor No. 5.

(10-2) Quality evaluation

In the process of quality evaluation for genomic DNAs of the samples, their concentration appropriateness and quality (less degradation) were confirmed by the following procedure.

- Run 1: concentration: PicoGreen; quality: agarose gel electrophoresis; purity: NanoDrop
- Run 2: concentration: PicoGreen; quality: agarose gel electrophoresis; purity: NanoDrop

(10-3) Library construction

Library construction was basically performed using SureSelect™ Reagent Kit (Agilent) in accordance with Protocol Version 1.3.1 for SureSelect™ Target Enrichment System for Illumina Paired-End Sequencing Library (Agilent).

First, the genomic DNA of a sample was sonicated using Ultrasonic DNA Shearing System (Covaris Inc.) to randomly fragment the genomic DNA into approximately 150 bp segments. The fragmented genomic DNAs were subjected to end repair, addition of "A" bases to 3' ends, and adapter ligation to form the template DNA. Thereafter, the DNA sample was used to perform PCR amplification. The PCR amplified product was subjected to enrichment of fragments including targeted regions using SureSelect Human Kinome Kit and again to PCR amplification, whereby a library was constructed.

The genes targeted by SureSelect Human Kinome Kit in this Example are listed in the following table.

[Table 18]

| Table 18: List of genes targeted by SureSelect Human Kinome Kit | | |
|---|---|---|
| Gene Group | No. | Names of Kinase Genes |
| Protein Kinase Genes | 517 | AAK1, AATK, ABL1, ABL2, ACTR2, ACVR1, ACVR1B, ACVR1C, ACVR2A, ACVR2B, ACVRL1, ADCK1, ADCK4, ADCK5, ADRBK1, ADRBK2, AKT1, AKT2, AKT3, ALK, ALPK1, ALPK2, ALPK3, AMHR2, ANKK1, ARAF, ATM, ATR, AURKA, AURKB, AURKC, AXL, BCKDK, BLK, BMP2K, BMPR1A, BMPR1B, BMPR2, BMX, BRAF, BRD2, BRD3, BRD4, BRDT, BRSK1, BRSK2, BTK, BUB1, BUB1B, C9orf96, CABC1, CAMK1, CAMK1D, CAMK1G, CAMK2A, CAMK2B, CAMK2D, CAMK2G, CAMK4, CAMKK1, CAMKK2, CAMKV, CASK, CCRK, CDC2, CDC2L2, CDC2L5, CDC2L6, CDC42BPA, CDC42BPB, CDC42BPG, CDC7, CDK10, CDK2, CDK3, CDK4, CDK5, CDK6, CDK7, CDK8, CDK9, CDKL1, CDKL2, CDKL3, CDKL4, CDKL5, CHEK1, CHEK2, CHUK, CIT, CLK1, CLK2, CLK3, CLK4, CNKSR2, CRKRS, CSF1R, CSK, CSNK1A1, CSNK1A1L, CSNK1D, CSNK1E, CSNK1G1, CSNK1G2, CSNK1G3, CSNK2A1, CSNK2A2, DAPK1, DAPK2, DAPK3, DCLK1, DCLK2, DCLK3, DDR1, DDR2, DMPK, DSTYK, DYRK1A, DYRK1B, DYRK2, DYRK3, DYRK4, EEF2K, EGFR, EIF2AK1, EIF2AK2, EIF2AK3, EIF2AK4, EPHA1, EPHA10, EPHA2, EPHA3, EPHA4, EPHA5, EPHA6, EPHA7, EPHA8, EPHB1, EPHB2, EPHB3, EPHB4, EPHB6, ERBB2, ERBB3, ERBB4, ERN1, ERN2, FASTK, FER, FES, FGFR1, FGFR2, FGFR3, FGFR4, FGR, FLJ25006, FLT1, FLT3, FLT4, FRK, FYN, GAK, GCK, GRK1, GRK4, GRK5, GRK6, GRK7, GSG2, GSK3A, GSK3B, GUCY2C, GUCY2D, GUCY2F, HCK, HIPK1, HIPK2, HIPK3, HIPK4, HSPB8, HUNT ICK, IGF1R, IKBKB, IKBKE, ILK, INSR, INSRR, IRAK1, IRAK2, IRAK3, IRAK4, ITK, JAK1, JAK2, JAK3, KALRN, KDR, KIAA1804, KIT, KSR1, KSR2, LATS1, LATS2, LCK, LIMK1, LIMK2, LMTK2, LMTK3, LRRK1, LRRK2, LTK, LYN, MAK, MAP2K1, MAP2K2, MAP2K3, MAP2K4, MAP2K5, MAP2K6, MAP2K7, MAP3K1, MAP3K10, MAP3K11, MAP3K12, MAP3K13, MAP3K14, MAP3K15, MAP3K2, MAP3K3, MAP3K4, MAP3K5, MAP3K6, MAP3K7, MAP3K8, MAP3K9, MAP4K1, MAP4K2, MAP4K3, MAP4K4, MAP4K5, MAPK1, MAPK10, MAPK11, MAPK12, MAPK13, MAPK14, MAPK15, MAPK3, MAPK4, MAPK6, MAPK7, MAPK8, MAPK9, MAPKAPK2, MAPKAPK3, MAPKAPK5, MARK1, MARK2, MARK3, MARK4, MAST1, MAST2, MAST3, MAST4, MASTL, MATK, MELK, MERTK, MET, MGC42105, MINK1, MKNK1, MKNK2, MLKL, MOS, MST1R, MTOR, MUSK, MYLK, MYLK2, MYLK3, MYLK4, MYO3A, MYO3B, NEK1, NEK10, NEK11, NEK2, NEK3, NEK4, NEK5, NEK6, NEK7, NEK8, NEK9, NLK, NPR1, NPR2, NRBP1, NRBP2, NRK, NTRK1, NTRK2, NTRK3, NUAK1, NUAK2, OBSCN, OXSR1, PAK1, PAK2, PAK3, PAK4, PAK6, PAK7, PASK, PBK, PCTK1, PCTK2, PCTK3, PDGFRA, PDGFRB, PDIK1L, PDK1, PDK2, PDK3, PDK4, PDPK1, PFTK1, PFTK2, PHKG1, PHKG2, PIM1, PIM2, PIM3, PINK1, PKLR, PKMYT1, PKN1, PKN2, PKN3, PLK1, PLK2, PLK3, PLK4, PNCK, PRAGMIN, PRKAA1, PRKAA2, PRKACA, PRKACB, PRKACG, PRKCA, PRKCB, PRKCD, PRKCE, PRKCG, PRKCH, PRKCI, PRKCQ, PRKCZ, PRKD1, PRKD2, PRKD3, PRKDC, PRKG1, PRKG2, PRKX, PRKY, PRPF4B, PSKH1, PSKH2, PTK2, |

(continued)

| Table 18: List of genes targeted by SureSelect Human Kinome Kit | | |
|---|---|---|
| Gene Group | No. | Names of Kinase Genes |
| | | PTK2B, PTK6, PTK7, PXK, RAC1, RAF1, RAGE, RET, RIOK1, RIOK2, RIOK3, RIPK1, RIPK2, RIPK3, RIPK4, RNASEL, ROCK1, ROCK2, ROR1, ROR2, ROS1, MST4, RPS6KA1, RPS6KA2, RPS6KA3, RPS6KA4, RPS6KA5, RPS6KA6, RPS6KB1, RPS6KB2, RPS6KC1, RPS6KL1, RYK, SBK1, SBK2, SCYL1, SCYL2, SCYL3, SGK1, SGK196, SGK2, SGK269, SGK3, SGK493, SIK1, SIK2, SIK3, SLK, SMG1, SNRK, SPEG, SRC, SRM, SRMS, SRPK1, SRPK2, SRPK3, STK10, STK11, STK16, STK17A, STK17B, STK19, STK24, STK25, STK3, STK31, STK32A, STK32B, STK32C, STK33, STK35, STK36, STK38, STK38L, STK39, STK4, STK40, STRADA, STRADB, STYK1, SYK, TAF1, TAF1L, TAOK1, TAOK2, TAOK3, TBCK, TBK1, TEC, TEK, TESK1, TESK2, TEX14, TGFBR1, TGFBR2, TIE1, TLK1, TLK2, TNIK, TNK1, TNK2, TNNI3K, TP53RK, TRIB1, TRIB2, TRIB3, TRIM24, TRIM28, TRIM33, TRIO, TRPM6, TRPM7, TRRAP, TSSK1B, TSSK2, TSSK3, TSSK4, TSSK6, TTBK1, TTBK2, TTK, TTN, TXK, TYK2, TYRO3, UHMK1, ULK1, ULK2, ULK3, ULK4, VRK1, VRK2, VRK3, WEE1, WEE2, WNK1, WNK2, WNK3, WNK4, YES1, YSK4, ZAK, ZAP70 |
| PI3K Domain Proteins | 12 | PIK3C2A, PIK3C2B, PIK3C2G, PIK3C3, PIK3CA, PIK3CB, PIK3CD, PIK3CG, PI4KA, PI4KB, PI4K2B, PI4K2A |
| Diglyceride Kinases | 13 | AGK, CERK, DGKA, DGKB, DGKD, DGKE, DGKG, DGKH, DGKI, DGKQ, DGKZ, SPHK1, SPHK2 |
| PIK3 Regulatory Components | 6 | PIK3R1, PIK3R2, PIK3R3, PIK3R4, PIK3R5, PIK3R6 |
| Inositol polyphosphate kinases [IPK domain] | 9 | IP6K1, IP6K2, IP6K3, IPMK, IPPK, ITPK1, ITPKA, ITPKB, ITPKC |
| PIP4/PIP5 Kinases | 9 | PIKFYVE, PIP4K2A, PIP4K2B, PIP4K2C, PIP5K1A, PIP5K1B, PIP5K1C, PIP5KL1, PIPSL |
| Cancer Genes | 20 | CDC6, CHD3, HRAS, KRAS, NRAS, PTEN, CDH1, TP53, CDKN2A, CDKN2B, APC, RB1, CTNNB1, BRCA1, BRCA2, NF1, NF2, GATA3, MYC, INPP4A |
| Additional Breast Cancer Genes | 16 | COL1A1, GAB1, HAUS3, IRS2, IRS4, KIAA1468, KLHL4, NFKB1, NFKBIA, NFKBIE, PALB2, RHEB, RNF220, SNX4, SP1, USP28 |
| More Cancer Genes | 10 | CCND1, CCND2, CCND3, ESR1, ESR2, FBXW7, IDH1, IDH2, MLH1, TERT |
| Total Genes | 612 | |

(10-4) Sequencing

DNA clusters were formed using TruSeq PE Cluster Kit v3 cBot - HS (illumina) in the DNA template amplification system (cBot) (illumina). These clusters were sequenced using TruSeq SBS Kit v3 - HS (illumina) in HiSeq2000 (illumina). As a result of the sequencing, read sequences with a data amount of 1.03-4.46 Gb were obtained from the genomic DNAs of respective cells/tissues, as shown in the table below.

[Table 19]

Table 19: Sequenced lengths

| Group ID | Sample ID | Description | RG Sample ID | Original (Gb) |
|---|---|---|---|---|
| Donor No. 1 | ngc2 | Non-gastric cancer tissues | MG_EX_2094_001 | 2.99 |
| Donor No. 1 | GC2_1 | Induced malignant stem cells | MG_EX_2063_001 | 1.30 |
| Donor No. 1 | GC2_2 | Induced malignant stem cells | MG_EX_2063_006 | 1.48 |
| Donor No. 1 | GC2_4 | Induced malignant stem cells | MG_EX_2063_007 | 1.23 |
| Donor No. 1 | GC2_5 | Induced malignant stem cells | MG_EX_2063_003 | 1.12 |
| Donor No. 1 | GC2_7 | Induced malignant stem cells | MG_EX_2063_004 | 1.49 |
| Donor No. 1 | GC2_10 | Induced malignant stem cells | MG_EX_2063_005 | 1.34 |
| Donor No. 1 | GC2_13 | Induced malignant stem cells | MG_EX_2063_011 | 1.29 |
| Donor No. 1 | GC2_16 | Induced malignant stem cells | MG_EX_2063_008 | 1.54 |
| Donor No. 2 | ncc3 | Non-colon cancer tissues | MG_EX_2094_003 | 4.46 |
| Donor No. 2 | CC3_5 | Induced malignant stem cells | MG_EX_2063_009 | 1.57 |
| Donor No. 2 | CC3_6 | Induced malignant stem cells | MG_EX_2063_010 | 3.32 |
| Donor No. 3 | ngc1 | Non-gastric cancer tissues | MG_EX_2093_001 | 1.03 |
| Donor No. 3 | gc1_4 | Induced malignant stem cells | MG_EX_2093_002 | 1.21 |
| Donor No. 3 | gc1_6 | Induced malignant stem cells | MG_EX_2093_003 | 1.36 |
| Donor No. 3 | gc1_7 | Induced malignant stem cells | MG_EX_2093_004 | 1.23 |
| Donor No. 3 | gc1_8 | Induced malignant stem cells | MG_EX_2093_005 | 1.28 |
| Donor No. 3 | gc1_9 | Induced malignant stem cells | MG_EX_2093_006 | 1.48 |
| Donor No. 3 | ngc1_6 | Induced non-malignant stem cells | MG_EX_2093_007 | 2.95 |
| Donor No. 3 | ngc1_7 | Induced non-malignant stem cells | MG_EX_2093_008 | 3.06 |
| Donor No. 4 | ncc1 | Non-colon cancer tissues | MG_EX_2093_009 | 3.69 |
| Donor No. 4 | cc1_1 | Induced malignant stem cells | MG_EX_2093_011 | 2.64 |
| Donor No. 4 | cc1_2 | Induced malignant stem cells | MG_EX_2093_032 | 3.58 |
| Donor No. 4 | cc1_7 | Induced malignant stem cells | MG_EX_2093_013 | 2.99 |
| Donor No. 4 | cc1_8 | Induced malignant stem cells | MG_EX_2093_014 | 3.50 |
| Donor No. 4 | CC1_9 | Induced malignant stem cells | MG_EX_2093_035 | 3.37 |
| Donor No. 4 | cc1_11 | Induced malignant stem cells | MG_EX_2093_016 | 3.12 |
| Donor No. 4 | cc1_12 | Induced malignant stem cells | MG_EX_2093_036 | 3.11 |
| Donor No. 4 | cc1_17 | Induced malignant stem cells | MG_EX_2093_017 | 3.84 |
| Donor No. 4 | cc1_18 | Induced malignant stem cells | MG_EX_2093_018 | 3.99 |
| Donor No. 4 | cc1_25 | Induced malignant stem cells | MG_EX_2093_019 | 3.13 |
| Donor No. 5 | ncc4 | Non-colon cancer tissues | MG_EX_2093_020 | 3.94 |
| Donor No. 5 | cc4_c | Induced malignant stem cells | MG_EX_2093_022 | 3.48 |
| Donor No. 5 | cc4_(3) | Induced malignant stem cells | MG_EX_2093_042 | 3.79 |
| Donor No. 5 | cc4_(6) | Induced malignant stem cells | MG_EX_2093_030 | 3.69 |
| Donor No. 5 | cc4_(9)_5 | Induced malignant stem cells | MG_EX_2093_049 | 2.93 |
| Donor No. 5 | cc4_(9)_7 | Induced malignant stem cells | MG_EX_2093_051 | 3.36 |
| Donor No. 5 | cc4_(9)_11 | Induced malignant stem cells | MG_EX_2093_053 | 2.94 |
| Donor No. 5 | cc4_(9)_13 | Induced malignant stem cells | MG_EX_2093_054 | 3.56 |
| Donor No. 5 | cc4_(3)_10 | Induced malignant stem cells | MG_EX_2093_027 | 3.81 |
| Donor No. 5 | cc4_(4) | Induced malignant stem cells | MG_EX_2093_029 | 3.37 |
| Donor No. 5 | cc4_6 | Induced malignant stem cells | MG_EX_2093_038 | 3.87 |
| Donor No. 5 | cc4_30 | Induced malignant stem cells | MG_EX_2093_040 | 2.77 |

(10-5) Bioinformatics

Adapter sequences and bad-quality bases were trimmed off from the read data using cutadapt method. The trimmed reads were then mapped to reference sequences using BWA (Burrows-Wheeler Aligner).

Next, SNVs (Single Nucleotide Variants)/InDels (insertions/deletions) were detected using GATK (The Genome Analysis Toolkit). The detected SNVs/InDels were annotated using dbSNP (Single Nucleotide Polymorphism database) (NCBI Build 135), CCDS (Consensus CDS) (NCBI release 20111122), RefSeq (NCBI Reference Sequence) (UCSC Genome Browser (dumped 20111122)) and other databases.

The following databases were used for bioinformatics analysis:

- Reference sequences: UCSC Genome Browser hg19
  (http://hgdownload.cse.ucsc.edu/goldenPath/hg19/chromosomes/)
- Targeted regions: Agilent SureSelect Human Kinome Kit
- dbSNP: NCBI Build 135
  (ftp: //ftp.ncbi.nlm.nih.gov/snp/organisms/human_9606/ASN1_flat/)
- CCDS: NCBI release 20111122
  (ftp: //ftp.ncbi.nlm.nih.gov/pub/CCDS/archive/HsGRCH37.3/CCDS.current.txt)
- RefSeq: UCSC Genome Browser (dumped 20111122)
  (ftp: //hgdownload.cse.ucsc.edu/apache/htdocs/goldenPath/hg19/database/refGene.txt.gz)
- Encode: UCSC Genome Browser ENCODE/GENCODE Version 7
  (ftp://hgdownload.cse.ucsc.edu/apache/htdocs/goldenPath/hg19/database/wgEncodeGencode BasicV7.txt.gz)
- 1000Genomes: release 20111011
  (ftp://ftp.1000genomes.ebi.ac.uk/voll/ftp/release/20110521/)

* Number of samples compiled: 1,092 samples (Among them, 89 are of Japanese (JPT) origin)

The following software versions were used for bioinformatics analysis:

- Burrows-Wheeler Aligner (BWA): 0.6.2
  (http://bio-bwa.sourceforge.net/index.shtml)
- The Genome Analysis Toolkit (GATK): 1.5-32-g2761da9
  (http://www.broadinstitute.org/gsa/wiki/index.php/The_Genome_Analysis_Toolkit)
- Picard: 1.73 (http://picard.sourceforge.net/command-line-overview.shtml)

The SNVs detected in 46 samples divided into 5 groups (Groups 1-5) were primarily refined down using the following criteria:

- SNVs passing the filtering using GATK scoring,
- SNVs predicted as non-synonymous mutations (missence, nonsense, read-through), and
- Pick up only SNVs with a depth (DepthOfCoverage value) of 8 or higher.

After the completion of the primary refining, secondary refinining was further performed using the following criterion:

- SNVs deemed to be somatic mutations (those having different genotypes among samples in the same group).

(10-6) Results

The SNVs passing the secondary refinining in respective sample groups were summarized in the table below.
[Table 20]

Table 20:

## Group 1

| Chrom | Chrom Start | Chrom End | Reference | Alternatives | Type | Gene | Function | ngc2 MG_EX_2 094_001 | GC2_1 MG_EX_2 063_001 | GC2_2 MG_EX_2 063_006 | GC2_4 MG_EX_2 063_007 | GC2_5 MG_EX_2 063_003 | GC2_7 MG_EX_2 063_004 | GC2_10 MG_EX_2 063_005 | GC2_13 MG_EX_2 063_011 | GC2_16 MG_EX_2 063_008 | QC QC |
|---|---|---|---|---|---|---|---|---|---|---|---|---|---|---|---|---|---|
| 1 | 1575715 | 1575715 | C | T | SNV | CDK11B,CDK11A | Missense | C/C | C/T | C/C | C/T | C/T | C/C | C/C | C/T | C/C | |
| 1 | 32828420 | 32828420 | G | A | SNV | TSSK3,LOC100128071,RP4-811H24.6 | Missense | G/G | G/G | G/G | G/G | G/G | G/G | G/G | G/A | G/G | |
| 1 | 38184063 | 38184063 | C | A | SNV | EPHA10 | Missense | C/A | C/C | C/C | C/C | C/C | C/A | C/C | C/C | C/C | O.K. |
| 1 | 228430947 | 228430947 | C | G | SNV | OBSCN | Missense | C/G | C/G | C/G | C/C | C/C | C/G | C/G | C/G | C/G | |
| 1 | 228464248 | 228464248 | T | G | SNV | OBSCN,AL353593.1 | Missense | T/G | T/G | T/T | T/G | T/G | T/G | T/G | T/G | T/G | O.K. |
| 2 | 148676144 | 148676144 | A | C | SNV | ACVR2A | Missense | A/C | A/C | A/A | A/C | A/A | A/C | A/C | A/C | A/C | |
| 2 | 174086046 | 174086046 | A | G | SNV | ZAK,MLK7-AS1,AC013461.1,AC013461.2 | Missense | A/A | A/A | A/A | A/A | A/A | A/A | A/A | A/G | A/A | |
| 2 | 174086076 | 174086076 | A | C | SNV | ZAK,MLK7-AS1,AC013461.1,AC013461.2 | Missense | A/A | A/A | A/A | A/A | A/A | A/A | A/A | A/C | A/A | O.K. |
| 2 | 179500729 | 179500729 | C | T | SNV | TTN,MIR548N,AC010680.1 | Missense | C/C | C/C | C/C | C/C | C/C | C/C | C/C | C/T | C/C | O.K. |
| 2 | 209195248 | 209195248 | C | A | SNV | PIKFYVE | Missense | C/A | C/C | C/C | C/C | C/C | C/C | C/C | C/C | C/C | |
| 2 | 242047605 | 242047605 | T | G | SNV | PASK | Missense | T/T | T/G | T/T | T/G | T/T | T/T | T/G | T/T | T/T | |
| 3 | 41267227 | 41267227 | A | G | SNV | CTNNB1 | Missense | A/A | A/G | A/A | A/A | A/A | A/A | A/A | A/A | A/A | O.K. |
| 3 | 43389767 | 43389767 | G | T | SNV | SNRK | Missense | G/G | G/G | G/G | G/G | G/G | G/G | G/T | G/T | G/T | |
| 3 | 196529982 | 196529982 | A | G | SNV | PAK2 | Missense | A/A | A/A | A/A | A/A | A/G | A/A | A/A | A/A | A/A | |
| 4 | 1804786 | 1804786 | G | A | SNV | FGFR3 | Missense | G/G | G/G | G/G | G/G | G/G | G/G | G/G | G/A | G/G | O.K. |
| 4 | 66467418 | 66467418 | A | C | SNV | EPHA5 | Missense | A/A | A/A | A/A | A/C | A/C | A/A | A/A | A/A | A/A | |
| 4 | 107168372 | 107168372 | T | G | SNV | TBCK | Missense | T/T | T/T | T/T | T/G | T/T | T/G | T/G | T/T | T/T | |
| 5 | 14509521 | 14509521 | A | G | SNV | TRIO | Missense | A/A | A/G | A/G | A/G | A/G | A/G | A/G | A/G | A/G | |
| 5 | 148897392 | 148897392 | T | G | SNV | CSNK1A1 | Missense | T/T | T/T | T/T | T/T | T/T | T/T | T/T | T/G | T/T | Questionable |
| 6 | 36489585 | 36489585 | C | A | SNV | STK38 | Missense | C/C | C/A | C/A | C/A | C/C | C/A | C/A | C/C | C/A | |
| 6 | 43111336 | 43111336 | G | T | SNV | PTK7 | Missense | G/T | G/G | G/G | G/G | G/G | G/G | G/G | G/G | G/G | |
| 6 | 91226381 | 91226381 | G | A | SNV | MAP3K7 | Missense | G/G | G/G | G/G | G/G | G/G | G/G | G/G | G/A | G/G | O.K. |
| 6 | 110942394 | 110942394 | G | T | SNV | CDK19 | Missense | G/T | G/G | G/G | G/G | G/G | G/G | G/G | G/G | G/G | |
| 7 | 14724963 | 14724963 | C | T | SNV | DGKB | Missense | C/C | C/C | C/C | C/C | C/C | C/C | C/C | C/C | C/T | O.K. |
| 7 | 40132405 | 40132405 | A | C | SNV | CDK13 | Missense | A/A | A/A | A/A | A/A | A/A | A/A | A/A | A/C | A/A | O.K. |
| 7 | 40132455 | 40132455 | A | T | SNV | CDK13 | Missense | A/A | A/A | A/A | A/A | A/A | A/A | A/A | A/T | A/A | O.K. |
| 7 | 40134451 | 40134451 | A | G | SNV | CDK13 | Missense | A/A | A/A | A/A | A/A | A/A | A/A | A/A | A/G | A/A | O.K. |
| 7 | 44259762 | 44259762 | T | G | SNV | CAMK2B | Missense | T/T | T/T | T/G | T/T | T/T | T/T | T/T | T/G | T/T | |
| 7 | 95216415 | 95216415 | C | A | SNV | PDK4 | Missense | C/C | C/A | C/A | C/C | C/A | C/A | C/C | C/A | C/A | |
| 7 | 98547196 | 98547196 | G | T | SNV | TRRAP | Missense | G/T | G/G | G/G | G/G | G/G | G/G | G/G | G/G | G/G | |
| 8 | 8239069 | 8239069 | C | A | SNV | SGK223,AC068353.1 | Missense | C/A | C/C | C/C | C/C | C/C | C/C | C/C | C/C | C/C | |
| 9 | 77403574 | 77403574 | C | A | SNV | TRPM6 | Missense | C/C | C/C | C/C | C/A | C/C | C/C | C/A | C/A | C/C | |
| 10 | 75585058 | 75585058 | G | A | SNV | CAMK2G | Missense | G/G | G/G | G/G | G/G | G/G | G/G | G/G | G/A | G/G | |

| Chr | Pos Start | Pos End | Ref | Alt | Type | Gene | Effect | S1 | S2 | S3 | S4 | S5 | S6 | S7 | S8 | Status |
|---|---|---|---|---|---|---|---|---|---|---|---|---|---|---|---|---|
| 10 | 121214789 | 121214789 | T | G | SNV | GRK5 | Missense | T/T | T/T | T/T | T/T | T/T | T/T | T/T | T/G | |
| 12 | 121678341 | 121678341 | G | T | SNV | CAMKK2,AC084018.1 | Missense | G/G | G/T | G/T | G/T | G/T | G/T | G/G | G/T | |
| 12 | 121712280 | 121712280 | T | G | SNV | CAMKK2,AC084018.1 | Missense | T/T | T/G | T/T | T/T | T/G | T/T | T/G | T/G | Questionable |
| 15 | 77474141 | 77474141 | A | C | SNV | PEAK1,AC087465.1 | Missense | A/A | A/A | A/A | A/A | A/A | A/A | A/C | A/A | Questionable |
| 15 | 77474144 | 77474144 | T | C | SNV | PEAK1,AC087465.1 | Missense | T/T | T/T | T/T | T/T | T/T | T/T | T/C | T/T | Questionable |
| 15 | 77474163 | 77474163 | C | T | SNV | PEAK1,AC087465.1 | Missense | C/C | C/C | C/C | C/C | C/C | C/C | C/T | C/C | Questionable |
| 15 | 77474172 | 77474172 | G | A | SNV | PEAK1,AC087465.1 | Missense | G/G | G/G | G/G | G/G | G/G | G/G | G/A | G/G | |
| 15 | 90627535 | 90627535 | A | T | SNV | IDH2 | Missense | A/A | A/A | A/A | A/A | A/T | A/A | A/A | A/A | O.K. |
| 15 | 99250869 | 99250869 | A | T | SNV | IGF1R | Missense | A/A | A/A | A/A | A/A | A/A | A/A | A/T | A/A | O.K. |
| 16 | 46744689 | 46744689 | C | A | SNV | MYLK3 | Missense | C/C | C/A | C/A | C/A | C/C | C/A | C/A | C/A | |
| 17 | 7796815 | 7796815 | G | C | SNV | CHD3 | Missense | G/C | G/C | G/C | G/C | G/C | G/C | G/C | G/G | |
| 17 | 7796819 | 7796819 | T | C | SNV | CHD3 | Missense | T/T | T/C | T/C | T/C | T/T | T/T | T/C | T/T | |
| 17 | 26369915 | 26369915 | G | A | SNV | NLK | Missense | G/G | G/G | G/G | G/G | G/G | G/G | G/A | G/G | O.K. |
| 18 | 59919898 | 59919898 | C | A | SNV | KIAA1468 | Missense | C/A | C/C | C/C | C/C | C/C | C/C | C/C | C/C | |
| 19 | 48997039 | 48997039 | C | G | SNV | LMTK3 | Missense | C/C | C/C | C/C | C/C | C/C | C/C | C/G | C/C | Questionable |
| 19 | 48997079 | 48997079 | C | T | SNV | LMTK3 | Missense | C/C | C/C | C/C | C/C | C/C | C/C | C/T | C/T | Questionable |
| 19 | 48997084 | 48997084 | G | C | SNV | LMTK3 | Missense | G/G | G/G | G/G | G/G | G/G | G/G | G/C | G/G | Questionable |
| 20 | 42204913 | 42204913 | A | C | SNV | SGK2 | Missense | A/A | A/C | A/C | A/G | A/A | A/C | A/A | A/C | |
| X | 105150441 | 105150441 | A | G | SNV | NRK | Missense | A/A | A/A | A/A | A/A | A/A | A/A | A/A | A/A | O.K. |

## Group 2

| Chrom | Chrom Start | Chrom End | Reference | Alternatives | Type | Gene | Function | ncc3 MG_EX_2094_003 | CC3_5 MG_EX_2063_009 | CC3_6 MG_EX_2063_010 | QC QC |
|---|---|---|---|---|---|---|---|---|---|---|---|
| 1 | 22919842 | 22919842 | C | G | SNV | EPHA8 | Missense | C/C | C/C | C/G | O.K. |
| 1 | 226923938 | 226923938 | A | C | SNV | ITPKB | Missense | C/C | A/C | C/C | Questionable |
| 1 | 228434292 | 228434292 | C | G | SNV | OBSCN | Missense | C/C | C/C | C/G | |
| 1 | 233497836 | 233497836 | C | A | SNV | KIAA1804,RP5-862P8.2 | Missense | C/A | C/C | C/C | |
| 2 | 209195248 | 209195248 | C | A | SNV | PIKFYVE | Missense | C/A | C/C | C/C | |
| 3 | 123988019 | 123988019 | C | A | SNV | KALRN | Missense | C/A | C/C | C/C | |
| 4 | 1804770 | 1804770 | A | C | SNV | FGFR3 | Missense | A/A | A/C | A/A | O.K. |
| 4 | 1804786 | 1804786 | G | A | SNV | FGFR3 | Missense | G/G | G/A | G/G | O.K. |
| 6 | 36489585 | 36489585 | C | A | SNV | STK38 | Missense | C/C | C/A | C/A | |
| 6 | 43111336 | 43111336 | G | T | SNV | PTK7 | Missense | G/T | G/G | G/G | |
| 6 | 110942394 | 110942394 | G | T | SNV | CDK19 | Missense | G/T | G/G | G/G | |
| 7 | 95216415 | 95216415 | C | A | SNV | PDK4 | Missense | C/A | C/C | C/A | |
| 7 | 98547196 | 98547196 | G | T | SNV | TRRAP | Missense | G/T | G/G | G/G | |
| 8 | 8239069 | 8239069 | C | A | SNV | SGK223,AC068353.1 | Missense | C/A | C/C | C/C | |
| 9 | 77403574 | 77403574 | C | A | SNV | TRPM6 | Missense | C/C | C/C | C/A | |
| 9 | 96055149 | 96055149 | T | G | SNV | WNK2 | Missense | T/T | T/T | T/G | Questionable |
| 10 | 75585058 | 75585058 | G | A | SNV | CAMK2G | Missense | G/G | G/A | G/G | O.K. |
| 11 | 108175544 | 108175544 | C | G | SNV | ATM | Missense | C/C | C/C | C/G | Questionable |
| 12 | 121678327 | 121678327 | C | T | SNV | CAMKK2,AC084018.1 | Missense | C/C | C/T | C/C | |
| 12 | 121678341 | 121678341 | G | T | SNV | CAMKK2,AC084018.1 | Missense | G/G | G/T | G/G | |
| 17 | 7796803 | 7796803 | T | C | SNV | CHD3 | Missense | T/T | T/C | T/C | Questionable |
| 17 | 41245693 | 41245693 | G | T | SNV | BRCA1 | Missense | G/T | G/G | G/G | |
| 18 | 18534948 | 18534948 | G | C | SNV | ROCK1 | Missense | G/C | G/G | G/G | |
| 19 | 49012702 | 49012702 | A | C | SNV | LMTK3 | Missense | A/A | A/C | A/C | Questionable |
| 20 | 42204913 | 42204913 | A | C | SNV | SGK2 | Missense | A/A | A/C | A/C | Questionable |

**Group 3**

| Chrom | Chrom Start | Chrom End | Reference | Alternatives | Type | Gene | Function | ngc1 MG_EX_2 093_001 | gc1_4 MG_EX_2 093_002 | gc1_6 MG_EX_2 093_003 | gc1_7 MG_EX_2 093_004 | gc1_8 MG_EX_2 093_005 | gc1_9 MG_EX_2 093_006 | ngc1_6 MG_EX_2 093_007 | ngc1_7 MG_EX_2 093_008 | QC |
|---|---|---|---|---|---|---|---|---|---|---|---|---|---|---|---|---|
| 1 | 9780836 | 9780836 | T | G | SNV | PIK3CD | Missense | T/T | T/G | T/G | T/G | T/G | T/G | T/T | T/T | Questionable |
| 1 | 16474932 | 16474932 | A | C | SNV | EPHA2 | Missense | A/C | A/C | A/A | A/C | A/C | A/C | A/A | A/A | |
| 1 | 38184063 | 38184063 | C | A | SNV | EPHA10 | Missense | C/C | C/C | C/C | C/C | C/C | C/C | C/A | C/A | |
| 1 | 228430947 | 228430947 | C | G | SNV | OBSCN | Missense | C/G | C/G | C/G | C/G | C/G | C/G | C/C | C/C | |
| 1 | 228434292 | 228434292 | G | G | SNV | OBSCN,AL353593.1 | Missense | G/C | G/C | G/C | G/C | G/C | G/C | G/G | G/G | |
| 1 | 228464168 | 228464168 | C | C | SNV | OBSCN | Missense | C/C | C/C | C/C | C/C | C/C | C/C | C/A | C/A | |
| 1 | 233497836 | 233497836 | C | A | SNV | KIAA1804,RP5-862P8.2 | Missense | C/C | C/C | C/C | C/C | C/C | C/C | C/A | C/A | |
| 2 | 112705138 | 112705138 | G | A | SNV | MERTK | Missense | G/G | G/A | G/A | G/A | G/G | G/A | G/G | G/G | O.K. |
| 2 | 158485099 | 158485099 | C | A | SNV | ACVR1C,AC019186.1 | Missense | C/C | C/C | C/C | C/C | C/C | C/C | C/A | C/A | |
| 2 | 179634421 | 179634421 | T | G | SNV | TTN | Missense | T/G | T/G | T/G | T/G | T/G | T/G | T/G | T/T | |
| 2 | 209195248 | 209195248 | C | A | SNV | PIKFYVE | Missense | C/C | C/C | C/C | C/C | C/C | C/C | C/A | C/C | Questionable |
| 2 | 242047605 | 242047605 | T | G | SNV | PASK | Missense | T/G | T/T | T/G | T/T | T/G | T/G | T/T | T/T | |
| 3 | 41278119 | 41278119 | C | A | SNV | CTNNB1 | Missense | C/A | C/A | C/A | C/A | C/A | C/A | C/C | C/C | |
| 3 | 43389767 | 43389767 | G | T | SNV | SNRK | Missense | G/G | G/T | G/G | G/T | G/G | G/G | G/G | G/G | |
| 3 | 123988019 | 123988019 | A | A | SNV | KALRN | Missense | C/C | C/C | C/C | C/C | C/C | C/C | C/A | C/A | Questionable |
| 3 | 184293716 | 184293716 | A | C | SNV | EPHB3,EIF2B5 | Missense | A/C | A/C | A/A | A/C | A/A | A/A | A/A | A/A | |
| 4 | 66467418 | 66467418 | G | C | SNV | EPHA5 | Missense | A/A | A/A | A/C | A/C | A/A | A/A | A/A | A/A | |
| 4 | 76522293 | 76522293 | G | T | SNV | CDKL2 | Missense | G/T | G/T | G/T | G/T | G/G | G/T | G/G | G/G | |
| 4 | 107168372 | 107168372 | T | G | SNV | TBCK | Missense | T/G | T/T | T/T | T/G | T/G | T/G | T/T | T/T | |
| 5 | 96518822 | 96518822 | T | G | SNV | RIOK2,CTD-2215E18.1,RP11-155G15.2 | Missense | T/T | T/G | T/G | T/T | T/G | T/T | T/T | T/T | Questionable |
| 6 | 43111336 | 43111336 | G | T | SNV | PTK7 | Missense | G/G | G/G | G/G | G/G | G/G | G/G | G/T | G/T | |
| 7 | 40134451 | 40134451 | A | G | SNV | CDK13 | Missense | A/A | A/A | A/A | A/G | A/A | A/A | A/A | A/A | O.K. |
| 7 | 44259762 | 44259762 | T | G | SNV | CAMK2B | Missense | T/G | T/G | T/T | T/G | T/G | T/G | T/T | T/T | |
| 7 | 95216415 | 95216415 | C | A | SNV | PDK4 | Missense | C/A | C/A | C/A | C/C | C/A | C/A | C/C | C/C | |
| 7 | 98547196 | 98547196 | G | T | SNV | TRRAP | Missense | G/G | G/G | G/G | G/G | G/G | G/G | G/T | G/T | |
| 7 | 138145436 | 138145436 | C | A | SNV | TRIM24 | Missense | C/C | C/C | C/C | C/C | C/C | C/C | C/C | C/A | |
| 7 | 138252385 | 138252385 | C | A | SNV | TRIM24 | Missense | C/C | C/A | C/C | C/C | C/A | C/A | C/A | C/C | |
| 8 | 8239069 | 8239069 | C | A | SNV | SGK223,AC068353.1 | Missense | C/C | C/C | C/C | C/C | C/C | C/C | C/A | C/A | |
| 9 | 21971137 | 21971137 | T | G | SNV | CDKN2A | Missense | T/G | T/T | T/G | T/G | T/G | T/G | T/T | T/T | |
| 9 | 77403574 | 77403574 | C | A | SNV | TRPM6 | Missense | C/A | C/A | C/A | C/A | C/A | C/C | C/A | C/A | Questionable |
| 13 | 110434668 | 110434668 | C | A | SNV | IRS2 | Missense | C/C | C/C | C/C | C/C | C/C | C/C | C/C | C/C | |
| 13 | 110437802 | 110437802 | A | C | SNV | IRS2 | Missense | A/C | A/C | A/A | A/C | A/C | A/C | A/A | A/A | |
| 15 | 91436551 | 91436551 | A | G | SNV | FES,AC068831.1 | Missense | A/A | A/G | A/A | A/G | A/G | A/G | A/A | A/A | Questionable |
| 15 | 99250895 | 99250895 | G | T | SNV | IGF1R | Missense | G/G | G/G | G/G | G/G | G/G | G/G | G/T | G/T | Questionable |
| 17 | 7796803 | 7796803 | T | C | SNV | CHD3 | Missense | T/C | T/C | T/C | T/C | T/C | T/C | T/C | T/C | |
| 17 | 7796806 | 7796806 | T | C | SNV | CHD3 | Missense | G/C | G/G | G/G | G/G | G/G | G/G | G/G | G/G | |
| 17 | 7796819 | 7796819 | T | C | SNV | CHD3 | Missense | T/C | T/T | T/T | T/T | T/T | T/T | T/T | T/T | |
| 17 | 19284136 | 19284136 | G | C | SNV | MAPK7 | Missense | G/C | G/G | G/G | G/C | G/C | G/G | G/G | G/G | |
| 17 | 27064863 | 27064863 | G | A | SNV | NEK8 | Missense | G/A | G/G | G/A | G/A | G/G | G/G | G/G | G/G | |

| | | | | | | | | | | | | | | | | Questionable |
|---|---|---|---|---|---|---|---|---|---|---|---|---|---|---|---|---|
| 17 | 41245693 | 41245693 | G | T | SNV | BRCA1 | Missense | G/G | G/G | G/G | G/G | G/G | G/G | G/G | G/T | G/T |
| 18 | 18534948 | 18534948 | G | C | SNV | ROCK1 | Missense | G/G | G/C | G/C | G/C | G/C | G/C | G/C | G/C | G/G |
| 18 | 59919898 | 59919898 | C | A | SNV | KIAA1468 | Missense | C/C | C/C | C/C | C/C | C/C | C/C | C/C | C/A | C/A |
| 19 | 40316422 | 40316422 | T | G | SNV | DYRK1B | Missense | T/G | T/T | T/T | T/T | T/T | T/G | T/G | T/T | T/T |
| 20 | 42204913 | 42204913 | A | C | SNV | SGK2 | Missense | A/A | A/C | A/C | A/C | A/C | A/A | A/A | A/A | A/A |
| X | 19398339 | 19398339 | C | G | SNV | MAP3K15 | Missense | C/C | C/C | C/G | C/C | C/C | C/C | C/C | C/C | C/C |

## Group 4

| Chrom | Chrom Start | Chrom End | Reference | Alternatives | Type | Gene | Function | ncc1 MG_EX_2093_009 | cc1_1 MG_EX_2093_011 | cc1_2 NO.1 MG_EX_2093_032 | cc1_7 MG_EX_2093_013 | cc1_8 MG_EX_2093_014 | CC1_9 NO.2 MG_EX_2093_035(CC1_9 | cc1_11 MG_EX_2093_016 | cc1_12 NO.1 MG_EX_2093_036 | cc1_17 MG_EX_2093_017 | cc1_18 MG_EX_2093_018 | cc1_25 MG_EX_2093_019 | QC QC |
|---|---|---|---|---|---|---|---|---|---|---|---|---|---|---|---|---|---|---|---|
| 1 | 1575715 | 1575715 | C | T | SNV | CDK11B,CDK11A | Missense | C/C | C/C | C/T | C/C | C/T | C/T | C/C | C/T | C/C | C/T | C/C | |
| 1 | 233497836 | 233497836 | C | A | SNV | KIAA1804,RP5-862P8.2 | Missense | C/A | C/C | C/A | C/A | C/C | C/C | C/A | C/C | C/C | C/C | C/A | |
| 2 | 29416635 | 29416635 | C | A | SNV | ALK | Missense | C/A | C/A | C/C | C/A | C/C | C/C | C/C | C/C | C/C | C/C | C/A | |
| 2 | 29448410 | 29448410 | T | G | SNV | ALK | Missense | T/G | T/G | T/G | T/T | T/T | T/G | T/G | T/T | T/T | T/G | T/G | |
| 2 | 37336419 | 37336419 | C | T | SNV | EIF2AK2 | Missense | C/C | C/C | C/C | C/C | C/C | C/C | C/C | C/C | C/C | C/T | C/C | O.K. |
| 2 | 158485099 | 158485099 | C | A | SNV | ACVR1C,AC019186.1 | Missense | C/C | C/C | C/C | C/A | C/C | C/A | C/C | C/A | C/C | C/C | C/A | Questionable |
| 2 | 179408086 | 179408086 | A | G | SNV | TTN,MIR548N,LOC100506866,AC009948.3 | Missense | A/A | A/A | A/A | A/A | A/A | A/G | A/A | A/A | A/A | A/A | A/A | O.K. |
| 2 | 179634421 | 179634421 | T | G | SNV | TTN | Missense | T/T | T/G | T/G | T/G | T/G | T/G | T/G | T/G | T/T | T/G | T/G | |
| 2 | 209195248 | 209195248 | C | A | SNV | PIKFYVE | Missense | C/A | C/A | C/C | C/A | C/C | C/A | C/C | C/A | C/A | C/C | C/A | |
| 3 | 41705179 | 41705179 | A | C | SNV | ULK4 | Missense | A/A | A/A | A/A | A/A | A/A | A/A | A/A | A/A | A/A | A/A | A/C | O.K. |
| 3 | 123988019 | 123988019 | C | A | SNV | KALRN | Missense | C/A | C/C | C/C | C/A | C/C | C/C | C/C | C/C | C/C | C/C | C/A | |
| 5 | 112769527 | 112769527 | C | T | SNV | MCC,TSSK1B,CTD-2201G3.1 | Missense | C/C | C/C | C/C | C/C | C/T | C/C | C/C | C/C | C/C | C/C | C/C | O.K. |
| 5 | 180048626 | 180048626 | C | T | SNV | FLT4 | Missense | C/C | C/T | C/C | C/C | C/C | C/C | C/C | C/C | C/C | C/C | C/C | |
| 6 | 31947203 | 31947203 | T | C | SNV | STK19,XXbac-BPG116M5.15 | Missense | T/T | T/T | T/C | T/T | T/T | T/T | T/T | T/T | T/T | T/T | T/T | O.K. |
| 6 | 36489585 | 36489585 | C | A | SNV | STK38 | Missense | C/A | C/C | C/A | C/A | C/A | C/A | C/A | C/A | C/A | C/A | C/A | |
| 6 | 110942394 | 110942394 | G | T | SNV | CDK19 | Missense | G/T | G/T | G/G | G/T | G/T | G/G | G/T | G/T | G/T | G/T | G/G | |
| 7 | 23808650 | 23808650 | G | T | SNV | STK31 | Missense | G/G | G/G | G/G | G/G | G/G | G/G | G/T | G/G | G/G | G/G | G/G | O.K. |
| 7 | 95216415 | 95216415 | C | A | SNV | PDK4 | Missense | C/C | C/A | C/A | C/A | C/C | C/A | C/C | C/C | C/C | C/C | C/C | |
| 7 | 98490141 | 98490141 | G | C | SNV | TRRAP | Missense | G/G | G/G | G/G | G/G | G/G | G/G | G/G | G/C | G/G | G/G | G/G | O.K. |
| 7 | 98547196 | 98547196 | G | T | SNV | TRRAP | Missense | G/T | G/T | G/T | G/T | G/T | G/T | G/T | G/T | G/T | G/T | G/T | |
| 7 | 138145436 | 138145436 | C | A | SNV | TRIM24 | Missense | C/C | C/A | C/C | C/A | C/C | C/C | C/A | C/C | C/C | C/C | C/A | |
| 8 | 8239069 | 8239069 | C | A | SNV | SGK223,AC068353.1 | Missense | C/A | C/A | C/A | C/A | C/A | C/C | C/A | C/A | C/A | C/A | C/C | |
| 8 | 144800905 | 144800905 | A | C | SNV | MAPK15,RP11-429J17.5 | Missense | A/A | A/A | A/C | A/A | A/A | A/A | A/A | A/A | A/A | A/A | A/C | |
| 10 | 99400747 | 99400747 | C | A | SNV | PI4K2A,RP11-548K23.11 | Missense | C/C | C/A | C/A | C/A | C/C | C/C | C/C | C/C | C/A | C/A | C/A | |
| 11 | 46369267 | 46369267 | G | A | SNV | DGKZ | Missense | G/A | G/G | G/G | G/G | G/G | G/G | G/G | G/A | G/G | G/A | G/G | |
| 12 | 1009680 | 1009680 | C | T | SNV | WNK1 | Missense | C/C | C/C | C/C | C/C | C/T | C/C | C/C | C/C | C/C | C/C | C/C | O.K. |
| 13 | 110434668 | 110434668 | C | A | SNV | IRS2 | Missense | C/A | C/A | C/A | C/A | C/A | C/C | C/A | C/A | C/A | C/A | C/A | |
| 13 | 110437802 | 110437802 | A | C | SNV | IRS2 | Missense | A/A | A/A | A/A | A/A | A/A | A/A | A/A | A/A | A/A | A/C | A/A | |

EP 2 749 642 A1

| Chr | Position | Position | Ref | Alt | Type | Gene | Consequence | Genotypes (samples) | Status |
|---|---|---|---|---|---|---|---|---|---|
| 14 | 24808802 | 24808802 | G | T | SNV | RIPK3 | Missense | G/G G/G G/G G/G G/G G/T G/G G/G G/G G/G G/G G/G G/G G/T G/G | |
| 15 | 91436551 | 91436551 | A | G | SNV | FES,AC068831.1 | Missense | A/A A/G A/G A/A A/G A/A A/G A/A A/A A/G A/G A/A A/G A/G A/A | |
| 15 | 99250895 | 99250895 | G | T | SNV | IGF1R | Missense | G/T G/T G/T G/T G/G G/T G/G G/T G/G G/T G/G G/T G/G G/T G/G | O.K. |
| 16 | 23690401 | 23690401 | C | T | SNV | PLK1 | Missense | C/C C/C C/C C/C C/C C/C C/C C/C [C/T] C/C C/C C/C C/C C/C C/C | |
| 16 | 46744689 | 46744689 | C | A | SNV | MYLK3 | Missense | C/A C/A C/C C/A C/C C/C C/C C/C C/C C/A C/C C/C C/C C/A C/A | |
| 17 | 7796803 | 7796803 | T | C | SNV | CHD3 | Missense | T/T T/T T/T T/T T/C T/C T/C T/T T/C T/T T/C T/T T/C T/T T/T | Questionable |
| 17 | 8789811 | 8789811 | G | A | SNV | PIK3R5 | Nonsense | [G/A] G/G G/G G/G G/G G/G G/G G/G G/G G/G G/G G/G G/G G/G G/A | O.K. |
| 17 | 37881392 | 37881392 | A | G | SNV | ERBB2,MIR4728 | Missense | A/A A/A A/A A/A A/A A/A A/A A/A A/A A/A A/A A/A A/A A/A A/A | O.K. |
| 17 | 41245693 | 41245693 | G | T | SNV | BRCA1 | Missense | G/G G/G G/G G/G G/G G/T G/G G/T G/G G/T G/T G/T G/G G/G G/G | |
| 18 | 18534948 | 18534948 | G | C | SNV | ROCK1 | Missense | G/C G/C G/C G/G G/C G/G G/G G/G G/C G/G G/G G/C G/C G/C G/C | |
| 19 | 2046399 | 2046399 | G | A | SNV | MKNK2 | Missense | G/G G/G G/G G/G G/G G/G G/G G/G G/G G/G G/G G/G G/G G/G G/G | |
| 19 | 47193933 | 47193933 | G | T | SNV | PRKD2 | Missense | G/G G/G G/G G/G G/G G/G G/G G/T G/G G/T G/G G/T G/G G/G G/G | O.K. |

## Group 5

| Chrom | Chrom Start | Chrom End | Reference | Alternatives | Type | Gene | Function | ncc4 MG_EX_2093_0 20 | cc4_c MG_EX_2093_0 22 | cc4_3 NO.1 MG_EX_2093_0 42 | cc4_6_mix MG_EX_2093_0 30 | cc4_9_5 NO.1 MG_EX_2093_0 49 | cc4_9_7 MG_EX_2093_0 51 | cc4_9_1 1 NO.2 MG_EX_2093_0 53 | cc4_9_1 3 NO.1 MG_EX_2093_0 54 | cc4_3_1 0 MG_EX_2093_0 27 | cc4_4 MG_EX_2093_0 29 | cc4_6 MG_EX_2093_0 38 | cc4_30 MG_EX_2093_0 40 | QC QC |
|---|---|---|---|---|---|---|---|---|---|---|---|---|---|---|---|---|---|---|---|---|
| 1 | 11303178 | 11303178 | C | T | SNV | MTOR | Missense | C/C | C/T | C/T | C/T | C/C | C/T | C/C | C/T | C/C | C/T | C/C | C/C | O.K. |
| 1 | 16455972 | 16455972 | C | T | SNV | EPHA2 | Missense | C/C | C/C | C/C | C/T | C/C | C/C | C/C | C/C | C/C | C/C | C/C | C/C | O.K. |
| 1 | 32828420 | 32828420 | G | A | SNV | TSSK3,LOC100128071,RP4-811H24.6 | Missense | G/G | G/G | G/G | G/G | G/G | G/A | G/G | G/G | G/G | G/G | G/G | G/G | |
| 1 | 38184063 | 38184063 | C | A | SNV | EPHA10 | Missense | C/A | C/A | C/A | C/A | C/A | C/C | C/A | C/A | C/A | C/A | C/A | C/A | |
| 1 | 43784969 | 43784969 | G | A | SNV | TIE1 | Missense | G/G | G/A | G/A | G/A | G/A | G/A | G/A | G/A | G/A | G/A | G/A | G/A | |
| 1 | 45101277 | 45101277 | A | G | SNV | RNF220,TMEM53 | Missense | A/A | A/A | A/A | A/A | A/A | A/G | A/A | A/A | A/A | A/A | A/G | A/A | |
| 1 | 45102063 | 45102063 | G | C | SNV | RNF220,TMEM53 | Missense | G/G | G/G | G/G | G/G | G/G | G/C | G/G | G/G | G/G | G/G | G/C | G/G | |
| 1 | 46497963 | 46497963 | A | T | SNV | MAST2 | Missense | A/A | A/A | A/A | A/A | A/A | A/T | A/A | A/A | A/A | A/A | A/A | A/A | |
| 1 | 89206855 | 89206855 | G | A | SNV | PKN2 | Missense | G/G | G/G | G/G | G/G | G/G | G/A | G/G | G/G | G/G | G/G | G/A | G/G | |
| 1 | 114940422 | 114940422 | T | A | SNV | TRIM33 | Missense | T/T | T/T | T/T | T/T | T/T | T/A | T/T | T/T | T/T | T/T | T/A | T/T | |
| 1 | 114940464 | 114940464 | G | T | SNV | TRIM33 | Missense | G/G | G/G | G/G | G/G | G/G | G/T | G/G | G/G | G/G | G/G | G/T | G/G | |
| 1 | 114940481 | 114940481 | G | C | SNV | TRIM33 | Missense | G/G | G/G | G/G | G/G | G/G | G/C | G/G | G/G | G/G | G/G | G/C | G/G | |
| 1 | 151209184 | 151209184 | A | G | SNV | PIP5K1A | Missense | A/A | A/G | A/A | A/G | A/A | A/G | A/A | A/G | A/A | A/A | A/A | A/A | |
| 1 | 156810871 | 156810871 | G | T | SNV | INSRR,NTRK1 | Missense | G/G | G/G | G/G | G/G | G/G | G/T | G/G | G/T | G/G | G/G | G/G | G/G | |
| 1 | 156823631 | 156823631 | G | T | SNV | INSRR,NTRK1 | Missense | G/G | G/G | G/G | G/G | G/G | G/G | G/G | G/G | G/T | G/G | G/G | G/G | |
| 1 | 156823679 | 156823679 | C | T | SNV | INSRR,NTRK1 | Missense | C/C | C/C | C/C | C/C | C/C | C/C | C/C | C/C | C/C | C/C | C/C | C/C | |
| 1 | 169831834 | 169831834 | G | A | SNV | SCYL3 | Missense | G/G | G/A | G/G | G/G | G/G | G/G | G/G | G/G | G/G | G/G | G/G | G/G | |
| 1 | 179077409 | 179077409 | A | G | SNV | ABL2 | Missense | A/A | A/A | A/A | A/A | A/A | A/G | A/A | A/A | A/A | A/A | A/A | A/A | O.K. |
| 1 | 179077641 | 179077641 | G | C | SNV | ABL2 | Missense | G/G | G/G | G/G | G/G | G/G | G/C | G/G | G/G | G/G | G/G | G/G | G/G | |
| 1 | 179077643 | 179077643 | G | A | SNV | ABL2 | Missense | G/G | G/G | G/G | G/G | G/G | G/A | G/G | G/G | G/G | G/G | G/G | G/G | |
| 1 | 179077662 | 179077662 | G | A | SNV | ABL2 | Missense | G/G | G/G | G/G | G/G | G/G | G/A | G/G | G/G | G/G | G/G | G/G | G/G | |
| 1 | 179077670 | 179077670 | G | A | SNV | ABL2 | Missense | G/G | G/G | G/G | G/G | G/G | G/A | G/G | G/G | G/G | G/G | G/G | G/G | |
| 1 | 179077884 | 179077884 | T | C | SNV | ABL2 | Missense | T/T | T/T | T/T | T/T | T/T | T/C | T/T | T/T | T/T | T/T | T/T | T/T | |
| 1 | 213349771 | 213349771 | T | C | SNV | RPS6KC1 | Missense | T/T | T/T | T/T | T/T | T/T | T/C | T/T | T/T | T/T | T/T | T/T | T/T | |
| 1 | 213349777 | 213349777 | A | G | SNV | RPS6KC1 | Missense | A/A | A/A | A/A | A/A | A/A | A/G | A/A | A/A | A/A | A/A | A/A | A/A | |
| 1 | 213415637 | 213415637 | A | G | SNV | RPS6KC1 | Missense | A/A | A/A | A/A | A/A | A/A | A/G | A/A | A/A | A/A | A/A | A/A | A/A | |
| 1 | 213415653 | 213415653 | T | C | SNV | RPS6KC1 | Missense | T/T | T/T | T/T | T/T | T/T | T/C | T/T | T/T | T/T | T/T | T/T | T/T | |
| 1 | 213415977 | 213415977 | A | G | SNV | RPS6KC1 | Missense | A/A | A/A | A/A | A/A | A/A | A/G | A/A | A/A | A/A | A/A | A/G | A/A | |
| 1 | 213415981 | 213415981 | A | C | SNV | RPS6KC1 | Missense | A/A | A/A | A/A | A/A | A/A | A/C | A/A | A/A | A/A | A/A | A/C | A/A | |
| 1 | 227300392 | 227300392 | T | C | SNV | CDC42BPA | Missense | T/T | T/T | T/T | T/T | T/T | T/C | T/T | T/T | T/T | T/T | T/T | T/T | |
| 1 | 227300421 | 227300421 | A | G | SNV | CDC42BPA | Missense | A/A | A/A | A/A | A/A | A/A | A/G | A/A | A/A | A/A | A/A | A/G | A/A | |
| 1 | 228456288 | 228456288 | C | T | SNV | OBSCN | Missense | C/C | C/C | C/C | C/C | C/C | C/C | C/C | C/C | C/C | C/C | C/C | C/T | |
| 1 | 228522915 | 228522915 | C | T | SNV | OBSCN | Missense | C/C | C/C | C/C | C/C | C/C | C/C | C/C | C/C | C/T | C/C | C/C | C/C | |
| 1 | 228547855 | 228547855 | A | G | SNV | OBSCN | Missense | A/A | A/G | A/A | A/A | A/A | A/A | A/A | A/A | A/A | A/A | A/A | A/A | |

EP 2 749 642 A1

| Chr | Position | Position | Ref | Alt | Type | Gene | Effect | S1 | S2 | S3 | S4 | S5 | S6 | S7 | S8 | S9 | S10 | S11 | S12 | S13 |
|---|---|---|---|---|---|---|---|---|---|---|---|---|---|---|---|---|---|---|---|---|
| 1 | 233464204 | 233464204 | C | T | SNV | KIAA1804,RP5-862P8.2 | Nonsense | C/C | C/C | C/C | C/C | C/C | C/C | C/C | C/T | C/C | C/C | C/C | C/C | C/C |
| 1 | 233497836 | 233497836 | C | A | SNV | KIAA1804,RP5-862P8.2 | Missense | C/A | C/C | C/C | C/A | C/C | C/A | C/C | C/C | C/A | C/A | C/A | C/C | C/A |
| 2 | 29448410 | 29448410 | T | G | SNV | ALK | Missense | T/G | T/T | T/G | T/G | T/G | T/G | T/G | T/G | T/T | T/T | T/G | T/G | T/G |
| 2 | 29451864 | 29451864 | T | C | SNV | ALK | Missense | T/T | T/T | T/T | T/T | T/T | T/C | T/T | T/T | T/T | T/T | T/T | T/T | T/T |
| 2 | 29451875 | 29451875 | T | A | SNV | ALK | Missense | T/T | T/T | T/T | T/T | T/T | T/A | T/T | T/T | T/T | T/T | T/T | T/T | T/T |
| 2 | 102480422 | 102480422 | T | G | SNV | MAP4K4 | Missense | T/T | T/G | T/T | T/T | T/T | T/G | T/T | T/T | T/T | T/T | T/T | T/T | T/T |
| 2 | 102480455 | 102480455 | C | T | SNV | MAP4K4 | Missense | C/C | C/C | C/C | C/C | C/C | C/T | C/C | C/C | C/C | C/C | C/C | C/C | C/C |
| 2 | 102480462 | 102480462 | C | G | SNV | MAP4K4 | Missense | C/C | C/C | C/C | C/C | C/C | C/G | C/C | C/C | C/C | C/C | C/C | C/C | C/C |
| 2 | 148657037 | 148657037 | G | A | SNV | ACVR2A,AC009480.3 | Missense | G/G | G/G | G/G | G/G | G/G | G/A | G/G | G/G | G/G | G/G | G/A | G/G | G/G |
| 2 | 148683687 | 148683687 | A | T | SNV | ACVR2A | Missense | A/A | A/A | A/A | A/T | A/A | A/A | A/A | A/A | A/A | A/A | A/T | A/A | A/A |
| 2 | 158485099 | 158485099 | C | A | SNV | ACVR1C,AC019186.1 | Missense | C/C | C/C | C/C | C/C | C/A | C/C | C/A | C/C | C/C | C/C | C/C | C/C | C/C |
| 2 | 171508642 | 171508642 | A | C | SNV | MYO3B,AC007277.3 | Missense | A/A | A/A | A/A | A/A | A/A | A/C | A/A | A/A | A/A | A/A | A/A | A/A | A/A |
| 2 | 172016886 | 172016886 | T | C | SNV | TLK1 | Missense | T/T | T/C | T/T | T/T | T/T | T/C | T/T | T/T | T/T | T/T | T/T | T/T | T/T |
| 2 | 174085893 | 174085893 | G | T | SNV | ZAK,MLK7-AS1,AC013461.1,AC013461.2 | Missense | G/G | G/G | G/G | G/G | G/G | G/T | G/G | G/G | G/G | G/G | G/G | G/G | G/G |
| 2 | 174085977 | 174085977 | A | G | SNV | ZAK,MLK7-AS1,AC013461.1,AC013461.2 | Missense | A/A | A/A | A/A | A/A | A/A | A/G | A/A | A/A | A/A | A/A | A/A | A/A | A/A |
| 2 | 174086010 | 174086010 | A | G | SNV | ZAK,MLK7-AS1,AC013461.1,AC013461.2 | Missense | A/A | A/A | A/A | A/A | A/A | A/G | A/A | A/A | A/A | A/A | A/A | A/A | A/A |
| 2 | 174086046 | 174086046 | A | G | SNV | ZAK,MLK7-AS1,AC013461.1,AC013461.2 | Missense | A/A | A/A | A/A | A/A | A/A | A/G | A/A | A/A | A/A | A/A | A/A | A/A | A/A |
| 2 | 174086076 | 174086076 | A | C | SNV | ZAK,MLK7-AS1,AC013461.1,AC013461.2 | Missense | A/A | A/A | A/A | A/A | A/A | A/C | A/A | A/A | A/A | A/A | A/A | A/A | A/A |
| 2 | 179407002 | 179407002 | C | T | SNV | TTN,MIR548N,LOC100506866,AC009948.3 | Missense | C/T | C/C | C/C | C/C | C/C | C/C | C/C | C/C | C/C | C/C | C/C | C/C | C/T |
| 2 | 179430475 | 179430475 | G | A | SNV | TTN,MIR548N,LOC100506866,AC009948.3 | Missense | G/G | G/G | G/G | G/A | G/G | G/G | G/G | G/G | G/G | G/G | G/A | G/G | G/G |
| 2 | 179431263 | 179431263 | T | G | SNV | TTN,MIR548N,LOC100506866,AC009948.3 | Missense | T/G | T/G | T/G | T/T | T/T | T/T | T/G | T/T | T/G | T/T | T/T | T/G | T/G |
| 2 | 179435267 | 179435267 | T | C | SNV | TTN,MIR548N,LOC100506866,AC009948.3 | Missense | T/T | T/T | T/T | T/T | T/T | T/T | T/T | T/T | T/T | T/T | T/T | T/T | T/T |

| | | | | | | | | | | | | | | | | | | |
|---|---|---|---|---|---|---|---|---|---|---|---|---|---|---|---|---|---|---|
| 2 | 179435405 | 179435405 | G | T | SNV | TTN,MIR548N,LOC100506866,AC009948.3 | Missense | G/G | G/G | G/G | G/G | G/G | G/G | G/G | G/G | G/G | G/T | G/G | G/G | |
| 2 | 179438353 | 179438353 | G | A | SNV | TTN,MIR548N,LOC100506866,AC009948.3 | Missense | G/G | G/G | G/A | G/G | G/A | G/G | G/A | G/G | G/G | G/A | G/A | G/A | |
| 2 | 179485507 | 179485507 | T | A | SNV | TTN,MIR548N | Missense | T/T | T/T | T/T | T/T | T/T | T/A | T/T | T/T | T/T | T/T | T/T | T/T | |
| 2 | 179485527 | 179485527 | G | C | SNV | TTN,MIR548N | Missense | G/G | G/G | G/G | G/G | G/G | G/C | G/G | G/G | G/G | G/G | G/C | G/G | |
| 2 | 179485528 | 179485528 | T | C | SNV | TTN,MIR548N | Missense | T/T | T/T | T/T | T/T | T/T | T/C | T/T | T/T | T/T | T/T | T/C | T/T | |
| 2 | 179590293 | 179590293 | C | T | SNV | TTN | Missense | C/C | C/T | C/T | C/T | C/T | C/T | C/T | C/T | C/T | C/T | C/T | C/T | |
| 2 | 179615168 | 179615168 | T | G | SNV | TTN | Missense | T/T | T/G | T/G | T/G | T/G | T/G | T/G | T/G | T/G | T/G | T/G | T/G | |
| 2 | 179634421 | 179634421 | T | G | SNV | TTN | Missense | T/T | T/G | T/T | T/T | T/G | T/G | T/G | T/G | T/G | T/G | T/T | T/G | |
| 2 | 179666963 | 179666963 | G | A | SNV | TTN | Missense | G/G | G/G | G/G | G/G | G/G | G/A | G/G | G/G | G/G | G/G | G/G | G/G | |
| 2 | 179666969 | 179666969 | T | C | SNV | TTN | Missense | T/T | T/T | T/T | T/T | T/T | T/C | T/T | T/T | T/T | T/T | T/T | T/T | |
| 2 | 201724917 | 201724917 | T | A | SNV | CLK1,PPIL3 | Missense | T/T | T/T | T/T | T/T | T/T | T/A | T/T | T/T | T/T | T/T | T/A | T/T | |
| 2 | 203420712 | 203420712 | G | A | SNV | BMPR2 | Missense | G/G | G/G | G/G | G/G | G/G | G/A | G/G | G/G | G/G | G/G | G/A | G/G | |
| 2 | 209195248 | 209195248 | C | A | SNV | PIKFYVE | Missense | C/A | C/A | C/A | C/A | C/A | C/A | C/A | C/A | C/A | C/A | C/C | C/A | |
| 2 | 220309688 | 220309688 | G | C | SNV | SPEG,DNPEP | Missense | G/G | G/G | G/G | G/G | G/G | G/C | G/G | G/G | G/G | G/G | G/C | G/G | |
| 2 | 220309717 | 220309717 | G | A | SNV | SPEG,DNPEP | Missense | G/G | G/G | G/G | G/G | G/G | G/A | G/G | G/G | G/G | G/G | G/A | G/G | |
| 2 | 220348345 | 220348345 | G | A | SNV | SPEG,DNPEP,AC053503.11 | Missense | G/G | G/A | G/G | G/G | G/G | G/A | G/G | G/G | G/G | G/G | G/A | G/G | |
| 2 | 242437702 | 242437702 | G | A | SNV | STK25 | Missense | G/G | G/G | G/G | G/G | G/G | G/G | G/G | G/G | G/G | G/A | G/G | G/G | |
| 3 | 10276299 | 10276299 | G | T | SNV | IRAK2 | Missense | G/G | G/G | G/T | G/G | G/G | G/T | G/G | G/G | G/G | G/G | G/T | G/T | |
| 3 | 12626014 | 12626014 | T | C | SNV | RAF1 | Readthrough | T/T | T/C | T/C | T/C | T/C | T/C | T/C | T/C | T/C | T/C | T/C | T/C | |
| 3 | 38524696 | 38524696 | C | T | SNV | ACVR2B | Missense | C/C | C/T | C/T | C/T | C/T | C/T | C/T | C/T | C/T | C/T | C/T | C/T | |
| 3 | 48725800 | 48725800 | G | C | SNV | IP6K2 | Missense | G/G | G/G | G/G | G/G | G/G | G/C | G/G | G/G | G/G | G/G | G/G | G/G | |
| 3 | 58385082 | 58385082 | A | G | SNV | PXK | Missense | A/A | A/A | A/A | A/A | A/A | A/G | A/A | A/A | A/A | A/A | A/G | A/A | |
| 3 | 96962823 | 96962823 | T | C | SNV | EPHA6 | Missense | T/T | T/T | T/T | T/T | T/T | T/C | T/T | T/T | T/T | T/T | T/C | T/T | |
| 3 | 96962937 | 96962937 | G | C | SNV | EPHA6 | Missense | G/G | G/G | G/G | G/G | G/G | G/C | G/G | G/G | G/G | G/G | G/C | G/G | |
| 3 | 119582272 | 119582272 | T | C | SNV | GSK3B | Missense | T/T | T/C | T/C | T/C | T/C | T/C | T/C | T/C | T/C | T/C | T/C | T/C | |
| 3 | 123988019 | 123988019 | C | A | SNV | KALRN | Missense | C/C | C/A | C/C | C/C | C/A | C/A | C/A | C/A | C/C | C/A | C/C | C/C | |
| 3 | 138433461 | 138433461 | G | T | SNV | PIK3CB | Missense | G/G | G/G | G/G | G/G | G/G | G/T | G/G | G/G | G/G | G/G | G/T | G/G | |
| 3 | 138433495 | 138433495 | C | T | SNV | PIK3CB | Missense | C/C | C/C | C/C | C/C | C/C | C/T | C/C | C/C | C/C | C/C | C/C | C/C | |
| 3 | 138433510 | 138433510 | T | C | SNV | PIK3CB | Missense | T/T | T/T | T/T | T/T | T/T | T/C | T/T | T/T | T/T | T/T | T/T | T/T | |
| 3 | 142178115 | 142178115 | T | C | SNV | ATR | Missense | T/T | T/T | T/T | T/T | T/T | T/C | T/T | T/T | T/T | T/T | T/C | T/T | |
| 3 | 142178137 | 142178137 | A | T | SNV | ATR | Missense | A/A | A/A | A/A | A/A | A/A | A/T | A/A | A/A | A/A | A/A | A/T | A/A | |
| 3 | 142178144 | 142178144 | C | T | SNV | ATR | Missense | C/C | C/C | C/C | C/C | C/C | C/T | C/C | C/C | C/C | C/C | C/T | C/C | |
| 3 | 170800127 | 170800127 | G | A | SNV | TNIK | Nonsense | G/G | G/G | G/G | G/A | G/G | G/G | G/G | G/G | G/G | G/G | G/G | G/G | |
| 3 | 178921553 | 178921553 | T | A | SNV | PIK3CA | Missense | T/T | T/A | T/A | T/A | T/A | T/A | T/A | T/A | T/A | T/A | T/A | T/A | O.K. |
| 3 | 184294942 | 184294942 | C | T | SNV | EPHB3,EIF2B5 | Missense | C/C | C/C | C/C | C/C | C/C | C/C | C/C | C/C | C/C | C/C | C/T | C/C | |
| 4 | 66242772 | 66242772 | T | A | SNV | EPHA5 | Missense | T/T | T/T | T/T | T/T | T/T | T/A | T/T | T/T | T/T | T/T | T/A | T/T | |
| 4 | 66467674 | 66467674 | C | A | SNV | EPHA5 | Missense | C/C | C/C | C/C | C/C | C/C | C/C | C/C | C/C | C/C | C/A | C/C | C/C | |
| 4 | 107168372 | 107168372 | T | G | SNV | TBCK | Missense | T/T | T/G | T/T | T/T | T/T | T/T | T/T | T/T | T/G | T/T | T/T | T/T | |
| 4 | 113303557 | 113303557 | T | C | SNV | ALPK1 | Missense | T/T | T/T | T/T | T/T | T/T | T/C | T/T | T/T | T/T | T/T | T/T | T/T | |
| 4 | 113303595 | 113303595 | A | G | SNV | ALPK1 | Missense | A/A | A/A | A/A | A/A | A/A | A/G | A/A | A/A | A/A | A/A | A/A | A/A | |
| 4 | 144378857 | 144378857 | T | C | SNV | GAB1 | Missense | T/T | T/T | T/T | T/T | T/T | T/C | T/T | T/T | T/T | T/T | T/T | T/T | |

EP 2 749 642 A1

109

| chr | start | end | ref | alt | type | gene | effect | | | | | | | | | |
|---|---|---|---|---|---|---|---|---|---|---|---|---|---|---|---|---|
| 5 | 14336693 | 14336693 | G | A | SNV | TRIO | Missense | G/A | G/A | G/A | G/A | G/A | G/A | G/A | G/A | G/G |
| 5 | 56178629 | 56178629 | C | T | SNV | MAP3K1 | Missense | C/T | C/T | C/T | C/T | C/T | C/T | C/T | C/T | C/C |
| 5 | 66459148 | 66459148 | C | T | SNV | MAST4 | Missense | C/T | C/T | C/T | C/T | C/T | C/T | C/T | C/T | C/C |
| 5 | 112155015 | 112155015 | C | A | SNV | APC | Missense | C/C | C/A | C/C | C/C | C/C | C/C | C/C | C/C | C/C |
| 5 | 112162876 | 112162876 | A | G | SNV | APC | Missense | A/A | A/A | A/A | A/A | A/A | A/A | A/A | A/A | A/A |
| 5 | 148897392 | 148897392 | T | G | SNV | CSNK1A1 | Missense | T/T | T/G | T/T | T/T | T/T | T/G | T/T | T/T | T/T |
| 6 | 2679676 | 2679676 | G | A | SNV | MYLK4 | Missense | G/G | G/A | G/G | G/A | G/G | G/G | G/G | G/G | G/A |
| 6 | 4031998 | 4031998 | A | G | SNV | PRPF4B | Missense | A/A | A/G | A/A | A/A | A/A | A/A | A/A | A/A | A/A |
| 6 | 4049307 | 4049307 | A | G | SNV | PRPF4B | Missense | G/G | A/G | G/G | G/G | G/G | A/G | G/G | G/G | A/G |
| 6 | 7402881 | 7402881 | A | G | SNV | RIOK1 | Missense | A/A | A/G | A/A | A/G | A/A | A/A | A/A | A/A | A/A |
| 6 | 30863200 | 30863200 | T | G | SNV | DDR1 | Missense | T/T | T/T | T/T | T/T | T/T | T/G | T/T | T/T | T/T |
| 6 | 35838096 | 35838096 | T | G | SNV | SRPK1 | Missense | T/T | T/T | T/T | T/T | T/T | T/G | T/T | T/T | T/T |
| 6 | 35838107 | 35838107 | C | A | SNV | SRPK1 | Missense | C/A | C/A | C/A | C/A | C/A | C/A | C/A | C/A | C/A |
| 6 | 36489585 | 36489585 | G | C | SNV | STK38 | Missense | G/G | G/G | G/G | G/G | G/G | G/A | G/G | G/G | G/C |
| 6 | 43230970 | 43230970 | G | C | SNV | TTBK1 | Missense | G/G | G/G | G/G | G/A | G/G | G/G | G/C | G/G | G/G |
| 6 | 91226381 | 91226381 | T | A | SNV | MAP3K7 | Missense | T/T | T/T | T/T | T/T | T/T | T/C | T/T | T/T | T/T |
| 6 | 94120411 | 94120411 | T | C | SNV | EPHA7 | Missense | T/T | T/C | T/T | T/T | T/T | T/C | T/T | T/T | T/T |
| 6 | 94120426 | 94120426 | T | C | SNV | EPHA7 | Missense | G/T | T/C | T/T | T/T | T/T | T/C | T/T | T/T | T/T |
| 6 | 110942394 | 110942394 | A | G | SNV | CDK19 | Missense | A/A | A/A | A/G | A/A | A/A | A/A | A/G | A/A | A/A |
| 6 | 112020765 | 112020765 | C | T | SNV | FYN | Missense | C/C | C/T | C/C | C/C | C/C | C/T | C/C | C/C | C/T |
| 6 | 112020774 | 112020774 | C | T | SNV | FYN | Missense | G/G | C/C | G/G | G/G | G/G | G/G | G/G | G/G | G/G |
| 6 | 112020775 | 112020775 | G | C | SNV | FYN | Missense | C/C | G/A | C/C | C/C | C/C | C/C | C/C | C/C | C/C |
| 6 | 112020835 | 112020835 | C | A | SNV | FYN | Missense | T/T | C/A | T/T | T/T | T/T | T/T | T/T | T/T | T/T |
| 6 | 112020838 | 112020838 | T | C | SNV | FYN | Missense | A/A | T/C | A/A | A/A | A/A | A/A | A/A | A/A | A/A |
| 6 | 116265534 | 116265534 | A | G | SNV | FRK | Missense | C/C | A/A | C/C | C/C | C/C | C/C | C/C | C/C | C/C |
| 6 | 116325142 | 116325142 | C | T | SNV | FRK | Missense | G/G | C/T | G/G | G/G | G/G | G/G | G/G | G/G | G/G |
| 6 | 150001059 | 150001059 | G | A | SNV | LATS1 | Missense | C/C | G/G | C/C | C/C | C/C | C/C | C/C | C/C | C/C |
| 6 | 150001196 | 150001196 | G | T | SNV | LATS1 | Missense | G/G | G/C | G/G | G/G | G/G | G/G | G/G | G/G | G/G |
| 7 | 39990535 | 39990535 | G | C | SNV | CDK13 | Missense | G/G | G/A | G/G | G/G | G/G | G/G | G/G | G/G | G/G |
| 7 | 39990770 | 39990770 | C | A | SNV | CDK13 | Missense | C/A | C/T | C/C | C/C | C/C | C/A | C/C | C/C | C/C |
| 7 | 40038986 | 40038986 | G | T | SNV | CDK13 | Missense | A/A | A/T | A/A | A/A | A/A | A/A | A/A | A/A | A/A |
| 7 | 40132387 | 40132387 | A | T | SNV | CDK13 | Missense | A/A | A/C | A/A | A/A | A/A | A/A | A/A | A/A | A/A |
| 7 | 40132405 | 40132405 | C | C | SNV | CDK13 | Missense | C/C | C/G | C/C | C/C | C/C | C/C | C/C | C/C | C/C |
| 7 | 40132406 | 40132406 | A | A | SNV | CDK13 | Missense | A/A | A/T | A/A | A/A | A/A | A/A | A/A | A/A | A/A |
| 7 | 40132455 | 40132455 | C | C | SNV | CDK13 | Missense | C/C | C/C | C/C | C/C | C/C | C/C | C/C | C/C | C/C |
| 7 | 40134241 | 40134241 | G | G | SNV | CDK13 | Missense | G/G | G/G | G/G | G/G | G/G | G/G | G/G | G/G | G/G |
| 7 | 40134343 | 40134343 | G | G | SNV | CDK13 | Missense | G/G | G/G | G/G | G/G | G/G | G/G | G/G | G/G | G/G |
| 7 | 40134352 | 40134352 | G | G | SNV | CDK13 | Missense | G/G | G/G | G/G | G/G | G/G | G/G | G/G | G/G | G/G |
| 7 | 40134362 | 40134362 | A | A | SNV | CDK13 | Missense | A/A | A/A | A/A | A/A | A/A | A/A | A/A | A/A | A/A |
| 7 | 40134451 | 40134451 | A | G | SNV | CDK13 | Missense | A/A | A/G | A/A | A/A | A/A | A/A | A/A | A/A | A/A |
| 7 | 40134544 | 40134544 | G | A | SNV | CDK13 | Missense | G/G | G/G | G/G | G/G | G/G | G/G | G/G | G/G | G/G |
| 7 | 56151076 | 56151076 | G | T | SNV | PHKG1 | Missense | G/G | G/T | G/G | G/G | G/G | G/G | G/G | G/G | G/G |
| 7 | 97823523 | 97823696 | A | G | SNV | LMTK2 | Missense | A/A | A/G | A/A | A/A | A/A | A/A | A/A | A/A | A/A |
| 7 | 98545950 | 137270035 | C | T | SNV | TRRAP | Missense | C/T | C/T | C/T | C/T | C/T | C/T | C/T | C/T | C/C |
| 7 | 137270035 | 137270035 | C | C | SNV | DGKI | Missense | C/C | C/C | C/C | C/C | C/C | C/C | C/C | C/C | C/C |

| Chr | Start | End | Ref | Alt | Type | Gene | Effect | | | | | | | | | | | | | |
|---|---|---|---|---|---|---|---|---|---|---|---|---|---|---|---|---|---|---|---|---|
| 7 | 138145420 | 138145420 | C | A | SNV | TRIM24 | Missense | C/C | C/A | C/C | C/C | C/C | C/A | C/C | C/C | C/C | C/C | C/C | C/C | C/C |
| 7 | 138145435 | 138145435 | G | T | SNV | TRIM24 | Missense | G/G | G/T | G/G | G/G | G/G | G/T | G/G | G/G | G/G | G/G | G/G | G/G | G/G |
| 7 | 138145436 | 138145436 | C | A | SNV | TRIM24 | Missense | C/C | C/C | C/A | C/C | C/A | C/C | C/A | C/A | C/A | C/C | C/A | C/C | C/C |
| 7 | 138145493 | 138145493 | C | T | SNV | TRIM24 | Missense | C/C | C/T | C/C | C/C | C/C | C/T | C/C | C/C | C/C | C/C | C/C | C/C | C/C |
| 7 | 138239512 | 138239512 | A | G | SNV | TRIM24 | Missense | A/A | A/A | A/A | A/A | A/A | A/G | A/A | A/A | A/A | A/A | A/A | A/A | A/A |
| 7 | 138239600 | 138239600 | G | T | SNV | TRIM24 | Missense | G/G | G/T | G/G | G/G | G/G | G/T | G/G | G/G | G/G | G/G | G/G | G/G | G/G |
| 7 | 139416737 | 139416737 | T | C | SNV | HIPK2 | Missense | T/T | T/C | T/T | T/T | T/T | T/C | T/T | T/T | T/T | T/T | T/T | T/T | T/T |
| 8 | 8239069 | 8239069 | C | A | SNV | SGK223,AC068 353.1 | Missense | C/A | C/A | C/A | C/A | C/A | C/A | C/A | C/A | C/A | C/A | C/A | C/A | C/A |
| 8 | 8239099 | 8239099 | G | T | SNV | SGK223,AC068 353.1 | Missense | G/G | G/T | G/G | G/G | G/G | G/T | G/G | G/G | G/G | G/G | G/G | G/G | G/G |
| 8 | 11420535 | 11420535 | G | A | SNV | BLK | Missense | G/G | G/G | G/G | G/G | G/G | G/G | G/G | G/G | G/G | G/G | G/G | G/G | G/G |
| 8 | 141900700 | 141900700 | T | C | SNV | PTK2 | Missense | T/T | T/C | T/T | T/T | T/T | T/C | T/T | T/T | T/T | T/T | T/T | T/T | T/T |
| 8 | 145617777 | 145617777 | G | A | SNV | ADCK5 | Missense | G/A | G/A | G/A | G/A | G/A | G/A | G/A | G/A | G/A | G/A | G/A | G/A | G/A |
| 9 | 21971137 | 21971137 | T | G | SNV | CDKN2A | Missense | T/T | T/T | T/T | T/T | T/T | T/T | T/T | T/T | T/T | T/T | T/T | T/T | T/T |
| 9 | 27157925 | 27157925 | G | A | SNV | TEK | Missense | G/G | G/A | G/G | G/G | G/G | G/A | G/G | G/G | G/G | G/G | G/G | G/G | G/G |
| 9 | 35792430 | 35792430 | T | G | SNV | NPR2 | Missense | T/T | T/G | T/T | T/T | T/T | T/G | T/T | T/T | T/T | T/T | T/T | T/T | T/T |
| 9 | 35792621 | 35792621 | A | C | SNV | NPR2 | Missense | A/A | A/C | A/A | A/A | A/A | A/C | A/A | A/A | A/A | A/A | A/A | A/A | A/A |
| 9 | 35792652 | 35792652 | A | C | SNV | NPR2 | Missense | A/A | A/C | A/A | A/A | A/A | A/C | A/A | A/A | A/A | A/A | A/A | A/A | A/A |
| 9 | 95397512 | 95397512 | A | T | SNV | IPPK | Missense | C/C | C/T | C/C | C/C | C/C | C/T | C/C | C/C | C/C | C/C | C/C | C/C | C/C |
| 9 | 95397572 | 95397572 | C | T | SNV | IPPK | Missense | G/G | G/G | G/G | G/G | G/G | G/G | G/G | G/G | G/G | G/G | G/G | G/G | G/G |
| 9 | 95397579 | 95397579 | G | A | SNV | IPPK | Missense | T/G | T/G | T/G | T/G | T/G | T/G | T/G | T/G | T/G | T/G | T/G | T/G | T/G |
| 9 | 96055149 | 96055149 | T | G | SNV | WNK2 | Missense | T/T | T/T | T/T | T/T | T/T | T/T | T/T | T/T | T/T | T/T | T/T | T/T | T/T |
| 9 | 96062366 | 96062366 | T | G | SNV | WNK2 | Missense | A/A | A/A | A/A | A/A | A/A | A/G | A/A | A/A | A/A | A/A | A/A | A/A | A/A |
| 9 | 96062368 | 96062368 | A | G | SNV | WNK2 | Missense | A/A | A/G | A/A | A/A | A/A | A/G | A/A | A/A | A/A | A/A | A/A | A/A | A/A |
| 10 | 43623623 | 43623623 | A | G | SNV | RET | Missense | A/A | A/G | A/A | A/A | A/A | A/G | A/A | A/A | A/A | A/A | A/A | A/A | A/A |
| 10 | 54053611 | 54053611 | A | G | SNV | PRKG1,RP11-573I11.2 | Missense | A/A | A/G | A/A | A/A | A/A | A/G | A/A | A/A | A/A | A/A | A/A | A/A | A/A |
| 10 | 75579353 | 75579353 | A | G | SNV | CAMK2G | Missense | A/A | A/G | A/A | A/A | A/A | A/G | A/A | A/A | A/A | A/A | A/A | A/A | A/A |
| 10 | 75579373 | 75579373 | G | A | SNV | CAMK2G | Missense | G/G | G/A | G/G | G/G | G/G | G/A | G/G | G/G | G/G | G/G | G/G | G/G | G/G |
| 10 | 75585058 | 75585058 | G | G | SNV | CAMK2G | Missense | G/G | G/G | G/G | G/G | G/G | G/G | G/G | G/G | G/G | G/G | G/G | G/G | G/G |
| 10 | 99400747 | 99400747 | C | A | SNV | PI4K2A,RP11-548K23.11 | Missense | C/A | C/C | C/A | C/A | C/A | C/C | C/A | C/A | C/A | C/A | C/A | C/A | C/A |
| 11 | 33374842 | 33374842 | A | T | SNV | HIPK3,AL1220 15.1 | Missense | A/A | A/T | A/A | A/A | A/A | A/T | A/A | A/A | A/A | A/A | A/A | A/A | A/A |
| 11 | 33374968 | 33374968 | T | A | SNV | HIPK3,AL1220 15.1 | Missense | T/T | T/A | T/T | T/T | T/T | T/A | T/T | T/T | T/T | T/T | T/T | T/T | T/T |
| 11 | 46388419 | 46388419 | C | A | SNV | DGKZ | Missense | T/T | T/C | T/T | T/T | T/T | T/C | T/T | T/T | T/T | T/T | T/T | T/T | T/T |
| 11 | 63672392 | 63672392 | G | A | SNV | MARK2 | Missense | A/A | A/A | A/A | A/A | A/A | A/G | A/A | A/A | A/A | A/A | A/A | A/A | A/A |
| 11 | 64014106 | 64014106 | C | T | SNV | PPP1R14B,RP11-783K16.13,RP11-783K16.5 | Missense | C/C | C/T | C/C | C/C | C/C | C/T | C/C | C/C | C/C | C/C | C/C | C/C | C/C |
| 11 | 64568297 | 64568297 | C | A | SNV | MAP4K2 | Missense | C/A | C/C | C/A | C/A | C/A | C/C | C/A | C/A | C/A | C/A | C/A | C/A | C/A |
| 11 | 69457880 | 69457880 | G | C | SNV | CCND1 | Missense | G/G | G/C | G/G | G/G | G/G | G/C | G/G | G/G | G/G | G/G | G/G | G/G | G/G |
| 11 | 108164101 | 108164101 | C | T | SNV | ATM | Missense | C/T | C/T | C/T | C/T | C/T | C/T | C/T | C/T | C/T | C/T | C/T | C/T | C/T |

| Chr | Position | Position | Ref | Alt | Type | Gene | Effect | | | | | | | | | | | | | | | | |
|---|---|---|---|---|---|---|---|---|---|---|---|---|---|---|---|---|---|---|---|---|---|---|---|
| 12 | 989896 | 989896 | C | T | SNV | WNK1 | Missense | C/C | C/C | C/C | C/C | C/C | C/T | C/C | C/C | C/C | C/C | C/C | C/C | C/C | C/C | C/C | C/C |
| 12 | 14836079 | 14836079 | A | C | SNV | GUCY2C,RP11-174G6.1 | Missense | A/A | A/A | A/A | A/A | A/A | A/A | A/A | A/A | A/A | A/A | A/A | A/A | A/A | A/A | A/A | A/C |
| 12 | 25368386 | 25368386 | T | T | SNV | KRAS | Missense | T/T | T/T | T/T | T/T | T/T | T/C | T/T | T/T | T/T | T/T | T/T | T/C | T/T | T/T | T/C | T/T |
| 12 | 25398284 | 25398284 | C | C | SNV | KRAS | Missense | C/C | C/A | C/A | C/A | C/A | C/A | C/A | C/A | C/A | C/A | C/A | C/A | C/A | C/A | C/A | C/A |
| 12 | 53776023 | 53776023 | A | G | SNV | SP1 | Missense | A/A | A/A | A/A | A/A | A/A | A/G | A/A | A/A | A/A | A/A | A/A | A/A | A/A | A/A | A/G | A/A |
| 12 | 53776185 | 53776185 | G | A | SNV | SP1 | Missense | G/G | G/G | G/G | G/G | G/G | G/A | G/G | G/G | G/G | G/G | G/G | G/G | G/G | G/G | G/G | G/G |
| 12 | 53776377 | 53776377 | A | C | SNV | SP1 | Missense | A/A | A/A | A/A | A/A | A/A | C/T | A/A | A/A | A/A | A/A | A/A | A/A | A/A | A/A | A/A | A/A |
| 12 | 68043724 | 68043724 | A | T | SNV | DYRK2 | Missense | C/C | C/C | C/C | C/C | C/C | C/C | C/C | C/C | C/C | C/C | C/C | C/C | C/C | C/C | A/C | C/C |
| 12 | 118619189 | 118619189 | T | G | SNV | TAOK3 | Missense | A/A | T/T | A/A | A/A | A/A | A/G | A/A | A/A | A/A | A/A | A/A | A/A | A/A | A/A | A/G | A/A |
| 12 | 118627667 | 118627667 | T | C | SNV | TAOK3 | Missense | T/T | T/T | T/T | T/T | T/T | T/C | T/T | T/T | T/T | T/T | T/T | T/T | T/T | T/T | T/C | T/T |
| 12 | 118627734 | 118627734 | T | C | SNV | TAOK3 | Missense | T/T | T/T | T/T | T/T | T/T | T/C | T/T | T/T | T/T | T/T | T/T | T/T | T/T | T/T | T/T | T/T |
| 13 | 32912805 | 32912805 | T | C | SNV | BRCA2 | Missense | T/T | T/T | T/T | T/T | T/C | T/T | T/C | T/T | T/T | T/T | T/C | T/T | T/T | T/T | T/T | T/T (O.K.) |
| 13 | 42795407 | 42795407 | A | T | SNV | DGKH | Missense | A/A | A/A | A/A | A/A | A/A | A/T | A/A | A/A | A/A | A/A | A/A | A/A | A/A | A/A | A/T | A/A |
| 13 | 42795467 | 42795467 | T | A | SNV | DGKH | Missense | T/T | T/T | T/T | T/T | T/T | T/A | T/T | T/T | T/T | T/T | T/T | T/T | T/T | T/T | T/T | T/T |
| 13 | 42795486 | 42795486 | A | G | SNV | DGKH | Missense | A/A | A/A | A/A | A/A | A/A | A/G | A/A | A/A | A/A | A/A | A/A | A/A | A/A | A/A | A/A | A/A |
| 13 | 99109545 | 99109545 | C | C | SNV | STK24 | Missense | C/C | C/G | C/G | C/G | C/G | C/G | C/G | C/G | C/G | C/G | C/G | C/G | C/G | C/G | C/G | C/G |
| 13 | 110434668 | 110434668 | C | C | SNV | IRS2 | Missense | C/A | C/A | C/A | C/A | C/A | C/A | C/A | C/A | C/A | C/A | C/A | C/A | C/A | C/A | C/A | C/A |
| 14 | 30046467 | 30046467 | C | C | SNV | PRKD1,MIR548AI | Missense | C/C | C/C | C/C | C/C | C/C | C/T | C/C | C/C | C/C | C/C | C/C | C/C | C/C | C/C | C/C | C/C |
| 14 | 30046484 | 30046484 | G | G | SNV | PRKD1,MIR548AI | Missense | G/G | G/G | G/G | G/G | G/G | G/C | G/G | G/G | G/G | G/G | G/G | G/G | G/G | G/G | G/G | G/G |
| 14 | 30046494 | 30046494 | T | T | SNV | PRKD1,MIR548AI | Missense | T/T | T/T | T/T | T/T | T/T | T/C | T/T | T/T | T/T | T/T | T/T | T/T | T/T | T/T | T/T | T/T |
| 14 | 30046502 | 30046502 | G | G | SNV | PRKD1,MIR548AI | Missense | G/G | G/G | G/G | G/G | G/G | G/C | G/G | G/G | G/G | G/G | G/G | G/G | G/G | G/G | G/G | G/G |
| 14 | 35872509 | 35872509 | C | C | SNV | NFKBIA | Missense | C/C | C/C | C/C | C/C | C/C | C/T | C/C | C/C | C/C | C/C | C/C | C/C | C/C | C/C | C/C | C/C |
| 14 | 71197492 | 71197492 | A | G | SNV | MAP3K9 | Missense | G/G | G/G | G/A | G/A | G/A | G/A | G/A | G/A | G/A | G/A | G/A | G/A | G/A | G/A | G/A | G/A |
| 15 | 40504749 | 40504749 | A | C | SNV | BUB1B | Missense | A/A | A/A | A/A | A/C | A/C | A/C | A/C | A/C | A/A | A/A | A/A | A/A | A/A | A/A | A/A | G/T? |
| 15 | 43122239 | 43122239 | C | T | SNV | TTBK2 | Missense | C/C | C/T | C/T | C/T | C/T | C/T | C/T | C/T | C/T | C/T | C/T | C/T | C/C | C/T | C/T | C/T |
| 15 | 77474141 | 77474141 | A | A | SNV | PEAK1,AC087465.1 | Missense | A/A | A/A | A/A | A/A | A/A | A/C | A/A | A/A | A/A | A/A | A/A | A/A | A/A | A/A | A/A | A/A |
| 15 | 77474144 | 77474144 | T | T | SNV | PEAK1,AC087465.1 | Missense | T/T | T/T | T/T | T/T | T/T | T/C | T/T | T/T | T/T | T/T | T/T | T/T | T/T | T/T | T/T | T/T |
| 15 | 77474163 | 77474163 | C | C | SNV | PEAK1,AC087465.1 | Missense | C/C | C/C | C/C | C/C | C/C | C/C | C/C | C/C | C/C | C/C | C/C | C/C | C/C | C/C | C/C | C/C |
| 15 | 77474172 | 77474172 | G | G | SNV | PEAK1,AC087465.1 | Missense | G/G | G/G | G/G | G/G | G/G | G/A | G/G | G/G | G/G | G/G | G/G | G/G | G/G | G/G | G/G | G/G |
| 15 | 91436551 | 91436551 | A | A | SNV | FES,AC068831 | Missense | A/G | A/A | A/A | A/A | A/A | A/G | A/A | A/A | A/A | A/A | A/A | A/A | A/A | A/A | A/A | A/G |
| 15 | 99192859 | 99192859 | C | G | SNV | IGF1R | Missense | C/C | C/C | C/C | C/C | C/C | C/G | C/C | C/C | C/C | C/C | C/C | C/C | C/C | C/C | C/G | C/C |
| 15 | 99250869 | 99250869 | A | T | SNV | IGF1R | Missense | A/A | A/A | A/A | A/A | A/A | A/T | A/A | A/A | A/A | A/A | A/A | A/A | A/A | A/A | A/A | A/A |
| 15 | 99250895 | 99250895 | G | G | SNV | IGF1R | Missense | G/G | G/G | G/G | G/G | G/T | G/T | G/G | G/G | G/G | G/G | G/G | G/G | G/G | G/G | G/G | G/G |
| 15 | 99251252 | 99251252 | A | C | SNV | IGF1R | Missense | A/A | A/A | A/A | A/A | A/A | C/T | A/A | A/A | A/A | A/A | A/A | A/A | A/A | A/A | A/A | A/A |
| 16 | 18860643 | 18860643 | C | G | SNV | SMG1 | Missense | C/C | C/T | C/T | C/T | C/T | G/G | C/T | C/T | C/T | C/T | C/T | C/T | C/T | C/T | C/T | C/C |
| 16 | 18860691 | 18860691 | G | A | SNV | SMG1 | Missense | G/G | G/G | G/G | G/G | G/G | G/A | G/G | G/G | G/G | G/G | G/G | G/G | G/G | G/G | G/G | G/A |

| 16 | 18907410 | 18907410 | G | A | SNV | SMG1 | Missense | G/G | G/A | G/A | G/G | G/A | G/A | G/A | G/A | G/G | G/A | G/A | G/A | |
|---|---|---|---|---|---|---|---|---|---|---|---|---|---|---|---|---|---|---|---|---|
| 16 | 18907521 | 18907521 | T | C | SNV | SMG1 | Missense | T/T | T/T | T/T | T/T | T/C | T/T | T/T | T/T | T/T | T/T | T/T | T/C | |
| 16 | 23692286 | 23692286 | C | T | SNV | PLK1 | Missense | C/C | C/T | C/T | C/T | C/T | C/T | C/T | C/T | C/T | C/T | C/T | C/T | O.K. |
| 16 | 46744689 | 46744689 | C | A | SNV | MYLK3 | Missense | C/A | C/A | C/C | C/A | C/C | C/C | C/C | C/A | C/A | C/C | C/A | C/C | |
| 16 | 67942747 | 67942747 | G | A | SNV | PSKH1 | Missense | G/G | G/G | G/G | G/G | G/G | G/A | G/G | G/G | G/G | G/G | G/G | G/G | |
| 16 | 67942794 | 67942794 | G | C | SNV | PSKH1 | Missense | G/G | G/G | G/G | G/G | G/G | G/C | G/G | G/G | G/G | G/G | G/G | G/G | |
| 16 | 67942809 | 67942809 | C | T | SNV | PSKH1 | Missense | C/C | C/C | C/C | C/C | C/C | C/T | C/C | C/C | C/C | C/C | C/C | C/C | |
| 16 | 67942815 | 67942815 | G | A | SNV | PSKH1 | Missense | G/G | G/G | G/G | G/G | G/G | G/A | G/G | G/G | G/G | G/G | G/G | G/G | |
| 17 | 7792338 | 7792338 | T | C | SNV | CHD3 | Missense | T/T | T/T | T/T | T/C | T/T | T/T | T/T | T/T | T/T | T/T | T/T | T/T | |
| 17 | 7796803 | 7796803 | T | C | SNV | CHD3 | Missense | T/C | T/C | T/T | T/T | T/C | T/C | T/C | T/C | T/T | T/C | T/T | T/T | |
| 17 | 7806028 | 7806028 | C | T | SNV | CHD3 | Missense | C/C | C/C | C/C | C/C | C/C | C/C | C/C | C/C | C/C | C/C | C/C | C/C | |
| 17 | 7810274 | 7810274 | G | T | SNV | CHD3 | Missense | G/G | G/G | G/G | G/G | G/G | G/G | G/G | G/T | G/G | G/G | G/G | G/G | |
| 17 | 25932583 | 25932583 | T | C | SNV | KSR1 | Missense | T/T | T/T | T/C | T/T | T/C | T/T | T/C | T/T | T/T | T/T | T/C | T/C | |
| 17 | 26369915 | 26369915 | G | A | SNV | NLK | Missense | G/G | G/G | G/G | G/G | G/G | G/A | G/G | G/G | G/G | G/G | G/A | G/A | |
| 17 | 27869759 | 27869759 | G | A | SNV | TAOK1 | Missense | G/G | G/G | G/G | G/G | G/G | G/A | G/G | G/G | G/G | G/G | G/G | G/G | |
| 17 | 27869819 | 27869819 | C | A | SNV | TAOK1 | Missense | C/C | C/C | C/C | C/C | C/C | C/A | C/C | C/C | C/C | C/C | C/C | C/C | |
| 17 | 29579999 | 29579999 | A | G | SNV | NF1 | Missense | A/A | A/A | A/A | A/A | A/A | A/G | A/A | A/A | A/A | A/A | A/G | A/A | |
| 17 | 37687090 | 37687090 | C | T | SNV | CDK12 | Missense | C/C | C/C | C/C | C/C | C/C | C/T | C/C | C/C | C/C | C/C | C/C | C/C | |
| 17 | 37687094 | 37687094 | G | A | SNV | CDK12 | Missense | G/G | G/G | G/G | G/G | G/G | G/A | G/G | G/G | G/G | G/G | G/G | G/G | |
| 17 | 40948585 | 40948585 | G | A | SNV | WNK4,AC016889.1 | Missense | G/G | G/G | G/G | G/G | G/G | G/A | G/G | G/G | G/G | G/G | G/A | G/G | |
| 17 | 41245693 | 41245693 | G | T | SNV | BRCA1 | Missense | G/G | G/T | G/G | G/T | G/T | G/T | G/T | G/T | G/G | G/T | G/T | G/G | |
| 17 | 60637441 | 60637441 | G | A | SNV | TLK2 | Missense | G/G | G/G | G/G | G/G | G/G | G/G | G/G | G/G | G/A | G/G | G/G | G/G | |
| 17 | 64298983 | 64298983 | T | A | SNV | PRKCA | Missense | T/T | T/T | T/T | T/T | T/T | T/A | T/T | T/T | T/T | T/T | T/T | T/T | |
| 17 | 64298989 | 64298989 | G | C | SNV | PRKCA | Missense | G/G | G/G | G/G | G/G | G/G | G/C | G/G | G/G | G/G | G/G | G/G | G/G | |
| 18 | 56246818 | 56246818 | G | A | SNV | ALPK2 | Missense | G/G | G/G | G/A | G/G | G/A | G/G | G/A | G/G | G/G | G/A | G/A | G/A | |
| 18 | 59947662 | 59947662 | A | G | SNV | KIAA1468 | Missense | A/A | A/A | A/A | A/A | A/A | A/G | A/A | A/A | A/A | A/A | A/G | A/A | |
| 19 | 3959103 | 3959103 | C | T | SNV | DAPK3 | Missense | C/C | C/C | C/T | C/C | C/T | C/C | C/T | C/C | C/C | C/C | C/T | C/T | |
| 19 | 10461521 | 10461521 | T | C | SNV | TYK2 | Missense | T/T | T/T | T/T | T/C | T/T | T/T | T/T | T/T | T/T | T/T | T/T | T/T | |
| 19 | 14203935 | 14203935 | A | T | SNV | PRKACA | Missense | A/A | A/A | A/A | A/A | A/A | A/T | A/A | A/A | A/A | A/A | A/A | A/A | |
| 19 | 15353818 | 15353818 | T | G | SNV | BRD4,AC020911.1 | Missense | T/T | T/G | T/G | T/T | T/T | T/T | T/T | T/T | T/T | T/T | T/T | T/T | |
| 19 | 15383904 | 15383904 | C | T | SNV | BRD4,AC020911.1 | Missense | C/C | C/C | C/C | C/C | C/C | C/T | C/C | C/C | C/C | C/C | C/C | C/C | |
| 19 | 47193933 | 47193933 | G | T | SNV | PRKD2 | Missense | G/G | G/G | G/T | G/T | G/T | G/G | G/G | G/T | G/G | G/G | G/G | G/G | |
| 19 | 48997039 | 48997039 | C | G | SNV | LMTK3 | Missense | C/C | C/C | C/C | C/C | C/C | C/C | C/G | C/C | C/C | C/C | C/C | C/C | |
| 19 | 48997079 | 48997079 | C | T | SNV | LMTK3 | Missense | C/C | C/C | C/C | C/C | C/C | C/T | C/C | C/C | C/C | C/C | C/T | C/C | |
| 19 | 48997084 | 48997084 | G | C | SNV | LMTK3 | Missense | G/G | G/G | G/G | G/G | G/G | G/C | G/G | G/G | G/G | G/G | G/C | G/G | |
| 20 | 468110 | 468110 | G | A | SNV | CSNK2A1 | Missense | G/G | G/G | G/G | G/G | G/G | G/G | G/A | G/G | G/G | G/G | G/G | G/G | |
| 20 | 2082732 | 2082732 | C | G | SNV | STK35 | Missense | C/G | C/C | C/C | C/C | C/C | C/C | C/C | C/C | C/C | C/C | C/C | C/C | |
| 20 | 2097369 | 2097369 | T | A | SNV | STK35 | Missense | T/T | T/T | T/T | T/T | T/T | T/A | T/T | T/T | T/T | T/T | T/T | T/T | |
| 20 | 2097923 | 2097923 | A | G | SNV | STK35 | Missense | A/A | A/A | A/A | A/A | A/A | A/G | A/A | A/A | A/A | A/A | A/A | A/A | |
| 20 | 42204913 | 42204913 | A | C | SNV | SGK2 | Missense | A/A | A/A | A/A | A/A | A/A | A/A | A/A | A/C | A/A | A/A | A/A | A/A | |
| 21 | 33246120 | 33246120 | C | T | SNV | HUNK | Missense | C/C | C/C | C/C | C/C | C/C | C/T | C/C | C/C | C/C | C/C | C/C | C/C | |
| 21 | 38884754 | 38884754 | A | G | SNV | DYRK1A | Missense | A/A | A/A | A/A | A/A | A/A | A/G | A/A | A/A | A/A | A/A | A/G | A/A | |
| 22 | 21067589 | 21067589 | C | G | SNV | PI4KA | Missense | C/G | C/G | C/G | C/G | C/G | C/C | C/G | C/C | C/G | C/G | C/G | C/G | |

| X | 21670542 | 21670542 | A | G | SNV | CNKSR2 | Missense | A/A | A/A | A/A | A/A | A/A | A/G | A/A | A/A | A/A | A/A | A/A | A/A |
|---|---|---|---|---|---|---|---|---|---|---|---|---|---|---|---|---|---|---|---|
| X | 47430344 | 47430344 | A | G | SNV | ARAF | Missense | A/A | A/A | A/G | A/A | A/G | A/A | A/G | A/A | A/A | A/A | A/G | A/G |
| X | 54265387 | 54265387 | T | C | SNV | WNK3 | Missense | T/T | T/T | T/T | T/T | T/T | T/C | T/T | T/T | T/T | T/T | T/C | T/T |
| X | 54265463 | 54265463 | C | T | SNV | WNK3 | Missense | C/C | C/C | C/C | C/C | C/C | C/T | C/C | C/C | C/C | C/C | C/C | C/C |
| X | 54265468 | 54265468 | C | T | SNV | WNK3 | Missense | C/C | C/C | C/C | C/C | C/C | C/T | C/C | C/C | C/C | C/C | C/C | C/C |
| X | 54265523 | 54265523 | A | G | SNV | WNK3 | Missense | A/A | A/A | A/A | A/A | A/A | A/G | A/A | A/A | A/A | A/A | A/A | A/A |
| X | 108697006 | 108697006 | T | C | SNV | GUCY2F | Missense | T/T | T/T | T/T | T/T | T/T | T/C | T/T | T/T | T/T | T/T | T/T | T/T |
| X | 108697016 | 108697016 | C | T | SNV | GUCY2F | Missense | C/C | C/C | C/C | C/C | C/C | C/T | C/C | C/C | C/C | C/C | C/C | C/C |

Finally, tertiary refinining was performed using the following criterion:

- SNVs that were found to have coincidence between the secondary refining results and the results of visual inspection of the read mapping results (as indicated by "OK" in the rightmost column). The respective SNVs from induced malignant stem cells that met this criterion were boxed off with a double line.

As a result of analyzing the SNVs of CTNNB1 and DGKB by the Sanger's sequencing method, they were verified to be those somatic mutations in induced malignant stem cells which were not found in the genomic DNAs (germline sequences) of the non-cancer tissue cells. Thus, the SNVs analyzed by the next-generation sequencer and detected by informatics analysis (primary analysis, secondary analysis, tertiary analysis based on visual determination) were proved to be accurate. Accordingly, the SNVs detected by the next-generation sequencer analysis and the informatics analysis can be determined to be those somatic mutations in induced malignant stem cells which are different from those in the genomic DNA sequences of the non-cancer tissue cells. Since the Agilent Human Kinome DNA kit is designed to target 612 types of cancer-related gene regions (kinases, kinase-related genes, and cancer-related genes), the investigated induced malignant stem cells can be described as cells characterized both by somatic mutations of cancer-related gene regions in endogenous genomic DNAs, and by expression of the ES cell-specific genes (OCT3/4, NANOG, SOX2, ZFP42). Further, since the SNVs detected in this Example were considered to be somatic mucations in cancer-related gene regions, they can be considered to be driver mutations involved in carcinogenesis and cancer progression. Therefore, the induced malignant stem cells can be described as cells characterized both by driver mutations of endogenous genomic DNAs which are involved in carcinogenesis and cancer progression, and by expression of the ES cell-specific genes (OCT3/4, NANOG, SOX2, ZFP42).

Example 11: Detection for an aberration of gene copy number variations of endogenous genomic DNA in induced malignant stem cells

In this Example, (1) (h) an aberration of gene copy number varitations of endogenous genomic DNA in induced malignant stem cells were detected, in comparison with genetic capy number variations in genomic DNA of cell populations derived from fresh non-cancer site tissues.

(11-1) Materials

The an aberration of gene copy number variations of endogenous genomic DNA was detected by subjecting induced malignant stem cells to the Comparative Genomic Hybridization (CGH) method using the Agilent CGH microarray (SurePrint G3 Human CGH Microarray Kit 1×1 M) analysis to conduct genome-wide analysis of change in DNA copy number variations.

The genomic DNAs of the following samples were used in the Agilent CGH microarray analysis:

- cell population (ncc3) derived from colon non-cancer site tissues, and induced malignant stem cells (CC3_6) prepared from fresh colon cancer tissues, which were collected from the individual of donor No. 2;
- cell population (ngc1) derived from gastric non-cancer site tissues, and induced malignant stem cells (GC1_9) prepared from fresh gastric cancer tissues, which were collected from the individual of donor No. 3;
- cell population (ncc1) derived from colon non-cancer site tissues, and induced malignant stem cells (CC1_17) prepared from fresh colon cancer tissues, which were collected from the individual of donor No. 4; and
- cell population (ncc4) derived from fresh colon non-cancer site tissues, cell population (cc4) derived from fresh colon cancer site tissues, and induced malignant stem cells (CC4-D) prepared from fresh colon cancer tissues, which were collected from the individual of donor No. 5.

This analysis detected an aberration of gene copy number variations (CNVs) of the endogenous genomic DNA in the induced malignant stem cells (CC3_6, GC1_9, CC1_17, CC4-D). The genomic DNAs of the cell populations derived from non-cancer site tissues were used as the negative control having normal genetic copy number variations of endogenous genomic DNA. The genomic DNAs of the cell population (cc4) derived from cancer site tissues were used as the positive control having an aberration of gene copy number variations of endogenous genomic DNA.

The details of the kits and samples used in this analysis were summarized in Table 21 below.

[Table 21]

Table 21: Summary of the tests used in the CGH analysis

1) Chip type

| Species | Human |
|---|---|
| Probe arrays analyzed | SurePrint G3 Human CGH Microarray Kit 1×1 M |
| Agilent Order Number* | 252152930109, 252152930111, 252152930112 252152930113, 252152930155 |

* Agilent Order Number is required for viewying the "Design File" information in the Agilent website.

2) Samples analyzed

| Sample No. | Name | | Sample No. | Name |
|---|---|---|---|---|
| AD0040_01 | ncc3 | | AD0040_13 | ncc1 [*1] |
| AD0040_02 | CC3_6 | | AD0040_14 | ncc4 [*1] |
| AD0040_03 | ngc1 | | AD0040_15 | cc4 [*1] |
| AD0040_04 | GC1_9 | | AD0040_16 | ncc4 [*1] |
| AD0040_05 | ncc1 | | AD0040_17 | CC3_6 [*2] |
| AD0040_06 | CC1_17 | | AD0040_18 | GC1_9 [*2] |
| AD0040_07 | ncc4 | | AD0040_19 | ncc1 [*2] |
| AD0040_08 | cc4 | | AD0040_20 | CC4-D [*2] |
| AD0040_09 | ncc4 | | AD0040_21 | CC3_6 [*3] |
| AD0040_10 | CC4-D | | AD0040_22 | GC1_9 [*3] |
| AD0040_11 | ncc3 [*1] | | AD0040_23 | ncc1 [*3] |
| AD0040_12 | ngc1 [*1] | | | |

*1: Sample after the 1st run of purification.    *2: Sample after the 2nd run of purification.

*3: Sample after the 3rd run of purification.

3) Comparative analyses

| Comprison No. | Rreference: Sample No. (name) | Test: Sample No. (name) |
|---|---|---|
| Set01 | AD0040_11 (ncc3) | AD0040_17 (cc3_6) |
| Set02 | AD0040_12 (ngc1) | AD0040_22 (gc1_9) |
| Set03 | AD0040 19 (ncc1) | AD0040_06 (cc1_17) |
| Set04 | AD0040_14 (ncc4) | AD0040_15 (cc4) |
| Set05 | AD0040_16 (ncc4) | AD0040_20 (cc4-d) |

(11-2) Protocol

This analysis was made by performing target preparation, hybridization, scanning, and data analysis using SurePrint G3 Human CGH Microarray Kit 1 ×1 M in accordance with the Protocol for Oligonucleotide Array-Based CGH for Genomic DNA Analysis Ver. 6.1 (URL: http://www.chem.agilent.com/en-us/Search/Library/_layouts/Agilent/PublicationSummary.aspx?whid=52010).

Targets were prepared using Genomic DNA Enzymatic Labeling Kit (Agilent Technologies). More specifically, genomic DNAs (gDNAs) were first prepared (0.5-3 μg) and were enzymatically digested with the restriction enzymes AluI (New England Biolabs Japan) and RsaI (New England Biolabs Japan); thereafter, the random primers included in Genomic DNA Enzymatic Labeling Kit were added, and Exo-Klenow reaction was performed using Exo-Klenow also included in Genomic DNA Enzymatic Labeling Kit to synthesize genomic DNAs with cy3- and cy5-labeled by cyanine 3-dUTP and cyanine 5-dUTP. The cy3- and cy5-labeled genomic DNAs were used to confirm their yield and quality through the quality test of genomic DNAs as described below in (11-3).

Afterwards, genomic DNAs were respectively prepared such that their amount was 500 ng, on the basis of the concentrations calculated after the quality control of respective DNA samples using the fluorometry for specifically quantitating double-stranded DNAs. The cy3- and cy5-labeled gDNAs were hybridized (65°C, 40 hours, 20 r.p.m.) onto Human Genome CGH Microarray (Agilent Technologies) using aCGH/ChIP-on-chip Hybridization Kit (Agilent Technologies), and were then washed using Oligo aCGH/ChIP-on-Chip Wash Buffer Kit (Agilent Technologies).

After the cy3- and cy5-labeled gDNAs were hybridized with the microarray in this manner, the microarray was scanned using a laser scanner such as High Resolution Microarray Scanner (Agilent Technologies) at an optimum wavelength for Cy3 and Cy5 to acquire an image. The acquired image was analyzed using a special-purpose analysis software (Feature Extraction; Agilent Technologies) to perform quality test of the sample genomic DNAs and generate the test results data.

(11-3) Quality test of sample genomic DNAs

The samples were first subjected to quality test of genomic DNAs using absorbance measurement and agarose gel electrophoresis to check to see if they were analyzable in an Agilent aCGH microarray, deeming that the samples satisfying the following criteria passed the check. To be specific, the criteria for absorbance measurement were as follows:

(i) genomic DNA concentration of 25 ng/$\mu$L or higher;
(ii) $OD_{260/280}$ ranges 1.8-2.0 and $OD_{260/230}$ ranges 2.0 or higher; and
(iii) no abnormality observed in absorption spectrum.

The criteria for agarose gel electrophoresis, which are based on the electrophoretic test of 100 ng of genomic DNAs as calculated from the absorbance measurement results, are as follows:

(i) a main band is observed between around 10 Kb to 20 Kb;
(ii) no contaminating band (including RNA) is observed;
(iii) a smear band showing progression of degradation is not observed; and
(iv) a divergence from the concentration predicted from absorbance is observed.

As a result of this quality test, the eleven samples (AD0040_01 (ncc3), AD0040_02 (CC3_6), AD0040_03 (ngc1), AD0040_04 (GC1_9), AD0040_05 (ncc1), AD0040_07 (ncc4), AD0040_08 (cc4), AD0040_09 (ncc4), AD0040_10 (CC4-D), AD0040_13 (ncc1 [*1]), AD0040_18 (GC1_9 [*2])) were excluded from the test because their extracted DNA concentrations did not meet the criteria.

(11-4) Agilent aCGH analysis

The data output by the Feature Extraction software after scanning of the microarray was subjected to copy number analysis (analysis by default-setting) using the genomics analysis software (Agilent Genomic Workbench; Agilent Technologies). The obtained data is summarized in Table 22 below.

In Table 22, "AD0040_Set01" to "AD0040_Set05" respectively correspond to "Set01" to "Set05" shown in "3) Comparative analyses" in Table 21. The Table 22 lists the chromosomes (Chr) of the subject cells having an aberration of gene copy numbers detected as compared with the reference cells, the positions on the chromosomes (Cytoband), and the detailed information of the positions (Position), thereby showing the probes applied to the positions. This table also shows in the "Gene Names, Annotations" column the representative names and genome annotations of the genes that are known in databases to be present in the positions on the listed chromosomes.

This table further summarizes in the "Amp/Del" and "P-value" columns the statuses of the aberration of gene copy numbers. In the "Amp/Del" column, increased and reduced genomic DNA copy numbers in the subject cells as compared with those of the reference cells are indicated by positive and negative values, respectively. The p-values from the results of the statistical analysis of the increases and decreases are listed in the "P-value" column.

[Table 22]

| Table22:Results of detectionfor an aberrationof gene copynumbers AD00040_Set01 | | | | | | |
|---|---|---|---|---|---|---|
| No. | Chr | Cytoband | Position | Amp/Del | P-value | Gene Names, Annotation |
| 1 | 17 | p13.3 | 1959569-1959686 | 1.958922 | 4.24E-24 | SMG6, CNV_72769 |

(continued)

| AD0040_Set02 | | | | | | |
|---|---|---|---|---|---|---|
| No. | Chr | Cytoband | Position | Amp/Del | P-value | Gene Names, Annotations |
| 1 | 1 | q32.1 | 203188201-203193723 | -1.154289 | 7.26E-11 | NFASC |
| 2 | 2 | p21 | 44083711-44096180 | -1.466304 | 1.64E-14 | CNV_78526, CNV_89620, CNV_73443... |
| 3 | 4 | p16.1 | 8575302-8575359 | -1.004219 | 2.68E-12 | CNV_3479 |
| 4 | 4 | p16.1 | 8881212-8882547 | -1.338401 | 2.49E-12 | CNV_3479, CNV_2497, CNV_0347... |
| 5 | 4 | q22.1 | 93434342-93961504 | -0.977201 | 9.66E-277 | GRID2, CNV_10054, CNV_4406... |
| 6 | 5 | p13.3 | 34268357-34369165 | -1.613071 | 5.91E-13 | CNV_3553, CNV_4438, CNV_2087... |
| 7 | 11 | p12 | 41852545-42432402 | -1.001829 | 1.55E-69 | CNV_65929, CNV_61127 |
| AD0040_Set03 | | | | | | |
| No. | Chr | Cytoband | Position | Amp/Del | P-value | Gene Names, Annotations |
| 1 | 1 | p36.22 | 11506047-11510410 | -0.91124 | 1.45E-10 | PTCHD2 |
| 2 | 1 | p36.13 | 19384235-19393273 | -1.077065 | 8.52E-16 | UBR4 |
| 3 | 2 | p21 | 44083711-44100016 | -0.835072 | 4.02E-13 | CNV_78526, CNV_89620, CxV_73443... |
| 4 | 2 | q37.3 | 237339565-237344964 | -1.262658 | 5.45E-13 | |
| 5 | 3 | p21.31 | 44941989-44944920 | -0.96966 | 1.85E-10 | ZDHHC3 |
| 6 | 3 | p21.1 | 51941606-51945028 | -1.390224 | 6.96E-37 | RRP9 |
| 7 | 3 | p21.1 | 52157853-52166715 | -0.741786 | 2.84E-11 | WDR51A, CNV_51113 |
| 8 | 3 | q26.31 | 172536286-172538403 | 0.77431 | 4.00E-11 | TNIK |
| 9 | 4 | p16.1 | 8575302-8575359 | -1.394291 | 2.38E-22 | CNV_3479 |
| 10 | 4 | p16.1 | 8882456-8882653 | -1.522356 | 1.87E-22 | CNV_3479, CNV_2497, CNV_0347... |
| 11 | 4 | p16.1 | 8884585-8885187 | -1.388218 | 1.04E-14 | CNV_3479, CNV_2497, CNV_0347... |
| 12 | 5 | p15.33 | 39807-103486 | -0.566404 | 4.31E-17 | CNV_3536, CNV_8470, CNV_37739... |
| 13 | 5 | q35.3 | 178915974-178920549 | -0.867024 | 7.47E-11 | RUFY1, CNV_3590, CNY_2611... |

(continued)

| No. | Chr | Cytoband | Position | Amp/Del | P-value | Gene Names, Annotations |
|---|---|---|---|---|---|---|
| | | | | AD0040_Set03 | | |
| 14 | 6 | p21.32 | 31913390-31914895 | -0.936089 | 5.63E-11 | C6orf48, CNV_3602, CNV_4492 |
| 15 | 6 | q24.2 | 144681079-145176611 | -0.803721 | 0 | UTRN, CNV_5395, CNV_51815... |
| 16 | 7 | p22.3 | 1976966-1985089 | -1.174472 | 1.33E-14 | MAD1L1, CNV_4523, CNV_30253... |
| 17 | 7 | p13 | 45115983-45117728 | -0.997113 | 1.17E-10 | TBRG4 |
| 18 | 7 | q36.3 | 158315132-158317964 | -1.435031 | 1.34E-11 | CNV_70131, CNV_65009 |
| 19 | 8 | p23.3 | 1322720-1340312 | -1.256454 | 4.83E-22 | CNV_100233, CNV_70182, CNV_36754... |
| 20 | 8 | p21.3 | 20951820-20964831 | -1.229753 | 1.85E-15 | CNV_3726, CNV_82520, CNV_9531... |
| 21 | 8 | p21.3 | 22263358-22269438 | -1.053818 | 1.67E-12 | PIWIL2, CNV_3726, CNV_2746 |
| 22 | 8 | p12 | 37827143-37827202 | -1.073419 | 3.14E-18 | |
| 23 | 8 | q24.21 | 129012024-129012764 | -1.283134 | 3.75E-15 | PVT1, CNV_37296 |
| 24 | 8 | q24.3 | 145783328-145788273 | -1.211438 | 2.21E-14 | KIAA1688, CNV_4614, CNV_70495 |
| 25 | 9 | p22.1 | 19760010-19770175 | -1.098789 | 8.48E-12 | SLC24A2, CNV_52762 |
| 26 | 9 | q34.3 | 137332375-137332434 | -1.133146 | 1.90E-16 | CNV_30337, CNV_4660 |
| 27 | 10 | q24.33 | 105011210-105018167 | -0.890401 | 3.98E-12 | |
| 28 | 10 | q26.3 | 134889416-134893492 | -1.403986 | 1.89E-11 | KNDC1, CNV_3829, CNV_29875... |
| 29 | 10 | q26.3 | 134978689-134996216 | -0.759088 | 2.38E-12 | CALY, CNV_3829, CNV_4721... |
| 30 | 11 | p15.5 | 1962010-1967283 | -1.272235 | 9.22E-16 | LOC100133545, CNV_29893, CNV_37117... |
| 31 | 11 | p13 | 35269915-35269974 | -1.58566 | 4.59E-21 | SLC1A2 |
| 32 | 11 | p11.2 | 45446292-45455071 | -0.999829 | 1.27E-10 | |
| 33 | 11 | p11.2 | 45536937-45547269 | -1.0113 | 3.33E-13 | |
| 34 | 11 | q13.2 | 68845113-68855981 | -0.849465 | 1.22E-14 | CNV_29915 |
| 35 | 11 | q13.5 | 75055163-75056846 | -1.095161 | 4.03E-13 | MAP6 |

(continued)

| AD0040_Set03 | | | | | | |
|---|---|---|---|---|---|---|
| No. | Chr | Cytoband | Position | Amp/Del | P-value | Gene Names, Annotations |
| 36 | 11 | q23.2 | 114474724-114494671 | -1.04596 | 2.07E-18 | CNV_3867, CNV_4763, CNV_30567... |
| 37 | 12 | p11.1 | 34417392-34756209 | -1.388555 | 8.75E-18 | CNV_3885, CNV_8723, CNV_9691... |
| 38 | 12 | q13.2 | 54376360-54377782 | -1.314247 | 1.93E-15 | ITGA7,CNV_3890 |
| 39 | 12 | q24.11 | 107744503-107749896 | -1.312198 | 5.02E-15 | SSH1 |
| 40 | 12 | q24.11 | 110069463-110074263 | -1.00593 | 6.16E-11 | CUX2 |
| 41 | 12 | q24.32 | 124804453-124812355 | -1.02832 | 1.86E-13 | CNV_9699, CNV_29926 |
| 42 | 12 | q24.33 | 133173882-133177340 | -1.318578 | 9.51E-14 | CNV_4404 |
| 43 | 13 | q12.11 | 19566409-19568792 | -1.815439 | 3.23E-30 | CNV_71680, CNV_71679 |
| 44 | 13 | q34 | 112553940-112565338 | -1.045415 | 5.56E-12 | ATP11A |
| 45 | 13 | q34 | 114770686-114776626 | -1.49958 | 1.55E-14 | CNV_29947, CNV_71818, CNV_101882... |
| 46 | 14 | q32.31 | 101314727-101318356 | -0.864536 | 6.27E-15 | CNV_8776 |
| 47 | 15 | q26.3 | 101555153-101558598 | -1.35669 | 5.78E-14 | CNV_3982, CNV_8807, CNV_7087 |
| 48 | 16 | p13.13 | 11173868-11178626 | -1.419855 | 4.38E-16 | CLEC16A |
| 49 | 16 | q24.1 | 85302753-85306926 | -0.995341 | 1.46E-12 | CNV_49791, CNV_58781, CNV_67070... |
| 50 | 16 | q24.2 | 86529114-86536801 | -1.053071 | 4.11E-11 | CNV_3134, CNV_30795 |
| 51 | 17 | q21.31 | 37885447-37885501 | -0.74336 | 2.82E-12 | ATP6VOA1 |
| 52 | 17 | q23.2 | 56404749-56407334 | -1.054468 | 7.56E-13 | BCAS3, CNV_4410, CNV_49891... |
| 53 | 17 | q25.2 | 72541570-72547858 | -1.107359 | 5.34E-17 | CNV_5336, CNV_53066, CNV_34522... |
| 54 | 17 | q25.3 | 75476251-75483572 | -0.90787 | 3.74E-13 | |
| 55 | 19 | p12 | 21094293-21098244 | -2.544831 | 6.72E-25 | ZNF714, CNV_78137, CNV_50112... |
| 56 | 19 | q13.11 | 39810209-39814923 | -1.255844 | 1.46E-16 | CNV_73367 |
| 57 | 19 | q13.31 | 48895798-48900793 | -0.799759 | 2.22E-11 | CNV_32261, CNV_47965, CNV_5106... |

(continued)

| AD0040_Set03 | | | | | | |
|---|---|---|---|---|---|---|
| No. | Chr | Cytoband | Position | Amp/Del | P-value | Gene Names, Annotations |
| 58 | 19 | q13.32 | 52729604-52729663 | -1.310343 | 1.75E-24 | ZNF541 |
| 59 | 20 | q13.33 | 61437907-61448929 | 0.973892 | 2.88E-15 | CHRNA4, CNV_31044 |
| 60 | 22 | q11.21 | 19712255-19715734 | -1.075087 | 7.15E-12 | P2RX6, SLC7A4, CNV_31071... |
| 61 | 22 | q13.32 | 47558995-47566106 | -0.956944 | 6.21E-12 | CNV_4134, CNV_50883 |
| 62 | 22 | q13.33 | 50695995-50697227 | -1.147529 | 2.70E-13 | CNV_30166 |
| 63 | X | p22.33 | 155819-169113 | -1.217427 | 1.36E-46 | PLCXD1, GTPBP6, CNV_83235... |
| 64 | X | p22.33 | 189104-190572 | -0.996498 | 7.72E-11 | CNV_67918 |
| 65 | X | p22.33 | 699908-706191 | -0.791549 | 1.88E-13 | CNV_34411 |
| 66 | X | p22.33 | 1562369-1566850 | -1.112982 | 2.22E-22 | P2RY8 |
| 67 | X | p22.33 | 1637614-1639274 | -0.69266 | 3.10E-11 | |
| 68 | X | p22.33 | 2646756-2647777 | -1.242813 | 1.07E-17 | CD99, CNV_4142, CNV_8292... |
| 69 | Y | p11.32 | 105819-119113 | -1.217427 | 1.36E-46 | CNV_83894, CNV_97143 |
| 70 | Y | p11.32 | 139104-140572 | -0.996498 | 7.72E-11 | PLCXD1 |
| 71 | Y | p11.32 | 649908-656191 | -0.791549 | 1.93E-13 | |
| 72 | Y | p11.32 | 1512369-1516850 | -1.112982 | 2.30E-22 | ASMTL |
| 73 | Y | p11.32 | 1587614-1589274 | -0.69266 | 3.18E-11 | P2RY8 |
| 74 | Y | p11.31 | 2596756-2597777 | -1.242813 | 1.10E-17 | |
| AD0040_Set04 | | | | | | |
| No. | Chr | Cytoband | Position | Amp/Del | P-value | Gene Names, Annotations |
| 1 | 2 | p25.2 | 6148711-6875000 | -0.564855 | 5.96E-120 | CNV_4274, CNV_35845, CNV_9920... |
| 2 | 3 | p21.31 | 50358198-50366080 | -0.859205 | 9.66E-11 | TUSC4, CYB561D2, CNV_3429... |
| 3 | 3 | p21.1 | 51937265-51945028 | -0.591105 | 1.32E-10 | RRP9 |
| 4 | 4 | p16.1 | 8575302-8575359 | -1.15809 | 1.02E-18 | CNV_3479 |
| 5 | 4 | p16.1 | 8882456-8882653 | -1.362018 | 6.33E-21 | CNV_3479, CNV_2497, CNV_0347... |
| 6 | 6 | p25.3 - p11.2 | 167917-58197184 | 0.17055 | 0 | DUSP22, IRF4, EXOC2... |
| 7 | 6 | p22.1 - p21.33 | 29854870-29902314 | -0.571876 | 8.20E-20 | HCG4, CNV_64460, CNV_64462... |

(continued)

| No. | Chr | Cytoband | Position | Amp/Del | P-value | Gene Names, Annotations |
|---|---|---|---|---|---|---|
| | | | AD0040_Set04 | | | |
| 8 | 6 | q11.1 - q27 | 62023384-170890108 | 0.151534 | 0 | KHDRBS2, LGSN, PTP4A1... |
| 9 | 7 | p22.3 | 1976966-1981109 | -1.268264 | 5.89E-12 | MAD1L1, CNV_4523, CNV_30253... |
| 10 | 7 | p21.3 - p21.2 | 13055490-13506713 | -0.56786 | 2.42E-78 | CNV_52086, CNV_1723, CNV_94383... |
| 11 | 7 | q11.23 | 72831668-72832641 | -1.0686 | 3.47E-10 | CNV_3685 |
| 12 | 8 | p12 | 37827143-37827202 | -0.769092 | 3.36E-12 | |
| 13 | 8 | q24.21 | 129012024-129012764 | -1.791079 | 2.12E-24 | PVT1, CNV_37296 |
| 14 | 8 | q24.3 | 142383673-142390195 | -1.229718 | 2.62E-12 | CNV_30288 |
| 15 | 10 | q26.3 | 134978689-134993118 | -0.606783 | 1.34E-10 | CALY, CNV_3829, CNV_4721... |
| 16 | 11 | p15.5 | 1114014-1115396 | -1.077322 | 7.79E-12 | CNV_3831, CNV_29887 |
| 17 | 11 | p13 | 35269915-35269974 | -1.947078 | 2.67E-30 | SLC1A2 |
| 18 | 11 | q13.2 | 68845113-68849973 | -1.00041 | 5.40E-10 | CNV_29915 |
| 19 | 11 | q13.3 | 69357011-69478523 | -0.261822 | 1.53E-11 | CNV_5631, CNV_4755, CNV_85835 |
| 20 | 11 | q13.5 | 75055163-75056846 | -1.196246 | 3.98E-15 | MAP6 |
| 21 | 11 | q23.2 | 114474724-114494671 | -1.007421 | 1.10E-17 | CNV_3867, CNV_4763, CNV_30567... |
| 22 | 13 | q12.11 | 19566409-19568792 | -1.034896 | 2.93E-13 | CNV_71680, |
| 23 | 13 | q14.2 - q34 | 48225461-115105297 | 0.370811 | 0 | FNDC3A, MLNR, CDADC1... |
| 24 | 13 | q34 | 114743988-114747979 | -0.528156 | 1.97E-10 | CNV_29947 |
| 25 | 13 | q34 | 114769518-114788319 | -0.408921 | 2.71E-17 | CNV_29947, CNV_71818, CNV_101882... |
| 26 | 16 | q24.2 | 86530833-86536801 | -1.078159 | 1.87E-11 | CNV_3134, CNV_30795 |
| 27 | 17 | q25.3 | 75476251-75483572 | -0.9129 | 2.28E-12 | |
| 28 | 19 | p12 | 21094293-21098244 | -1.806828 | 8.86E-16 | ZNF714, CNV_78137, CNV_50112... |
| 29 | 20 | p12.3 - p11.1 | 8891768-26075841 | 0.388611 | 0 | PLCB4, C20orf103, PAK7... |
| 30 | 20 | q11.21 - q13.33 | 29844444-62949149 | 0.408412 | 0 | TPX2, MYLK2, FOXS1... |

(continued)

**AD0040_Set04**

| No. | Chr | Cytoband | Position | Amp/Del | P-value | Gene Names, Annotations |
|---|---|---|---|---|---|---|
| 31 | 22 | q11.21 | 19712255-19715734 | -1.186638 | 5.68E-14 | P2RX6, SLC7A4, CNV_31071... |
| 32 | 22 | q11.21 | 20125513-20144135 | -0.844823 | 3.83E-16 | HIC2, CNV_31071, CNV_4117... |
| 33 | 22 | q13.32 | 47558995-47566106 | -0.695915 | 2.91E-10 | CNV_4134, CNV_50883 |
| 34 | X | p22.33 | 155819-164781 | -1.341537 | 2.66E-46 | PLCXD1, GTPBP6, CNV_83235... |
| 35 | X | p22.33 | 187113-190572 | -1.174457 | 2.36E-26 | CNV_67918 |
| 36 | X | p22.33 | 303009-314555 | -0.483352 | 2.29E-11 | CNV_73888 |
| 37 | X | p22.33 | 1471240-1472998 | -1.153505 | 1.48E-15 | CNV_73906 |
| 38 | X | p22.33 | 1562369-1566850 | -1.120176 | 1.44E-19 | P2RY8 |
| 39 | Y | p11.32 | 105819-114781 | -1.341537 | 2.66E-46 | CNV_83894, CNV_97143 |
| 40 | Y | p11.32 | 137113-140572 | -1.174457 | 2.36E-26 | PLCXD1 |
| 41 | Y | p11.32 | 253009-264555 | -0.483352 | 2.29E-11 | PPP2R3B |
| 42 | Y | p11.32 | 1421240-1422998 | -1.153505 | 1.48E-15 | IL3RA |
| 43 | Y p11.32 | | 1512369-1516850 | -1.120176 | 1.44E-19 | ASMTL |

**AD0040_Set05**

| No. | Chr | Cytoband | Position | Amp/Del | P-value | Gene Names, Annotations |
|---|---|---|---|---|---|---|
| 1 | 1 | p36.33 - p11.1 | 759762-121329506 | -0.311631 | 0 | LOC643837, FAM41C, FLJ39609... |
| 2 | 1 | p36.22 | 11506047-11510410 | -1.623647 | 1.52E-15 | PTCHD2 |
| 3 | 1 | p34.3 | 34590539-34590598 | -1.185761 | 1.16E-12 | CNV_29576, CNV_29577 |
| 4 | 2 | p25.2 | 6148711-6875000 | -1.046482 | 0 | CNV_4274, CNV_35845, CNV_9920... |
| 5 | 2 | p21 | 44083711-44100016 | -0.962256 | 9.63E-17 | CNV_78526, CNV_89620, CNV_73443... |
| 6 | 2 | q37.3 | 237339565-237344964 | -1.12891 | 5.17E-11 | |
| 7 | 3 | p21.31 | 44941989-44944920 | -1.10293 | 2.01E-12 | ZDHHC3 |
| 8 | 3 | p21.1 | 51941606-51941665 | -1.570327 | 5.43E-42 | |
| 9 | 3 | p21.1 | 52157853-52166715 | -0.764702 | 1.40E-11 | WDR51A, CNV_51113 |
| 10 | 3 | p14.3 | 55514963-55520108 | -0.807534 | 3.32E-11 | ERC2, CNV_3430 |
| 11 | 4 | p16.1 | 8575302-8575359 | -1.596547 | 6.08E-27 | CNV_3479 |

(continued)

| AD0040_Set05 | | | | | | |
|---|---|---|---|---|---|---|
| No. | Chr | Cytoband | Position | Amp/Del | P-value | Gene Names, Annotations |
| 12 | 4 | p16.1 | 8881212-8885187 | -0.836567 | 6.94E-15 | CNV_3479, CNV_2497, CNV_0347 |
| 13 | 4 | q13.1 | 64932715-64958903 | -1.212414 | 2.11E-10 | SRD5A2L2 |
| 14 | 4 | q25 | 108607270-108770678 | 0.347478 | 7.39E-35 | PAPSS1 |
| 15 | 4 | q31.21 | 143422425-143437437 | -0.798176 | 1.20E-12 | INPP4B |
| 16 | 4 | q35.1 | 186948059-186972601 | -0.780162 | 7.04E-11 | SORBS2, CNV_53588, CNV_68870 |
| 17 | 6 | p25.3 | 1603954-1615979 | -0.826792 | 2.96E-11 | GMDS |
| 18 | 6 | p22.1 - p21.33 | 29854870-29917547 | -1.780237 | 1.89E-72 | HCG4, HLA-G, CNV_64460... |
| 19 | 6 | p21.32 | 32605385-32631881 | -0.849512 | 1.39E-20 | HLA-DRB5, HLA-DRB6, CNV_3603... |
| 20 | 6 | p21.2 | 37661196-37665381 | -1.211464 | 4.15E-14 | CNV_8512 |
| 21 | 6 | p12.3 | 45968671-45975445 | -0.688034 | 2.69E-12 | CLIC5, CNV_0078 |
| 22 | 6 | p12.1 | 53929240-53934834 | -3.201252 | 1.28E-18 | CNV_3614, CNV_31288, CNV_8516... |
| 23 | 6 | q16.1 | 95408458-95417756 | -0.921046 | 1.17E-12 | CNV_52028, CNV_34592, CNV_52029 |
| 24 | 6 | q16.3 | 103910750-103946150 | -2.99956 | 1.93E-25 | CNV_53366, CNV_99645, CNV_99646 |
| 25 | 6 | q25.3 | 159115154-159119516 | -1.197527 | 1.06E-12 | EZR |
| 26 | 6 | q27 | 166262779-166267277 | -1.136934 | 1.12E-12 | C6orf176, CNV_3652 |
| 27 | 7 | p22.1 | 5770846-5779002 | -0.826805 | 2.09E-10 | RNF216, CNV_53516 |
| 28 | 7 | p21.3 - p21.2 | 13055490-13506713 | -0.968181 | 1.09E-192 | CNV_52086, CNV_1723, CNV_94383... |
| 29 | 7 | p11.2 | 55538137-55543418 | -1.237556 | 4.78E-11 | ECOP |
| 30 | 7 | q22.1 | 100239082-100247277 | -0.804057 | 3.13E-11 | EPHB4, CNV_4550 |
| 31 | 7 | q36.1 | 151531289-151531319 | -1.121547 | 7.31E-12 | MLL3 |
| 32 | 8 | p21.3 | 20951820-20964831 | -1.007687 | 3.40E-11 | CNV_3726, CNV_82520, CNV_95311... |
| 33 | 8 | p21.3 | 22263358-22269438 | -0.89917 | 3.41E-10 | PIWIL2, CNV_3726, CNV_2746 |
| 34 | 8 | p12 | 37827143-37827202 | -1.36743 | 2.58E-26 | |

(continued)

| | | | | | | |
|---|---|---|---|---|---|---|
| AD0040_Set05 | | | | | | |
| No. | Chr | Cytoband | Position | Amp/Del | P-value | Gene Names, Annotations |
| 35 | 8 | q24.21 | 129012024-129012764 | -1.861445 | 2.64E-25 | PVT1, CNV_37296 |
| 36 | 9 | q34.12 | 132642863-132652875 | -0.67207 | 2.11E-11 | ABL1 |
| 37 | 9 | q34.13 | 134879638-134884316 | -1.056401 | 2.75E-10 | |
| 38 | 9 | q34.3 | 137332375-137332434 | -0.900988 | 2.47E-14 | CNV_30337, CNV_4660 |
| 39 | 10 | p12.31 | 21459641-21463968 | -1.099716 | 5.33E-13 | NEBL, C10orf113 |
| 40 | 10 | q24.33 | 105011210-105018167 | -0.808046 | 1.54E-10 | |
| 41 | 10 | q26.3 | 132819421-132829669 | -0.947973 | 6.81E-11 | TCERG1L |
| 42 | 10 | q26.3 | 134987375-134991871 | -1.053537 | 6.23E-12 | CALY, CNV_3829, CNV_4721... |
| 43 | 10 | q26.3 | 135281682-135287473 | -1.2869 | 1.47E-12 | CNV_2896, CNV_8673, CNV_8671... |
| 44 | 11 | p15.5 | 417922-438827 | -0.538444 | 3.01E-11 | ANO9, CNV_29880, CNV_29882... |
| 45 | 11 | p15.5 | 1962010-1967283 | -1.137617 | 8.69E-14 | LOC100133545, CNV_29893, CNV_37117... |
| 46 | 11 | p13 | 35269915-35269974 | -2.362248 | 1.00E-32 | SLC1A2 |
| 47 | 11 | p11.2 | 45446292-45455071 | -1.35674 | 1.76E-15 | |
| 48 | 11 | p11.2 | 45536937-45547269 | -1.261453 | 1.54E-16 | |
| 49 | 11 | q13.1 | 64373699-64389963 | -0.539648 | 4.61E-11 | EHD1, CNV_5422, CNV_4752... |
| 50 | 11 | q13.2 | 68845113-68855981 | -0.686771 | 1.60E-11 | CNV_29915 |
| 51 | 11 | q13.5 | 75055163-75056846 | -1.181678 | 8.74E-15 | MAP6 |
| 52 | 11 | q14.1 | 79146889-79150365 | -0.988344 | 6.22E-11 | |
| 53 | 11 | q23.2 | 114474724-114494671 | -1.222935 | 1.46E-22 | CNV_3867, CNV_4763, CNV_30567... |
| 54 | 12 | p13.33 | 1603701-1609148 | -1.002602 | 4.34E-11 | WNT5B |
| 55 | 12 | p13.33 | 2459007-2462164 | -1.00103 | 1.59E-11 | CACNA1C |
| 56 | 12 | q13.13 | 50170516-50187346 | -0.683817 | 1.06E-10 | SLC4A8, CNV_86368 |
| 57 | 12 | q13.2 | 54376360-54377782 | -2.255378 | 1.44E-30 | ITGA7, CNV_3890 |
| 58 | 13 | q12.11 | 19566409-19568792 | -1.412693 | 2.83E-20 | CNV_71680, CNV_71679 |
| 59 | 13 | q12.3 | 30605647-30656414 | -0.695439 | 8.71E-16 | HSPH1 |
| 60 | 13 | q14.2 - q34 | 48225461-115105297 | 0.525393 | 0 | FNDC3A, MLNR, CDADC1... |
| 61 | 13 | q32.3 | 100080292-100084653 | -0.515989 | 5.93E-11 | TMTC4 |

(continued)

| AD0040_Set05 | | | | | | |
|---|---|---|---|---|---|---|
| No. | Chr | Cytoband | Position | Amp/Del | P-value | Gene Names, Annotations |
| 62 | 13 | q33.1 | 100621234-100625172 | -0.546582 | 6.26E-12 | NALCN |
| 63 | 13 | q34 | 112346947-112529339 | -0.110391 | 8.73E-16 | C13orf35, ATP11A, CNV_3926 |
| 64 | 13 | q34 | 112553940-112565338 | -0.317672 | 1.46E-10 | ATP11A |
| 65 | 13 | q34 | 114769518-114788319 | -0.256096 | 7.01E-17 | CNV_29947, CNV_71818, CNV_101882... |
| 66 | 13 | q34 | 114912404-114924113 | -0.175258 | 7.39E-11 | CNV_29948, CNV_71824, CNV_71823 |
| 67 | 14 | q32.31 | 100635039-100643492 | -0.954125 | 3.26E-10 | CNV_76722, CNV_87348 |
| 68 | 14 | q32.31 | 101132700-101136328 | -1.233816 | 8.99E-13 | CNV_47864, CNV_8776 |
| 69 | 14 | q32.31 | 101314727-101318356 | -0.770087 | 8.77E-13 | CNV_8776 |
| 70 | 15 | q26.3 | 100833003-100835108 | -1.990568 | 6.52E-26 | |
| 71 | 15 | q26.3 | 101555153-101558598 | -1.202154 | 1.99E-13 | CNV_3982, CNV_8807, CNV_7087 |
| 72 | 16 | q24.2 | 86529114-86536801 | -1.129622 | 2.27E-16 | CNV_3134, CNV_30795 |
| 73 | 17 | p13.3 | 2246758-2258130 | -0.70325 | 4.76E-12 | MNT, LOC284009, CNV_67107 |
| 74 | 17 | q25.2 | 72510034-72513509 | -1.558487 | 2.02E-19 | CNV_5336, CNV_53066, CNV_34522... |
| 75 | 17 | q25.2 | 72541570-72547858 | -1.030258 | 1.02E-15 | CNV_5336, CNV_53066, CNV_34522... |
| 76 | 17 | q25.3 | 74127687-74135747 | -0.797471 | 3.04E-11 | |
| 77 | 19 | p13.3 | 5652790-5656012 | -1.400095 | 3.53E-18 | LONP1 |
| 78 | 19 | p13.2 | 11589908-11592624 | -0.988635 | 1.65E-10 | ZNF627 |
| 79 | 19 | p12 | 21094293-21098244 | -2.160212 | 6.95E-20 | ZNF714, CNV_78137, CNV_50112... |
| 80 | 19 | q13.11 | 37739553-37743272 | -1.188304 | 2.87E-14 | CNV_78177, CNV_89217 |
| 81 | 19 | q13.11 | 39810209-39814923 | -1.252695 | 8.59E-15 | CNV_73367 |
| 82 | 19 | q13.32 | 52729604-52729663 | -1.123367 | 6.31E-20 | ZNF541 |
| 83 | 19 | q13.33 | 56185087-56190375 | -1.032553 | 3.03E-12 | |
| 84 | 20 | p12.3 - p11.1 | 8900134-26075841 | 0.575452 | 0 | PLCB4, C20orf103, PAK7... |

(continued)

| | AD0040_Set05 | | | | | |
|---|---|---|---|---|---|---|
| No. | Chr | Cytoband | Position | Amp/Del | P-value | Gene Names, Annotations |
| 85 | 20 | p11.21 | 23912869-23925414 | -0.344259 | 2.48E-13 | GGTLC1, CNV_5129 |
| 86 | 20 | q11.21 - q13.33 | 29652452-62911874 | 0.592922 | 0 | ID1, COX4I2, BCL2L1... |
| 87 | 20 | q11.23 | 34796540-34803426 | -0.243389 | 3.26E-11 | NDRG3 |
| 88 | 20 | q13.32 | 57462934-57470482 | -0.379787 | 4.92E-14 | CNV_67720 |
| 89 | 20 | q13.33 | 61290383-61294386 | -0.665497 | 9.88E-15 | CNV_5347, CNV_4106, CNV_5144 |
| 90 | 21 | q22.3 | 41510016-41514904 | -1.296857 | 8.53E-13 | BACE2 |
| 91 | 22 | q11.21 | 20125513-20147529 | -0.707834 | 1.98E-14 | HIC2, CNV_31071, CNV_4117... |
| 92 | 22 | q13.32 | 47558995-47566106 | -0.853665 | 1.76E-11 | CNV_4134, CNV_50883 |
| 93 | X | p22.33 | 155819-169113 | -1.326927 | 4.52E-52 | PLCXD1, GTPBP6, CNV_83235... |
| 94 | X | p22.33 | 187113-190572 | -1.064823 | 8.62E-18 | CNV_67918 |
| 95 | X | p22.33 | 699908-706191 | -1.17171 | 2.02E-17 | CNV_34411 |
| 96 | X | p22.33 | 1562369-1566850 | -1.091515 | 2.82E-15 | P2RY8 |
| 97 | X | p22.33 | 1820491-1831380 | -1.061682 | 1.00E-11 | CNV_67930, CNV_33161, CNV_4142 |
| 98 | X | p22.33 | 2194563-2201252 | -1.147887 | 5.04E-13 | DHRSX, CNV_4142 |
| 99 | X | p22.33 | 2309297-2310369 | -1.404613 | 2.43E-12 | DHRSX, CNV_4142 |
| 100 | X | p22.33 | 2646756-2647777 | -1.782833 | 8.15E-23 | CD99, CNV_4142, CNV_8292... |
| 101 | X | p22.13 | 17789072-17792098 | -1.216957 | 1.03E-14 | RAI2, CNV_67948 |
| 102 | X | q26.2 | 130912192-130913849 | -1.27548 | 7.72E-16 | |
| 103 | Y | p11.32 | 105819-119113 | -1.326927 | 8.24E-56 | CNV_83894, CNV_97143 |
| 104 | Y | p11.32 | 137113-140572 | -1.064823 | 1.51E-16 | PLCXD1 |
| 105 | Y | p11.32 | 649908-656191 | -1.17171 | 2.56E-16 | |
| 106 | Y | p11.32 | 1512369-1516850 | -1.091515 | 2.99E-14 | ASMTL |
| 107 | Y | p11.31 | 1770491-1781380 | -1.061682 | 6.06E-11 | CNV_33187 |
| 108 | Y | p11.31 | 2144563-2151252 | -1.147887 | 3.28E-12 | DHRSX, CNV_83906, CNV_83907... |
| 109 | Y | p11.31 | 2259297-2260369 | -1.404613 | 1.02E-11 | DHRSX |
| 110 | Y | p11.31 | 2596756-2597777 | -1.782833 | 7.51E-22 | |
| Amp = Amplification Del = Deletion | | | | | | |

The genomics analysis results revealed that any of the following cells: CC3_6 generated by the procedure described in Example 2 (corresponding to AD0040_17 tested in Set01), GC1_9 generated by the procedure described in Example 4 (corresponding to AD0040_22 tested in Set02), CC1_17 generated by the procedure described in Example 6 (corresponding to AD0040_06 tested in Set03), and CC4-D generated by the procedure described in Example 8 (corresponding to AD0040_20 tested in Set05), had an aberration of gene copy numbers of endogenous genomic DNA.

The aberration of copy number variations (CNVs) detected by the procedure described in this Example can be determined to be an aberration of CNVs in induced malignant stem cells when they are different from those CNVs in the genomic DNAs of the non-cancer tissue cells. The induced malignant stem cells analyzed in this Example can be described as cells characterized both by an aberration of gene copy number variations (CNVs) of endogenous genomic DNA and by expression of the ES cell-specific genes (OCT3/4, NANOG, SOX2, ZFP42).

Example 12: Detection for an aberration of microsatellites of endogenous genomic DNA in induced malignant stem cells

In this Example, (1) (i) instability occurring in endogenous genomic DNA microsatellites in induced malignant stem cells was generated, in comparison with cell populations derived from non-cancer site tissues or cell populations derived from normal tissues.

(12-1) Materials

The microsatellite instability (MSI) testing was performed by the following MSI analysis procedures: the tumor sites and induced malignant stem cells of the same patient (donor) as well as the non-tumor sites and normal tissues of the same patient were subjected to extraction of DNAs, which were PCR amplified using the primers fluorescently labeled for the five Bethesda markers (BAT25, BAT26, D2S123, D5S346, D17S250) recommended to be used in MSI analyses (Boland CR, et al., Cancer Res. 58: 5248-57, 1998; Loukola, A, et al., Cancer Res., 1 Jun 2001, 61 (11): 4545-9) and were then subjected to capillary electrophoresis using a fluorescent DNA sequencer. After data analysis was performed by the Gene Mapper software, the presence or absence of change in the number of repetitions between the tumor sites and non-tumor sites was determined for each marker based on the difference in waveform pattern, and general determination was made based on the determination results for the five markers.

The following samples were used in the microsatellite instability analysis:

- cell population (ngc3) derived from colon non-cancer site tissues, and induced malignant stem cells (CC3_5, CC3_6) prepared from fresh colon cancer tissues, which were collected from the individual of donor No. 2;
- cell population (ngc1) derived from fresh gastric non-cancer site tissues, induced non-malignant stem cells (NGC1_6) prepared from fresh gastric non-cancer site tissues, and induced malignant stem cells (GC1_6, GC1_9, GC1_10) prepared from fresh gastric cancer tissues, which were collected from the individual of donor No. 3;
- cell population (ncc1) derived from colon non-cancer site tissues, cell population (cc1) derived from fresh colon cancer site tissues, and induced malignant stem cells (CC1_1, CC1_2, CC1_7, CC1_8, CC1_9, CC1_11, C1_12, CC1_17, CC1_18) prepared from fresh colon cancer tissues, which were collected from the individual of donor No. 4;
- cell population (ncc4) derived from colon non-cancer site tissues, cell population (cc4) derived from fresh colon cancer site tissues, and induced malignant stem cells (CC4_c, CC4_(3), CC4_(6), CC4_(3)_10, CC4_(4), CC4_30, CC4-10, CC4-31, CC4(1), CC4(2), CC4-D) prepared from fresh colon cancer tissues, which were collected from the individual of donor No. 5;
- fibroblasts (7F3956) collected from the individual of donor No. 6, and induced pluripotent stem cells (NFB1_2) prepared from the same; and
- fibroblasts (7F3949) collected from the individual of donor No. 7, and induced pluripotent stem cells (NFB2_17) prepared from the same fibroblasts.

(12-2) Analysis procedure

(12-2-1) DNA extraction

The samples obtained from the donors mentioned in (12-1) were subjected to extraction of genomic DNAs using DNeasy Blood & Tissue Kit (50) (QIAGEN; Cat No. 69504) following the instructions of the manufacturer. The obtained genomic DNAs were so prepared as to give a concentration of 10 ng/$\mu$L.

(12-2-2) PCR

The thus-prepared genomic DNAs, which were used as a template, were subjected to PCR using the primer sets for the respective five Bethesda markers. For each sample, the mixes shown below were prepared in a PCR plate or 8-strip tube. However, a sample that contained the positive control (Genomic DNA MCF-7; Funakoshi) instead of the sample genomic DNAs, and a sample that did not contain genomic DNAs serving as a template but contained the negative control (TE Buffer, 1X) were also included in each analysis.

[Table 23]

| Table 23: Formulations of PCR solutions | | |
|---|---|---|
| | Frozen tissues / Whole blood | Formalin-fixed tissues / Formalin-fixed paraffin-embedded (FFPE) tissues |
| Sample genomic DNA (10 ng/$\mu$L) | 2.5 $\mu$L | 1.0 $\mu$L |
| 10X PCR buffer | 2.5 $\mu$L | 2.5 $\mu$L |
| dNTP (2 mM each) | 2.5 $\mu$L | 2.5 $\mu$L |
| MgCl$_2$ (25 mM) | 1.5 $\mu$L | 1.5 $\mu$L |
| Fluorescently labeled forward primer (20$\mu$M) | 0.5 $\mu$L | 0.5 $\mu$L |
| Reverse primer (20$\mu$M) | 0.5 $\mu$L | 0.5 $\mu$L |
| AmpliTaq Gold (5U/$\mu$L) | 0.125 $\mu$L | 0.125 $\mu$L |
| dH$_2$O | 14.875 $\mu$L | 16.375 $\mu$L |
| Total | 25.0 $\mu$L | 25.0 $\mu$L |

The primers used in this analysis had the following nucleotide sequences:

- BAT25 marker:

    Forward primer: tcgcctccaagaatgtaagt (SEQ ID NO: 1)
    Reverse primer: tctggattttaactatggctc (SEQ ID NO: 2)

- BAT26 marker:

    Forward primer: tgactactttgacttcagcc (SEQ ID NO: 3)
    Reverse primer: aaccattcaacattttaacc (SEQ ID NO: 4)

- D2S123 marker:

    Forward primer: aaacaggatgcctgccttta (SEQ ID NO: 5)
    Reverse primer: ggactttccacctatgggac (SEQ ID NO: 6)

- D5S346 marker:

    Forward primer: actcactctagtgataaatcggg (SEQ ID NO: 7)
    Reverse primer: agcagataagacaagtattactag (SEQ ID NO: 8)

- D17S250 marker:

    Forward primer: ggaagaatcaaatagacaat (SEQ ID NO: 9)
    Reverse primer: gctggccatatatatatttaaacc (SEQ ID NO: 10).

After dispensing all of the constitutional components, the PCR plate or 8-strip tube was capped and subjected to vortexing, which was followed by spinning down the solution.

Then, with the heat block of a thermal cycler (Peltier Thermal Cycler (PTC-220) (MJ Research), GeneAmp PCR System (9700) (Applied Biosystems), or Veriti Thermal Cycler (Applied Biosystems) having been heated at 94°C, the PCR plate or 8-strip tube was mounted on the preheated heat block to effect PCR on the conditions shown below.

[Table 24]

| Table 24: PCR conditions | |
|---|---|
| Type of analysis materials | |
| Frozen tissues / Whole blood (35 cycles) | Formalin-fixed tissues / Formalin-fixed paraffin-embedded (FFPE) tissues (45 cycles) |
| * Advanced mode-MSI-MSI | * Advanced mode-MSI-MSI45 |
| 1. Incubate at 95.0°C for 10 minutes. | 1. Incubate at 95.0°C for 10 minutes. |
| 2. Incubate at 95.0°C for 45 seconds. | 2. Incubate at 95.0°C for 45 seconds. |
| 3. Incubate at 57.0°C for 45 seconds. | 3. Incubate at 57.0°C for 45 seconds. |
| 4. Incubate at 72.0°C for 1 minute. | 4. Incubate at 72.0°C for 1 minute. |
| 5. Repeat steps 2 to 4 thirty-five times. | 5. Repeat steps 2 to 4 forty-five times. |
| 6. Incubate at 72.0°C for 10 minutes. | 6. Incubate at 72.0°C for 10 minutes. |
| 7. Store at 4°C to 10°C. | 7. Store at 4°C to 10°C. |

After the completion of the PCR, the PCR plate or 8-strip tube was removed from the heat block and subjected to vortexing, which was followed by spinning down the reaction solution. The concentration of the PCR product was determined by absorbance measurement. If necessary, the PCR product was appropriately diluted with 1X TE Buffer. The thus-prepared samples were used for analysis by capillary electrophoresis.

(12-2-3) Capillary electrophoresis

The PCR product prepared in (12-2-2) was separated by capillary electrophoresis, and the lengths of respective amplified DNAs were determined for each sample.

Ten microliters of Hi-Di Formamide (+ROX) was dispensed in a 96-well plate for capillary electrophoresis by the number of samples required. The 96-well plate for capillary electrophoresis was loaded with 1 μL/well of the PCR product, covered with a dedicated lid, and subjected to vortexing, which was followed by spinning down the sample solution.

The capillary electrophoresis, which was performed using Genetic Analyzer (3100) or Genetic Analyzer (3130x1) (each manufactured by Applied Biosystems), detected forward-primer-derived fluorophores (refer to Table 25 below) and thereby determined the lengths of the intended PCR products.

[Table 25]

Table 25: Characteristics of Bethesda markers

| Name | Fluorophore | Product size | Sequential structure |
|---|---|---|---|
| BAT25 | HEX | 120 bp | Monobase (A) repetitions |
| BAT26 | NED | 116 bp | Monobase (A) repetitions |
| D2S123 | NED | 197-227 | Dibase (CA) repetitions |
| D5S346 | FAM | 96-122 bp | Dibase (CA) repetitions |
| D17S250 | FAM | 141-169 bp | Dibase (CA) repetitions |

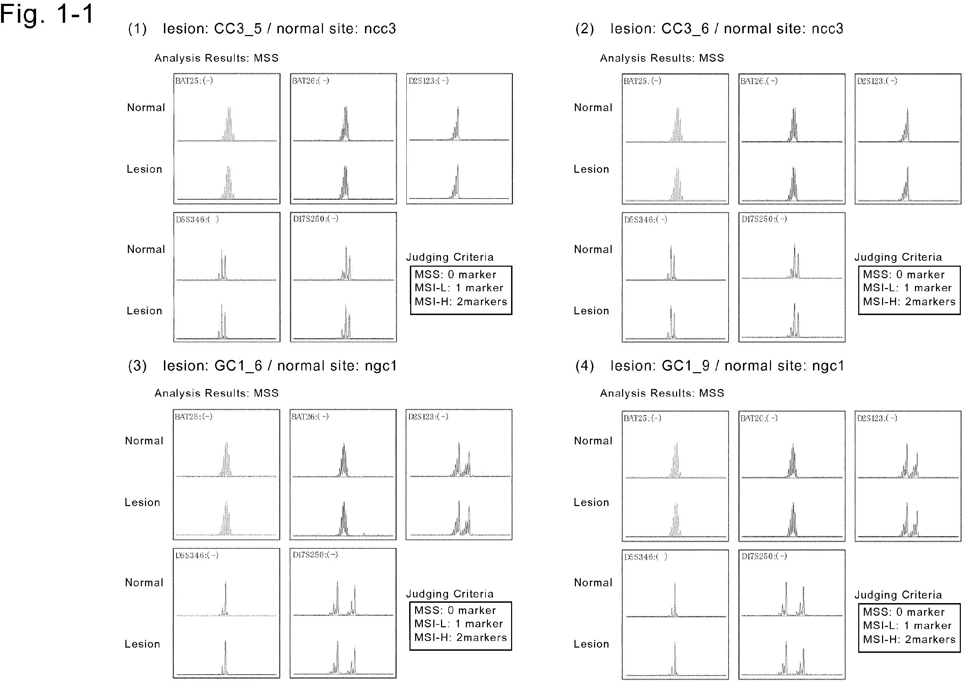

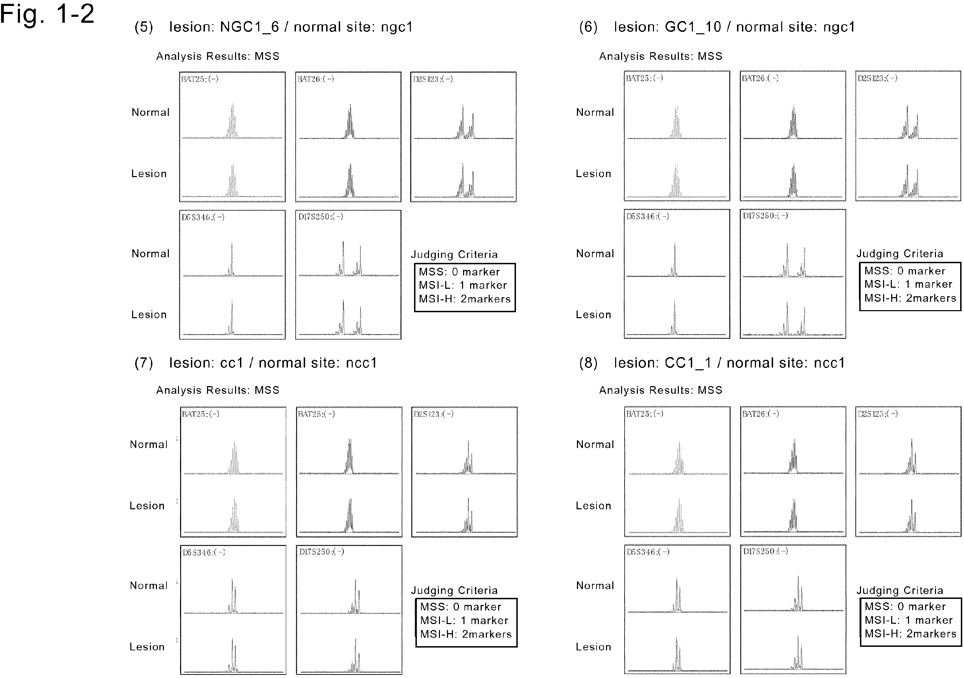

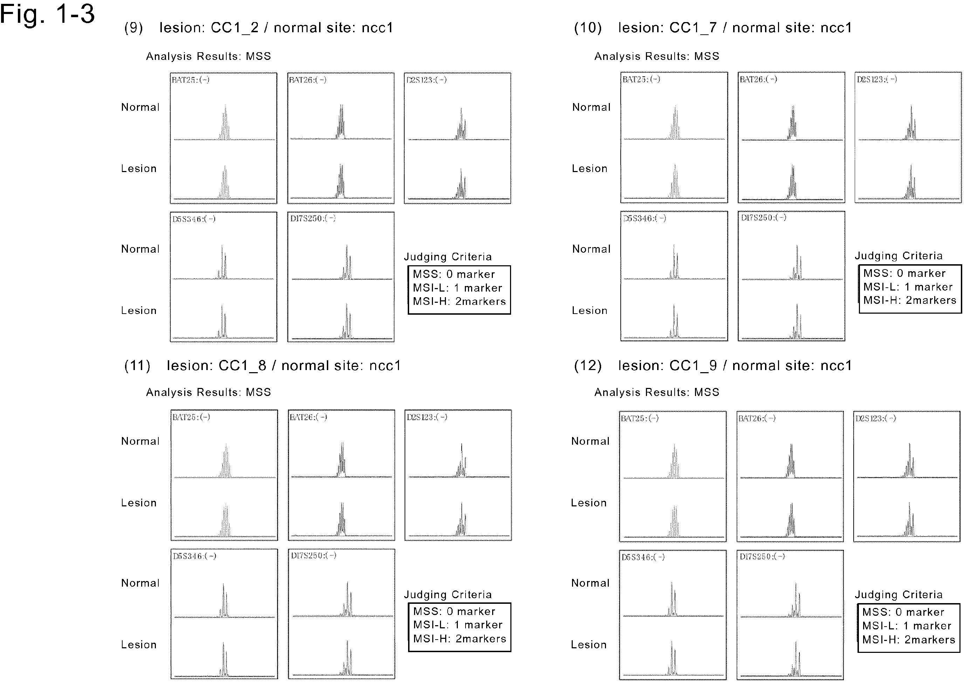

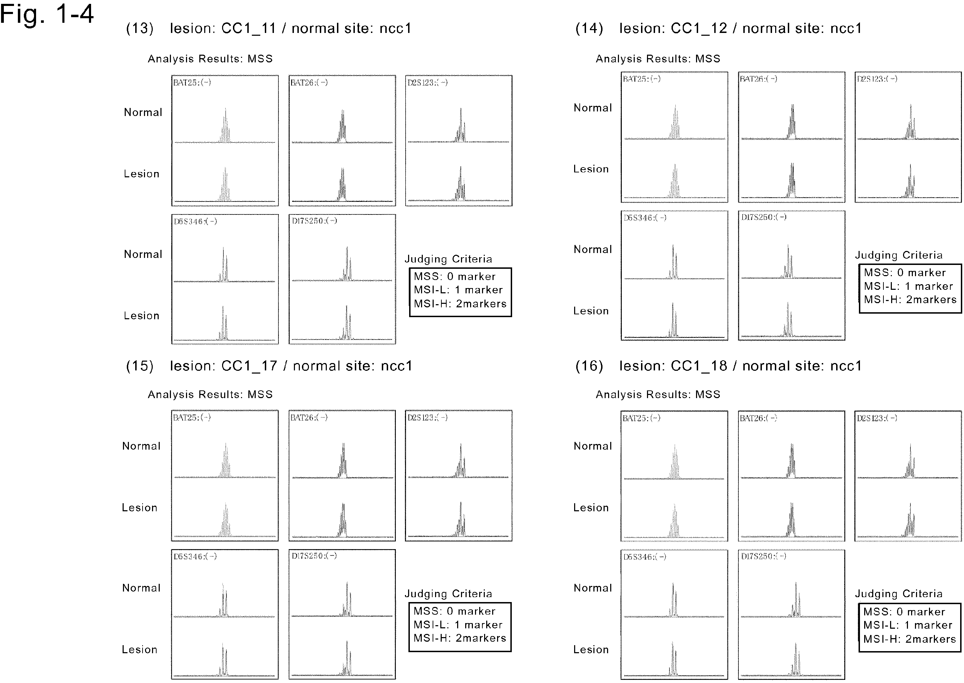

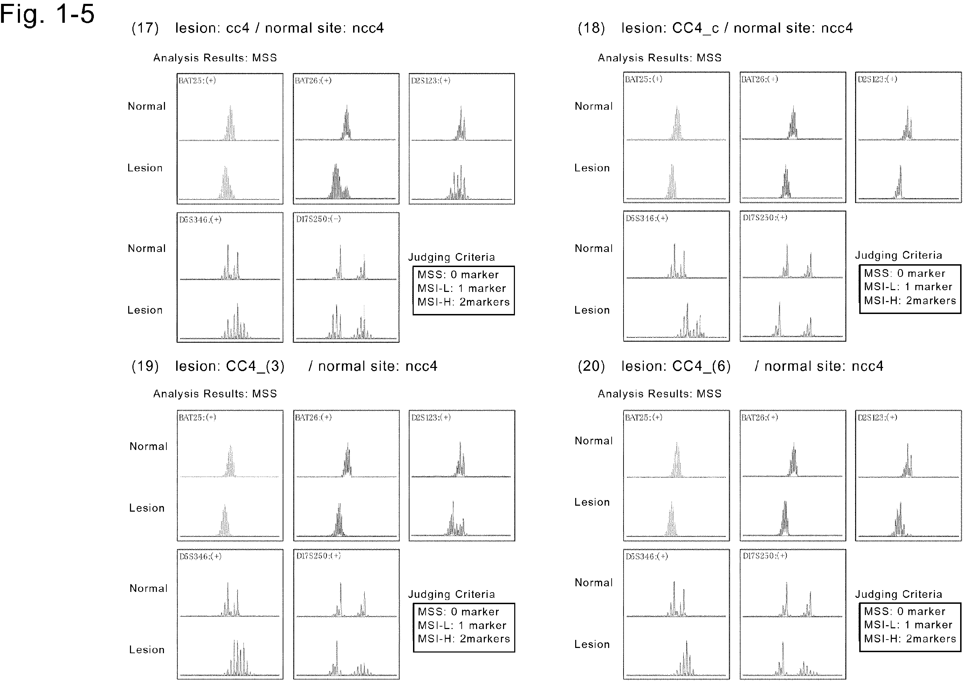

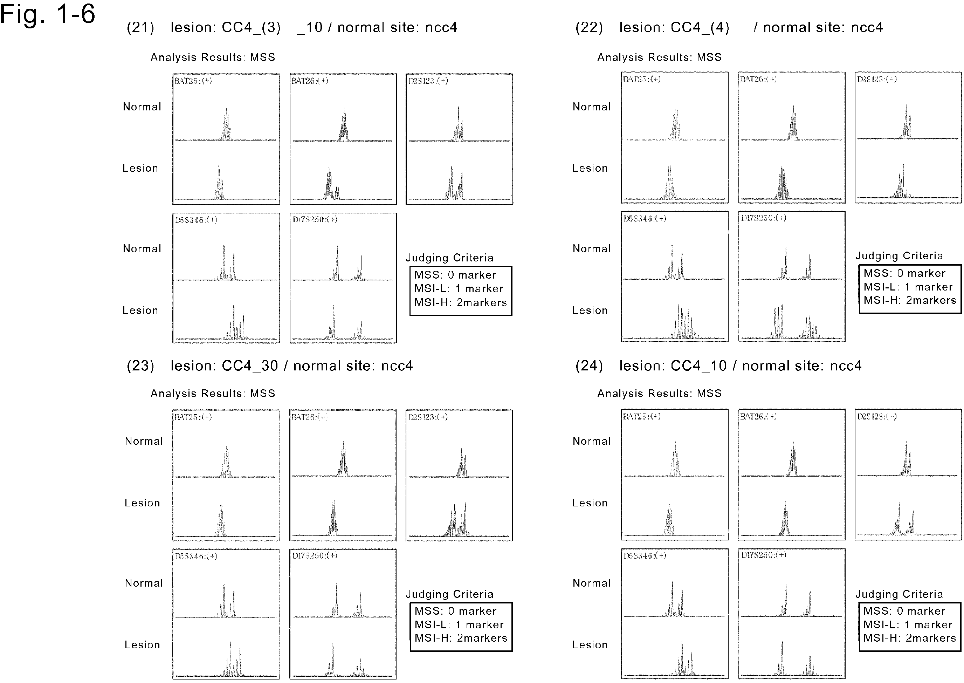

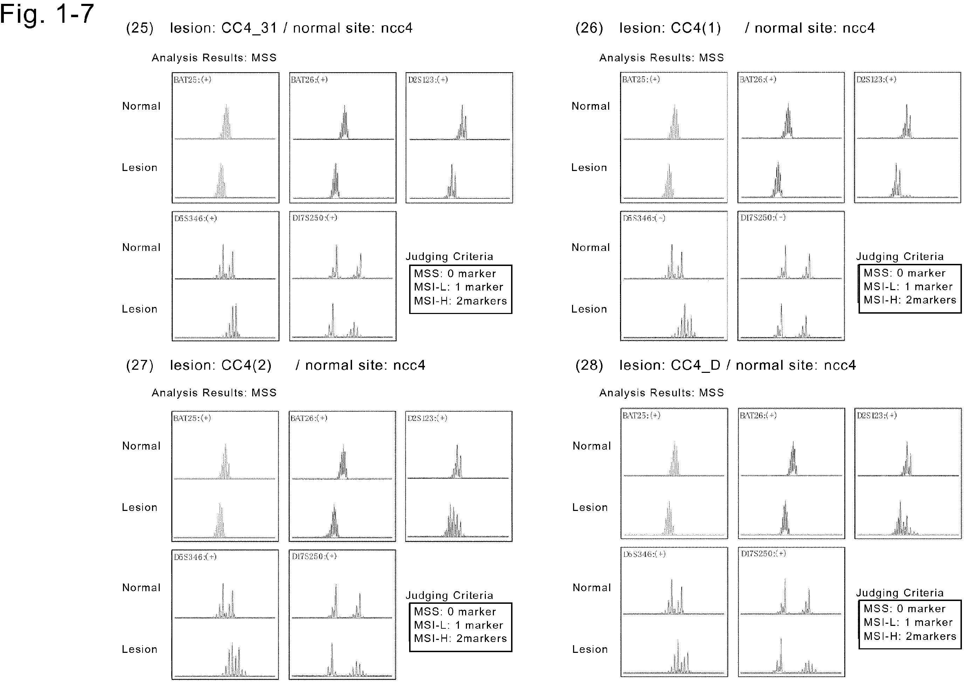

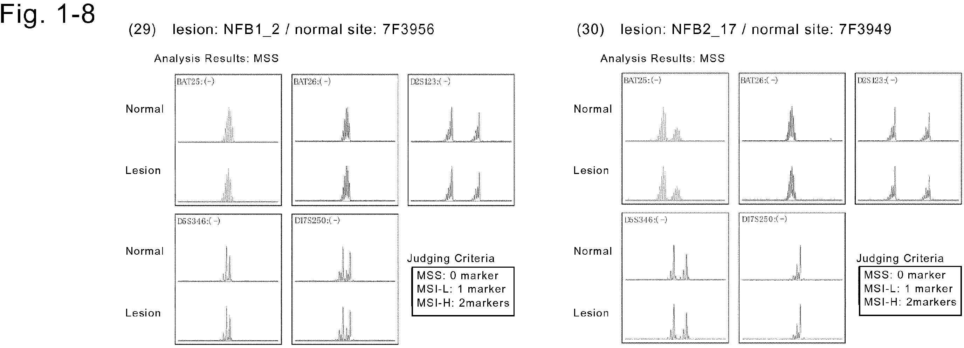

The obtained results are shown in Fig. 1. The sets of compared samples shown in the respective graphs are summarized in Table 26 below.

[Table 26]

| Table 26: Aberration of microsatellites | | | | |
|---|---|---|---|---|
| Sample | Cell characteristics | Control sample | Fig. No. | Results |
| Donor No. 2 | | | | |
| CC3_5 | Induced malignant stem cells | ncc3 | (1) | MSS |
| CC3_6 | Induced malignant stem cells | ncc3 | (2) | MSS |
| Donor No. 3 | | | | |
| GC1_6 | Induced malignant stem cells | ngcl | (3) | MSS |
| GC1_9 | Induced malignant stem cells | ngcl | (4) | MSS |
| NGC1_6 | Induced non-malignant stem cells | ngcl | (5) | MSS |
| GC1_10 | Induced malignant stem cells | ngcl | (6) | MSS |
| Donor No. 4 | | | | |
| cc1 | Colon cancer tissues | ncc1 | (7) | MSS |
| CC1_1 | Induced malignant stem cells | ncc1 | (8) | MSS |
| CC1_2 | Induced malignant stem cells | ncc1 | (9) | MSS |
| CC1_7 | Induced malignant stem cells | ncc1 | (10) | MSS |
| CC1_8 | Induced malignant stem cells | ncc1 | (11) | MSS |
| CC1_9 | Induced malignant stem cells | ncc1 | (12) | MSS |
| CC1_11 | Induced malignant stem cells | ncc1 | (13) | MSS |
| CC1_12 | Induced malignant stem cells | ncc1 | (14) | MSS |
| CC1_17 | Induced malignant stem cells | ncc1 | (15) | MSS |
| CC1_18 | Induced malignant stem cells | ncc1 | (16) | MSS |
| Donor No. 5 | | | | |
| cc4 | Colon cancer tissues | ncc4 | (17) | MSI-H |
| CC4_c | Induced malignant stem cells | ncc4 | (18) | MSI-H |
| CC4_(3) | Induced malignant stem cells | ncc4 | (19) | MSI-H |
| CC4_(6) | Induced malignant stem cells | ncc4 | (20) | MSI-H |
| CC4_(3)_10 | Induced malignant stem cells | ncc4 | (21) | MSI-H |
| CC4_(4) | Induced malignant stem cells | ncc4 | (22) | MSI-H |
| CC4_30 | Induced malignant stem cells | ncc4 | (23) | MSI-H |
| CC4-10 | Induced malignant stem cells | ncc4 | (24) | MSI-H |
| CC4-31 | Induced malignant stem cells | ncc4 | (25) | MSI-H |
| CC4 (1) | Induced malignant stem cells | ncc4 | (26) | MSI-H |
| CC4 (2) | Induced malignant stem cells | ncc4 | (27) | MSI-H |
| CC4-D | Induced malignant stem cells | ncc4 | (28) | MSI-H |
| Donor No. 6 | | | | |
| NFB1_2 | Induced pluripotent stem cells | 7F3956 | (29) | MSS |
| Donor No. 7 | | | | |
| NFB2_17 | Induced pluripotent stem cells | 7F3949 | (30) | MSS |

The induced malignant stem cells (CC4_c, CC4_(3), CC4_(6), CC4_(3)_10, CC4_(4), CC4_30, CC4-10, CC4-31, CC4 (1), CC4 (2), CC4-D) analyzed in this Example can be considered as cells characterized both by an aberration of microsatellites of endogenous genomic DNA and by expression of the ES cell-specific genes (OCT3/4, NANOG, SOX2, ZFP42).

Example 13: Detection for abnormal expression (mRNA) of endogenous gene in induced malignant stem cells

In this Example, (1) (c) abnormal expression (increased or reduced/lost expression) of endogenous oncogenes or endogenous tumor suppressor genes in induced malignant stem cells was detected, in comparison with those in induced pluripotent stem cells.

(13-1) Materials

The (1) (c) abnormal expression (increased or reduced/lost expression) of endogenous oncogenes or endogenous tumor suppressor genes in induced malignant stem cells was detected by genome-widely detecting mRNA expression of tyrosine kinases or cancer drug targets.
The following samples were used to detect (1) (c) abnormal expression (increased or reduced/lost expression) of endogenous oncogenes or endogenous tumor suppressor genes in induced malignant stem cells:

- induced malignant stem cells (GC2_1, GC2_5, GC2_10) prepared from fresh gastric cancer tissues collected from the individual of donor No. 1;
- induced malignant stem cells (CC1_1) prepared from fresh colon cancer tissues collected from the individual of donor No. 4;
- induced malignant stem cells (CC4_c, CC4-D) prepared from fresh colon cancer tissues collected from the individual of donor No. 5; and
- induced pluripotent stem cells (NFB2_17) prepared from the fibroblasts (7F3949) collected from the individual of donor No. 7.

(13-2) Summary

Total RNA was prepared from each of the eight induced malignant stem cells mentioned above, and testing was performed using the prepared total RNAs in Whole Human Genome Oligo Microarray Kit (4×44K) (Agilent).
Whole Human Genome Oligo Microarray Kit (4×44K) is a tool that enables a comprehensive expression analysis of transcripts encoded in the human genome. The probe sequences to be used in this kit were determined by checking the sequence information collected from multiple reliable databases against the human genome assembly. This kit also contains the probes designed for not only mRNAs but also non-coding RNAs such as snoRNA and pseudogene transcripts.
As labeled as "4×44K", this kit contains 4 microarrays on one glass slide, each of which contains about 44,000 (i.e, 44K) spots. Since this kit contains 4 microarrays, it is capable of testing 4 samples at the same time, enabling high-throughput analysis. It is also characterized by being capable of performing test using a minimum of 25 ng of total RNA per array.

(13-3) Quality evaluation

The process of quality evaluation of sample genomic DNAs involves the following procedures using sample RNA solutions in the respective apparatus:

- determination of electrophoresis patterns by the fluorescent detector Bioanalyzer (Agilent); and
- quantitation of total RNA amounts by the spectrophotometer NanoDrop (NanoDrop).

(13-4) Synthesis of complementary RNA (cRNA)

Complementary RNA (cRNA) was synthesized from the targeted RNA using Agilent Quick Amp Labeling Kit in accordance with the Agilent protocol. Double-stranded cDNA was synthesized from the total RNA (100 ng) prepared from each sample, and cRNA was synthesized from the prepared cDNA by *in vitro* transcription. In this process, cyanine-dye-labeled CTP (cyanine 3-CTP) was incorporated to effect fluorescent labeling.

(13-5) Hybridization

The cyanine-dye-labeled cRNA prepared in (13-4) was hybridized with Whole Human Genome Oligo Microarray (4×44K) using Agilent Gene Expression Hybridization Kit. To be specific, the labeled cRNA was added to a hybridization buffer and allowed to hybridize on Whole Human Genome Oligo Microarray (4×44K) for 17 hours. After washing, the DNA microarray image was scanned by Agilent Microarray Scanner, and fluorescence signals from the spots were digitized by Feature Extraction Software (v.10.7.3.1).

(13-6) Experimental results

As a result of the quality evaluation of the samples described in "(13-1) Materials", the quality of all the samples was assured both by the determination of electrophoresis patterns and by the quantitation of total RNA amounts.

Next, cRNA was synthesized using each of the obtained samples, and the amounts of fluorescently-labeled cRNAs were determined. As a result, it was confirmed that the cRNAs had been obtained in the amount required for hybridization with a microarray chip.

So, hybridization was performed with the microarray chip with the probes designed for the genes shown in the tyrosine kinase list (refer to Table 27 below) and the genes shown in the cancer drug targets list (refer to Table 28 below).

[Table 27]

| Table 27: List of tyrosine kinases investigated for abnormal expression | | | | |
|---|---|---|---|---|
| Unigene | GeneBank | Symbol | Description | Gene Name |
| Hs.431048 | NM_005157 | ABL1 | C-abl oncogene 1, non-receptor tyrosine kinase | ABL, JTK7, bcr, abl, c-ABL, p150, v-abl |
| Hs.159472 | NM_005158 | ABL2 | V-abl Abelson murine leukemia viral oncogene homolog 2 | ABLL, ARG, FLJ22224, FLJ31718, FLJ41441 |
| Hs.654469 | NM_004304 | ALK | Anaplastic lymphoma receptor tyrosine kinase | CD246, NBLST3 |
| Hs.590970 | NM_001699 | AXL | AXL receptor tyrosine kinase | JTK11, UFO |
| Hs.146591 | NM_001715 | BLK | B lymphoid tyrosine kinase | MGC10442, MODY11 |
| Hs.159494 | NM_000061 | BTK | Bruton agammaglobulinemia tyrosine kinase | AGMX1, AT, ATK, BPK, IMD1, MGC126261, MGC126262, PSCTK1, XLA |
| Hs.654394 | NM_005211 | CSF1R | Colony stimulating factor 1 receptor | C-FMS, CD115, CSFR, FIM2, FMS |
| Hs.77793 | NM_004383 | CSK | C-src tyrosine kinase | MGC117393 |
| Hs.631988 | NM_001954 | DDR1 | Discoidin domain receptor tyrosine kinase 1 | CAK, CD167, DDR, EDDR1, HGK2, MCK10, NEP, NTRK4, PTK3, PTK3A, RTK6, TRKE |
| Hs.275757 | NM_006182 | DDR2 | Discoidin domain receptor tyrosine kinase 2 | MIG20a, NTRKR3, TKT, TYRO10 |
| Hs.488293 | NM_005228 | EGFR | Epidermal growth factor receptor | ERBB, ERBB1, HER1, PIG61, mENA |
| Hs.89839 | NM_005232 | EPHA1 | EPH receptor A1 | EPH, EPHT, EPHT1, MGC163163 |
| Hs.171596 | NM_004431 | EPHA2 | EPH receptor A2 | ARCC2, ECK |
| Hs.123642 | NM_005233 | EPHA3 | EPH receptor A3 | EK4, ETK, ETK1, HEK, HEK4, TYRO4 |
| Hs.371218 | NM_004438 | EPHA4 | EPH receptor A4 | HEK8, SEK, TYRO1 |
| Hs.654492 | NM_004439 | EPHA5 | EPH receptor A5 | CEK7, EHK1, HEK7, TYRO4 |
| Hs.73962 | NM_004440 | EPHA7 | EPH receptor A7 | EHK3, HEK11 |

(continued)

| | | | Table 27: List of tyrosine kinases investigated for abnormal expression | |
|---|---|---|---|---|
| Unigene | GeneBank | Symbol | Description | Gene Name |
| Hs.283613 | NM_020526 | EPHA8 | EPH receptor A8 | EEK, EK3, HEK3, KIAA1459 |
| Hs.116092 | NM_004441 | EPHB1 | EPH receptor B1 | ELK, EPHT2, FLJ37986, Hek6, NET |
| Hs.523329 | NM_004442 | EPHB2 | EPH receptor B2 | CAPB, DRT, EK5, EPHT3, ERK, Hek5, MGC87492, PCBC, Tyro5 |
| Hs.2913 | NM_004443 | EPHB3 | EPH receptor B3 | ETK2, HEK2, TYRO6 |
| Hs.437008 | NM_004444 | EPHB4 | EPH receptor B4 | HTK, MYK1, TYRO11 |
| Hs.380089 | NM_004445 | EPHB6 | EPH receptor B6 | HEP, MGC129910, MGC129911 |
| Hs.446352 | NM_004448 | ERBB2 | V-erb-b2 erythroblastic leukemia viral oncogene homolog 2, neuro/glioblastoma derived oncogene homolog (avian) | CD340, HER-2, HER-2, neu, HER2, MLN 19, NEU, NGL, TKR1 |
| Hs.118681 | NM_001982 | ERBB3 | V-erb-b2 erythroblastic leukemia viral oncogene homolog 3 (avian) | ErbB-3, HER3, LCCS2, MDA-BF-1, MGC88033, c-erbB-3, c-erbB3, erbB3-S, p180-ErbB3, p45-sErbB3, p85-sErbB3 |
| Hs.390729 | NM_005235 | ERBB4 | V-erb-a erythroblastic leukemia viral oncogene homolog 4 (avian) | HER4, MGC138404, p180erbB4 |
| Hs.221472 | NM_005246 | FER | Fer (fps/fes related) tyrosine kinase | TYK3 |
| Hs.7636 | NM_002005 | FES | Feline sarcoma oncogene | FPS |
| Hs.264887 | NM_015850 | FGFR1 | Fibroblast growth factor receptor 1 | BFGFR, CD331, CEK, FGFBR, FGFR-1, FLG, FLJ99988, FLT-2, FLT2, HBGFR, KAL2, N-SAM, OGD, bFGF-R-1 |
| Hs.533683 | NM_000141 | FGFR2 | Fibroblast growth factor receptor 2 | BEK, BFR-1, CD332, CEK3, CFD1, ECT1, FLJ98662, JWS, K-SAM, KGFR, TK14, TK25 |
| Hs.1420 | NM_000142 | FGFR3 | Fibroblast growth factor receptor 3 | ACH, CD333, CEK2, HSFGFR3EX, JTK4 |
| Hs.165950 | NM_002011 | FGFR4 | Fibroblast growth factor receptor 4 | CD334, JTK2, MGC20292, TKF |
| Hs.1422 | NM_005248 | FGR | Gardner-Rasheed feline sarcoma viral (v-fgr) oncogene homolog | FLJ43153, MGC75096, SRC2, c-fgr, c-src2, p55-Fgr, p55c-fgr, p58c-fgr |
| Hs.654360 | NM_002019 | FLT1 | Fms-related tyrosine kinase 1 (vascular endothelial growth factor/vascular permeability factor receptor) | FLT, VEGFR1 |
| Hs.507590 | NM_004119 | FLT3 | Fms-related tyrosine kinase 3 | CD135, FLK2, STK1 |
| Hs.646917 | NM_002020 | FLT4 | Fms-related tyrosine kinase 4 | FLT41, LMPH1A, PCL, VEGFR3 |
| Hs.89426 | NM_002031 | FRK | Fyn-related kinase | GTK, PTK5, RAK |
| Hs.390567 | NM_002037 | FYN | FYN oncogene related to SRC, FGR, YES | MGC45350, SLK, SYN |
| Hs.655210 | NM_002110 | HCK | Hemopoietic cell kinase | JTK9 |

(continued)

| Unigene | GeneBank | Symbol | Description | Gene Name |
|---------|----------|--------|-------------|-----------|
| | | | Table 27: List of tyrosine kinases investigated for abnormal expression | |
| Hs.643120 | NM_000875 | IGF1R | Insulin-like growth factor 1 receptor | CD221, IGFIR, IGFR, JTK13, MGC142170, MGC142172, MGC18216 |
| Hs.487062 | NM_000876 | IGF2R | Insulin-like growth factor 2 receptor | CD222, CIMPR, M6P-R, MPR1, MPRI |
| Hs.465744 | NM_000208 | INSR | Insulin receptor | CD220, HHF5 |
| Hs.248138 | NM_014215 | INSRR | Insulin receptor-related receptor | IRR |
| Hs.558348 | NM_005546 | ITK | IL2-inducible T-cell kinase | EMT, LYK, MGC126257, MGC126258, PSCTK2 |
| Hs.207538 | NM_002227 | JAK1 | Janus kinase 1 | JAK1A, JAK1B, JTK3 |
| Hs.656213 | NM_004972 | JAK2 | Janus kinase 2 | JTK10 |
| Hs.515247 | NM_000215 | JAK3 | Janus kinase 3 | JAK-3, JAK3_HUMAN, JAKL, L-JAK, LJAK |
| Hs.479756 | NM_002253 | KDR | Kinase insert domain receptor (a type III receptor tyrosine kinase) | CD309, FLK1, VEGFR, VEGFR2 |
| Hs.479754 | NM_000222 | KIT | V-kit Hardy-Zuckerman 4 feline sarcoma viral oncogene homolog | C-Kit, CD117, PBT, SCFR |
| Hs.470627 | NM_005356 | LCK | Lymphocyte-specific protein tyrosine kinase | LSK, YT16, p561ck, pp581ck |
| Hs.434481 | NM_002344 | LTK | Leukocyte receptor tyrosine kinase | TYK1 |
| Hs.699154 | NM_002350 | LYN | V-yes-1 Yamaguchi sarcoma viral related oncogene homolog | FLJ26625, JTK8 |
| Hs.631845 | NM_002378 | MATK | Megakaryocyte-associated tyrosine kinase | CHK, CTK, DKFZp434N1212, HHYLTK, HYL, HYLTK, Lsk, MGC1708, MGC2101 |
| Hs.306178 | NM_006343 | MERTK | C-mer proto-oncogene tyrosine kinase | MER, MGC133349, RP38, c-mer |
| Hs.132966 | NM_000245 | MET | Met proto-oncogene (hepatocyte growth factor receptor) | AUTS9, HGFR, RCCP2, c-Met |
| Hs.517973 | NM_002447 | MST1R | Macrophage stimulating 1 receptor (c-met-related tyrosine kinase) | CD136, CDw136, PTK8, RON |
| Hs.521653 | NM_005592 | MUSK | Muscle, skeletal, receptor tyrosine kinase | MGC126323, MGC126324 |
| Hs.406293 | NM_002529 | NTRK1 | Neurotrophic tyrosine kinase, receptor, type 1 | DKFZp781I14186, MTC, TRK, TRK1, TRKA, Trk-A, p140-TrkA |
| Hs.494312 | NM_006180 | NTRK2 | Neurotrophic tyrosine kinase, receptor, type 2 | GP145-TrkB, TRKB |
| Hs.410969 | NM_002530 | NTRK3 | Neurotrophic tyrosine kinase, receptor, type 3 | TRKC, gp145(trkC) |
| Hs.74615 | NM_006206 | PDGFRA | Platelet-derived growth factor receptor, alpha polypeptide | CD140A, MGC74795, PDGFR2, RHEPDGFRA |

(continued)

| Unigene | GeneBank | Symbol | Description | Gene Name |
|---|---|---|---|---|
| | | | Table 27: List of tyrosine kinases investigated for abnormal expression | |
| Hs.509067 | NM_002609 | PDGFR B | Platelet-derived growth factor receptor, beta polypeptide | CD140B, JTK12, PDGFR, PDGFR1 |
| Hs.395482 | NM_005607 | PTK2 | PTK2 protein tyrosine kinase 2 | FADK, FAK, FAK1, FRNK, pp125FAK |
| Hs.491322 | NM_004103 | PTK2B | PTK2B protein tyrosine kinase 2 beta | CADTK, CAKB, FADK2, FAK2, PKB, PTK, PYK2, RAFTK |
| Hs.51133 | NM_005975 | PTK6 | PTK6 protein tyrosine kinase 6 | BRK, FLJ42088 |
| Hs.90572 | NM_002821 | PTK7 | PTK7 protein tyrosine kinase 7 | CCK4 |
| Hs.350321 | NM_020630 | RET | Ret proto-oncogene | CDHF12, CDHR16, HSCR1, MEN2A, MEN2B, MTC1, PTC, RET-ELE1, RET51 |
| Hs.654491 | NM_005012 | ROR1 | Receptor tyrosine kinase-like orphan receptor 1 | MGC99659, NTRKR1, dJ537F10.1 |
| Hs.98255 | NM_004560 | ROR2 | Receptor tyrosine kinase-like orphan receptor 2 | BDB, BDB1, MGC163394, NTRKR2 |
| Hs.1041 | NM_002944 | ROS1 | C-ros oncogene 1, receptor tyrosine kinase | MCF3, ROS, c-ros-1 |
| Hs.654562 | NM_002958 | RYK | RYK receptor-like tyrosine kinase | D3S3195, JTK5, JTK5A, RYK1 |
| Hs.195659 | NM_005417 | SRC | V-src sarcoma (Schmidt-Ruppin A-2) viral oncogene homolog (avian) | ASV, SRC1, c-SRC, p60-Src |
| Hs.411061 | NM_080823 | SRMS | Src-related kinase lacking C-terminal regulatory tyrosine and N-terminal myristylation sites | C20orf148, SRM, dJ697K14.1 |
| Hs.371720 | NM_003177 | SYK | Spleen tyrosine kinase | DKFZp313N1010, FLJ25043, FLJ37489 |
| Hs.479670 | NM_003215 | TEC | Tec protein tyrosine kinase | MGC126760, MGC126762, PSCTK4 |
| Hs.89640 | NM_000459 | TEK | TEK tyrosine kinase, endothelial | CD202B, TIE-2, TIE2, VMCM, VMCM1 |
| Hs.78824 | NM_005424 | TIE1 | Tyrosine kinase with immunoglobulin-like and EGF-like domains 1 | JTK14, TIE |
| Hs.203420 | NM_003985 | TNK1 | Tyrosine kinase, non-receptor, 1 | MGC46193 |
| Hs.518513 | NM_005781 | TNK2 | Tyrosine kinase, non-receptor, 2 | ACK, ACK1, FLJ44758, FLJ45547, p21cdc42Hs |
| Hs.479669 | NM_003328 | TXK | TXK tyrosine kinase | BTKL, MGC22473, PSCTK5, PTK4, RLK, TKL |
| Hs.75516 | NM_003331 | TYK2 | Tyrosine kinase 2 | JTK1 |
| Hs.381282 | NM_006293 | TYRO3 | TYRO3 protein tyrosine kinase | BYK, Dtk, FLJ16467, RSE, Sky, Tif |
| Hs.194148 | NM_005433 | YES1 | V-yes-1 Yamaguchi sarcoma viral oncogene homolog 1 | HsT441, P61-YES, Yes, c-yes |

(continued)

| Table 27: List of tyrosine kinases investigated for abnormal expression | | | | |
|---|---|---|---|---|
| Unigene | GeneBank | Symbol | Description | Gene Name |
| Hs.234569 | NM_001079 | ZAP70 | Zeta-chain (TCR) associated protein kinase 70kDa | FLJ17670, FLJ17679, SRK, STD, TZK, ZAP-70 |

[Table 28]

| Table 28: List of cancer drug targets investigated for abnormal expression | | | | |
|---|---|---|---|---|
| Unigene | GeneBank | Symbol | Description | Gene Name |
| Hs.709181 | NM_004996 | ABCC1 | ATP-binding cassette, sub-family C (CFTR/MRP), member 1 | ABC29, ABCC, DKFZp686N04233, DKFZp781G125, GS-X, MRP, MRP1 |
| Hs.525622 | NM_005163 | AKT1 | V-akt murine thymoma viral oncogene homolog 1 | AKT, MGC99656, PKB, PKB-ALPHA, PRKBA, RAC, RAC-ALPHA |
| Hs.631535 | NM_001626 | AKT2 | V-akt murine thymoma viral oncogene homolog 2 | PKBB, PKBBETA, PRKBB, RAC-BETA |
| Hs.592510 | NM_001880 | ATF2 | Activating transcription factor 2 | CRE-BP1, CREB2, HB16, MGC111558, TREB7 |
| Hs.250822 | NM_003600 | AURKA | Aurora kinase A | AIK, ARK1, AURA, AURORA2, BTAK, MGC34538, STK15, STK6, STK7 |
| Hs.442658 | NM_004217 | AURKB | Aurora kinase B | AIK2, AIM-1, AIM1, ARK2, AurB, IPL1, STK12, STK5, aurkb-sv1, aurkb-sv2 |
| Hs.98338 | NM_003160 | AURKC | Aurora kinase C | AIE2, AIK3, ARK3, AurC, STK13, aurora-C |
| Hs.150749 | NM_000633 | BCL2 | B-cell CLL/lymphoma 2 | Bc1-2 |
| Hs.728893 | NM_001168 | BIRC5 | Baculoviral IAP repeat containing 5 | API4, EPR-1 |
| Hs.437705 | NM_001789 | CDC25A | Cell division cycle 25 homolog A (S. pombe) | CDC25A2 |
| Hs.334562 | NM_001786 | CDK1 | Cyclin-dependent kinase 1 | CDC2, CDC28A, DKFZp686L20222, MGC111195, P34CDC2 |
| Hs.19192 | NM_001798 | CDK2 | Cyclin-dependent kinase 2 | p33(CDK2) |
| Hs.95577 | NM_000075 | CDK4 | Cyclin-dependent kinase 4 | CMM3, MGC14458, PSK-J3 |
| Hs.647078 | NM_004935 | CDK5 | Cyclin-dependent kinase 5 | PSSALRE |
| Hs.184298 | NM_001799 | CDK7 | Cyclin-dependent kinase 7 | CAK1, CDKN7, MO15, STK1, p39MO15 |
| Hs.382306 | NM_001260 | CDK8 | Cyclin-dependent kinase 8 | K35, MGC126074, MGC126075 |
| Hs.150423 | NM_001261 | CDK9 | Cyclin-dependent kinase 9 | C-2k, CDC2L4, CTK1, PITALRE, TAK |
| Hs.520898 | NM_001908 | CTSB | Cathepsin B | APPS, CPSB |

(continued)

| Unigene | GeneBank | Symbol | Description | Gene Name |
|---------|----------|--------|-------------|-----------|
| | | | Table 28: List of cancer drug targets investigated for abnormal expression | |
| Unigene | GeneBank | Symbol | Description | Gene Name |
| Hs.121575 | NM_001909 | CTSD | Cathepsin D | CLN10, CPSD, MGC2311 |
| Hs.716407 | NM_001912 | CTSL1 | Cathepsin L1 | CATL, CTSL, FLJ31037, MEP |
| Hs.181301 | NM_004079 | CTSS | Cathepsin S | FLJ50259, MGC3886 |
| Hs.488293 | NM_005228 | EGFR | Epidermal growth factor receptor | ERBB, ERBB1, HER1, PIG61, mENA |
| Hs.446352 | NM_004448 | ERBB2 | V-erb-b2 erythroblastic leukemia viral oncogene homolog 2, neuro/glioblastoma derived oncogene homolog (avian) | CD340, HER-2, HER-2, neu, HER2, MLN 19, NEU, NGL, TKR1 |
| Hs.118681 | NM_001982 | ERBB3 | V-erb-b2 erythroblastic leukemia viral oncogene homolog 3 (avian) | ErbB-3, HER3, LCCS2, MDA-BF-1, MGC88033, c-erbB-3, c-erbB3, erbB3-S, p180-ErbB3, p45-sErbB3, p85-sErbB3 |
| Hs.390729 | NM_005235 | ERBB4 | V-erb-a erythroblastic leukemia viral oncogene homolog 4 (avian) | HER4, MGC138404, p180erbB4 |
| Hs.208124 | NM_000125 | ESR1 | Estrogen receptor 1 | DKFZp686N23123, ER, ESR, ESRA, Era, NR3A1 |
| Hs.729020 | NM_001437 | ESR2 | Estrogen receptor 2 (ER beta) | ER-BETA, ESR-BETA, ESRB, ESTRB, Erb, NR3A2 |
| Hs.11392 | NM_004469 | FIGF | C-fos induced growth factor (vascular endothelial growth factor D) | VEGF-D, VEGFD |
| Hs.654360 | NM_002019 | FLT1 | Fms-related tyrosine kinase 1 (vascular endothelial growth factor/vascular permeability factor receptor) | FLT, VEGFR1 |
| Hs.646917 | NM_002020 | FLT4 | Fms-related tyrosine kinase 4 | FLT41, LMPH1A, PCL, VEGFR3 |
| Hs.444356 | NM_002086 | GRB2 | Growth factor receptor-bound protein 2 | ASH, EGFRBP-GRB2, Grb3-3, MST084, MSTP084, NCKAP2 |
| Hs.523836 | NM_000852 | GSTP1 | Glutathione S-transferase pi 1 | DFN7, FAEES3, GST3, GSTP, PI |
| Hs.88556 | NM_004964 | HDAC1 | Histone deacetylase 1 | DKFZp686H12203, GON-10, HD1, RPD3, RPD3L1 |
| Hs.404802 | NM_024827 | HDAC11 | Histone deacetylase 11 | FLJ22237, HD11 |
| Hs.3352 | NM_001527 | HDAC2 | Histone deacetylase 2 | HD2, RPD3, YAF1 |
| Hs.519632 | NM_003883 | HDAC3 | Histone deacetylase 3 | HD3, RPD3, RPD3-2 |
| Hs.20516 | NM_006037 | HDAC4 | Histone deacetylase 4 | AHO3, BDMR, HA6116, HD4, HDAC-A, HDACA, KIAA0288 |
| Hs.6764 | NM_006044 | HDAC6 | Histone deacetylase 6 | FLJ16239, HD6 |
| Hs.200063 | NM_0010984 16 | HDAC7 | Histone deacetylase 7 | DKFZp586J0917, FLJ99588, HD7A, HDAC7A |
| Hs.310536 | NM_018486 | HDAC8 | Histone deacetylase 8 | HD8, HDACL1, RPD3 |

| Table 28: List of cancer drug targets investigated for abnormal expression | | | | |
|---|---|---|---|---|
| Unigene | GeneBank | Symbol | Description | Gene Name |
| Hs.597216 | NM_001530 | HIF1A | Hypoxia inducible factor 1, alpha subunit (basic helix-loop-helix transcription factor) | HIF-1alpha, HIF1, HIF1-ALPHA, MOP1, PASD8, bHLHe78 |
| Hs.37003 | NM_005343 | HRAS | V-Ha-ras Harvey rat sarcoma viral oncogene homolog | C-BAS, HAS, C-H-RAS, C-HA-RAS1, CTLO, H-RASIDX, HAMSV, HRAS 1, K-RAS, N-RAS, RASH1 |
| Hs.525600 | NM_0010179 63 | HSP90AA 1 | Heat shock protein 90kDa alpha (cytosolic), class A member 1 | FLJ31884, HSP86, HSP89A, HSP90A, HSP90N, HSPC1, HSPCA, HSPCAL1, HSPCAL4, HSPN, Hsp89, Hsp90, LAP2 |
| Hs.192374 | NM_003299 | HSP90B1 | Heat shock protein 90kDa beta (Grp94), member 1 | ECGP, GP96, GRP94, TRA1 |
| Hs.160562 | NM_000618 | IGF1 | Insulin-like growth factor 1 (somatomedin C) | IGF-I, IGF1A, IGFI |
| Hs.643120 | NM_000875 | IGF1R | Insulin-like growth factor 1 receptor | CD221, IGFIR, IGFR, JTK13, MGC142170, MGC142172, MGC18216 |
| Hs.523414 | NM_000612 | IGF2 | Insulin-like growth factor 2 (somatomedin A) | C11orf43, FLJ22066, FLJ44734, IGF-II, PP9974 |
| Hs.521181 | NM_00109862 9 | IRF5 | Interferon regulatory factor 5 | SLEB10 |
| Hs.479756 | NM_002253 | KDR | Kinase insert domain receptor (a type III receptor tyrosine kinase) | CD309, FLK1, VEGFR, VEGFR2 |
| Hs.479754 | NM_000222 | KIT | V-kit Hardy-Zuckerman 4 feline sarcoma viral oncogene homolog | C-Kit, CD117, PBT, SCFR |
| Hs.505033 | NM_004985 | KRAS | V-Ki-ras2 Kirsten rat sarcoma viral oncogene homolog | C-K-RAS, K-RAS2A, K-RAS2B, K-RAS4A, K-RAS4B, KI-RAS, KRAS1, KRAS2, NS, NS3, RASK2 |
| Hs.484551 | NM_002392 | MDM2 | Mdm2 p53 binding protein homolog (mouse) | HDMX, MGC5370, MGC71221, hdm2 |
| Hs.497492 | NM_002393 | MDM4 | Mdm4 p53 binding protein homolog (mouse) | DKFZp781B1423, HDMX, MDMX, MGC132766, MRP1 |
| Hs.338207 | NM_004958 | MTOR | Mechanistic target of rapamycin (serine/threonine kinase) | FLJ44809, FRAP, FRAP1, FRAP2, RAFT1, RAPT1 |
| Hs.654408 | NM_003998 | NFKB1 | Nuclear factor of kappa light polypeptide gene enhancer in B-cells 1 | DKFZp686C01211, EBP-1, KBF1, MGC54151, NF-kappa-B, NF-kappaB, NFKB-p105, NFKB-p50, NFkappaB, p105, p50 |
| Hs.486502 | NM_002524 | NRAS | Neuroblastoma RAS viral (v-ras) oncogene homolog | ALPS4, N-ras, NRAS1, NS6 |
| Hs.158336 | NM_006181 | NTN3 | Netrin 3 | NTN2L |

(continued)

| Table 28: List of cancer drug targets investigated for abnormal expression | | | | |
|---|---|---|---|---|
| Unigene | GeneBank | Symbol | Description | Gene Name |
| Hs.177766 | NM_001618 | PARP1 | Poly (ADP-ribose) polymerase 1 | ADPRT, ADPRT 1, ADPRT1, PARP, PARP-1, PPOL, pADPRT-1 |
| Hs.409412 | NM_005484 | PARP2 | Poly (ADP-ribose) polymerase 2 | ADPRT2, ADPRTL2, ADPRTL3, PARP-2, pADPRT-2 |
| Hs.718412 | NM_006437 | PARP4 | Poly (ADP-ribose) polymerase family, member 4 | ADPRTL1, PARP-4, PARPL, PH5P, VAULT3, VPARP, VWA5C, p193 |
| Hs.74615 | NM_006206 | PDGFRA | Platelet-derived growth factor receptor, alpha polypeptide | CD140A, MGC74795, PDGFR2, RHEPDGFRA |
| Hs.509067 | NM_002609 | PDGFRB | Platelet-derived growth factor receptor, beta polypeptide | CD140B, JTK12, PDGFR, PDGFR1 |
| Hs.32405 | NM_000926 | PGR | Progesterone receptor | NR3C3, PR |
| Hs.175343 | NM_002645 | PIK3C2A | Phosphoinositide-3-kinase, class 2, alpha polypeptide | CPK, DKFZp686L193, MGC142218, PI3-K-C2(ALPHA), PI3-K-C2A |
| Hs.464971 | NM_002647 | PIK3C3 | Phosphoinositide-3-kinase, class 3 | MGC61518, VPS34, hVps34 |
| Hs.553498 | NM_006218 | PIK3CA | Phosphoinositide-3-kinase, catalytic, alpha polypeptide | MGC142161, MGC142163, PI3K, p110-alpha |
| Hs.592049 | NM_005030 | PLK1 | Polo-like kinase 1 | PLK, STPK13 |
| Hs.398157 | NM_006622 | PLK2 | Polo-like kinase 2 | SNK |
| Hs.632415 | NM_004073 | PLK3 | Polo-like kinase 3 | CNK, FNK, PRK |
| Hs.172052 | NM_014264 | PLK4 | Polo-like kinase 4 | SAK, STK18 |
| Hs.531704 | NM_002737 | PRKCA | Protein kinase C, alpha | AAG6, MGC129900, MGC129901, PKC-alpha, PKCA, PRKACA |
| Hs.460355 | NM_002738 | PRKCB | Protein kinase C, beta | MGC41878, PKC-beta, PKCB, PRKCB1, PRKCB2 |
| Hs.155342 | NM_006254 | PRKCD | Protein kinase C, delta | MAY1, MGC49908, PKCD, nPKC-delta |
| Hs.580351 | NM_005400 | PRKCE | Protein kinase C, epsilon | MGC125656, MGC125657, PKCE, nPKC-epsilon |
| Hs.196384 | NM_000963 | PTGS2 | Prostaglandin-endoperoxide synthase 2 (prostaglandin G/H synthase and cyclooxygenase) | COX-2, COX2, GRIPGHS, PGG, HS, PGHS-2, PHS-2, hCox-2 |
| Hs.247077 | NM_001664 | RHOA | Ras homolog gene family, member A | ARH12, ARHA, RH012, RHOH12 |
| Hs.502876 | NM_004040 | RHOB | Ras homolog gene family, member B | ARH6, ARHB, MST081, MSTP081, RHOH6 |
| Hs.492203 | NM_198253 | TERT | Telomerase reverse transcriptase | EST2, TCS1, TP2, TRT, hEST2, hTRT |

(continued)

| Table 28: List of cancer drug targets investigated for abnormal expression | | | | |
|---|---|---|---|---|
| Unigene | GeneBank | Symbol | Description | Gene Name |
| Hs.370267 | NM_003747 | TNKS | Tankyrase, TRF1-interacting ankyrin-related ADP-ribose polymerase | PARP-5a, PARP5A, PARPL, TIN1, TINF1, TNKS1, pART5 |
| Hs.156346 | NM_001067 | TOP2A | Topoisomerase (DNA) II alpha 170kDa | TOP2, TP2A |
| Hs.475733 | NM_001068 | TOP2B | Topoisomerase (DNA) II beta 180kDa | TOPIIB, top2beta |
| Hs.654481 | NM_000546 | TP53 | Tumor protein p53 | FLJ92943, LFS1, P53, TRP53 |
| Hs.435136 | NM_003329 | TXN | Thioredoxin | DKFZp686B1993, MGC61975, TRX, TRX1 |
| Hs.728817 | NM_003330 | TXNRD1 | Thioredoxin reductase 1 | GRIM-12, MGC9145, TR, TR1, TRXR1, TXNR |

As a result of the image analysis performed after the hybridization and washing, it was confirmed that the hybridization had been performed without any problems. The images and digital data analyzed by Feature Extraction Software after the hybridization were stored on the storage media.

In this analysis, the normalized value of the digital data for the hybridization of a test cell (induced malignant stem cell) sample was divided by the normalized value from the digital data for the hybridization of a control cell sample and the quotient was used as a measure of variation in expression. Probes for which the quotients deviated from 1 (i.e., $\log_2 1 = 0$) were considered to indicate a variation in expression; those showing quotients greater than 2 ($\log_2 2 = 1$) or smaller than 0.5 ($\log_2 0.5 = -1$) were selected. For each of the comparisons shown below, the genes shown in the tyrosine kinase list and those shown in the cancer drug targets list were analyzed to make respective lists of the genes showing quotients greater than 2 ($\log_2 2 = 1$) or smaller than 0.5 ($\log_2 0.5 = -1$), as compared with the quotient for the control (taken as 1). The analysis was performed using GeneSpring 12.1.

The results are shown in Table 29. The Normalized column represents a variation in expression in logarithmic values.

[Table 29]

| Table 29: List of tyrosine kinases that exhibited change in mRNA expression | | | | |
|---|---|---|---|---|
| [ge2_1] vs [Nfb2_17] | | | | |
| ProbeName | GeneSymbol | GenbankAccession | Chrom | Normalized |
| A_23_P110253 | KIT | NM_000222 | chr4 | 1.1215057 |
| A_23_P26810 | TP53 | NM_000546 | chr17 | 1.8496804 |
| A_23_P352266 | BCL2 | NM_000633 | chr18 | 1.2866602 |
| A_24_P367289 | DDR1 | NM_013994 | chr6 | 1.1509409 |
| A_23_P150609 | IGF2 | NM_001007139 | chr11 | 4.350153 |
| A_23_P39682 | ZAP70 | NM_001079 | chr2 | 3.6025705 |
| A_24_P169234 | ZAP70 | NM_001079 | chr2 | 2.661715 |
| A_23_P200067 | EPHB2 | NM_004442 | chr1 | 1.0295329 |
| A_23_P349416 | ERBB3 | NM_001982 | chr12 | 1.1306019 |
| A_23_P50678 | MATK | NM_139354 | chr19 | 1.8611522 |
| | | | | |
| A_23_P9255 | SYK | NM_003177 | chr9 | -1.738316 |

(continued)

| Table 29: List of tyrosine kinases that exhibited change in mRNA expression | | | | |
|---|---|---|---|---|
| [ge2_1] vs [Nfb2_17] | | | | |
| ProbeName | GeneSymbol | GenbankAccession | Chrom | Normalized |
| A_23_P147431 | LYN | NM_002350 | chr8 | -1.00478 |
| A_24_P359859 | HDAC4 | NM_006037 | chr2 | -1.224655 |
| A_24_P308096 | JAK3 | NM_000215 | chr19 | -1.139309 |
| | | | | |
| [ge2_5] vs [Nfb2_17] | | | | |
| ProbeName | GeneSymbol | GenbankAccession | Chrom | Normalized |
| A_23_P26810 | TP53 | NM_000546 | chr17 | 2.0851178 |
| A_24_P367289 | DDR1 | NM_013994 | chr6 | 1.2263508 |
| A_23_P150609 | IGF2 | NM_001007139 | chr11 | 3.066241 |
| A_23_P103932 | FGR | NM_005248 | chr1 | 1.545176 |
| A_23_P349416 | ERBB3 | NM_001982 | chr12 | 1.0128937 |
| A_23_P50678 | MATK | NM_139354 | chr19 | 2.065824 |
| | | | | |
| A_23_P9255 | SYK | NM_003177 | chr9 | -1.597535 |
| A_23_P398566 | NR4A3 | NM_173198 | chr9 | -1.014949 |
| A_32_P377880 | GDNF | | chr5 | -1.032389 |
| A_23_P30254 | PLK2 | NM_006622 | chr5 | -1.331514 |
| A_24_P359859 | HDAC4 | NM_006037 | chr2 | -1.107391 |
| A_23_P300033 | PDGFRA | NM_006206 | chr4 | -1.075276 |
| A_23_P4764 | INSR | NM_000208 | chr19 | -1.041312 |
| A_24_P308096 | JAK3 | NM_000215 | chr19 | -1.078475 |
| | | | | |
| [ge2_10] vs [Nfb2_17] | | | | |
| ProbeName | GeneSymbol | GenbankAccession | Chrom | Normalized |
| A_23_P110253 | KIT | NM_000222 | chr4 | 1.2368736 |
| A_23_P26810 | TP53 | NM_000546 | chr17 | 2.148375 |
| A_23_P110851 | TERT | NM_198253 | chr5 | 1.0629959 |
| A_23_P352266 | BCL2 | NM_000633 | chr18 | 1.1217065 |
| A_24_P367289 | DDR1 | NM_013994 | chr6 | 1.2499332 |
| A_23_P150609 | IGF2 | NM_001007139 | chr11 | 3.9652386 |
| A_24_P59667 | JAK3 | BC028068 | chr19 | 1.0839491 |
| A_23_P103932 | FGR | NM_005248 | chr1 | 2.0364814 |
| A_23_P349416 | ERBB3 | NM_001982 | chr12 | 1.0935488 |
| A_23_P50678 | MATK | NM_139354 | chr19 | 2.613666 |
| | | | | |
| A_23_P9255 | SYK | NM_003177 | chr9 | -1.854024 |

(continued)

| [ge2_10] vs [Nfb2_17] | | | | |
|---|---|---|---|---|
| ProbeName | GeneSymbol | GenbankAccession | Chrom | Normalized |
| A_23_P398566 | NR4A3 | NM_173198 | chr9 | -1.35792 |
| A_23_P98183 | HRAS | NM_005343 | chr11 | -1.118774 |
| A_24_P281101 | ABL1 | NM_005157 | chr9 | -1.076638 |
| A_32_P377880 | GDNF | | chr5 | -1.731892 |
| A_32_P183765 | ERBB4 | NM_005235 | chr2 | -1.81109 |
| A_24_P359859 | HDAC4 | NM_006037 | chr2 | -1.097812 |
| A_23_P300033 | PDGFRA | NM_006206 | chr4 | -1.736645 |
| A_24_P308096 | JAK3 | NM_000215 | chr19 | -1.214444 |
| | | | | |
| [cc1_1] vs [Nfb2_17] | | | | |
| ProbeName | GeneSymbol | GenbankAccession | Chrom | Normalized |
| A_23_P398566 | NR4A3 | NM_173198 | chr9 | 1.5130844 |
| A_23_P352266 | BCL2 | NM_000633 | chr18 | 1.08148 |
| A_23_P150609 | IGF2 | NM_001007139 | chr11 | 1.5628881 |
| A_24_P410605 | ROR1 | HC080541 | chr1 | 1.2041416 |
| A_23_P30254 | PLK2 | NM_006622 | chr5 | 1.1082869 |
| A_23_P103932 | FGR | NM_005248 | chr1 | 1.6589236 |
| A_23_P349416 | ERBB3 | NM_001982 | chr12 | 1.2875051 |
| A_23_P50678 | MATK | NM_139354 | chr19 | 2.2917066 |
| | | | | |
| A_23_P9255 | SYK | NM_003177 | chr9 | -1.062065 |
| A_32_P100379 | PDGFRA | AA599881 | chr4 | -1.001573 |
| A_23_P202245 | RET | NM_020975 | chr10 | -1.13012 |
| A_24_P246467 | ATF2 | NM_001880 | chr2 | -1.022381 |
| A_24_P71973 | KDK | NM_002253 | chr4 | -1.186252 |
| A_24_P419239 | EPHA8 | NM_001006943 | chr1 | -1.148476 |
| A_23_P208132 | BCL2 | M13995 | Chr18 | -1.259025 |
| A_24_P916496 | PRKCA | NM_002737 | chr17 | -1.381505 |
| A_32_P183765 | ERBB4 | NM_005235 | chr2 | -1.128573 |
| A_24_P274219 | EPHA4 | NM_004438 | chr2 | -1.453237 |
| A_32_P144342 | PARP4 | NM_006437 | chr13 | -1.067832 |
| A_23_P95060 | EPHB3 | NM_004443 | chr3 | -1.409284 |
| A_24_P359859 | HDAC4 | NM_006037 | chr2 | -1.657354 |
| A_23_P4764 | INSR | NM_000208 | chr19 | -1.199753 |
| A_23_P119899 | EPHA4 | NM_004438 | chr2 | -1.270102 |
| A_23_P108501 | EPHA4 | NM_004438 | chr2 | -1.791599 |
| A_24_P308096 | JAK3 | NM_000215 | chr19 | -1.053361 |

(continued)

| [cc1_1] vs [Nfb2_17] | | | | |
|---|---|---|---|---|
| ProbeName | GeneSymbol | GenbankAccession | Chrom | Normalized |
| | | | | |
| A_23_P149281 | EPHA2 | NM_004431 | chr1 | 1.7232876 |
| A_23_P359245 | MET | NM_000245 | chr7 | 3.9879103 |
| A_23_P9255 | SYK | NM_003177 | chr9 | 3.422285 |
| A_23_P308603 | SRC | NM_005417 | chr20 | 1.2612085 |
| A_23_P30024 | NFKB1 | NM_003998 | chr4 | 1.2639065 |
| A_23_P61633 | TNK2 | NM_005781 | chr3 | 1.9869528 |
| A_23_P144054 | PRKCD | NM_006254 | chr3 | 1.6901083 |
| A_24_P410678 | JAK1 | NM_002227 | chr1 | 1.9977531 |
| A_23_P215790 | EGFR | NM_005228 | chr7 | 2.4525843 |
| A_24_P367289 | DDR1 | NM_013994 | chr6 | 1.272727 |
| A_23_P212830 | FGFR3 | NM_000142 | chr4 | 1.7352262 |
| A_23_P213114 | TEC | NM_003215 | chr4 | 1.409246 |
| A_23_P117175 | PARP4 | NM_006437 | chr13 | 1.0518188 |
| A_24_P303770 | CTSB | NM_147780 | chr8 | 3.4885387 |
| A_23_P97005 | JAK1 | NM_002227 | chr1 | 2.184863 |
| A_23_P95060 | EPHB3 | NM_004443 | chr3 | 1.5370007 |
| A_23_P52556 | CTSD | NM_001909 | chr11 | 2.0220432 |
| A_23_P153311 | TYK2 | NM_003331 | chr19 | 1.779275 |
| A_23_P26124 | RORA | NM_134260 | chr15 | 2.7408571 |
| A_23_P256312 | MST1R | NM_002447 | chr3 | 5.284766 |
| A_23_P200067 | EPHB2 | NM_004442 | chr1 | 3.5468144 |
| A_23_P215944 | CTSB | NM_147780 | chr8 | 3.0934038 |
| A_23_P155360 | HDAC 11 | NM_024827 | chr3 | 2.316156 |
| A_23_P349416 | ERBB3 | NM_001982 | chr12 | 2.6570024 |
| A_23_P152024 | CSK | NM_004383 | chr15 | 1.1600018 |
| A_24_P397928 | CTSB | NM_147780 | chr8 | 3.8767343 |
| | | | | |
| A_23_P202334 | FGFR2 | NM_022970 | chr10 | -2.5042696 |
| A_24_P397107 | CDC25A | NM_001789 | chr3 | -2.0764656 |
| A_23_P72050 | PTK2 | NM_153831 | chr8 | -1.0391893 |
| A_23_P103361 | LCK | NM_005356 | chr1 | -5.5401087 |
| A_24_P56388 | HIF1A | NM_181054 | chr14 | -1.9960356 |
| A_23_P94533 | CTSL1 | NM_001912 | chr9 | -4.4751987 |
| A_23_P114783 | PARP1 | NM_001618 | chr1 | -1.9163618 |
| A_24_P205137 | HDAC6 | BC011498 | chrX | -1.2546759 |
| A_23_P209879 | ATF2 | AK128731 | chr2 | -1.1258135 |

(continued)

| [cc1_1] vs [Nfb2_17] | | | | |
|---|---|---|---|---|
| ProbeName | GeneSymbol | GenbankAccession | Chrom | Normalized |
| A_23_P110851 | TERT | NM_198253 | chr5 | -4.7348857 |
| A_24_P246467 | ATF2 | NM_001880 | chr2 | -1.1050744 |
| A_23_P145935 | EPHB6 | NM_004445 | chr7 | -2.3128839 |
| A_23_P118815 | BIRC5 | NM_001012271 | chr17 | -1.1888933 |
| A_23_P150609 | IGF2 | NM_001007139 | chr11 | -3.3835301 |
| A_24_P916496 | PRKCA | NM_002737 | chr17 | -1.6827946 |
| A_23_P60180 | ABL1 | NM_005157 | chr9 | -1.2428389 |
| A_24_P274219 | EPHA4 | NM_004438 | chr2 | -1.7993469 |
| A_23_P417282 | IGF1R | NM_000875 | chr15 | -3.9472814 |
| A_23_P24997 | CDK4 | NM_000075 | chr12 | -1.1817236 |
| A_23_P300033 | PDGFRA | NM_006206 | chr4 | -1.8508711 |
| A_23_P119899 | EPHA4 | NM_004438 | chr2 | -1.5421052 |
| A_23_P155969 | PLK4 | NM_014264 | chr4 | -1.1462336 |
| A_24_P206624 | FGFR2 | NM_022970 | chr10 | -1.5162249 |
| A_23_P157333 | EPHA1 | NM_005232 | Chr7 | -1.1080103 |
| A_23_P130182 | AURKB | NM_004217 | Chr17 | -1.3989334 |
| A_23_P122304 | HDAC2 | NM_001527 | chr6 | -1.3593063 |
| A_23_P208389 | AXL | NM_021913 | chr19 | -4.377584 |
| | | | | |
| [cc4_d] vs [Nfb2_17] | | | | |
| ProbeName | GeneSymbol | GenbankAccession | Chrom | Normalized |
| A_23_P149281 | EPHA2 | NM_004431 | Chr1 | 1.1973152 |
| A_23_P123608 | JAK2 | NM_004972 | chr9 | 1.1758513 |
| A_23_P359245 | MET | NM_000245 | chr7 | 3.1477613 |
| A_23_P9255 | SYK | NM_003177 | chr9 | 3.8926134 |
| A_23_P308603 | SRC | NM_005417 | chr20 | 1.102274 |
| A_23_P30024 | NFKB1 | NM_003998 | chr4 | 1.6260166 |
| A_23_P61633 | TNK2 | NM_005781 | chr3 | 1.7674026 |
| A_23_P144054 | PRKCD | NM_006254 | chr3 | 1.3304882 |
| A_24_P410678 | JAK1 | NM_002227 | chr1 | 2.264883 |
| A_23_P215790 | EGFR | NM_005228 | chr7 | 1.4954863 |
| A_23_P39682 | ZAP70 | NM_001079 | chr2 | 2.68653 |
| A_23_P212830 | FGFR3 | NM_000142 | chr4 | 1.5743127 |
| A_23_P213114 | TEC | NM_003215 | chr4 | 1.0232491 |
| A_23_P117175 | PARP4 | NM_006437 | chr13 | 1.1623564 |
| A_24_P169234 | ZAP70 | NM_001079 | chr2 | 2.2829566 |
| A_24_P303770 | CTSB | NM_147780 | chr8 | 3.775197 |

(continued)

| [cc4_d] vs [Nfb2_17] | | | | |
|---|---|---|---|---|
| ProbeName | GeneSymbol | GenbankAccession | Chrom | Normalized |
| A_23_P97005 | JAK1 | NM_002227 | chr1 | 1.905427 |
| A_23_P95060 | EPHB3 | NM_004443 | chr3 | 1.2916594 |
| A_23_P52556 | CTSD | NM_001909 | chr11 | 2.415269 |
| A_23_P156953 | IGF2R | NM_000876 | chr6 | 1.0904293 |
| A_23_P153311 | TYK2 | NM_003331 | chr19 | 1.5666256 |
| A_23_P256312 | MST1R | NM_002447 | chr3 | 5.0612764 |
| A_23_P500501 | FGFR3 | NM_000142 | chr4 | 1.2674742 |
| A_23_P200067 | EPHB2 | NM_004442 | chr1 | 3.541532 |
| A_23_P215944 | CTSB | NM_147780 | chr8 | 3.5871096 |
| A_23_P155360 | HDAC11 | NM_024827 | chr3 | 2.0750299 |
| A_23_P349416 | ERBB3 | NM_001982 | chr12 | 2.7191648 |
| A_23_P152024 | CSK | NM_004383 | chr15 | 1.2959967 |
| A_24_P397928 | CTSB | NM_147780 | chr8 | 4.4394937 |
| | | | | |
| A_23_P110253 | KIT | NM_000222 | chr4 | -2.6171603 |
| A_23_P202334 | FGFR2 | NM_022970 | chr10 | -3.698885 |
| A_24_P397107 | CDC25A | NM_001789 | chr3 | -1.698369 |
| A_23_P147431 | LYN | NM_002350 | chr8 | -1.0074892 |
| A_32_P100379 | PDGFRA | AA599881 | chr4 | -1.9433503 |
| A_23_P103361 | LCK | NM_005356 | chr1 | -4153756 |
| A_24_P56388 | HIF1A | NM_181054 | chr14 | -2.1315765 |
| A_23_P94533 | CTSL1 | NM_001912 | chr9 | -4.598218 |
| A_24_P313504 | PLK1 | NM_005030 | chr16 | -1.0595436 |
| A_23_P114783 | PARP1 | NM_001618 | chr1 | -1.6551466 |
| A_24_P205137 | HDAC6 | BC011498 | chrX | -1.5821996 |
| A_23_P209879 | ATF2 | AK128731 | chr2 | -1.3147001 |
| A_23_P110851 | TERT | NM_198253 | chr5 | -4.4801784 |
| A_24_P246467 | ATF2 | NM_001880 | chr2 | -1.2144108 |
| A_23_P208132 | BCL2 | M13995 | chr18 | -1.3138638 |
| A_23_P164507 | YES1 | NM_005433 | chr18 | -1.6291232 |
| A_23_P502142 | FYN | NM_002037 | chr6 | -1.1607904 |
| A_24_P281101 | ABL1 | NM_005157 | chr9 | -1.5211582 |
| A_23_P500271 | IRF5 | NM_002200 | chr7 | -1.5505834 |
| A_23_P145935 | EPHB6 | NM_004445 | chr7 | -2.4464698 |
| A_23_P118815 | BIRC5 | NM_001012271 | chr17 | -1.2690687 |
| A_23_P150609 | IGF2 | NM_001007139 | chr11 | -1.746458 |
| A_24_P916496 | PRKCA | NM_002737 | chr17 | -1.0500383 |

(continued)

| [cc4_d] vs [Nfb2_17] | | | | |
|---|---|---|---|---|
| ProbeName | GeneSymbol | GenbankAccession | Chrom | Normalized |
| A_23_P163027 | PARP2 | NM_005484 | chr14 | -1.1376743 |
| A_23_P301304 | FGFR1 | NM_023110 | chr8 | -8.119717 |
| A_32_P183765 | ERBB4 | NM_005235 | chr2 | -6.158379 |
| A_23_P121423 | CDC25A | NM_001789 | chr3 | -1.4154968 |
| A_23_P14769 | FES | NM_002005 | chr15 | -3.5244155 |
| A_23_P118834 | TOP2A | NM_001067 | chr17 | -1.0636063 |
| A_24_P274219 | EPHA4 | NM_004438 | chr2 | -2.3304243 |
| A_24_P128145 | ATF2 | NM_001880 | chr2 | -1.4385839 |
| A_23_P63190 | NRAS | NM_002524 | chr1 | -1.0414906 |
| A_23_P417282 | IGF1R | NM_000875 | chr15 | -6.7087193 |
| A_24_P59667 | JAK3 | BC028068 | chr19 | -4.943627 |
| A_23_P103932 | FGR | NM_005248 | chr1 | -1.1052666 |
| A_23_P300033 | PDGFRA | NM_006206 | chr4 | -2.3364549 |
| A_23_P119899 | EPHA4 | NM_004438 | chr2 | -1.9351697 |
| A_23_P155969 | PLK4 | NM_014264 | chr4 | -1.4981489 |
| A_24_P206624 | FGFR2 | NM_022970 | chr10 | -3.0886965 |
| A_24_P320545 | PTK7 | NM_002821 | chr6 | -2.7910752 |
| A_24_P4171 | FGFR1 | NM_023111 | chr8 | -3.8551974 |
| A_23_P108501 | EPHA4 | NM_004438 | chr2 | -1.8843508 |
| A_23_P168443 | EPHB4 | NM_004444 | chr7 | -1.0193481 |
| A_23_P92754 | FGFR4 | NM_213647 | chr5 | -1.0031805 |
| A_23_P130182 | AURKB | NM_004217 | chr17 | -1.8129711 |
| A_23_P122304 | HDAC2 | NM_001527 | chr6 | -1.103261 |
| A_24_P383478 | ESR1 | NM_000125 | chr6 | -1.1072159 |
| A_23_P208389 | AXL | NM_021913 | chr19 | -4.4775496 |
| A_23_P20927 | TNKS | NM_003747 | chr8 | -1.1630383 |

[Table 30]

| Table 30: List of cancer drug targets that exhibited change in mRNA expression | | | | |
|---|---|---|---|---|
| [gc2_1] vc [Nfb2_17] | | | | |
| ProbeName | GeneSymbol | GenebankAccession | Chrom | Normalized |
| A_23_P110253 | KIT | NM_000222 | chr4 | 1.1215057 |
| A_23_P26810 | TP53 | NM_000546 | chr17 | 1.8486804 |
| A_23_P352266 | BCL2 | NM_000633 | chr18 | 1.2866602 |
| A_23_P150609 | IGF2 | NM_001007139 | chr11 | 4.350153 |
| A_23_P349416 | ERBB3 | NM_001982 | chr12 | 1.1306019 |

(continued)

| Table 30: List of cancer drug targets that exhibited change in mRNA expression | | | | |
|---|---|---|---|---|
| [gc2_1] vc [Nfb2_17] | | | | |
| ProbeName | GeneSymbol | GenebankAccession | Chrom | Normalized |
| | | | | |
| A_24_P359859 | HDAC4 | NM_006037 | chr2 | -1.2246552 |
| | | | | |
| [gc2_5] vc [Nfb2_17] | | | | |
| ProbeName | GeneSymbol | GenebankAccession | Chrom | Normalized |
| A_23_P26810 | TP53 | NM_000546 | chr17 | 2.0851178 |
| A_23_P150609 | IGF2 | NM_001007139 | chr11 | 3.0662441 |
| A_23_P349416 | ERBB3 | NM_001982 | chr12 | 1.0128937 |
| | | | | |
| A_32_P377880 | GDNF | | chr5 | -1.0323887 |
| A_23_P30254 | PLK2 | NM_006622 | chr6 | -1.3315144 |
| A_24_P359859 | HDAC4 | NM_006037 | chr2 | -1.1073914 |
| A 23 P300033 | PDGFRA | NM_006206 | chr4 | -1.0752759 |
| | | | | |
| [gc2_10] vc [Nfb2_17] | | | | |
| ProbeName | GeneSymbol | GenebankAccession | Chrom | Normalized |
| A_23_P110253 | KIT | NM_000222 | chr4 | 1.2368736 |
| A_23_P26810 | TP53 | NM_000546 | chr17 | 2.148375 |
| A_23_P110851 | TERT | NM_198253 | chr5 | 1.0629959 |
| A_23_P352266 | BCL2 | NM_000633 | chr18 | 1.1217065 |
| A_23_P150609 | IGF2 | NM_001007139 | chr11 | 3.9652386 |
| A_23_P349416 | ERBB3 | NM_001982 | chr12 | 1.0935488 |
| | | | | |
| A_23_P98183 | HRAS | NM_005343 | chr11 | -1.1187744 |
| A_32_P377880 | GDNF | | chr5 | -1.7318916 |
| A_32_P183765 | ERBB4 | NM_005235 | chr2 | -1.8110895 |
| A_24_P359859 | HDAC4 | NM_006037 | chr2 | -1.0978117 |
| A_23_P300033 | PDGFRA | NM_006206 | chr4 | -1.7366452 |
| | | | | |
| [gc1_1] vc [Nfb2_17] | | | | |
| ProbeName | GeneSymbol | GenebankAccession | Chrom | Normalized |
| A_23_P352266 | BCL2 | NM_000633 | chr18 | 1.08148 |
| A_23_P150609 | IGF2 | NM_001007139 | chr11 | 1.5628881 |
| A_23_P30254 | PLK2 | NM_006622 | chr5 | 1.1082869 |
| A_23_P349416 | ERBB3 | NM_001982 | chr12 | 1.2875051 |
| A_32_P100379 | PDGFRA | AA599881 | chr4 | -1.0015731 |

(continued)

| [gc1_1] vc [Nfb2_17] | | | | |
|---|---|---|---|---|
| ProbeName | GeneSymbol | GenebankAccession | Chrom | Normalized |
| A_24_P246467 | ATF2 | NM_001880 | chr2 | -1.0223808 |
| A_24_P71973 | KDR | NM_002253 | chr4 | -1.1862516 |
| A_23_P208132 | BCL2 | M13995 | chr18 | -1.2590246 |
| A_24_P916496 | PRKCA | NM_002737 | chr17 | -1.381505 |
| A_32_P183765 | ERBB4 | NM_005235 | chr2 | -1.1285734 |
| A_32_P144342 | PARP4 | NM_006437 | chr13 | -1.0678315 |
| A_24_P359859 | HDAC4 | NM_006037 | chr2 | -1.6573544 |
| | | | | |
| [gc4_c] vc [Nfb2_17] | | | | |
| ProbeName | GeneSymbol | GenebankAccession | Chrom | Normalized |
| A_23_P30024 | NFKB1 | NM_003998 | chr4 | 1.2639065 |
| A_23_P144054 | PRKCD | NM_006254 | chr3 | 1.6901083 |
| A_23_P215790 | EGFR | NM_005228 | chr7 | 2.4525843 |
| A_23_P117175 | PARP4 | NM_006437 | chr13 | 1.0518188 |
| A_24_P303770 | CTSB | NM_147780 | chr8 | 3.4885387 |
| A_23_P52556 | CTSD | NM_001909 | chr11 | 2.0220432 |
| A_23_P215944 | CTSB | NM_147780 | chr8 | 3.0934038 |
| A_23_P155360 | HDAC11 | NM_024827 | chr3 | 2.316156 |
| A_23_P349416 | ERBB3 | NM_001982 | chr12 | 2.6570024 |
| A_24_P397928 | CTSB | NM_147780 | chr8 | 3.8767343 |
| | | | | |
| A_24_P397107 | CDC25A | NM_001789 | chr3 | -2.0764656 |
| A_24_P56388 | HIF1A | NM_181054 | chr14 | -1.9960356 |
| A_23_P94533 | CTSL1 | NM_001912 | chr9 | -4.4751987 |
| A_23_P114783 | PARP1 | NM_001618 | chr1 | -1.9163618 |
| A_24_P205137 | HDAC6 | BC011498 | chrX | -1.2546759 |
| A_23_P209879 | ATF2 | AK128731 | chr2 | -1.1258135 |
| A_23_P110851 | TERT | NM_198253 | chr5 | -4.7348857 |
| A_24_P246467 | ATF2 | NM_001880 | chr2 | -1.1050744 |
| A_23_P118815 | BIRC5 | NM_001012271 | chr17 | -1.1888933 |
| A_23_P150609 | IGF2 | NM_001007139 | chr11 | -3.3835301 |
| A_24_P916496 | PRKCA | NM_002737 | chr17 | -1.6827946 |
| A_23_P417282 | IGF1R | NM_000875 | chr15 | -3.9472814 |
| A_23_P24997 | CDK4 | NM_000075 | chr12 | -1.1817236 |
| A_23_P300033 | PDGFRA | NM_006206 | chr4 | -1.8508711 |
| A_23_P155969 | PLK4 | NM_014264 | chr4 | -1.1462336 |
| A_23_P130182 | AURKB | NM_004217 | chr17 | -1.3989334 |

(continued)

| [gc4_c] vc [Nfb2_17] | | | | |
|---|---|---|---|---|
| ProbeName | GeneSymbol | GenebankAccession | Chrom | Normalized |
| A_23_P122304 | HDAC2 | NM_001527 | chr6 | -1.3593063 |
| | | | | |

| [gc4_d] vc [Nfb2_17] | | | | |
|---|---|---|---|---|
| ProbeName | GeneSymbol | GenebankAccession | Chrom | Normalized |
| A_23_P30024 | NFKB1 | NM_003998 | chr4 | 1.6260166 |
| A_23_P144054 | PRKCD | NM_006254 | chr3 | 1.3304882 |
| A_23_P215790 | EGFR | NM_005228 | chr7 | 1.4954863 |
| A_23_P117175 | PARP4 | NM_006437 | chr13 | 1.1623564 |
| A_24_P303770 | CTSB | NM_147780 | chrX | 3.775197 |
| A_23_P52556 | CTSD | NM_001909 | chr11 | 2.415269 |
| A_23_P215944 | CTSB | NM_147780 | chr8 | 3.5871096 |
| A_23_P155360 | HDAC 11 | NM_024827 | chr3 | 2.0750299 |
| A_23_P349416 | ERBB3 | NM_001982 | chr12 | 2.7191648 |
| A_24_P397928 | CTSB | NM_147780 | chr8 | 4.4394937 |
| | | | | |
| A_23_P110253 | KIT | NM_000222 | chr4 | -2.6171603 |
| A_24_P397107 | CDC25A | NM_001789 | chr3 | -1.698369 |
| A_32_P100379 | PDGFRA | AA599881 | chr4 | -1.9433503 |
| A_24_P56388 | HIF1A | NM_181054 | chr14 | -2.1315765 |
| A_23_P94533 | CTSL1 | NM_001912 | chr9 | -4.598218 |
| A_24_P313504 | PLK1 | NM_005030 | chr16 | -1.0595436 |
| A_23_P114783 | PARP1 | NM_001618 | chr1 | -1.6551466 |
| A_24_P205137 | HDAC6 | BC011498 | chrX | -1.5821996 |
| A_23_P209879 | ATF2 | AK128731 | chr2 | -1.3147001 |
| A_23_P110851 | TERT | NM_198253 | chr5 | -4.4801784 |
| A_24_P246467 | ATF2 | NM_001880 | chr2 | -1.2144108 |
| A_23_P208132 | BCL2 | M13995 | chr18 | -1.3138638 |
| A_23_P500271 | IRF5 | NM_002200 | chr7 | -1.5505834 |
| A_23_P118815 | BIRC5 | NM_001012271 | chr17 | -1.2690687 |
| A_23_P150609 | IGF2 | NM_001007139 | chr11 | -1.746458 |
| A_24_P916496 | PRKCA | NM_002737 | chr17 | -1.0500383 |
| A_23_P163027 | PARP2 | NM_005484 | chr14 | -1.1376743 |
| A_32_P183765 | ERBB4 | NM_005235 | chr2 | -6.158379 |
| A_23_P121423 | CDC25A | NM_001789 | chr3 | -1.4154968 |
| A_23_P118834 | TOP2A | NM_001067 | chr17 | -1.0636063 |
| A_24_P128145 | ATF2 | NM_001880 | chr2 | -1.4385839 |
| A_23_P63190 | NRAS | NM_002524 | chr1 | -1.0414906 |

(continued)

| [gc4_d] vc [Nfb2_17] | | | | |
|---|---|---|---|---|
| ProbeName | GeneSymbol | GenebankAccession | Chrom | Normalized |
| A_23_P417282 | IGF1R | NM_000875 | chr15 | -6.7087193 |
| A_23_P300033 | PDGFRA | NM_006206 | chr4 | -2.3364549 |
| A_23_P155969 | PLK4 | NM_014264 | chr4 | -1.4981489 |
| A_23_P130182 | AURKB | NM_004217 | chr17 | -1.8129711 |
| A_23_P122304 | HDAC2 | NM_001527 | chr6 | -1.103261 |
| A_24_P383478 | ESR1 | NM_000125 | chr6 | -1.1072159 |
| A_23_P20927 | TNKS | NM_003747 | chr8 | -1.1630383 |

To be specific, analysis was made for abnormal expression of tyrosine kinase genes or cancer drug target genes. The induced malignant stem cells analyzed in this Example can be considered as cells characterized both by abnormal expression of tyrosine kinase genes or cancer drug target genes and by expression of the ES cell-specific genes (OCT3/4, NANOG, SOX2, ZFP42).

Example 14: Detection for abnormal expression of endogenous microRNA in induced malignant stem cells

In this Example, (1) (d) abnormal expression (increased or reduced/lost expression) of non-coding RNAs such as endogenous cancer-related microRNAs in induced malignant stem cells was detected, in comparison with those in induced pluripotent stem cells or induced non-malignant stem cells.

(14-1) Materials

The (1) (d) abnormal expression (increased or reduced/lost expression) of non-coding RNAs such as endogenous cancer-related microRNAs in induced malignant stem cells was detected by genome-widely detecting expression of cancer-related miRNAs.

The following samples were used in the detection for (1) (d) abnormal expression (increased or reduced/lost expression) of non-coding RNAs such as endogenous cancer-related microRNAs in induced malignant stem cells:

- induced malignant stem cells (GC2_1, GC2_5, GC2_10) prepared from fresh gastric cancer tissues collected from the individual of donor No. 1;
- induced malignant stem cells (GC1_6) prepared from fresh gastric cancer tissues, and induced non-malignant stem cells (NGC1_7) prepared from fresh gastric non-cancer tissues , which were collected from the individual of donor No. 3;
- induced malignant stem cells (CC4_c) prepared from fresh colon cancer tissues collected from the individual of donor No. 5; and
- induced pluripotent stem cells (NFB2_17) prepared from the fibroblasts (7F3949) collected from the individual of donor No. 7.

(14-2) Experimental procedure

(14-2-1) Sample preparation

Total RNA was prepared from each of six induced malignant stem cells, one induced non-malignant stem cell, and one induced pluripotent stem cell as mentioned above, and the prepared total RNAs were used in Agilent SurePrint G3 Human miRNA Microarray Rel.16.0 to detect abnormal expression (increased or reduced/lost expression) of non-coding RNAs such as endogenous cancer-related microRNAs in the induced malignant stem cells.

(14-2-2) Quality evaluation

The process of quality evaluation of sample total RNA solutions involves the following evaluations to perform quality test:

- evaluation by the fluorescent detector Bioanalyzer (determination of electrophoresis patterns); and
- evaluation by the spectrophotometer NanoDrop (quantitation of total RNA amounts).

(14-2-3) Fluorescent labeling of miRNAs

The total RNAs prepared in (14-2-1) were fluorescently labeled using miRNA Complete Labeling Reagent and Hyb Kit in accordance with the Agilent protocol. To be specific, each sample total RNA (100 ng) was dephosphorylated with Calf Intestine Alkaline Phosphatase (CIP), and the RNA molecule on its 3' terminal side was labeled with a cyanine 3-cytidine bisphosphate (pCp-Cy3) molecule using T4 RNA Ligase.

(14-2-4) Hybridization

The RNA samples labeled in (14-2-3) were hybridized with SurePrint G3 Human miRNA Microarray Rel.16.0 using Agilent miRNA Complete Labeling Reagent and Hyb Kit. To be specific, each of the labeled RNAs was added to a hybridization buffer and allowed to hybridize on SurePrint G3 Human miRNA Microarray Rel.16.0 for 20 hours. After washing, the DNA microarray image was scanned by Agilent Microarray Scanner, and fluorescence signals from the spots were digitized by Feature Extraction Software (v.10.7.3.1).

(14-3) Experimental results

As a result of the quality evaluation of the RNA samples labeled in (14-2-3), the quality of all the samples was assured both by the determination of electrophoresis patterns and by the quantitation of total RNA amounts. Next, the total amounts of the resulting fluorescently-labeled RNAs were used for hybridization.

For the miRNAs shown in the cancer-related miRNA list, hybridization was performed using each of the following comprisons:

Nfb2-17 (control) vs GC1_6 vs NGC1_7 vs CC4_c vs GC2_1 vs GC2_5 vs GC2_10; and
NGC1_7 (control) vs GC1_6,

and then miRNA expressions were compared for investigation.

As a result of the image analysis performed after the hybridizations and washings, it was confirmed that the hybridizations had been performed without any problems. The images and digital data analyzed by Feature Extraction Software after the hybridization were stored on the storage media.

The miRNAs were annotated using miRBase Release 18, and hybridization was performed onto a microarray chip with the probes designed for the miRNAs shown in the following table (refer to Table 31 below).

[Table 31]

Table 31: List of cancer-related miRNAs investigated for abnormal expression

| miRBase or NCBI Accession No. | Mature miRNA ID or Gene Symbol |
| --- | --- |
| MIMAT0000062 | hsa-let-7a-5p |
| MIMAT0000770 | hsa-miR-133b |
| MIMAT0000421 | hsa-miR-122-5p |
| MIMAT0001413 | hsa-miR-20b-5p |
| MIMAT0000765 | hsa-miR-335-5p |
| MIMAT0000226 | hsa-miR-196a-5p |
| MIMAT0000443 | hsa-miR-125a-5p |
| MIMAT0000433 | hsa-miR-142-5p |
| MIMAT0000095 | hsa-miR-96-5p |
| MIMAT0000279 | hsa-miR-222-3p |
| MIMAT0000759 | hsa-miR-148b-3p |
| MIMAT0000092 | hsa-miR-92a-3p |

(continued)

| miRBase or NCBI Accession No. | Mature miRNA ID or Gene Symbol |
| --- | --- |
| MIMAT0000454 | hsa-miR-184 |
| MIMAT0000271 | hsa-miR-214-3p |
| MIMAT0000068 | hsa-miR-15a-5p |
| MIMAT0000732 | hsa-miR-378a-3p |
| MIMAT0000063 | hsa-let-7b-5p |
| MIMAT0000266 | hsa-miR-205-5p |
| MIMAT0000256 | hsa-miR-181a-5p |
| MIMAT0000425 | hsa-miR-130a-3p |
| MIMAT0000431 | hsa-miR-140-5p |
| MIMAT0000075 | hsa-miR-20a-5p |
| MIMAT0002809 | hsa-miR-146b-5p |
| MIMAT0000426 | hsa-miR-132-3p |
| MIMAT0002819 | hsa-miR-193b-3p |
| MIMAT0000261 | hsa-miR-183-5p |
| MIMAT0000686 | hsa-miR-34c-5p |
| MIMAT0000244 | hsa-miR-30c-5p |
| MIMAT0000243 | hsa-miR-148a-3p |
| MIMAT0000447 | hsa-miR-134 |
| MIMAT0000414 | hsa-let-7g-5p |
| MIMAT0000430 | hsa-miR-138-5p |
| MIMAT0000726 | hsa-miR-373-3p |
| MIMAT0000064 | hsa-let-7c |
| MIMAT0000066 | hsa-let-7e-5p |
| MIMAT0000275 | hsa-miR-218-5p |
| MIMAT0000100 | hsa-miR-29b-3p |
| MIMAT0000449 | hsa-miR-146a-5p |
| MIMAT0000758 | hsa-miR-135b-5p |
| MIMAT0000462 | hsa-miR-206 |
| MIMAT0000422 | hsa-miR-124-3p |
| MIMAT0000076 | hsa-miR-21-5p |
| MIMAT0002821 | hsa-miR-181d |
| MIMAT0000688 | hsa-miR-301a-3p |
| MIMAT0000617 | hsa-miR-200c-3p |
| MIMAT0000098 | hsa-miR-100-5p |
| MIMAT0000254 | hsa-miR-10b-5p |
| MIMAT0000646 | hsa-miR-155-5p |
| MIMAT0000416 | hsa-miR-1 |
| MIMAT0000451 | hsa-miR-150-5p |

(continued)

| miRBase or NCBI Accession No. | Mature miRNA ID or Gene Symbol |
|---|---|
| MIMAT0000415 | hsa-let-7i-5p |
| MIMAT0000419 | hsa-miR-27b-3p |
| MIMAT0000252 | hsa-miR-7-5p |
| MIMAT0004604 | hsa-miR-127-5p |
| MIMAT0000086 | hsa-miR-29a-3p |
| MIMAT0000440 | hsa-miR-191-5p |
| MIMAT0000065 | hsa-let-7d-5p |
| MIMAT0000441 | hsa-miR-9-5p |
| MIMAT0000067 | hsa-let-7f-5p |
| MIMAT0000253 | hsa-miR-10a-5p |
| MIMAT0000257 | hsa-miR-181b-5p |
| MIMAT0000417 | hsa-miR-15b-5p |
| MIMAT0000069 | hsa-miR-16-5p |
| MIMAT0000267 | hsa-miR-210 |
| MIMAT0000070 | hsa-miR-17-5p |
| MIMAT0000096 | hsa-miR-98-5p |
| MIMAT0000255 | hsa-miR-34a-5p |
| MIMAT0000081 | hsa-miR-25-3p |
| MIMAT0000436 | hsa-miR-144-3p |
| MIMAT0000424 | hsa-miR-128 |
| MIMAT0000435 | hsa-miR-143-3p |
| MIMAT0000272 | hsa-miR-215 |
| MIMAT0000073 | hsa-miR-19a-3p |
| MIMAT0004614 | hsa-miR-193a-5p |
| MIMAT0000072 | hsa-miR-18a-5p |
| MIMAT0000423 | hsa-miR-125b-5p |
| MIMAT0000445 | hsa-miR-126-3p |
| MIMAT0000084 | hsa-miR-27a-3p |
| MIMAT0000724 | hsa-miR-372 |
| MIMAT0000450 | hsa-miR-149-5p |
| MIMAT0000418 | hsa-miR-23b-3p |
| MIMAT0000264 | hsa-miR-203a |
| MIMAT0000090 | hsa-miR-32-5p |
| MIMAT0000258 | hsa-miR-181c-5p |

In this analysis, the normalized value of the digital data for the hybridization of a subject cell (induced malignant stem cell) sample was divided by the normalized value from the digital data for the hybridization of a control cell sample and the quotient was used as a measure of variation in expression. Probes for which the quotients deviated from 1 (i.e., $\log_2 1 = 0$) were considered to indicate a variation in expression; those showing quotients greater than 2 ($\log_2 2 = 1$) or

smaller than 0.5 (log$_2$0.5 = -1) were selected. For each of the comparisons shown below, the genes shown in the tyrosine kinase list and those shown in the cancer drug targets list were analyzed to make respective lists of the genes showing quotients greater than 2 (log$_2$2 = 1) or smaller than 0.5 (log$_2$0.5 = -1), as compared with the quotient for the control (taken as 1). The analysis was performed using GeneSpring 12.1.

The results are shown in Table 32 below. The Normalized column represents a variation in expression in logarithmic values.

[Table 32]

| Table 32: List of cancer-related miRNAs that exhibited change in microRNA expression | | | | |
|---|---|---|---|---|
| [gc1_6] vs [Nfb2_17] | | | | |
| systematic_name | chr | mirbase accession No | Normalized | active_sequence |
| hsa-let-7i-5p | chr12 | MIMAT0000415 | 1.4402232 | AACAGCACAAACTACTACCTC |
| hsa-miR-124-3p | chr8 | MIMAT0000422 | 1.8801897 | GGCATTCACCGCGTGC |
| hsa-miR-134 | chr14 | MIMAT0000447 | 2.88347 | CCCCTCTGGTCAA |
| hsa-miR-200c-3p | chr12 | MIMAT0000617 | 2.0046523 | TCCATCATTACCCGG |
| hsa-miR-210 | chr11 | MIMAT0000267 | 2.03550687 | TCAGCCGCTGTCACAC |
|  |  |  |  |  |
| hsa-miR-372 | chr19 | MIMAT0000724 | -1.3848338 | ACGCTCAAATGTCGCAGC |
| hsa-miR-373-3p | chr19 | MIMAT0000726 | -1.0936382 | ACACCCCAAAATCGAAGC |
|  |  |  |  |  |
|  |  |  |  |  |
| [Ngc1_7] vs [Nfb2_17] | | | | |
| systematic_name | chr | mirbase accession No | Normalized | active_sequence |
| hsa-let-7a-5p | chr9 | MIMAT0000062 | 1.9411173 | AACTATACAACCTACTACCT |
| hsa-let-7c | chr21 | MIMAT0000064 | 2.650042 | AACCATACAACCTACTACC |
| hsa-let-7e-5p | chr19 | MIMAT0000066 | 3.3564239 | AACTATACAACCTCCTACC |
| hsa-let-7f-5p | chr9 | MIMAT0000067 | 2.2589548 | AACTATACACAATCTACTACCTC |
| hsa-let-7i-5p | chr12 | MIMAT00000415 | 2.7469358 | AACAGCACAAACTACTACCTC |
| hsa-miR-124-3p | chr8 | MIMAT00000422 | 1.2417734 | GGCATTCACCGCGTGC |
| hsa-miR-125b-5p | chr11 | MIMAT00000423 | 1.9817171 | TCACAAGTTAGGGTCTC |
| hsa-miR-134 | chr14 | MIMAT00000447 | 2.736024 | CCCCTCTGGTCAA |
| hsa-miR-200c-3p | chr12 | MIMAT00000617 | 1.5896351 | TCCATCATTACCCGG |
| hsa-miR-21-5p | chr17 | MIMAT0000076 | 1.1359663 | TCAACATCAGTCTGATAAGC |
| hsa-miR-210 | chr11 | MIMAT00000267 | 1.642601 | TCAGCCGCTAGTCACAC |
| hsa-miR-23b-3p | chr9 | MIMAT00000418 | 1.1542001 | GGTAATCCCTGGCAATG |
| hsa-miR-27a-3p | chr19 | MIMAT0000084 | 1.0835547 | GCGGAACTTAGCCACTG |
|  |  |  |  |  |
| hsa-miR-205-5p | chr1 | MIMAT0000266 | -1.3146505 | CAGACTCCGGTGGAAT |
| hsa-miR-372 | chr19 | MIMAT0000724 | -3.3881433 | ACGCTCAAATGTCGCAGC |
| hsa-miR-373-3p | chr19 | MIMAT00000726 | -2.8184555 | ACACCCCAAAATCGAAGC |
|  |  |  |  |  |

(continued)

| [cc4_c] vs [Nfb2_17] | | | | |
|---|---|---|---|---|
| systematic_name | chr | mirbase accession No | Normalized | active_sequence |
| hsa-let-7a-5p | chr9 | MIMAT0000062 | 5.025595 | AACTATACAACCTACTACCT |
| hsa-let-7c | chr21 | MIMAT0000064 | 4.789489 | AACCATACAACCTACTACC |
| hsa-let-7e-5p | chr19 | MIMAT0000066 | 4.7925954 | AACTATACAACCTCCTACC |
| hsa-let-7f-5p | chr9 | MIMAT0000067 | 5.5231466 | AACTATACAATCTACTACCTC |
| hsa-let-7i-5p | chr12 | MIMAT0000415 | 4.0543323 | AACAGCACAAACTACTACCTC |
| hsa-miR-125a-5p | chr19 | MIMAT0000443 | 1.8090844 | TCACAGGTTAAAGGGTCTC |
| hsa-miR-125b-5p | chr11 | MIMAT0000423 | 2.9458146 | TCACAAGTTAGGGTCTC |
| hsa-miR-15a-5p | chr13 | MIMAT0000068 | 1.1630578 | CACAAACCATTATGTGCTGCT |
| hsa-miR-183-5p | chr7 | MIMAT0000261 | 1.2848263 | AGTGAATTCTACCAGTGCCA |
| hsa-miR-200c-3p | chr12 | MIMAT0000617 | 4.535967 | TCCATCATTACCCGG |
| hsa-miR-21-5p | chr17 | MIMAT0000676 | 2.9038973 | TCAACATCAGTCTGATAAGC |
| hsa-miR-210 | chr11 | MIMAT0000267 | 3.795586 | TCAGCCGCTGTCACAC |
| hsa-miR-222-3p | chrX | MIMAT0000279 | 1.8606999 | ACCCAGTAGCCAG |
| hsa-miR-23b-3p | chr9 | MIMAT0000418 | 3.2681499 | GGTAATCCCTGGCAATG |
| hsa-miR-27a-3p | chr19 | MIMAT0000084 | 3.7551851 | GCGGAACTTAGCCACTG |
| hsa-miK-27b-3p | chr9 | MIMAT0000419 | 2.5687003 | GCAGAACTTAGCCACTGT |
| hsa-miR-29a-3p | chr7 | MIMAT0000086 | 3.972498 | TAACCGATTTCAGATGGTGC |
| hsa-miR-29b-3p | chr1 | MIMAT0000100 | 4.44877906 | AACACTGATTTCAAATGGTGC |
| hsa-miR-34c-5p | chr11 | MIMAT0000686 | 1.3082383 | GCAATCAGCTAACTACACTG |
| hsa-miR-7-5p | chr9 | MIMAT0000252 | 2.2134001 | ACAACAAAATCACTAGTCTTCC |
| hsa-miR-96-5p | chr7 | MIMAT0000095 | 1.0930905 | AGCAAAAATGTGCTAGTGCCAA |
| | | | | |
| hsa-miR-130a-3p | chr11 | MIMAT0000425 | -4.732003 | ATGCCCTTTTAACATTGCA |
| hsa-miR-18a-5p | chr13 | MIMAT0000072 | -1.4401569 | CTATCTGCACTAGATGCA |
| hsa-miR-20b-5p | chrX | MIMAT0001413 | -3.2925744 | CTACCTGCACTATGAGCAC |
| hsa-miR-301a-3p | chr17 | MIMAT0000688 | -1.2117661 | GCTTTGACAATACTATTGCAC |
| | | | | |
| [gc2_1] vs [Nfb2_17] | | | | |
| systematic_name | chr | mirbase accession No | Normalized | active_sequence |
| hsa-let-7e-5p | chr19 | MIMAT0000066 | 1.6011363 | AACTATACAACCTCCTACC |
| hsa-miR-134 | chr14 | MIMAT0000447 | 1.8531758 | CCCCTCTGGTCAA |
| | | | | |
| hsa-let-7a-5p | chr9 | MIMAT0000062 | -1.233491 | AACTATACAACCTACTACCT |
| hsa-miR-125b-5p | chr11 | MIMAT0000423 | -1.4247212 | TCACAAGTTAGGGTCTC |
| hsa-miR-130a-3p | chr11 | MIMAT0000425 | -5.267291 | ATGCCCTTTTAACATTGCA |
| hsa-miR-16-5p | chr3 | MIMAT0000069 | -5.227989 | CGCCAATATTTACGTGCTG |
| hsa-miR-17-5p | chr13 | MIMAT0000070 | -3.190412 | CTACCTGCACTGTAAGC |

(continued)

| [gc2_1] vs [Nfb2_17] | | | | |
|---|---|---|---|---|
| systematic_name | chr | mirbase accession No | Normalized | active_sequence |
| hsa-miR-200c-3p | chr12 | MIMAT0000617 | -1.3934362 | TCCATCATTACCCGG |
| hsa-miR-205-5p | chr1 | MIMAT0000266 | -1.5770755 | CAGACTCCGGTGGAAT |
| hsa-miR-20a-5p | chr13 | MIMAT0000075 | -4.7841616 | CTACCTGCACTATAAGCAC |
| hsa-miR-20b-5p | chrX | MIMAT0001413 | -3.1519804 | CTACCTGCACTATGAGCAC |
| hsa-miR-21-5p | chr17 | MIMAT0000076 | -6.310871 | TCAACATCAGTCTGATAAGC |
| hsa-miR-25-3p | chr7 | MIMAT0000081 | -1.8991919 | TCAGACCGAGACAAGTGC |
| hsa-miR-27b-3p | chr9 | MIMAT0000419 | -2.5748281 | GCAGAACTTAGCCACTGT |
| hsa-miR-34a-5p | chr1 | MIMAT0000255 | -2.6079917 | ACAACCAGCTAAGACACTGC |
| | | | | |
| [gc2_1] vs [Nfb2_17] | | | | |
| systematic_name | chr | mirbase accession No | Normalized | active_sequence |
| hsa-miR-134 | chr14 | MIMAT0000447 | 1.2985052 | CCCCTCTGGTCAA |
| systematic_name | | | | |
| hsa-let-7a-5p | chr9 | MIMAT0000062 | -1.3341377 | AACTATACAACCTACTACCT |
| hsa-miR-125a-5p | chr19 | MIMAT0000443 | -1.1494529 | TCACAGGTTAAAGGGTCTC |
| hsa-miR-125b-5p | chr11 | MIMAT0000423 | -2.410604 | TCACAAGTTAGGGTCTC |
| hsa-miR-130a-3p | chr11 | MIMAT0000425 | -5.014146 | ATGCCCTTTTAACATTGCA |
| hsa-miR-16-5p | chr3 | MIMAT0000069 | -4.81643 | CGCCAATATTTACGTGCTG |
| hsa-miR-17-5p | chr13 | MIMAT0000070 | -3.465736 | CTACCTGCACTGTAAGC |
| hsa-miR-205-5p | chr1 | MIMAT0000266 | -2.340828 | CAGACTCCGGTGGAAT |
| hsa-miR-20a-5p | chr13 | MIMAT0000075 | -4.9512577 | CTACCTGCACTATAAGCAC |
| hsa-miR-20b-5p | chrX | MIMAT0001413 | -3.2693667 | CTACCTGCACTATGAGCAC |
| hsa-miR-21-5p | chr17 | MIMAT0000076 | -6.380228 | TCAACATCAGTCTGATAAGC |
| hsa-miR-210 | chr11 | MIMAT0000267 | -1.6277646 | TCAGCCGCTGTCACAC |
| hsa-miR-25-3p | chr7 | MIMAT0000081 | -1.4775734 | TCAGACCGAGACAAGTGC |
| hsa-miR-34a-5p | chr1 | MIMAT0000255 | -2.8327525 | ACAACCAGCTAAGACACTGC |
| hsa-miR-373-3p | chr19 | MIMAT0000726 | -2.9304783 | ACACCCCAAAATCGAAGC |
| | | | | |
| [gc2_1] vs [Nfb2_17] | | | | |
| systematic_name | chr | mirbase accession No | Normalized | active_sequence |
| hsa-let-7e-5p | chr19 | MIMAT0000066 | 2.1798716 | AACTATACAACCTCCTACC |
| hsa-miR-134 | chr14 | MIMAT0000447 | 2.8293352 | CCCCTCTGGTCAA |
| hsa-miR-193b-3p | chr16 | MIMAT0002819 | 1.0982502 | AGCGGGACTTTGAGGG |
| | | | | |
| hsa-miR-130a-3p | chr11 | MIMAT0000425 | -5.0291395 | ATGCCCTTTTAACATTGCA |
| hsa-miR-16-5p | chr3 | MIMAT0000069 | -4.9559364 | CGCCAATATTTACGTGCTG |
| hsa-miR-17-5p | chr13 | MIMAT0000070 | -3.6190734 | CTACCTGCACTGTAAGC |

(continued)

| [gc2_1] vs [Nfb2_17] | | | | |
|---|---|---|---|---|
| systematic_name | chr | mirbase accession No | Normalized | active_sequence |
| hsa-miR-183-5p | chr7 | MIMAT0000261 | -1.8860053 | AGTGAATTCTACCAGTGCCA |
| hsa-miR-200c-3p | chr12 | MIMAT0000617 | -1.7152803 | TCCATCATTACCCGG |
| hsa-miR-205-5p | chr1 | MIMAT0000266 | -2.908281 | CAGACTCCGGTGGAAT |
| hsa-miR-20a-5p | chr13 | MIMAT0000075 | -5.1203127 | CTACCTGCACTATAAGCAC |
| hsa-miR-20b-5p | chrX | MIMAT0001413 | -3.5017633 | CTACCTGCACTATGAGCAC |
| hsa-miR-21-5p | chr17 | MIMAT0000076 | -5.843651 | TCAACATCAGTCTGATAAGC |
| hsa-miR-25-3p | chr7 | MIMAT0000081 | -1.5153098 | TCAGACCGAGACAAGTGC |
| hsa-miR-27b-3p | chr9 | MIMAT0000419 | -2.2972195 | GCAGAACTTAGCCACTGT |
| hsa-miR-29a-3p | chr7 | MIMAT0000086 | -3.5608726 | TAACCGATTTCAGATGGTGC |
| hsa-miR-34a-5p | chr1 | MIMAT0000255 | -2.705739 | ACAACCAGCTAAGACACTGC |
| hsa-miR-373-3p | chr19 | MIMAT0000726 | -3.2015352 | ACACCCCAAAATCGAAGC |
| | | | | |
| [gc1_6] vs [Ngc1_1] | | | | |
| systematic_sequence | chr | mirbase accession No | Normalized | active_sequence |
| hsa-miR-372 | chr19 | MIMAT0000724 | 2.0033095 | ACGCTCAAATGTCGCAGC |
| hsa-miR-373-3p | chr19 | MIMAT0000726 | 1.7248173 | ACACCCCAAAATCGAAGC |
| | | | | |
| hsa-let-7a-5p | chr9 | MIMAT0000062 | -1.0900092 | AACTATACAACCTACTACCT |
| hsa-let-7b-5p | chr22 | MIMAT0000063 | -1.9368484 | AACCACACAACCTACTACC |
| hsa-let-7c | chr21 | MIMAT0000064 | -1.800859 | AACCATACAACCTACTACC |
| hsa-let-7d-5p | chr9 | MIMAT0000065 | -1.5442965 | AACTATGCAACCTACTACC |
| hsa-let-7e-5p | chr19 | MIMAT0000066 | -2.6838303 | AACTATACAACCTCCTACC |
| hsa-let-7f-5p | chr9 | MIMAT0000067 | -1.4209971 | AACTATACAATCTACTACCTC |
| hsa-let-7g-5p | chr3 | MIMAT0000414 | -1.935283 | AACTGTACAAACTACTACCTC |
| hsa-let-7i-5p | chr12 | MIMAT0000415 | -1.3067126 | AACAGCACAAACTACTACCTC |
| hsa-miR-125a-5p | chr19 | MIMAT0000443 | -1.3733852 | TCACAGGTTAAAGGGTCTC |
| hsa-miR-125b-5p | chr11 | MIMAT0000423 | -1.3929782 | TCACAAGTTAGGGTCTC |
| hsa-miR-23b-3p | chr9 | MIMAT0000418 | -1.014039 | GGTAATCCCTGGCAATG |
| hsa-miR-27a-3p | chr19 | MIMAT0000084 | -1.0465813 | GCGGAACTTAGCCACTG |

The induced malignant stem cells analyzed in this Example can be considered as cells characterized both by abnormal expression of cancer-related miRNAs and by expression of the ES cell-specific genes (OCT3/4, NANOG, SOX2, ZFP42).

Example 15: Detection for abnormal expression of endogenous cancer-related proteins in induced malignant stem cells

In this Example, (1) (e) abnormal expression (increased or reduced/lost expression) of endogenous cancer-related proteins in induced malignant stem cells was detected, in comparison with those in cell populations derived from colon non-cancer site tissues.

(15-1) Materials

The (1) (e) abnormal expression (increased or reduced/lost expression) of endogenous cancer-related proteins in induced malignant stem cells was detected using the protein identification analysis (iTRAQ labeling) technique.

The following samples were used in the detection for (1) (e) abnormal expression (increased or reduced/lost expression) of endogenous cancer-related proteins in induced malignant stem cells:

- cell population (ncc4) derived from colon non-cancer site tissues, cell population (cc4) derived from fresh colon cancer site tissues, and induced malignant stem cells (CC4_D) prepared from fresh colon cancer tissues , which were collected from the individual of donor No. 5.

(15-2) Experimental procedure

In order to detect abnormal expression (increased or reduced/lost expression) of endogenous cancer-related proteins in induced malignant stem cells, protein identification analysis (iTRAQ (isobaric Tags for Relative and Absolute Quantitation) labeling) was performed.

(15-2-1) Summary

ProteinPilot is a software that is capable of effectively identifying and quantitating proteins by searching for genetic variants and modifications, etc. simultaneously with the aid of automatic extensive peptide identification (using the Paragon algorithm). This software was used to perform experimentation with unused filters using Protein Summary_control ncc4 (colon non-cancer tissues as a negative control).

In this test, the proteins present in the respective samples were identified and the relative quantitative ratios of cc4 or cc4-d with respect to ncc4 were determined.

(15-2-2) Sample preparation

The tissues and cells were prepared and then disrupted to prepare protein-containing samples for mass spectrometry. In this process of the protein preparation, buffer replacement was performed because the buffer might contain any substances that might interfere with trypsin and/or other iTRAQ labeling reagents. The buffer was replaced with 50 mM of TEAB using the ultrafiltration cartridge (Spin Concentrators, 5K MWCO, 4 mL, 25 (P/N5185-5991); Agilent Technologies).

The concentrations of the proteins contained in the obtained samples were measured using Pierce BCA Protein Assay Kit (Pierce), and the samples were so adjusted as to give the same concentrations. For each sample, 100 $\mu$g of proteins were treated by in-solution digestion and subjected to trypsin digestion, S-S bond cleavage, and -SH-group protection (alkylation) with methylmethanethiosulfonate (MMTS); thereafter, for each sample, the peptides were iTRAQ-labeled with different iTRAQ reagents using AB SCIEX iTRAQ Reagent-8Plex Kit and were mixed to obtain a sample for mass spectrometry. Afterwards, the sample was purified on the cation exchange column AB SCIEX CEX and then fractionated into eight fractions.

Next, the resulting iTRAQ-labeled sample was separated and concentrated by liquid chromatography using the DiNa system (nano-LC; KYA TECH Corp.). In this process, the sample was spotted onto a plate while being fractionated using a reverse phase column.

Fractionation time: 115 minutes

Spotting time: Starting from 15 minutes and ending at 110 minutes, at intervals of 1 spot/30 sec, giving a total of 191 separated spots.

[Table 33]

| Table 33: Gradient conditions | | |
|---|---|---|
| Time (min) | Buffer A | Buffer B |
| 0.1 | 100.0 | 0.0 |
| 12.0 | 95.0 | 5.0 |
| 75.0 | 50.0 | 50.0 |

(continued)

| Table 33: Gradient conditions | | |
|---|---|---|
| Time (min) | Buffer A | Buffer B |
| 90.0 | 0.0 | 100.0 |
| 100.0 | 100.0 | 0.0 |
| 115.0 | 100.0 | 0.0 |

- Buffer A: 2% acetonitrile, 0.1 % TFA
- Buffer B: 70% acetonitrile, 0.1% TFA
- Matrix: α-cyano-4-hydroxycinnamic acid (CHCA) in 70% acetonitrile and 0.1 % TFA

The prepared sample was subjected to mass spectrometry by MALDI TOF/MS using the mass spectrometer 4800 MALDI-TOF/TOF Analyzer (AB SCIEX). The obtained data was searched against Homo sapiens entries in the NCBI database using the Paragon algorithm as a search algorithm in the data analysis software ProteinPilot v4.0.

(15-2) Experimental results

The mass spectrometry results are shown below. As a result of this analysis, 328 proteins were identified with a confidence of greater than 2.0 (99), 445 proteins with a confidence of greater than 1.3 (95), 552 proteins with a confidence of greater than 0.47 (66), and 1173 proteins with a confidence (cutoff applied) of greater than 0.05 (10%).

For each of these proteins, the relative ratio of the protein amount in CC4-D to that in ncc4 and the relative ratio of the protein amount in cc4 to that in ncc4 were analyzed in detail to detect increased or reduced expression, or loss. In the table that follows, the deep (red) colored ratios represent that the protein amount in the test cells of interest increased as compared with that in the test cells (ncc4).

[Table 34]

Table 34:   Results of the comparisons between cc4-d and ncc4 and between cc4 and ncc4

EP 2 749 642 A1

| Proteins identified | | | | | | Relative quantity (test vs control) | | | | | |
|---|---|---|---|---|---|---|---|---|---|---|---|
| No | Unused | Accession | Name | Species | Peptides (95%) | Ratio cc4-d /ncc4 | PVal cc4-d /ncc4 | EF cc4-d /ncc4 | Ratio cc4 /ncc4 | PVal cc4 /ncc4 | EF cc4 /ncc4 |
| 1 | 123.32 | gi\|260268505 | FLJ00343 protein [Homo sapiens] | Homo sapiens | | 0.560032 | 5.36E-10 | 1.168248 | 0.504615 | 3.20E-13 | 1.156442 |
| 2 | 101.89 | gi\|62897671 | beta actin variant [Homo sapiens] | Homo sapiens | 83 | 0.946325 | 0.87032 | 2.208678 | 0.983919 | 0.933201 | 1.574453 |
| 3 | 74.82 | gi\|49256423 | Keratin 8 [Homo sapiens] | Homo sapiens | 49 | 1.613629 | 0.000207 | 1.255066 | 0.792461 | 0.001592 | 1.144895 |
| 4 | 67.04 | gi\|2804273 | alpha actinin 4 [Homo sapiens] | Homo sapiens | 38 | 1.062203 | 0.565599 | 1.239879 | 0.806067 | 0.000347 | 1.110951 |
| 5 | 58.17 | gi\|62897129 | heat shock 70kDa protein 8 isoform 1 variant [Homo sapiens] | Homo sapiens | 33 | 1.527137 | 1.59E-05 | 1.16716 | 1.086241 | 0.26813 | 1.163893 |
| 6 | 55.07 | gi\|153792590 | heat shock protein HSP 90-alpha isoform 1 [Homo sapiens] | Homo sapiens | 28 | 1.239452 | 0.034761 | 1.217399 | 1.192639 | 0.060753 | 1.203626 |
| 7 | 51.12 | gi\|47077229 | unnamed protein product [Homo sapiens] | Homo sapiens | 27 | 0.38024 | 1.33E-06 | 1.345993 | 0.350468 | 3.47E-07 | 1.341462 |
| 8 | 49.24 | gi\|73909156 | Annexin A2 [Homo sapiens] | Homo sapiens | 26 | 0.847976 | 0.00914 | 1.127112 | 0.795586 | 0.000248 | 1.114798 |
| 9 | 45.98 | gi\|386758 | GRP78 precursor, partial [Homo sapiens] | Homo sapiens | 29 | 1.131177 | 0.096346 | 1.15835 | 1.062354 | 0.235124 | 1.107954 |
| 10 | 44.66 | gi\|62897747 | keratin 18 variant [Homo sapiens] | Homo sapiens | 26 | 1.860548 | 0.009871 | 1.57499 | 0.912151 | 0.323955 | 1.208845 |
| 11 | 44.40 | gi\|31645 | glyceraldehyde-3-phosphate dehydrogenase [Homo sapiens] | Homo sapiens | 34 | 1.144378 | 0.150923 | 1.207561 | 1.006087 | 0.939007 | 1.178002 |
| 12 | 44.07 | gi\|62897945 | enolase 1 variant [Homo sapiens] | Homo sapiens | 25 | 1.012062 | 0.870375 | 1.161622 | 1.198625 | 0.006784 | 1.134798 |
| 13 | 42.40 | gi\|4757900 | calreticulin precursor [Homo sapiens] | Homo sapiens | 23 | 1.007391 | 0.912039 | 1.147609 | 0.983405 | 0.851475 | 1.202654 |
| 14 | 41.42 | gi\|119598292 | pyruvate kinase, muscle, isoform CRA_c [Homo sapiens] | Homo sapiens | 24 | 1.251887 | 0.003167 | 1.150005 | 1.081263 | 0.218509 | 1.136878 |
| 15 | 41.25 | gi\|61656607 | tumor rejection antigen (gp96) 1 [Homo sapiens] | Homo sapiens | 20 | 0.847624 | 0.003619 | 1.111043 | 1.098374 | 0.047552 | 1.097166 |
| 16 | 39.20 | gi\|4502027 | serum albumin preproprotein [Homo sapiens] | Homo sapiens | 28 | 0.454498 | 1.01E-06 | 1.268322 | 0.611886 | 4.85E-06 | 1.180226 |
| 17 | 37.95 | gi\|4504349 | hemoglobin subunit beta [Homo sapiens] | Homo sapiens | 25 | 0.481127 | 0.000291 | 1.400245 | 1.495818 | 0.016659 | 1.376216 |

161

| 18 | 37.91 | gi\|21757045 | unnamed protein product [Homo sapiens] | Homo sapiens | 22 | 0.4933 | 7.27E-06 | 1.278554 | 0.605778 | 5.06E-05 | 1.225898 |
|---|---|---|---|---|---|---|---|---|---|---|---|
| 19 | 35.89 | gi\|301611275 | PREDICTED: tubulin alpha-1A chain isoform 2 [Xenopus (Silurana) tropicalis] | Homo sapiens | 22 | 0.981931 | 0.985605 | 28116.6 | 1.128998 | 0.8435 | 466.0424 |
| 20 | 34.65 | gi\|35655 | unnamed protein product [Homo sapiens] | Homo sapiens | 15 | 1.012729 | 0.877944 | 1.183162 | 1.001908 | 0.978969 | 1.159252 |
| 21 | 33.35 | gi\|77702086 | heat shock protein 60 [Homo sapiens] | Homo sapiens | 17 | 0.941231 | 0.597426 | 1.264821 | 1.189914 | 0.042775 | 1.182581 |
| 22 | 32.58 | gi\|189053448 | unnamed protein product [Homo sapiens] | Homo sapiens | 18 | 1.297549 | 0.006781 | 1.195314 | 0.779176 | 0.014091 | 1.212236 |
| 23 | 32.05 | gi\|194391174 | unnamed protein product [Homo sapiens] | Homo sapiens | 15 | 1.455006 | 0.005367 | 1.266299 | 1.050007 | 0.513229 | 1.173959 |
| 24 | 31.76 | gi\|23958133 | Tubulin, beta 2C [Homo sapiens] | Homo sapiens | 18 | 0.701878 | 0.377246 | 20.6639 | 0.977554 | 0.937242 | 18.47744 |
| 25 | 30.95 | gi\|2245365 | ER-60 protein [Homo sapiens] | Homo sapiens | 16 | 1.058279 | 0.50226 | 1.193981 | 1.408656 | 6.89E-05 | 1.137767 |
| 26 | 30.55 | gi\|62089306 | spectrin, alpha, non-erythrocytic 1 (alpha-fodrin) variant [Homo sapiens] | Homo sapiens | 13 | 1.017545 | 0.851399 | 1.207047 | 0.871579 | 0.037076 | 1.13725 |
| 27 | 29.43 | gi\|119581485 | peptidylprolyl isomerase A-like, isoform CRA_c [Homo sapiens] | Homo sapiens | 18 | 1.063778 | 0.299234 | 1.131461 | 0.933235 | 0.466704 | 1.220235 |
| 28 | 29.14 | gi\|55743098 | collagen alpha-3 (VI) chain isoform 1 precursor [Homo sapiens] | Homo sapiens | 17 | 0.547746 | 0.001464 | 1.398174 | 0.548646 | 0.001245 | 1.387925 |
| 29 | 28.43 | gi\|180570 | creatine kinase [Homo sapiens] | Homo sapiens | 18 | 1.093389 | 0.332036 | 1.209965 | 0.901443 | 0.278849 | 1.218414 |
| 30 | 28.05 | gi\|158259881 | unnamed protein product [Homo sapiens] | Homo sapiens | 16 | 0.940473 | 0.662761 | 1.36697 | 1.011293 | 0.939808 | 1.39423 |
| 31 | 27.90 | gi\|62897565 | transgelin variant [Homo sapiens] | Homo sapiens | 20 | 0.387216 | 4.84E-05 | 1.432545 | 0.29626 | 6.49E-07 | 1.372638 |
| 32 | 27.56 | gi\|226529917 | triosephosphate isomerase isoform 2 [Homo sapiens] | Homo sapiens | 15 | 0.962377 | 0.720446 | 1.254188 | 0.850908 | 0.069175 | 1.192507 |
| 33 | 27.46 | gi\|74746925 | RecName: Full=Putative elongation factor 1-alpha-like 3; Short=EF-1-alpha-like 3; AltName: Full=Eukaryotic elongation factor 1 A-like 3; Short=eEF1A-like 3; AltName: Full=Eukaryotic translation elongation factor 1 alpha-1 pseudogene 5 | HUMAN | 16 | 1.073135 | 0.392697 | 1.190802 | 1.075938 | 0.283745 | 1.153672 |

| 34 | 27.05 | gi\|386803 | 40-kDa keratin protein, partial [Homo sapiens] | Homo sapiens | 18 | 1.342861 | 0.113725 | 1.460768 | 0.823584 | 0.121749 | 1.291432 |
|---|---|---|---|---|---|---|---|---|---|---|---|
| 35 | 26.69 | gi\|260099723 | L-lactate dehydrogenase A chain isoform 3 [Homo sapiens] | Homo sapiens | 17 | 1.107067 | 0.444627 | 1.319651 | 1.184717 | 0.054884 | 1.189564 |
| 36 | 26.53 | gi\|179279 | ATP synthase beta subunit [Homo sapiens] | Homo sapiens | 14 | | | | | | |
| 37 | 25.44 | gi\|61743954 | neuroblast differentiation-associated protein AHNAK isoform 1 [Homo sapiens] | HUMAN | 15 | 0.794159 | 0.059871 | 1.272589 | 0.658111 | 5.88E-06 | 1.151681 |
| 38 | 24.43 | gi\|2204207 | glutathione S-transferase [Homo sapiens] | Homo sapiens | 17 | 1.861575 | 0.010512 | 1.547622 | 0.796859 | 0.191598 | 1.438775 |
| 39 | 23.89 | gi\|48145549 | PGK1 [Homo sapiens] | Homo sapiens | 12 | 1.224599 | 0.010918 | 1.160336 | 1.136932 | 0.066544 | 1.148238 |
| 40 | 23.30 | gi\|27529744 | KIAA0866 protein [Homo sapiens] | Homo sapiens | 13 | 0.469884 | 0.000181 | 1.351449 | 0.435387 | 1.69E-05 | 1.288141 |
| 41 | 22.94 | gi\|30354619 | YWHAZ protein, partial [Homo sapiens] | Homo sapiens | 12 | 1.174357 | 0.342181 | 1.452179 | 0.852079 | 0.194032 | 1.301555 |
| 42 | 22.58 | gi\|194379168 | unnamed protein product [Homo sapiens] | Homo sapiens | 29 | 0.774576 | 0.001067 | 1.140885 | 0.65126 | 0.000151 | 1.19206 |
| 43 | 22.00 | gi\|49457147 | LGALS3 [Homo sapiens] | Homo sapiens | 13 | 1.402313 | 0.004855 | 1.229034 | 0.757581 | 0.026011 | 1.26619 |
| 44 | 21.40 | gi\|62898077 | tropomyosin 2 (beta) isoform 2 variant [Homo sapiens] | Homo sapiens | 23 | 0.540962 | 0.02233 | 1.660436 | 0.427952 | 0.003685 | 1.652215 |
| 45 | 21.20 | gi\|342187211 | fructose-bisphosphate aldolase A isoform 2 [Homo sapiens] | Homo sapiens | 13 | 1.126492 | 0.277932 | 1.252891 | 0.962078 | 0.56841 | 1.151721 |
| 46 | 20.37 | gi\|20149594 | heat shock protein HSP 90-beta [Homo sapiens] | Homo sapiens | 26 | 1.044394 | 0.938238 | 3.380513 | 1.161247 | 0.438581 | 1.511139 |

This analysis revealed that the induced malignant stem cells showed increased expression of the following cancer-related proteins:

- keratin 8 [Homo sapiens],
- keratin 18 variant [Homo sapiens],
- glutathione S-transferase [Homo sapiens],
- heat shock 70kDa protein 8 isoform 1 variant [Homo sapiens], and
- LGALS3 [Homo sapiens].

The induced malignant stem cells analyzed in this Example can be considered as cells characterized both by abnormal expression of cancer-related proteins and by expression of the ES cell-specific genes (OCT3/4, NANOG, SOX2, ZFP42).

Example 16: Detection of an aberration of endogenous cancer-related metabolisms in induced malignant stem cells

In this Example, (1)(f) an aberration of endogenous cancer-related metabolisms (hypermetabolism or hypometabolism) in induced malignant stem cells were detected, in comparison with those in induced pluripotent stem cells.

(16-1) Materials

The (1)(f) aberration of endogenous cancer-related metabolism (hypermetabolism or hypometabolism) in induced malignant stem cells were detected by measuring different intracellular metabolites by capillary electrophoresis-time of flight mass spectrometry (CE-TOFMS) in cation or anion mode.

The following samples were used in the detection for (1)(f) the aberration of endogenous cancer-related metabolism in induced malignant stem cells:

- induced malignant stem cells (CC1_2, CC1_7) prepared from fresh colon cancer tissues collected from the individual of donor No. 4;
- induced malignant stem cells (CC4_c, CC4_D) prepared from fresh colon cancer tissues collected from the individual of donor No. 5; and
- induced pluripotent stem cells (NFB1_4) prepared from fibroblasts (7F3956) collected from the individual of donor No. 6.

All of these cells were proliferated to about 80% confluence in 10 cm culture dishes.

(16-2) Experimental procedure

(16-2-1) Summary

The four induced malignant stem cell samples (CC1-2, CC1-7, CC4-c, CC4-d) as well as the induced pluripotent stem cells (nfb1-4) were measured by capillary electrophoresis-time of flight mass spectrometry (CE-TOFMS) in cation or anion mode. In this Example, analysis was made using substances registered in HMT's (Human Metabolome Technologies) metabolite library and "known-unknown" peak library as subject substandces.

(16-2-2) Procedure

The four induced malignant stem cell samples (CC1-2, CC1-7, CC4-c, CC4-d) as well as the induced pluripotent stem cells (nfb1-4) were subjected to culture. The samples were washed and collected following the protocol given below, and were then subjected to detection.

(i) Description of instruments used

- Internal standard solution 1 (10 mM; HMT)
- Methanol (Wako Pure Chemical; for LC/MS, Cat No. 134-14523)
- Volumetric flask (Iwaki Glass; TE-32, 50 mL)
- Mannitol (Wako Pure Chemical; Cat No. 133-00845)

- Milli-Q water (prepared by the Millipore Milli-Q water purification system)
- Cell Scraper (Sumitomo Bakelite; 250 mm × 17 mm, MS-93170)
- Centrifuge turbes (Falcon Blue Max Jr., 15 mL, Cat No. 352097)
- Micropipettes and microchips (Eppendorf)
- Automatic pipetter and pipettes (Nunc; 5 mL 159625, 10 mL 159633)

(ii) Sample preparation

(ii-1) Preparation of a methanol solution for metabolite extraction Instrument and reagents used

- Internal standard solution 1 (10 mM, HMT)
- Methanol (Wako Pure Chemical; for LC/MS, Cat No. 134-14523)
- Volumetric flask (Iwaki Glass; TE-32, 50 mL)

Internal standard solution 1 was diluted 1000-fold with methanol to prepare a methanol solution for metabolite extraction. In this prepration process, the volumetric flask containing the standard solution was filled up to the calibration mark with the diluent so that mixing was done well. The methanol solution for metabolite extraction was prepared at the time of use.

(ii-2) Preparation of a cell washing solution

Instrument and reagents used

- Mannitol (Wako Pure Chemical; Cat No. 133-00845)
- Milli-Q water (produced by the Millipore Milli-Q water purification system)

A 5% (w/w) mannitol solution was prepared using mannitol and Milli-Q water. The cell washing solution was used in an amount of about 15 mL per dish.

(iii) Washing

(iii-1) A cell culture medium was aspirated off, and then the surface of the culture dish was washed with 10 mL of the cell washing solution *1. The cell washing solution used was at room temperature.
(iii-2) After removing the cell washing solution, washing was done again with 2 mL of the cell washing solution. The cell washing solution used in the second washing was aspirated off from the edge of the culture dish with care*2.

*1 The cell washing solution used was the 5% (w/w) mannitol solution prepared in "(ii-2) Preparation of a cell washing solution."
*2 The cell washing solution contained a very high concentration of mannitol, which might be precipitated during a metabolite extraction step. Thus, the cell washing solution was aspirated off carefully to reduce the amount of residual mannitol as much as possible.

(iv) Dissociation

Instrument and reagents used

- Cell Scraper (Sumitomo Bakelite; 250 mm × 17 mm, MS-93170)

(iv-1) After the cell washing solution was aspirated off, 1.3 mL of the methanol solution for metabolite extraction* was added to the culture dish. The methanol solution used was at room temperature.
(iv-2) The culture dish was rocked gently such that its entire surface was covered with the methanol solution.
(iv-3) Cells were dissociated using a cell scraper so as to prevent cell masses from being broken up, and the released cells were gathered in one place. The cell dissociation step was performed carefully because the results might vary if cells remained on the culture dish or were dissociated inadequately.

* The methanol solution prepared in "(ii-1) Preparation of a methanol solution for metabolite extraction" was used.

(v) Collection

- Centrifuge turbes (Falcon Blue Max Jr., 15 mL, Cat No. 352097)

(v-1) After the completion of the cell dissociation step, 1.0 mL of the methanol solution for metabolite extraction was aspirated from the culture dish and transferred to the 15 mL centrifuge tube.
(v-2) The cell masses remaining in the culture dish were gathered and transferred to the 15 mL centrifuge tube.
(v-3) The 15 mL centrifuge tube containing the methanol solution and the cell masses was stored at 80°C.

One thousand microliters of chloroform and 400 $\mu$L of Milli-Q water were added to the resulting sample, and the suspension was stirred and centrifuged (2,300$\times$g, 4°C, 5 min). After the completion of the centrifugation, 400 $\mu$L each of the aqueous phase was transferred to two ultrafiltration tubes (Millipore; Ultrafree-MC PLHCC HMT centrifugal filter unit, 5 kDa). The aqueous phase was centrifuged (9,100$\times$g, 4°C, 120 min) and subjected to ultrafiltration. The filtrate was evaporated to dryness and then dissolved again in 25 $\mu$L of Milli-Q water before being subjected to measurement.

(16-2-3) Measurement

In this test, measurements were performed in cation and anion modes under the conditions 1) and 2) shown below. Judging from the resulting peak strength and shape, the 5- and 10-fold diluted samples were used in the measurements in cation and anion modes, respectively.
1) Cationic metabolites (cation mode)

- Apparatus

Agilent CE-TOFMS system (Agilent Technologies)
Capillary: Fused silica capillary, 50 $\mu$m i.d. $\times$ 80 cm

- Measurement conditions

Run buffer: Cation buffer solution (p/n: H3301-1001)
Rinse buffer: Cation buffer solution (p/n: H3301-1001)
Sample injection: Pressure injection, 50 mbar, 10 sec.
CE voltage: Positive, 27 kV
MS ionization: ESI positive
MS capillary voltage: 4,000 V
MS scan range: *m/z* 50-1,000
Sheath liquid: HMT sheath liquid (p/n: H3301-1020)

2) Anionic metabolites (anion mode)

- Apparatus

Agilent CE-TOFMS system (Agilent Technologies) #1
Capillary: Fused silica capillary, 50 $\mu$m i.d. $\times$ 80 cm

- Measurement conditions

Run buffer: Anion buffer solution (p/n: H3302-1021)
Rinse buffer: Anion buffer solution (p/n: H3302-1022)
Sample injection: Pressure injection, 50 mbar, 25 sec.
CE voltage: Positive, 30 kV
MS ionization: ESI negative

MS capillary voltage: 3,500 V
MS scan range: *m/z* 50-1,000
Sheath liquid: HMT sheath liquid (p/n: H3301-1020)

(16-3) Data processing and analysis

(16-3-1) Data processing

The peaks detected by CE-TOFMS were automatically extracted using the automatic integration software MasterHands ver. 2.9.0.9 (developed by Keio University) to obtain the peak data, i.e., mass-to-charge ratio (*m/z*), migration time (Migrationtime; MT), and peak area. The obtained peak area was converted into a relative area using the following equation:

$$\text{Relative area} = \text{Peak Area of interest} / \text{Area of internal reference material.}$$

These data sets contained the data for adduct ions such as $Na^+$ and $K^+$, and that for fragment ions generated by dehydration, deammoniation and other factors, and thus the data for these molecular weight-related ions was excluded. However, not the data for all of such ions could be precisely screened because substance-specific adducts and fragments were also present. The precisely screened peaks were subjected to checking and sorting among samples based on the *m/z* and MT values.

(16-3-2) Search for candidate metabolites

The detected peaks were searched and checked against all the substances registered in HMT's metabolite library and "known-unknown" library based on the *m/z* and MT values. The acceptable error for searching were as follows: MT: $\pm 0.5$ min; *m/z*: $\pm 10$ ppm (mass error (ppm) = (measured value - theoretical value) / measured value $\times 10^6$).

(16-3-3) Statistical analyses (PCA, HCA)

Principal component analysis (PCA) was made using SampleStat ver.3.14 (developed by HMT). Hierarchical cluster analysis (HCA) and Heatmap visualization were performed using PeakStat ver.3.18 (developed by HMT). As for the clustering results, reference can be made to the separately attached excel file, in which all candidate compounds can be confirmed. In the process of both of these analyses, standardization ($\mu=0$, $\sigma=1$) of each peak was performed as a data preprocessing step.

(16-3-4) Drawing of a metabolic pathway

The quantitative data of metabolite was drawn on metabolic pathway maps. The metabolic pathways were drawn using VANTED 4 (Visualization and Analysis of Networks containing Experimental Data). Some of the abbreviated metabolite names used in the drawing were different from those registered in HMT's compound databases. The metabolic pathways were constructed based on the enzymes found in humans (not shown).

(16-4) Results

(16-4-1) Search for candidate metabolites

The five cultured cells were subjected to metabolomic analysis by CE-TOFMS. On the basis of the *m/z* and MT values of the substances registered in HMT's metabolite library and "known-unknown" library, 201 peaks (102 for cationic peaks, and 99 for anionic peaks) were assigned to candidate compounds (Table 35). As for these candidate compounds, reference can be made to the separately attached Excel file.
[Table 35]

Table 35:

| HMT DB [†] Compound name | m/z | MT | Relative area cc1-2 | cc1-7 | cc4-c | cc4-d | nfb1-4 |
|---|---|---|---|---|---|---|---|
| Propionic acid | 73.029 | 10.77 | N.D. | N.D. | 1.6E-02 | 1.7E-02 | N.D. |
| Pyruvic acid | 87.009 | 12.75 | 1.0E-01 | N.D. | 7.0E-02 | 9.8E-02 | 7.2E-02 |
| Isobutyric acid | 87.045 | 9.71 | 2.5E-02 | 3.4E-02 | 2.6E-02 | 2.5E-02 | 1.8E-02 |
| Lactic acid | 89.025 | 10.70 | 3.7E+01 | 1.9E+01 | 1.4E+01 | 3.1E+01 | 1.9E+01 |
| Isovaleric acid | 101.061 | 9.11 | 3.0E-02 | 5.0E-02 | 3.3E-02 | 3.9E-02 | 2.9E-02 |
| 3-Hydroxybutyric acid | 103.040 | 9.41 | 4.6E-02 | 2.1E-02 | 3.0E-01 | 1.4E-01 | 2.0E-02 |
| 2-Hydroxybutyric acid | 103.041 | 9.64 | 1.8E-02 | N.D. | 9.3E-03 | N.D. | N.D. |
| Glyceric acid | 105.020 | 10.23 | 3.4E-02 | 2.4E-02 | N.D. | 7.4E-03 | 1.6E-02 |
| XA0002 | 110.985 | 15.39 | 1.6E-01 | 2.0E-01 | N.D. | N.D. | 1.2E-01 |
| Fumaric acid | 115.003 | 24.57 | 2.2E-01 | 3.7E-01 | 1.4E-01 | 1.7E-01 | 2.9E-01 |
| 2-Oxoisovaleric acid | 115.040 | 10.05 | 7.3E-02 | 4.2E-02 | 7.3E-02 | 1.3E-01 | 5.8E-02 |
| Hexanoic acid | 115.076 | 8.79 | N.D. | 3.0E-02 | 1.3E-02 | 1.2E-02 | 1.3E-02 |
| Succinic acid | 117.019 | 20.47 | 3.1E-01 | 4.0E-01 | 1.5E-01 | 2.4E-01 | 2.5E-01 |
| 2-Hydroxyvaleric acid | 117.055 | 9.12 | N.D. | N.D. | 2.7E-02 | 4.4E-02 | N.D. |
| Benzoic acid | 121.030 | 9.70 | 4.5E-02 | 1.5E-02 | N.D. | N.D. | 1.0E-02 |
| Isethionic acid | 124.990 | 11.48 | 5.0E-02 | N.D. | N.D. | N.D. | N.D. |
| 5-Oxoproline | 128.035 | 9.36 | 7.7E-01 | 8.7E-01 | 3.5E-01 | 1.4E-01 | 7.5E-01 |
| 4-Methyl-2-oxovaleric acid 3-Methyl-2-oxovaleric acid | 129.055 | 9.44 | 4.2E-01 | 2.4E-01 | 3.5E-01 | 5.4E-01 | 2.7E-01 |
| Heptanoic acid | 129.091 | 8.45 | 1.1E-02 | N.D. | N.D. | N.D. | 9.5E-03 |
| N-Acetylalanine | 130.050 | 8.77 | 4.2E-02 | 2.4E-02 | 2.5E-02 | 1.2E-02 | 2.4E-02 |
| 2-Hydroxy-4-methylvaleric acid | 131.072 | 8.77 | N.D. | N.D. | N.D. | 1.0E-02 | N.D. |
| Malic acid | 133.014 | 20.77 | 2.5E+00 | 3.7E+00 | 1.5E+00 | 1.8E+00 | 2.8E+00 |
| Threonic acid | 135.030 | 9.12 | 2.2E-01 | 3.0E-01 | 2.6E-01 | 2.3E-01 | 2.1E-01 |
| p-Toluic acid | 135.045 | 9.03 | 8.0E-02 | 1.2E-01 | 6.7E-02 | 5.8E-02 | 7.8E-02 |
| Ethanolamine phosphate | 140.012 | 7.92 | 7.2E-03 | N.D. | 1.4E-01 | 1.4E-01 | N.D. |
| Octanoic acid | 143.108 | 8.26 | 1.8E-02 | 1.3E-02 | N.D. | 8.9E-03 | 1.1E-02 |
| 2-Oxoglutaric acid | 145.013 | 20.84 | 3.9E-01 | 2.6E-01 | 1.2E-01 | 3.1E-01 | 2.7E-01 |
| 2-Oxoglutaric acid | 145.013 | 20.84 | 3.9E-01 | 2.6E-01 | 1.2E-01 | 3.1E-01 | 2.7E-01 |
| N-Acetylserine | 146.045 | 8.70 | 1.2E+01 | 1.3E+01 | 1.2E+01 | 7.8E+00 | 1.1E+01 |
| 2-Hydroxyglutaric acid | 147.030 | 16.61 | 1.5E-01 | 1.4E-01 | 2.4E-02 | 2.5E-02 | 8.8E-02 |
| Xanthine | 151.026 | 8.30 | N.D. | N.D. | 3.5E-02 | 1.6E-02 | N.D. |
| Cysteinesulfinic acid | 152.002 | 9.40 | 2.7E-02 | 1.3E-01 | N.D. | N.D. | 9.7E-02 |
| Pelargonic acid | 157.123 | 8.03 | 3.4E-02 | 2.8E-02 | 2.2E-02 | 2.4E-02 | 2.4E-02 |
| 3-Hydroxy-3-methylglutaric acid | 161.046 | 15.16 | N.D. | 2.0E-02 | N.D. | N.D. | N.D. |
| Phosphoenolpyruvic acid | 166.974 | 20.55 | 9.1E-02 | 5.5E-02 | N.D. | 2.0E-02 | 4.0E-02 |
| Glyceraldehyde 3-phosphate | 168.990 | 11.44 | 2.1E-02 | 3.9E-02 | N.D. | 1.1E-02 | 1.4E-02 |
| Dihydroxyacetone phosphate | 168.990 | 12.42 | 4.7E-01 | 2.7E-01 | 2.5E-01 | 3.0E-01 | 3.1E-01 |
| Glycerol 3-phosphate | 171.006 | 11.92 | 1.4E-01 | 4.6E-02 | 5.2E-02 | 1.0E-01 | 4.5E-02 |
| XA0013 | 172.991 | 10.71 | 1.3E-02 | N.D. | 9.3E-03 | 1.4E-02 | 7.7E-03 |
| cis-Aconitic acid | 173.008 | 27.05 | 4.2E-02 | N.D. | 1.1E-01 | 1.0E-01 | N.D. |
| N-Acetylaspartic acid | 174.040 | 14.28 | 7.2E-01 | 4.3E-01 | N.D. | N.D. | 6.6E-01 |
| N-Carbamoylaspartic acid | 175.036 | 15.17 | 1.8E-02 | 3.4E-02 | N.D. | 1.4E-02 | 2.4E-02 |
| O-Phosphoserine | 184.001 | 12.05 | 2.1E-02 | 4.3E-02 | N.D. | N.D. | 1.7E-02 |
| 3-Phosphoglyceric acid | 184.985 | 19.24 | 1.7E-01 | 1.2E-01 | 8.3E-02 | 1.0E-01 | 1.0E-01 |
| 2-Phosphoglyceric acid | 184.985 | 18.79 | 2.6E-02 | 2.3E-02 | N.D. | N.D. | N.D. |
| N-Acetylglutamic acid | 188.055 | 12.95 | 3.6E-02 | 3.0E-02 | N.D. | N.D. | 3.6E-02 |
| N-Acetylmethionine | 190.055 | 8.03 | 2.2E-02 | 1.5E-02 | N.D. | N.D. | 1.2E-02 |

| | | | | | | | |
|---|---|---|---|---|---|---|---|
| Isocitric acid | 191.018 | 27.68 | 2.3E-02 | N.D. | 8.2E-02 | 8.2E-02 | N.D. |
| XA0019 | 191.019 | 8.23 | 3.4E-01 | 4.3E-01 | 2.3E-01 | 1.1E-01 | 3.6E-01 |
| Citric acid | 191.019 | 25.42 | 7.6E-01 | 2.6E-01 | 2.4E+00 | 2.4E+00 | 2.5E-01 |
| Gluconic acid | 195.050 | 8.11 | 7.6E-01 | 2.3E-01 | 5.6E-02 | 5.7E-02 | 4.7E-01 |
| Erythrose 4-phosphate | 199.002 | 11.62 | N.D. | 3.2E-02 | N.D. | N.D. | N.D. |
| Lauric acid | 199.170 | 7.62 | 6.0E-02 | 6.0E-02 | 5.5E-02 | 6.3E-02 | 4.1E-02 |
| Mucic acid | 209.031 | 13.80 | N.D. | N.D. | 1.8E-02 | 1.3E-02 | 9.6E-03 |
| Phosphocreatine | 210.028 | 12.03 | 1.4E-01 | 7.6E-02 | N.D. | N.D. | 4.7E-02 |
| O-Succinylhomoserine | 218.066 | 11.99 | 2.0E-02 | 1.1E-02 | N.D. | 7.4E-03 | 1.1E-02 |
| Pantothenic acid | 218.102 | 7.63 | 4.0E-01 | 2.6E-01 | 3.9E-01 | 1.7E-01 | 2.1E-01 |
| XA0027 | 227.200 | 7.38 | N.D. | N.D. | N.D. | 1.1E-02 | N.D. |
| Ribulose 5-phosphate | 229.011 | 10.78 | 4.2E-02 | 4.4E-02 | 2.6E-02 | 3.3E-02 | 3.1E-02 |
| Ribose 5-phosphate | 229.011 | 10.40 | 1.6E-02 | 2.1E-02 | 1.1E-02 | 1.2E-02 | 1.3E-02 |
| XA0033 | 242.079 | 7.61 | 1.6E+00 | 2.0E+00 | 2.9E+00 | 2.9E+00 | 1.8E+00 |
| Biotin | 243.082 | 7.60 | 9.6E-02 | 1.4E-01 | 1.8E-01 | 2.0E-01 | 1.3E-01 |
| myo-Inositol 1-phosphate myo-Inositol 3-phosphate | 259.019 | 10.12 | 1.4E-02 | N.D. | N.D. | N.D. | 6.4E-03 |
| Glucose 1-phosphate | 259.021 | 9.94 | 7.7E-02 | 6.0E-02 | 8.2E-02 | 5.8E-02 | 4.8E-02 |
| Glucose 6-phosphate | 259.021 | 9.69 | 1.0E-01 | 3.9E-02 | 2.2E-01 | 2.1E-01 | 3.0E-02 |
| Fructose 6-phosphate | 259.021 | 9.81 | 4.9E-02 | 3.2E-02 | 4.9E-02 | 5.3E-02 | 1.9E-02 |
| 2,3-Diphosphoglyceric acid | 264.951 | 18.09 | 3.8E-02 | N.D. | N.D. | 1.2E-02 | 3.5E-02 |
| 6-Phosphogluconic acid | 275.016 | 14.36 | N.D. | N.D. | N.D. | 1.8E-02 | N.D. |
| Sedoheptulose 7-phosphate | 289.032 | 9.50 | 2.2E-02 | 3.5E-02 | 2.3E-02 | 2.4E-02 | 3.5E-02 |
| N-Acetylglucosamine 1-phosphate | 300.047 | 9.42 | 3.0E-02 | 1.8E-02 | 2.0E-02 | 2.0E-02 | 2.0E-02 |
| N-Acetylneuraminic acid | 308.097 | 7.14 | 7.9E-03 | 8.6E-03 | 1.1E-01 | 1.3E-01 | 6.9E-03 |
| CMP | 322.043 | 9.47 | N.D. | N.D. | N.D. | 3.7E-03 | N.D. |
| UMP | 323.026 | 9.65 | 6.7E-03 | 6.5E-03 | 1.1E-02 | 1.1E-02 | 5.2E-03 |
| Fructose 1,6-diphosphate | 338.987 | 14.28 | 7.8E-01 | 4.4E-01 | 3.4E-01 | 4.6E-01 | 3.8E-01 |
| AMP | 346.054 | 9.15 | 3.8E-02 | 3.3E-02 | 3.2E-02 | 3.4E-02 | 2.4E-02 |
| IMP | 347.036 | 9.43 | N.D. | N.D. | N.D. | 4.3E-03 | N.D. |
| GMP | 362.048 | 9.03 | 1.1E-02 | 1.3E-02 | 5.2E-03 | 6.8E-03 | 7.4E-03 |
| XA0055 | 368.998 | 14.02 | 4.5E-02 | N.D. | 3.1E-02 | 4.5E-02 | 2.5E-02 |
| NADPH_divalent | 371.537 | 11.13 | 4.9E-02 | 3.4E-02 | 5.0E-02 | 3.3E-02 | 3.1E-02 |
| PRPP | 388.943 | 16.50 | 3.3E-02 | N.D. | 2.8E-02 | 2.4E-02 | 2.1E-02 |
| FAD_divalent | 391.569 | 7.78 | N.D. | N.D. | 7.4E-03 | 9.2E-03 | 3.5E-03 |
| CDP | 402.008 | 11.30 | 7.2E-03 | 9.4E-03 | N.D. | 5.2E-03 | 6.8E-03 |
| UDP | 402.994 | 11.47 | 4.1E-02 | 4.2E-02 | 2.0E-02 | 2.3E-02 | 3.0E-02 |
| ADP | 426.021 | 10.73 | 3.4E-01 | 2.5E-01 | 2.2E-01 | 2.4E-01 | 2.0E-01 |
| GDP | 442.016 | 10.52 | 9.2E-02 | 7.3E-02 | 5.1E-02 | 4.6E-02 | 6.3E-02 |
| XA0065 | 445.052 | 6.88 | N.D. | N.D. | 3.5E-02 | 1.8E-02 | N.D. |
| dTTP | 480.980 | 11.85 | 2.0E-02 | 1.4E-02 | N.D. | N.D. | 1.6E-02 |
| CTP | 481.975 | 12.11 | 1.6E-01 | 1.6E-01 | 2.0E-01 | 9.3E-02 | 1.5E-01 |
| UTP | 482.960 | 12.32 | 9.0E-01 | 7.3E-01 | 9.0E-01 | 5.4E-01 | 7.8E-01 |
| ATP | 505.987 | 11.61 | 5.6E+00 | 3.1E+00 | 3.6E+00 | 2.8E+00 | 3.2E+00 |
| GTP | 521.983 | 11.26 | 4.6E-01 | 2.8E-01 | 2.9E-01 | 1.7E-01 | 3.0E-01 |
| UDP-glucose UDP-galactose | 565.047 | 8.52 | 4.9E-01 | 4.6E-01 | 5.6E-01 | 3.5E-01 | 4.4E-01 |
| UDP-glucuronic acid | 579.026 | 10.85 | 1.6E-01 | 1.5E-01 | 1.3E-01 | 1.0E-01 | 1.0E-01 |
| ADP-glucose GDP-fucose | 588.074 | 8.23 | 1.2E-02 | N.D. | 2.0E-02 | 1.7E-02 | 6.9E-03 |
| GDP-glucose | 604.069 | 8.22 | 6.9E-03 | N.D. | 7.4E-03 | 6.4E-03 | N.D. |
| UDP-N-acetylgalactosamine | 606.074 | 8.34 | 6.8E-01 | 5.3E-01 | 7.6E-01 | 3.5E-01 | 5.8E-01 |
| CMP-N-acetylneuraminate | 613.139 | 8.08 | N.D. | N.D. | 1.4E-01 | 9.8E-02 | N.D. |
| NAD$^+$ | 662.102 | 6.52 | 1.5E-01 | 9.7E-02 | 2.0E-01 | 1.8E-01 | 1.0E-01 |

| | | | | | | | |
|---|---|---|---|---|---|---|---|
| NADH | 664.118 | 8.09 | 3.0E-02 | 3.6E-02 | 2.3E-02 | 2.0E-02 | 1.7E-02 |
| NADP$^+$ | 742.069 | 9.26 | N.D. | N.D. | 6.4E-03 | N.D. | N.D. |
| Urea | 61.040 | 20.20 | 9.4E-01 | 6.9E-01 | N.D. | N.D. | 9.9E-01 |
| Ethanolamine | 62.061 | 6.35 | N.D. | N.D. | 2.6E-02 | 2.9E-02 | N.D. |
| Isobutylamine | 74.097 | 7.02 | 1.5E-02 | 1.7E-02 | 1.6E-02 | 1.7E-02 | 1.1E-02 |
| Gly | 76.039 | 8.25 | 7.2E+00 | 8.8E+00 | 1.6E+01 | 8.7E+00 | 6.4E+00 |
| Putrescine | 89.107 | 4.71 | N.D. | N.D. | 1.7E-01 | 1.4E-01 | N.D. |
| β-Ala | 90.055 | 7.32 | 3.1E-01 | 2.3E-01 | 5.0E-01 | 2.3E-01 | 2.6E-01 |
| Ala | 90.055 | 8.94 | 1.4E+01 | 1.0E+01 | 1.9E+01 | 9.0E+00 | 8.4E+00 |
| 1-Aminocyclopropane-1-carboxylic acid Homoserinelactone | 102.055 | 7.09 | 6.6E-03 | 9.5E-03 | N.D. | 4.4E-03 | 7.8E-03 |
| 3-Aminoisobutyric acid | 104.070 | 7.82 | N.D. | N.D. | 1.3E-01 | N.D. | N.D. |
| N-Ethylglycine | 104.071 | 9.76 | N.D. | N.D. | N.D. | N.D. | 3.7E-02 |
| 2-Aminobutyric acid | 104.071 | 9.52 | N.D. | 6.3E-02 | 1.1E-02 | N.D. | N.D. |
| Choline | 104.107 | 6.85 | 9.5E-01 | 1.5E+00 | 1.2E+00 | 2.9E+00 | 1.1E+00 |
| GABA | 105.073 | 7.58 | 2.5E+00 | 1.4E+00 | 4.4E-02 | 3.9E-02 | 1.5E+00 |
| Ser | 106.049 | 9.88 | 2.6E+00 | 3.3E+00 | 2.0E+00 | 2.7E+00 | 2.4E+00 |
| Diethanolamine | 106.086 | 7.63 | N.D. | N.D. | 7.4E-03 | 1.9E-02 | N.D. |
| Hypotaurine | 110.027 | 17.50 | 5.5E-01 | 1.8E+00 | 4.0E-02 | 2.4E-02 | 9.9E-01 |
| 1-Pyrroline 5-carboxylic acid | 114.055 | 10.94 | 7.9E-03 | 2.8E-02 | 2.8E-02 | 2.7E-02 | 3.2E-02 |
| Pro | 117.073 | 10.66 | 6.7E-01 | 4.7E-01 | 1.3E+00 | 1.0E+00 | 4.1E-01 |
| Guanidoacetic acid | 118.061 | 8.19 | 8.5E-02 | 3.2E-02 | 1.0E-02 | 4.2E-02 | 9.6E-03 |
| 5-Aminovaleric acid | 118.086 | 8.02 | 2.8E-02 | 3.4E-02 | 3.6E-02 | 3.0E-02 | 3.1E-02 |
| Val | 118.086 | 9.92 | 5.2E+00 | 4.5E+00 | 1.1E+01 | 1.1E+01 | 4.0E+00 |
| Betaine | 118.086 | 11.13 | 8.3E-02 | 1.1E-01 | 3.5E+00 | 8.2E+00 | 9.9E-02 |
| Thr | 120.065 | 10.39 | 5.1E+00 | 6.0E+00 | 1.7E+01 | 1.1E+01 | 3.7E+00 |
| Homoserine | 120.066 | 9.99 | 1.1E-02 | N.D. | N.D. | N.D. | N.D. |
| Betaine aldehyde_+H$_2$O | 120.101 | 7.44 | N.D. | N.D. | 3.5E-03 | 1.0E-02 | 2.1E-03 |
| Cys | 122.026 | 11.17 | 3.5E-03 | N.D. | N.D. | N.D. | N.D. |
| 2-Amino-2-(hydroxymethyl)-1,3-propanediol | 122.081 | 8.21 | N.D. | N.D. | 2.9E-03 | 7.5E-03 | N.D. |
| Nicotinamide | 123.055 | 7.37 | 1.1E-02 | 1.1E-02 | 2.9E-02 | 3.2E-02 | 1.1E-02 |
| Imidazole-4-acetic acid | 127.049 | 8.00 | 2.2E-02 | 1.5E-02 | 1.3E-02 | 1.2E-02 | 1.4E-02 |
| XC0016 | 129.065 | 8.73 | 4.1E-01 | 3.5E-01 | 3.1E-01 | 3.5E-01 | 2.0E-01 |
| Pipecolic acid | 130.086 | 10.18 | 5.3E-02 | 4.7E-02 | N.D. | N.D. | 6.0E-02 |
| trans-Glutaconic acid | 131.033 | 21.78 | 2.5E-02 | 3.3E-02 | N.D. | N.D. | 2.4E-02 |
| N-Acetylputrescine | 131.117 | 8.39 | N.D. | N.D. | 1.7E-02 | 6.4E-03 | N.D. |
| Hydroxyproline | 132.065 | 11.84 | 3.3E-02 | 3.0E-02 | 1.4E+00 | 7.1E-01 | 3.4E-02 |
| Creatine | 132.076 | 8.81 | 6.3E-01 | 2.6E-01 | 6.3E-03 | 5.6E-03 | 1.5E-01 |
| Leu | 132.101 | 10.21 | 5.2E+00 | 4.8E+00 | 8.4E-01 | 2.3E+00 | 4.3E+00 |
| Ile | 132.101 | 10.10 | 5.1E+00 | 4.0E+00 | 1.6E+01 | 1.6E+01 | 3.7E+00 |
| Asn | 133.060 | 10.35 | 2.5E+00 | 2.2E+00 | 7.4E-01 | 3.4E-01 | 1.3E+00 |
| Gly-Gly | 133.059 | 8.34 | 5.6E-02 | N.D. | N.D. | 8.5E-03 | N.D. |
| Ornithine | 133.096 | 6.81 | 1.3E+00 | 1.0E+00 | 1.5E-01 | 1.3E-01 | 1.1E+00 |
| Thiaproline | 134.027 | 13.66 | 1.6E-01 | 1.7E-01 | 8.4E-02 | 8.0E-02 | 1.1E-01 |
| Asp | 134.044 | 11.39 | 1.7E+00 | 3.2E+00 | 3.1E+00 | 1.9E+00 | 2.0E+00 |
| Adenine | 136.061 | 7.54 | 9.3E-03 | N.D. | 9.0E-03 | 7.1E-03 | 8.4E-03 |
| 1-Methylnicotinamide | 137.071 | 7.33 | 1.1E-02 | 1.5E-01 | 5.3E-02 | 2.0E-02 | 1.2E-02 |
| 4-Guanidinobutyric acid | 146.092 | 8.22 | 2.3E-01 | 5.5E-02 | 1.3E-02 | 1.7E-02 | 5.9E-02 |
| Acetylcholine | 146.117 | 7.60 | N.D. | N.D. | 1.5E-02 | 4.7E-02 | N.D. |
| γ-Butyrobetaine | 146.117 | 8.06 | 3.6E-02 | 1.2E-02 | 1.1E-01 | 5.3E-02 | 1.5E-02 |
| Spermidine | 146.164 | 4.54 | 1.3E-02 | 2.1E-02 | 4.5E-02 | 9.3E-02 | 8.8E-03 |
| Gln | 147.076 | 10.60 | 1.7E+01 | 1.2E+01 | 6.3E+00 | 4.4E+00 | 8.7E+00 |
| Lys | 147.112 | 6.87 | 2.2E+00 | 2.0E+00 | 1.4E-01 | 6.2E-01 | 2.1E+00 |

| | | | | | | | |
|---|---|---|---|---|---|---|---|
| Glu | 148.060 | 10.76 | 2.2E+01 | 2.8E+01 | 2.4E+01 | 1.7E+01 | 2.2E+01 |
| Met | 150.058 | 10.58 | 3.7E-01 | 5.6E-01 | 3.1E-01 | 4.0E-01 | 5.0E-01 |
| His | 156.076 | 7.27 | 1.2E+00 | 1.1E+00 | 2.3E+00 | 1.9E+00 | 9.4E-01 |
| Imidazolelactic acid | 157.061 | 8.78 | N.D. | N.D. | 9.3E-03 | 1.7E-02 | N.D. |
| 2-Aminoadipic acid | 162.075 | 10.75 | 1.4E-01 | 3.5E-01 | 1.6E-02 | 7.3E-02 | 1.8E-01 |
| Carnitine | 162.111 | 8.46 | N.D. | N.D. | 1.2E-02 | 6.6E-03 | N.D. |
| Methionine sulfoxide | 166.053 | 11.60 | 3.9E-02 | 4.2E-02 | N.D. | 1.9E-02 | 3.0E-02 |
| Phe | 166.086 | 10.94 | 3.6E+00 | 3.2E+00 | 9.8E+00 | 8.1E+00 | 2.8E+00 |
| Pyridoxal | 168.065 | 8.73 | 2.3E-02 | 1.7E-02 | 3.0E-02 | 3.4E-02 | 1.6E-02 |
| Pyridoxine | 170.081 | 8.60 | 6.3E-02 | 5.1E-02 | 2.3E-02 | 3.6E-02 | 4.3E-02 |
| Metronidazole | 172.072 | 9.39 | 8.3E-03 | N.D. | 4.0E-03 | N.D. | 4.8E-03 |
| $N$-Acetylornithine | 175.107 | 9.44 | 2.9E-02 | N.D. | N.D. | N.D. | 7.0E-03 |
| Arg | 175.118 | 7.10 | 1.3E+00 | 1.9E+00 | 7.2E-01 | 1.7E+00 | 1.7E+00 |
| Citrulline | 176.102 | 10.89 | 5.2E-03 | N.D. | 1.9E-02 | 3.8E-02 | 5.7E-03 |
| Gluconolactone | 179.054 | 21.73 | 2.4E-01 | 8.7E-02 | 2.5E-02 | 2.5E-02 | 1.8E-01 |
| Tyr | 182.080 | 11.20 | 2.2E+00 | 1.8E+00 | 5.4E-01 | 6.8E-01 | 1.6E+00 |
| Phosphorylcholine | 184.072 | 19.68 | 7.6E+00 | 1.2E+01 | 1.5E+01 | 1.6E+01 | 1.1E+01 |
| $N^8$-Acetylspermidine | 188.175 | 6.34 | N.D. | N.D. | 9.2E-02 | 5.8E-02 | N.D. |
| $N$-Acetyllysine | 189.122 | 9.70 | 2.7E-02 | N.D. | N.D. | N.D. | 8.8E-03 |
| $N^6$-Acetyllysine | 189.122 | 11.27 | 5.2E-02 | 7.6E-03 | 5.9E-03 | 3.6E-03 | 1.6E-02 |
| $N^6,N^6,N^6$-Trimethyllysine | 189.159 | 7.17 | 3.7E-02 | 9.8E-03 | 1.2E-02 | 4.7E-03 | 1.9E-02 |
| Gly-Asp | 191.065 | 9.78 | 6.9E-02 | 3.9E-02 | 3.9E-02 | 2.7E-02 | 3.6E-02 |
| $N$-Acetylhistidine | 198.089 | 9.71 | 7.4E-03 | N.D. | N.D. | N.D. | N.D. |
| Spermine | 203.222 | 4.48 | 3.3E-02 | 1.8E-02 | 7.9E-03 | 1.8E-02 | 1.5E-02 |
| $O$-Acetylcarnitine | 204.122 | 8.92 | N.D. | N.D. | 2.1E-02 | 2.2E-02 | N.D. |
| Trp | 205.096 | 10.87 | 4.2E-02 | 2.0E-01 | 2.2E-01 | 4.0E-01 | 1.6E-01 |
| Kynurenine | 209.091 | 9.79 | 3.4E-01 | 2.5E-02 | 2.1E+00 | 1.0E+00 | 4.7E-02 |
| XC0065 | 221.091 | 12.84 | 1.8E-01 | 4.6E-02 | N.D. | N.D. | 4.8E-02 |
| Cystathionine | 223.074 | 9.82 | 4.1E+00 | 3.3E+00 | N.D. | N.D. | 2.8E+00 |
| 3-Hydroxykynurenine | 225.085 | 9.63 | N.D. | N.D. | 1.5E-02 | 1.4E-02 | N.D. |
| 2'-Deoxycytidine | 228.096 | 9.37 | 3.4E-03 | 5.4E-03 | N.D. | N.D. | 6.6E-03 |
| $\gamma$-Glu-2-aminobutyric acid | 233.113 | 12.08 | 1.1E-02 | N.D. | 6.5E-03 | N.D. | 4.8E-03 |
| Ser-Glu | 235.092 | 10.55 | 2.0E-02 | N.D. | N.D. | N.D. | 7.3E-03 |
| $\gamma$-Glu-Cys | 251.069 | 12.59 | 1.4E-02 | 1.9E-02 | N.D. | N.D. | 1.1E-02 |
| Glycerophosphocholine | 258.109 | 20.66 | 2.9E+00 | 1.4E+00 | 4.3E+00 | 3.8E+00 | 2.3E+00 |
| Thiamine | 265.110 | 6.60 | 7.7E-02 | 5.1E-02 | 1.0E-01 | 1.1E-01 | 5.0E-02 |
| Adenosine | 268.103 | 9.77 | 7.6E-03 | 9.4E-03 | N.D. | N.D. | 6.6E-03 |
| Glu-Glu | 277.103 | 10.70 | 3.5E-02 | 9.0E-03 | 4.8E-03 | N.D. | 1.5E-02 |
| 1-Methyladenosine | 282.119 | 9.85 | 4.8E-03 | N.D. | 2.0E-02 | 7.1E-03 | 3.7E-03 |
| Guanosine | 284.099 | 12.29 | 9.2E-03 | 6.1E-03 | N.D. | N.D. | N.D. |
| Argininosuccinic acid | 291.129 | 9.35 | 4.9E-02 | 1.4E-01 | 1.4E-02 | 3.1E-02 | 1.2E-01 |
| 5'-Deoxy-5'-methylthioadenosine | 298.096 | 9.98 | 4.3E-03 | 5.0E-03 | 3.6E-03 | 3.7E-03 | 4.9E-03 |
| Streptomycin sulfate_+H₂O_divalent | 300.643 | 6.87 | N.D. | N.D. | 2.1E-03 | 1.0E-02 | N.D. |
| Arg-Glu | 304.161 | 7.45 | 9.7E-03 | 2.9E-03 | N.D. | N.D. | 4.4E-03 |
| Glutathione (GSSG)_divalent | 307.082 | 11.98 | 3.5E+00 | 3.8E+00 | 7.9E+00 | 7.0E+00 | 4.2E+00 |
| Glutathione (GSH) | 308.093 | 12.97 | 3.9E+00 | 4.5E+00 | 2.8E-01 | 2.3E-01 | 4.6E+00 |
| XC0132 | 325.159 | 8.64 | 4.1E-02 | 5.6E-02 | 9.3E-03 | 1.4E-02 | 2.1E-02 |
| XC0137 | 350.100 | 13.21 | 3.7E-02 | 3.0E-02 | 4.5E-03 | 4.7E-03 | 3.7E-02 |
| Riboflavin | 377.147 | 20.92 | N.D. | N.D. | N.D. | 2.0E-02 | N.D. |
| $S$-Lactoylglutathione | 380.113 | 13.43 | N.D. | N.D. | N.D. | N.D. | 8.0E-03 |
| $S$-Adenosylmethionine | 399.144 | 7.10 | 6.1E-02 | 9.8E-02 | 4.9E-02 | 4.6E-02 | 7.2E-02 |
| Cysteine glutathione disulfide | 427.094 | 11.48 | 4.1E-02 | 2.6E-02 | 4.1E-02 | 6.6E-02 | 1.4E-02 |

Each ID consists of the initial of the measurement mode used and a serial number; C represents cation mode, and A represents anion mode.

N.D. is the abbreviation for "not detected", indicating that the relative area was subjected to analysis but was below limit of detection.

† List of candidate metabolites found by checking the m/z and MT values for the detected peaks against HMT's databases.

For "GABA" and "Pro" which are metabolites having saturated peak strength, the relative areas of $^{13}$C spectra were used.

The table is sorted by ID (in ascending order).

The results revealed the following:

- the induced malignant stem cells cc1-7 showed an aberration of endogenous cancer-related metabolism, i.e., increased fumarate respiration (related metabolites: fumaric acid, malic acid, succinic acid, 2-oxoglutaric acid) (refer to the colored columns in Table 35);
- the induced malignant stem cells cc1-2 showed an aberration of endogenous cancer-related metabolism, i.e., increased Warburg's effect (related metabolites: pyruvic acid, lactic acid) (refer to the colored columns in Table 35); and
- the induced malignant stem cells cc4-c and cc-4d showed an aberration of endogenous cancer-related metabolisms, i.e., increased reverse flux from glutamine to the TCA cycle (related metabolites: Glu, Gln, 2-oxoglutaric acid, isocitric acid, cis-aconitic acid, citric acid) and increased glutaminolysis (related metabolites: Glu, Gln, 2-oxoglutaric acid, ornithine) (refer to the columns boxed off a double line in Table 35).

The foregoing is a description of the metabolisms that are characteristic of cancer cells and which were shown by the induced malignant stem cells. Fumarate respiration and Warburg's effect are both phenomena that may occur during undernutrition. Enhanced Warburg's effect was determined on the basis of the accumulations of pyruvic acid and lactic acid. In the determination of enhanced fumarate respiration, the sequential accumulations of fumaric acid, malic acid, succinic acid, and 2-oxoglutaric acid in the latter part of the TCA cycle were used as an indicator.

Enhanced reverse flux from glutamine to the TCA cycle and enhanced glutaminolysis occurred for the purpose of effective collection of energy from pathways other than the glycolysis system because glutamine is an important nitrogen source for cell proliferation.

The induced pluripotent stem cells nfb1-4 were used as a control (normal stem cells).

(16-4-2) Statistical analyses (PCA, HCA)

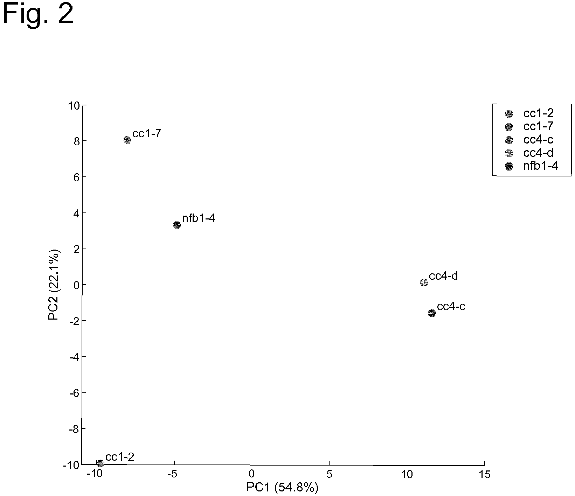

The results of the principal component analysis performed using the detected peaks are shown in Fig. 2. The results of the hierarchical cluster analysis and Heatmap visualization are shown in Table 36. As a data preprocessing step, standardization ($\mu$=0, $\sigma$=1) was performed with eps (=0) being substituted for N.D.

[Table 36]

Table 36:

| HMT DB [†] | | | Standardized Relative Area [§] | | | | |
|---|---|---|---|---|---|---|---|
| Compound name | m/z | MT | cc1-2 | cc1-7 | cc4-c | cc4-d | nfb1-4 |
| N-Ethylglycine | 104.071 | 9.76 | -0.447 | -0.447 | -0.447 | -0.447 | 1.789 |
| S-Lactoylglutathione | 380.113 | 13.43 | -0.447 | -0.447 | -0.447 | -0.447 | 1.789 |
| 1-Pyrroline 5-carboxylic acid | 114.055 | 10.94 | -1.750 | 0.359 | 0.333 | 0.266 | 0.792 |
| Sedoheptulose 7-phosphate | 289.032 | 9.50 | -0.913 | 1.081 | -0.756 | -0.498 | 1.085 |
| Met | 150.058 | 10.58 | -0.611 | 1.287 | -1.160 | -0.262 | 0.746 |
| Argininosuccinic acid | 291.129 | 9.35 | -0.376 | 1.228 | -1.034 | -0.704 | 0.886 |
| Cysteinesulfinic acid | 152.002 | 9.40 | -0.406 | 1.343 | -0.851 | -0.851 | 0.764 |
| 2'-Deoxycytidine | 228.096 | 9.37 | 0.110 | 0.754 | -1.015 | -1.015 | 1.165 |
| 5'-Deoxy-5'-methylthioadenosine | 298.096 | 9.98 | 0.061 | 1.091 | -1.105 | -0.914 | 0.866 |
| O-Phosphoserine | 184.001 | 12.05 | 0.258 | 1.512 | -0.907 | -0.907 | 0.045 |
| p-Toluic acid | 135.045 | 9.03 | -0.021 | 1.662 | -0.581 | -0.942 | -0.118 |
| S-Adenosylmethionine | 399.144 | 7.10 | -0.195 | 1.564 | -0.762 | -0.917 | 0.310 |
| Hypotaurine | 110.027 | 17.50 | -0.179 | 1.517 | -0.858 | -0.879 | 0.398 |
| 2-Aminoadipic acid | 162.075 | 10.75 | -0.103 | 1.564 | -1.063 | -0.608 | 0.209 |
| Malic acid | 133.014 | 20.77 | 0.043 | 1.425 | -1.091 | -0.783 | 0.406 |
| Fumaric acid | 115.003 | 24.57 | -0.177 | 1.433 | -1.075 | -0.701 | 0.520 |
| N-Carbamoylaspartic acid | 175.036 | 15.17 | 0.018 | 1.260 | -1.441 | -0.320 | 0.484 |
| 1-Aminocyclopropane-1-carboxylic acid Homoserinelactone | 102.055 | 7.09 | 0.264 | 1.049 | -1.544 | -0.343 | 0.574 |
| CDP | 402.008 | 11.30 | 0.407 | 1.050 | -1.619 | -0.139 | 0.301 |
| Arg | 175.118 | 7.10 | -0.380 | 0.936 | -1.570 | 0.511 | 0.503 |
| Ser | 106.049 | 9.88 | 0.010 | 1.433 | -1.338 | 0.222 | -0.326 |
| Succinic acid | 117.019 | 20.47 | 0.418 | 1.411 | -1.293 | -0.367 | -0.170 |
| Glyceraldehyde 3-phosphate | 168.990 | 11.44 | 0.256 | 1.533 | -1.170 | -0.419 | -0.201 |
| Ribose 5-phosphate | 229.011 | 10.40 | 0.404 | 1.569 | -0.815 | -0.718 | -0.439 |
| XC0132 | 325.159 | 8.64 | 0.649 | 1.413 | -0.953 | -0.728 | -0.381 |
| GMP | 362.048 | 9.03 | 0.702 | 1.352 | -1.094 | -0.588 | -0.372 |
| Ribulose 5-phosphate | 229.011 | 10.78 | 0.900 | 1.163 | -1.189 | -0.289 | -0.584 |
| 2-Phosphoglyceric acid | 184.985 | 18.79 | 1.203 | 0.981 | -0.728 | -0.728 | -0.728 |
| Guanosine | 284.099 | 12.29 | 1.418 | 0.702 | -0.706 | -0.706 | -0.706 |
| UDP-glucuronic acid | 579.026 | 10.85 | 1.214 | 0.797 | -0.069 | -0.987 | -0.955 |
| NADH | 664.118 | 8.09 | 0.611 | 1.402 | -0.277 | -0.674 | -1.063 |
| Thiaproline | 134.027 | 13.66 | 0.870 | 1.219 | -0.879 | -0.961 | -0.249 |
| UDP | 402.994 | 11.47 | 0.945 | 1.076 | -1.127 | -0.821 | -0.074 |
| 2-Hydroxyglutaric acid | 147.030 | 16.61 | 1.066 | 0.920 | -1.008 | -0.999 | 0.021 |
| Asn | 133.060 | 10.35 | 1.154 | 0.855 | -0.738 | -1.176 | -0.096 |
| 5-Oxoproline | 128.035 | 9.36 | 0.630 | 0.935 | -0.712 | -1.390 | 0.538 |
| XA0019 | 191.019 | 8.23 | 0.393 | 1.085 | -0.517 | -1.465 | 0.505 |
| γ-Glu-Cys | 251.069 | 12.59 | 0.587 | 1.204 | -1.032 | -1.032 | 0.273 |
| XA0002 | 110.985 | 15.39 | 0.672 | 1.126 | -1.047 | -1.047 | 0.297 |
| Adenosine | 268.103 | 9.77 | 0.648 | 1.060 | -1.067 | -1.067 | 0.425 |
| trans-Glutaconic acid | 131.033 | 21.78 | 0.543 | 1.073 | -1.068 | -1.068 | 0.521 |
| Glutathione (GSH) | 308.093 | 12.97 | 0.538 | 0.816 | -1.076 | -1.100 | 0.823 |

| | | | | | | | |
|---|---|---|---|---|---|---|---|
| Pipecolic acid | 130.086 | 10.18 | 0.701 | 0.507 | -1.082 | -1.082 | 0.955 |
| N-Acetylglutamic acid | 188.055 | 12.95 | 0.830 | 0.522 | -1.087 | -1.087 | 0.822 |
| XC0137 | 350.100 | 13.21 | 0.874 | 0.451 | -1.085 | -1.076 | 0.835 |
| Urea | 61.040 | 20.20 | 0.839 | 0.337 | -1.066 | -1.066 | 0.955 |
| N-Acetylaspartic acid | 174.040 | 14.28 | 1.027 | 0.186 | -1.040 | -1.040 | 0.866 |
| dTTP | 480.980 | 11.85 | 1.031 | 0.431 | -1.070 | -1.070 | 0.678 |
| Ornithine | 133.096 | 6.81 | 1.035 | 0.545 | -1.056 | -1.092 | 0.569 |
| GABA | 105.073 | 7.58 | 1.305 | 0.274 | -1.005 | -1.010 | 0.437 |
| N-Acetylmethionine | 190.055 | 8.03 | 1.265 | 0.529 | -1.016 | -1.016 | 0.238 |
| Tyr | 182.080 | 11.20 | 1.157 | 0.630 | -1.137 | -0.942 | 0.292 |
| Cystathionine | 223.074 | 9.82 | 1.081 | 0.646 | -1.062 | -1.062 | 0.398 |
| Lys | 147.112 | 6.87 | 0.817 | 0.616 | -1.327 | -0.824 | 0.718 |
| Leu | 132.101 | 10.21 | 0.931 | 0.709 | -1.431 | -0.635 | 0.426 |
| Methionine sulfoxide | 166.053 | 11.60 | 0.766 | 0.925 | -1.522 | -0.420 | 0.252 |
| Pyridoxine | 170.081 | 8.60 | 1.301 | 0.537 | -1.343 | -0.461 | -0.035 |
| Glyceric acid | 105.020 | 10.23 | 1.305 | 0.589 | -1.226 | -0.669 | 0.001 |
| Phosphoenolpyruvic acid | 166.974 | 20.55 | 1.433 | 0.397 | -1.190 | -0.609 | -0.031 |
| O-Succinylhomoserine | 218.066 | 11.99 | 1.419 | 0.118 | -1.359 | -0.323 | 0.145 |
| Octanoic acid | 143.108 | 8.26 | 1.150 | 0.393 | -1.563 | -0.187 | 0.207 |
| 2-Oxoglutaric acid | 145.013 | 20.84 | 1.189 | -0.076 | -1.553 | 0.410 | 0.029 |
| Lactic acid | 89.025 | 10.70 | 1.341 | -0.535 | -1.040 | 0.752 | -0.519 |
| Glycerol 3-phosphate | 171.006 | 11.92 | 1.491 | -0.737 | -0.584 | 0.577 | -0.748 |
| Guanidoacetic acid | 118.061 | 8.19 | 1.599 | -0.131 | -0.821 | 0.197 | -0.844 |
| Fructose 1,6-diphosphate | 338.987 | 14.28 | 1.718 | -0.239 | -0.802 | -0.096 | -0.582 |
| ADP | 426.021 | 10.73 | 1.664 | 0.008 | -0.615 | -0.145 | -0.912 |
| 3-Phosphoglyceric acid | 184.985 | 19.24 | 1.690 | -0.002 | -0.923 | -0.382 | -0.382 |
| Spermine | 203.222 | 4.48 | 1.574 | -0.021 | -1.179 | -0.013 | -0.361 |
| Dihydroxyacetone phosphate | 168.990 | 12.42 | 1.727 | -0.528 | -0.806 | -0.274 | -0.118 |
| Gly-Gly | 133.059 | 8.34 | 1.768 | -0.529 | -0.529 | -0.181 | -0.529 |
| N-Acetylglucosamine 1-phosphate | 300.047 | 9.42 | 1.775 | -0.655 | -0.391 | -0.374 | -0.356 |
| Cys | 122.026 | 11.17 | 1.789 | -0.447 | -0.447 | -0.447 | -0.447 |
| Isethionic acid | 124.990 | 11.48 | 1.789 | -0.447 | -0.447 | -0.447 | -0.447 |
| N-Acetylhistidine | 198.089 | 9.71 | 1.789 | -0.447 | -0.447 | -0.447 | -0.447 |
| Homoserine | 120.066 | 9.99 | 1.789 | -0.447 | -0.447 | -0.447 | -0.447 |
| ATP | 505.987 | 11.61 | 1.727 | -0.521 | -0.040 | -0.765 | -0.400 |
| Gly-Asp | 191.065 | 9.78 | 1.694 | -0.180 | -0.181 | -0.962 | -0.371 |
| GTP | 521.983 | 11.26 | 1.539 | -0.192 | -0.122 | -1.256 | 0.030 |
| N-Acetylalanine | 130.050 | 8.77 | 1.518 | -0.088 | -0.050 | -1.296 | -0.085 |
| Gln | 147.076 | 10.60 | 1.484 | 0.445 | -0.683 | -1.044 | -0.202 |
| GDP | 442.016 | 10.52 | 1.480 | 0.411 | -0.758 | -1.026 | -0.108 |
| Phosphocreatine | 210.028 | 12.03 | 1.503 | 0.383 | -0.890 | -0.890 | -0.105 |
| Pelargonic acid | 157.123 | 8.03 | 1.583 | 0.384 | -0.849 | -0.582 | -0.535 |
| Creatine | 132.076 | 8.81 | 1.621 | 0.213 | -0.795 | -0.798 | -0.242 |
| Benzoic acid | 121.030 | 9.70 | 1.675 | 0.046 | -0.758 | -0.758 | -0.206 |
| XC0065 | 221.091 | 12.84 | 1.693 | -0.118 | -0.746 | -0.746 | -0.083 |
| Imidazole-4-acetic acid | 127.049 | 8.00 | 1.726 | -0.116 | -0.628 | -0.746 | -0.236 |
| 4-Guanidinobutyric acid | 146.092 | 8.22 | 1.739 | -0.225 | -0.686 | -0.649 | -0.179 |
| $N^6$-Acetyllysine | 189.122 | 11.27 | 1.736 | -0.471 | -0.558 | -0.672 | -0.036 |
| N-Acetylornithine | 175.107 | 9.44 | 1.736 | -0.573 | -0.573 | -0.573 | -0.015 |
| N-Acetyllysine | 189.122 | 9.70 | 1.691 | -0.611 | -0.611 | -0.611 | 0.143 |

| | | | | | | | |
|---|---|---|---|---|---|---|---|
| Ser-Glu | 235.092 | 10.55 | 1.669 | -0.625 | -0.625 | -0.625 | 0.207 |
| *myo*-Inositol 1-phosphate *myo*-Inositol 3-phosphate | 259.019 | 10.12 | 1.602 | -0.657 | -0.657 | -0.657 | 0.370 |
| $N^6,N^6,N^6$-Trimethyllysine | 189.159 | 7.17 | 1.635 | -0.526 | -0.368 | -0.937 | 0.196 |
| Glu-Glu | 277.103 | 10.70 | 1.633 | -0.269 | -0.587 | -0.942 | 0.165 |
| Arg-Glu | 304.161 | 7.45 | 1.574 | -0.129 | -0.849 | -0.849 | 0.253 |
| Gluconic acid | 195.050 | 8.11 | 1.486 | -0.267 | -0.859 | -0.857 | 0.498 |
| Gluconolactone | 179.054 | 21.73 | 1.356 | -0.252 | -0.896 | -0.902 | 0.694 |
| 2,3-Diphosphoglyceric acid | 264.951 | 18.09 | 1.123 | -0.925 | -0.925 | -0.253 | 0.981 |
| Heptanoic acid | 129.091 | 8.45 | 1.243 | -0.726 | -0.726 | -0.726 | 0.935 |
| Metronidazole | 172.072 | 9.39 | 1.398 | -0.968 | 0.154 | -0.968 | 0.384 |
| $\gamma$-Glu-2-aminobutyric acid | 233.113 | 12.08 | 1.375 | -0.970 | 0.467 | -0.970 | 0.097 |
| 2-Hydroxybutyric acid | 103.041 | 9.64 | 1.554 | -0.675 | 0.470 | -0.675 | -0.675 |
| NADPH_divalent | 371.537 | 11.13 | 1.030 | -0.608 | 1.147 | -0.675 | -0.893 |
| Glucose 1-phosphate | 259.021 | 9.94 | 0.870 | -0.347 | 1.188 | -0.504 | -1.207 |
| Pantothenic acid | 218.102 | 7.63 | 1.116 | -0.199 | 0.947 | -1.132 | -0.732 |
| UTP | 482.960 | 12.32 | 0.862 | -0.277 | 0.903 | -1.537 | 0.049 |
| UDP-*N*-acetylgalactosamine | 606.074 | 8.34 | 0.635 | -0.308 | 1.140 | -1.484 | 0.016 |
| UDP-glucose UDP-galactose | 565.047 | 8.52 | 0.338 | -0.045 | 1.354 | -1.413 | -0.234 |
| CTP | 481.975 | 12.11 | 0.163 | 0.098 | 1.272 | -1.532 | -0.001 |
| *N*-Acetylserine | 146.045 | 8.70 | 0.285 | 1.110 | 0.321 | -1.604 | -0.111 |
| Glu | 148.060 | 10.76 | -0.155 | 1.305 | 0.375 | -1.459 | -0.066 |
| Asp | 134.044 | 11.39 | -0.908 | 1.171 | 1.000 | -0.684 | -0.579 |
| 5-Aminovaleric acid | 118.086 | 8.02 | -1.217 | 0.698 | 1.269 | -0.639 | -0.111 |
| Hexanoic acid | 115.076 | 8.79 | -1.259 | 1.545 | -0.095 | -0.125 | -0.066 |
| Isovaleric acid | 101.061 | 9.11 | -0.756 | 1.585 | -0.364 | 0.349 | -0.814 |
| 3-Hydroxy-3-methylglutaric acid | 161.046 | 15.16 | -0.447 | 1.789 | -0.447 | -0.447 | -0.447 |
| Erythrose 4-phosphate | 199.002 | 11.62 | -0.447 | 1.789 | -0.447 | -0.447 | -0.447 |
| 2-Aminobutyric acid | 104.071 | 9.52 | -0.541 | 1.762 | -0.140 | -0.541 | -0.541 |
| 1-Methylnicotinamide | 137.071 | 7.33 | -0.641 | 1.718 | 0.042 | -0.492 | -0.626 |
| Threonic acid | 135.030 | 9.12 | -0.683 | 1.521 | 0.439 | -0.312 | -0.965 |
| Isobutyric acid | 87.045 | 9.71 | -0.055 | 1.487 | 0.042 | -0.147 | -1.327 |
| Isobutylamine | 74.097 | 7.02 | 0.048 | 0.869 | 0.137 | 0.628 | -1.682 |
| Lauric acid | 199.170 | 7.62 | 0.427 | 0.468 | -0.056 | 0.854 | -1.693 |
| XC0016 | 129.065 | 8.73 | 1.122 | 0.295 | -0.186 | 0.349 | -1.580 |
| AMP | 346.054 | 9.15 | 1.149 | 0.140 | -0.059 | 0.359 | -1.590 |
| Fructose 6-phosphate | 259.021 | 9.81 | 0.590 | -0.585 | 0.604 | 0.870 | -1.479 |
| GDP-glucose | 604.069 | 8.22 | 0.720 | -1.090 | 0.868 | 0.592 | -1.090 |
| ADP-glucose GDP-fucose | 588.074 | 8.23 | 0.135 | -1.405 | 1.077 | 0.741 | -0.548 |
| Glycerophosphocholine | 258.109 | 20.66 | -0.047 | -1.313 | 1.177 | 0.752 | -0.568 |
| 1-Methyladenosine | 282.119 | 9.85 | -0.299 | -0.940 | 1.681 | 0.007 | -0.448 |
| $\gamma$-Butyrobetaine | 146.117 | 8.06 | -0.228 | -0.838 | 1.620 | 0.208 | -0.761 |
| Kynurenine | 209.091 | 9.79 | -0.418 | -0.778 | 1.589 | 0.360 | -0.753 |
| 3-Hydroxybutyric acid | 103.040 | 9.41 | -0.495 | -0.702 | 1.644 | 0.257 | -0.704 |
| *N*-Acetylputrescine | 131.117 | 8.39 | -0.629 | -0.629 | 1.662 | 0.226 | -0.629 |
| Xanthine | 151.026 | 8.30 | -0.654 | -0.654 | 1.610 | 0.352 | -0.654 |
| XA0065 | 445.052 | 6.88 | -0.671 | -0.671 | 1.565 | 0.447 | -0.671 |
| Hydroxyproline | 132.065 | 11.84 | -0.670 | -0.675 | 1.564 | 0.450 | -0.669 |

| | | | | | | | |
|---|---|---|---|---|---|---|---|
| Carnitine | 162.111 | 8.46 | -0.682 | -0.682 | 1.530 | 0.514 | -0.682 |
| Thr | 120.065 | 10.39 | -0.627 | -0.464 | 1.570 | 0.398 | -0.877 |
| His | 156.076 | 7.27 | -0.452 | -0.656 | 1.402 | 0.676 | -0.970 |
| Pro | 117.073 | 10.66 | -0.290 | -0.786 | 1.440 | 0.579 | -0.943 |
| NAD⁺ | 662.102 | 6.52 | -0.005 | -1.065 | 1.154 | 0.833 | -0.917 |
| cis-Aconitic acid | 173.008 | 27.05 | -0.139 | -0.962 | 1.082 | 0.980 | -0.962 |
| Glucose 6-phosphate | 259.021 | 9.69 | -0.210 | -0.886 | 1.087 | 1.001 | -0.993 |
| Citric acid | 191.019 | 25.42 | -0.403 | -0.868 | 1.081 | 1.069 | -0.879 |
| Isocitric acid | 191.018 | 27.68 | -0.347 | -0.894 | 1.067 | 1.069 | -0.894 |
| Thiamine | 265.110 | 6.60 | -0.058 | -0.954 | 0.759 | 1.240 | -0.987 |
| Pyridoxal | 168.065 | 8.73 | -0.125 | -0.883 | 0.744 | 1.276 | -1.012 |
| UMP | 323.026 | 9.65 | -0.493 | -0.566 | 1.127 | 1.006 | -1.074 |
| Ile | 132.101 | 10.10 | -0.603 | -0.767 | 1.111 | 1.073 | -0.813 |
| Ethanolamine phosphate | 140.012 | 7.92 | -0.667 | -0.761 | 1.093 | 1.097 | -0.761 |
| 3-Hydroxykynurenine | 225.085 | 9.63 | -0.730 | -0.730 | 1.152 | 1.037 | -0.730 |
| Propionic acid | 73.029 | 10.77 | -0.729 | -0.729 | 1.021 | 1.167 | -0.729 |
| Ethanolamine | 62.061 | 6.35 | -0.729 | -0.729 | 0.999 | 1.187 | -0.729 |
| N-Acetylneuraminic acid | 308.097 | 7.14 | -0.726 | -0.716 | 0.985 | 1.200 | -0.743 |
| Nicotinamide | 123.055 | 7.37 | -0.742 | -0.737 | 0.962 | 1.220 | -0.703 |
| O-Acetylcarnitine | 204.122 | 8.92 | -0.730 | -0.730 | 1.059 | 1.131 | -0.730 |
| Putrescine | 89.107 | 4.71 | -0.724 | -0.724 | 1.265 | 0.908 | -0.724 |
| Val | 118.086 | 9.92 | -0.557 | -0.727 | 1.209 | 0.959 | -0.884 |
| Phe | 166.086 | 10.94 | -0.583 | -0.716 | 1.334 | 0.809 | -0.844 |
| N⁸-Acetylspermidine | 188.175 | 6.34 | -0.700 | -0.700 | 1.452 | 0.649 | -0.700 |
| CMP-N-acetylneuraminate | 613.139 | 8.08 | -0.712 | -0.712 | 1.385 | 0.750 | -0.712 |
| Glutathione (GSSG)_divalent | 307.082 | 11.98 | -0.898 | -0.716 | 1.306 | 0.835 | -0.527 |
| FAD_divalent | 391.569 | 7.78 | -0.957 | -0.957 | 0.802 | 1.229 | -0.117 |
| Mucic acid | 209.031 | 13.80 | -1.007 | -1.007 | 1.281 | 0.545 | 0.187 |
| β-Ala | 90.055 | 7.32 | 0.034 | -0.679 | 1.716 | -0.636 | -0.435 |
| Ala | 90.055 | 8.94 | 0.469 | -0.463 | 1.538 | -0.709 | -0.836 |
| 3-Aminoisobutyric acid | 104.070 | 7.82 | -0.447 | -0.447 | 1.789 | -0.447 | -0.447 |
| NADP⁺ | 742.069 | 9.26 | -0.447 | -0.447 | 1.789 | -0.447 | -0.447 |
| Gly | 76.039 | 8.25 | -0.580 | -0.170 | 1.728 | -0.199 | -0.780 |
| XA0033 | 242.079 | 7.61 | -1.084 | -0.379 | 1.086 | 1.034 | -0.656 |
| Biotin | 243.082 | 7.60 | -1.305 | -0.180 | 0.753 | 1.211 | -0.479 |
| Phosphorylcholine | 184.072 | 19.68 | -1.435 | 0.005 | 0.791 | 1.065 | -0.426 |
| Trp | 205.096 | 10.87 | -1.267 | 0.004 | 0.088 | 1.508 | -0.334 |
| Choline | 104.107 | 6.85 | -0.745 | -0.043 | -0.377 | 1.728 | -0.563 |
| 2-Hydroxy-4-methylvaleric acid | 131.072 | 8.77 | -0.447 | -0.447 | -0.447 | 1.789 | -0.447 |
| 6-Phosphogluconic acid | 275.016 | 14.36 | -0.447 | -0.447 | -0.447 | 1.789 | -0.447 |
| IMP | 347.036 | 9.43 | -0.447 | -0.447 | -0.447 | 1.789 | -0.447 |
| CMP | 322.043 | 9.47 | -0.447 | -0.447 | -0.447 | 1.789 | -0.447 |
| XA0027 | 227.200 | 7.38 | -0.447 | -0.447 | -0.447 | 1.789 | -0.447 |
| Riboflavin | 377.147 | 20.92 | -0.447 | -0.447 | -0.447 | 1.789 | -0.447 |
| Betaine aldehyde_+H₂O | 120.101 | 7.44 | -0.749 | -0.749 | 0.076 | 1.676 | -0.254 |
| Citrulline | 176.102 | 10.89 | -0.550 | -0.886 | 0.365 | 1.586 | -0.514 |
| 2-Hydroxyvaleric acid | 117.055 | 9.12 | -0.698 | -0.698 | 0.633 | 1.462 | -0.698 |
| Imidazolelactic acid | 157.061 | 8.78 | -0.683 | -0.683 | 0.523 | 1.525 | -0.683 |
| Spermidine | 146.164 | 4.54 | -0.674 | -0.433 | 0.266 | 1.632 | -0.790 |
| Betaine | 118.086 | 11.13 | -0.650 | -0.641 | 0.308 | 1.629 | -0.646 |

| | | | | | | | |
|---|---|---|---|---|---|---|---|
| Diethanolamine | 106.086 | 7.63 | -0.638 | -0.638 | 0.267 | 1.646 | -0.638 |
| 2-Amino-2-(hydroxymethyl)-1,3-propanediol | 122.081 | 8.21 | -0.635 | -0.635 | 0.252 | 1.652 | -0.635 |
| Acetylcholine | 146.117 | 7.60 | -0.607 | -0.607 | 0.124 | 1.697 | -0.607 |
| Streptomycin sulfate_+H$_2$O_divalent | 300.643 | 6.87 | -0.555 | -0.555 | -0.085 | 1.751 | -0.555 |
| Cysteine glutathione disulfide | 427.094 | 11.48 | 0.164 | -0.570 | 0.158 | 1.464 | -1.216 |
| 2-Oxoisovaleric acid | 115.040 | 10.05 | -0.042 | -1.031 | -0.037 | 1.632 | -0.521 |
| 4-Methyl-2-oxovaleric acid 3-Methyl-2-oxovaleric acid | 129.055 | 9.44 | 0.440 | -1.031 | -0.058 | 1.449 | -0.800 |
| XA0013 | 172.991 | 10.71 | 0.733 | -1.574 | 0.072 | 0.970 | -0.201 |
| XA0055 | 368.998 | 14.02 | 0.865 | -1.580 | 0.097 | 0.836 | -0.218 |
| Pyruvic acid | 87.009 | 12.75 | 0.837 | -1.666 | 0.037 | 0.721 | 0.071 |
| PRPP | 388.943 | 16.50 | 0.946 | -1.672 | 0.499 | 0.249 | -0.022 |
| Adenine | 136.061 | 7.54 | 0.642 | -1.747 | 0.587 | 0.090 | 0.428 |

(16-4-3) Drawing of a metabolic pathway

These candidate compounds were drawn on the maps of glycolysis/gluconeogenesis pathway, pentose phosphate pathway, citric acid cycle, urea cycle, purine metabolic pathway, pyrimidine purine metabolic pathway, nicotinic acid/nicotinamide metabolic pathway, and various amino acid metabolic pathways (not shown).

The induced malignant stem cells analyzed in this Example can be considered as cells characterized both by an aberration of endogenous cancer-related metabolisms and by expression of the ES cell-specific genes (OCT3/4, NANOG, SOX2, ZFP42).

Example 17: Detection for aberrations of endogenous cancer-related sugar chains in induced malignant stem cells

In this Example, (1)(g) aberrations of endogenous cancer-related sugar chains (detected, increased/reduced, or loss of sugar chains) in induced malignant stem cells were analysed, in comparison with those in induced pluripotent stem cells.

(17-1) Materials

The (1) (g) aberrations of endogenous cancer-related sugar chains in induced malignant stem cells were detected by purifying sugar chains from sample cells, subjecting them to mass spectrometry by MALDI-TOF MS, and comparing their sugar chain patterns with those of control samples.

The following samples were used in the detection for (1) (g) aberrations of endogenous cancer-related sugar chains in induced malignant stem cells:

- cell population (ncc1) derived from colon non-cancer site tissues, cell population (cc1) derived from fresh colon cancer site tissues, and induced malignant stem cells (CC1_2, CC1_7) prepared from fresh colon cancer tissues , which were collected from the individual of donor No. 4;
- cell population (ncc4) derived from colon non-cancer site tissues, cell population (cc4) derived from fresh colon cancer site tissues, and induced malignant stem cells (CC4_c, CC4_D) prepared from fresh colon cancer tissues, which were collected from the individual of donor No. 5; and
- induced pluripotent stem cells (NFB1_4) prepared from fibroblasts (7F3956) collected from the individual of donor No. 6.

(17-2) Sugar chain purification and detection procedures

(17-2-1) Releasing of sugar chains from cultured cells and tissues

The cells proliferated to about 80% confluence in a 100 mm-diameter culture dish were dissociated using a scraper and washed by centrifugation with 40 mL of PBS, and 125 $\mu$L of the 0.5% Triton X-100 solution (Wako Pure Chemical; Cat No. 582-83991) was added to the cells. Next, the cells were disrupted using a QIA shredder (QIAGEN) and washed off with 125 μL of pure water to collect the total quantity (250 μL) of solution.

To 250 μL of the thus-prepared disrupted cell solution, the following solutions were added:

- an aqueous solution (25 μL) of 1 M ammonium bicarbonate (prepared at the time of use) (Wako Pure Chemical; Cat No. 017-02875), and
- an aqueous solution (25 μL) of 120 mM dithiothreitol (DTT; Sigma-Aldrich; Cat No. D9779) (prepared at the time of use),

and the mixture was voltexed to ensure complete dissolution.

After the resulting solution was placed at 60°C for 30 minutes, an aqueous solution (50 μL) of 123 mM iodoacetamide (IAA; Wako Pure Chemical; Cat No. 093-02152) was added, and the mixture was placed at room temperature for an hour under shading, to which trypsin was further added (2000 units; Sigma-Aldrich, T-0303). Trypsin was first dissolved in 1 mM HCl at 40 units/μL and 50 μL of the solution was added to the sample solution to give 2000 units. The trypsin-containing solution was placed in an incubator at 37°C for at least one hour and then heated in a heat block at 90°C for 5 minutes to thereby inactivate the trypsin.

Next, N-glycosidase (PNGase F; Roche Applied Science; Cat No. 11365193001) (25 μL=5 units) was added, and the solution was placed in an incubator at 37°C overnight (for at least 12 hours) to thereby release sugar chains from the cells.

(17-2-2) Sugar chain purification and labeling

The sugar chains released from the cultured cells/tissues or glycoproteins by the procedure described above were purified using the sugar chain purification kit BlotGlyco ® (BS-45603) produced by Sumitomo Bakelite Co., Ltd.

In the steps described below, the step of "washing polymer beads" involves placing a washing solution into a reservoir containing the beads and centrifuging the reservoir in a tabletop centrifuge for about 5 seconds and removing the washing solution.

The solutions used in these steps had the following composition:

- 2% (v/v) acetic acid/acetonitrile: Prepared by adding 200 μL of acetic acid (Wako Pure Chemical; Cat No. 012-00245) to 9.8 mL of acetonitrile (ACN) (Wako Pure Chemical; Cat No. 015-08633) and mixing them.
- 2 M guanidine solution: Prepared by dissolving 1.9 g of guanidine hydrochloride (Wako Pure Chemical; Cat No. 070-01825) in 10 mL of pure water, which can be stored at ordinary temperatures for about one month.
- 1% triethylamine/methanol: Prepared by adding 100 μL oftriethylamine (Wako Pure Chemical; Cat No. 202-02646) to 9.9 mL of methanol (Wako Pure Chemical; Cat No. 136-09475) and mixing them.
- 10 mM hydrochloric acid: Prepared by diluting concentrated hydrochloric acid (Wako Pure Chemical; Cat No. 083-01095) with pure water as appropriate.

Step 1: Dispensing polymer beads

1) Insert a reaction tube into a 2 mL Eppendorf tube.
2) Add 500 μL of pure water to a tube containing polymer beads (dry) and vortex the tube to disperse the polymer beads.
3) Take 50 μL ofthe polymer bead dispersion using a pipette and inject the dispersion into the bottom of the reaction tube.
4) Centrifuge the reaction tube in a tabletop centrifuge for about 5 seconds to drain water.

Step 2: Sugar chain capture reaction

1) Insert the reaction tube into a 1.5 mL Eppendorf tube.
2) Add 180 μL of 2% acetic acid/acetonitrile.
3) Add a sugar chain sample solution (20 μL).
4) Insert the tube into a heat block at 80°C and heat it for an hour without cover it with a lid.
5) Check to see that the solvent is completely evaporated and the beads are dried up. If not, continue heating for another 15 minutes.

Step 3: Washing polymer beads

1) Insert the reaction tube into the 2 mL Eppendorf tube and place it into a tabletop centrifuge.
2) Wash the polymer beads with 200 $\mu$L of the 2 M guanidine solution twice.
3) Wash the polymer beads with 200 $\mu$L of pure water twice.
4) Wash the polymer beads with 200 $\mu$L of 1% triethylamine/methanol twice.

Step 4: Capping functional groups of polymer beads

1) Prepare a 10% acetic anhydride solution just before use. More specifically, 900 $\mu$L of methanol takes into an Eppendorftube, add 100 $\mu$L of acetic anhydride (Wako Pure Chemical; Cat No. 012-08545), and mix them.
2) Insert the reaction tube into the 1.5 mL Eppendorf tube.
3) Add 100 $\mu$L of the 10% acetic anhydride solution to the polymer beads.
4) Place the tube at room temperature for 30 minutes.

Step 5: Washing polymer beads

1) Insert the reaction tube into the 2 mL Eppendorf tube.
2) Centrifuge the tube to drain the acetic anhydride solution.
3) Wash the polymer beads with 200 $\mu$L of 10 mM hydrochloric acid twice.
4) Wash the polymer beads with 200 $\mu$L of methanol twice.
5) Wash the polymer beads with 200 $\mu$L of dimethyl sulfoxide (DMSO) (Wako Pure Chemical; Cat No. 048-21985) twice.

Step 6: Sialic acid protection (methyl esterification)

1) Insert the reaction tube into the 1.5 mL Eppendorf tube.
2) Prepare a solution of 500 mM 1-methyl-3-*p*-tolyltriazene (MTT) (Tokyo Chemical Industry; Cat No. M0641, sialic acid methyl esterification reagent) just before use. Meter MTT (74.6 mg) in an Eppendorftube, to which 1 mL of DMSO is added to dissolve.
3) Add the MTT solution (100 $\mu$L) to the polymer beads.
4) Heat the tube in a heat block at 60°C for an hour without coverd it with a lid.

Step 7: Washing polymer beads

1) Insert the reaction tube into the 2 mL Eppendorf tube.
2) Centrifuge the tube to drain the MTT solution.
3) Wash the polymer beads with 200 $\mu$L of methanol twice.
4) Wash the polymer beads with 200 $\mu$L of pure water twice.

Step 8: Re-releasing/Labeling of sugar chains

1) Insert the reaction tube into the 1.5 mL Eppendorf tube.
2) Add 220 $\mu$L of pure water to a tube containing a labeling compound for MALDI-TOF MS (labeled as "Labeling reagent for MALDI-TOF MS") and vortex the tube to dissolve the compound (reagent concentration: 20 mM). The solution of the labeling compound for MALDI-TOF MS was divided into smaller portions and cryopreserved at -20°C or lower to avoid Repeated freeze-thaw cycles.
3) Add 20 μL of the solution of the labeling compound for MALDI-TOF MS to the polymer beads.
4) Add 180 μL of 2% acetic acid/acetonitrile.
5) Heat the tube in a heat block at 80°C for an hour without cover it with a lid.
6) Check to see that the solvent is completely evaporated and the beads are dried up. If not, continue heating for another 15 minutes.

Step 9: Collection of labeled sugar chains

1) Insert the reaction tube into a new 1.5 mL Eppendorf tube.
3) Add 50 μL of pure water to the polymer beads.
3) Centrifuge the tube to collect the solution in the Eppendorftube. The collected solution contains labeled sugar chains and the unreacted solution of the labeling compound for MALDI-TOF MS.

Step 10: Removal of excess reagent (in the case of MALDI-TOF MS)

Excess reagent was removed according to the following procedure:

1) Add 950 μL of acetonitrile to the collected sugar chain solution (about 50 μL) and mix them (dilute the collected sugar chain solution to give a 95% acetonitrile solution).
2) Place the "cleanup column" in a centrifuge.
3) Wash the cleanup column with 200 μL of pure water once.
4) Wash the cleanup column with 200 μL of acetonitrile twice.
5) Add the total quantity ofthe sugar chain solution prepared in 1) to the cleanup column.
6) Wait for 10 minutes to allow the solution to flow down under gravity.
7) Perform centrifugation to allow the remaining solution to pass through.
8) Discard the waste liquid accumulated in the tube, insert the column into the tube again, and place it in a centrifuge.
9) Wash the cleanup column with 300 μL of acetonitrile twice.
10) Discard the waste liquid accumulated in the tube, insert the cleanup column into the tube again, and perform centrifugation to remove acetonitrile more thoroughly.
11) Insert the cleanup column into a new 1.5 mL Eppendorf tube.
12) Add 50 μL of pure water.
13) Perform centrifugation to collect the labeled sugar chain solution in the Eppendorf tube.
14) The thus-collected sample was subjected to MALDI-TOF MS measurement.

(17-2-3) Releasing of sugar chains from glycoproteins, purification, and labeling

In order to confirm the reproducibility of the operations of the sugar chain purification kit BlotGlyco ® (BS-45603) produced by Sumitomo Bakelite Co., Ltd., the reference sample (bovine serum IgG) was also used. The reference sample was subjected to sugar chain purification and labeling in accordance with the BlotGlyco ® protocol (A) entitled "Operation Protocol for MALDI-TOF MS Analysis" (Ver. 120601). The purified and labeled sugar chain solution was measured by MALDI-TOF MS to confirm that the sugar chains had been collected successfully.

One milligram of glycoproteins in the reference sample (bovine serum IgG) was treated with trypsin and N-glycosidase (PNGase F) according to the following procedure.

First, the tube containing the glycoproteins (1 mg) was loaded with the following solutions:

- an aqueous solution of 1 M ammonium bicarbonate (5 μL) (prepared at the time of use),
- Pure water (50 μL), and
- an aqueous solution of 120 mM DTT (5 μL) (prepared at the time of use),

and the tube was vortexed to allow the glycoproteins to dissolve completely.

After the solution was placed at 60°C for 30 minutes, an aqueous solution (10 μL) of 123 mM IAA was added, and the tube was placed at room temperature for an hour under shading. To the resulting solution was further added trypsin (400 units). Trypsion was prepared by dissolving trypsin in a 1mM HCl solution at 40 unit/μL and 10 μL oftrypsion solution was added to the sample solution. The trypsin-containing solution was placed in an incubator at 37°C for at least one hour and then heated in a heat block at 90°C for 5 minutes to thereby inactivate the trypsin.

Next, N-glycosidase (PNGase F) (5 µL=5 units) was added, and the solution was placed in an incubator at 37°C overnight (for at least 12 hours) to thereby release sugar chains from the glycoproteins.

The thus-released sugar chains were purified and labeled according to the steps described in "(17-2-2) Sugar chain purification and labeling".

(17-2-4) MALDI-TOF MS measurement

1) Prepare a matrix solution. Meter 10 mg of 2,5-dihydroxybenzoic acid (DHB; MALDI matrix grade) in a sample tube, add acetonitrile (300 µL) and pure water (700 µL), and allow it to dissolve.
2) Mix the matrix solution and the labeled sugar chain solution at a ratio of 1:1 (e.g., 1 µL + 1 µL).
3) Spot 1 µL of the sample solution prepared in 2) onto a MALDI target plate and let it stand still to dry up. Also spot the sample solution prepared by dilution at 1/10, 1/100 and so on with the matrix solution.
4) MALDI-TOF MS: Use Autoflex III TOF/TOF (Bruker Daltonics) to perform MALDI-TOF MS measurement.

* Noise peaks derived from a re-release reagent are observed in the range of m/z 900 to 1100, which was measured on condition that any peaks for m/z 1200 and below be cut off.

(17-2-5) Calculating mass number

**[0465]** The reduced terminals of sugar chains were labeled with the labeling compound for MALDI-TOF MS (exact mass: 447.22) by dehydration condensation (Mol. Cell. Proteomics 4, 1977-1989 (2005)).

The carboxyl groups of sialic acid were methy esterified (mass number: +14.02 per one sialic acid residue).

The sugar chains labeled using the sugar chain purification kit BlotGlyco ® (BS-45603) produced by Sumitomo Bakelite Co., Ltd. were mainly detected as a proton adduct [M+H]+ (some were also detected as [M+Na]+ or [M+K]+).

The mass number M of the sugar chain detected as [M+H]+ was calculated using the following equation:

$$\text{Mass number M of sugar chain} = [\text{Observed m/z}] - 447.22 + 18.01 - 14.02 \times \text{N} - 1.00.$$

(17-3) Results of sugar chain analysis

Among the investigated cells, the sugar chains specific to the four induced malignant stem cells (CC4-c, CC4-d, CC1-2, CC1-7) are listed in Table 37 below.

[Table 37]

| Table 37A: Sugar chains specific to the induced malignant stem cells (CC4-c) | | | | | |
|---|---|---|---|---|---|
| Obsd. *m/z* | S/N ratio | Intensity | Area | dmass | Estimated glycan composition ('GlycoMod' tool) |
| 1997.63 | 5.6 | 165.1 | 260 | -0.165 | (Hex)1 (HexNAc)1 (Deoxyhexose)2 + (Man)3(GlcNAc)2 |
| 2474.7 | 4.2 | 98.26 | 215 | -0.215 | (Hex)7 + (Man)3(GlcNAc)2 |
| 2508.78 | 3.4 | 78.56 | 182 | -0.206 | (Hex)2 (HexNAc)2 (Deoxyhexose)3 + (Man)3(GlcNAc)2 |
| Table 37B: Sugar chains specific to the induced malignant stem cells (CC4-d) | | | | | |
| Obsd. *m/z* | S/N ratio | Intensity | Area | dmass | Estimated glycan composition ('GlycoMod' tool) |
| 1997.48 | 12.6 | 306.91 | 312 | -0.315 | (Hex)1 (HexNAc)1 (Deoxyhexose)2 + (Man)3(GlcNAc)2 |
| 2474.55 | 4 | 84.99 | 166 | -0.365 | (Hex)7 + (Man)3(GlcNAc)2 |
| 2508.58 | 5 | 109.57 | 180 | -0.401 | (Hex)2 (HexNAc)2 (Deoxyhexose)3 + (Man)3(GlcNAc)2 |
| Table 37C: Sugar chains specific to the induced malignant stem cells (CC1-2) | | | | | |
| Obsd. *m/z* | S/N ratio | Intensity | Area | dmass | Estimated glycan composition ('GlycoMod' tool) |
| 2521.94 | 7.1 | 180.33 | 426 | -0.039 | (Hex)2 (HexNAc)2 (Deoxyhexose)1 (NeuAc)1 + (Man)3(GlcNAc)2 |

(continued)

| Table 37C: Sugar chains specific to the induced malignant stem cells (CC1-2) | | | | | |
|---|---|---|---|---|---|
| Obsd. *m/z* | S/N ratio | Intensity | Area | dmass | Estimated glycan composition ('GlycoMod' tool) |
| 2668.01 | 6.4 | 146.99 | 412 | -0.031 | (Hex)2 (HexNAc)2 (Deoxyhexose)2 (NeuAc)1 + (Man)3(GlcNAc)2 |
| 2827.12 | 4.3 | 86.57 | 322 | 0.023 | (Hex)2 (HexNAc)2 (Deoxyhexose)1 (NeuAc)2 + (Man)3(GlcNAc)2 |

| Table 37D: Sugar chains specific to the induced malignant stem cells (CC1-7) | | | | | |
|---|---|---|---|---|---|
| Obsd. *m/z* | S/N ratio | Intensity | Area | dmass | Estimated glycan composition ('GlycoMod' tool) |
| 2521.51 | 5.8 | 116.2 | 252 | -0.474 | (Hex)2 (HexNAc)2 (Deoxyhexose)1 (NeuAc)1 + (Man)3(GlcNAc)2 |
| 2667.55 | 3.8 | 68.42 | 180 | -0.486 | (Hex)2 (HexNAc)2 (Deoxyhexose)2 (NeuAc)1 + (Man)3(GlcNAc)2 |
| 2826.68 | 3.8 | 69.65 | 169 | -0.415 | (Hex)2 (HexNAc)2 (Deoxyhexose)1 (NeuAc)2 + (Man)3(GlcNAc)2 |

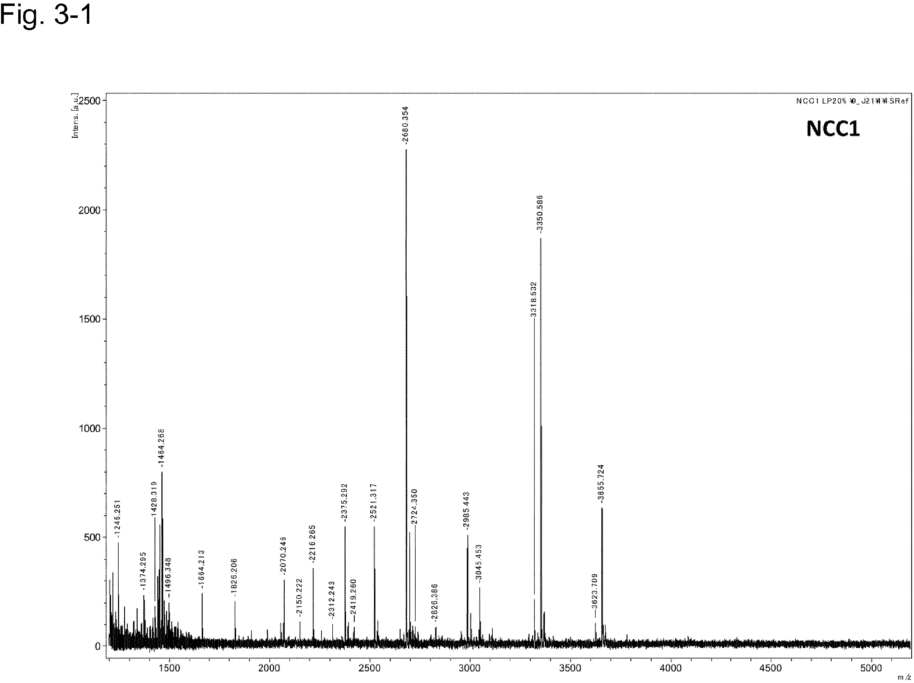

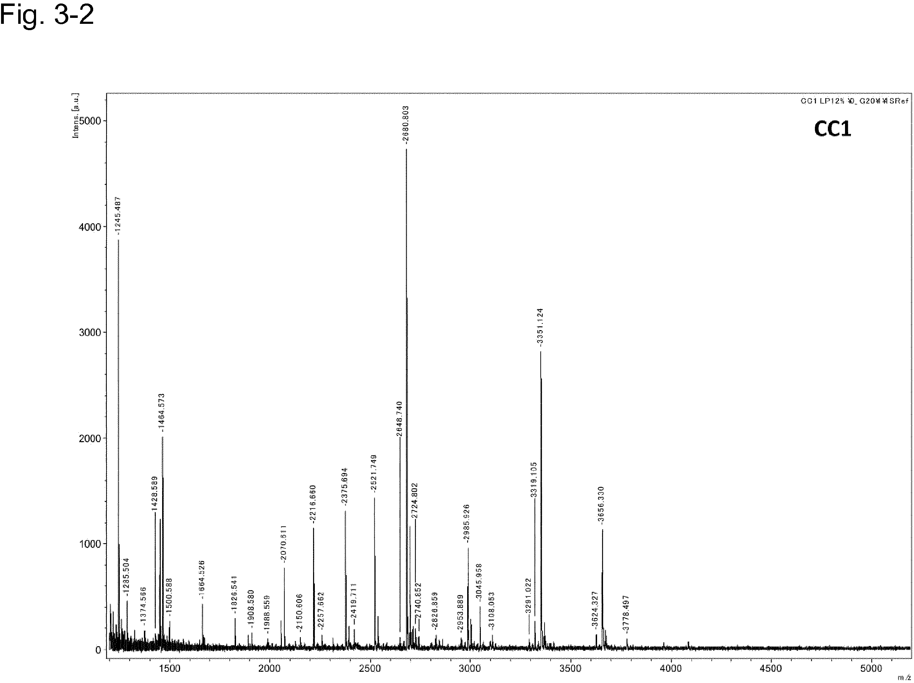

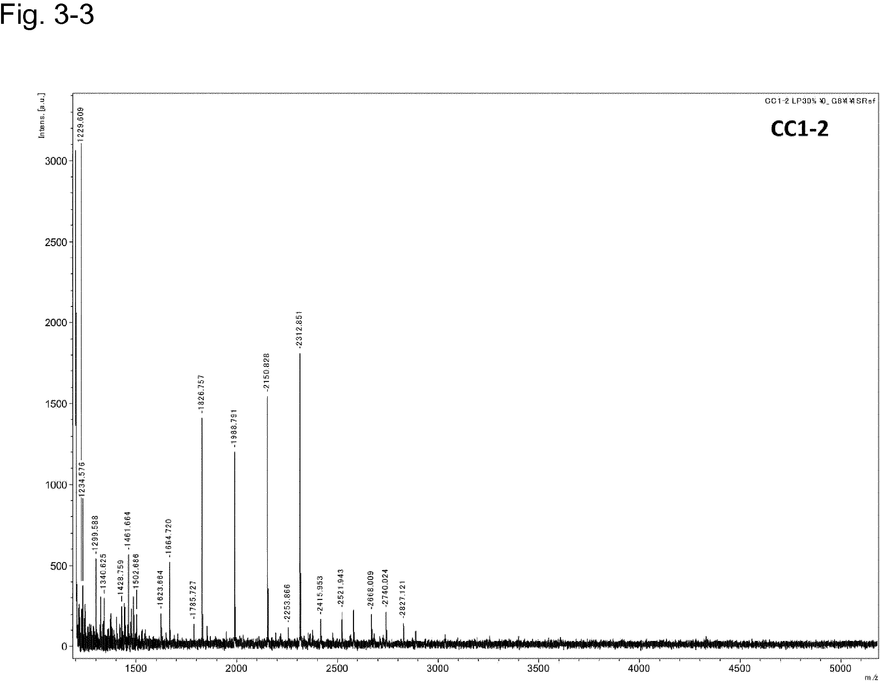

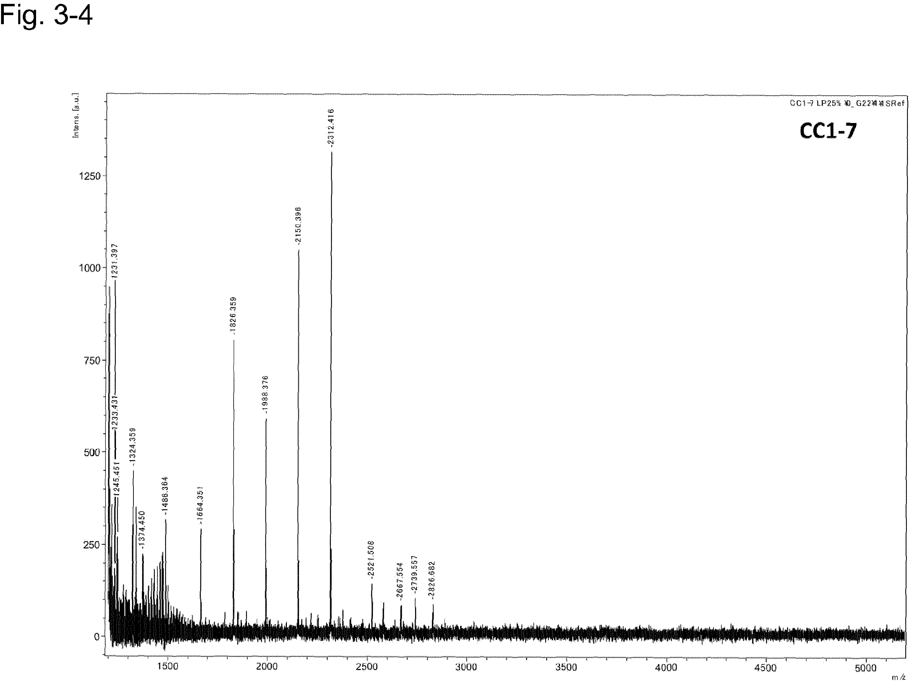

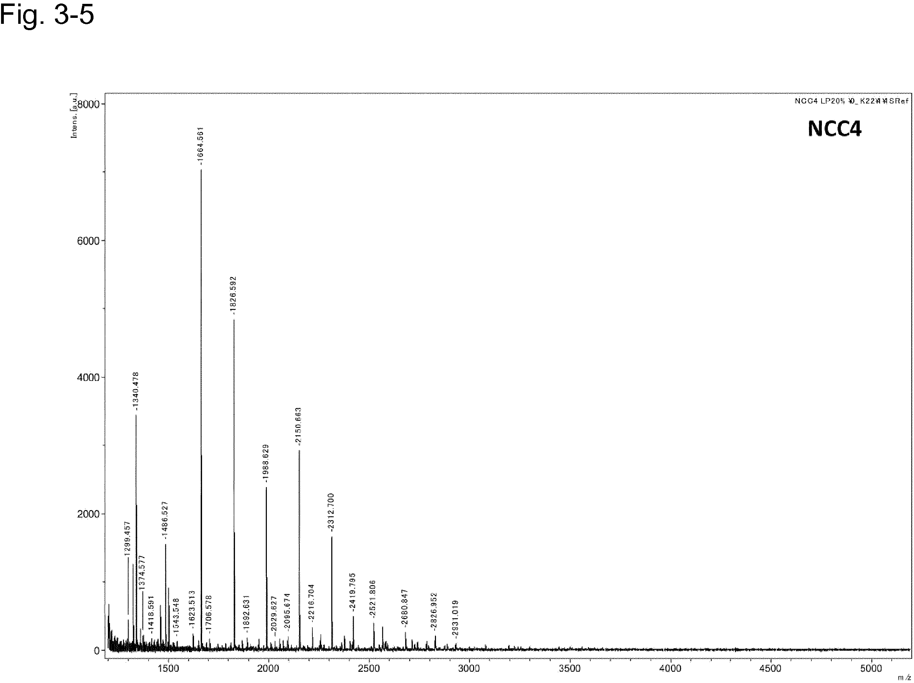

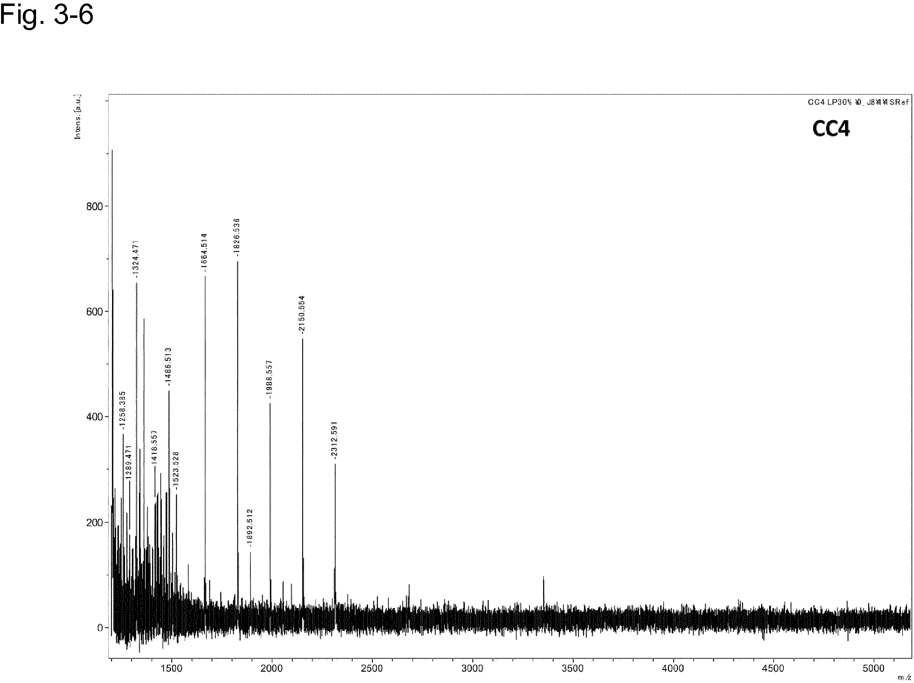

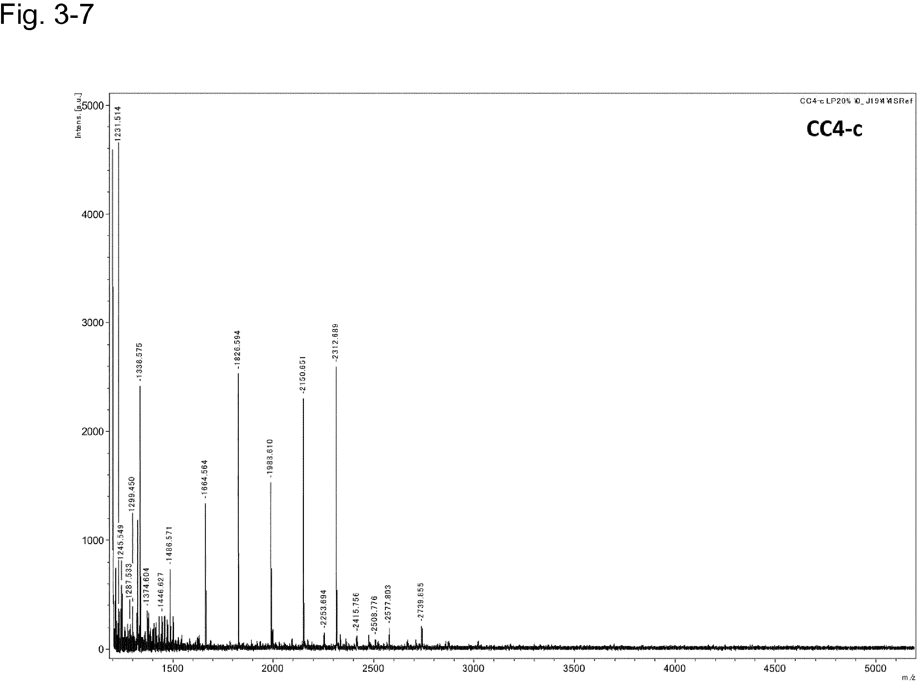

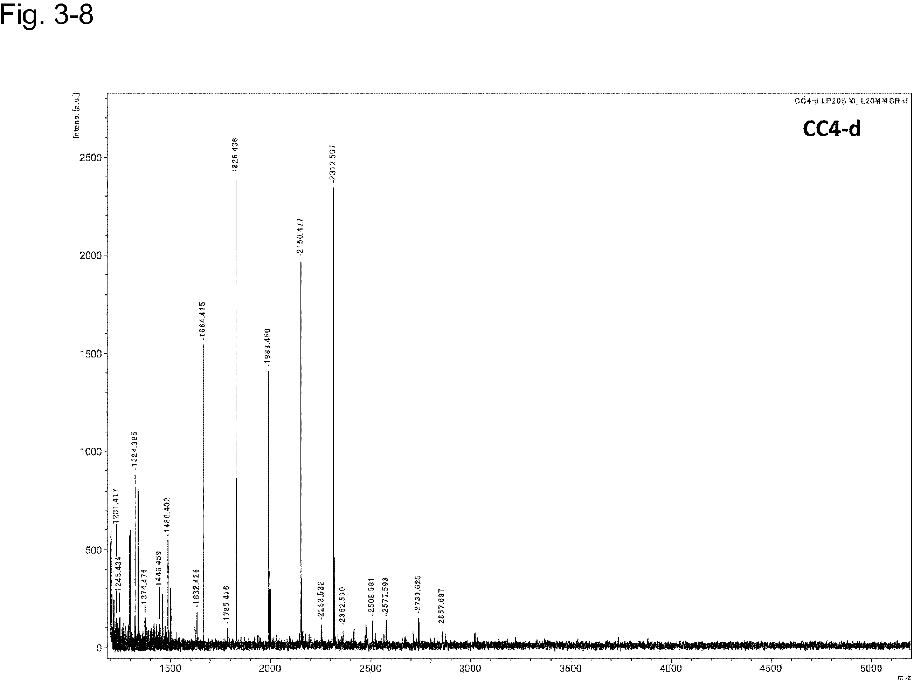

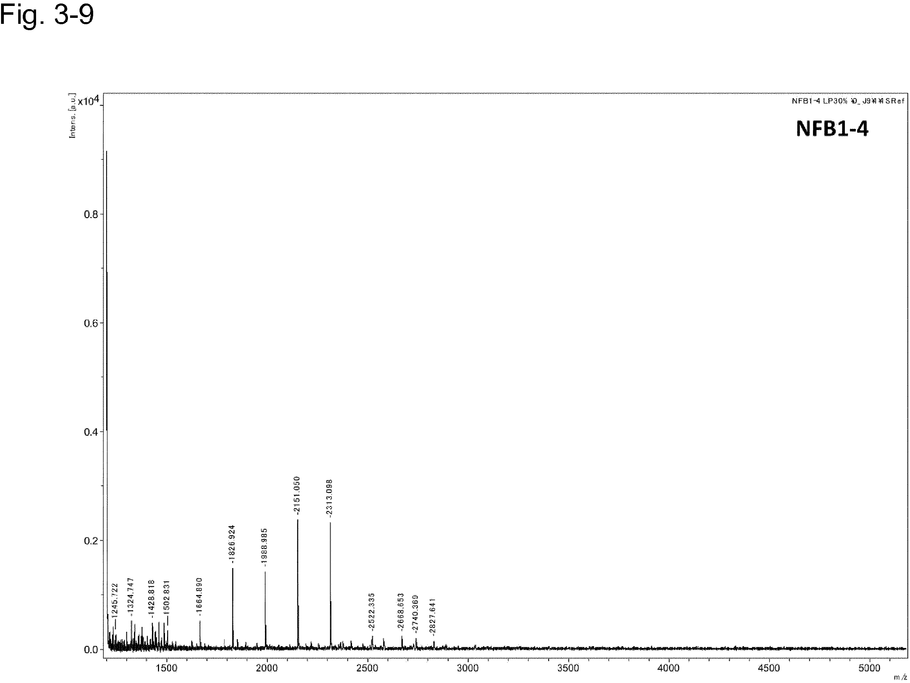

The results of the comparative analyses of sugar chains are shown in Fig. 3. In this figure, the following comparisons were made:

- comparison among the sugar chains ofthe cell population (ncc1) derived from colon non-cancer site tissues (Fig. 3-1), those ofthe cell population (cc1) derived from fresh colon cancer site tissues (Fig. 3-2), and those of the induced malignant stem cells (CC1_2, CC1_7) prepared from fresh colon cancer tissues (Figs. 3-3 and 3-4), which were collected from the individual of donor No. 4;
- comparison among the sugar chains of the cell population (ncc4) derived from colon non-cancer site tissues (Fig. 3-5), those of the cell population (cc4) derived from fresh colon cancer site tissues (Fig. 3-6), and those of the induced malignant stem cells (CC4_c, CC4_D) prepared from fresh colon cancer tissues (Figs. 3-7 and 3-8), which were collected from the individual of donor No. 5; and
- comparison of the above-noted sugar chains with those of the induced pluripotent stem cells (NFB1_4) prepared from fibroblasts (7F3956) (Fig. 3-9) collected from the individual of donor No. 6.

As a result of these comparisons, it was found that the induced malignant stem cells analyzed in this Example can be considered as cells characterized both by aberrations of endogenous cancer-related sugar chains and by expression of the ES cell-specific genes (OCT3/4, NANOG, SOX2, ZFP42). In other words, it was found that the induced malignant stem cells analyzed in this Example can be considered as cells characterized both by having the structure of cancer-related sugar chains and by expression of the ES cell-specific genes (OCT3/4, NANOG, SOX2, ZFP42).

Example 18: Detection for expression of embryonic stem (ES) cell-specific genes in induced malignant stem cells (1)

In this Example, (2) expression of the ES cell-specific genes (SOX2 gene, NANOG gene, OCT3/4 (POU5F1) gene, ZFP42 gene) and the housekeeping gene (GAPDH) in induced malignant stem cells was detected.

(18-1) Materials

The (2) the ES cell-specific genes in induced malignant stem cells was detected by analyzing the levels of the intended genes expressed in the induced malignant stem cells prepared in the present invention using reverse transcription quantitative-PCR (RT-qPCR).

The following samples were used in the detection for (2) the ES cell-specific genes in induced malignant stem cells:

- induced malignant stem cells (GC2_1, GC2_5, GC2_10) prepared from fresh gastric cancer tissues collected from the individual of donor No. 1;
- induced malignant stem cells (CC3_5, CC3_6) prepared from fresh colon cancer tissues collected from the individual of donor No. 2;

- induced malignant stem cells (GC1_4, GC1_6, GC1_7, GC1_8, GC1_9, GC1_10) prepared from fresh gastric cancer tissues, and induced non-malignant stem cells (NGC 1_6, NGC 1_7) prepared from fresh gastric cancer tissues, which were collected from the individual of donor No. 3;
- induced malignant stem cells (CC1_1, CC1_2, CC1_7, CC1_8, CC1_9, CC1_11, CC1_12, CC1_17, CC1_18, CC1_25) prepared from fresh colon cancer tissues collected from the individual of donor No. 4; and
- induced malignant stem cells (CC4_c, CC4_(3), CC4_(6), CC4_(3)_10, CC4_(4), CC4_30, CC4-10, CC4-31, CC4 (1), CC4 (2), CC4-D) prepared from fresh colon cancer tissues collected from the individual of donor No.5.

The induced pulriponent stem (iPS) cells (NFB2-17 cells, 201B7 cells) were used as a positive control expressing the ES cell-specific genes. The NFB2-17 cells are induced pluripotent stem cells that the present inventors prepared from the fibroblasts of the individual of donor No. 7, and the 201B7 cells are induced pluripotent stem cells that were prepared by the Center for iPS Cell Research and Application, Kyoto University and procured from the RIKEN BioResource Center.

(18-2) Procedure

RNA purification:

RNA was purified using miRNeasy Mini Kit (50) (QIAGEN; Cat No. 217004) in accordance with the QIAGEN protocol attached to this kit.

RT-qPCR:

cDNA was synthesized from the thus-purified RNA by performing reaction in a 0.2 mL 8-strip PCR tube (WATSON; Cat No. 337-0208-C) closed with its corresponding 0.2 mL 8-strip PCR cap (WATSON; Cat No. 337-02CP-C) using iScript™ Advanced cDNA Synthesis Kit for RT-qPCR (BIO-RAD; including 5×iScript advanced reaction mix and iScript advanced reverse transcriptase) in accordance with the BIO-RAD protocol attached to this kit. PCR was performed using iCycler (BIO-RAD).

Reverse transcription was performed as follows.
[Table 38]

| Table 38: Reaction solution for use in cDNA synthesis reaction | |
|---|---|
| Component | Volume |
| 5×iScript advanced reaction mix | 2 $\mu$L |
| iScript advanced reverse transcriptase | 0.5 $\mu$L |
| RNA template (Total RNA) | 1 $\mu$g |
| Nuclease-free water | Moderate |
| Total | 10 $\mu$L |

The reaction solution having the composition shown in Table 38 above was placed in respective reaction tubes, and was incubated at 42°C for 30 minutes to perform reverse transcription and then incubated at 85°C for 5 minutes to inactivate reverse transcriptase.
The thus-prepared cDNA was used as a template to perform PCR. The PCR was performed using a reaction mixture with the the following conposition:
[Table 39]

| Table 39: Formulation of PCR solution | |
|---|---|
| SsoAdvanced SYBR Green SuperMix | 2.5 $\mu$L |
| Forward Primer (300 nM) | 0.175 $\mu$L |

(continued)

| Table 39: Formulation of PCR solution | |
|---|---|
| Reverse Primer (300 nM) | 0.175 μL |
| dH2O | 0.9 μL |
| Template | 1.25 μL |
| Total volume | 5 μL |

on the CFX96 Real-Time System (BIO-RAD) in a 96-well PCR plate (Hard-Shell PCR Plate, 96-Well WHT/CLR; BIORAD). The PCR conditions were based on the following protocol: the incubation of 95°C for 30 seconds, followed by 39 cycles of thermal cycle consisting of 95°C for 5 seconds and 60°C for 5 seconds, and then once of the thermal cycle of 95°C for 5 seconds and 65°C for 5 seconds.

The primers used for the PCR performed in this Example are as shown below.

[Table 40]

| Table 40: List of the PCR primers used for respective genes | | | | |
|---|---|---|---|---|
| Primer name | 5'-sequence-3' | Tm | Primer Size | Product size (bp) |
| GAPDH-F | ggcctccaaggagtaagacc | 60.07 | 20 | 147 |
| GAPDH-R | aggggtctacatggcaactg | 59.99 | 20 | |
| OCT3/4-F | agtgagaggcaacctggaga | 59.99 | 20 | 110 |
| OCT3/4-R | acactcggaccacatccttc | 59.97 | 20 | |
| SOX2-F | tggtacggtaggagctttgc | 60.27 | 20 | 80 |
| SOX2-R | tttttcgtcgcttggagact | 59.99 | 20 | |
| NANOG-F | cagtctggacactggctgaa | 60.02 | 20 | 149 |
| NANOG-R | ctcgctgattaggctccaac | 59.98 | 20 | |
| ZFP42-F | gaaagcgcttctctctggac | 59.31 | 20 | 150 |
| ZFP42-R | tcgtatttgcatgcgttagg | 59.72 | 20 | |

(18-3) Results

The results of the PCR performed in this Example are shown in Table 41 below.

[Table 41]

| Table 41A: Expression of ES cell-specific genes (1) and GAPDH | | | | | |
|---|---|---|---|---|---|
| Sample | Cq (Ct) | | | | |
| | SOX2 | NANOG | OCT3/4 | ZFP42 | GAPDH |
| GC2-1 | 19.16 | 20.41 | 17.27 | 22.99 | 15.48 |
| GC2-5 | 21.06 | 23.93 | 18.93 | 25.29 | 17.16 |
| GC2-10 | 19.33 | 20.38 | 17.27 | 23.31 | 15.58 |
| CC3-5 | 20.24 | 21.11 | 17.73 | 29.25 | 15.51 |
| CC3-6 | 20.49 | 22.57 | 18.56 | 26.71 | 16.84 |
| GC1-4 | 20.33 | 22.37 | 18.32 | 24.40 | 16.21 |
| GC1-6 | 19.93 | 22.74 | 18.32 | 25.12 | 16.37 |
| GC1-7 | 19.59 | 21.26 | 18.18 | 24.06 | 15.81 |

(continued)

| Table 41A: Expression of ES cell-specific genes (1) and GAPDH | | | | | |
|---|---|---|---|---|---|
| Sample | Cq (Ct) | | | | |
| | SOX2 | NANOG | OCT3/4 | ZFP42 | GAPDH |
| GC1-8 | 20.14 | 19.56 | 17.14 | 22.21 | 15.24 |
| GC1-9 | 19.72 | 21.32 | 17.37 | 22.15 | 15.44 |
| GC1-10 | 19.41 | 21.38 | 17.33 | 23.76 | 15.84 |
| NGC1-6 | 19.44 | 20.61 | 17.31 | 22.86 | 15.26 |
| NGC1-7 | 19.92 | 21.04 | 17.52 | 23.41 | 15.35 |
| CC1-1 | 19.79 | 21.28 | 17.22 | 22.80 | 15.08 |
| CC1-2 | 19.56 | 21.89 | 17.94 | 24.02 | 15.70 |
| CC1-7 | 19.24 | 21.15 | 17.33 | 23.07 | 15.27 |
| CC1-8 | 19.62 | 20.96 | 16.99 | 23.22 | 15.02 |
| CC1-9 | 19.12 | 21.23 | 17.04 | 23.56 | 15.08 |
| CC1-11 | 19.36 | 21.24 | 17.14 | 23.16 | 15.14 |
| CC1-12 | 19.47 | 21.78 | 16.82 | 23.15 | 15.19 |
| CC1-17 | 19.64 | 20.55 | 17.11 | 22.30 | 15.73 |
| CC1-18 | 19.55 | 22.12 | 18.12 | 25.57 | 16.26 |
| CC1-25 | 19.32 | 21.55 | 17.29 | 24.05 | 15.25 |
| CC4-C | 30.05 | 31.22 | 24.09 | N.T. | 15.46 |
| CC4-D | 32.21 | 31.48 | 24.67 | 34.47 | 16.63 |
| CC4_1 | 27.61 | 31.71 | 24.92 | 37.16 | 16.09 |
| CC4_2 | 33.72 | 31.38 | 24.97 | 36.61 | 17.06 |
| CC4(3) | 28.56 | 30.76 | 24.34 | 36.46 | 15.90 |
| CC4(3)-10 | 29.23 | 30.42 | 23.50 | 38.23 | 15.22 |
| CC4(4) | 28.37 | 30.57 | 23.60 | 37.26 | 15.48 |
| CC4(6) | 26.28 | 30.28 | 23.90 | 34.78 | 15.28 |
| CC4-10 | 31.45 | 32.98 | 24.14 | 36.16 | 15.62 |
| CC4-30 | 30.34 | 30.31 | 23.58 | 36.83 | 15.48 |
| CC4-31 | 32.54 | 30.66 | 23.85 | 39.34 | 15.12 |
| 201B7 | 19.57 | 21.44 | 17.42 | 25.45 | 17.07 |
| Table 41B: Expression of ES cell-specific genes (2) and GAPDH | | | | | |
| | Cq (Ct) | | | | |
| | SOX2 | NANOG | OCT3/4 | ZFP42 | GAPDH |
| GC2-2 | 20.62 | 21.56 | 17.35 | 24.22 | 16.35 |
| GC2-4 | 20.09 | 21.96 | 17.39 | 24.27 | 16.37 |
| GC2-7 | 20.65 | 21.36 | 17.23 | 24.49 | 16.15 |
| GC2-13 | 23.19 | 23.60 | 19.04 | 28.15 | 17.33 |
| GC2-16 | 22.25 | 22.66 | 18.35 | 26.36 | 17.61 |
| CC4-C | 31.15 | 32.63 | 24.10 | 37.26 | 15.20 |

(continued)

| Table 41B: Expression of ES cell-specific genes (2) and GAPDH | | | | | |
|---|---|---|---|---|---|
| | Cq (Ct) | | | | |
| | SOX2 | NANOG | OCT3/4 | ZFP42 | GAPDH |
| CC4_(9)-5 | 37.24 | 31.35 | 24.86 | 39.45 | 16.36 |
| CC4_(9)-11 | 33.77 | 32.85 | 25.76 | 38.08 | 17.44 |
| CC4_(9)-13 | 30.91 | 31.50 | 25.58 | 38.40 | 17.11 |
| NFB2-17 | 21.33 | 24.06 | 18.76 | 28.69 | 16.91 |
| 201B7 | 20.53 | 22.38 | 17.93 | 25.86 | 17.77 |

The expression of the ES cell-specific genes (POU5F1 gene, NANOG gene, SOX2 gene, ZFP42 gene) in induced malignant stem cells was detected by qRT-PCR. As is evident from the results, all the induced malignant stem cells investigated in this Example expressed the ES cell-specific genes (POU5F1 gene, NANOG gene, SOX2 gene, ZFP42 gene). The induced malignant stem cells (GC2_1, GC2_5, GC2_10, CC3_5, CC3_6, , GC1_4, GC1_6, GC1_7, GC1_8, GC1_9, GC1_10, NGC1_6, NGC1_7, CC1_1, CC1_2, CC1_7, CC1_8, CC1_9, CC1_11, CC1_12, CC1_17, CC1_18, CC1_25) expressed the ES cell-specific genes (POU5F1 gene, NANOG gene, SOX2 gene, ZFP42 gene) in amounts almost comparable to (ranging 1/8 to 8 times) the induced pluripotent stem cells (NFB2-17 cells, 201B7 cells).

Example 19: Detection for expression of embryonic stem (ES) cell-specific genes in induced malignant stem cells (2)

In this Example, (2) expression of the ES cell-specific genes in induced malignant stem cells was detected by microarrays. More specifically, gene expression was analyzed using the total RNAs extracted from the four induced malignant stem cells (cc1_1, gc2_1, gc2_5, gc2_10) in Agilent Whole Human Genome Oligo Microarray (4×44K).

(19-1) Materials

The (2) the ES cell-specific genes in induced malignant stem cells was detected by making microarray-based analysis of the expression of the intended genes in the induced malignant stem cells prepared in the present invention.
The following samples were used in the detection for (2) the ES cell-specific genes in induced malignant stem cells:

- induced malignant stem cells (GC2_1, GC2_5, GC2_10) prepared from fresh gastric cancer tissues collected from the individual of donor No. 1; and
- induced malignant stem cells (CC1_1) prepared from fresh colon cancer tissues collected from the individual of donor No. 4.

(19-2) Analysis procedure

In this Example, analysis was performed by basically the same procedure as described in Example 13. More specifically, complementary RNA (cRNA) was synthesized from the targeted RNA extracted from each of the above-described cells and was fluorescently labeled with a cyanine dye, using Agilent Quick Amp Labeling Kit in accordance with the Agilent protocol. The cyanine-dye-labeled cRNA was added to a hybridization buffer and allowed to hybridize for 17 hours with Whole Human Genome Oligo Microarray (4×44K) using Agilent Gene Expression Hybridization Kit. After washing, the DNA microarray image was scanned by Agilent Microarray Scanner, and fluorescence signals from the spots were digitized by Feature Extraction Software (v.10.7.3.1).

(19-3) Experimnental results

As a result of the quality evaluation of the samples described in "(19-2) Materials", the quality of all the samples was assured both by the determination of electrophoresis patterns and by the quantitation of total RNA amounts.
Next, each of the obtained samples were used to synthesize cRNA, and the amounts of fluorescently-labeled cRNAs were determined. As a result, it was confirmed that the cRNAs had been obtained in the amount required for hybridization with a microarray chip.

So, hybridization was performed onto the Agilent Whole Human Genome Oligo DNA Microarray (4×44K) chip with 31 expression probes designed for the following 23 genes characteristically expressed in the human embryonic stem cells. Table 42 below lists the GeneSymbols and Genebank Accession Nos. of these genes.

[Table 42]

| Table 42: Genes characteristically expressed in the human embryonic stem cells | |
|---|---|
| **GeneSymbol** | **GenbankAccession** |
| **ACVR2B** | **M_001106** |
| **CD24** | **L33930** |
| **CDH1** | **NM_004360** |
| **CYP26A1** | **NM_057157** |
| **DNMT3B** | **NM_175850** |
| **DPPA4** | **NM_018189** |
| **EDNRB** | **NM_003991** |
| **FLT1** | **NM_002019** |
| **GABRB3** | **NM_000814** |
| **GATA6** | **NM_005257** |
| **GDF3** | **NM_020634** |
| **GRB7** | **M_005310** |
| **LIN28** | **NM_024674** |
| **NANOG** | **NM_024865** |
| **NODAL** | **NM_018055** |
| **PODXL** | **NM_005397** |
| **POU5F1** | **NM_002701** |
| **SALL4** | **NM_020436** |
| **SOX2** | **NM_003106** |
| **TDGF1** | **NM_003212** |
| **TERT** | **NM_198253** |
| **ZFP42** | **NM_174900** |
| **ZIC3** | **NM_003413** |

As a result of the image analysis performed after the hybridization and washing, it was confirmed that the hybridization had been successfully performed. The images and digital data after the hybridization analyzed by Feature Extraction Software were stored on the storage media.

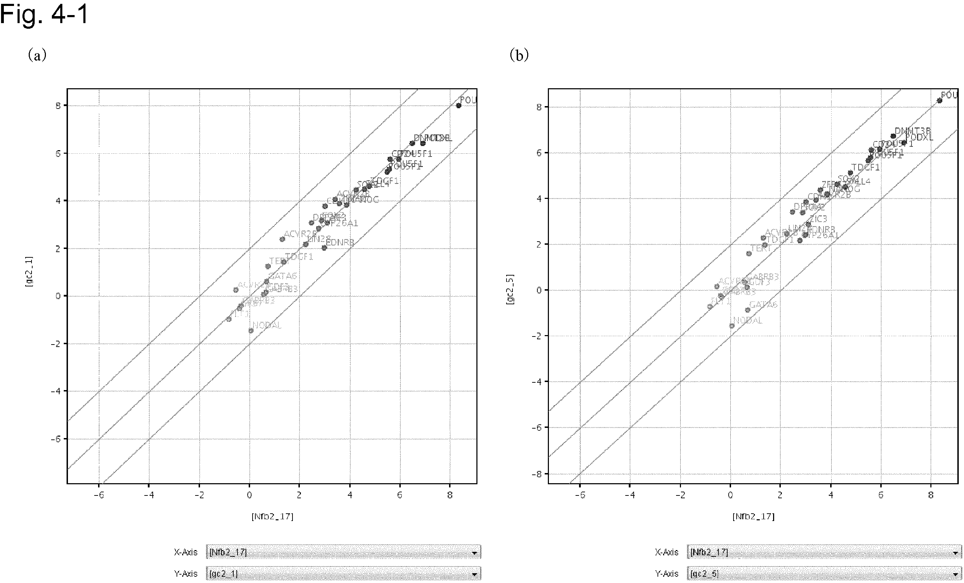

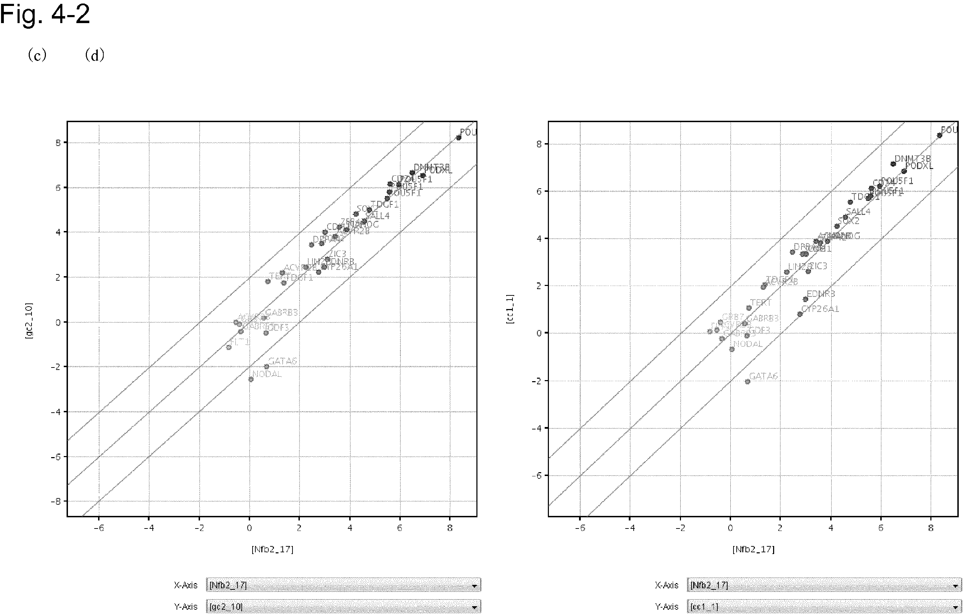

The analysis was performed using GeneSpring. All the 31 probes for the 23 genes characteristically expressed in the human embryonic stem cells as shown in Table 42 were expressed in the human induced malignant stem cells GC2-1, GC2-5, GC2-10 and CC1-1 in amounts almost comparable to (ranging 1/4 to 4 times or 1/8 to 4 times) the human induced pluripotent stem cells NFB2-17 (Fig. 4 (a) to (d)). The lines drawn in each of these figures represent the levels of 1/4 and 4 times.

It was also found that the four human induced malignant stem cells GC2-1, GC2-5, GC2-10 and CC1-1 expressed the 23 ES cell-specific genes at almost comparable levels (within the range of 1/8 to 8 times) to the induced pluripotent stem cells 201B7.

**Claims**

1. An induced malignant stem cell capable of *in vitro* proliferation that is **characterized by** satisfying the following two requirements:

    (1) having at least one aberration selected from among (a) an aberration of methylation (high or low degree of methylation) in a tumor suppressor gene or a cancer-related genetic region in endogenous genomic DNA, (b) a somatic mutation of a tumor suppressor gene or a somatic mutation of an endogenous cancer-related gene in endogenous genomic DNA, (c) abnormal expression (increased or reduced/lost expression) of an endogenous oncogene or an endogenous tumor suppressor gene, (d) abnormal expression (increased or reduced/lost expression) of a noncoding RNA such as an endogenous cancer-related microRNA, (e) abnormal expression of an endogenous cancer-related protein, (f) an aberration of endogenous cancer-related metabolism (hypermetabolism or hypometabolism), (g) an aberration of endogenous cancer-related sugar chain, (h) an aberration of copy number variations in endogenous genomic DNA, and (i) instability of microsatellites in endogenous genomic DNA in an induced malignant stem cell; and
    (2) expressing genes including POU5F1 gene, NANOG gene, SOX2 gene, and ZFP42 gene.

2. The induced malignant stem cell capable of *in vitro* proliferation according to claim 1, wherein the aberration of methylation in a tumor suppressor gene or a cancer-related genetic region in endogenous genomic DNA under (1) (a) above is an aberration of methylation at the 5 position of cytosine base (C) in CpGs located between the genome start point and the genome terminal point of the genomic DNAs (GeneSymbol_NO.) listed in the following table:

[Table 1]

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 1 | ABL2 | 177465262 | 177465849 | 587 | 68 |
| 1 | AF1Q | 149298304 | 149298628 | 324 | 19 |
| 1 | ALU_cons | 159390719 | 159391402 | 683 | 26 |
| 1 | ALU_M1 | 151803096 | 151804508 | 1412 | 23 |
| 1 | ARNT | 149115560 | 149115763 | 203 | 15 |
| 1 | BCL9 | 145181137 | 145181448 | 311 | 31 |
| 1 | CD34_01 | 206150852 | 206151248 | 396 | 33 |
| 1 | CR2_01 | 205694281 | 205694660 | 379 | 34 |
| 1 | EPS15_01 | 51757105 | 51757518 | 413 | 47 |
| 1 | FH | 239748987 | 239749478 | 491 | 47 |
| 1 | HRPT2_001 | 191357448 | 191357799 | 351 | 45 |
| 1 | MUC1_001 | 153429956 | 153430351 | 395 | 23 |
| 1 | MUTYH | 45578118 | 45578622 | 504 | 57 |
| 1 | MYCL1 | 40140552 | 40140860 | 308 | 25 |
| 1 | NTRK_02 | 155095323 | 155095679 | 356 | 24 |
| 1 | NTRK1_001 | 155097132 | 155097659 | 527 | 46 |
| 1 | PAX7_001 | 18829422 | 18829659 | 237 | 15 |
| 1 | PAX7_002 | 18830507 | 18830936 | 429 | 38 |
| 1 | PBX1_001 | 162812066 | 162812615 | 549 | 51 |
| 1 | PDE4DIP | 143751199 | 143751586 | 387 | 32 |

(continued)

| | Table 1: Tumor suppressor genes or cancer-related genetic regions that might cause an aberration of methylation (condition (1) (a)) | | | | |
|---|---|---|---|---|---|
| Chromosome | GeneSymbol_NO | Genome Start Point | Genome Terminal Point | Length of Genome | No. of CpGs |
| 1 | PLOD_01 | 11917237 | 11917716 | 479 | 40 |
| 1 | PMX1 | 168900324 | 168900826 | 502 | 36 |
| 1 | PRCC_01 | 155004541 | 155004781 | 240 | 13 |
| 1 | PRDM16_001 | 2975298 | 2975662 | 364 | 29 |
| 1 | RBM15_001 | 110682735 | 110683276 | 541 | 35 |
| 1 | rhoC_01 | 113051149 | 113051563 | 414 | 41 |
| 1 | RUNX3_01_01 | 25130851 | 25131313 | 462 | 36 |
| 1 | RUNX3_02_01 | 25129323 | 25129742 | 419 | 37 |
| 1 | SATalpha | 121151051 | 121151958 | 907 | 22 |
| 1 | SDHB | 17252913 | 17253355 | 442 | 33 |
| 1 | SDHC_01 | 159550943 | 159551171 | 228 | 15 |
| 1 | SDHC_02 | 159550688 | 159550946 | 258 | 16 |
| 1 | SFPQ_001 | 35430695 | 35431047 | 352 | 35 |
| 1 | SIL_001 | 47552254 | 47552599 | 345 | 33 |
| 1 | STL_01 | 112963027 | 112963505 | 478 | 30 |
| 1 | STL_02 | 112963759 | 112964145 | 386 | 33 |
| 1 | TAF15 | 28842061 | 28842518 | 457 | 39 |
| 1 | TAL1 | 47463678 | 47464167 | 489 | 66 |
| 1 | THRAP3_001 | 36462320 | 36462698 | 378 | 39 |
| 1 | TPM3_001 | 152422158 | 152422410 | 252 | 19 |
| 1 | TPR_001 | 184610787 | 184611141 | 354 | 35 |
| 1 | TRIM33_001 | 114855575 | 114855910 | 335 | 24 |
| 2 | ALK | 29997217 | 29997654 | 437 | 29 |
| 2 | ALU_M5 | 201833637 | 201834637 | 1000 | 24 |
| 2 | ATIC | 215884932 | 215885516 | 584 | 51 |
| 2 | BCL11A_001 | 60634369 | 60634750 | 381 | 32 |
| 2 | CCT4_01 | 61968924 | 61969309 | 385 | 38 |
| 2 | CMKOR1_001 | 237141716 | 237142026 | 310 | 21 |
| 2 | COX7A2L_01 | 42441636 | 42441989 | 353 | 27 |
| 2 | DBI_1_01 | 119840575 | 119840964 | 389 | 31 |
| 2 | ERCC3 | 127767790 | 127768382 | 592 | 51 |
| 2 | FEV | 219557998 | 219558487 | 489 | 55 |
| 2 | HOXD11_001 | 176679790 | 176680265 | 475 | 47 |
| 2 | HOXD13_001 | 176665504 | 176665745 | 241 | 22 |
| 2 | MSH2 | 47483700 | 47484020 | 320 | 34 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 2 | MSH6_01 | 47863130 | 47863577 | 447 | 37 |
| 2 | MYCN_01 | 15999936 | 16000536 | 600 | 74 |
| 2 | MYCN_02 | 15998311 | 15998682 | 371 | 23 |
| 2 | NEDD5_1_01 | 241903199 | 241903499 | 300 | 38 |
| 2 | NEDD5_2_01 | 241903907 | 241904446 | 539 | 65 |
| 2 | PAX3_001 | 222871383 | 222871886 | 503 | 38 |
| 2 | PAX8_01 | 113751340 | 113751782 | 442 | 39 |
| 2 | PAX8_02 | 113751758 | 113752141 | 383 | 31 |
| 2 | PAX8_03 | 113751013 | 113751358 | 345 | 21 |
| 2 | PMS1_01 | 190356952 | 190357548 | 596 | 66 |
| 2 | PMS1_02 | 190357523 | 190357814 | 291 | 25 |
| 2 | REL_001 | 60961862 | 60962359 | 497 | 56 |
| 3 | AF3p21 | 48697853 | 48698277 | 424 | 41 |
| 3 | APOD_01 | 196827214 | 196827678 | 464 | 36 |
| 3 | BCL5_001 | 188937964 | 188938293 | 329 | 19 |
| 3 | BCL6 | 188944627 | 188944968 | 341 | 18 |
| 3 | CTNNB1 | 41215651 | 41216173 | 522 | 58 |
| 3 | ECT2_2_01 | 173951176 | 173951692 | 516 | 33 |
| 3 | EIF4A2 | 187984488 | 187984899 | 411 | 37 |
| 3 | EVI1 | 170346825 | 170347202 | 377 | 31 |
| 3 | FANCD2 | 10042822 | 10043297 | 475 | 41 |
| 3 | GMPS | 157071533 | 157072104 | 571 | 60 |
| 3 | MDS1_001 | 170862884 | 170863415 | 531 | 54 |
| 3 | MLF1_001 | 159771428 | 159771821 | 393 | 32 |
| 3 | MLH1_001 | 37009285 | 37009728 | 443 | 39 |
| 3 | MRPL3_01 | 132704180 | 132704683 | 503 | 39 |
| 3 | PIK3CA_001 | 180349337 | 180349623 | 286 | 30 |
| 3 | PPARG | 12304698 | 12305108 | 410 | 53 |
| 3 | RAR_beta_01 | 25444793 | 25445114 | 321 | 15 |
| 3 | RASSF1 | 50352936 | 50353401 | 465 | 52 |
| 3 | RPN1_001 | 129851885 | 129852431 | 546 | 52 |
| 3 | TFG_001 | 101910877 | 101911384 | 507 | 56 |
| 3 | TFRC_001 | 197293205 | 197293682 | 477 | 47 |
| 3 | VHL_001 | 10158220 | 10158764 | 544 | 69 |
| 3 | ZNF9_001 | 130385378 | 130385695 | 317 | 41 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 4 | ARHH_01 | 39734501 | 39735101 | 600 | 81 |
| 4 | ARHH_02 | 39734502 | 39735102 | 600 | 81 |
| 4 | CCNA2_01 | 122964129 | 122964654 | 525 | 50 |
| 4 | CD38_01 | 15389339 | 15389561 | 222 | 22 |
| 4 | CHIC2_001 | 54625371 | 54625921 | 550 | 65 |
| 4 | FBXW7_001 | 153675457 | 153675857 | 400 | 27 |
| 4 | FGFR3_001 | 1765563 | 1766100 | 537 | 52 |
| 4 | FIP1L1 | 53938235 | 53938640 | 405 | 30 |
| 4 | KIT_001 | 55218601 | 55219070 | 469 | 54 |
| 4 | MLLT2 | 88147105 | 88147579 | 474 | 54 |
| 4 | NMU_01 | 56196612 | 56197197 | 585 | 49 |
| 4 | PDGFRA | 54789115 | 54789455 | 340 | 23 |
| 4 | PHOX2B_001 | 41444001 | 41444391 | 390 | 30 |
| 4 | RAP1GDS1_001 | 99401375 | 99401820 | 445 | 56 |
| 4 | TEC | 47966387 | 47966780 | 393 | 42 |
| 5 | AF5q31 | 132327102 | 132327594 | 492 | 49 |
| 5 | APC | 112224722 | 112225026 | 304 | 26 |
| 5 | ATP6V0E_01 | 172343262 | 172343800 | 538 | 45 |
| 5 | CCNB1_01 | 68498405 | 68499005 | 600 | 44 |
| 5 | CCNH_1_01 | 86743924 | 86744401 | 477 | 28 |
| 5 | CCNH_2_01 | 86744377 | 86744807 | 430 | 35 |
| 5 | F2R_001 | 76047137 | 76047535 | 398 | 28 |
| 5 | FACL6 | 131374976 | 131375381 | 405 | 46 |
| 5 | FLT4 | 180009095 | 180009474 | 379 | 53 |
| 5 | GNB2L1_01 | 180602827 | 180603383 | 556 | 37 |
| 5 | GRAF | 142130593 | 142130977 | 384 | 37 |
| 5 | hB23_1_01 | 170747765 | 170748284 | 519 | 43 |
| 5 | hB23_2_01 | 170747765 | 170748284 | 519 | 43 |
| 5 | HDAC3_01 | 140996361 | 140996781 | 420 | 36 |
| 5 | KCNMB1_01 | 169748654 | 169748935 | 281 | 8 |
| 5 | NPM1 | 170747765 | 170748284 | 519 | 43 |
| 5 | NSD1 | 176492070 | 176492590 | 520 | 51 |
| 5 | NSD1 | 176492070 | 176492590 | 520 | 51 |
| 5 | OXCT_01 | 41906021 | 41906603 | 582 | 50 |
| 5 | RANBP17_001 | 170221621 | 170222169 | 548 | 61 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 5 | TLX3_001 | 170669340 | 170669753 | 413 | 38 |
| 5 | U2AF1RS1_001 | 112255229 | 112255506 | 277 | 6 |
| 6 | C2_1_01 | 31977367 | 31977872 | 505 | 57 |
| 6 | CCNC_01 | 100122997 | 100123403 | 406 | 34 |
| 6 | CCND3_001 | 42016614 | 42017089 | 475 | 34 |
| 6 | DEK_001 | 18372723 | 18373251 | 528 | 65 |
| 6 | ERalpha_02 | 152170751 | 152171138 | 387 | 34 |
| 6 | ESR1_01_01 | 152170469 | 152170794 | 325 | 27 |
| 6 | FANCE | 35527814 | 35528258 | 444 | 42 |
| 6 | FGFR1OP_01 | 167331449 | 167331820 | 371 | 39 |
| 6 | FGFR10P_02 | 167331449 | 167331821 | 372 | 39 |
| 6 | FOXO3A_01 | 108988490 | 108988869 | 379 | 48 |
| 6 | FOXO3A_02 | 108988061 | 108988515 | 454 | 38 |
| 6 | GOPC_001 | 118030207 | 118030715 | 508 | 40 |
| 6 | HIST1H4I | 27215048 | 27215399 | 351 | 32 |
| 6 | HMGA1_001 | 34312298 | 34312861 | 563 | 51 |
| 6 | HSPCB_001 | 44322980 | 44323329 | 349 | 25 |
| 6 | IGF2R_001 | 160310331 | 160310780 | 449 | 59 |
| 6 | IGF2R_002 | 160346693 | 160347065 | 372 | 29 |
| 6 | IGF2R_003 | 160431853 | 160432481 | 628 | 45 |
| 6 | IRF4_001 | 336391 | 336863 | 472 | 48 |
| 6 | MLLT4 | 167971238 | 167971475 | 237 | 18 |
| 6 | Notch4-01 | 32271333 | 32271746 | 413 | 41 |
| 6 | PIM1_01 | 37246325 | 37246801 | 476 | 47 |
| 6 | PIM1_02 | 37246775 | 37247064 | 289 | 18 |
| 6 | PLAGL1_001 | 144371140 | 144371644 | 504 | 47 |
| 6 | PRDM1_01 | 106640781 | 106641136 | 355 | 32 |
| 6 | SFRS3_001 | 36669855 | 36670055 | 200 | 22 |
| 6 | SLC22A1_001 | 160474825 | 160475241 | 416 | 23 |
| 6 | SLC22A2_001 | 160599289 | 160599657 | 368 | 22 |
| 6 | SLC22A3_001 | 160688805 | 160689077 | 272 | 20 |
| 6 | SLC22A3_002 | 160703745 | 160704226 | 481 | 33 |
| 6 | TFEB_001 | 41810490 | 41811016 | 526 | 54 |
| 7 | ASB4_001 | 94995075 | 94995493 | 418 | 22 |
| 7 | BRAF | 140270275 | 140270618 | 343 | 16 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 7 | CAS1_001 | 93977337 | 93977656 | 319 | 34 |
| 7 | CBL | 107171268 | 107171726 | 458 | 34 |
| 7 | CDK6_001 | 92300956 | 92301485 | 529 | 42 |
| 7 | COPG2_001 | 130004373 | 130004597 | 224 | 14 |
| 7 | DNCI1_001 | 95239821 | 95240171 | 350 | 40 |
| 7 | EGFR | 55053588 | 55053949 | 361 | 20 |
| 7 | ELN_01 | 73080258 | 73080525 | 267 | 20 |
| 7 | ETV1_001 | 13995856 | 13996164 | 308 | 19 |
| 7 | GRB10_001 | 50817597 | 50818104 | 507 | 64 |
| 7 | HIP1 | 75205858 | 75206444 | 586 | 55 |
| 7 | HLXB9_001 | 156496339 | 156496819 | 480 | 41 |
| 7 | HOXA1_AB01 | 27101762 | 27102043 | 281 | 18 |
| 7 | HOXA1_SQ05 | 27109677 | 27110061 | 384 | 24 |
| 7 | HOXA10_AB01 | 27180431 | 27180694 | 263 | 23 |
| 7 | HOXA10_SQ02 | 27180440 | 27180963 | 523 | 40 |
| 7 | HOXA11_AB01 | 27191976 | 27192283 | 307 | 17 |
| 7 | HOXA11_SQ01 | 27191540 | 27192000 | 460 | 28 |
| 7 | HOXA13_SQ01 | 27205189 | 27205499 | 310 | 20 |
| 7 | HOXA13_SQ03 | 27205751 | 27206281 | 530 | 72 |
| 7 | HOXA3_AB01 | 27116719 | 27117005 | 286 | 26 |
| 7 | HOXA3_SQ01 | 27116526 | 27117002 | 476 | 45 |
| 7 | HOXA4_AB01 | 27136693 | 27136896 | 203 | 17 |
| 7 | HOXA4_SQ02 | 27136272 | 27136715 | 443 | 55 |
| 7 | HOXA5_AB01 | 27149932 | 27150276 | 344 | 30 |
| 7 | HOXA5_SQ03 | 27149843 | 27150375 | 532 | 39 |
| 7 | HOXA6_AB01 | 27153596 | 27153836 | 240 | 18 |
| 7 | HOXA7_AB01 | 27162508 | 27162921 | 413 | 31 |
| 7 | HOXA7_SQ03 | 27162898 | 27163116 | 218 | 23 |
| 7 | HOXA9_AB01 | 27171578 | 27171938 | 360 | 26 |
| 7 | HOXA9_SQ03 | 27171098 | 27171594 | 496 | 48 |
| 7 | JAZF1_001 | 28186641 | 28187157 | 516 | 47 |
| 7 | MEST_001 | 129913454 | 129913912 | 458 | 35 |
| 7 | MESTIT1_001 | 129918328 | 129918858 | 530 | 34 |
| 7 | MET_001 | 116099294 | 116099611 | 317 | 40 |
| 7 | PDK4_001 | 95063383 | 95063843 | 460 | 39 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 7 | PEG10_001 | 94131513 | 94131935 | 422 | 23 |
| 7 | PIK3CG_01 | 106295442 | 106295890 | 448 | 36 |
| 7 | PMS2 | 6014874 | 6015442 | 568 | 43 |
| 7 | PON1_001 | 94791654 | 94792056 | 402 | 20 |
| 7 | PON2_001 | 94901962 | 94902368 | 406 | 41 |
| 7 | PON3_001 | 94863460 | 94863887 | 427 | 43 |
| 7 | PTPRN2_2_01 | 158073675 | 158074048 | 373 | 34 |
| 7 | SBDS_001 | 66097520 | 66098025 | 505 | 46 |
| 7 | SGCE_001 | 94123033 | 94123358 | 325 | 22 |
| 7 | SMO | 128616273 | 128616798 | 525 | 47 |
| 7 | TIF1_001 | 137795321 | 137795843 | 522 | 59 |
| 8 | AL080059_1_01 | 98359116 | 98359534 | 418 | 57 |
| 8 | AL080059_2_01 | 98358787 | 98359140 | 353 | 35 |
| 8 | CA3_01 | 86537987 | 86538472 | 485 | 33 |
| 8 | CBFA2T1_01 | 93184596 | 93185070 | 474 | 54 |
| 8 | CBFA2T1_02 | 93184184 | 93184618 | 434 | 55 |
| 8 | COX6C_001 | 100974721 | 100974933 | 212 | 19 |
| 8 | MYC | 128819501 | 128820026 | 525 | 40 |
| 8 | NBS1_001 | 91065688 | 91066174 | 486 | 54 |
| 8 | NCOA2_01 | 71478600 | 71479056 | 456 | 28 |
| 8 | NCOA2_02 | 71479039 | 71479412 | 373 | 26 |
| 8 | PCM1 | 17824948 | 17825351 | 403 | 33 |
| 8 | PLAG1_001 | 57286077 | 57286414 | 337 | 26 |
| 8 | RECQL4 | 145713246 | 145713583 | 337 | 23 |
| 8 | TCEA1_001 | 55097189 | 55097737 | 548 | 59 |
| 8 | WHSC1L1_001 | 38359472 | 38360010 | 538 | 54 |
| 9 | ABL1 | 132577525 | 132577958 | 433 | 34 |
| 9 | CDKN2A_01_02 | 21964963 | 21965374 | 411 | 26 |
| 9 | CDKN2A_02_01 | 21984999 | 21985288 | 289 | 27 |
| 9 | CDKN2A_p14ARF | 21985592 | 21986033 | 441 | 39 |
| 9 | CKS2_2_01 | 91115463 | 91115793 | 330 | 28 |
| 9 | CKS2_3_01 | 91115773 | 91116340 | 567 | 62 |
| 9 | COL5A1_01 | 136673725 | 136674245 | 520 | 68 |
| 9 | FANCC_01 | 97119241 | 97119819 | 578 | 61 |
| 9 | FANCC_02 | 97119240 | 97119819 | 579 | 61 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 9 | FANCG_001 | 35069478 | 35070016 | 538 | 41 |
| 9 | FNBP1_001 | 131845061 | 131845514 | 453 | 61 |
| 9 | JAK2_001 | 4974748 | 4975284 | 536 | 56 |
| 9 | MLLT3 | 20610652 | 20611134 | 482 | 37 |
| 9 | NOTCH1_001 | 138560542 | 138560790 | 248 | 23 |
| 9 | NR4A3 | 101624591 | 101625034 | 443 | 26 |
| 9 | NUP214 | 132990566 | 132991025 | 459 | 42 |
| 9 | p16_01 | 21964963 | 21965171 | 208 | 13 |
| 9 | PAX5_001 | 37024038 | 37024514 | 476 | 36 |
| 9 | PAX6_01 | 37027794 | 37028366 | 572 | 45 |
| 9 | PAX6_02 | 37026880 | 37027346 | 466 | 27 |
| 9 | PAX6_03 | 37024512 | 37024773 | 261 | 21 |
| 9 | PSIP2 | 15500124 | 15500613 | 489 | 52 |
| 9 | PSIP2_001 | 15500616 | 15501143 | 527 | 64 |
| 9 | PTCH_01 | 97308581 | 97308982 | 401 | 39 |
| 9 | PTCH_02 | 97308959 | 97309439 | 480 | 24 |
| 9 | PTCH_03 | 97309851 | 97310140 | 289 | 12 |
| 9 | SET_001 | 130490719 | 130490890 | 171 | 8 |
| 9 | SYK_001 | 92603461 | 92603893 | 432 | 37 |
| 9 | TAL2 | 107458199 | 107458779 | 580 | 67 |
| 9 | TSC1_001 | 134809948 | 134810385 | 437 | 39 |
| 10 | BMPR1A_02 | 88506944 | 88507236 | 292 | 22 |
| 10 | COPEB_001 | 3816825 | 3817186 | 361 | 39 |
| 10 | D10S170_01 | 61335497 | 61335783 | 286 | 14 |
| 10 | D10S171_02 | 61336400 | 61336698 | 298 | 27 |
| 10 | FGFR2 | 123347301 | 123347592 | 291 | 33 |
| 10 | FRAT1_001 | 99070069 | 99070493 | 424 | 23 |
| 10 | GDI2_01 | 5895432 | 5896023 | 591 | 66 |
| 10 | MGMT_01_03 | 131155099 | 131155394 | 295 | 38 |
| 10 | MKI67_01 | 129813761 | 129814000 | 239 | 14 |
| 10 | MLLT10 | 21862747 | 21863293 | 546 | 60 |
| 10 | mpp5_01 | 57790897 | 57791267 | 370 | 30 |
| 10 | MYST4_01 | 76256270 | 76256743 | 473 | 43 |
| 10 | MYST4_02 | 76255917 | 76256358 | 441 | 23 |
| 10 | NCOA4_001 | 51242282 | 51242680 | 398 | 35 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 10 | NFKB2_001 | 104143617 | 104144117 | 500 | 40 |
| 10 | NFKB2_002 | 104144801 | 104145274 | 473 | 23 |
| 10 | NFKB2_003 | 104145185 | 104145668 | 483 | 30 |
| 10 | NFKB2_004 | 104144093 | 104144445 | 352 | 29 |
| 10 | NFKB2_005 | 104144383 | 104144828 | 445 | 33 |
| 10 | PTEN_02 | 89613072 | 89613626 | 554 | 65 |
| 10 | RAI17_001 | 80591728 | 80592105 | 377 | 12 |
| 10 | RET_001 | 42891820 | 42892158 | 338 | 20 |
| 10 | SSH3BP1 | 27189110 | 27189610 | 500 | 40 |
| 10 | SUFU | 104253634 | 104254215 | 581 | 58 |
| 10 | TLX1_001 | 102881084 | 102881395 | 311 | 22 |
| 11 | ARHGEF12 | 119712481 | 119712891 | 410 | 49 |
| 11 | ASCL2_001 | 2247867 | 2248329 | 462 | 59 |
| 11 | ATM_001 | 107598808 | 107599243 | 435 | 38 |
| 11 | BC050616_001 | 2377913 | 2378292 | 379 | 33 |
| 11 | CARS_001 | 3035200 | 3035521 | 321 | 31 |
| 11 | CARS_001 | 3034784 | 3035181 | 397 | 31 |
| 11 | CCND1_01 | 69160261 | 69160818 | 557 | 54 |
| 11 | CCND1_02 | 69160263 | 69160817 | 554 | 54 |
| 11 | CCND1_1_01 | 69160261 | 69160817 | 556 | 54 |
| 11 | CCND1_2_01 | 69162041 | 69162617 | 576 | 56 |
| 11 | CCND1_3_01 | 69164429 | 69164933 | 504 | 34 |
| 11 | CD44_01 | 35117193 | 35117609 | 416 | 34 |
| 11 | CD59_01 | 33713926 | 33714365 | 439 | 36 |
| 11 | CD81_001 | 2354853 | 2355382 | 529 | 76 |
| 11 | CD81_002 | 2363131 | 2363578 | 447 | 34 |
| 11 | CD81_003 | 2374118 | 2374563 | 445 | 27 |
| 11 | CDKN1C_001 | 2861490 | 2861724 | 234 | 15 |
| 11 | CDKN1C_002 | 2863931 | 2864321 | 390 | 39 |
| 11 | CRY2_01 | 45825594 | 45826171 | 577 | 52 |
| 11 | DDB2 | 47193104 | 47193534 | 430 | 24 |
| 11 | DDX10_001 | 108040712 | 108041221 | 509 | 38 |
| 11 | DDX6_001 | 118166720 | 118167251 | 531 | 60 |
| 11 | EXT2 | 44073738 | 44074158 | 420 | 54 |
| 11 | FANCF_01 | 22603534 | 22603929 | 395 | 34 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 11 | FANCF_02 | 22603322 | 22603606 | 284 | 19 |
| 11 | FLI1_01 | 128067717 | 128068237 | 520 | 31 |
| 11 | FLI1_02 | 128070143 | 128070375 | 232 | 19 |
| 11 | H19_001 | 1969797 | 1970340 | 543 | 29 |
| 11 | H19_002 | 1974299 | 1974540 | 241 | 22 |
| 11 | H19_003 | 1975988 | 1976465 | 477 | 32 |
| 11 | H19_004 | 1983261 | 1983752 | 491 | 38 |
| 11 | H19_005 | 1990257 | 1990744 | 487 | 28 |
| 11 | HCCA2_001 | 1726222 | 1726591 | 369 | 36 |
| 11 | HCCA2_002 | 1731116 | 1731640 | 524 | 35 |
| 11 | HCCA2_003 | 1741642 | 1741958 | 316 | 46 |
| 11 | HEAB_001 | 57181484 | 57181963 | 479 | 38 |
| 11 | HRAS_001 | 526559 | 527157 | 598 | 61 |
| 11 | HRAS_002 | 524576 | 524948 | 372 | 28 |
| 11 | HSPA8_1_01 | 122438457 | 122438798 | 341 | 25 |
| 11 | HSPA8_2_01 | 122438090 | 122438482 | 392 | 36 |
| 11 | IFITM1_01 | 300575 | 300909 | 334 | 19 |
| 11 | IGF2_001 | 2110661 | 2111061 | 400 | 24 |
| 11 | IGF2_002 | 2118423 | 2118844 | 421 | 49 |
| 11 | IGF2_003 | 2121965 | 2122388 | 423 | 38 |
| 11 | IGF2_004 | 2133388 | 2133777 | 389 | 29 |
| 11 | IL10RA_01 | 117361721 | 117362144 | 423 | 29 |
| 11 | KCNQ1_001 | 2421953 | 2422332 | 379 | 25 |
| 11 | KCNQ1_002 | 2423321 | 2423593 | 272 | 13 |
| 11 | KCNQ1_003 | 2510596 | 2510967 | 371 | 19 |
| 11 | KCNQ1_004 | 2511955 | 2512234 | 279 | 16 |
| 11 | KCNQ1_005 | 2550439 | 2550859 | 420 | 24 |
| 11 | KCNQ1_006 | 2552907 | 2553207 | 300 | 22 |
| 11 | KCNQ1_007 | 2559808 | 2560120 | 312 | 18 |
| 11 | KCNQ1_008 | 2677736 | 2678041 | 305 | 27 |
| 11 | KCNQ1_009 | 2769537 | 2769998 | 461 | 45 |
| 11 | KCNQ1_010 | 2774363 | 2774757 | 394 | 22 |
| 11 | KCNQ1_011 | 2785075 | 2785484 | 409 | 27 |
| 11 | KCNQ1_012 | 2828008 | 2828543 | 535 | 40 |
| 11 | KCNQ1_013 | 2840667 | 2841147 | 480 | 40 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 11 | KCNQ1ON_001 | 2846868 | 2847276 | 408 | 26 |
| 11 | MEN1_01 | 64334283 | 64334680 | 397 | 27 |
| 11 | MEN1_02 | 64333711 | 64334310 | 599 | 48 |
| 11 | MLL_02 | 117811321 | 117811673 | 352 | 22 |
| 11 | MRPL23_001 | 1925380 | 1925658 | 278 | 25 |
| 11 | MRPL23_002 | 1930709 | 1931081 | 372 | 17 |
| 11 | MRPL23_003 | 1934031 | 1934272 | 241 | 17 |
| 11 | MRPL23_004 | 1934636 | 1935034 | 398 | 23 |
| 11 | MRPL23_005 | 1939642 | 1939882 | 240 | 12 |
| 11 | MRPL23_006 | 1942563 | 1942961 | 398 | 27 |
| 11 | MRPL23_007 | 1947611 | 1947817 | 206 | 13 |
| 11 | MYOD_01_02 | 17697769 | 17698203 | 434 | 47 |
| 11 | NAP1L4_001 | 2922455 | 2922829 | 374 | 20 |
| 11 | NAP1L4_002 | 2969310 | 2969834 | 524 | 46 |
| 11 | NUMA1 | 71469069 | 71469354 | 285 | 27 |
| 11 | NUP98_01 | 3774899 | 3775243 | 344 | 28 |
| 11 | NUP98_02 | 3775642 | 3775908 | 266 | 20 |
| 11 | OSBPL5_001 | 3071292 | 3071628 | 336 | 21 |
| 11 | OSBPL5_002 | 3098082 | 3098500 | 418 | 18 |
| 11 | OSBPL5_003 | 3138080 | 3138622 | 542 | 38 |
| 11 | OSBPL5_004 | 3142680 | 3143112 | 432 | 59 |
| 11 | OSBPL5_005 | 3180978 | 3181397 | 419 | 24 |
| 11 | OSBPL5_006 | 3195952 | 3196500 | 548 | 46 |
| 11 | OSBPL5_007 | 3210207 | 3210674 | 467 | 26 |
| 11 | OSBPL5_008 | 3210650 | 3211020 | 370 | 17 |
| 11 | PAFAH1B2_001 | 116519863 | 116520360 | 497 | 43 |
| 11 | PCSK7_001 | 116607790 | 116608224 | 434 | 41 |
| 11 | PHLDA2_001 | 2906487 | 2907015 | 528 | 63 |
| 11 | PICALM_001 | 85457748 | 85458234 | 486 | 43 |
| 11 | PICALM_002 | 85457381 | 85457760 | 379 | 29 |
| 11 | PICALM_01 | 85457748 | 85458189 | 441 | 43 |
| 11 | PRO1073 | 65021396 | 65021824 | 428 | 42 |
| 11 | SDHD | 111462512 | 111462918 | 406 | 30 |
| 11 | SDHD_001 | 111462512 | 111462918 | 406 | 30 |
| 11 | SLC22A18_001 | 2880014 | 2880448 | 434 | 28 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 11 | SLC22A18_002 | 2886839 | 2887277 | 438 | 39 |
| 11 | SLC22A18_003 | 2899297 | 2899799 | 502 | 32 |
| 11 | SYTB_001 | 1803689 | 1803982 | 293 | 18 |
| 11 | TH_001 | 2144026 | 2144524 | 498 | 49 |
| 11 | TNNT3_001 | 1904057 | 1904357 | 300 | 20 |
| 11 | TNNT3_002 | 1905454 | 1905833 | 379 | 22 |
| 11 | TNNT3_003 | 1906788 | 1906996 | 208 | 10 |
| 11 | TNNT3_004 | 1915394 | 1915868 | 474 | 26 |
| 11 | TRPM5_001 | 2391916 | 2392414 | 498 | 32 |
| 11 | TRPM5_002 | 2398577 | 2399002 | 425 | 30 |
| 11 | WT1_001 | 32413036 | 32413392 | 356 | 47 |
| 11 | WT1_001 | 32411966 | 32412340 | 374 | 19 |
| 11 | WT1_002 | 32412703 | 32413062 | 359 | 28 |
| 11 | ZNF145_001 | 113435107 | 113435523 | 416 | 31 |
| 11 | ZNF195_001 | 3391545 | 3391986 | 441 | 21 |
| 11 | ZNF195_002 | 3401174 | 3401406 | 232 | 20 |
| 11 | ZNF215_001 | 6904285 | 6904809 | 524 | 43 |
| 12 | ATF1 | 49443878 | 49444208 | 330 | 30 |
| 12 | BCL7A | 120840600 | 120841134 | 534 | 40 |
| 12 | BTG1 | 91062777 | 91063262 | 485 | 48 |
| 12 | CCND2_001 | 4253140 | 4253668 | 528 | 30 |
| 12 | CCND2_002 | 4253833 | 4254352 | 519 | 35 |
| 12 | CDK4 | 56435611 | 56436146 | 535 | 59 |
| 12 | ELKS | 970533 | 971067 | 534 | 66 |
| 12 | G3PD_01 | 6513830 | 6514401 | 571 | 57 |
| 12 | GLI_01 | 56139875 | 56140298 | 423 | 29 |
| 12 | HAL_01 | 94913485 | 94913865 | 380 | 24 |
| 12 | HMGA_01 | 64505847 | 64506048 | 201 | 17 |
| 12 | HMGA2_001 | 64504118 | 64504535 | 417 | 32 |
| 12 | HOXC11_001 | 52652861 | 52653329 | 468 | 23 |
| 12 | HOXC13_001 | 52619125 | 52619630 | 505 | 52 |
| 12 | NACA | 55405193 | 55405733 | 540 | 37 |
| 12 | PTPN11 | 111341140 | 111341705 | 565 | 60 |
| 12 | SLC38A4_001 | 45511305 | 45511701 | 396 | 29 |
| 12 | TCF1 | 119900742 | 119901144 | 402 | 21 |

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 12 | ZNF384_001 | 6668855 | 6669283 | 428 | 27 |
| 13 | AL137718_01 | 59635924 | 59636310 | 386 | 29 |
| 13 | BRCA2 | 31787393 | 31787925 | 532 | 48 |
| 13 | ERCC5 | 102296508 | 102296807 | 299 | 19 |
| 13 | FLT1_3_01 | 27966522 | 27966938 | 416 | 35 |
| 13 | FLT3 | 27572720 | 27573293 | 573 | 45 |
| 13 | FOXO1A_01 | 40139038 | 40139631 | 593 | 56 |
| 13 | FOXO1A_02 | 40139039 | 40139631 | 592 | 56 |
| 13 | FOXO1A_03 | 40136475 | 40136743 | 268 | 20 |
| 13 | HTR2A_001 | 46367732 | 46368191 | 459 | 9 |
| 13 | RB1_001 | 47775605 | 47776155 | 550 | 70 |
| 13 | ZMF198_001 | 19429932 | 19430275 | 343 | 24 |
| 14 | BCL11B_001 | 98808281 | 98808691 | 410 | 25 |
| 14 | CHGA_01 | 92458933 | 92459492 | 559 | 53 |
| 14 | CR601144_001 | 20528074 | 20528492 | 418 | 23 |
| 14 | DAD1_01 | 22127736 | 22128244 | 508 | 39 |
| 14 | D103_001 | 101095604 | 101096110 | 506 | 49 |
| 14 | DLK1_001 | 100190642 | 100191182 | 540 | 34 |
| 14 | DLK1_002 | 100245239 | 100245459 | 220 | 12 |
| 14 | DLK1_003 | 100262866 | 100263271 | 405 | 59 |
| 14 | DLK1_004 | 100270299 | 100270717 | 418 | 28 |
| 14 | DLK1_005 | 100271281 | 100271557 | 276 | 15 |
| 14 | GOLGA5 | 92330376 | 92330722 | 346 | 28 |
| 14 | GPHN_01 | 66045075 | 66045469 | 394 | 32 |
| 14 | GPHN_02 | 66044621 | 66045096 | 475 | 52 |
| 14 | GPHN_03 | 66044061 | 66044371 | 310 | 23 |
| 14 | HSPCA_001 | 101675929 | 101676415 | 486 | 36 |
| 14 | KTN1_001 | 55116307 | 55116841 | 534 | 71 |
| 14 | MEG3_001 | 100360100 | 100360493 | 393 | 25 |
| 14 | MEG3_001 | 100419237 | 100419637 | 400 | 28 |
| 14 | MEG3_002 | 100362061 | 100362394 | 333 | 19 |
| 14 | MEG3_003 | 100362585 | 100362810 | 225 | 13 |
| 14 | MEG3_004 | 100363911 | 100364143 | 232 | 16 |
| 14 | MEG3_005 | 100418029 | 100418475 | 446 | 27 |
| 14 | N_MYC_1_01 | 20563331 | 20563858 | 527 | 38 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 14 | N_MYC_2_01 | 20562467 | 20562884 | 417 | 29 |
| 14 | NIN_001 | 50368041 | 50368421 | 380 | 15 |
| 14 | PSME2_01 | 23686078 | 23686449 | 371 | 19 |
| 14 | RAD51L1 | 67211132 | 67211662 | 530 | 64 |
| 14 | TCL1A_01 | 95249899 | 95250387 | 488 | 33 |
| 14 | TCL1A_02 | 95250513 | 95250722 | 209 | 9 |
| 14 | TRIP11_001 | 91576058 | 91576347 | 289 | 29 |
| 14 | TSHR_001 | 80490972 | 80491378 | 406 | 27 |
| 15 | AF15Q14 | 38673556 | 38673925 | 369 | 24 |
| 15 | ANXA2_01 | 58477484 | 58477917 | 433 | 32 |
| 15 | ATP10A_001 | 23509898 | 23510365 | 467 | 29 |
| 15 | ATP10A_002 | 23532141 | 23532509 | 368 | 20 |
| 15 | ATP10A_003 | 23658607 | 23659121 | 514 | 43 |
| 15 | ATP10A_004 | 23785703 | 23786045 | 342 | 23 |
| 15 | ATP10A_005 | 23878503 | 23878788 | 285 | 16 |
| 15 | Beta_NAP_01 | 81175787 | 81176040 | 253 | 19 |
| 15 | BLM_001 | 89061315 | 89061847 | 532 | 47 |
| 15 | BUB1B_001 | 38240272 | 38240679 | 407 | 31 |
| 15 | GABRB3_001 | 24425349 | 24425703 | 354 | 26 |
| 15 | GABRB3_002 | 24466474 | 24466763 | 289 | 18 |
| 15 | GABRB3_003 | 24568107 | 24568366 | 259 | 13 |
| 15 | NDN_001 | 21482868 | 21483396 | 528 | 42 |
| 15 | NDN_002 | 21674174 | 21674644 | 470 | 29 |
| 15 | NDN_003 | 21897782 | 21898201 | 419 | 32 |
| 15 | NDN_004 | 22057019 | 22057428 | 409 | 23 |
| 15 | NDN_005 | 22223252 | 22223670 | 418 | 29 |
| 15 | NTRK3 | 86600898 | 86601498 | 600 | 59 |
| 15 | PML_001 | 72077492 | 72077906 | 414 | 27 |
| 15 | RAD51_1_01 | 38774114 | 38774530 | 416 | 33 |
| 15 | RAD51_2_01 | 38774749 | 38775137 | 388 | 37 |
| 15 | RASGRF1_001 | 77169886 | 77170323 | 437 | 46 |
| 15 | SNRPN_001 | 22273701 | 22274205 | 504 | 35 |
| 15 | SNRPN_002 | 22471921 | 22472397 | 476 | 41 |
| 15 | SNRPN_003 | 22569356 | 22569698 | 342 | 28 |
| 15 | SNRPN_004 | 22644252 | 22644787 | 535 | 41 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 15 | SNRPN_005 | 22674608 | 22674881 | 273 | 23 |
| 15 | SNRPN_006 | 22751410 | 22751904 | 494 | 31 |
| 15 | TCF12_01 | 54998178 | 54998751 | 573 | 69 |
| 15 | TCF12_02 | 54997492 | 54997723 | 231 | 20 |
| 15 | UBE3A_001 | 23234955 | 23235465 | 510 | 73 |
| 15 | UBE3A_002 | 23392822 | 23393324 | 502 | 29 |
| 16 | CBFA2T3_001 | 87534056 | 87534546 | 490 | 36 |
| 16 | CBFB | 65619930 | 65620344 | 414 | 22 |
| 16 | CDH1_001 | 67328704 | 67329209 | 505 | 50 |
| 16 | CDH11_001 | 63713205 | 63713703 | 498 | 47 |
| 16 | CREBBP_001 | 3870965 | 3871413 | 448 | 46 |
| 16 | CYLD | 49333974 | 49334203 | 229 | 19 |
| 16 | DC13_1_01 | 79597712 | 79598092 | 380 | 41 |
| 16 | DC13_2_01 | 79598348 | 79598723 | 375 | 32 |
| 16 | DDIT3 | 31098230 | 31098474 | 244 | 13 |
| 16 | E_cad_02 | 67329401 | 67329750 | 349 | 24 |
| 16 | ERCC4 | 13921687 | 13921995 | 308 | 20 |
| 16 | FANCA | 88410663 | 88411053 | 390 | 46 |
| 16 | FUS | 31098697 | 31099112 | 415 | 39 |
| 16 | KIAA0683_01 | 1483654 | 1483960 | 306 | 30 |
| 16 | MAF_001 | 78191338 | 78191880 | 542 | 69 |
| 16 | MHC2TA_01 | 10880484 | 10880911 | 427 | 28 |
| 16 | MYH11_001 | 15858290 | 15858793 | 503 | 44 |
| 16 | TSC2_001 | 2037916 | 2038277 | 361 | 43 |
| 17 | ALO17 | 75849710 | 75850074 | 364 | 19 |
| 17 | ASPSCR1 | 77529129 | 77529451 | 322 | 32 |
| 17 | BHD | 17080723 | 17081162 | 439 | 27 |
| 17 | BIRC5_01 | 73721633 | 73722084 | 451 | 42 |
| 17 | BRCA1 | 38531626 | 38532076 | 450 | 25 |
| 17 | CA4_01 | 55582147 | 55582640 | 493 | 50 |
| 17 | CLTC_001 | 55051668 | 55052177 | 509 | 45 |
| 17 | COL1A1_001 | 45633408 | 45633912 | 504 | 36 |
| 17 | ERBB2_01 | 35110079 | 35110362 | 283 | 23 |
| 17 | ERBB2_02 | 35110081 | 35110361 | 280 | 23 |
| 17 | ETV4_01 | 38978023 | 38978479 | 456 | 36 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 17 | ETV4_02 | 38978021 | 38978479 | 458 | 36 |
| 17 | EXOC7_01 | 71611344 | 71611677 | 333 | 29 |
| 17 | FOXK2_01 | 78070361 | 78070585 | 224 | 29 |
| 17 | GAS7_001 | 10042696 | 10043211 | 515 | 61 |
| 17 | HCMOGT_1_001 | 19999746 | 20000273 | 527 | 56 |
| 17 | HLF | 50697142 | 50697471 | 329 | 45 |
| 17 | MAP2K4_001 | 11864591 | 11865051 | 460 | 49 |
| 17 | MAP2K4_002 | 11865434 | 11865718 | 284 | 22 |
| 17 | MLLT6_01 | 34113070 | 34113580 | 510 | 32 |
| 17 | MLLT6_03 | 34114090 | 34114402 | 312 | 15 |
| 17 | MSF | 72789206 | 72789610 | 404 | 33 |
| 17 | MSI2_001 | 52688381 | 52688824 | 443 | 48 |
| 17 | NF1 | 26445739 | 26446339 | 600 | 45 |
| 17 | Nm23_01 | 46585758 | 46586275 | 517 | 40 |
| 17 | p53_03 | 7532346 | 7532539 | 193 | 20 |
| 17 | PECAM1_01 | 59817588 | 59817941 | 353 | 14 |
| 17 | PER1-001 | 7996232 | 7996656 | 424 | 31 |
| 17 | PRKAR1A | 64019428 | 64019890 | 462 | 41 |
| 17 | PSMB6_01 | 4646233 | 4646687 | 454 | 23 |
| 17 | RARA | 35751090 | 35751589 | 499 | 47 |
| 17 | SUZ12_001 | 27287847 | 27288203 | 356 | 43 |
| 17 | TNTFRSF6_001 | 71448337 | 71448803 | 466 | 42 |
| 17 | TP53_001 | 7532164 | 7532609 | 445 | 36 |
| 18 | ATP5A1_1_01 | 41938228 | 41938674 | 446 | 33 |
| 18 | BCL2_001 | 59138023 | 59138387 | 364 | 50 |
| 18 | FVT1_001 | 59184906 | 59185126 | 220 | 21 |
| 18 | IMPACT_001 | 20260282 | 20260730 | 448 | 41 |
| 18 | MADH4 | 46810401 | 46810721 | 320 | 33 |
| 18 | SS18 | 21924328 | 21924906 | 578 | 51 |
| 18 | TCEB3C_001 | 42809465 | 42809852 | 387 | 28 |
| 19 | AKT2_01 | 45482785 | 45483311 | 526 | 57 |
| 19 | AKT2_02 | 45482787 | 45483311 | 524 | 57 |
| 19 | AURKC_001 | 62433770 | 62434304 | 534 | 38 |
| 19 | AURKC_002 | 62443713 | 62443972 | 259 | 24 |
| 19 | BCL3 | 49943692 | 49944195 | 503 | 67 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 19 | BCL3_001 | 49943692 | 49944195 | 503 | 67 |
| 19 | CDC34_01 | 482976 | 483323 | 347 | 31 |
| 19 | CEBPA_01 | 38485154 | 38486420 | 1266 | 156 |
| 19 | COL5A3_01 | 9981936 | 9982276 | 340 | 36 |
| 19 | COX6B1_1_01 | 40825966 | 40826291 | 325 | 31 |
| 19 | COX6B1_2_01 | 40825956 | 40826257 | 301 | 28 |
| 19 | ELL_001 | 18494063 | 18494512 | 449 | 32 |
| 19 | ERCC2 | 50565436 | 50565898 | 462 | 41 |
| 19 | FSTL3_001 | 626625 | 626920 | 295 | 18 |
| 19 | ICAM1_01 | 10241875 | 10242277 | 402 | 35 |
| 19 | KSRP_1_01 | 6376068 | 6376343 | 275 | 13 |
| 19 | KSRP_2_01 | 6376069 | 6376343 | 274 | 13 |
| 19 | MECT1 | 18655112 | 18655621 | 509 | 63 |
| 19 | MLLT1_001 | 6230380 | 6230801 | 421 | 45 |
| 19 | STK11_01 | 1157536 | 1157912 | 376 | 27 |
| 19 | STK11_02 | 1157893 | 1158270 | 377 | 24 |
| 19 | TCF3_01 | 1597499 | 1597737 | 238 | 14 |
| 19 | TFPT_001 | 59310656 | 59311052 | 396 | 31 |
| 19 | TPM4_001 | 16048692 | 16049124 | 432 | 42 |
| 19 | USP29_001 | 62302435 | 62302863 | 428 | 31 |
| 19 | USP29_002 | 62309367 | 62309891 | 524 | 46 |
| 19 | USP29_003 | 62322196 | 62322469 | 273 | 27 |
| 19 | ZIM2_001 | 61968659 | 61968953 | 294 | 13 |
| 19 | ZIM2_002 | 61998579 | 61998953 | 374 | 30 |
| 19 | ZIM2_003 | 62041908 | 62042346 | 438 | 27 |
| 19 | ZIM2_004 | 62043142 | 62043554 | 412 | 29 |
| 19 | ZIM2_005 | 62043954 | 62044200 | 246 | 9 |
| 19 | ZIM2_006 | 62067585 | 62067965 | 380 | 30 |
| 19 | ZIM3_001 | 62375472 | 62375840 | 368 | 24 |
| 19 | ZMF264_001 | 62394699 | 62395208 | 509 | 49 |
| 19 | ZNF272_001 | 62483493 | 62483962 | 469 | 44 |
| 19 | ZNF331_001 | 58715785 | 58716233 | 448 | 24 |
| 20 | DSTN_01 | 17498585 | 17499165 | 580 | 68 |
| 20 | GNAS_001 | 56848822 | 56849135 | 313 | 30 |
| 20 | GNAS_01 | 56897562 | 56898110 | 548 | 56 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| 20 | GNAS_02 | 56898967 | 56899284 | 317 | 33 |
| 20 | MAFB_001 | 38750860 | 38751343 | 483 | 59 |
| 20 | MYBL2_1_01 | 41729003 | 41729471 | 468 | 57 |
| 20 | MYBL2_2_01 | 41729004 | 41729471 | 467 | 57 |
| 20 | NNAT_001 | 35581984 | 35582269 | 285 | 24 |
| 20 | SS18L1_001 | 60151349 | 60151613 | 264 | 37 |
| 20 | SS18L1_002 | 60152674 | 60153181 | 507 | 55 |
| 20 | TOP1_001 | 39090892 | 39091362 | 470 | 57 |
| 20 | TPD52L2_001 | 61966654 | 61966989 | 335 | 22 |
| 21 | COL6A2_01 | 46356772 | 46357061 | 289 | 24 |
| 21 | ERG_001 | 38955346 | 38955681 | 335 | 20 |
| 21 | OLIG2 | 33317392 | 33317712 | 320 | 22 |
| 21 | RUNX1_001 | 35184917 | 35185243 | 326 | 24 |
| 21 | TMPRSS2_001 | 41802132 | 41802569 | 437 | 30 |
| 22 | BCR_01 | 21853331 | 21853838 | 507 | 69 |
| 22 | BCR_02 | 21853333 | 21853838 | 505 | 69 |
| 22 | CHEK2_001 | 27467870 | 27468262 | 392 | 27 |
| 22 | CLTCL1_001 | 17659116 | 17659652 | 536 | 56 |
| 22 | EP300 | 39817467 | 39817757 | 290 | 22 |
| 22 | EWSR1 | 27994181 | 27994700 | 519 | 58 |
| 22 | GNAZ_01 | 21742354 | 21742845 | 491 | 86 |
| 22 | MKL1_001 | 39362391 | 39363197 | 806 | 76 |
| 22 | MN1 | 26526421 | 26527018 | 597 | 45 |
| 22 | MYH9_001 | 35113893 | 35114426 | 533 | 43 |
| 22 | NDUFA6_01 | 40816187 | 40816786 | 599 | 49 |
| 22 | NF2_001 | 28329371 | 28329908 | 537 | 63 |
| 22 | PDGFB | 37970352 | 37970936 | 584 | 63 |
| 22 | ZMF278_001 | 30072715 | 30073093 | 378 | 30 |
| X | GPC3 | 132947001 | 132947234 | 233 | 21 |
| X | MLLT7 | 70232993 | 70233375 | 382 | 25 |
| X | MSN | 64804313 | 64804586 | 273 | 18 |
| X | MTCP1_001 | 153952418 | 153952966 | 548 | 70 |
| X | NONO_001 | 70420123 | 70420434 | 311 | 24 |
| X | NED017_01 | 102727169 | 102727608 | 439 | 24 |
| X | PAK_3_01 | 110225987 | 110226378 | 391 | 30 |

(continued)

| Table 1: Tumor suppressor genes or cancer-related genetic regions that might cause an aberration of methylation (condition (1) (a)) | | | | | |
|---|---|---|---|---|---|
| Chromosome | GeneSymbol_NO | Genome Start Point | Genome Terminal Point | Length of Genome | No. of CpGs |
| X | SEPT6_001 | 118710422 | 118710923 | 501 | 51 |
| X | TFE3 | 48787429 | 48787872 | 443 | 40 |

**3.** The induced malignant stem cell capable of *in vitro* proliferation according to claim 1, wherein the somatic mutation of a tumor suppressor gene or a somatic mutation of an endogenous cancer-related gene in endogenous genomic DNA under (1) (b) above comprises a passenger mutation.

**4.** The induced malignant stem cell capable of *in vitro* proliferation according to claim 1, wherein the somatic mutation of a tumor suppressor gene or a somatic mutation of an endogenous cancer-related gene in endogenous genomic DNA under (1) (b) above is a driver mutation.

**5.** The induced malignant stem cell capable of *in vitro* proliferation according to claim 1, wherein the somatic mutation of a tumor suppressor gene or a somatic mutation of an endogenous cancer-related gene in endogenous genomic DNA under (1) (b) occurs in at least one of the genes listed in the following table:

[Table 2]

| Table 2: Tumor suppressor genes or endogenous cancer-related genes that might cause somatic mutation (condition (1) (b)) | | |
|---|---|---|
| Chromosome | GeneSymbol | refseq_id |
| 1 | CDK11B | NM_033486, NM_033487, NM_033488, NM_033489, NM_033492, NM_033493, NM_024011, NM_033529 |
| 1 | CDK11A | NM_024011, NM_033486, NM_033487, NM_033488, NM_033489, NM_033492, NM_033493, NM_033529 |
| 1 | PRKCZ | NM_002744, NM_001033581, NM_001033582, NM_001146310 |
| 1 | C1orf86 | NM_001033581, NM_001033582, NM_001146310, NM_002744 |
| 1 | PIK3CD | NM_005026, NM_001009566, NM_014944 |
| 1 | CLSTN1 | NM_005026, NM_001009566, NM_014944 |
| 1 | SRM | NM_003132 |
| 1 | MTOR | NM_004958 |
| 1 | EPHA2 | NM_004431 |
| 1 | PINK1 | NM_032409 |
| 1 | EPHA8 | NM_001006943, NM_020526 |
| 1 | EPHB2 | NM_004442, NM_017449 |
| 1 | PDIK1L | NR_026685, NM_152835, NR_026686 |
| 1 | RPS6KA1 | NM_002953, NM_001006665, NR_031740 |
| 1 | MIR1976 | NM_001006665, NM_002953, NR_031740 |
| 1 | MAP3K6 | NM_004672 |
| 1 | FGR | NM_001042729, NM_001042747, NM_005248 |
| 1 | LCK | NM_005356, NM_001042771 |
| 1 | TSSK3 | NM_052841 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 1 | STK40 | NM_032017 |
| 1 | EPHA10 | NM_001099439, NM_173641 |
| 1 | TIE1 | NM_005424 |
| 1 | RNF220 | NM_018150 |
| 1 | PLK3 | NM_004073, NM_001013632 |
| 1 | TCTEX1D4 | NM_004073, NM_001013632 |
| 1 | TOE1 | NM_007170, NM_025077 |
| 1 | TESK2 | NM_007170, NM_025077 |
| 1 | MAST2 | NM_015112 |
| 1 | PIK3R3 | NM_001114172, NM_003629 |
| 1 | MKNK1 | NM_001135553, NM_003684, NM_198973, NR_024174, NR_024176 |
| 1 | SPATA6 | NM_019073 |
| 1 | PRKAA2 | NM_006252 |
| 1 | ROR1 | NM_001083592, NM_005012 |
| 1 | RAVER2 | NM_002227, NM_018211 |
| 1 | JAK1 | NM_002227, NM_018211 |
| 1 | FPGT | NM_001112808. NM_003838 |
| 1 | TNNI3K | NM_001112808, NM_003838, NM_015978 |
| 1 | PRKACB | NM_002731, NM_207578, NM_182948 |
| 1 | PKN2 | NM_006256 |
| 1 | CDC7 | NM_001134419, NM_003503, NM_001134420 |
| 1 | BRDT | NM_207189, NM_001726 |
| 1 | HIPK1 | NM_152696, NM_198268, NM_198269, NM_181358 |
| 1 | TRIM33 | NM_015906, NM_033020 |
| 1 | NRAS | NM_002524 |
| 1 | PIP5K1A | NM_001135636, NM_001135637, NM_001135638, NM_003557 |
| 1 | PSMD4 | NM_002810 |
| 1 | PI4KB | NM_002651 |
| 1 | NPR1 | NM_000906 |
| 1 | CLK2 | NM_003993 |
| 1 | HCN3 | NM_000298, NM_020897, NM_181871 |
| 1 | PKLR | NM_000298, NM_020897, NM_181871 |
| 1 | SH2D2A | NM_001007792, NM_001161441, NM_001161442, NM_001161443, NM_001161444, NM_003975 |
| 1 | NTRK1 | NM_001007792, NM_001161441, NM_001161442, NM_001161443, NM_001161444, NM_003975, NM_014215, NM_001012331, NM_002529 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 1 | INSRR | NM_001007792, NM_014215 |
| 1 | UHMK1 | NM_001184763, NM_144624, NM_175866 |
| 1 | DDR2 | NM_001014796, NM_006182 |
| 1 | C1orf112 | NM_018186, NM_020423, NM_181093 |
| 1 | SCYL3 | NM_018186, NM_020423, NM_181093 |
| 1 | ABL2 | NM_001136000, NM_001168236, NM_001168237, NM_001168238, NM_001168239, NM_005158, NM_007314, NM_001136001 |
| 1 | RNASEL | NM_021133 |
| 1 | NEK7 | NM_133494 |
| 1 | PIK3C2B | NM_002646 |
| 1 | DSTYK | NM_015375, NM_199462 |
| 1 | NUAK2 | NM_030952 |
| 1 | CDK18 | NM_002596, NM_212502, NM_212503 |
| 1 | IKBKE | NM_014002 |
| 1 | DYRK3 | NM_001004023, NM_003582 |
| 1 | MAPKAPK2 | NM_004759, NM_032960 |
| 1 | CAMK1G | NM_020439 |
| 1 | NEK2 | NM_002497 |
| 1 | RPS6KC1 | NM_001136138. NM_012424 |
| 1 | MARK1 | NM_018650 |
| 1 | ITPKB | NM_002221 |
| 1 | CABC1 | NM_020247 |
| 1 | CDC42BPA | NM_003607, NM_014826 |
| 1 | OBSCN | NM_001098623, NM_052843 |
| 1 | KIAA1804 | NM_032435 |
| 1 | SDCCAG8 | NM_006642, NM_181690 |
| 1 | AKT3 | NM_006642, NM_181690, NM_005465 |
| 2 | ROCK2 | NM_004850 |
| 2 | TRIB2 | NM_021643, NR_027303 |
| 2 | NRBP1 | NM_013392 |
| 2 | ALK | NM_004304 |
| 2 | EIF2AK2 | NM_001135651, NM_001135652, NM_002759 |
| 2 | PRKD3 | NM_005813 |
| 2 | CDKL4 | NM_001009565 |
| 2 | MAP4K3 | NM_003618 |
| 2 | PKDCC | NM_138370 |

(continued)

| Chromosome | GeneSymbol | refseq_id |
|---|---|---|
| | Table 2: Tumor suppressor genes or endogenous cancer-related genes that might cause somatic mutation (condition (1) (b)) |
| 2 | PRKCE | NM_005400 |
| 2 | VRK2 | NM_001130480, NM_001130481, NM_001130482, NM_001130483, NM_001136027, NM_006296, NM_001114636, NM_018062 |
| 2 | FANCL | NM_001114636, NM_001130480, NM_001130481, NM_001130482, NM_001130483, NM_001136027, NM_006296, NM_018062 |
| 2 | ACTR2 | NM_001005386, NM_005722 |
| 2 | AAK1 | NM_014911 |
| 2 | EIF2AK3 | NM_004836 |
| 2 | ZAP70 | NM_001079, NM_207519 |
| 2 | INPP4A | NM_001134224, NM_001134225, NM_001566, NM_004027 |
| 2 | MAP4K4 | NM_004834, NM_145686, NM_145687 |
| 2 | BUB1 | NM_004336 |
| 2 | MERTK | NM_006343 |
| 2 | MAP3K2 | NM_006609 |
| 2 | YSK4 | NM_001018046, NM_025052 |
| 2 | ACVR2A | NM_001616 |
| 2 | ACVR1C | NM_001111031, NM_001111032, NM_001111033, NM_145259 |
| 2 | ACVR1 | NM_001105, NM_001111067 |
| 2 | STK39 | NM_013233 |
| 2 | MYO3B | NM_001083615, NM_001171642, NM_138995 |
| 2 | TLK1 | NM_001136554, NM_001136555, NM_012290 |
| 2 | PDK1 | NM_002610 |
| 2 | ZAK | NM_016653, NM_133646 |
| 2 | MIR548N | NM_003319, NM_133378, NM_133432, NM_133437, NR_031666 |
| 2 | TTN | NM_003319, NM_133378, NM_133432, NM_133437, NR_031666, NM_133379 |
| 2 | STK17B | NM_004226 |
| 2 | CLK1 | NM_001162407, NM_004071, NR_027855, NR_027856 |
| 2 | STRADB | NM_018571 |
| 2 | CDK15 | NM_139158 |
| 2 | BMPR2 | NM_001204 |
| 2 | IDH1 | NM_005896 |
| 2 | PIKFYVE | NM_001178000, NM_015040, NM_152671 |
| 2 | ERBB4 | NM_001042599, NM_005235 |
| 2 | RNF25 | NM_015690, NM_022453 |
| 2 | STK36 | NM_015690, NM_022453 |
| 2 | STK16 | NM_001008910, NR_026909, NM_006000 |

| Table 2: Tumor suppressor genes or endogenous cancer-related genes that might cause somatic mutation (condition (1) (b)) | | |
|---|---|---|
| Chromosome | GeneSymbol | refseq_id |
| 2 | TUBA4A | NM_001008910, NR_026909, NM_006000 |
| 2 | SPEG | NM_005876, NM_001173476 |
| 2 | EPHA4 | NM_004438 |
| 2 | DGKD | NM_003648, NM_152879 |
| 2 | PASK | NM_015148 |
| 2 | STK25 | NM_006374 |
| 3 | OGG1 | NM_002542, NM_003656, NM_016819, NM_016820, NM_016821, NM_016826, NM_016827, NM_016828, NM_016829 |
| 3 | CAMK1 | NM_002542, NM_003656, NM_016819, NM_016820, NM_016821, NM_016826, NM_016827, NM_016828, NM_016829 |
| 3 | IRAK2 | NM_001570 |
| 3 | ATG7 | NM_001136031, NM_001144912, NM_006395 |
| 3 | RAF1 | NM_002880 |
| 3 | KCNH8 | NM_144633 |
| 3 | NEK10 | NM_199347 |
| 3 | TGFBR2 | NM_001024847, NM_003242 |
| 3 | DCLK3 | NM_033403 |
| 3 | MLH1 | NM_000249, NM_001167617, NM_001167618, NM_001167619 |
| 3 | OXSR1 | NM_005109 |
| 3 | ACVR2B | NM_001106 |
| 3 | CTNNB1 | NM_001098209, NM_001098210, NM_001904 |
| 3 | ULK4 | NM_017886 |
| 3 | SNRK | NM_001100594, NM_017719 |
| 3 | IP6K2 | NM_001005909, NM_016291, NM_001005910, NM_001005911, NM_001146178, NM_001146179, NR_027437, NR_027438 |
| 3 | IP6K1 | NM_001006115, NM_153273 |
| 3 | CAMKV | NM_024046 |
| 3 | MST1R | NM_002447 |
| 3 | MAPKAPK3 | NM 004635 |
| 3 | NEK4 | NM_003157 |
| 3 | PRKCD | NM_006254, NM_212539 |
| 3 | PXK | NM_017771 |
| 3 | EPHA3 | NM_005233, NM_182644 |
| 3 | EPHA6 | NM_001080448, NM_173655 |
| 3 | GSK3B | NM_001146156, NM_002093 |
| 3 | MYLK | NM_053025, NM_053026, NM_053027, NM_053028, NM_053031, NM_053032 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 3 | KALRN | NM_001024660, NM_003947, NR_028136, NM_007064 |
| 3 | SNX4 | NM_003794 |
| 3 | PIK3R4 | NM_014602 |
| 3 | NEK11 | NM_001146003, NM_024800, NM_145910 |
| 3 | RYK | NM_001005861, NM_002958 |
| 3 | EPHB1 | NM_004441 |
| 3 | PIK3CB | NM_006219 |
| 3 | GRK7 | NM_139209 |
| 3 | ATR | NM_001184 |
| 3 | PRKCI | NM_002740 |
| 3 | TNIK | NM_001161560, NM_001161561, NM_001161562, NM_001161563, NM_001161564, NM_001161565, NM_001161566, NM_015028, NR_027767 |
| 3 | PIK3CA | NM_006218 |
| 3 | EPHB3 | NM_004443 |
| 3 | MAP3K13 | NM_004721 |
| 3 | DGKG | NM_001080744, NM_001080745, NM_001346 |
| 3 | TNK2 | NM_001010938, NM_005781 |
| 3 | PAK2 | NM_002577 |
| 4 | GAK | NM_005255 |
| 4 | DGKQ | NM_001347 |
| 4 | FGFR3 | NM_000142, NM_001163213, NM_022965 |
| 4 | POLN | NM_024511, NM_181808 |
| 4 | HAUS3 | NM_024511, NM_181808 |
| 4 | GRK4 | NM_001004056, NM_001004057, NM_182982 |
| 4 | STK32B | NM_018401 |
| 4 | KCNIP4 | NM_001035003, NM_001035004, NM_147182, NM_147183 |
| 4 | PI4K2B | NM_018323 |
| 4 | TXK | NM_003328 |
| 4 | TEC | NM_003215 |
| 4 | PDGFRA | NM_006206 |
| 4 | KIT | NM_000222, NM_001093772 |
| 4 | KDR | NM_002253 |
| 4 | EPHA5 | NM_004439, NM_182472 |
| 4 | CDKL2 | NM_003948 |
| 4 | BMP2K | NM_017593, NM_198892 |
| 4 | PRKG2 | NM_006259 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 4 | MAPK10 | NM_002753, NM_138980, NM_138981, NM_138982 |
| 4 | BMPR1B | NM_001203 |
| 4 | NFKB1 | NM_001165412, NM_003998 |
| 4 | TBCK | NM_001163435, NM_001163436, NM_001163437, NM_033115, NM_001142415, NM_004757, NM_001142416 |
| 4 | AIMP1 | NM_001142415, NM_001163435, NM_001163436, NM_001163437, NM_004757, NM_033115, NM_001142416 |
| 4 | ALPK1 | NM_001102406, NM_025144 |
| 4 | CAMK2D | NM_001221, NM_172127, NM_172128, NM_172114, NM_172115, NM_172129 |
| 4 | PLK4 | NM_014264 |
| 4 | ELF2 | NM_201999 |
| 4 | GAB1 | NM_002039, NM_207123 |
| 4 | DCLK2 | NM_001040260, NM_001040261 |
| 4 | FBXW7 | NM_001013415, NM_018315, NM_033632 |
| 4 | NEK1 | NM_012224 |
| 5 | TERT | NM_198253, NM_198255 |
| 5 | TRIO | NM_007118 |
| 5 | PRKAA1 | NM_006251, NM_206907 |
| 5 | MGC42105 | NM_153361 |
| 5 | MAP3K1 | NM_005921 |
| 5 | PLK2 | NM_006622 |
| 5 | MAST4 | NM_001164664, NM_198828, NM_015183 |
| 5 | PIK3R1 | NM_181523, NM_181524, NM_181504 |
| 5 | CDK7 | NM_001799 |
| 5 | SV2C | NM_014979 |
| 5 | RIOK2 | NM_018343, NM_001159749 |
| 5 | FER | NM_005246 |
| 5 | CAMK4 | NM_001744 |
| 5 | APC | NM_001127511, NM_000038, NM_001127510 |
| 5 | MCC | NM_001085377, NM_032028 |
| 5 | TSSK1B | NM_001085377, NM_032028 |
| 5 | CSNK1G3 | NM_001031812, NM_001044722, NM_001044723, NM_004384 |
| 5 | CDKL3 | NM_001113575, NM_016508 |
| 5 | STK32A | NM_001112724, NM_145001 |
| 5 | CSNK1A1 | NM_001025105, NM_001892 |
| 5 | CSF1R | NM_005211 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 5 | PDGFRB | NM_002609 |
| 5 | CAMK2A | NM_015981, NM_171825 |
| 5 | ITK | NM_005546 |
| 5 | ODZ2 | NM_001122679 |
| 5 | STK10 | NM_005990 |
| 5 | FGFR4 | NM_002011, NM_213647, NM_022963 |
| 5 | GRK6 | NM_001004105, NM_001004106, NM_002082 |
| 5 | COL23A1 | NM_173465 |
| 5 | CLK4 | NM_020666 |
| 5 | MAPK9 | NM_002752, NM_139068, NM_139069, NM_139070, NM_001135044 |
| 5 | FLT4 | NM_182925, NM_002020 |
| 6 | MYLK4 | NM_001012418 |
| 6 | RIPK1 | NM_003804 |
| 6 | PRPF4B | NM_003913 |
| 6 | RIOK1 | NM_031480, NM_153005 |
| 6 | PIP5K1P1 | NR_027712 |
| 6 | MAK | NM_005906 |
| 6 | DDR1 | NM_001954, NM_013993, NM_013994 |
| 6 | DOM3Z | NM_005510, NR_026717, NM_004197, NM_032454 |
| 6 | STK19 | NM_005510, NR_026717, NM_004197, NM_032454 |
| 6 | BRD2 | NM_005104, NM_001113182 |
| 6 | IP6K3 | NM_001142883, NM_054111 |
| 6 | SRPK1 | NM_003137 |
| 6 | MAPK14 | NM_001315, NM_139012, NM_139013, NM_139014 |
| 6 | MAPK13 | NM_002754 |
| 6 | STK38 | NM_007271 |
| 6 | PIM1 | NM_002648 |
| 6 | CCND3 | NM_001136017, NM_001136125, NM_001136126, NM_001760 |
| 6 | PTK7 | NM_002821, NM_152880, NM_152881, NM_152882 |
| 6 | TTBK1 | NM_032538 |
| 6 | POLH | NM_006502 |
| 6 | NFKBIE | NM_004556 |
| 6 | ICK | NM_014920, NM_016513 |
| 6 | TTK | NM_001166691, NM_003318 |
| 6 | MAP3K7 | NM_003188, NM_145331, NM_145332, NM_145333 |
| 6 | EPHA7 | NM_004440 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 6 | CDK19 | NM_015076 |
| 6 | FYN | NM_002037, NM_153047, NM_153048 |
| 6 | FRK | NM_002031 |
| 6 | ROS1 | NM_002944 |
| 6 | LAMA2 | NM_000426, NM_001079823 |
| 6 | SGK1 | NM_001143676, NM_001143677, NM_001143678, NM_005627 |
| 6 | MAP3K5 | NM_005923 |
| 6 | LATS1 | NM_004690 |
| 6 | ESR1 | NM_000125, NM_001122740, NM_001122741, NM_001122742 |
| 6 | MAP3K4 | NM_005922, NM_006724 |
| 6 | RPS6KA2 | NM_001006932, NM_021135 |
| 7 | AIMP2 | NM_001134335, NM_006303, NM_014413 |
| 7 | EIF2AK1 | NM_001134335, NM_006303, NM_014413 |
| 7 | RAC1 | NM_006908, NM_018890 |
| 7 | DGKB | NM_004080, NM_145695 |
| 7 | STK31 | NM_001122833, NM_031414, NM_032944 |
| 7 | CDK13 | NM_003718, NM_031267 |
| 7 | STK17A | NM_004760 |
| 7 | GCK | NM_000162, NM_033507, NM_033508 |
| 7 | CAMK2B | NM_001220, NM_172078, NM_172079, NM_172080, NM_172081, NM_172082, NM_172083, NM_172084 |
| 7 | EGFR | NM_005228, NM_201282, NM_201283, NM_201284 |
| 7 | PHKG1 | NM_006213 |
| 7 | LIMK1 | NM_002314 |
| 7 | CDK14 | NM_012395 |
| 7 | CDK6 | NM_001145306, NM_001259 |
| 7 | PDK4 | NM_002612 |
| 7 | LMTK2 | NM_014916 |
| 7 | TRRAP | NM_003496 |
| 7 | EPHB4 | NM_004444 |
| 7 | SRPK2 | NM_182691, NM_182692 |
| 7 | PIK3CG | NM_002649 |
| 7 | MET | NM_000245, NM_001127500 |
| 7 | DGKI | NM_004717 |
| 7 | TRIM24 | NM_003852, NM_015905 |
| 7 | HIPK2 | NM_001113239, NM_022740 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 7 | BRAF | NM_004333 |
| 7 | AGK | NM_018238 |
| 7 | FLJ40852 | NM_001105558, NR_015392 |
| 7 | WEE2 | NM_001105558, NR_015392 |
| 7 | EPHB6 | NM_004445 |
| 7 | EPHA1 | NM_005232 |
| 7 | CDK5 | NM_001164410, NM_004935 |
| 7 | FASTK | NM_006712, NM_033015 |
| 7 | RHEB | NM_005614 |
| 8 | SGK223 | NM_001080826 |
| 8 | BLK | NM_001715 |
| 8 | PTK2B | NM_004103, NM_173174, NM_173175, NM_173176 |
| 8 | PBK | NM_018492 |
| 8 | FGFR1 | NM_001174063, NM_001174064, NM_001174065, NM_001174066, NM_001174067, NM_015850, NM_023105, NM_023106, NM_023110, NM_023107, NM_023108 |
| 8 | IKBKB | NM_001556 |
| 8 | SGK196 | NM_032237 |
| 8 | PRKDC | NM_001081640, NM_006904 |
| 8 | LYN | NM_001111097, NM_002350 |
| 8 | MOS | NM_005372 |
| 8 | SGK3 | NM_001033578, NM_013257, NM_170709 |
| 8 | PSKH2 | NM_033126 |
| 8 | RIPK2 | NM_003821 |
| 8 | STK3 | NM_006281 |
| 8 | PKHD1L1 | NM_177531 |
| 8 | TRIB1 | NM_025195 |
| 8 | MYC | NM_002467 |
| 8 | PTK2 | NM_005607, NM_153831 |
| 8 | MAPK15 | NM_139021 |
| 8 | NRBP2 | NM_178564 |
| 8 | ADCK5 | NM_174922, NM_013291 |
| 8 | CPSF1 | NM_174922, NM_013291 |
| 9 | JAK2 | NM_004972 |
| 9 | CDKN2A | NM_000077, NM_058195, NM_058197 |
| 9 | CDKN2BAS | NM_004936, NM_078487, NR_003529 |
| 9 | CDKN2B | NM_004936, NM_078487, NR_003529 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 9 | TEK | NM_000459 |
| 9 | TAF1L | NM_153809 |
| 9 | PTENP1 | NR_023917 |
| 9 | TESK1 | NM_006285, NM_001782 |
| 9 | CD72 | NM_006285, NM_001782 |
| 9 | NPR2 | NM_003995, NM_172312 |
| 9 | SPAG8 | NM_003995, NM_172312 |
| 9 | MELK | NM_014791 |
| 9 | PIP5K1B | NM_003558 |
| 9 | PRKACG | NM_002732 |
| 9 | TRPM6 | NM_001177310, NM_001177311, NM_017662 |
| 9 | NTRK2 | NM_001018064, NM_006180, NM_001007097, NM_001018065, NM_001018066 |
| 9 | DAPK1 | NM_004938 |
| 9 | CDK20 | NM_001039803, NM_001170639, NM_001170640, NM_012119, NM_178432 |
| 9 | SYK | NM_001135052, NM_003177, NM_001174168, NM_001174167 |
| 9 | ROR2 | NM_004560 |
| 9 | CENPP | NM_001012267, NM_022755 |
| 9 | IPPK | NM_001012267, NM_022755 |
| 9 | WNK2 | NM_006648, NM_001098808 |
| 9 | C9orf129 | NM_001098808, NM_006648 |
| 9 | TGFBR1 | NM_001130916, NM_004612 |
| 9 | MUSK | NM_001166280, NM_001166281, NM_005592 |
| 9 | NEK6 | NM_001166167, NM_001145001, NM_001166168, NM_001166170, NM_001166171, NM_014397, NM_001166169 |
| 9 | CDK9 | NM_001261 |
| 9 | PIP5KL1 | NM_001135219, NM_173492 |
| 9 | PKN3 | NM_013355, NM_032799 |
| 9 | ZDHHC12 | NM_013355, NM_032799 |
| 9 | ABL1 | NM_007313, NM_005157 |
| 9 | C9orf96 | NM_153710, NM_020385 |
| 9 | REXO4 | NM_153710, NM_020385 |
| 9 | NCRNA00094 | NM_007371, NR_015427 |
| 9 | BRD3 | NM_007371, NR_015427 |
| 10 | PRKCQ | NM_006257 |
| 10 | GATA3 | NM_001002295, NM_002051 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 10 | CAMK1D | NM_020397, NM_153498 |
| 10 | PIP4K2A | NM_005028 |
| 10 | MYO3A | NM_017433 |
| 10 | MASTL | NM_001172303, NM_001172304, NM_032844 |
| 10 | MAP3K8 | NM_005204 |
| 10 | RET | NM_020630, NM_020975 |
| 10 | FAM35B | NR_027632 |
| 10 | FAM35B2 | NR_027634 |
| 10 | MAPK8 | NM_002750, NM_139046, NM_139047, NM_139049 |
| 10 | PRKG1 | NM_001098512, NM_006258 |
| 10 | IPMK | NM_152230 |
| 10 | CDK1 | NM_001170406, NM_001170407, NM_001786, NM_033379 |
| 10 | CAMK2G | NM_001222, NM_172169, NM_172170, NM_172171, NM_172173 |
| 10 | BMPR1A | NM_004329 |
| 10 | PTEN | NM_000314 |
| 10 | PIPSL | NR_002319 |
| 10 | PI4K2A | NM_018425 |
| 10 | CHUK | NM_001278 |
| 10 | SLK | NM_014720 |
| 10 | GRK5 | NM_005308 |
| 10 | FGFR2 | NM_000141, NM_001144914, NM_001144915, NM_001144916, NM_001144917, NM_001144918, NM_022970, NM_001144913, NM_001144919 |
| 10 | STK32C | NM_173575 |
| 11 | HRAS | NM_001130442, NM_005343, NM_176795 |
| 11 | BRSK2 | NM_003957 |
| 11 | ILK | NM_001014794, NM_001014795, NM_004517, NM_006284 |
| 11 | TAF10 | NM_001014794, NM_001014795, NM_004517, NM_006284 |
| 11 | STK33 | NM_030906 |
| 11 | WEE1 | NM_003390, NM_001143976 |
| 11 | CSNK2A1P | NM_198516, NR_002207 |
| 11 | GALNTL4 | NM_198516, NR_002207 |
| 11 | PIK3C2A | NM_002645 |
| 11 | HIPK3 | NM_001048200, NM_005734 |
| 11 | DGKZ | NM_201532, NM_201533, NM_003646, NM_001105540 |
| 11 | MARK2 | NM_001039469, NM_001163296, NM_001163297, NM_004954, NM_017490 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 11 | RPS6KA4 | NM_001006944, NM_003942, NR_031602 |
| 11 | MIR1237 | NM_001006944, NM_003942, NR_031602 |
| 11 | MAP4K2 | NM_004579 |
| 11 | CDC42BPG | NM_017525 |
| 11 | SCYL1 | NM_001048218, NM_020680, NM_001130144, NM_001164266, NM_021070 |
| 11 | LTBP3 | NM_001048218, NM_020680, NM_001130144, NM_001164266, NM_021070 |
| 11 | MAP3K11 | NM_002419 |
| 11 | ADRBK1 | NM_001619 |
| 11 | RPS6KB2 | NM_003952 |
| 11 | CCND1 | NM_053056 |
| 11 | PAK1 | NM_001128620, NM_002576 |
| 11 | ATM | NM_000051, NM_138292 |
| 11 | SIK2 | NM_015191, NM_181699, NM_181700 |
| 11 | PPP2R1B | NM_015191, NM_181699, NM_181700 |
| 11 | ANKK1 | NM_178510 |
| 11 | USP28 | NM_020886 |
| 11 | SIK3 | NM_025164 |
| 11 | CHEK1 | NM_001114121, NM_001114122, NM_001274 |
| 12 | WNK1 | NM_001184985, NM_014823, NM_018979, NM_213655 |
| 12 | CCND2 | NM_001759 |
| 12 | DYRK4 | NM_003845 |
| 12 | STYK1 | NM_018423 |
| 12 | GUCY2C | NM_004963 |
| 12 | PIK3C2G | NM_004570 |
| 12 | KRAS | NM_004985, NM_033360 |
| 12 | STK38L | NM_015000 |
| 12 | LRRK2 | NM_198578 |
| 12 | YAF2 | NM_005748 |
| 12 | IRAK4 | NM_001114182, NM_001145256, NM_001145257, NM_001145258, NM_016123 |
| 12 | ACVRL1 | NM_000020, NM_001077401 |
| 12 | ACVR1B | NM_004302, NM_020327, NM_020328 |
| 12 | SP1 | NM_138473, NM_003109 |
| 12 | AMHR2 | NM_001164690, NM_001164691, NM_020547 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 12 | PCBP2 | NM_001098620, NM_001128911, NM_001128912, NM_001128913, NM_001128914, NM_005016, NM_006301, NM_031989 |
| 12 | MAP3K12 | NM_001098620, NM_001128911, NM_001128912, NM_001128913, NM_001128914, NM_005016, NM_006301, NM_031989 |
| 12 | DGKA | NM_001345, NM_201444, NM_201445, NM_201554 |
| 12 | CDK2 | NM_001798, NM_052827 |
| 12 | ERBB3 | NM_001005915, NM_001982 |
| 12 | PIP4K2C | NM_001146258, NM_001146259, NM_001146260, NM_024779 |
| 12 | TSPAN31 | NM_000075, NM_005981 |
| 12 | CDK4 | NM_000075, NM_005981 |
| 12 | TBK1 | NM_013254 |
| 12 | IRAK3 | NM_001142523, NM_007199 |
| 12 | DYRK2 | NM_003583, NM_006482 |
| 12 | CDK17 | NM_002595, NM_001170464 |
| 12 | SCYL2 | NM_017988 |
| 12 | NUAK1 | NM_014840 |
| 12 | C12orf47 | NM_003668, NM_139078, NR_015404 |
| 12 | MAPKAPK5 | NM_003668, NM_139078, NR_015404 |
| 12 | KSR2 | NM_173598 |
| 12 | TAOK3 | NM_016281 |
| 12 | HSPB8 | NM_014365 |
| 12 | CIT | NM_007174, NR_031589 |
| 12 | MIR1178 | NM_007174, NR_031589 |
| 12 | CAMKK2 | NM_006549, NM_153499, NM_153500, NM_172216, NM_172226, NM_172214, NM_172215 |
| 12 | ULK1 | NM_003565 |
| 13 | LATS2 | NM_014572 |
| 13 | CDK8 | NM_001260 |
| 13 | FLT3 | NM_004119 |
| 13 | FLT1 | NM_002019, NM_001160030, NM_001159920, NM_001160031 |
| 13 | BRCA2 | NM_000059 |
| 13 | MIR548F5 | NM_004734, NR_031646 |
| 13 | DCLK1 | NM_004734, NR_031646 |
| 13 | CSNK1A1L | NM_145203 |
| 13 | DGKH | NM_152910, NM_178009 |
| 13 | RB1 | NM_000321 |
| 13 | NEK5 | NM_199289 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 13 | NEK3 | NM_001146099, NM_002498, NM_152720, NR_027415 |
| 13 | STK24 | NM_001032296, NM_003576 |
| 13 | IRS2 | NM_003749 |
| 13 | GRK1 | NM_002929 |
| 14 | TSSK4 | NM_001184739, NM_174944 |
| 14 | RIPK3 | NM_006871 |
| 14 | PRKD1 | NM_002742 |
| 14 | NFKBIA | NM_020529 |
| 14 | CDKL1 | NM_004196 |
| 14 | MAP4K5 | NM_006575, NM_198794 |
| 14 | PRKCH | NM_006255 |
| 14 | ESR2 | NM_001040275, NM_001040276, NM_001437 |
| 14 | MAP3K9 | NM_033141 |
| 14 | RPS6KL1 | NM_031464 |
| 14 | NEK9 | NM_033116 |
| 14 | ADCK1 | NM_001142545, NM_020421 |
| 14 | RPS6KA5 | NM_004755, NM_182398 |
| 14 | ITPK1 | NM_001142594, NM_001142593, NM_014216 |
| 14 | VRK1 | NM_003384 |
| 14 | RAGE | NM_014226 |
| 14 | CDC42BPB | NM_006035 |
| 14 | MARK3 | NM_001128918, NM_001128919, NM_001128920, NM_001128921, NM_002376 |
| 14 | AKT1 | NM_001014431, NM_001014432, NM_005163 |
| 15 | NF1P1 | NR_028506 |
| 15 | LOC646214 | NR_027053 |
| 15 | FAM7A3 | NR_026859, NR_026858, NR_027470 |
| 15 | FAM7A | NM_139320, NM_148911 |
| 15 | FAM7A2 | NR_026858, NR_027470, NR_026859 |
| 15 | FAM7A1 | NR_026858, NR_027470, NR_026859 |
| 15 | EIF2AK4 | NM_001013703 |
| 15 | BUB1B | NM_001211, NM_001128628, NM_001128629 |
| 15 | PAK6 | NM_001128628, NM_001128629, NM_001211, NM_020168 |
| 15 | ITPKA | NM_002220 |
| 15 | LTK | NM_001135685, NM_002344, NM_206961 |
| 15 | TYRO3 | NM_006293 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 15 | TTBK2 | NM_173500 |
| 15 | TRPM7 | NM_017672 |
| 15 | MAPK6 | NM_002748 |
| 15 | DAPK2 | NM_014326 |
| 15 | CSNK1G1 | NM_022048 |
| 15 | MAP2K1 | NM_002755, NM_006049 |
| 15 | SNAPC5 | NM_002755, NM_006049 |
| 15 | MAP2K5 | NM_002757, NM_145160 |
| 15 | CLK3 | NM_003992, NM_001130028 |
| 15 | CSK | NM_001127190, NM_004383 |
| 15 | ULK3 | NM_001099436 |
| 15 | PTPN9 | NM_002833 |
| 15 | ETFA | NM_000126, NM_001127716 |
| 15 | SGK269 | NM_024776 |
| 15 | ALPK3 | NM_020778 |
| 15 | NTRK3 | NM_001012338, NM_002530, NM_001007156 |
| 15 | IDH2 | NM_002168 |
| 15 | FES | NM_001143785, NM_002005, NM_001143783, NM_001143784 |
| 15 | IGF1R | NM_000875 |
| 15 | LRRK1 | NM_024652 |
| 16 | PDPK1 | NM_002613, NM_031268 |
| 16 | LOC652276 | NR_015441 |
| 16 | FLJ42627 | NR_024492 |
| 16 | PAQR4 | NM_004203, NM_152341, NM_182687 |
| 16 | PKMYT1 | NM_004203, NM_152341, NM_182687 |
| 16 | SMG1 | NM_015092 |
| 16 | LOC100271836 | NR_027155 |
| 16 | EEF2K | NM_013302 |
| 16 | LOC641298 | NR_027154 |
| 16 | PALB2 | NM_024675 |
| 16 | PLK1 | NM_005030, NM_033266 |
| 16 | ERN2 | NM_005030, NM_033266 |
| 16 | PRKCB | NM_002738, NM_212535 |
| 16 | SBK1 | NM_001024401 |
| 16 | LOC440354 | NR_002473, NR_002453 |
| 16 | TAOK2 | NM_004783, NM_016151 |

(continued)

| Chromosome | GeneSymbol | refseq_id |
|---|---|---|
| | Table 2: Tumor suppressor genes or endogenous cancer-related genes that might cause somatic mutation (condition (1) (b)) | |
| 16 | LOC100271831 | NM_001040056, NM_001109891, NM_002746, NR_027081 |
| 16 | MAPK3 | NM_001040056, NM_001109891, NM_002746, NR_027081 |
| 16 | LOC595101 | NR_002453, NR_002473 |
| 16 | PHKG2 | NM_000294, NM_001172432 |
| 16 | BCKDK | NM_001122957, NM_005881 |
| 16 | MYLK3 | NM_182493 |
| 16 | CSNK2A2 | NM_001896 |
| 16 | PSKH1 | NM_006742, NM_001907 |
| 16 | CTRL | NM_006742, NM_001907 |
| 16 | CDH1 | NM_004360 |
| 16 | MLKL | NM_001142497, NM_152649 |
| 16 | CDK10 | NM_001098533, NM_001160367, NM_052987, NM_052988, NR_027702, NR_027703, NM_152339 |
| 16 | SPATA2L | NM_001098533, NM_001160367, NM_052987, NM_052988, NR_027702, NR_027703, NM_152339 |
| 17 | ITGAE | NM_002208, NM_031965 |
| 17 | GSG2 | NM_002208, NM_031965 |
| 17 | CAMKK1 | NM_032294, NM_172206, NM_172207 |
| 17 | ANKFY1 | NM_016376, NM_020740 |
| 17 | MINK1 | NM_001024937, NM_015716, NM_153827, NM_170663, NM_000080 |
| 17 | NE | NM_001024937, NM_015716, NM_153827, NM_170663, NM_000080 |
| 17 | TNK1 | NM_003985, NM_020360 |
| 17 | PLSCR3 | NM_003985, NM_020360 |
| 17 | TP53 | NM_000546, NM_001126112, NM_001126113, NM_001126114, NM_001126115, NM_001126116, NM_001126117, NM_001143990, NM_001143991 |
| 17 | WRAP53 | NM_000546, NM_001126112, NM_001126113, NM_001126114, NM_001143990, NM_001143991 |
| 17 | CHD3 | NM_001005271, NM_001005273, NM_005852 |
| 17 | GUCY2D | NM_000180 |
| 17 | AURKB | NM_004217 |
| 17 | PIK3R6 | NM_001010855 |
| 17 | PIK3R5 | NM_001142633, NM_014308 |
| 17 | MAP2K4 | NM_003010 |
| 17 | MAPK7 | NM_139032, NM_139033, NM_002749, NM_139034 |
| 17 | ULK2 | NM_001142610, NM_014683 |
| 17 | MAP2K3 | NM_145109, NM_002756 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 17 | KSR1 | NM_014238 |
| 17 | NLK | NM_016231 |
| 17 | SGK494 | NM_001174103 |
| 17 | NEK8 | NM_178170 |
| 17 | TAOK1 | NM_020791 |
| 17 | NF1 | NM_000267, NM_001042492, NM_001128147 |
| 17 | MYO1D | NM_015194 |
| 17 | ACCN1 | NM_001094 |
| 17 | PIP4K2B | NM_003559 |
| 17 | CDK12 | NM_015083, NM_016507 |
| 17 | ERBB2 | NM_001005862, NM_004448 |
| 17 | CDC6 | NM_001254 |
| 17 | WNK4 | NM_032387 |
| 17 | BRCA1 | NM_007294, NM_007297, NM_007298, NM_007299, NM_007300, NR_027676 |
| 17 | C17orf65 | NM_178542 |
| 17 | LOC100133991 | NM_003954, NR_024434, NR_024435 |
| 17 | MAP3K14 | NM_003954, NR_024434, NR_024435 |
| 17 | PDK2 | NM_002611 |
| 17 | COL1A1 | NM_000088 |
| 17 | ANKFN1 | NM_153228 |
| 17 | DGKE | NM_003647 |
| 17 | TEX14 | NM_031272, NM_198393 |
| 17 | RPS6KB1 | NM_003161 |
| 17 | TLK2 | NM_001112707, NM_006852 |
| 17 | MAP3K3 | NM_002401, NM_203351, NM_030576 |
| 17 | LIMD2 | NM_002401, NM_203351, NM_030576 |
| 17 | STRADA | NM_001003786, NM_001003787, NM_001003788, NM_001165969, NM_001165970, NM_153335 |
| 17 | ERN1 | NM_001433 |
| 17 | PRKCA | NM_002737 |
| 17 | MAP2K6 | NM_002758 |
| 17 | CDK3 | NM_001258 |
| 17 | SPHK1 | NM_001142601, NM_021972, NM_182965, NM_001142602 |
| 17 | BAIAP2 | NM_001080395, NM_001144888, NM_006340, NM_017451 |
| 17 | AATK | NM_001080395, NM_001144888, NM_006340, NM_017451 |
| 17 | CSNK1D | NM_001893, NM_139062 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 18 | YES1 | NM_005433 |
| 18 | ROCK1 | NM_005406 |
| 18 | RIOK3 | NM_003831 |
| 18 | PIK3C3 | NM_002647 |
| 18 | MAPK4 | NM_002747 |
| 18 | ALPK2 | NM_052947 |
| 18 | KIAA1468 | NM_020854 |
| 19 | STK11 | NM_000455 |
| 19 | CSNK1G2 | NM_001319 |
| 19 | MKNK2 | NM_017572, NM_199054 |
| 19 | PIP5K1C | NM_012398 |
| 19 | MATK | NM_002378, NM_139354, NM_139355 |
| 19 | DAPK3 | NM_001348 |
| 19 | MAP2K2 | NM_030662 |
| 19 | INSR | NM_000208, NM_001079817 |
| 19 | MAP2K7 | NM_145185 |
| 19 | TYK2 | NM_003331 |
| 19 | MAST1 | NM_014975 |
| 19 | PRKACA | NM_002730, NM_207518 |
| 19 | PKN1 | NM_002741, NM_213560 |
| 19 | BRD4 | NM_058243, NM_014299 |
| 19 | JAK3 | NM_000215 |
| 19 | MAST3 | NM_015016 |
| 19 | PIK3R2 | NM_005027 |
| 19 | TSSK6 | NM_032037 |
| 19 | LOC284441 | NR_003128 |
| 19 | CCNE1 | NM_001238, NM_057182 |
| 19 | MAP4K1 | NM_001042600, NM_007181 |
| 19 | PAK4 | NM_001014831, NM_001014832, NM_001014834, NM_001014835, NM_005884 |
| 19 | DYRK1B | NM_004714, NM_006483, NM_006484 |
| 19 | MAP3K10 | NM_002446 |
| 19 | AKT2 | NM_001626 |
| 19 | HIPK4 | NM_144685 |
| 19 | ADCK4 | NM_001142555, NM_024876 |
| 19 | ITPKC | NM_025194, NM_198476 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 19 | C19orf54 | NM_025194, NM_198476 |
| 19 | AXL | NM_001699, NM_021913 |
| 19 | GSK3A | NM_019884 |
| 19 | MARK4 | NM_031417 |
| 19 | DMPK | NM_001081560, NM_001081562, NM_001081563, NM_004409 |
| 19 | PRKD2 | NM_001079880, NM_001079881, NM_001079882, NM_016457 |
| 19 | LMTK3 | NM_001080434 |
| 19 | SPHK2 | NM_020126 |
| 19 | VRK3 | NM_001025778, NM_016440 |
| 19 | PRKCG | NM_002739 |
| 19 | BRSK1 | NM_032430 |
| 19 | PBK2 | NM_001101401 |
| 19 | AURKC | NM_001015878, NM_001015879, NM_003160 |
| 19 | TRIM28 | NM_005762 |
| 20 | TRIB3 | NM_021158 |
| 20 | CSNK2A1 | NM_001895, NM_177559, NM_177560 |
| 20 | STK35 | NM_080836 |
| 20 | PAK7 | NM_020341, NM_177990 |
| 20 | MYLK2 | NM_033118 |
| 20 | HCK | NM_001172129, NM_001172130, NM_001172131, NM_001172132, NM_001172133, NM_002110 |
| 20 | RALY | NM_007367, NM_016732 |
| 20 | SRC | NM_005417, NM_198291 |
| 20 | SGK2 | NM_170693, NM_016276 |
| 20 | STK4 | NM_006282 |
| 20 | TP53RK | NM_033550 |
| 20 | AURKA | NM_003600, NM_198433, NM_198434, NM_198435, NM_198436, NM_198437 |
| 20 | PTK6 | NM_005975 |
| 20 | SRMS | NM_080823 |
| 21 | HUNK | NM_014586 |
| 21 | DYRK1A | NM_101395, NM_130436, NM_001396, NM_130438 |
| 21 | RIPK4 | NM_020639 |
| 21 | SIK1 | NM_173354 |
| 22 | TSSK2 | NM_022719, NM_053006 |
| 22 | DGCR14 | NM_022719, NM_053006 |
| 22 | PI4KAP1 | NR_003563 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| 22 | PI4KA | NM_002650, NM_058004 |
| 22 | PI4KAP2 | NR_003700 |
| 22 | MAPK1 | NM_002745, NM_138957 |
| 22 | ADRBK2 | NM_005160 |
| 22 | CHEK2 | NM_001005735, NM_007194, NM_145862 |
| 22 | NF2 | NM_000268, NM_016418, NM_181825, NM_181828, NM_181829, NM_181830, NM_181831, NM_181832, NM_181833 |
| 22 | LIMK2 | NM_005569, NM_001031801, NM_016733 |
| 22 | CSNK1E | NM_001894, NM_152221 |
| 22 | CERK | NM_022766 |
| 22 | PIM3 | NM_001001852 |
| 22 | MAPK12 | NM_002969 |
| 22 | MAPK11 | NM_002751 |
| X | PRKX | NM_005044 |
| X | BMX | NM_203281, NM_001721 |
| X | CDKL5 | NM_003159, NM_001037343, NM_000330 |
| X | RS1 | NM_000330, NM_001037343, NM_003159 |
| X | PDHA1 | NM_000284, NM_001001671, NM_001173454, NM_001173455, NM_001173456 |
| X | MAP3K15 | NM_000284, NM_001001671, NM_001173454, NM_001173455, NM_001173456 |
| X | RPS6KA3 | NM_004586 |
| X | CNKSR2 | NM_001168647, NM_001168648, NM_001168649, NM_014927 |
| X | PDK3 | NM_001142386, NM_005391 |
| X | CASK | NM_001126054, NM_001126055, NM_003688 |
| X | CDK16 | NM_033018, NM_006201, NM_001170460 |
| X | ARAF | NM_001654, NM_006950, NM_133499 |
| X | SYN1 | NM_001654, NM_006950, NM_133499 |
| X | PIM2 | NM_006875 |
| X | WNK3 | NM_001002838, NM_020922 |
| X | TAF1 | NM_004606, NM_138923, NR_001568 |
| X | BCYRN1 | NM_004606, NM_138923, NR_001568 |
| X | PHKA1 | NM_001122670, NM_001172436, NM_002637 |
| X | LOC139201 | NR_029423 |
| X | NCRNA00182 | NR_028379 |
| X | RPS6KA6 | NM_014496 |
| X | KLHL4 | NM_019117, NM_057162 |

(continued)

| Table 2: Tumor suppressor genes or endogenous cancer-related genes that might cause somatic mutation (condition (1) (b)) | | |
|---|---|---|
| Chromosome | GeneSymbol | refseq_id |
| X | BTK | NM_000061 |
| X | NRK | NM_198465 |
| X | IRS4 | NM_003604 |
| X | GUCY2F | NM_001522 |
| X | PAK3 | NM_001128166, NM_001128167, NM_002578, NM_001128168, NM_001128172, NM_001128173 |
| X | MST4 | NM_001042453, NM_016542, NM_001042452 |
| X | PNCK | NM_001039582, NM_001135740 |
| X | SRPK3 | NM_001170760, NM_001170761, NM_014370, NM_004135, NM_174869 |
| X | IDH3G | NM_001170760, NM_001170761, NM_014370, NM_004135, NM_174869 |
| X | IRAK1 | NM_001025242, NM_001025243, NM_001569 |
| Y | PRKY | NR_028062 |

or is depicted in at least one amino acid mutation (mutation ID) in the proteins listed in the following table:

[Table 3]

Table 3: Tumor suppressor genes or endogenous cancer-related genes that might cause somatic mutation (condition (1) (b))

| Chromosome | Gene Symbol | Mut_ID |
|---|---|---|
| 1 | AKT3 | E17K |
| 1 | EPHA10 | E124K |
| 1 | KRAS | Q61L/Q61R/Q61P |
| 1 | NRAS | A18T |
| 1 | NRAS | A59T |
| 1 | NRAS | G12 |
| 1 | NRAS | G12C/G12R/G12S |
| 1 | NRAS | G12V/G12A/G12D |
| 1 | NRAS | G13 |
| 1 | NRAS | G13C/G13R/G13S |
| 1 | NRAS | G13V/G13A/G13D |
| 1 | NRAS | G48S |
| 1 | NRAS | Q61 |
| 1 | NRAS | Q61 |
| 1 | NRAS | Q61 |
| 1 | NRAS | Q61E/Q61K |
| 1 | NRAS | Q61H |
| 1 | NRAS | Q61L/Q61R/Q61P |
| 2 | CXCR4 | V160I |
| 2 | ERBB4 | E452K |
| 2 | ERBB4 | R393W |
| 2 | SOS1 | H888Q |

| | | |
|---|---|---|
| 2 | SOS1 | R248H |
| 2 | SOS1 | R688Q |
| 3 | CTNNB1 | A13T |
| 3 | CTNNB1 | A21T |
| 3 | CTNNB1 | D32A |
| 3 | CTNNB1 | D32G |
| 3 | CTNNB1 | D32H/N/Y |
| 3 | CTNNB1 | D32V |
| 3 | CTNNB1 | G34E |
| 3 | CTNNB1 | G34E/V |
| 3 | CTNNB1 | G34R |
| 3 | CTNNB1 | G34R |
| 3 | CTNNB1 | G34V |
| 3 | CTNNB1 | S33/F/Y/C |
| 3 | CTNNB1 | S33C |
| 3 | CTNNB1 | S33F |
| 3 | CTNNB1 | S33P |
| 3 | CTNNB1 | S33Y |
| 3 | CTNNB1 | S37A |
| 3 | CTNNB1 | S37A |
| 3 | CTNNB1 | S37C |
| 3 | CTNNB1 | S37C/F/Y |
| 3 | CTNNB1 | S37F |
| 3 | CTNNB1 | S37P |

| 3 | CTNNB1 | S37Y | 3 | PIK3CA | N1044K |
|---|--------|------|---|--------|--------|
| 3 | CTNNB1 | S45 | 3 | PIK3CA | N1068fs*4 |
| 3 | CTNNB1 | S45A | 3 | PIK3CA | N345K |
| 3 | CTNNB1 | S45C | 3 | PIK3CA | P539R |
| 3 | CTNNB1 | S45C/F/Y | 3 | PIK3CA | Q546E/K |
| 3 | CTNNB1 | S45F | 3 | PIK3CA | Q546H |
| 3 | CTNNB1 | S45P | 3 | PIK3CA | Q546K |
| 3 | CTNNB1 | S45P | 3 | PIK3CA | Q546R/P |
| 3 | CTNNB1 | S45Y | 3 | PIK3CA | R1023Q |
| 3 | CTNNB1 | T41A | 3 | PIK3CA | R38H |
| 3 | CTNNB1 | T41A/S | 3 | PIK3CA | R88Q |
| 3 | CTNNB1 | T41I | 3 | PIK3CA | R88Q |
| 3 | CTNNB1 | T41I | 3 | PIK3CA | S326F |
| 3 | CTNNB1 | T41I | 3 | PIK3CA | T1025A |
| 3 | CTNNB1 | T41P | 3 | PIK3CA | T1025S/I |
| 3 | CTNNB1 | T41S | 3 | PIK3CA | Y1021C |
| 3 | CTNNB1 | V22_G38del | 3 | PIK3CA | Y1021C |
| 3 | CTNNB1 | V22A | 3 | PIK3CA | Y1021H |
| 3 | CTNNB1 | W25_D32del | 3 | VHl | F148fs*11 |
| 3 | MLH1 | V384D | 3 | VHl | L158Q |
| 3 | NEK10 | E379K | 3 | VHl | L85P |
| 3 | PIK3CA | A1035T | 3 | VHl | L89H |
| 3 | PIK3CA | A1035V | 3 | VHl | P81S |
| 3 | PIK3CA | C420R | 3 | VHl | R161* |
| 3 | PIK3CA | C901F | 3 | VHl | R167W |
| 3 | PIK3CA | E418K | 4 | FBXW7 | R465C |
| 3 | PIK3CA | E542K | 4 | FBXW7 | R465H |
| 3 | PIK3CA | E542Q/K | 4 | FBXW7 | R479G |
| 3 | PIK3CA | E542V | 4 | FBXW7 | R479Q/L |
| 3 | PIK3CA | E545A | 4 | FGFR3 | A281V |
| 3 | PIK3CA | E545G | 4 | FGFR3 | A391E |
| 3 | PIK3CA | E545G/A | 4 | FGFR3 | G370C |
| 3 | PIK3CA | E545K | 4 | FGFR3 | K650Q/K650E |
| 3 | PIK3CA | E545Q/K | 4 | FGFR3 | K650T/K650M |
| 3 | PIK3CA | G1007R | 4 | FGFR3 | Y373C |
| 3 | PIK3CA | H1047R/H1047L | 4 | KIT | A829P |
| 3 | PIK3CA | H1047Y | 4 | KIT | C809G |
| 3 | PIK3CA | H1065L | 4 | KIT | D52N |
| 3 | PIK3CA | H701P | 4 | KIT | D579del |
| 3 | PIK3CA | I1058F | 4 | KIT | D716N |
| 3 | PIK3CA | M1004I | 4 | KIT | D816E |
| 3 | PIK3CA | M1043I/M1043I | 4 | KIT | D816F |
| 3 | PIK3CA | M1043V | 4 | KIT | D816H/D816Y |

| 4 | KIT | D816V | 4 | KIT | V559_V560del |
|---|-----|-------|---|-----|--------------|
| 4 | KIT | D816V/G/A | 4 | KIT | V559A |
| 4 | KIT | D820E | 4 | KIT | V559D/V559A/V559G |
| 4 | KIT | D820G/A | 4 | KIT | V559del |
| 4 | KIT | D820H/Y | 4 | KIT | V559I |
| 4 | KIT | D820Y | 4 | KIT | V560D/V560G |
| 4 | KIT | E561K | 4 | KIT | V560del |
| 4 | KIT | E839K | 4 | KIT | V560E |
| 4 | KIT | F584S | 4 | KIT | V569G |
| 4 | KIT | G565R | 4 | KIT | V654A |
| 4 | KIT | K492R | 4 | KIT | V654A |
| 4 | KIT | K550_K558del | 4 | KIT | V825A |
| 4 | KIT | K558_E562del | 4 | KIT | W557R |
| 4 | KIT | K558_V560del | 4 | KIT | W557R/W557R/W557G |
| 4 | KIT | K558N | 4 | KIT | Y503_F504insAY |
| 4 | KIT | K558R | 4 | KIT | Y553_Q556del |
| 4 | KIT | K642E | 4 | KIT | Y553K |
| 4 | KIT | K642E | 4 | KIT | Y553N |
| 4 | KIT | K685E | 4 | KIT | Y568D |
| 4 | KIT | L576P | 4 | KIT | Y570_L576del |
| 4 | KIT | L576P | 4 | KIT | Y675C |
| 4 | KIT | M535I | 4 | KIT | Y823D |
| 4 | KIT | M535T | 4 | PDGFRA | D1071N |
| 4 | KIT | M535V | 4 | PDGFRA | D842_D846>E |
| 4 | KIT | M552L | 4 | PDGFRA | D842_D846>G |
| 4 | KIT | N566D | 4 | PDGFRA | D842_D846>N |
| 4 | KIT | N655K | 4 | PDGFRA | D842_H845del |
| 4 | KIT | N822H/Y | 4 | PDGFRA | D842_M844del |
| 4 | KIT | N822K | 4 | PDGFRA | D842_S847>EA |
| 4 | KIT | N822K | 4 | PDGFRA | D842F |
| 4 | KIT | P551_V555del | 4 | PDGFRA | D842I |
| 4 | KIT | P551_V555del | 4 | PDGFRA | D842V |
| 4 | KIT | P573A | 4 | PDGFRA | D842V |
| 4 | KIT | P573L | 4 | PDGFRA | D842Y |
| 4 | KIT | P585P | 4 | PDGFRA | D842Y |
| 4 | KIT | R634W | 4 | PDGFRA | D846Y |
| 4 | KIT | R739G | 4 | PDGFRA | E996K |
| 4 | KIT | S709F | 4 | PDGFRA | F808L |
| 4 | KIT | T574A | 4 | PDGFRA | H845_N848>P |
| 4 | KIT | T670E | 4 | PDGFRA | I843_D846del |
| 4 | KIT | T670I | 4 | PDGFRA | I843_S847>T |
| 4 | KIT | T670I | 4 | PDGFRA | N659K |
| 4 | KIT | T753A | 4 | PDGFRA | N870S |

| 4 | PDGFRA | R841_D842del | 7 | BRAF | F595S |
|---|--------|--------------|---|------|-------|
| 4 | PDGFRA | S566_E571>K | 7 | BRAF | G464R |
| 4 | PDGFRA | S566_E571>R | 7 | BRAF | G464V/G464E |
| 4 | PDGFRA | S566_E571>R | 7 | BRAF | G466 |
| 4 | PDGFRA | T674I | 7 | BRAF | G466R |
| 4 | PDGFRA | V561D | 7 | BRAF | G466V |
| 4 | PDGFRA | Y849C | 7 | BRAF | G469 |
| 5 | APC | APC_E1379* | 7 | BRAF | G469 |
| 5 | APC | APC_Q1338* | 7 | BRAF | G469 |
| 5 | APC | E1306* | 7 | BRAF | G469A |
| 5 | APC | E1309fs*4 | 7 | BRAF | G469S/G469E/G469A |
| 5 | APC | Q1367* | 7 | BRAF | G469S/G469E/G469A |
| 5 | APC | Q1378* | 7 | BRAF | G469S/G469E/G469A |
| 5 | APC | Q1429* | 7 | BRAF | G469V/G469R |
| 5 | APC | R1114* | 7 | BRAF | G469V/G469R |
| 5 | APC | R1450* | 7 | BRAF | G469V/G469R |
| 5 | APC | R876* | 7 | BRAF | G596R |
| 5 | APC | S1465fs*3 | 7 | BRAF | G615E |
| 5 | APC | T1661fs*9 | 7 | BRAF | I463S |
| 5 | CSF1R | L301* | 7 | BRAF | I592M |
| 5 | CSF1R | L301S | 7 | BRAF | I592V |
| 5 | CSF1R | Y969* | 7 | BRAF | K601del |
| 5 | CSF1R | Y969C | 7 | BRAF | K601E |
| 5 | CSF1R | Y969F | 7 | BRAF | K601E |
| 5 | CSF1R | Y969H | 7 | BRAF | K601N |
| 5 | FBX4 | G30N | 7 | BRAF | L597 |
| 5 | FBX4 | L23Q | 7 | BRAF | L597 |
| 5 | FBX4 | P76T | 7 | BRAF | L597Q/L597V |
| 5 | FBX4 | S12L | 7 | BRAF | L597Q/L597V |
| 5 | FBX4 | S8R | 7 | BRAF | L597S/L597R |
| 5 | FBX4 | S8R | 7 | BRAF | L597S/L597R |
| 5 | MEK | P124L | 7 | BRAF | N581S |
| 5 | MEK | Q56P | 7 | BRAF | R443T |
| 5 | MET | R1170Q | 7 | BRAF | R444Q |
| 5 | MET | T992I | 7 | BRAF | R444W |
| 7 | BRAF | D587A | 7 | BRAF | R444W |
| 7 | BRAF | D587E | 7 | BRAF | R462I |
| 7 | BRAF | D594E | 7 | BRAF | S605F |
| 7 | BRAF | D594V/D594G | 7 | BRAF | S605N |
| 7 | BRAF | E586K | 7 | BRAF | T599_V600insTT |
| 7 | BRAF | E586K | 7 | BRAF | T599I |
| 7 | BRAF | F468C | 7 | BRAF | V471F |
| 7 | BRAF | F595L | 7 | BRAF | V600 |

| 7 | BRAF | V600 | | 7 | EGFR | H773_V774insPH |
|---|------|------|---|---|------|-----------------|
| 7 | BRAF | V600A | | 7 | EGFR | H773>NPY |
| 7 | BRAF | V600D | | 7 | EGFR | H773R |
| 7 | BRAF | V600D | | 7 | EGFR | K745R |
| 7 | BRAF | V600E/V600K | | 7 | EGFR | L730F |
| 7 | BRAF | V600E/V600K | | 7 | EGFR | L747_E749del,A750P |
| 7 | BRAF | V600M | | 7 | EGFR | L747_E749del,A750P |
| 7 | BRAF | V600R/V600L | | 7 | EGFR | L747_P753>Q |
| 7 | BRAF | V600R/V600L | | 7 | EGFR | L747_P753>S |
| 7 | EGFR | A289V | | 7 | EGFR | L747_R748>FP |
| 7 | EGFR | A750P | | 7 | EGFR | L747_S752del, P753S |
| 7 | EGFR | D761N | | 7 | EGFR | L747_S752del,Q ins |
| 7 | EGFR | D761Y | | 7 | EGFR | L747_S752del,Q ins |
| 7 | EGFR | D770_N771>AGG | | 7 | EGFR | L747_T750del, P ins |
| 7 | EGFR | D770_N771>AGG | | 7 | EGFR | L747_T750del,P ins |
| 7 | EGFR | D770_N771insG | | 7 | EGFR | L747_T751>P |
| 7 | EGFR | D770_N771insG | | 7 | EGFR | L747_T751>P |
| 7 | EGFR | E709A/E709G/E709V | | 7 | EGFR | L747_T751>S |
| 7 | EGFR | E709K/E709H | | 7 | EGFR | L747_T751del |
| 7 | EGFR | E734K | | 7 | EGFR | L747_T751del |
| 7 | EGFR | E746_A750del | | 7 | EGFR | L858M |
| 7 | EGFR | E746_A750del | | 7 | EGFR | L858R |
| 7 | EGFR | E746_A750del, V ins | | 7 | EGFR | L858R |
| 7 | EGFR | E746_A750del, V ins | | 7 | EGFR | L861Q |
| 7 | EGFR | E746_A750del,T751A | | 7 | EGFR | M766_A767insAI |
| 7 | EGFR | E746_S752>A | | 7 | EGFR | N771_P772>SVDNR |
| 7 | EGFR | E746_S752>D | | 7 | EGFR | N771_P772>SVDNR |
| 7 | EGFR | E746_T751>A | | 7 | EGFR | P733L |
| 7 | EGFR | E746_T751del | | 7 | EGFR | P753S |
| 7 | EGFR | E746_T751del, I ins | | 7 | EGFR | P772_H773insV |
| 7 | EGFR | E746_T751del,I ins | | 7 | EGFR | R108K |
| 7 | EGFR | E746_T751del,S752D | | 7 | EGFR | S752_I759del |
| 7 | EGFR | E746_T751del,V ins | | 7 | EGFR | S752_I759del |
| 7 | EGFR | E746K | | 7 | EGFR | S752_I759del |
| 7 | EGFR | G598V | | 7 | EGFR | S752Y |
| 7 | EGFR | G719A | | 7 | EGFR | S768I |
| 7 | EGFR | G719D | | 7 | EGFR | SNP C2255T |
| 7 | EGFR | G719S/G719C | | 7 | EGFR | T263P |
| 7 | EGFR | G735S | | 7 | EGFR | T751A |
| 7 | EGFR | G810D | | 7 | EGFR | T790M |
| 7 | EGFR | G810S | | 7 | EGFR | T790M |
| 7 | EGFR | H773_V774insH | | 7 | EGFR | V742A |
| 7 | EGFR | H773_V774insNPH | | 7 | EGFR | V769_D770insASV |

| 7 | EGFR | V769_D770insASV | 9 | ABL1 | L248V |
|---|------|-----------------|---|------|-------|
| 7 | EGFR | V769_D770insASV | 9 | ABL1 | M244V |
| 7 | EGFR | V769_D770insASV | 9 | ABL1 | M351T |
| 7 | EGFR | V769_D770insCV | 9 | ABL1 | Q252H |
| 7 | EGFR | V774_C775insHV | 9 | ABL1 | T315I |
| 7 | EGFR | W731* | 9 | ABL1 | Y253F |
| 7 | EPHB6 | G404S | 9 | ABL1 | Y253H |
| 7 | EPHB6 | R679Q | 9 | CDKN2A | D84Y |
| 7 | MAP2K2 | F57C | 9 | CDKN2A | E61* |
| 7 | MAP2K2 | F57I | 9 | CDKN2A | E69* |
| 7 | MAP2K2 | F57L | 9 | CDKN2A | E88* |
| 7 | MAP2K2 | K61E | 9 | CDKN2A | H83Y |
| 7 | MAP2K2 | R388Q | 9 | CDKN2A | R58* |
| 7 | MET | H1112R | 9 | CDKN2A | R80* |
| 7 | MET | H1112Y | 9 | GNAQ | Q209L |
| 7 | MET | M1250T | 9 | GNAQ | Q209L/P |
| 7 | MET | M1268T | 9 | GNAQ | R183Q |
| 7 | MET | R970C | 9 | JAK2 | V617F |
| 7 | MET | T1010I | 9 | ROR2 | A793S |
| 7 | MET | T992I | 10 | FGFR2 | S252W |
| 7 | MET | Y1230C | 10 | FGFR2 | Y376C |
| 7 | MET | Y1235D | 10 | PTEN | K267fs*9 |
| 7 | MET | Y1248C | 10 | PTEN | K6fs*4 |
| 7 | MET | Y1248H | 10 | PTEN | N323fs*2 |
| 8 | FGFR1 | P252T | 10 | PTEN | N323fs*21 |
| 8 | FGFR1 | S125L | 10 | PTEN | P248fs*5 |
| 8 | MYC | A59V | 10 | PTEN | R130* |
| 8 | MYC | N101T | 10 | PTEN | R130fs*4 |
| 8 | MYC | P260A | 10 | PTEN | R130G |
| 8 | MYC | P57S | 10 | PTEN | R130Q |
| 8 | MYC | S77F | 10 | PTEN | R173C |
| 8 | MYC | T73I | 10 | PTEN | R173H |
| 8 | PTK2B | G414V | 10 | PTEN | R233* |
| 8 | PTK2B | R429C | 10 | PTEN | R335* |
| 9 | ABL1 | D276G | 10 | PTEN | V317fs*3 |
| 9 | ABL1 | E255K | 10 | RET | A664D |
| 9 | ABL1 | E255V | 10 | RET | A883F |
| 9 | ABL1 | E355G | 10 | RET | C634R |
| 9 | ABL1 | F311L | 10 | RET | C634R |
| 9 | ABL1 | F317L | 10 | RET | C634W |
| 9 | ABL1 | F359V | 10 | RET | C634W |
| 9 | ABL1 | G250E | 10 | RET | C634Y |
| 9 | ABL1 | H396R | 10 | RET | C634Y |

| 10 | RET | D631_L633>E | | 12 | KRAS | Q61H/Q61H |
|----|-----|-------------|---|----|------|-----------|
| 10 | RET | D631G | | 12 | KRAS | T58I |
| 10 | RET | D898_E901del | | 12 | PTPN11 | T507K |
| 10 | RET | E632_A640>VRP | | 13 | FLT3 | D835del |
| 10 | RET | E632_L633>V | | 13 | FLT3 | D835H/D835Y |
| 10 | RET | E632_L633del | | 13 | FLT3 | I836del |
| 10 | RET | E632_L633del | | 13 | FLT3 | |
| 10 | RET | E768D | | 13 | FLT3 | |
| 10 | RET | F612_C620del | | 13 | FLT4 | D835E |
| 10 | RET | F612_C620del | | 13 | FLT4 | D835E |
| 10 | RET | M918T | | 13 | FLT5 | I836M |
| 10 | RET | M918T | | 13 | RB1 | C706F |
| 11 | HRAS | G12C | | 13 | RB1 | E137* |
| 11 | HRAS | G12R | | 13 | RB1 | E748* |
| 11 | HRAS | G12V/G12D | | 13 | RB1 | L199* |
| 11 | HRAS | G13C/G13R/G13S | | 13 | RB1 | L660fs*2 |
| 11 | HRAS | Q61H/Q61H | | 13 | RB1 | R320* |
| 11 | HRAS | Q61K | | 13 | RB1 | R358* |
| 11 | HRAS | Q61L/Q61R/Q61P | | 13 | RB1 | R455* |
| 12 | CDK | R24C | | 13 | RB1 | R552* |
| 12 | CDK | R24H | | 13 | RB1 | R556* |
| 12 | CDK4 | R24C | | 13 | RB1 | R579* |
| 12 | CDK4 | R24H | | 14 | AKT1 | E17del |
| 12 | KRAS | A146T | | 14 | AKT1 | E319G |
| 12 | KRAS | A59T | | 14 | AKT1 | E17K |
| 12 | KRAS | A59V | | 14 | AKT1 | E17K |
| 12 | KRAS | G12 | | 14 | AKT1 | L357P |
| 12 | KRAS | G12 | | 14 | AKT1 | P388T |
| 12 | KRAS | G12A/G12C/G12D | | 14 | AKT1 | Q43X |
| 12 | KRAS | G12A/G12C/G12D | | 14 | AKT1 | V167A |
| 12 | KRAS | G12F/G12R | | 14 | AKT1 | V461L |
| 12 | KRAS | G12F/G12R | | 15 | MAP2K1 | D67N |
| 12 | KRAS | G12S/G12V | | 15 | MAP2K1 | E203Q/K |
| 12 | KRAS | G12S/G12V | | 15 | MAP2K1 | F53S |
| 12 | KRAS | G13A | | 15 | MAP2K1 | K57N |
| 12 | KRAS | G13A/D/V | | 15 | MAP2K1 | Y134C |
| 12 | KRAS | G13R | | 17 | ERBB2 | A775_G776 insYVMA |
| 12 | KRAS | G13V/G13D | | 17 | ERBB2 | D769H |
| 12 | KRAS | G60D | | 17 | ERBB2 | G776S/G776LC |
| 12 | KRAS | L19F | | 17 | ERBB2 | G776VC |
| 12 | KRAS | Q22K | | 17 | ERBB2 | L755P |
| 12 | KRAS | Q61 | | 17 | ERBB2 | P780_Y781 insGSP |
| 12 | KRAS | Q61E/Q61K | | 17 | ERBB2 | P780_Y781 insGSP |

| 17 | ERBB2 | S779_P780 insVGS | | 19 | GNA11 | Q209 |
|----|-------|------------------|---|----|-------|------|
| 17 | ERBB3 | V777L | | 19 | GNA11 | R183C |
| 17 | TP53 | D281G | | 19 | JAK3 | A572V |
| 17 | TP53 | D281H/Y | | 19 | JAK3 | P132T |
| 17 | TP53 | G245R/S/C | | 19 | JAK3 | V722I |
| 17 | TP53 | G245S | | 19 | STK11 | D194N |
| 17 | TP53 | R175H | | 19 | STK11 | D194V |
| 17 | TP53 | R175H/L | | 19 | STK11 | E199* |
| 17 | TP53 | R248G/W | | 19 | STK11 | E199K |
| 17 | TP53 | R248Q | | 19 | STK11 | E57fs*7 |
| 17 | TP53 | R248W | | 19 | STK11 | F264fs*22 |
| 17 | TP53 | R273C | | 19 | STK11 | G196V |
| 17 | TP53 | R273C | | 19 | STK11 | P281fs*6 |
| 17 | TP53 | R273H | | 19 | STK11 | P281L |
| 17 | TP53 | R273H/L | | 19 | STK11 | Q170* |
| 17 | TP53 | R306* | | 19 | STK11 | Q37* |
| 17 | TP53 | V143A | | 19 | STK11 | W332* |
| 19 | AKT2 | R371H | | 20 | SRC | Q531* |
| 19 | AKT2 | S302G | | | | |

**6.** The induced malignant stem cell capable of *in vitro* proliferation according to claim 1, wherein the abnormal expression (increased or reduced/lost expression) of an endogenous oncogene or an endogenous tumor suppressor gene under (1) (c) above occurs in at least one of the genes mentioned in (1) (b).

**7.** The induced malignant stem cell capable of *in vitro* proliferation according to claim 6, wherein the abnormal expression of an endogenous oncogene or an endogenous tumor suppressor gene is an increased expression of the endogenous oncogene or a reduced/lost expression of the endogenous tumor suppressor gene.

**8.** The induced malignant stem cell capable *of in vitro* proliferation according to claim 1, wherein the abnormal expression (increased or reduced/lost expression) of a noncoding RNA such as an endogenous cancer-related microRNA under (1) (d) above occurs in at least one of the microRNAs listed in the following table:

[Table 4]

| Table 4: Cancer-related microRNAs that might cause abnormal expression (condition (1) (d)) | |
|---|---|
| Cancers | miRNA |
| Brain Cancer | let-7g ; mir-10a ; mir-124-2 ; mir-126 ; mir-149 ; mir-155 ; mir-15b Cluster (mir-15b, mir-16-2) ; mir-17 cluster (mir-17, mir-18a, mir-19a, mir-19b-1, mir-20a, mir-92a-1) ; miR-191 Cluster (miR-191, miR-425) ; mir-210 ; mir-218-1 ; mir-218-2 ; mir-23b Cluster (mir-23b, mir-24-1, mir-27b) ; mir-301a ; mir-30c-1 Cluster (mir-30c-1, mir-30e) ; mir-32 ; mir-34a ; mir-378 ; mir-7-1 |
| Breast Cancer | mir-155 ; mir-17 cluster (mir-17, mir-18a, mir-19a, mir-19b-1, mir-20a, mir-92a-1) |
| Colon Cancer | mir-17 cluster (mir-17, mir-18a, mir-19a, mir-19b-1, mir-20a, mir-92a-1) ; mir-378 |
| Head & Neck Cancer | let-7i ; mir-10a ; mir-155 ; mir-15b Cluster (mir-15b, mir-16-2) ; mir-17 cluster (mir-17, mir-18a, mir-19a, mir-19b-1, mir-20a, mir-92a-1) ; mir-210 ; mir-218-1 ; mir-218-2 ; mir-23b Cluster (mir-23b, mir-24-1, mir-27b) ; mir-30c-1 Cluster (mir-30c-1, mir-30e) ; mir-34a ; mir-378 |

(continued)

| Table 4: Cancer-related microRNAs that might cause abnormal expression (condition (1) (d)) | |
|---|---|
| Cancers | miRNA |
| Kidney Cancer | mir-210 |
| Liver Cancer | mir-126 ; mir-17 cluster (mir-17, mir-18a, mir-19a, mir-19b-1, mir-20a, mir-92a-1) ; miR-191 Cluster (miR-191, miR-425) ; mir-193b ; mir-23b Cluster (mir-23b, mir-24-1, mir-27b) ; mir-30c-1 Cluster (mir-30c-1, mir-30e) |
| Lung Cancer | let-7i ; mir-1-1 ; mir-126 |
| Lymphoma | mir-155 ; mir-23b Cluster (mir-23b, mir-24-1, mir-27b) ; mir-378 |
| Ovarian Cancer | let-7i ; mir-126 ; mir-155 ; mir-196a-1 ; mir-34a ; mir-34c Cluster (mir-34c, mir-34b) |
| Pancreatic Cancer | mir-10a ; mir-155 ; mir-210 ; mir-23b Cluster (mir-23b, mir-24-1, mir-27b) |
| Prostate Cancer | mir-149 ; mir-15b Cluster (mir-15b, mir-16-2) |
| Skin Cancer | mir-149 ; mir-15b Cluster (mir-15b, mir-16-2) ; mir-17 cluster (mir-17 ; mir-18a ; mir-19a ; mir-19b-1 ; mir-20a ; mir-92a-1) mir-193b ; mir-23b Cluster (mir-23b, mir-24-1, mir-27b) |

9. The induced malignant stem cell capable *of in vitro* proliferation according to claim 1, wherein the abnormal expression of an endogenous cancer-related protein under (1) (e) above is either due to an increased expression or reduced/lost expression of protein as compared with the expression in induced pluripotent stem cells or due to the expression of a cancer-specific antigen.

10. The induced malignant stem cell capable of *in vitro* proliferation according to claim 9, wherein the protein that might show abnormal expression (increased expression or reduced/lost expression) or the cancer-specific antigen is either one of Muc-1, VEGF-C, HnRNP A2/B1, E2F3, MAGE A4, MMP-9, Cytokeratin-19, E2F1, c-kit, Muc-4, Cytokeratin-20, c-met, L-myc, MDR1, hCG$\beta$, COX-2, CA125, MAGE A12, NSE, c-myc, CD44, Her2/Neu, RCAS1, bcl-2, FGFR2, HIF-1$\alpha$, GPC3, Cyclin D1, mdm2, Cytokeratin-7, MMP-2, Survivin, hTERT, Gli1, Thyroglobulin, VEGF-A, AFP, CEA, CGA, EGFR, MAGE A1, MAGE A3/A6, Muc-7, ProGRP, PSA, SCC, IGF2, DLK-1, and WT-1.

11. The induced malignant stem cell capable *of in vitro* proliferation according to claim 1, wherein the aberration of endogenous cancer-related metabolism (hypermetabolism or hypometabolism) under (1) (f) is **characterized by** having a metabolomic aberration compared with induced pluripotent stem cells or showing an enhancement in the glycolysis system as compared with induced pluripotent stem cells.

12. The induced malignant stem cell capable of *in vitro* proliferation according to claim 1, wherein the aberration of endogenous cancer-related sugar chain under (1) (g) is either due to abnormal expression of sugar chain as compared with induced pluripotent stem cells or due to the expression of cancer-specific sugar chain.

13. The induced malignant stem cell capable of *in vitro* proliferation according to claim 1, wherein the aberration of copy number variations in endogenous genomic DNA under (1) (h) indicates an increase or decrease in the genetic copy number in the cell under test as compared with the genomic DNA of a reference cell.

14. The induced malignant stem cell capable of *in vitro* proliferation according to claim 1, wherein the instability of microsatellites in endogenous genomic DNA in an induced malignant stem cell under (1) (i) is an alteration in the repeat number of microsatellites in mismatch repair genes *MLH1 gene, MSH2 gene, MSH6 gene,* and *PMS2 gens.*

15. The induced malignant stem cell capable of *in vitro* proliferation according to any one of claims 1 to 14, wherein the genes under (2) above are expressed in amounts ranging from one eighth to eight times the amounts expressed in an undifferentiated embryonic stem cell as a control.

16. The induced malignant stem cell capable of *in vitro* proliferation according to any one of claims 1 to 15, wherein the cell is a human cell.

17. A process for producing an induced malignant stem cell capable *of in vitro* proliferation, **characterized by** performing an induction step where a starter somatic cell prepared from a fresh cancer tissue or a non-cancer tissue taken from a carcinogenic mammal is placed in such a state that the genetic product or products of one to six genes selected from among *POU5F1 gene, SOX2 gene, c-Myc gene, KLF4 gene, LIN28 gene,* and *NANOG gene* are present in the starter somatic celL

18. The process for producing an induced malignant stem cell capable of *in vitro* proliferation according to claim 17, wherein the genetic product is a gene, RNA, or a protein.

19. The process for producing an induced malignant stem cell capable *of in vitro* proliferation according to claim 17 or 18, wherein the fresh cancer tissue is fresh cancer tissue of a solid cancer or of a carcinoma.

20. The process for producing an induced malignant stem cell capable *of in vitro* proliferation according to any one of claims 17 to 19, wherein the starter somatic cell is prepared from a fresh cancer tissue selected from among stomach cancer, colon cancer, breast cancer, kidney cancer, lung cancer, and liver cancer.

21. A cancer cell induced and prepared from the induced malignant stem cell capable *of in vitro* proliferation according to claim 1.

22. A method of screening selected from among a method of screening for a target in the discovery of a cancer therapeutic drug, a method of screening for a candidate for cancer therapeutic drug, and a method of screening for a cancer diagnostic drug, which is **characterized by** using the induced malignant stem cell capable of *in vitro* proliferation according to claim 1 or the cancer according to claim 21.

23. The method of screening according to claim 22, which is performed using either one of a nucleic acid such as siRNA, cDNA, microRNA, or antisense RNA/DNA, a low-molecular weight compound, a peptide, and an antibody, or combinations thereof.

24. A process for preparing a cancer vaccine **characterized by** using the induced malignant stem cell capable of *in vitro* proliferation according to claim 1 or the cancer according to claim 21.

25. A process for preparing a cancer model animal **characterized by** transplanting the induced malignant stem cell capable of *in vitro* proliferation according to claim 1 or the cancer according to claim 21 into a laboratory animal.

26. A method of identifying a methylator phenotype, a mutator phenotype, a driver mutation, or a target in drug discovery that are characteristic of cancer by omics analysis using the induced malignant stem cell capable of *in vitro* proliferation according to claim 1.

27. The method of identifying according to claim 26, wherein the omics analysis is selected from epigenomic analysis, genomic analysis, trascriptome analysis, proteome analysis, glycome analysis, or metabolme analysis.

28. A methylator phenotype, a mutator phenotype, a driver mutation, or a target in drug discovery that is characteristic of cancer, as identified by the method of identifying according to claim 26 or 27.

29. A pharmaceutical candidate directed to the methylator phenotype, mutator phenotype, driver mutation, or target in drug discovery that is characteristic of cancer according to claim 28.

30. The pharmaceutical candidate according to claim 29, which is a nucleic acid such as siRNA, cDNA, microRNA, or antisense RNA/DNA, a low-molecular weight compound, a peptide, or an antibody.

Fig. 1-1

238

# Fig. 1-2

(5) lesion: NGC1_6 / normal site: ngc1

Analysis Results: MSS

(6) lesion: GC1_10 / normal site: ngc1

Analysis Results: MSS

(7) lesion: cc1 / normal site: ncc1

Analysis Results: MSS

(8) lesion: CC1_1 / normal site: ncc1

Analysis Results: MSS

Judging Criteria
MSS: 0 marker
MSI-L: 1 marker
MSI-H: 2markers

Fig. 1-3

(9)  lesion: CC1_2 / normal site: ncc1

(10)  lesion: CC1_7 / normal site: ncc1

(11)  lesion: CC1_8 / normal site: ncc1

(12)  lesion: CC1_9 / normal site: ncc1

# Fig. 1-4

**(13) lesion: CC1_11 / normal site: ncc1**

Analysis Results: MSS

Judging Criteria
- MSS: 0 marker
- MSI-L: 1 marker
- MSI-H: 2markers

**(14) lesion: CC1_12 / normal site: ncc1**

Analysis Results: MSS

Judging Criteria
- MSS: 0 marker
- MSI-L: 1 marker
- MSI-H: 2markers

**(15) lesion: CC1_17 / normal site: ncc1**

Analysis Results: MSS

Judging Criteria
- MSS: 0 marker
- MSI-L: 1 marker
- MSI-H: 2markers

**(16) lesion: CC1_18 / normal site: ncc1**

Analysis Results: MSS

Judging Criteria
- MSS: 0 marker
- MSI-L: 1 marker
- MSI-H: 2markers

# Fig. 1-5

(17)  lesion: cc4 / normal site: ncc4

Analysis Results: MSS

Judging Criteria

MSS: 0 marker
MSI-L: 1 marker
MSI-H: 2markers

(18)  lesion: CC4_c / normal site: ncc4

Analysis Results: MSS

Judging Criteria

MSS: 0 marker
MSI-L: 1 marker
MSI-H: 2markers

(19)  lesion: CC4_(3)  / normal site: ncc4

Analysis Results: MSS

Judging Criteria

MSS: 0 marker
MSI-L: 1 marker
MSI-H: 2markers

(20)  lesion: CC4_(6)  / normal site: ncc4

Analysis Results: MSS

Judging Criteria

MSS: 0 marker
MSI-L: 1 marker
MSI-H: 2markers

# Fig. 1-6

(21)   lesion: CC4_(3)  _10 / normal site: ncc4

Analysis Results: MSS

BAT25:(+) · BAT26:(+) · D2S123:(+)

Normal

Lesion

D5S346:(+) · D17S250:(+)

Normal

Lesion

Judging Criteria
| |
|---|
| MSS: 0 marker |
| MSI-L: 1 marker |
| MSI-H: 2markers |

(22)   lesion: CC4_(4)    / normal site: ncc4

Analysis Results: MSS

BAT25:(+) · BAT26:(+) · D2S123:(+)

Normal

Lesion

D5S346:(+) · D17S250:(+)

Normal

Lesion

Judging Criteria
| |
|---|
| MSS: 0 marker |
| MSI-L: 1 marker |
| MSI-H: 2markers |

(23)   lesion: CC4_30 / normal site: ncc4

Analysis Results: MSS

BAT25:(+) · BAT26:(+) · D2S123:(+)

Normal

Lesion

D5S346:(+) · D17S250:(+)

Normal

Lesion

Judging Criteria
| |
|---|
| MSS: 0 marker |
| MSI-L: 1 marker |
| MSI-H: 2markers |

(24)   lesion: CC4_10 / normal site: ncc4

Analysis Results: MSS

BAT25:(+) · BAT26:(+) · D2S123:(+)

Normal

Lesion

D5S346:(+) · D17S250:(+)

Normal

Lesion

Judging Criteria
| |
|---|
| MSS: 0 marker |
| MSI-L: 1 marker |
| MSI-H: 2markers |

EP 2 749 642 A1

# Fig. 1-7

(25)  lesion: CC4_31 / normal site: ncc4

Analysis Results: MSS

BAT25:(+)   BAT26:(+)   D2S123:(+)

Normal

Lesion

D5S346:(+)   D17S250:(+)

Normal

Lesion

Judging Criteria
MSS: 0 marker
MSI-L: 1 marker
MSI-H: 2markers

(26)  lesion: CC4(1)  / normal site: ncc4

Analysis Results: MSS

BAT25:(+)   BAT26:(+)   D2S123:(+)

Normal

Lesion

D5S346:(+)   D17S250:(+)

Normal

Lesion

Judging Criteria
MSS: 0 marker
MSI-L: 1 marker
MSI-H: 2markers

(27)  lesion: CC4(2)  / normal site: ncc4

Analysis Results: MSS

BAT25:(+)   BAT26:(+)   D2S123:(+)

Normal

Lesion

D5S346:(+)   D17S250:(+)

Normal

Lesion

Judging Criteria
MSS: 0 marker
MSI-L: 1 marker
MSI-H: 2markers

(28)  lesion: CC4_D / normal site: ncc4

Analysis Results: MSS

BAT25:(+)   BAT26:(+)   D2S123:(+)

Normal

Lesion

D5S346:(+)   D17S250:(+)

Normal

Lesion

Judging Criteria
MSS: 0 marker
MSI-L: 1 marker
MSI-H: 2markers

EP 2 749 642 A1

Fig. 1-8

(29)  lesion: NFB1_2 / normal site: 7F3956

Analysis Results: MSS

(30)  lesion: NFB2_17 / normal site: 7F3949

Analysis Results: MSS

# Fig. 2

Fig. 3-1

NCC1 LP20% 10_J21\1\1 SRef

NCC1

247

Fig. 3-2

Fig. 3-3

Fig. 3-4

Fig. 3-5

Fig. 3-6

Fig. 3-7

CC4-c

Fig. 3-8

EP 2 749 642 A1

Fig. 3-9

Fig. 4-1

(a)

(b)

# Fig. 4-2

(c)

(d)

**INTERNATIONAL SEARCH REPORT**

| International application No. |
| --- |
| PCT/JP2012/081723 |

A. CLASSIFICATION OF SUBJECT MATTER
*C12N5/095*(2010.01)i, *C12N5/09*(2010.01)i, *C12N5/10*(2006.01)i

According to International Patent Classification (IPC) or to both national classification and IPC

B. FIELDS SEARCHED

Minimum documentation searched (classification system followed by classification symbols)
C12N1/00-7/08

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JMEDPlus/JST7580(JDreamII), BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
| --- | --- | --- |
| X | KUMANO,K., et al., "Generation of induced pluripotent stem cells from primary chronic myelogeneous leukemia patient sample" Blood, 2010, Vol.116,No.21, p.518, Abst1206 | 1-27 |
| X | GRAD,I., et al., "Nanog priming before full reprogramming may generate germ cell tumors" Eur. Cells & Materials, 2011.11.09, Vol.22, p.258-274 | 17,18 |
| P,X | WO 2011/148983 A1 (National Cancer Center), 01 December 2011 (01.12.2011), (Family: none) | 1-27 |
| A | WO 2011/026222 A1 (MCMASTER UNIVERSITY), 10 March 2011 (10.03.2011), & CA 2688760 A | 1-27 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 30 January, 2013 (30.01.13) | 12 February, 2013 (12.02.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/081723

C (Continuation).   DOCUMENTS CONSIDERED TO BE RELEVANT

| Category* | Citation of document, with indication, where appropriate, of the relevant passages | Relevant to claim No. |
|---|---|---|
| A | LEE,M-Y., et al., "Transcriptional regulation of Rex1(zfp42) in normal prostate epithelial cells and prostate cancer cells" J.Cell Physiol., 2010, Vol.224, p.17-27 | 1-27 |
| A | Hajime HIROSE et al., "Kokei Gan no Gan Kansaibo", Japanese Journal of Cancer and Chemotherapy, 2010, vol.37, no.13, pages 2809 to 2812 | 1-27 |
| A | Hideki MASAKI et al., "Gan Kansaibo o Hyoteki to shita Soyaku no Genjo", Experimental Medicine, 2008, vol.26, no.8, pages 1232 to 1238 | 1-27 |
| A | Naoki MAKITA, Shunji NAGAHARA, "Recent progress of nucleic acid delivery system mediated by atelocollagen", Drug Delivery System, 2010, vol.25-6, pages 607 to 614 | 1-27 |
| A | Fumitaka TAKESHITA, Takahiro OCHIAI, "miRNA delivery system", Genome Medicine, 2009, vol.9, no.1, pages 49 to 52 | 1-27 |
| A | Takeshi YAMAGUCHI, Shunji NAGAI, "Ten'isei Daichogan ni Okeru panitumumab no Saikin no Tenkai ni Tsuite", Biotherapy, 2010, vol.24, no.5, pages 408 to 411 | 1-27 |
| A | Yataro DAIGO, Yusuke NAKAMURA, "Haigan no Shinki Biomarker Tansaku", The LUNG perspectives, 2008, vol.16, no.4, pages 69 to 75 | 1-27 |

Form PCT/ISA/210 (continuation of second sheet) (July 2009)

## INTERNATIONAL SEARCH REPORT

| International application No. |
| --- |
| PCT/JP2012/081723 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
    because they relate to subject matter not required to be searched by this Authority, namely:

2. ☒ Claims Nos.: 28-30
    because they relate to parts of the international application that do not comply with the prescribed requirements to such an extent that no meaningful international search can be carried out, specifically:
    The invention described in claims 28-30 relate to phenotypes, mutations, targets and candidates for medicinal agents for the phenotypes, the mutations and the targets which are produced by an omics-based analysis. (Continued to extra sheet)

3. ☐ Claims Nos.:
    because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2012/081723

Continuation of Box No.II-2 of continuation of first sheet(2)

However, it is quite unclear as to what types of substances are included specifically within the scopes of the phenotypes, the mutations, the targets for candidates for medicinal agents and the candidates, and therefore the statements in these claims are remarkably unclear.

Therefore, meaningful opinion cannot be stated with respect to novelty, inventiveness and industrial applicability of the inventions set forth in the afore-mentioned claims.

Form PCT/ISA/210 (extra sheet) (July 2009)

**EP 2 749 642 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008283972 A
- JP 2008307007 A
- JP HEI2227075 A
- JP 3229590 B
- JP 3499528 B


**Non-patent literature cited in the description**

- **HOCHEDLINGER K ; JAENISCH R et al.** *Genes Dev.,* 2004, vol. 18, 1875-1885
- **NAKAGAWA M ; YAMANAKA S et al.** *Nat Biotechnol.,* 2008, vol. 26, 101-106
- **TAKAHASHI K ; YAMANAKA S et al.** *Cell,* 2007, vol. 131, 861-872
- **MASAKI H ; ISHIKAWA T et al.** *Stem Cell Res.,* 2008, vol. 1, 105-115
- *International Society for Stem Cell Research,* 2009, 285
- **UTIKAL J et al.** *J Cell Sci.,* 2009, vol. 122, 3502-3510
- **CARETTE JE et al.** *Blood,* 2010, vol. 115, 4039-4042
- **NAGAI K et al.** *Biochem Biophys Res Commun.,* 2010, vol. 395, 258-263
- **MIYOSHI et al.** *Proc Natl Acad Sci USA.,* 2010, vol. 107, 40-5
- **GISSELSSON D. et al.** *Exp Cell Res,* 2010, vol. 316, 3379-3386
- **VISVADER JE ; LINDEMAN GJ.** *Nat Rev Cancer.,* 2008, vol. 8, 755-768
- **STEPHENS P.J. et al.** *Cell,* 2011, vol. 144, 27-40
- **NAVIN N. ; HICKS J.** *Genome Med,* 2011, vol. 3, 31
- **NAVIN N.** *Nature,* 2011, vol. 472, 90-94
- **TOSHIO KITAMURA.** Retrovirus-mediated gene transfer and expression cloning: Powerful tools in functional genomics. *Experimental Hematology,* 2003, vol. 31 (11), 1007-14
- *Proc. Natl. Acad. Sci. USA,* 1995, vol. 92, 6733-6737
- *Nucleic Acids Research,* 1990, vol. 18, 3587-3596
- *Journal of Virology,* 1998, vol. 72, 8150-8157
- *Nucleic Acids Res.,* 1995, vol. 23, 3816-3821
- *Proc. Natl. Acad. Sci., USA,* 1987, vol. 84, 7413
- **THOMSON JA et al.** Embryonic stem cell lines derived from human blastocysts. *Science,* 06 November 1998, vol. 282 (5391), 1145-7
- *Science,* 04 December 1998, vol. 282 (5395), 1827
- **HIROFUMI SUEMORI et al.** Efficient establishment of human embryonic stem cell lines and long term maintenance with stable karyotype by enzymatic bulk passage. *Biochemical and Biophysical Research Communications,* 2006, vol. 345, 926-32
- **BOLAND CR et al.** *Cancer Res.,* 1998, vol. 58, 5248-57
- **LOUKOLA, A et al.** *Cancer Res.,* 01 June 2001, vol. 61 (11), 4545-9
- *Mol. Cell. Proteomics,* 2005, vol. 4, 1977-1989